# EUROPEAN PATENT SPECIFICATION

(11) **EP 1 580 265 B1**
(45) Date of publication and mention of the grant of the patent: **29.09.2010**
(21) Application number: 03770029.1
(22) Date of filing: 30.10.2003
(51) Int. Cl.: C12N 15/09, C12Q 1/68, G01N 33/50

(54) **NUCLEIC ACIDS ISOLATED FROM NEUROBLASTOMA AT STAGE 4S**
AUS EINEM NEUROBLASTOM IN STUFE 4S ISOLIERTENUKLEINSÄUREN
ACIDES NUCLEIQUES ISOLES D'UN NEUROBLASTOME AU STADE 4S

(30) Priority: 30.10.2002 JP 2002316586
(43) Date of publication of application: 28.09.2005
(73) Proprietor: Hisamitsu Pharmaceutical Co., Inc., Tosu-shi, Saga 841-0017 (JP); Chiba-Prefecture, Chiba-shi, Chiba 260-8667 (JP)
(72) Inventor: NAKAGAWARA, Akira, Chiba Cancer Center Res. Inst., Chiba-shi, Chiba 206-0801 (JP); OHIRA, Miki, c/o Chiba Cancer Center Res. Inst., Chiba-shi, Chiba 260-0801 (JP)
(74) Representative: Grünecker, Kinkeldey, Stockmair & Schwanhäusser Anwaltssozietät
(86) International application number: PCT/JP2003/013932
(87) International publication number: WO 2004/039975

(56) References cited:
- DATABASE EMBL [Online] 11 September 2001 (2001-09-11), "Homo sapiens BAC clone RP11-650J17 from 4, complete sequence." XP002377844 retrieved from EBI accession no. EM_PRO:AC093879 Database accession no. AC093879
- KAWAMOTO T ET AL: "ASSOCIATION BETWEEN FAVORABLE NEUROBLASTOMA AND HIGH EXPRESSION OF THE NOVEL METALLOPROTEINASE GENE, NBLA3145/XCE, CLONED BY DIFFERENTIAL SCREENING OF THE FULL-LENGTH-ENRICHED OLIGO-CAPPING NEUROBLASTOMA CDNA LIBRARIES" MEDICAL AND PEDIATRIC ONCOLOGY, LISS, NEW YORK, NY, US, vol. 35, no. 6, December 2000 (2000-12), pages 628-631, XP009053429 ISSN: 0740-8226
- ISLAM ASHRAFUL ET AL: "High expression of Survivin, mapped to 17q25, is significantly associated with poor prognostic factors and promotes cell survival in human neuroblastoma" ONCOGENE, vol. 19, no. 5, 3 February 2000 (2000-02-03), pages 617-623, XP002377836 ISSN: 0950-9232
- AOYAMA M ET AL: "High expression of human RIM gene in neuroblastomas with favorable biologies" SOCIETY FOR NEUROSCIENCE ABSTRACTS, vol. 27, no. 1, 2001, page 715, XP009065593 & 31ST ANNUAL MEETING OF THE SOCIETY FOR NEUROSCIENCE; SAN DIEGO, CALIFORNIA, USA; NOVEMBER 10-15, 2001 ISSN: 0190-5295
- TANG X X ET AL: "Implications of EPHB6, EFNB2, and EFNB3 expressions in human neuroblastoma." PROCEEDINGS OF THE NATIONAL ACADEMY OF SCIENCES OF THE UNITED STATES OF AMERICA. 26 SEP 2000, vol. 97, no. 20, 26 September 2000 (2000-09-26), pages 10936-10941, XP002377837 ISSN: 0027-8424
- EGGERT, A. ET AL: 'High-level expression of angiogenic factors is associated with advanced tumor stage in human neuroblastomas' CLIN CANCER RES. vol. 6, no. 5, 2000, pages 1900 - 1908, XP002976278
- GALLEGO, S. ET AL: 'Differential polymerase chain reaction with serial dilutions for quantification of MYCN gene amplification in neuroblastoma' ANTICANCER RES vol. 18, no. 2A, 1998, pages 1211 - 1215, XP002977603
- SHUICHI YAMANE: 'Shinkei Gashugun shuyo ni okeru Saibo Shogaisei T saibo ni yoru Ninshiki sareru Shuyo Taishuku Kogen no Idenshi Hatsugen, (Gene expression of tumor rejection antigens recognized by cytolytic T lymphocytes in neuroblastoma-related tumors)' JOURNAL OF KYOTO PREFECTURAL UNIVERSITY OF MEDICINE vol. 108, no. 3, 1999, pages 381 - 388, XP002976279
- THE SANGER CENTER, ET AL: 'Toward a Complete Human Genome Sequence' GENOME RES vol. 8, no. 11, 1998, pages 1097 - 1108, XP000910017 & DATABASE GENBANK [Online] Database accession no. (AC093879)

## Description

### Technical Field

This invention relates to the use of nucleic acids derived from a gene expressed in human neuroblastoma. More particularly, the invention relates to the use of nucleic acids derived from a gene expressed in human stage 4s neuroblastoma. Further, this invention relates to diagnostic kits for stage 4s neuroblastoma comprising nucleic acid primers or nucleic acid microarrays utilizing those nucleic acids.

### Background Art

### (Tumorgenesis and Genes)

Individual tumors exhibit distinct characteristic natures, and their biological properties are not necessarily identical even though the basic principle of oncogenesis is the same. Rapid advances in the understanding of cancer from a molecular biological and molecular genetic perspective in recent years have opened the way to an explanation of oncogenesis and the so-called tumor cell biology on the genetic level.

### (Neuroblastoma)

Neuroblastoma is a pediatric cancer occurring in sympathetic gangliocytes and adrenal medullary cells which originate from cells of the peripheral sympathetic nervous system. Of these sympathetic neurons, neural crest cells in the initial stage of development migrate to the abdomen, differentiating and maturing at sites where sympathetic ganglia are formed. Some of these cells migrate further to the adrenal bodies, penetrating through the adrenal cortex which is already in the process of formation, and reaching the medulla and forming medullary substance there. The neural crest cells also serve as a source of other peripheral nerve cells, differentiating into dorsal root ganglia (sensory nerves), skin pigment cells, thyroid C cells, some pulmonary cells, intestinal gangliocytes, and the like.

### (Prognosis for Neuroblastoma)

Neuroblastoma is characterized by a varied clinical profile (Nakagawara, Shinkeigashu no Hassei to Sono Bunshi Kiko [Neuroblastoma Development and Molecular Mechanism], Shoni Naika [Japanese Journal of Pediatric Medicine], Vol. 30, p. 143, 1998). For example, neuroblastoma occurring at less than one year of age has very favorable prognosis, with the majority undergoing differentiation and cell death, and results in spontaneous regression (which may be referred to as "favorable prognosis type"). Currently, most neuroblastomas discovered by a positive result in the commonly performed mass screening of 6-month-old infant urine are of the type which tend to undergo this spontaneous regression. On the other hand, neuroblastoma occurring at age 1 or higher is highly malignant and leads to death of the infant in the majority of cases despite therapy (which may be referred to as "unfavorable prognosis type"). It is also hypothesized that a somatic mutation occurs in highly malignant neuroblastomas in infants older than one year of age, which are of monoclonal nature, whereas in naturally regressing neuroblastomas, the genetic mutation remains at only a germ line mutation. See Knudson AG, et al.: Regression of neuroblastoma IV-S: A genetic hypothesis, N. Engl. J. Med., Vol. 302, p. 1254, 1980. In addition, there are known neuroblastomas of the intermediate type that are clinically positioned between those two types.

When the neuroblastomas are classified according to the progress of tumorization, they are as follows:
Stage 1: the tumor occurs primarily in adrenal gland or sympathetic ganglia and is confined.
Stage 2: the tumor is characterized by being confined to the site of origin and regional metastasis only to lymph nodes, which does not extends beyond the median line.
Stage 3: the tumor extends beyond the median line to invade into the opposite side or to metastasize into lymph nodes.
Stage 4: the tumor causes distant metastasis to bone, bone marrow or the orbital region.
Stage 4s: the tumor occurs at less than one year of age and causes distant metastasis to bone marrow, skin or liver.

The neuroblastomas of the favorable prognosis type are tumors at stages 1, 2, and 4s, while the neuroblastomas of the unfavorable and intermediate types are tumors at stages 3 and 4. The tumor at stage 4s is peculiar and normally occurs in an infant at several months after birth. Although the tumor grows and metastasizes quickly, it suddenly stops growing and then disappears spontaneously. Thus, the tumors that regress spontaneously and the tumors that grow malignantly are clearly distinct, with respect to the age of onset, the site of metastasis, and the progress.

### (Genes which allow the prediction of prognosis for neuroblastoma)

With recent advances in molecular biology research, it has become clear that expression of the high affinity nerve growth factor (NGF) receptor TrkA is closely connected with control of differentiation and cell death (see Nakagawara A., The NGF story and neuroblastoma, Med. Pediatr. Oncol., vol. 31, p. 113, 1998). Trk is a membrane-spanning receptor, existing as the three major types, Trk-A, -B and -C.

These Trk family receptors play an important role in specific nerve cell differentiation and survival in the central nervous and peripheral nervous systems (see Nakagawara, et al., Shinkeigasaiboushu ni Okeru Neurotrophin Juyoutai no Hatsugen to Yogo [Expression of Neurotrophin Receptors and Prognosis in Neuroblastoma], Shoni Geka (Japanese Journal of Pediatric Surgery), Vol. 29, pp. 425-432, 1997). The survival and differentiation of tumor cells is controlled by signals from Trk tyrosine kinase and Ret tyrosine kinase. In particular, the role of TrkA receptor is most significant, with TrkA expression being notably high in neuroblastomas of the favorable prognosis type, and its signals exerting a powerful control over the survival and differentiation of tumor cells, and cell death (apoptosis). In neuroblastomas of the unfavorable prognosis type, on the other hand, TrkA expression is significantly suppressed, while tumor development is aided by a mechanism in which survival is promoted by signals from TrkB and Ret, instead.

It has become clear that amplification of the neural oncogene N-myc is associated with the prognosis of neuroblastoma (see Nakagawara, Nou-shinkeishuyo no Tadankai Hatsugan [Multistage Oncogenesis of Cerebral and Neural Tumors], Molecular Medicine, Vol. 364, p. 366, 1999). This gene, first cloned in neuroblastoma, is ordinarily only present in a single copy per haploid set in normal cells and neuroblastomas of the favorable prognosis type, whereas it has been found to be amplified several dozen times in neuroblastomas of the unfavorable prognosis type.

In addition to the genes described above, CD44, PTN, caspase and others are known as the gene whose expression is high in neuroblastomas of the favorable prognosis type, whereas SVV (survivin), MK (midkine) and others are known as the gene whose expression is high in neuroblastomas of the unfavorable prognosis type.

Furthermore, the present inventors found that a group of novel genes was highly expressed in neuroblastomas of the favorable prognosis type (International Publication PCT/JP01631 pamphlet). By contrast, the present inventors found that a different group of novel genes was highly expressed in neuroblastomas of the unfavorable prognosis type (International Publication PCT/JP01629 pamphlet).

Up till the present time, however, there has hardly been any information concerning the genes which are expressed in stage 4s neuroblastoma, particularly with specificity. Further, since stage 4s neuroblastoma regresses spontaneously, there is an urgent need to identify the causative genes.

### Disclosure of the Invention

This invention has been accomplished in light of the problems inherent in the above-described prior art, and its object is to identify the nucleic acid sequences of genes which are generally related to the favorable or unfavorable prognosis of neuroblastoma, and to allow for providing such genetic information and for diagnosis for the prognosis of neuroblastoma (whether favorable or unfavorable). This invention specifically aims at diagnosing the prognosis of neuroblastoma, classifying the progress of the neuroblastoma, and enabling the determination of stage 4s neuroblastoma.

As a result of conducting diligent research, the present inventors have examined the prognoses of neuroblastomas and have succeeded in constructing cDNA libraries from the respective clinical tissues of the favorable and unfavorable prognosis types. Approximately 2,400 clones were respectively cloned from these two types of cDNA libraries and were classified according to the prognoses of neuroblastomas (whether favorable or unfavorable). Profiling of the genes in the respective subsets was then carried out.

Thus, the present inventors found that a group of genes showed differential expression levels among the abovementioned subsets and showed enhanced expression levels only in the clinical tissues of the favorable prognosis type. Moreover, the present inventors found that a group of genes showed enhanced expression levels only in the clinical tissues of the unfavorable prognosis type. Based on such finding, the present inventors made it possible to provide nucleic acid sequence data which would allow the detection and cloning of genes whose expression levels are at least enhanced either only in the clinical tissues of the favorable prognosis type or only in the clinical tissues of the unfavorable prognosis type.

Furthermore, the present inventors have succeeded in constructing cDNA libraries similarly from the clinical tissues of stage 4s neuroblastoma. Approximately 2,700 clones were cloned from this library. The subset of this library and the subsets of the libraries from the clinical tissues of the favorable and unfavorable prognosis types were analyzed and the profiling of approximately 16,000 genes that were expressed among these subsets was carried out. Consequently, 452 genes were identified that showed differential expression levels among the abovementioned subsets. When these genes were sequenced, they were found to comprise 308 novel genes and 144 known genes as the reminder. The genes were classified according to their expression patterns among the respective subsets, and grouped into seven groups.

Based on such finding, the present inventors made it possible to provide genetic information (such as nucleic acid sequence data) which would allow the detection and cloning of genes exhibiting expression patterns characteristic of stage 4s neuroblastoma. Further based on the nucleic acid sequence data, the present inventors made it possible to provide diagnostic agents and diagnostic kits which allow for methods of diagnosis for the prognosis of neuroblastoma (particularly, the classification of the progress), including determination on stage 4s neuroblastoma, upon which this invention has been completed.

Specifically, this invention provides:
1. A diagnostic kit for stage 4s neuroblastoma comprising a pair of primers as the active component, wherein one primer comprises a DNA sequence set forth in SEQ ID NO:175 or a DNA complementary thereto and the other primer comprises a DNA sequence set forth in SEQ ID NO:176 or a sequence complementary thereto.
2. A method for determining stage 4s neuroblastoma, the method comprising detecting the presence or absence of a nucleic acid comprising the nucleic acid sequence set forth in SEQ ID NO:1 in the Sequence Listing from a clinical tissue sample of neuroblastoma.
3. Use of a nucleic acid microarray for diagnosing stage 4s neuroblastoma, wherein the microarray comprises a solid phase support and a nucleic acid which comprises the nucleic acid sequence set forth in SEQ ID NO:1 in the Sequence Listing immobilized on the solid phase support, wherein the diagnosing comprises detecting the presence or absence of a nucleic acid comprising the sequence set forth in SEQ ID NO:1 in a clinical tissue sample using the nucleic acid sequence set forth in SEQ ID NO:1.
4. Use of a nucleic acid microarray for diagnosing stage 4s neuroblastoma, comprising a solid phase support and a nucleic acid which comprises the nucleic acid sequence set forth in SEQ ID NO:175 or SEQ ID NO:176 immobilized on the solid phase support, wherein the diagnosing comprises detecting the presence or absence of a nucleic acid comprising the sequence set forth in SEQ ID NO:1 in a clinical tissue sample using the nucleic acid sequence set forth in SEQ ID NO:175 or SEQ ID NO:176.

### Best Mode for Carrying Out the Invention

The nucleic acids (which will be referred to as "the nucleic acid(s) of this invention") derived from the gene which is expressed in neuroblastoma according to this invention (which will be referred to as "the gene(s) of this invention") including their utility, will be described in detail by referring to the preferred embodiments of the invention.

As stated above, the nucleic acids of this invention are derived from the gene of the invention and they make up the gene or are obtained from the gene by an *in vivo* or *in vitro* process. Herein, they will be referred to as "the nucleic acid(s) of the invention," which include nucleic acid fragments corresponding to portions of the gene. When the nucleic acid chain length is short, it can be synthesized by chemical techniques.

The term "nucleic acid(s)" as used in this specification refers to, for example, RNA or RNA, or polynucleotides derived therefrom which are active as DNA or RNA, and preferably refers to DNA or RNA. The particularly preferred nucleic acid has a DNA sequence that is identical with the human cDNA sequence disclosed in this specification or that is complementary to the sequence.

The term "hybridize under stringent conditions" as used in this specification means that two nucleic acid (or fragments thereof) hybridize to each other under the hybridization conditions as described in Sambrook, J. et al. in "expression or cloned genes in E. coli", Molecular Cloning: A Laboratory Manual (1989), Cold Spring Harbor Laboratory Press, New York, USA, pp. 9.47-9.62 and pp. 11.45-11.61.

More specifically, the "stringent conditions" refers to hybridization at approximately 45 °C with 6.0 x SSC, followed by washing at 50 °C with 2.0 x SSC. The stringency may be selected by choosing a salt concentration in the washing step from approximately 2.0 x SSC, 50 °C as low stringency to approximately 0.2 x SSC, 50 °C as high stringency. Also, the temperature in the washing step may be increased from room temperature, or approximately 22 °C as low stringency conditions, to approximately 65 °C as high stringency conditions.

The term "nucleic acid(s)" as used in this specification refers to an isolated nucleic acid(s) and to a nucleic acid or a polynucleotide containing substantially no cellular substances or culture medium, if prepared by recombinant DNA techniques, or containing substantially no precursor chemical substances or other chemical substances, if prepared by chemical synthesis.

The term "favorable prognosis type" as used in this specification refers to a condition of human neuroblastoma in which the tumor exists with confinement or has become a regressing or benign sympathetic ganglion neoplasm, and is judged to have low malignancy based on N-myc or other tumor markers (TrkA, chromosomal aberration or the like) by the practicing physician. Neuroblastoma of the favorable prognosis type is considered a case of stage 1 or 2, with an onset age of less than one year and survival without recurrence for 5 or more years after surgery, and with no noted amplification of N-myc in the clinical tissue; however, it is not limited to such a specific case. The term "unfavorable prognosis type" as used in this specification refers to conditions of human neuroblastoma in which progression of the tumor has been observed, and it is judged to have high malignancy based on N-myc or other tumor markers by the practicing physician. Neuroblastoma of the unfavorable prognosis type is considered a case of stage 4, with an onset age of greater than one year, death within 3 years after surgery and noted amplification of N-myc in the clinical tissue; however, it is not limited to such a specific case.

Although stage 4s neuroblastoma is classified as the favorable prognosis type in accordance with the clinical molecular biology classification as described above, it will be treated differently from "the favorable prognosis type" for the sake of convenience in this specification.

Neuroblastoma is a tumor consisting of actual neurons, of which only two types are known in humans, and analysis of the genes expressed therein is expected to provide very useful knowledge for understanding the biology of neurons. Specifically, it is extremely difficult, and practically impossible, to obtain a site-specific homogeneous tissue from the brain or peripheral nerves. On the other hand, neuroblastoma consist of an almost homogeneous population of neurons (though tumorized) derived from peripheral sympathetic neurons, and thus offers a high possibility of obtaining homogeneous expression of neuro-related genes. Furthermore, since neuroblastoma is a type of cancer, it is characteristically pointed out that there are many important genes expressed in the immature stage of neurogenesis.

Clinically and biologically, neuroblastoma can be further distinctly classified into the favorable prognosis type and the unfavorable prognosis types. Cancer cells from neuroblastoma of the favorable prognosis type are characterized by having a very slow rate of proliferation, with spontaneous regression beginning at some point. Findings to date have conformed that neuronal differentiation and apoptosis (neuronal cell death) occur in the spontaneous regression, and that the differentiation which occurs in the maturation stages of normal neurons and programmed cell death are phenomena very closely resembling each other. Consequently, it is highly probable that the analysis of genes expressed in such tumors will lead to obtaining important genetic information relating to neuronal differentiation and apoptosis.

The nucleic acids of this invention originating in the gene of the invention from which the useful genetic information described above can be obtained have been found in clinical tissues of stage 4s neuroblastomas (which may be referred to as "4s" hereafter). When the expression levels of this gene is compared between a clinical tissue of the favorable prognosis type (which may be abbreviated as "F(favorable)" hereafter) and a clinical tissue of the unfavorable prognosis type (which may be abbreviated as "U(unfavorable)" hereafter), the gene has the characteristics described below.

Specifically, 452 genes are described below which were obtained in the manner described above and of which were sequenced at least partially have been classified according to their expression patterns between the respective subsets to form seven groups, which will be described in the following.

### Group I

The genes belonging to this group have expression levels (4s) that are on the same order as those of UF, but are lower than those of F. These genes are further classified into subgroups, resulting in I-1, I-2 and 1-3. Table 1 should be consulted on the gene expression pattern of each subgroup.

The specific clones belonging to I-1 are nbla20026 (SEQ ID NO:171), nbla20421 (SEQ ID NO:172), nbla22298 (SEQ ID NO:173), nbla22549 (SEQ ID NO:174), and nbla23020, all of which are novel genes.

The specific clones belonging to I-2 are as follows: nbla20113, nbla20146 (SEQ ID NO:137), nbla20170 (SEQ ID NO:138), nbla20216 (SEQ ID NO:139), nbla20253, nbla20549, nbla20657 (SEQ ID NO:140), nbla20688 (SEQ ID NO:141), nbla20755 (SEQ ID NO:142), nbla20835, nbla20968, nbla21013 (SEQ ID NO:143), nbla21087, nbla21172 (SEQ ID NO:144), nbla21189, nbla21200 (SEQ ID NO:145), nbla21214, nbla21255 (SEQ ID NO:146), nbla21337, nbla21344, nbla21345 (SEQ ID NO:147), nbla21410 (SEQ ID NO:148), nbla21522 (SEQ ID NO:149), nbla21631 (SEQ ID NO:150), nbla21788 (SEQ ID NO:151), nbla21897 (SEQ ID NO:152), nbla21956, nbla22116 (SEQ ID NO:153), nbla22223 (SEQ ID NO:154), nbla22228, nbla22344 (SEQ ID NO:155), nbla22351, nbla22361, nbla22474, nbla22629, nbla22939 (SEQ ID NO:156), nbla23084 (SEQ ID NO:157), nbla23103 (SEQ ID NO:158), nbla23234 (SEQ ID NO:159), nbla23300 (SEQ ID NO:160), nbla23369 (SEQ ID NO:161), nbla23436 (SEQ ID NO:162), nbla23511 (SEQ ID NO:163), nbla23664 (SEQ ID NO:164), nbla23775, nbla23860 (SEQ ID NO:165), nbla23877 (SEQ ID NO:166), nbla23998 (SEQ ID NO:167), nbla24043 (SEQ ID NO:168), nbla24182, nbla24285, nbla24402 (SEQ ID NO:169), nbla24434, nbla24460, nbla24762, nbla24821 (SEQ ID NO:170), nbla24893, nbla24973, and nbla24986, all of which are novel genes; and nbla20279, nbla20687, nbla20924, nbla21168, nbla21303, nbla21483, nbla2l838, nbla21917, nbla22099, nbla22438, nbla23111, nbla23208, nbla24118, nbla24279, nbla24771, and nbla24871, all of which are known genes.

The specific clones belonging to I-3 are as follows: nbla20084 (SEQ ID NO:129), nbla21081 (SEQ ID NO:130), nbla21420 (SEQ ID NO:131), nbla21761, nbla22452 (SEQ ID NO:132), nbla22595 (SEQ ID NO:133), nbla22676 (SEQ ID NO:134), nbla22909 (SEQ ID NO:135), nbla23456, nbla24297, nbla24435 (SEQ ID NO:136), and nbla24719, all of which are novel genes; and nbla20117, nbla20238, nbla20904, nbla23293, nbla23297, nbla23311, nbla23589, nbla23629, nbla23862, nbla2413, and nbla24761, all of which are known genes.

### Group II

The genes belonging to this group have expression levels (4s) that are on the same order as those of F, but are higher than those of UF. These genes are further classified into subgroups, resulting in II-1, II-2 and II-3. Table 1 should be consulted on the gene expression pattern of each subgroup.

The specific clones belonging to II-1 are as follows: nbla20365 (SEQ ID NO:117), nbla20378 (SEQ ID NO:118), nbla20511 (SEQ ID NO:119), nbla21039 (SEQ ID NO:120), nbla21107 (SEQ ID NO:121), nbla21367 (SEQ ID NO:122), nbla21790 (SEQ ID NO:123), nbla21855, nbla22253 (SEQ ID NO:124), nbla22355 (SEQ ID NO:125), nbla22704, nbla22832 (SEQ ID NO:126), nbla23394, nbla23512, nbla23755 (SEQ ID NO:127), nbla24084, nbla24376, and nbla24549 (SEQ ID NO:128), all of which are novel genes; and nbla20624, nbla22029, nbla22424, nbla22594 and nbla22622, all of which are known genes.

The specific clones belonging to II-2 are as follows: nbla20001 (SEQ ID NO:58), nbla20083 (SEQ ID NO:59), nbla20125, nbla20182 (SEQ ID NO:60), nbla20231, nbla20248 (SEQ ID NO:61), nbla20250 (SEQ ID NO:62), nbla20268, nbla20330 (SEQ ID NO:63), nbla20395, nbla23973, nbla23983 (SEQ ID NO:64), nbla24041, nbla24082, nbla24104, nbla24111 (SEQ ID NO:65), nbla24142 (SEQ ID NO:66), nbla24157 (SEQ ID NO:67), nbla24230 (SEQ ID NO:68), nbla24239, nbla20541 (SEQ ID NO:69), nbla20555 (SEQ ID NO:70), nbla20638, nbla20645 (SEQ ID NO:71), nbla20713 (SEQ ID NO:72), nbla20765, nbla20789, nbla20792, nbla20798, nbla21024, nbla24250 (SEQ ID NO:73), nbla24254 (SEQ ID NO:74), nbla24327 (SEQ ID NO:75), nbla24363, nbla24510 (SEQ ID NO:76), nbla24554 (SEQ ID NO:77), nbla24604 (SEQ ID NO:78), nbla24622, nbla24646, nbla24672, nbla21037 (SEQ ID NO:79), nbla21077, nbla21089, nbla21130, nbla21161 (SEQ ID NO:80), nbla21170 (SEQ ID NO:81), nbla21198 (SEQ ID NO:82), nbla21266, nbla21298 (SEQ ID NO:83), nbla21379 (SEQ ID NO:84), nbla24705 (SEQ ID NO:85), nbla24709, nbla24748, nbla24831, nbla24972, nbla21385 (SEQ ID NO:86), nbla21413, nbla21416 (SEQ ID NO:87), nbla21520, nbla21599 (SEQ ID NO:88), nbla21681 (SEQ ID NO:89), nbla21878 (SEQ ID NO:90), nbla21922 (SEQ ID NO:91), nbla21936, nbla22004-2 (SEQ ID NO:92), nbla22004-1 (SEQ ID NO:93), nbla22028, nbla22085 (SEQ ID NO:94), nbla22093, nbla22119 (SEQ ID NO:95), nbla22149 (SEQ ID NO:96), nbla22161 (SEQ ID NO:97), nbla22218, nbla22252 (SEQ ID NO:98), nbla22347 (SEQ ID NO:99), nbla22352 (SEQ ID NO:100), nbla22394 (SEQ ID NO:101), nbla22423 (SEQ ID NO:102), nbla22439 (SEQ ID NO:103), nbla22451, nbla22455, nbla22464, nbla22465, nbla22487, nbla22633 (SEQ ID NO:104), nbla22669, nbla22698 (SEQ ID NO:105), nbla22726, nbla22886, nbla22896 (SEQ ID NO:106), nbla23012, nbla23038, nbla23167 (SEQ ID NO:107), nbla23339 (SEQ ID NO:108), nbla23352 (SEQ ID NO:109), nbla23575 (SEQ ID NO:110), 23592 (SEQ ID NO:111), nbla23601 (SEQ ID NO:112), nbla23630 (SEQ ID NO:113), nbla23718, nbla23719, nbla23754 (SEQ ID NO:114), nbla23892 (SEQ ID NO:115), nbla23951, and nbla23956 (SEQ ID NO:116), all of which are novel genes; and nbla20393, nbla20423, nbla20510, nbla20833, nbla20931, nbla20943, nbla21258, nbla21268, nbla21273, nbla21412, nbla21578, nbla21614, nbla21624, nbla21655, nbla21670, nbla21787, nbla21954, nbla21979, nbla22043, nbla22137, nbla22192, nbla22325, nbla22327, nbla22337, nbla22482, nbla22763, nbla22788, nbla22839, nbla22851, nbla22935, nbla22937, nbla23238, nbla23327, nbla23360, nbla23519, nbla23553, nbla23554, nbla23683, nbla23812, nbla23823, nbla23849, nbla23882, nbla23910, nbla24064, nbla24405, nbla24897, and nbla24913, all of which are known genes.

The specific clones belonging to II-3 are as follows: nbla20134, nbla20181, nbla20264 (SEQ ID NO:31), nbla20269 (SEQ ID NO:32), nbla20276, nbla204O6 (SEQ ID NO:33), nbla20709, nbla20782, nbla20788, nbla20949 (SEQ ID NO:34), nbla21046, nbla21122, nbla21211, nbla21233, nbla21251 (SEQ ID NO:35), nbla2l334 (SEQ ID NO:36), nbla21356 (SEQ ID NO:37), nbla21375, nbla21418 (SEQ ID NO:38), nbla21480 (SEQ ID NO:39), nbla21509 (SEQ ID NO:40), nbla21524, nbla21527 (SEQ ID NO:41), nbla21551 (SEQ ID NO:42), nbla21735 (SEQ ID NO:43), nbla21843, nbla21934, nbla22153, nbla22247 (SEQ ID NO:44), nbla22382, nbla22477 (SEQ ID NO:45), nbla22571, nbla22639 (SEQ ID NO:46), nbla22789, nbla23060, nbla23174 (SEQ ID NO:47), nbla23198 (SEQ ID NO:48), nbla23218, nbla23328 (SEQ ID NO:49), nbla23420 (SEQ ID NO:50), nbla23483 (SEQ ID NO:51), nbla23545, nbla23653, nbla23666, nbla23760, nbla23808 (SEQ ID NO:52), nbla23830, nbla23851 (SEQ ID NO:53), nbla23942, nbla24011 (SEQ ID NO:54), nbla24131, nbla24235 (SEQ ID NO:55), nbla24556 (SEQ ID NO:56), nbla24800 (SEQ ID NO:57), and nbla24908, all of which are novel genes; and nbla20133, nbla20263, nbla20723, nbla20748, nbla20915, nbla21016, nbla21034, nbla21067, nbla21167, nbla21319, nbla21331, nbla21516, nbla21682, nbla21691, nbla2l822, nbla21976-2, nbla21977, nbla22159, nbla22168, 22215-1, nbla22244, nbla22263, nbla22548, nbla23033, nbla23231, nbla23284, nbla23329-1, nbla23384, nbla23556, nbla23674, nbla23879-2, nbla24098, nbla24329, nbla24334, nbla24439-1, nbla24443, nbla24507, nbla24836, nbla24958, and nbla24989, all of which are known genes.

### Group III

The genes belonging to this group have expression levels (4s) that are on the same order as those of F, but are lower than those of UF. These genes are further classified into subgroups, resulting in III-1, III-2 and III-3. Table 1 should be consulted on the gene expression pattern of each subgroup.

The specific clones belonging to III-1 are nbla20874 (novel gene) and nbla23262 (known gene).

The specific clones belonging to III-2 are as follows: nbla20604, nbla21226, nbla21908 (SEQ ID NO:27), nbla21928, nbla22027 (SEQ ID NO:28), nbla22082 (SEQ ID NO:29), nbla22643, nbla23303(SEQ ID NO:30), nbla23649, and nbla24468, all of which are novel genes; and nbla20141, nbla20446, nbla21538, nbla21558, nbla21623, nbla21969, nbla22219, nbla23272, nbla23307 and nbla24117, all of which are known genes.

The specific clones belonging to III-3 are as follows: nbla20578 (SEQ ID NO:26), and nbla21212, both of which are novel genes; and nbla23478, nbla23896 and nbla24920, all of which are known genes.

### Group IV

The genes belonging to this group have expression levels (4s) that are on the same order as those of UF, but are higher than those of F (F<4s=UF). The specific genes belonging to this group are nbla23899 (SEQ ID NO:25) and nbla24526, both of which are novel genes.

### Group V

The genes belonging to this group have expression levels (4s) that are lower than those of F, but are higher than those of UF. These genes are further classified into subgroups, resulting in V-1, V-2, V-3, V-4 and V-5. Table 1 should be consulted on the gene expression pattern of each subgroup.

The specific clone belonging to V-1 is nbla22031 (known gene). The specific clone belonging to V-2 is nbla22305 (known gene).

The specific clones belonging to V-3 are as follows: nbla20123 (SEQ ID NO:17), nbla20382 (SEQ ID NO:18), nbla20660 (SEQ ID NO:19), nbla20666 (SEQ ID NO:20), nbla21239 (SEQ ID NO:21), nbla21729 (SEQ ID NO:22), nbla21831 (SEQ ID NO:23), nbla22826 (SEQ ID NO:24), and nbla24521, all of which are novel genes; and nbla20235 and nbla22607, both of which are known genes.

The specific clones belonging to V-4 are nbla20787 (SEQ ID NO:15), nbla22284 (SEQ ID NO:16) and nbla24756, all of which are novel genes.

The specific clones belonging to V-5 are nbla24348 and nbla24686, both of which are novel genes.

### Group VI

The genes belonging to this group have expression levels (4s) that are lower than either of F and UF, or are higher than either of F and UF. These genes are further classified into subgroups, resulting in VI-1, VI-2, VI-3, VI-4, VI-5, VI-6, VI-7 and VI-8. Table 1 should be consulted on the gene expression pattern of each subgroup.

The specific clones belonging to VI-1 are nbla21297 (SEQ ID NO:14) (novel gene) and nbla22443 (known gene).

The specific clones belonging to VI-2 are as follows: nbla20211, nbla20469, nbla21250, nbla22182 (SEQ ID NO:12), nbla22761, nbla23256 (SEQ ID NO:13), nbla23631, nbla23711, nbla24532, and nbla24951, all of which are novel genes; and nbla21750, nbla22129, nbla22808, nbla23064, and nbla23358, all of which are known genes.

The specific clone belonging to VI-3 is nbla20226 (SEQ ID NO:11) (novel gene).

The specific clones belonging to VI-4 are nbla21650 (SEQ ID NO:7), nbla22094 (SEQ ID NO:8), nbla22739 (SEQ ID NO:9), and nbla23525 (SEQ ID NO:10), all of which are novel genes.

The specific clones belonging to VI-5 are nbla23701 (SEQ ID NO:5) and nbla23890 (SEQ ID NO:6), both of which are novel genes.

The specific clone belonging to VI-6 is nbla20087 (known gene).

The specific clones belonging to VI-7 are nbla22689 (SEQ ID NO:2), nbla22968, nbla24079, nbla24135 (SEQ ID NO:3), and nbla24350 (SEQ ID NO:4), all of which are novel genes.

The specific clone belonging to VI-8 is nbla22256 (novel gene).

### Group VII

The gene belonging to this group (only one) is expressed only in 4s. The specific clone is nbla22420 (SQ ID NO:1) (novel gene).

The gene groups have been divided into novel genes and known genes with respect to each group. A summary is shown in Table 1.

**Table 1**

| Group | Expression pattern | Novel genes | Known genes | Total |
|---|---|---|---|---|
| I-1 | F»4s=UF | 5 | 0 | 5 |
| I-2 | F>4s=UF | 59 | 16 | 75 |
| I-3 | F≥4s=UF | 12 | 11 | 23 |
| II-1 | F=4s»UF | 18 | 5 | 23 |
| II-2 | F=4s>UF | 105 | 47 | 152 |
| II-3 | F=4s≥UF | 55 | 40 | 95 |
| III-1 | F=4s«UF | 1 | 1 | 2 |
| III-2 | F=4s<UF | 10 | 10 | 20 |
| III-3 | F=4s<UF | 2 | 3 | 5 |
| IV | F<4s=UF | 2 | 0 | 2 |
| V-1 | F>4s»UF | 0 | 1 | 1 |
| V-2 | F≥4s»UF | 0 | 1 | 1 |
| V-3 | F>4s>UF | 9 | 2 | 11 |
| V-4 | F≥4s>UF | 3 | 0 | 3 |
| V-5 | F≥4s≥UF | 2 | 0 | 2 |
| VI-1 | F»4s<UF | 1 | 1 | 2 |
| VI-2 | F>4s<UF | 10 | 5 | 15 |
| VI-3 | F>4s≤UF | 1 | 0 | 1 |
| VI-4 | F≥4s≤UF | 4 | 0 | 4 |
| VI-5 | F<4s»UF | 2 | 0 | 2 |
| VI-6 | F≤4s»UF | 0 | 1 | 1 |
| VI-7 | F<4s>UF | 5 | 0 | 5 |
| VI-8 | F≤4s≥UF | 1 | 0 | 1 |
| VII | 4s only | 1 | 0 | 1 |
| Total clone number | | 308 | 144 | 452 |

In the table and the above classification, "="shows that the gene expression levels are nearly equal between the subsets.

Specifically, with respect to a group of genes belonging to group VI, when their expression levels in the stage 4s neuroblastoma and the expression levels of the same genes in clinical samples of the neuroblastomas of the favorable and unfavorable prognosis types are compared, they are specific in the stage 4s neuroblastoma: that is, their expression levels are significantly higher or lower than in either type of neuroblastomas. Therefore, if the presence of at least one of those genes is detected in a clinical tissue sample, it can be judged that there is a high probability of the sample being stage 4s neuroblastoma.

The gene belonging to Group VII is detected only in a clinical tissue of stage 4s neuroblastoma. Therefore, if the presence of this gene is detected in a clinical tissue sample, it can be judged that there is a high probability of the sample being stage 4s neuroblastoma.

Further, with respect to a group of genes belonging to one of the remaining groups, when their expression levels in the stage 4s neuroblastoma and the expression levels of the same genes in clinical samples of the neuroblastomas of the favorable and unfavorable prognosis types are compared, the expression patterns as described above can be found. Therefore, if plural expression patterns of those genes are detected and analyzed, it can be judged as to whether the clinical tissue sample to be assayed is stage 4s neuroblastoma. Particularly, when the nucleic acids described herein are used for this purpose, it is preferred that a nucleic acid microarray (which will be described below) be constructed and provided for determining neuroblastomas.

Thus, the nucleic acids described herein are useful as tumor markers to diagnose the favorable or unfavorable, prognosis of neuroblastoma. Specifically, the nucleic acid described herein will allow for providing various genetic information on or relating to the prognosis of human neuroblastoma through the following means.

### (1) Probes for use in hybridization

The nucleic acid described herein can be used as a probe (the nucleic acid probe of this invention) for hybridization to detect the gene of the invention expressed in neuroblastoma. The nucleic acid described herein can also be used as a probe for hybridization in order to determine gene expression in various tumors and normal tissues, to identify the distribution of the gene expression.

When the nucleic acid described herein is used as a probe for hybridization, there are no particular limitations on the actual method of hybridization. As preferred methods there may be mentioned, for example, Northern hybridization, Southern hybridization, colony hybridization, dot hybridization, fluorescence *in situ* hybridization (FISH), *in situ* hybridization (ISH), DNA chip methods, and microarray methods.

As one application example of the hybridization methods, the nucleic acid described herein can be used as a probe for Northern hybridization to measure the length of mRNA or to quantitatively detect gene expression in a clinical tissue sample to be assayed.

As another application example, the nucleic acid of this invention can be used as a probe for Southern hybridization to detect the presence or absence of a DNA sequence in the genomic DNA of a clinical tissue sample to be assayed.

As still another application example, the nucleic acid described herein can also be used as a probe for the FISH method to identify the location of a gene of the invention on a chromosome.

As a further application example, the nucleic acid described herein can also be used as a probe for the ISH method to identify the tissue distribution of expression of a gene of the invention.

When the nucleic acid of this invention is used as a probe for hybridization, a base length of at least 20 is necessary; and among the nucleic acids of this invention, a nucleic acid comprising 20 or more contiguous bases is preferably used. More preferably, the nucleic acid comprising 40 or more contiguous bases is used and most preferably a nucleic acid comprising 60 or more contiguous bases is used. Further, there may be used a nucleic acid comprising the full-length of any of the nucleic acid sequences set forth in SEQ ID NO:1 to SEQ ID NO:174 in the Sequence Listing.

The nucleic acid probe techniques in the various hybridization methods described above are well known to one skilled in the art, and for example, the conditions suitable for hybridization between a probe of this invention with each individual base length and the target polynucleotide may be readily determined. In order to obtain hybridization conditions optimal to probes containing varying base lengths, Sambrook et al. "Molecular Cloning: A Laboratory Manual" (loc. cit.) may be consulted and followed for such manipulations which are well known to one skilled in the art.

The probe may preferably be labeled for use in an easily detectable fashion. The detectable label may be any type and any element or compound which can be detected either visually or using devices. As commonly used detectable labels, there may be mentioned radioactive isotopes, avidin and biotin and fluorescent substances (FITC or Rhodamins). The radioactive isotopes are ³²P, ¹⁴C, ¹²⁵I, ³H, ³⁵S etc. Biotin-abeled nucleotides may be incorporated into nucleic acids by nick translation, or chemical or enzymatic means. The biotin-labeled probes are detected after hybridization using labeling means such as avidin/streptavidin, fluorescent labels, enzymes, gold colloidal complexes or the like. The nucleic acid probe of this invention may also be labeled by binding with a protein. For this purpose, a radioactive or fluorescent histone single-stranded binding protein may be used, for example. In this manner, a suitably labeled probe constitutes a diagnostic agent of this invention.

### (2) Primers for use in PCR

In order to detect the gene described herein other than through the hybridization, primers can be designed after any nucleic acid (DNA) sequence contained in the nucleic acid described herein and the polymerase chain reaction (PCR) method can be used. For example, mRNA may be extracted from a clinical tissue sample to be assayed, and the gene expression can be semi-quantitatively measured by RT-PCR. Such method may be carried out by a method well known to one skilled in the art. For example, "Molecular Cloning: A Laboratory Manual," (loc. cit.) and or Idenshibyo Nyumon [Introduction to Genetic Diseases] (Takahisa, S.: Nankodo Publishing) may be consulted.

When the nucleic acid (DNA) described herein is used as a PCR primer, a base length of 10 to 60 is necessary; and among portions of the nucleic acid sequences described herein, the nucleic acid having 10 to 60 contiguous bases is preferably used. More preferably, one having 15 to 30 bases is used. Generally, a primer sequence with a GC content of 40-60% is preferred. Also, there should preferably be no difference in the Tm values of the two primers used for amplification. Desirably, the primers do not anneal at their 3'-ends and do not adopt any secondary structures within the primers.

### (3) Gene screening

The nucleic acid of this invention can also be used to detect the expression (or the distribution) of a gene of the invention which is expressed in a tissue sample of neuroblastoma. This is accomplished by using the nucleic acid of this invention as a primer for PCR as described above.

The expression distribution of the gene can also be detected using a microarray. That is, the nucleic acid of the invention may be directly attached to the chip or array. There is known a method by which nucleic acids (DNA) are spotted to a substrate for the purpose of attaching them to a chip or array by using a high precision dispenser (see, for example, United State Patent No. 5,807,522). mRNA extracted from a clinical tissue sample may be labeled with a fluorescent substance or the like, hybridized thereto, and an analysis can be made of the type of tissue cells with high expression of the gene. The DNA attached to the chip or the array may be the reaction product of PCR using the nucleic acid or its fragment of this invention. As an alternative method, the nucleic acid fragment of this invention (DNA fragment) may be directly synthesized on a substrate to form a DNA chip or a DNA array (see, for example, United State Patent No. 5,424,186).

### (4) DNA cloning

The nucleic acid described herein can be used for cloning a gene which is expressed in human neuroblastoma. For example, by using the nucleic acid of the invention as a probe for Northern hybridization or colony hybridization, or as a primer for PCR, cloning of a gene containing the nucleic acid described herein is possible. The genes capable of being cloned include, among others, genes displaying differential expression levels between neuroblastomas of the unfavorable prognosis type and neuroblastomas of the unfavorable prognosis type, genes expressed in stage 4s neuroblastoma, genes displaying expression in a pattern different from the expression patterns in other tissues or cancer cells, genes expressed in a cell cycle dependent manner, genes induced by nerve differentiation, and genes whose expression is regulated by oncogenes or cancer suppressor genes.

### (5) Methods for diagnosing tumor prognosis and tumor markers to be used therefore

As mentioned above, expression of the gene described herein was found in stage 4s neuroblastoma (including neuroblastomas of the favorable and unfavorable prognosis types). Therefore, the nucleic acid described herein can be used as a probe for hybridization, or as a primer for PCR to investigate the expression pattern of the gene in a clinical tissue sample to be assayed taken from the subject, which enables the diagnosis for prognosis (i.e., determining stage 4s neuroblastoma). The methods of detecting the gene include Northern blotting hybridization, *in situ* hybridization and RT-PCR, as mentioned above among others.

When hybridization is employed, the amount of the nucleic acid hybridizing to the nucleic acid probe mentioned above in a clinical tissue sample to be assayed is compared with the control sample (e.g., clinical tissue samples from neuroblastomas of the favorable and unfavorable prognosis types) and the gene expression is determined. For the respective nucleic acids used to detect the gene expression patterns, their expression patterns may then be, for example, compared with those listed in Table 1 and analyzed to enable the diagnosis for prognosis. For this purpose, the nucleic acid microarray mentioned above may desirably be used. When RT-PCR is employed instead, mRNA is extracted from the sample and reverse-transcribed into DNA, amplification is performed using the aforementioned primers, and the gene expression is semi-quantitatively measured. Similarly to the manner mentioned above, the diagnosis for prognosis can be made. For this purpose, it is preferred to utilize a diagnostic kit containing a pair of the primers as essential components. In addition to the primer components, the diagnostic kit also includes known components such as PCR buffer, detergent solution and enzymes.

### (6) Antisense oligonucleotides

Described below are antisense oligonucleotides to the nucleic acids described herein. The antisense oligonucleotides are capable of hybridizing to the nucleic acids described herein, and include antisense DNAs and antisense RNAs. Antisense DNA inhibits transcription of mRNA from DNA, while antisense RNA inhibits translation of mRNA. These antisense oligonucleotides may be synthesized using an automated synthesizer or by PCR using the nucleic acids described herein as templates. The antisense oligonucleotides also encompass antisense oligonucleotide derivatives having improved binding affinity for DNA or mRNA, tissue selectivity, cell permeability, nuclease resistance and intracellular stability. These derivatives may be synthesized using the antisense technology known in the art.

Antisense oligonucleotides having sequences complementary to the sequences near the translation initiation codon of the mRNA, those of the ribosome-binding site, and those of the capping site or the splicing site are capable of inhibiting synthesis of the RNA, and therefore, will exhibit a particularly notable inhibitory effect on gene expression.

### (7) Gene therapy

Described below are nucleic acid sequences encoding the therapeutic genes to be used in gene therapy. Thus, the nucleic acid described herein can be transferred into a vector for use in gene transportation, whereby the transgene (i.e., the gene of the invention) can be expressed by an arbitrary expression promoter and can be used for the gene therapy.

### 1. Vectors

The transferable viral vectors may be prepared from DNA or RNA viruses. Such vectors may be any type of viral vectors from an MoMLV vector, a herpes virus vector, an Adenovirus vector, an AAV vector, a HIV vector, a SIV vector, a Sendai virus vector and the like. One or more proteins among the constituent protein group of a viral vector are substituted by the constituent proteins of a different species of virus, or alternatively a part of the nucleic acid sequence constituting genetic information is substituted by the nucleic acid sequence of a different species of virus to form a viral vector of the pseudo-type, which can also be used in this invention. For example, there is mentioned a pseudo-type viral vector wherein the Env protein (an envelop protein of HIV) is substituted by the VSV-G protein (an envelop protein of vesicular stomatitis virus or VSV) (Naldini L., et al., Science vol. 272, p.263, 1996). Further, viruses having a host spectrum other than humans are usable as the viral vector insofar as they are efficacious. As for the vectors other than those of viral origin, there may be used complexes of calcium phosphate and nucleic acid, ribosomes, cation-lipid complexes, Sendai virus liposomes, polymer carriers having polycation as the backbone main chain and others. In addition, methods such as electroporation and gene guns may be used as a gene transfer system.

### 2. Expression promoters

As for the expression cassettes to be used for the therapeutic gene, any cassettes without any particular limitations may be used insofar as they can cause genes to express in the target cells. One skilled in the art can readily select such expression cassettes. Preferably, they are expression cassettes capable of gene expression in the cells derived from an animal, more preferably, expression cassettes capable of gene expression in the cells derived from a mammal, and most preferably expression cassettes capable of gene expression in the cells derived from human. The gene promoters that can be used as expression cassettes include: for example, virus-derived promoters from an Adenovirus, a cytomegalovirus, a human immunodeficiency virus, a simian virus 40, a Rous sarcoma virus, a herpes simplex virus, a murine leukemia virus, a sinbis virus, a hepatitis type A virus, a hepatitis type B virus, a hepatitis type C virus, a papilloma virus, a human T cell leukemia virus, an influenza virus, a Japanese encephalitis virus, a JC virus, parbovirus B19, a poliovirus, and the like; mammals-derived promoters such as albumin, SR α, a heat shock protein, and an elongation factor; chimera type promoters such as a CAG promoter; and the promoters whose expression can be induced by tetracyclines, steroids and the like.

### 3. Drugs

The drugs to be used in the gene therapy may be prepared as a recombinant viral vector containing the therapeutic gene that is designed for therapeutic purposes as described above. More specifically, a recombinant virus vector containing the gene described herein may be prepared by dissolving it in an appropriate solvent such as water, physiological saline or an isotonized buffer solution. Here, polyethylene glycol, glucose, various amino acids, collagen, albumin or the like can be then added as protective materials to form preparations.

### 4. Administration method and dosage

There are no particular limitations on the method of administrating the drug mentioned above to humans. For example, parental administration, including injection is preferably carried out. The use level of the drug varies depending on the method of use, the purpose of use, etc.; and one skilled in the art can easily select as appropriate and optimize it. In the case of injection, for example, the daily dosage is preferably administered at about 0.1 µg/kg to 1,000 mg/kg per day, and more preferably at about 1 µg/kg to 100 mg/kg per day.

This invention will be described hereafter in greater detail by way of the examples; however, the technical scope of the invention will not be restricted to those examples.

### (Examples)

The invention will be described hereafter based on the examples more concretely; however, the invention will not be restricted to the examples described below.

### (Preparation Example 1) Construction of cDNA library from neuroblastoma

### 1. Obtaining samples

Clinical tissue samples of human neuroblastoma (stage 4s) were quasi-aseptically frozen immediately after surgical extraction and then preserved at -80 °C.

### 2. Preparation of mRNA

A 2-3 g portion of the sample described in 1 was treated with a Total RNA Extraction Kit (QIAGEN Inc.) and the total RNA was extracted. The extracted total RNA was purified using an oligo dT cellulose column (Collaborative Research, Inc.) to obtain a pool of mRNA with a polyA structure. Following the procedure described below, a cDNA library was prepared according to the oligo capping method (Y. Suzuki et al., Gene, U.S.A., Vol. 200, pp.149-156, 1997).

### 3. Dephosphorylation of mRNA

A 100-200 µg portion of the mRNA pool prepared in 2 above was dissolved in 67.3 µl of sterile ultra-purified water containing 0.1% diethyl pyrocarbonate (DEPC) (DEPC-H₂O), and then 20 µl of 5xBAP buffer [Tris-HCl (500 mM, pH = 7.0)/mercaptoethanol (50 mM)], 2.7 µl of RNasin (40 unit/µl: Promega Inc.) and 10 µl of BAP (0.25 unit/µl, bacteria-derived alkali phosphatase: Takara Shuzo Co. Ltd.) were added. The mixture was reacted at 37 °C for 1 hour to effect dephosphorylation of the 5' end of the mRNA. This was followed by phenol/chloroform treatment twice, and finally by ethanol precipitation to obtain a purified dephosphorylated mRNA pool.

### 4. Decapping of dephosphorylated mRNA

The total amount of the dephosphorylated mRNA pool prepared in 3 above was dissolved in 75.3 µl of sterile ultra-purified water containing 0.1% DEPC, and then 20 µl of 5xTAP buffer [sodium acetate (250 mM, pH = 5.5)/mercaptoethanol (50 mM), EDTA (5 mM, pH = 8.0)], 2.7 µl of RNasin (40 unit/µl) and 2 µl of TAP (tobacco acid pyrophosphatase: 20 unit/µl) were added. The mixture was reacted at 37 °C for 1 hour to effect decapping treatment of the 5' end of the dephosphorylated mRNA. The dephosphorylated mRNA of incomplete length with no capped structure remained without decapping, and with the 5' end dephosphorylated. This was followed by phenol/chloroform treatment and ethanol precipitation to obtain a purified decapped mRNA pool.

### 5. Preparation of oligo-capped mRNA

The total amount of the decapped mRNA pool prepared in 4 above was dissolved in 11 µl of sterile ultra-purified water containing 0.1 % DEPC, and then 4 µl of 5'-oligo RNA (5'-AGCAUCGAGUCGGCCUUGGCCUACUGG-3': SEQ ID NO:1079; 100 ng/µl), 10 µl of 10 x ligation buffer [Tris-HCl (500 mM, pH = 7.0)/mercaptoethanol (100 mM)], 10 µl of magnesium chloride (50 mM), 2.5 µl of ATP (24 mM), 2.5 µl of RNasin (40 unit/µl), 10 µl of T4 RNA ligase (25 unit/µl: Takara Shuzo Co. Ltd.) and 50 µl of polyethylene glycol (50% w/v, PEG8000: Sigma Corporation) were added. The mixture was reacted at 20 °C for 3 hours for ligation of the 5'-oligo RNA to the 5' end of the decapped mRNA. The dephosphorylated mRNA of incomplete length with no capped structure resulted in no ligation to the 5'-oligo RNA. This was followed by phenol/chloroform treatment and ethanol precipitation to obtain a purified oligo-capped mRNA pool.

### 6. Removal of DNA from oligo-capped mRNA

The oligo-capped mRNA pool prepared in 6 above was dissolved in 70.3 µl of sterile ultra-purified water containing 0.1% DEPC, and then 4 µl of Tris-HCl (1 M, pH = 7.0), 5.0 µl of DTT (0.1 M), 16 µl of magnesium chloride (50 mM), 2.7 µl of RNasin (40 unit/µl) and 2 µl of DNaseI (5 unit/µl: Takara Shuzo Co. Ltd.) were added. The mixture was reacted at 37 °C for 10 minutes to digest the excess DNA. This was followed by phenol/chloroform treatment and ethanol precipitation and column purification (S-400HR: Pharmacia Biotech Inc.), to obtain a purified DNA(-) oligo-capped mRNA pool.

### 7. Preparation of 1st strand cDNA

The DNA(-) oligo-capped mRNA pool prepared in 7 above was reverse-transcribed using Super Script II (kit manufactured by Life Tech Oriental, Inc.) to obtain a pool of 1st strand cDNA.

The pool of DNA(-) oligo-capped mRNA was dissolved in 21 µl of sterile distilled water, and then 10 µl of 10 x First Strand buffer (kit accessory), 8 µl of dNTP mix (5 mM, kit accessory), 6 µl of DTT (0.1 M, kit accessory), 2.5 µl of oligo-dT adapter primer (5 pmol/µl, 5'-GCGGCTGAAGACGGCCTATGTGGCCTTTTTTTTTTTTTTTTT-3': SEQ ID NO:1080), 2.0 µl of RNasin (40 unit/µl) and 2 µl of Super Script II RTase (kit accessory) were added. The mixture was reacted at 42 °C for 3 hours to effect reverse transcription. This was followed by phenol/chloroform treatment, alkali treatment and neutralization treatment to digest all the RNA and purification was carried out by ethanol precipitation.

### 8. Preparation of 2nd strand cDNA

The 1st strand cDNA pool prepared in 7 above was subjected to PCR amplification using Gene Amp (kit manufactured by Perkin Elmer Inc.). The pool of 1st strand cDNA was dissolved in 52.4 µl of sterile distilled water, and then 30 µl of 3.3 x Reaction buffer (kit accessory), 8 µl of dNTP mix (2.5 mM, kit accessory), 4.4 µl of magnesium acetate (25 mM, kit accessory), 1.6 µl of Primer F (10 pmol/µl, 5'-AGCATCGAGTCGGCCTTGTTG-3': SEQ ID NO:1081), 1.6 µl of Primer R (10 pmol/µl, 5'-GCGCTGAAGACGGCCTATGT-3': SEQ ID NO:1082) and 2 µl of rTth (kit accessory) were added. Mineral oil (100 µl) was gently added to the mixture and overlayed thereon. After denaturing the reaction solution at 94 °C for 5 minutes, a cycle of 94 °C for 1 minute, 52 °C for 1 minute and 72 °C for 10 minutes was repeated 12 times, and then the solution was allowed to stand at 72 °C for 10 minutes to complete the PCR reaction. This was followed by purification with phenol/chloroform treatment and ethanol precipitation to obtain a 2nd strand cDNA pool.

### 9. SfiI treatment of 2nd strand cDNA

The 2nd strand cDNA pool prepared in 8 above was dissolved in 87 µl of sterile distilled water, and then 10 x NEB buffer (NEB Inc.), 100 x BSA (bovine serum albumin manufactured by NEB Inc.) and 2 µl of SfiI (restriction endonuclease, 20 unit/µl, manufactured by NEB Inc.) were added. The mixture was reacted overnight at 50 °C to effect SfiI restriction endonuclease treatment. This was followed by purification with phenol/chloroform treatment and ethanol precipitation to obtain a pool of cDNA which had been SfiI-treated at both ends.

### 10. Size fractionation of SfiI-treated cDNA

The SfiI-treated cDNA pool prepared in 9 above was electrophoresed on 1% agarose gel and a fraction with >2 kb was purified using Geneclean II (Bio101 Inc.). The purified cDNA pool was dissolved in 100 µl of sterile distilled water and allowed to stand at 37 °C for 6 hours. This was followed by purification with phenol/chloroform treatment and ethanol precipitation to obtain a long-chain cDNA pool.

### 11. cDNA library

The long-chain cDNA pool prepared in 10 above was ligated into the cloning vector pME18S-FL3 (provided by Prof. Sumio Kanno of the Institute of Medical Science, Tokyo University) using a DNA Ligation Kit ver.1 (kit manufactured by Takara Shuzo Co. Ltd.). The long-chain cDNA pool was dissolved in 8 µl of sterile distilled water, and then 1 µl of pME18S-FL3 pretreated with restriction endonuclease DraIII, 80 µl of Solution A (kit accessory) and 10 µl of Solution B (kit accessory) were added and reaction was conducted at 16 °C for 3 hours. This was followed by purification with phenol/chloroform treatment and ethanol precipitation to obtain a cDNA library.

### (Example 1) Transformation into E. coli

### 1. Cloning

The cDNA library prepared in Preparation Example 1, Item 12 above was used for transformation into *E*. *coli* (TOP-10: Invitrogen Corporation). Specifically, the cDNA library was dissolved in 10 µl of sterile distilled water and mixed with TOP-10. The mixture was then incubated on ice for 30 minutes, at 40 °C for 1 minute and on ice for 5 minutes. After adding 500 µl of SOB medium, shake culturing was performed at 37 °C for 60 minutes. Appropriate amounts thereof were seeded onto an ampicillin-containing agar media and culturing was continued at 37 °C for 24 hours to obtain *E*. *coli* clones. There, 5075 clones were picked up randomly.

### 2. Preservation of E. coli clones (Preparation of glycerol stock)

The *E*. *coli* clones on agar media obtained in 1 above were collected with toothpick and suspended in 120 µl of LB medium prepared in a 96-well plate. The 96-well plate was then allowed to stand overnight at 37 °C for culturing of the *E. coli*. 60% Glycerol solution (72 µl) was then added and preserved at -20 °C (glycerol stock).

### (Example 2) Nucleic acid sequencing

### 1. Preparation of plasmid

The glycerol stock (10 µl) prepared in Example 1, Item 2 above was transferred to a 15 ml-centrifugation tube, and then 3 ml of LB medium and 50 µg/ml of ampicillin were added and shaking was carried out overnight at 37 °C for culturing of the *E. coli.* A QIAprep Spin Miniprep Kit (QIAGEN Inc.) was then used to extract and purify a plasmid DNA from the *E*. *coli.*

### 2. Analysis of both end sequences

Both end sequences of the plasmid DNA prepared in 1 above were determined using a DNA Sequencing Kit (kit manufactured by ABI). There were combined 600 ng of plasmid DNA, 8 µl of premix (kit accessory) and 3.2 pmol of primers, and sterile distilled water was added to a total of 20 µl. After denaturing the mixture at 96 °C for 2 minutes, a cycle of 96 °C for 10 seconds, 50 °C for 5 seconds and 60 °C for 4 minutes was repeated 25 times for reaction. The product was then purified by ethanol precipitation. Sequence determination was carried out by polyacrylamide gel electrophoresis under denaturing conditions, using ABI377 (ABI).

### (Example 3) Homology search with database

An internet-mediated DNA sequence homology search was conducted for DNA sequence data obtained from the both end-sequence analysis in Example 2. The search was conducted using the BLAST database of the NCBI (National Center of Biotechnology Information, http://www.ncbi.nblm.nih.gov/BLAST). As a result of the homology search, approximately 2,700 genes were identified. These genes were classified and repeat sequences were eliminated using a RepeatMasker software to obtain 1598 genes. Out of the genes, novel genes were 963 and known genes were 635.

With respect to 308 of novel genes among those genes, those which could be sequenced are shown in the Sequence Listing with their partially decoded sequences.

### (Example 4) Comparison of gene expression levels by semi-quantitative PCR

### 1. Obtaining samples

Clinical tissue samples of human neuroblastoma (stage 4s) were quasi-aseptically frozen immediately after surgical extraction and then preserved at -80 °C. These samples were provided in 8 specimens. In a similar manner, clinical tissue samples of human neuroblastomas of the favorable and unfavorable prognosis types were provided in 12 specimens each.

Prognosis assay of the neuroblastoma samples of the favorable and unfavorable prognosis types was carried out based on the following criteria:
Favorable prognosis type:
   Stage 1 or 2
   Age of onset: <1
   Survival for ≥5 years after surgery without recurrence
   No amplification of N-myc
Unfavorable prognosis type:
   Stage 4
   Age of onset: ≥1
   Death within 3 years after surgery
   Amplification of N-myc

### 2. Differential screening

Semi-quantitative RT-PCR of each specimen was performed in the manner described below.

### a) Reverse transcription (RT)

The RNA from the specimen was reverse-transcribed into cDNA using Super Script II reverse transcriptase (GIBCO). Specifically, 48 µl of a solution comprising 20 µg of the total RNA, 8 µl of random primers (1 µg/µl) (Takara Shuzo Co., Ltd.), and sterile ultra-purified water containing DEPC in an amount as necessary was prepared. This solution was incubated at 65 °C for 15 minutes and was placed on ice after the reaction was complete. Sterile ultra-purified water containing 24 µl of 5 x first strand buffer (GIBCO), 12 µl of 0.1 M DTT (GIBCO), 30 µl of dNTPs (Takara Shuzo Co., Ltd.), 4 µl of Super Script II reverse transcriptase and 2 µl of DEPC were mixed to prepare 72 µl of a mixed solution. This mixed solution was added to the ice-cooled solution described above to make a total of 120 µl, and was allowed to react at 42 °C for 1.5 hours and then at 95 °C for 5 minutes. This was preserved at -20 °C, which was provided for the mother liquor of PCR template.

Thus prepared cDNA solution was diluted with DDW to an appropriate dilution and then it was normalized (concentrations adjusted) with GAPDH primers. The base sequences of the GAPDH primers used were as follows:
5'-ACCTGACCTGCCGTCTAGAA-3' (forward: SEQ ID NO:1077) and
5'-TCCACCACCCTGTTGCTGTA-3' (reverse: SEQ ID NO:1078).

Subsequently, each sample with its concentration adjusted by dilution with DDW was subjected to PCR as described below.

### b) PCR

PCR was performed with rTaq polymerase (Takara Shuzo Co., Ltd.). Appropriate primers were designed against the genes (whether novel or known) identified in the cDNA library from the stage 4s neuroblastoma. Differential screening of three pairs of cDNA sample populations with their concentrations adjusted was performed. Specifically, 2 µl of cDNA, 5 µl of sterile distilled water, 1 µl of 10 x rTaq buffer, 1 µl of 2 mM dNTPs, 0.5 µl each of the synthesized primer set (forward and reverse), and 0.5 µl of rTaq were combined. After denaturing this mixture at 95 °C for 2 minutes, a cycle of 95 °C for 15 seconds, 58 °C for 15 seconds and 72 °C for 20 seconds was repeated 35 times, and then the mixture was allowed to stand at 72 °C for 20 minutes, thereby performing PCR. Where no band showed up, PCR conditions were studied by increasing the number of cycles. Thus, annealing temperatures and cycle numbers for the respective primers could be determined.

The PCR products under thus determined conditions were electrophoresed on 1.5% agarose gel for 20 minutes, stained with ethidium bromide. The concentrations of the bands were compared among three pairs of specimens (i.e., stage 4s neuroblastoma, neuroblastomas of the favorable and unfavorable prognosis types).

As previously described, Table 1 summarizes the obtained patterns according to the specimen subsets. Results from the expressed pattern analysis are as previously discussed.

The primers used were selected under suitable primer selection conditions (base number, Tm, GC%) by inputting into a Primer3 software, the end sequences of the genes to be detected (Example 3). The primer sequences corresponding to the specific clones previously described are provided in the Sequence Listing (SEQ ID NO:175 to SEQ ID NO:1076).

### Industrial Applicability

As described above, by utilizing the information obtainable from the genes or the nucleic acids described herein, the genes are detected in a clinical tissue sample to be assayed thereby to allow the diagnosis for prognosis of neuroblastoma (principally for determining stage 4s neuroblastoma). Specifically, the information obtainable form the genes or the nucleic acids is utilized as tumor markers to allow the preparation of diagnostic agents as well as the design of diagnostic microarrays, both of which can be used for the diagnosis of prognosis.

If the accurate diagnosis of stage 4s neuroblastoma can be made, it will be important information in deciding whether or not the patient in question needs treatment. Where the case warrants, an unnecessary surgical operation may be avoided.

## Claims

1. A diagnostic kit for stage 4s neuroblastoma comprising a pair of primers as the active component, wherein one primer comprises a DNA sequence set forth in SEQ ID NO:175 or a DNA complementary thereto and the other primer comprises a DNA sequence set forth in SEQ ID NO:176 or a sequence complementary thereto.

2. A method for determining stage 4s neuroblastoma, the method comprising detecting the presence or absence of a nucleic acid comprising the nucleic acid sequence set forth in SEQ ID NO:1 in the Sequence Listing from a clinical tissue sample of neuroblastoma.

3. Use of a nucleic acid microarray for diagnosing stage 4s neuroblastoma, wherein the microarray comprises a solid phase support and a nucleic acid which comprises the nucleic acid sequence set forth in SEQ ID NO:1 in the Sequence Listing immobilized on the solid phase support, wherein the diagnosing comprises detecting the presence or absence of a nucleic acid comprising the sequence set forth in SEQ ID NO:1 in a clinical tissue sample using the nucleic acid sequence set forth in SEQ ID NO:1.

4. Use of a nucleic acid microarray for diagnosing stage 4s neuroblastoma, comprising a solid phase support and a nucleic acid which comprises the nucleic acid sequence set forth in SEQ ID NO:175 or SEQ ID NO:176 immobilized on the solid phase support, wherein the diagnosing comprises detecting the presence or absence of a nucleic acid comprising the sequence set forth in SEQ ID NO:1 in a clinical tissue sample using the nucleic acid sequence set forth in SEQ ID NO:175 or SEQ ID NO:176.

## Patentansprüche

1. Diagnostischer Kit für ein Neuroblastom im Stadium 4s, umfassend ein Primerpaar als wirksamen Bestandteil, wobei ein Primer eine in SEQ ID NO: 175 dargelegte DNA-Sequenz oder eine dazu komplementäre DNA umfasst und der andere Primer eine in SEQ ID NO: 176 dargelegte DNA-Sequenz oder eine dazu komplementäre Sequenz umfasst.

2. Verfahren zum Bestimmen eines Neuroblastoms im Stadium 4s, wobei das Verfahren das Nachweisen der Anwesenheit oder Abwesenheit einer Nucleinsäure, umfassend die in SEQ ID NO: 1 des Sequenzprotokolls dargelegte Nucleinsäuresequenz, aus einer klinischen Gewebeprobe eines Neuroblastoms umfasst.

3. Verwendung eines Nucleinsäure-Mikroarrays zum Diagnostizieren eines Neuroblastoms im Stadium 4s, wobei der Mikroarray eine Festphasen-Unterlage und eine auf der Festphasen-Unterlage immobilisierte Nucleinsäure umfasst, welche die in SEQ ID NO: 1 im Sequenzprotokoll dargelegte Nucleinsäuresequenz umfasst, wobei das Diagnostizieren das Nachweisen der Anwesenheit oder Abwesenheit einer Nucleinsäure, umfassend die in SEQ ID NO: 1 dargelegte Sequenz, in einer klinischen Gewebeprobe unter Verwendung der in SEQ ID NO: 1 dargelegten Nucleinsäuresequenz umfasst.

4. Verwendung eines Nucleinsäure-Mikroarrays zum Diagnostizieren eines Neuroblastoms im Stadium 4s, umfassend eine Festphasen-Unterlage und eine auf der Festphasen-Unterlage immobilisierte Nucleinsäure, welche die in SEQ ID NO: 175 oder SEQ ID NO: 176 dargelegte Nucleinsäuresequenz umfasst, wobei das Diagnostizieren das Nachweisen der Anwesenheit oder Abwesenheit einer Nucleinsäure, umfassend die in SEQ ID NO: 1 dargelegte Sequenz, in einer klinischen Gewebeprobe unter Verwendung der in SEQ ID NO: 175 oder SEQ ID NO: 176 dargelegten Nucleinsäuresequenz umfasst.

## Revendications

1. Kit de diagnostic pour un neuroblastome de stade 4s, comprenant une paire d'amorces à titre de composant actif, dans lequel une amorce comprend une séquence d'ADN présentée dans la SEQ ID NO : 175 ou un ADN complémentaire de celle-ci, et l'autre amorce comprend une séquence d'ADN présentée dans la SEQ ID NO : 176 ou une séquence complémentaire de celle-ci.

2. Procédé pour déterminer un neuroblastome de stade 4s, le procédé comprenant la détection de la présence ou de l'absence d'un acide nucléique comprenant la séquence d'acide nucléique présentée dans la SEQ ID NO : 1 du Listage de Séquences, à partir d'un échantillon tissulaire clinique de neuroblastome.

3. Utilisation d'une biopuce d'acide nucléique pour diagnostiquer un neuroblastome de stade 4s, dans laquelle la biopuce comprend un support en phase solide et un acide nucléique qui comprend la séquence d'acide nucléique présentée dans la SEQ ID NO : 1 du Listage de Séquences, immobilisé sur le support en phase solide, dans laquelle le diagnostic comprend la détection de la présence ou de l'absence d'un acide nucléique comprenant la séquence présentée dans la SEQ ID NO : 1 dans un échantillon tissulaire clinique par utilisation de la séquence d'acide nucléique présentée dans la SEQ ID NO : 1.

4. Utilisation d'une biopuce d'acide nucléique pour diagnostiquer un neuroblastome de stade 4s, comprenant un support en phase solide et un acide nucléique qui comprend la séquence d'acide nucléique présentée dans la SEQ ID NO : 175 ou la SEQ ID NO : 176 immobilisé sur le support en phase solide, dans laquelle le diagnostic comprend la détection de la présence ou de l'absence d'un acide nucléique comprenant la séquence présentée dans la SEQ ID NO : 1 dans un échantillon tissulaire clinique par utilisation de la séquence d'acide nucléique présentée dans la SEQ ID NO : 175 ou la SEQ ID NO : 176.


### SEQUENCE LISTING

<110> Hisamitsu Pharmaceutical Co., Inc. Chiba-Prefecture
<120> Nucleic acids isolated from stage 4s neuroblastoma
<130> FP03-0298-00
<150> JP 2002-316586
   <151> 2002-10-30
<160> 1082
<170> PatentIn Ver. 2.1
<210> 1
   <211> 1570
   <212> DNA
   <213> Homo sapiens
<220>
   <223> nbla22420
<400> 1
   aatggaaaca cagagcgtgt tttctgacca cacttgtaaa tagaattatg agcataactt 60
   tttttgtact taaagtttgc cctaggcata tacaagtcag ttcttctaag caagatagtt 120
   tcagttaaat gttgttattt gcttttggat agcctttgat catatggaca gaaataaatc 180
   aggtataata aaacacacac aaagtattcc agaaaaaatt gtatttgttt ttgactaata 240
   agtaaataca actatttttc ttggtttgta ttagttttta gatatttttg aaagaatgga 300
   ttcaatcttt taaaaattaa gaggtaactg atttatgaac acagattaac aatcattttg 360
   agacattaaa aataccatct gtacatgaga aaattataat ggtaatcaac aaaatttcag 420
   tacttcccag aatctggttt tgaaacttta ttatgtttta ggggaaaagc tctcattttt 480
   ctgtttgctt agatgagtta gatcactcat ttaaaatctg aagaagtcaa attatttttt 540
   ataaagatcc agaataatag tgtatgtatt tctaaataat ctgaatatgt ttacattggt 600
   tttttttttt taaacctagg ctaggaaggg attacctatt atctaacaaa catagtgcaa 660
   ctgtatagat aaggggcaaa cttcaaagat tggatattgt ttattatgtg aaagatacat 720
   aggtctggct atgatttgga agtcctaggt aactggttag gcttttcagg attgacagca 780
   gctgtgcaga aattttgtta aatgcttatc attttaaaaa gctgtattca aaatatttct 840
   aattttcact attttttaat gtaaatgttt ttgagagtca aagaagattc tatactttta 900
   cttatgaagc agtttgttgt tgtttgttca tttctttttt tggtatgggg tctttctctg 960
   ttgcccaagg ccggagtatg tagtggtgca atcacagctc gctgcaggct taaactcctg 1020
   gtctcaagcc atttttctgc ctcagccttt ctagtagctg ggagtacagg caaatgctac 1080
   tgccccaagc taatttatgt tttattttta ttttttgtag agacagggtc tcgctgtgtt 1140
   gtgcaggctg atctctaact cctgggctca agctatctcc ccactttgcc tcctcaagtg 1200
   ttgggtttat aggcgtgagc tatggtgccc agcctgaggc agtcttaacg ataatttgtt 1260
   ttttctgatc aaaatctacc aaaatggccg gctgcgctgg ctcacgcctg taatcccagt 1320
   actttgagag accgaggtgg gtggatctct tgaggtcagg agtccaagac cagcctggca 1380
   aacatggtga aaccccgtct ctactaaaaa tacaaaatag ccgggcatgg tggcatgcac 1440
   ctgtaattcc agctactcgg gagactgagg caggagaatt acttgaaccc aggaggtgga 1500
   ggttgtagca agccaagatc acgccactgc attccagcct gggcgacaga gtgagactct 1560
   gtctcaaaaa                                                        1570
<210> 2
   <211> 2400
   <212> DNA
   <213> Homo sapiens
<220>
   <223> nbla22689
<400> 2
   gaaaacaaaa ggagacgaag gacgcatgcg tttggtgagt cccggattct ggtgggttct 60
   tccgctcagg ctgggtgaag cgcttccggg tcgccgccgg cagcagcctc ccggcgcgat 120
   gaagacactg aggctcagag aggttaagtg actcagccaa ggtcaaacag ctagtaagtg 180
   gtggagccag gactcaaagc caggagccat gtccactttg ttcccctcac tcttccctcg 240
   tgtgactgag actctgtggt ttaatctgga tcgaccctgt gtggaagaga cagagctgca 300
   gcagcaggaa cagcagcatc aggcctggct ccaaagcatc gcggagaaag acaacaacct 360
   ggttcctatt ggcaagccag cctcagaggc ctgtagggct tacaggctct gtcctgccca 420
   ccagcactat gatgacgagg aagaagagga tgatgaagat gatgaggata gtgaagagga 480
   ctcagaggat gatgaggata tgcaggacat ggacgagatg aatgactaca atgagtcacc 540
   ggatgatgga gaggtcaatg aggtaggcaa ggggtatggg ggagggcctc tgttcctgga 600
   cccttgctcc tgacccagtt gatggccaag gggtacagaa accctggatc cagccagggg 660
   caggatctgg ggctgaggct ggctgaggcc cctccccacc cacacccagc ctcctctcca 720
   ggtggacatg gaaggcaacg aacaggatca ggaccagtgg atgatctagg tagagtatcc 780
   acagtaggtt cccaattcca gcacacaagc aggggccttc tcctccacca gccgcatcag 840
   gatctgacct atgaggggag atggctgttg cagaagacat gggagatgga tgcagggccc 900
   ctgataaaag atatctcaaa tgcctacctg cctcactgca gctcccaacc agccggggtc 960
   tcatctgtct cttgtaccat agccccagct gccctcctgg tccccgtctc ctacagtgta 1020
   gtcttcacac cagccctgga atttttccaa caaatctgac cttattactc cttggctcct 1080
   gtgagctgaa ggcctttggg attgaacttg ggattctcag cctggcattc aggaccttgg 1140
   acctgatcct atcctacctt tccaggttca tctctcagta cttcccacct gtggcctgta 1200
   tcacagccat cccaaacaac tgtgcccaga atccatcaag ctgtctcatt ccttcatgcc 1260
   acatgtgtat atgtggctgg ctttgccctt cccaccccca tcgccatctg cctggccaac 1320
   tcagaacttc cagattcagt tcaaatgttg ctctttctcc atgaagtccc aggcagaaac 1380
   aaccacccta tctttcagat ttatgaaagg tctctgttag aatttgtagt ttcattcccc 1440
   ttttattgct catcaaatgt atttctgatc ttggaattgg atgaactttt atttatttat 1500
   ttttgagacc aagtcttgct gtgttgccca ggctggagtg cagtagcatg atcacggctc 1560
   actgcagcct tgaccaccca ggctcaggca atcctcccac ctcagcattt ccagtagctg 1620
   gaaccacagt tactcaccac cacacccggc taatttttaa attttttgta gaaacggggg 1680
   tcttgctttg ttacccaggc tagtctcgaa ctcctgggct caagtgatcc tcctgctttg 1740
   gcctcccaaa gtgctgggat tacaggcatg agccaccatg cccagccagt gaatttcttt 1800
   tcttttcttt ttcttttttt tttttttttg agacaggttc ttgctctgtc acccatgctg 1860
   gagtgcagtg gcacaatcac agctcactgc agcctcagcc tcctgggctc aagcaatcct 1920
   cccacctcag cctcccaagt agctgggacc acaggcatgt gccaccatgc ctgggtaatt 1980
   tttgtatttt ttgtagagat gggtttttgc catgttgccc aagccggtct caaactcctg 2040
   agctcaagca atctgcccac ctcggcctct caaagtgctg ggattacagg caccagccac 2100
   cacacagccg aatttcttaa ataagaccct aaaagcactt atgctgggat tgagataaat 2160
   ccaggcagac agctacccta aatggtatgt ggaagcctcc atggtggaga ggaaagatgt 2220
   ggagacagat aattacaaag ctatgggtta tctgctgaga tggttattcc actgtgtatt 2280
   atggttcctt tgaggccagc atttgtggct cattcatctc tgtggcctct acccctctcc 2340
   ctggcaccta gcacattcct aatacaaaag aggtggcaat aaatgtttgc tgaataaaaa 2400
<210> 3
   <211> 1958
   <212> DNA
   <213> Homo sapiens
<220>
   <223> nbla24135
<400> 3
   gaggcctggg gtggggacgc gaggacacca gcgtagaaga gcttacatca gaatcgagct 60
   ttgtgggcgc tccgggattt ggccctttag cgcggatcct agacaacagg ttttggacct 120
   cgagagctgc agaactgagg ctactggtgc cgccagcctg ctggctccgc ctctgcctca 180
   gtttcttccc ctatggcccg cgtgccgctg gggcggagtc tcactctgtc acccaggctg 240
   gagcacaatg gcatgacctc agctcaccac aacttccgcc tcccaggttc aagggattct 300
   cctgcctcag cctcccaagt agctgagatt ataggcagtg aaccccttga gcacggggcc 360
   cgcgcctggc ttgttctccg ctgtctccag cacctaggac agggcctggc acgaagtagg 420
   tgcacagtga gtagtgaatg ctggagtgaa tagatgcaag agggctggtg tcttttagaa 480
   agcagcgctc agtggctgag aactcctggg ttccctgctg ggcaagggtt aggcgtacat 540
   ttgccagggt gttaaaggag gaacgcaggg ttcaaatccc agctccactt aacctccccc 600
   acactgcggc gacgccgcgc tttttttccg acccaactga gccggaagtg gaggcgcggg 660
   cttcccatga tgccccgcga gacctttatt ctaaccgcaa ggagtagcgg aggggaggtc 720
   gtgatggcgg cgccggaggc ggaggttctg tcctcagccg cagtccctga tttggagtgg 780
   tatgagaagt ccgaagaaac tcacgcctcc cagatagaac tacttgagac aagctctacg 840
   caggaacctc tcaacgcttc ggaggccttt tgcccaagag actgcatggt accagtggtg 900
   tttcctgggc ctgtgagcca ggaaggctgc tgtcagttta cttgtgaact tctaaagcat 960
   atcatgtatc aacgccagca gctccctctg ccctatgaac agcttaagca cttttaccga 1020
   aaaccttctc cccaggcaga ggagatgctg aagaagaaac ctcgggccac cactgaggtg 1080
   agcagcagga aatgccaaca agccctggca gaactggaga gtgtcctcag ccacctggag 1140
   gacttctttg cacggacact agtaccgcga gtgctgattc tccttggggg caatgcccta 1200
   agccccaagg agttctatga actcgacttg tctctgctgg ccccctacag cgtggaccag 1260
   agcctgagca cagcagcttg tttgcgccgt ctcttccgag ccatattcat ggctgatgcc 1320
   tttagcgagc ttcaggctcc tccactcatg ggcaccgtcg tcatggcaca gggacaccgc 1380
   aactgtggag aagattggtt tcgacccaag ctcaactatc gagtgcccag ccggggccat 1440
   aaactgactg tgaccctgtc atgtggcaga ccttccatcc gaaccacggc ttgggaagac 1500
   tacatttggt tccaggcacc agtgacattt aaaggcttcc gcgagtgaat gagtgcttct 1560
   taatcctaaa aacacaatgg ctgaattatc tttctccatg tggcgctgaa tcacccatct 1620
   ggtttggagc tagagttgct tcctggtgag agaggaagca actctccttc tggttgtctg 1680
   cctcccctca gatttcctga taggctgatg gcatgtggct gtgactgtga ctgtaatcat 1740
   tgctgaacaa catctctttg aatcaaaggt tgattttccc agagggtgct gggtcaggca 1800
   tttctattag gagttggaaa gcaaaaatgg gtccatagac actctatgga ggtgtccctt 1860
   tctgctcttt gctgtgtcct ttcagaattt ttaccaggaa cataatgtgg atgtgactta 1920
   tgaacttaaa tataaaataa atagattctt attaaaaa                        1958
<210> 4
   <211> 1436
   <212> DNA
   <213> Homo sapiens
<220>
   <223> nbla24350
<400> 4
   agtccgggtg gtttcttccg accgaccgtc agcactcgac aaataactga gcagctgctg 60
   gggccgggaa caccgcgggg acaggccctc actgtgagga taatgaccat accgggtcct 120
   gggagacctc ctgaactgca gcggcaggga accccgacac ccagtgagtc tgagagcctc 180
   acagctgccc gcctggctga ctcccatcag gtctgaagca ccctcccgac agtcatggtg 240
   gctgtttttg tctttcccag gagaaatgaa tggcactggc aacctgggcc tcgtgcctgt 300
   tttcctgaag ccatgtgtac ttggcttctg gaccgtggcg cacctgaccc cagaaggcgg 360
   tgcacttact gtaaggctga tgggccttag agaacacctc cccagcgcct acgcgcaatc 420
   aggaccgcgg acgcctcatg tctgcctggg aggtctccaa agggccaaac actcccggac 480
   tcggccctgc aggagtcatt tgctgtagac catcccccag tgccacatac cactggagaa 540
   agctgagtcc agaggagctc aaacttgaaa acacaatctc tctggagggt caaggcctgg 600
   cagggcagcc tgaatggaat ccaacgttac ctgtgactaa gagccaactg ggagtgagac 660
   aagggtcctc tggtctccct ggatgacggg agatgcgcgc ctcatcgtgt gatgtcaaga 720
   accactgctg ggcctaccct gagcagggag cagggagcgg cactgtcatg cttgttgctg 780
   gagccagcaa aggatgaggc tatgcctcag cttccgctcc gctccactca gtgctggcct 840
   catcgcccca cccagggggc agaactctcc ccaggagccc acggtgctgg gcagaggcag 900
   aggccacttg ggcggtcagc ccagagctgg gtgggcccgg ccagcgggac tttgcggcct 960
   ccccaccctc cggatctcct gatcaggcgt aacccaaccc gggcagctcc ttcggctcca 1020
   ccatccagag acaagctgac ttccgataat gactttattt taacatattt aattacagac 1080
   ataaaatagc tggggagggg ggtgagcccc agcctagccc caccatgggg ctataggagg 1140
   ggaggcgcag gcggggcccc cctgctgacc ctctctctgg gggtcttcct atggcggggc 1200
   cctattgctt gagtggggga ggagccatgc aaatgagggg ggcagggcag ccactcggcc 1260
   ccaccccacc ccgaggacgg cctccccaca gaatgcccag gctgtgcccc cagccccagc 1320
   tgctccacct ccttcttctc tgtccaggga gcagaccctc tggccagccc ctgactctgc 1380
   ccctaccccc tctgcaaacc taaaggggaa taaatacaaa ctttacaaag taaaaa     1436
<210> 5
   <211> 3062
   <212> DNA
   <213> Homo sapiens
<220>
   <223> nbla23701
<400> 5
   gagaggcggg cgcctaccag ccggcagctc cggagctgcc cgcgccatgt ccgcgcacaa 60
   tcggggcacc gagctcggta aggggcccgc ggggctcccc atcccctctc cctcgcgttc 120
   agcgccgccg ggactagcgc ggggcctgct gccgcccagt gccctggctg tgggtccccg 180
   aggggttttc gctggggcgg gaagcagtgg cgtctggtca gccctcaccc caagtaaagg 240
   ccgaacccgg cacgttcgcg ccgcttgtct ttgcacctaa gcttttactc tggtatgcgg 300
   aaggagtagg aaagggttag attattatct tcctgccttt tcgttcactc tagctcgctg 360
   gttggaaaac ccaacaaccc aaaaaacaaa acccaaaaca aacaaccccc aagcaggtaa 420
   aaacagataa aaaccttctt tctcctcctt ttaatagaat acttgtgtaa tttaatgcag 480
   tatttccgta gataatttta accgtaacct tgaagtggcc gtgctcgtgg aaaagttgtc 540
   agccgtctgt gctcaaaatg taacactgca gattcatggg attttagagt tacaaagatt 600
   tgttaaagta cctgtattat ttcccagttt tcatcttttt ttatattgtt caaatactgg 660
   caagaaacct tagttcagat ttcttttttt ttttttttta ttgatcattc ttgggtgttt 720
   ctcgcagagg gggatttggc agggtcatag gacagtagtg gagggaaggt cagctgataa 780
   acaagtgaac aaaggtctct ggttttccta ggcagaggac cctgcggcct tccgcagtgt 840
   ttgtgtccct gggtacttaa gattagggag tggtgatgac tcttaacgag catgctgcct 900
   tcaagcatct gtttaacaaa gcacatcttg caccgccctt aatccattta accctgagtg 960
   gacacagcac atgtttcaga gagcacaggg ttggggataa ggtcacagat caacaggatc 1020
   ccaaggcaga agagaatttt tcttcagatt tcttaacatg tgaaaaattt ataattcaaa 1080
   cagcaaaacc atgatcaaga gaaggtttaa gcgtctcgtt taagtattat agcttggata 1140
   tctgtgtatc caggatcttt aacttcttac ctgtgtgact tcggacaaat taataacttt 1200
   gcgcttaagt ttcttcatct gtaaaatggt tattttagtg gtagttacct tataaggccg 1260
   ttaggagatt aaataggata catgtaaagt agtttggtat attgtggaca cctagtaagt 1320
   cttcagtata gatagtatta gtatatggag ttatggtttt aggggctaat tttgagaaaa 1380
   ttggctgtaa attatatgta acacatacag gtaggtcctt ttcgccctcc ttaaaagtga 1440
   ctggtactta aacagtctgc acttccaaga ggtgttctgg attttttgtc gaatggtaag 1500
   agagtaaatc tatcatttta aagacagttg atttactaac ctggttgatt ttgttttagt 1560
   cactgtcctc tagctgatta tgttttaaac tctagtccta tctctggaac gtggtcttta 1620
   gtaataacgg cattatttct tagattggaa tatccttgaa ggtggtggat atggggcagg 1680
   tttggggtgg tgtcttacct gggtattccc aggaatatga ccatgtgact atgcatacat 1740
   caaggatgtg ccctaaattt cccaaaactt agacatttta aatttttctt tcaaaaaaca 1800
   taattgaacc atttttaaat ttatttattt gcagtaatta gaatcaatca cttccattca 1860
   tttgttgaaa agtaatagac ataaataatt gccaggtaga acaatagtaa atgtggtttt 1920
   tatgcagcta tcgaaatgat catagctttg tatttattat cttatttgtt aaaatcagat 1980
   ttttttcctt cacgggtatt aatccttaat ccaaacaggt ttaaactgaa atgctaaaat 2040
   aagttatttg aattaggtac tagggaaaaa aatctttcag tattaattta tgcagtatat 2100
   taactgatga tttttaaaat agttttctaa ttgaaagtct ttttaataaa catcgtaact 2160
   aatttctaaa ataaattaac atttttgctt cccttttctt attacaaaag gaattcatgg 2220
   ttattgtaaa aattctagaa aatacagtta gcacaaaaat gttgtaatat tattactagt 2280
   ccaatcactg ttatttatga tttggtgtat gtacttctag ttcatggact taaaaaaaca 2340
   ttgagttcct ttgagactaa acctgaccct catgattaaa aagtctttaa ggaaaacatt 2400
   ggcatttgga tgtatgaaag atgttttcca aatagggaat gtaccctcta gctttcatat 2460
   tagaggatgg ggcccagcat tctgagtttt aacaaatcct gtgggtagta ctgaagcata 2520
   cccaagtttg agaaccaatg gcttaatgat ctccaaggta ctatcaagtt ttgtacctag 2580
   actattatgc cctatatagt ctattaaaat gtacagatat tcttctattt tattagatgc 2640
   cacttaacta ttgcctaaaa tgcaggtgtc acgtgggtag tgatctttct tttgttcact 2700
   gatgtgtccc aagtacctag aatagtgttt ggtacacaga aggccctcaa aaatgtcttg 2760
   aggctgggca tggtggctca tgcctatagt cctggcactt tgggaggctc aaggcagccg 2820
   gatcacttga gatcagaagt tggagaccag cctggccaac atggcaaaac cctatctctg 2880
   ctaaaaatac aaaaattagc tgggcatagt ggcgcatgcc tgtagtccca gctacttggg 2940
   aggctgaggt acgagaatcg cttgaaccca gagagtggag gttgcagtga gctggaattg 3000
   tgccactgca ctccattggg caacagactg gagacagact gtgtctcaaa aaaagataaa 3060
   aa                                                                3062
<210> 6
   <211> 2900
   <212> DNA
   <213> Homo sapiens
<220>
   <223> nbla23890
<400> 6
   agcgccgagg cggtaccttc agcctgcaat gagaggaacc cgggagagcc cccgggagcc 60
   agcgaagagc ttggctgctg cgtccagggc tgctgctgcc gccgcggctg cttgaaactc 120
   ctcaaagttg agagccggct agagggtgcc gcccgccggg agccggaggg aaaggaagtc 180
   ggaaggtgca agagtgacag acacggacag acggacgcgc agaccttcgg aaggcactgc 240
   gtaggcagcc tccccggagc ccacgaggct ccccagcacc gttcactggt gggaggctga 300
   gccggtggaa aagacaccgg gaagagactc agaggcgacc ataatgtcgt tacgtgtaca 360
   cactctgccc accctgcttg gagccgtcgt cagaccgggc tgcagggagc tgctgtgttt 420
   gctgatgatc acagtgactg tgggccctgg tgcctctggg gtgtgcccca ccgcttgcat 480
   ctgtgccact gacatcgtca gctgcaccaa caaaaacctg tccaaggtgc ctgggaacct 540
   tttcagactg attaagagac tggacctgag ttataacaga attgggcttc tggattctga 600
   gtggattcca gtatcgtttg caaagctgaa caccctaatt cttcgtcata acaacatcac 660
   cagcatttcc acgggcagtt tttccacaac tccaaatttg aagtgtcttg acttatcgtc 720
   caataagctg aagacggtga gaaatgctgt attccaagag ttgaaggttc tggaagtgct 780
   tctgctttac aacaatcaca tatcctatct cgatccttca gcgtttggag ggctctccca 840
   gttgcagaaa ctctacttaa gtggaaattt tctcacacag tttccgatgg atttgtatgt 900
   tggaaggttc aagctggcag aactgatgtt tttagatgtt tcttataacc gaattccttc 960
   catgccaatg caccacataa atttagtgcc aggaaaacag ctgagaggca tctaccttca 1020
   tggaaaccca tttgtctgtg actgttccct gtactccttg ctggtctttt ggtatcgtag 1080
   gcactttagc tcagtgatgg attttaagaa cgattacacc tgtcgcctgt ggtctgactc 1140
   caggcactcg cgtcaggtac ttctgctcca ggatagcttt atgaattgct ctgacagcat 1200
   catcaatggt tcctttcgtg cgcttggctt tattcatgag gctcaggtcg gggaaagact 1260
   gatggtccac tgtgacagca agacaggtaa tgcaaatacg gatttcatct gggtgggtcc 1320
   agataacaga ctgctagagc cggataaaga gatggaaaac ttttacgtgt ttcacaatgg 1380
   aagtctggtt atagaaagcc ctcgttttga ggatgctgga gtgtattctt gtatcgcaat 1440
   gaataagcaa cgcctgttaa atgaaactgt ggacgtcaca ataaatgtga gcaatttcac 1500
   tgtaagcaga tcccatgctc atgaggcatt taacacagct tttaccactc ttgctgcttg 1560
   cgtggccagt atcgttttgg tacttttgta cctctatctg actccatgcc cctgcaagtg 1620
   taaaaccaag agacagaaaa atatgctaca ccaaagcaat gcccattcat cgattctcag 1680
   tcctggcccc gctagtgatg cctccgctga tgaacggaag gcaggtgcag gtaaaagagt 1740
   ggtgtttttg gaacccctga aggatactgc agcagggcag aacgggaaag tcaggctctt 1800
   tcccagcgag gcagtgatag ctgagggcat cctaaagtcc acgaggggga aatctgactc 1860
   agattcagtc aattcagtgt tttctgacac accttttgtg gcgtccactt aatttgtgcc 1920
   tatatttgta tgatgtcata atttaatctg ttcatattta actttgtgtg tggtctgcaa 1980
   aataaacagc aggacagaaa ttgtgttgtt ttgttctttg aaatacaacc aaattctctt 2040
   aaaatgattg gtaggaaatg aggtaaagta cttcagttcc tcaatgtgcc atagaaagat 2100
   ggggttgttt tccaaagttt aagttctaga tcacaatatc ttagctttta gcactattgg 2160
   taatttcaga gtaggcccaa aggtgatatg actcccattg tccctttatt taggatattg 2220
   aaagaaaaaa taaactttat gtattagtgt cctttaaaaa tagactttgc taacttacta 2280
   gtaccagagt tattttaaag aaaaacacta gtgtccaatt tcatttttaa aagatgtaga 2340
   aagaagaatc aagcatcaat taattataaa gcctaaagca aagttagatt tgggggttat 2400
   tcagccaaaa ttaccgtttt agaccagaat gaatagacta cactgataaa atgtactgga 2460
   taatgccaca tcctatatgg tgttatagaa atagtgcaag gaaagtacat ttgtttgcct 2520
   gtcttttcat tttgtacatt cttcccattc tgtattcttg tacaaaagat ctcattgaaa 2580
   atttaaagtc atcataattt gttgccataa atatgtaagt gtcaatacca aaatgtctga 2640
   gtaacttctt aaatccctgt tctagcaaac taatattggt tcatgtgctt gtgtatatgt 2700
   aaatcttaaa ttatgtgaac tattaaatag accctactgt actgtgcttt ggacatttga 2760
   attaatgtaa atatatgtaa tctgtgactt gatattttgt tttatttggc tatttaaaaa 2820
   cataaatcta aaatgtctta tgttatcaga ttatgctatt ttgtataaag caccactgat 2880
   agcaaatctc tctccaaaaa 2900
<210> 7
   <211> 2708
   <212> DNA
   <213> Homo sapiens
<220>
   <223> nbla21650
<400> 7
   atccaaaaga ttatcatttc aacatgcaat cctattttaa aaataactag tgaggtacct 60
   gacaaaaaaa aatccctttt catactaagt ccagaagatc tttgtgtatt ttatactcat 120
   aggacatctg agtttggatg ttaccttttt attggaaata tgggatctgt acttagattt 180
   cactgaattt acattgaaaa ggtaggttca catacccaag ttgtctcaca cgttcctaaa 240
   tgttttctgg taactggatg gagtatcagt ttttatattt atctttgcat tagctaaaaa 300
   acaaattaat agttcaggtc ctcagccgca cacaggcagt tttctccacg gtccaaattg 360
   ttgcccgaat tcacccagac cccgctgtcc tccgcttttt catgcagaca ttcaaacaac 420
   tgcctccctt cctcctggca cccctcctgg cacccccatc ccatcgccag cagcctccaa 480
   accagtttcc ctcctgtcct catctcagcc acccatgact cacacacaca tctgtctccc 540
   ctggcccact tttcacctgg tcctcataat ctatgcataa acattaacgt accacaggtc 600
   aatctgcata ctgattactt ctgctctggt caaattcttg ctttcaggat caggaggctt 660
   tctccccaca ccaaactggg cctgaggaaa tagtgtcttg tcttcctgtc acccctcccg 720
   tagttgcatg tctaatgaga caaggggtgt ctcaggtgaa gcaggacagg gaggatgcca 780
   gcacttgggt ggtagaggtt tgaggagtgc ctgttggggg atgtgttggg gaaggaggac 840
   ttttcacata tggctcattg tgtcgggatg atttcgttgt taaataagca cctacaggat 900
   gatttcacat tccatacttc taagttttta taatttaaat tctttccgcc aggctgggtt 960
   tttttttttt tccaaacttt aaatctgtgg ctagaattgg tttgatttac ctaatcctgc 1020
   ccctgagatt tagccccatc cctgagagcc ccctcagagc cacccacagc caggacacct 1080
   ctgctggcct ccccttcccc agccttccaa cttgtggcag gcccccggct ctggcctccc 1140
   cctatatggg aatgagccag ctgcaccgct gctgacagtg gctgggataa tcctccctga 1200
   gctgttccaa ggattagtcc tgctgccctg tgcccagctc ccacacaacg ggtttcgggg 1260
   gctgtggacc ctgtgccagg aaaggaaggg cgcagctcct gcaatgcgga gcagccaggg 1320
   cagtgggcac caggctttag cctccctttc tcaccctaca gagggcaggc ccttcagctc 1380
   cattctcctc caaggctgca gagggggcag gaattggggg tgacaggaga gctgtaaggt 1440
   ctccagtggg tcattctggg cccagagatg ggtgctgaag ctcccacgcc tgcctgtgaa 1500
   aatggagtcc tctctcacct gggagagcca ggtgctgccc cgagaaggat gcatttatgg 1560
   cttcatgaag tctttcctga cccccgatgc tgctgactat aggtaagtct gagcaaatct 1620
   gggggagcct catcttggca tgagaaagag atggcttctt ctaagcccac tggccgtgat 1680
   cccaggatta taacacattc tggctcaagt ccagactatt tgtagaacac aggagatcct 1740
   ccatgagagg tagtataata tagaggatat gtgtgcttac taagaggctg cctgtctgac 1800
   cttggacaag ttctttttat ttatttattt attttttata gagacaaagt ctcactatgt 1860
   tgctcaggct ggtcttgaac tcctggcctc aagcgatcct cccaccttag cctcccaaag 1920
   agttgggatt atagacatga gccactgcac ctggccgacc ttgggcaagt tcttaaaccc 1980
   ttcaaagcct catttttctc caatcataaa agggaaagat ggtaatattt tcccctccaa 2040
   attcttgtaa gtattaaaca ttgtatatgt attttgaaca cgattaagct ctaaacactt 2100
   gttaggaagc aggagtagca tttgaaacaa acagctcttt tcccacaggt cggatgccct 2160
   cacagaattg agattatgta cgtaaaacac caggtgccta acccggcaca gagcaggagg 2220
   gctaagcgtg acatccagca cgtggtcagt ggaatccagt attcctaccc acctctctag 2280
   tctcccctcc acccctctcc ctttcagagg caccaagctg cttgtggtct tgtctattcc 2340
   cactccctgc ctgactgaac attttctcca cctcctgatc atcagcagca gaaactggct 2400
   gctcttcctc ctgggtagac agccagactg tatttcccag ctgcccctgc agtgagatgt 2460
   ggccatcgga gccagcattg gccaatggac tctgcatggg agtgacgcat gctgcctcca 2520
   ggcttgtccc taaaacctcc cacgtgtcct ccgcctgctc ttcccacttc caaggagcac 2580
   ggcaattgtg gaagacccag attagtgatg gcagaaccat agatgggagg aacctgggtc 2640
   cctgacttaa agtatcatgg atttggatgt tcccttagtg agaaataaac ttccattgtg 2700
   tttaaaaa 2708
<210> 8
   <211> 2312
   <212> DNA
   <213> Homo sapiens
<220>
   <223> nbla22094
<400> 8
   gctttttcaa tttattgata tggtttaaat gctcccacag aattgtgtga gagagcaata 60
   tgtcattgat tgaaaagtgg gaaacaaact ggtagtaatg gtcaggattt ccccttttca 120
   gaactttggt gcatttgaag tgcctgacaa tgtagtccag cttccctcct gttttaccta 180
   gagggctgga gatatgaggg cccaaagggg ccacaactgt tatcttaagt ggactgaaag 240
   gaagacgaaa ttaaaactag cttctactcc acttgtagga aatgtgcttt taatctttgg 300
   tgtagcccag ccttctagga acaaaagtat cctatgttgg caactgcagt aacaaaacag 360
   ttatggagag tatggaggag agccagtaac tcctaaaggt cttgttcctt tgacttttct 420
   tctcaaacaa acatgagata ttcatgaatt gcaatggcaa acgtttttta ggttcgccaa 480
   tatgaaaatg taaagcagtt ttaagatgat taatattaaa ataggccaag tgcggtggct 540
   cacaactgta attccagcac tttgagagcc caaagtggga ggatcacttg gcctccgaag 600
   ttcaagacca acctgggcaa cacagatgtc atctctagaa aaaaaaaaaa tttttttttt 660
   taattggccg ggcatggtgg catgtgcctg tggtcctagc tactcgggag gcttaaagcc 720
   gggaggcaga ggttgccgtg ggccgggatc gcgccactgc actacagcct gaccgacaaa 780
   gcaagactca gtctcaaaaa aaaaaaaacc aaccaaccat tcactaagtg catgtaagca 840
   aatctaccct ggttgtccca aattgggatt caaccacttt agaagtcttg ttagacattt 900
   tttcagttga tacataatag ttgtatgtac ttaccgagca tgtgatattg atatgtgcat 960
   acaatatata atgatcacat cagaataact ggaatatcca tcacctcaaa caatgatcat 1020
   ttctaaaaga acattccaaa gctgctcttt tagctgtttt gaaatataca ataaattatt 1080
   aattgttgga aaacttttga aagttatctt taagctgctt ttttggacaa gaggtatata 1140
   attgcaatac agatggatat taacttccac tgtatatttc attaaagctg gtaaaatttt 1200
   tttaaaggat ctaaaatttt gccatgtaag gaacttaagc atctttatgt ttaattgcaa 1260
   aatttttata ttcccaatat aaaaatttct cttcaagtat ttcctgcatt gccatttttt 1320
   agcatgtttg gctattctgc tatgtaacct acctagtgat actcgctgga gacagtccgt 1380
   ctacaggcat gtctgatagg cacaagttct ttattcacac aaaactaaca tatagagtag 1440
   aatttatggg atgatgatgt cgtttgggat agaggtatgg aaaaaactgc attatgtcca 1500
   aaactttact acagtggagc cagtcaacat gtgtacaact taacacctaa caaaaatggc 1560
   tccaaaaagt atacatagca ctatttctgt tcatcccatc tgaatggaaa attttactta 1620
   gctggtaatt ctcaaaatgt tttgttgact cagggaaggg gaaacatatt ttacatgcac 1680
   agaatgcttc agaacttttc tgctcggcta ccaatctgcc atgtaggttg ataatcaaag 1740
   tcctaaagta cagtttagtt ctttgggcct acagggacac cttgttgact aactggcttc 1800
   agccaatttt tttcagttca cacacaagat caatttcttt gtcagcaaat accttttaga 1860
   aaaagtacac tacaaacaca cttggaaaac attttattaa gtactgtata aacagctatt 1920
   tagataataa ttgcatagaa ctataccaag gtaattgtgt ctttaaggaa caactaccaa 1980
   gtgaacaaga tgagcaaagt cctctattat acaagatttc cttcggtgga acattatggt 2040
   gacaaagcag cgtaatgagc tcttaagcag attgattttt atcaaactgg acatatcaga 2100
   attccttatg tataagagaa atatgcacat gctcctttca agaaaagagt gataacccac 2160
   catggaatta cctccagttt aaacatgtac tcttgactgc caaaaatatc gagatatgtt 2220
   aagcaagata aagcagcaga acacgcttta aaatatgttg atctctttct gtaatctaca 2280
   tgttaatatt aaatgttctt atccttgaaa aa 2312
<210> 9
   <211> 2110
   <212> DNA
   <213> Homo sapiens
<220>
   <223> nbla22739
<400> 9
   tagctttatc acttttctca ttccatatga ttgcttcttt agttaactag cccttgaaaa 60
   cttcatttta ggacttattt gttttaatgt acagatgtgg gaaaaccaca caaattccgc 120
   agtttattct ggatgattct ctgagtggac cacctgagaa ggtagccaac atcatctgta 180
   cccaaccccg acgaatctct gcaatctctg ttgctgaacg cgttgctaaa gaaagagcag 240
   agagggtggg tctgaccgtg ggataccaga ttcggttaga aagtgtcaag gtttgtatgc 300
   tctgcttatt tcctggtaac agaaatttat ggtttttagg tataaaaagt tttgggggtt 360
   aggagattca tgggcaattt gggatatata ctttcaggtt atttttaaat taatgattac 420
   ctttggtaat catttattta aatatttaga aatatttaga aatattttgg tataagaact 480
   cttatggcca ggcgcggtgg ctcacacctg taatcccagc actttgagag gccaaggcag 540
   gtggatcacc tgaggtcggg agttcgagac cagcctgacc aacatggaga aaccccatct 600
   ctactaaaaa tacaaaaatt agccgagtgt ggtggcacac gcctgtaatc ccagctactc 660
   gggagtctga ggcaggagaa tcccttgaac ctgggagacg gaagttgcaa tgagccgaga 720
   ttgcgccatt gcactccagc ctgggcaaaa agaggaaaat tctgtctcaa aaaaaaaaaa 780
   aacaaaaaaa ctcttatttg gttgtactaa atttcctctg taaagctttt tattttttat 840
   tggcagaagt catctagtaa agactgtttt gctcttgaac ttgggacata atccatttaa 900
   ccaaataagg agcagacaga ttgagaactg ttttcattat tcactgtttt ttaatgcttt 960
   ttatgaaaat cttaacattg tgatatgaag tagaaaggct tttattactg tccctggcaa 1020
   gaaactatgt ttagtatggt ttcctattaa atggaactgc tggtgtttcc aatatttttt 1080
   atcactatcc attcaaaatg gctttccagt aatgtttcct ttttttgaaa attttattaa 1140
   tgatttatat tgccctttca tgtgtaagtc ctcagccacc agactgttat actgcaccac 1200
   gggagtgctg ctgagaaggc tagaaggaga tacagctcta caaggagttt cccatatcat 1260
   tgttgatgaa gttcatgaga ggacagaaga aaggtaaaac aaagactttc ccagggaaca 1320
   cacactcacc tgaattgaag gcatggcaga aaaaattgtt ttctagttcc aattcagttt 1380
   catgcagcta gtaatggtaa tttgccacaa ggaaggccta tgttagagaa gagcaactgc 1440
   tttcttgatc tccagggtct gtaacactaa aaaggacagc acatgctcat cacttattag 1500
   atggagtcat cctgttaggt tagaaggtat acttcacacc atcctgggca ttatgctaag 1560
   ttgaataccg tacttagtag aaataacaga tgtcatgcat gctgtggctg aatgtatctt 1620
   cttccttgtt tatttggcca ttcagtcctg acattgattc atgtatttat tgagcctgca 1680
   ttaaatgcca agtgatatat tagttgctgg ggatacagtg atgaacaagc atgtatggct 1740
   ccccctcatc tcttacagtc cagtagaaaa aacaaataat gaacaagtaa acaagcaaat 1800
   gattgtaaat tggaataagc actatgaagg aataaacggc atgctgtgtt tggagggaga 1860
   gacccataga tgctcaaaga tcatatctct gtaagatgac aatttaaatt caaaactgaa 1920
   gtatggccgg gcgcaatggc tcacacccat aatccctgca ctttgggagg ccaaggtggg 1980
   aggatcgctt gagctcagga gttggagccc cacctgggca acatagtgag accatgtttc 2040
   cacgaaaagt aaaaaaaaca aaacaaaaca aaaaatagcc agtagtcatt ctactgggga 2100
   tacagaaaaa                                                   2110
<210> 10
   <211> 2416
   <212> DNA
   <213> Homo sapiens
<220>
   <223> nbla23525
<400> 10
   tcactatggc ggttggagga acggcagtga tcacacgtcg gctgctggga agatctggat 60
   tctcgtttca ggtttcgggg tgggggtggg gagaaagggt cgatgatttc cttttttcgt 120
   cgggtataga cgggattacc tagtgccttc acaatcggtc agagctggat tcagattcct 180
   gctcgccaac gcccagcttg ggcaaggctc ctgttctttc tgtgtctcgg tttccatgtt 240
   tgtaaaatgg tgataataat agtatctacc tcagagacgt gtactgtata atagtgcgta 300
   taaggcacgt aatgtgaagc ctggcccctg aagatattag ctattgttat ggagataaat 360
   aatacgcgta atagaatgag aaaaattata aattatataa attcgctaat tgtagtgccc 420
   tttctgccat caacttcttt cctagaataa attaaagata aaatagatat accaattttt 480
   accaatgaaa taatttgtta tttgggaatt gcctcaaaat agcagagatt gtaattttcc 540
   tatattgaaa agttaaataa aaggtggggg ggggggagtg caagaaagaa agagatggag 600
   aacgagagcg agctggagat gaaccacatg cgatgagtag gccttgtttg gatcctgaat 660
   cgaacacacc aactgtaaaa atatgtttaa gacgcatcgg gaaaattggg acacggattc 720
   gatatttgat gttttttagg gaattgatgt cagttttttt aggcgtcaca gtagtattgt 780
   gattatgttt tcaaattgtc cttttttgta gagacatacg aaaatattta cggattaaat 840
   aatgtctggg attggcttct aaatacatta atgactagga tttgcttcaa aataatctca 900
   gcggtagggg gaaatgggga ggggtataga tgaaacaaaa ttggccctaa attaataata 960
   tttttttgct gagtgatagg cagtgggttg cgtatattaa tctgctttcc ttggtatacg 1020
   tttaaatttt tctataataa aatacagaag tcagatattc cggtgagctt gaaaaagtcg 1080
   ggggtggggg ggagcagtgg gtggggttat agatgaaaca agatggcctt cagttggtaa 1140
   ttgttgaaag ctggatgatg gattcgtgta ggtttataat actatttctt ttttatttca 1200
   tccatttgaa attatattta aggaaagtta aaaaacaaat ttgtcagaaa ttatacaaat 1260
   gtacaataaa ttaaatttga aaatgtggcc acaagaaagg aaaaagaaac acttgtacat 1320
   tatttatcag ctttggtgtc ctttgtgtgt gatgaaattg cattggctga tgtagaagaa 1380
   agccatatct catatctttt tattttatgt tctttcttgt ccttttgttt gaccttctag 1440
   gtcaccatca gaaaagctaa gtttgctgta tagtgaggat caggagatct gatcctgatt 1500
   gcagaacctt ccctgattac agaatcttgg gtaagtgcct cccttctgtc ctcagttctc 1560
   aaacaggata ataccacata accttcctaa ctgtccagga atattttgaa aattaataag 1620
   ctcctatctg ggaaagtagt ctaaattctg agaagggaag ggtggagcta agtccattga 1680
   tagttccagt atagaaagtg cataagcaac agagggcttt gtaatcttac atcccttgat 1740
   aaaagatact acagtcaatc tcctgtagta gttccacagt tccatagatt acatttttcc 1800
   ttggagcatc ctatatgcag catagtttag tggttaagag caaatacttc ctgaatttaa 1860
   atcctggctc tgccacttaa ctatgtgatc ttgggcaaga tatttactca ctttatacct 1920
   aagttcctcg tatatgaaac agaggtgata ataatagttc ctacttcata ggattgttga 1980
   gaggattaca tgtaaagtac caggacagtg catggcacat gtaagtattt gctttaataa 2040
   taattatggt tctgttagtc ctgataatct catgttttat ctacacattt acacctactt 2100
   ctaaaagcag tggatatatt tctttttgga attgtgtaaa aaaaaaataa taattaatac 2160
   cgttggtttc tctcctcatt ttccagaagc agcagttacc actagaactg aattccgaaa 2220
   ttatgacttc tggcttgtct taacaatcta gaaaggtttc aaatatattg atcatattta 2280
   tttatgaggg aatttccagg agctataatt ttagctagca gttcaaacca aatttataaa 2340
   taagcaaatc ttttcactga atattcagtc tgctaacagc ttttgtatca ttcctccttt 2400
   gtctcagact taaaaa 2416
<210> 11
   <211> 1710
   <212> DNA
   <213> Homo sapiens
<220>
   <223> nbla20226
<400> 11
   taatgcttgc tagccagccc tcacttccta ctgtgcagcc cacagaccat taccaggcta 60
   tgacttgggt actggggacc cctgatttaa acaagagaaa tttattttct cacagttcca 120
   taggccagaa atccgagatc aagtttctgg tcaatttggt ttcttgtgag ggctctcctc 180
   ctggcttgta gacagccacc ttctctctgt gtcctcacgt ggcctttctt cagtgcatgt 240
   gtgtggggag agaaagagag agagagagag agagtgagac aggttcctct tgttataaag 300
   tgaccaatgc aatttaatta ggactccacc tgtatgagct cagttaatct taattacctc 360
   ccacagaccc catctcccag tagtcacaca ggggttaggg cttcaatgta tgaatttggg 420
   gggaacacag ttcagtccat agcacttcat tttatttttt tcctacattt aatcacctta 480
   ttgaattttc tgaatagcag ttatcactgc tggatatttt tcttactcgt gtatttatct 540
   gtttagtttt cactatcatg atttatctcc ccagagtaga atgcaaactc cattagacca 600
   ctattgtttc ttgttcatca tgatactccc agagcctaga acagtgccta gcacaaacag 660
   gacaccagaa aacatttgct tatgaagaga agagcttata ttctgtgaga gcttcaccag 720
   agcacatttt ctgaacactt cctaataacg tgacttctca tcagtacaag aaaaaccacc 780
   ccctggtgtt tcagaacagt tgttgagagg gaaaacagaa gtggagtatt tttgtcttca 840
   gctgttcatg catattctta ctttctctct agatgtctat tactgcatac acagagaata 900
   aagtgtgcca atctgacttc ctaactctaa ttgcaatcag gttgaaatga tgagtgattc 960
   ttggtccccg ttcttcagag gaggtacata tggcaggttg atcaatgttt aaatggaaac 1020
   gtgatctgtt atatagtgag cccagcagtg aaactctctt gttagcacat tcatttgtgt 1080
   gtgtgtgggt cggtgggggg cggattctac cttatatttt tcccatactg tatttatctt 1140
   ctcattataa atatttctaa aataaaaata gaacaatatt tctttgattt cttttgcatg 1200
   attattgata agactggcat tatcaaagaa gaaagcacat cagtgttaac aagggagaat 1260
   cggatttaaa ttatggcaaa tttgagaaga aatgtgtaag ttttagtaga aagagttagt 1320
   aaaaaacata cagaaataca aaaggattga cattattttc accacaataa tggagagtca 1380
   gggtgttcca tcttaggatc atggaattgt aattgaaaaa aaacatgtaa acaaaatggt 1440
   cattagaggt agtgtcctta gtgtgctcta tattgggagg tctgaaggag gaatgagaat 1500
   gaggtttgcg cctcatacaa aatatgagat catagaggga gaatttgagt tatttataaa 1560
   agttaatttt aatctctgtg ctagatggtg gctctgaaaa atgcagacac attgcttcta 1620
   ttctggttaa actaagatag gtaataactg ttacacttat acatcatgtt tctcattcgt 1680
   cattgttgct tggggaaaaa aaagcaaaaa                                  1710
<210> 12
   <211> 1714
   <212> DNA
   <213> Homo sapiens
<220>
   <223> nbla22182
<400> 12
   aattaacagt atatagttcc accattcttt tcacactgaa tatcagtata actgactgcc 60
   atccatccat tatatttata ctgtttaaaa tgtaacatgt gatagagact tttttaaatg 120
   cagtgatcat agtttttacc catcttcatg aagccaacct tggaagcagg acatggatag 180
   acagttacta tggatctttt tataggggat attatttttt ctagattatg tgtaacaaat 240
   cattccataa atgagttcat accttgttca aaaatagcac aatatttttt ttatgttaga 300
   tttacattat aacagacaaa gtgaagcaaa agattttgga attaagaaaa gtaaattgag 360
   taacagttcc actcaatgcc tatcaaatat tacctttttc atataagatt cagaatcttt 420
   caccaccatg tgtccaaata gtgtctttaa tttaaaactt taatagactg agttctacaa 480
   aggaaaaaac cctttaatat aaaagtaaaa ttaaacctca atttgctttc atcctttaac 540
   aggttcacta ccagtaacag gaattagttt ccctgtagaa acatcttata tataatgact 600
   tatgaaggaa actcactaga aagttataat aacagcatcc catttcttcc aaggactgtg 660
   ttttaatgta aatgttctct gctattatta aataggcccc tatttatgga tcagacaaga 720
   tcattctgta tatttgttct ctttcatatt gaaatgtttt tgattgggga ggagggagat 780
   ttacctaatg ctgtgtatat ataatattat ttgaacaaga agaaaacaca caaaaatgat 840
   agtatcattc tagtttggaa gtatcactct ttaaatgaaa acagggtatt tattgtaatg 900
   taaatcatgc tttatgcaaa gataatgtac caaacccatg agcagaaatc ccaccaggcc 960
   tcacatggac ctaaactggg agccagaagg ctgttaggaa cccatgagca ttcttttccc 1020
   atttcttgcc gttgattctg tctttgcatg gctgcttttt tctttctcgg cagctagctc 1080
   tctcccttgc tctattaccc agaccatgtg gcctatggaa aatggcagcc aatggcatcc 1140
   aagttcacct gtcacagttc cacccacact gcatatttct gtctttctca gtcccactcc 1200
   caaattccca aagaagagat ttcacttacc cagtttggtc catcccaata cagccagaag 1260
   gcaaggccat gtatgtataa atttagtcac caaaaatgca tttctgtggg caactaagaa 1320
   gggaagtggt tattgtgagc ttcgtagaca tcaccaaagg tgtctgcttt tgtctggatc 1380
   atcaagaaca aaggatttga agtaccattt tttaaaattt agattttgtg ccggcatgct 1440
   ggctcacacc tgtaatccca gcactttggg aggctgaggt gggcggatca cctgaggtcg 1500
   ggagttcaag accagcctga ccaacatgga gaaaccctgt ctctactaaa aatacaaaat 1560
   cagccaggct tggtggtgca tgcatgtaat cccagctact caggaggctg aggcaggaga 1620
   atctcttgaa cccaggaggt ggaggttggg gtgagctgag atcgctccat tgcactccag 1680
   cctgggcaac gagagcaaaa ctacatctca aaaa 1714
<210> 13
   <211> 1931
   <212> DNA
   <213> Homo sapiens
<220>
   <223> nbla23256
<400> 13
   cttaaatgtc agcatgtgtt catttttaac aggggtcgat tttctaatcc agcccattgt 60
   atttaaatgt gaaatagata tttttagata gcttcatctt tggcatcttt agcaaatgaa 120
   ctagctacag gagtataact tttgatgata ttttgctatc tgaggtttaa gcgtttaatt 180
   agattaaaat tcacccttca aatggagaac tcagaataag taaaatgatc agagatgact 240
   ttgtagcttc ccacctctaa taatttattc cactgttggt tatagtaatg atattgggta 300
   gtggtttggg ggcaggagat tactttttac caggttatca tttcagtatg tgttctgaag 360
   ctgatgtctt ctgataccat aatttttaca tataaatgag taaagaagaa atgtaatcag 420
   aactgtgttt gaatgcatat ctttttagtt ttgcaaaata gcatggatgt tgtaagagaa 480
   ctggaaattt agggaagttt ttaggaattc tgaaatcctt ctaggtgcct ctcagctccc 540
   cattggtttc tctatgtagc caggtaaagc catattttgt gtatgacatc agaaattgct 600
   tgtcattttg aaatttatgt ctacatttgt cttcccaggg gctcatatat tttaaaggta 660
   tacattttta tttttagaat caagtattga tttttttgtg aataaattac tataatgatg 720
   ccaattaatt gaaaatcatt tctactatta taggatgagt gaaacttaca gatgaattta 780
   aagtttcatt ctagtaattt tttatttaaa aaggattaga gattttataa tctgtcctac 840
   agttatcatt tttgaaccca atcctttgtg tattaaagaa tattatttaa aattccattt 900
   ttgaaaagct catgtcattg ctaaaggttt tgagattcta caggaagacc ttgtagacct 960
   ttttgtcacc ctttcgaaat tgaccagtat tctttctaat tgaagctttt accttttaag 1020
   taattttgac aacaatattt gttctggctg ttactataca atattgaata aattatagta 1080
   ggagggtgat ctaagattat ttctttctga aataatgata gcttagaaac ttgttaaaca 1140
   gagccttggg aatgtatggg aacttgaagt atatgcattt ggaaaacatt taatgaactt 1200
   ttttttttaa tgtagatatt aaaaattatt ttttctaaaa ttaatgttat actaaaatca 1260
   tagtttgaat tgctgacata ttaattgtgg attaaataat ctatatctta cagactgaat 1320
   catattcatg ttgttgatgt cctttagaac agagaatggg taatgtgtag attaactata 1380
   gagacattac cagtgtacat aaaagctatt aaaaatctta atattgtaat ttagcactgt 1440
   attccctcta cctagttatt tttcctcttc agctttcagc cattttctgt atactttagt 1500
   ttttagtttt tggcatcccc tctggtttga aacctatctc tctacctttc taacattttc 1560
   tatttagttt aaatatgtct ttatgcagtt atacaataac tctttgccct tgaggactga 1620
   atggtttcct ttcctataga agagttgttt tcaagctttt tttctcttgt ctccacattc 1680
   atataagcag tctgctctga tcagtagaat ttctcggata gaggtgatca cttgaagaat 1740
   gagggaggga gggtgtagtt tttaataaaa actctctaga ggttcttgtg tcccctccac 1800
   tgagaatcac acttgagagc ccatccttcc tataagattt atatctgacc tccttgaccc 1860
   gtcactctgc taaacagaaa cgttctttca tgttttgaat gtgggaagga caagcaactt 1920
   gtagacaaaa a 1931
<210> 14
   <211> 2064
   <212> DNA
   <213> Homo sapiens
<220>
   <223> nbla21297
<400> 14
   acattgatgg aaatgtatgg aaagcataca gttggaccga gaaactaatt ctcagagaaa 60
   ataacttgac tgaattacac aaggattcat ttgaaggcct gctatccctc cagtatttag 120
   atttatcctg caataaaata cagtctattg aaagacatac atttgaacca ctaccatttt 180
   tgaagtttat aaatcttagt tgcaatgtaa ttacagaact cagctttgga acatttcagg 240
   cctggcacgg aatgcagttt ttacataagt taattctcaa tcacaatcct ctgacaactg 300
   ttgaagatcc gtatctcttt aaattgccag cattaaaata tctagacatg ggaacaacgc 360
   tagtcccact tacaacactt aagaacattc tcatgatgac tgttgaactg gaaaaactct 420
   gaagaagcat cggtagggaa tccagaagga gcgttcatga aggtgttaca agcccggaag 480
   aactacacaa gcactgagct gattgttgag ccagaggagc cctcagacag cagtggcatc 540
   aacttgtcag gctttgggag tgagcagcta gacaccaatg acgagagtga ttttatcagt 600
   acactaagtt acatcttgcc ttatttctca gcggtaaacc tagatgtgaa atcactgtta 660
   ctaccgttaa ttaaactgcc aaccacaggt gagagacaga tggaaagact taacccacgc 720
   tatttccatt ttagaaagtg caaaggctag agttacaaat acgaagacgt ctaaaccaat 780
   cgtacatgcc agaaaaaaat accgctttca caaaactcgc tcccacgtga cccacagaac 840
   acccaaagtc aaaaagagtc caaaggtcag aaagaaaagt tatctgagta gactgatgct 900
   cgcaaacagg cttccattct ctgcagcgaa gagcctcata aattcccctt cacaaggggc 960
   tttttcatcc ttaggagacc tgagtcctca agaaaaccct tttctggaag tatctgctcc 1020
   ttcagaacat tttatagaaa agaataatac aaaacacaca actgcaagaa atgcctttga 1080
   agaaaatgat tttatggaaa acactaacat gccagaagga accatctctg aaaacacaaa 1140
   ctacaatcat cctcctgagg cagattccgc tgggactgca ttcaacttag ggccaactgt 1200
   taaacaaact gagacaaaat gggaatacaa caacgtgggc actgacctgt cccccgagcc 1260
   caaaagcttc aattacccat tgctctcgtc cccaggtgat cagtttgaaa ttcagctaac 1320
   ccagcagcta cagtccctta tccccaacaa caatgtgaga aggctcattg ctcatgttat 1380
   ccggaccttg aagatggact gctctggggc ccatgtgcaa gtgacctgtg ccaagctcat 1440
   ctccaggaca ggccacctga tgaagcttct cagtgggcag caggaagtaa aggcatccga 1500
   gatagaatgg gatacggacc aatggaagat tgagaactac attaatgaga gcacagaagc 1560
   ccagagtgaa cagaaagaga agtcgcttga gctcaaaaaa gaagttccag gatatggcta 1620
   tactgacaaa ctcatcttgg cattaattgt tactggaata ctaacgattt tgattatact 1680
   tttctgcctc attgtgatat gttgtcaccg aaggtcatta caagaagatg aagaaggatt 1740
   ctcaaggggc attttcagat ttctgccacg gaggggatgc tcttcgcgaa gggagagtca 1800
   ggatggactt tcctcatttg gacagccgct ctggtttaaa gatatgtaca aacctctcag 1860
   tgccacaaga ataaataatc atgcatggaa gctgcacaag aagtcatcta atgaggacaa 1920
   gatcctcaac agggaccctg ggacagcgaa agccccaacg gaggaggagg agagtgaagc 1980
   cctgccatag gaggagaaca cagcccacct caggcctcct gcaaaaatac atagaataaa 2040
   caacaacagt tactaaatga aaaa 2064
<210> 15
   <211> 1650
   <212> DNA
   <213> Homo sapiens
<220>
   <223> nbla20787
<400> 15
   atttactaag agtaattggg tttaggatgt tggaaatttt tagcttgggg gaaaaaacat 60
   tcttatgaag gagataggtt ctcttctgag tttgtcataa tatagattgg tgtctttgga 120
   aaatggccac aattttaaga attcaattat gcatataaaa tgataattat tggaattcca 180
   cagtaacaga tttaaacagt cttaaattgt ttatctcctt tactgtaatg tattgaaatt 240
   tttagagaaa ttttagttgt taacatttta ttaagtgcca gtgtcagaat ataacaaatt 300
   atagtttctt atgaatgaca ggcctacagt tattattctg gattatttga tggaggacaa 360
   acttacctgt atttgttagt caagctgtga aaataaggtg gattacaaaa gatgtgaaaa 420
   aaattttagt ctgtagactc agtaattttc tataatttac tgttaatctc atttgaacat 480
   ggattaggta caatttataa attaattcaa gtcagggtct ttaggtatca ggtgccagag 540
   agatatttaa cagatttccc tacctaaatt tatgtatatg tactgtctaa aacaatactt 600
   ttttaaaaaa aaggaacagt tgggagaaaa taaatataat gaaaaattcc cagaggctag 660
   cacttggatt ctaacacgta tgctattgta ttatccatta gttctgtaat atttaatttt 720
   agattctttt atttttttaa ttggcaaagc acaaggtgct gtataacagt gtcatttaga 780
   gttttataga aagcttcaac ctgagttctg cgttataaag cctggagaaa gctaagctta 840
   gaacataact tgctgaagta taattatctt tttgtagcag gaatttatgt gccagaggtg 900
   agagtctttc tggtactgat tttttgagac caaggataaa aggatcgttt tgtaagacat 960
   gccatggcaa tggctggttg ggggacagtt tccgcccaag cttggcctat tttatttttc 1020
   ctcataccta ctttcaaagt catttaggta tttgaagcct tatttcccac gtagtaacac 1080
   tttctggctt ttgcagtttc tttttttgtt tggttttgtt ttttgcatgg aatggggatc 1140
   aaacaacccg aagaagaaca cattttgatc aagcaaaatg tttgcttcaa atttcagaag 1200
   tttattttac agaaattaaa ttaagtagtt tgacatcctt ttctctgttt cacacatata 1260
   ttaggttggt gcataagtaa ttgtggtttt tgccatgact tttatggcaa aacctgcaat 1320
   tacttttgca ccaacttaat acatctatat acatatatat atacgcgcac acacttgttc 1380
   agaagttatg ttgtggcctt ggatttgttt ttccccttgg aaatggttct taactctggg 1440
   attttagaag gttagaatat tttttcaaga gaacagtggt actcaaaaga atgaaaggtg 1500
   gtccctacat tttctgtatt catcacttaa aatttttaat ttttccgaga actacaagta 1560
   acatttgaac catgctgctg ttgtacctta aacaaaaact cagtgataac cagtatttag 1620
   tctattaaaa atgctctttt tgaagaaaaa 1650
<210> 16
   <211> 3050
   <212> DNA
   <213> Homo sapiens
<220>
   <223> nbla22284
<400> 16
   gatgcggaag aaagagatgc tcggaaagtt ctaataaaat ggaaagatag catccctagc 60
   atttttttct tgcttataga gatattccat gggatagcaa atcctgtgtc atggagatga 120
   agtcaaaatt cctgattcca aaaggttttg agaaaacaaa gagggggaat gacgtaagaa 180
   agataggcat gagcatgtgg taactaggtt agcacgtgtg cttcccagcc caggagcgac 240
   caaatcctgt ggtggcgtca ggtgtgcagt ggagaggaat atagaggctg tatggcctcc 300
   ctcagtgagg gcagggcaag agggatcact ctgagagaac aaaaataggc cccaagttgc 360
   taagcagtga ttgggaacct tcctttcctt ggcggagatg catgacattc cctaccgatc 420
   cccagacaca gcctgtggga ctcttaggag aaatggtgat ttactgaata actgacccgt 480
   tgccgagatg agtacaatga agtggaggtg atgaactcaa atcgtcttcc agggccaggc 540
   ggctgaccgg ggtgagcgta gtggcccgct ggggaccatg gccgccctga cagccacacc 600
   cacctggagc tgacttggtt ctggctgttg ctgccactgt gaaatctgta tctctctcca 660
   tctctgctct actatccccg gccttgccag acagtgttct ttttcggaag aagtctagat 720
   ttttgcatga aaaaactcaa tctttaaagg tcgactcaga acattttaag gaggcctcca 780
   cttggtctga tgcagtcttg ctaattaaga actaaaggcc ttctgacctt cttggtgctc 840
   atgctgtacg gcatctgaat gtctcgaccg agtccgagcc gtgcagctgt cctccacctg 900
   cgaaagtaat gagaatccta tcacgggaca taaggatagg tctaaacagg gtccatgcca 960
   agaaaacagt ggggtgctct cccaggcctc tcccctgtcc actaaccctg gccttgccgg 1020
   ctgccttcca ggctctgggg gaagagctcc tgcattcttc cctggccacc ttggctccag 1080
   ggctccccag agagcctctt ccctccccaa gtacctgaga aagatgagag aggcacgtgc 1140
   tctgctggga aggtccagtg agcggttcaa gggcctggaa tctccctacg gccaagtcta 1200
   agggttctgg gattctgggc tttgtgggct ttgcttgctt gctgggaatg ggctttccct 1260
   gtcccgccct gccccacctc gcctctgtct ctcagaagct ccagaaccca gcagtgacct 1320
   gcaaaatgtg gcctctgatg ggggcttagg gtgggagatg gggagagcct acattgtctt 1380
   ttgctccttg aaaactttaa tagctcctat tttccagaga atggtgcttt gtgagcaaca 1440
   tgcgagtaag agagaaatag gaggaagggg gagtaggggc ggatgggaga agagtggctc 1500
   gtttttacct ctcactgcct tgacattttg tgaacgtgaa gcttaaactt tctgggctta 1560
   caagacccag gggcacgtca gctccttaga tgggctcagc ctgacacata attcttaaac 1620
   ctttcctgtt taagaaactt ctagaggctg tgtactctca ccaatcctct tcgagaattt 1680
   gttcatgtgt atttccccat tatatggatg aggctcagga taacagcata gtggctacct 1740
   tctactgagt tttgaggtgc taataagtat gtttgtctga ggctgcacat gtgggtggct 1800
   ctgtgtgtat gatccaaggg acaaaatgac gatgtaggaa ccagcaagaa cggaatctgg 1860
   gctgatgctt cagtctccac ctgggtgatg gctagcctcc cgccctccac caccgcatcc 1920
   cacacgtgct gcgcactgtc cccgtgtctc ctggagaacc aaactggaga aaacctttct 1980
   gagtatctct catagtaccc cttccttaag aagatgtggt ttagagcatg tgtgcaatcc 2040
   tgcctctgta attaggaaac ggagcccgag gctttccatt gttggttgaa cccaggacag 2100
   ctggtgctat tcacaggctg aagaactggg cagttcttac ttgggtctgt cctaggatgt 2160
   ggaggaagtt caggactaac gctaggcaga gagtatgact cggtttaccc agcctagggg 2220
   cctctggatg ggaacactcc attccaagat ctcagcagag cagggcttcc tggcttgagg 2280
   ctggaagcct ttgggaagag gcccagctgg gacattccct gggcacctgt cttccgctga 2340
   agggagcaag gtgccctctg ggactgacag ccatgaccct ctgtgccatc ctcaatcctt 2400
   gagccatata tcaagagtcc tctagagccg gatggtcctc aaaagtctgt ccaaggaatg 2460
   ccaacgttca ccgggctctg agaaacgacg caaatctctg agctggggac cacttggaga 2520
   accggcttag taacagtcct gatcttcgca agccagcttc ttctgcatct gaggggctcc 2580
   tggcgcccag aggaggcaga cagatgtctt ctagctgagt ttctaaccgc atgatgagac 2640
   tcagaccttc cgctgcacta gaaaatctgc aacagtgtcc ctgagtcact tctccttagt 2700
   gggcagactc gtgttagatt tgtggaaccc agctctctga tttactcctt ttggaaaacc 2760
   catggaattt catgtataag gctttcattt gtattttaag gtttttctgt ttgttttgag 2820
   tatatacatg gtgctcaata gcaacatctt agcagatgaa gcagtttatg attccactcc 2880
   ctcctgtatg acaggtagcc actatactga atcaaggtgc tgaactcaaa tcacaaaatt 2940
   ctggcttacc gatacaacaa ccaatacatc tttgttctgt aatgtaaaat ttgactcctt 3000
   acttttataa cttattaaag ttaaaatgtc tgtgtttttg caatcaaaaa 3050

<210> 17
   <211> 1733
   <212> DNA
   <213> Homo sapiens
<220>
   <223> nbla20123
<400> 17
   gatacactga accccacgcc tccaacgcaa ggtgaaaggc atcacaaaat aggcactgag 60
   tctgcccctt ggatgaagtt agcatttttt ggcccgagga gcatctgctc tggcactgaa 120
   acagcaatac cgacacggag acgagagcca tgcaaaaaca ctcagtttcc gagccccagc 180
   caggacagtg cgagtgagta tcctgctttt ctttgtggtt agaatcaagt gtcatctaaa 240
   aataaccggt tggtaggaag aagtcactgc atagtacaat gccaagaaac ccggggatca 300
   gagagtcctc cgataactga tgctgctcgg ggctcacgtt tgtttggaaa actaaatctg 360
   cctccatttt ctgtgccgga aaaatcatcg cttcctgcca ccacagaaac cttacctttt 420
   gcagaagctg ggaaccggag tacttagcag caatggattt tatctcccca ccaaaagccg 480
   aggcccagag cttcacccta cagggagaag gggcacagga agatatgtaa cacccgtgtc 540
   acagtcaaca cgcacgcaca cgcacacgca cacatgggac tatggctgaa ggagcagtgc 600
   gatgtaacat gttttaaaag aagaaaagat agaaaaagcg gcttggtaga aactgccagc 660
   accaaaactg caaagcgcag cgcgggaggg ggcccgaggg gggtcgcgga gttaagaatg 720
   cgcaaagtct cccaggctct ctaaaaagac cactgagttt cattcgaacc actgcccgag 780
   gactcgaccc cccaaactgg gcatcacctg gcaaatagca gtcagaagaa atccacccat 840
   ccccccccca aaaaaagaag tggggcggca gtagagcaaa gaggggggaa attcagcggc 900
   ccatggaagt tggattcggt aaccaggctc caaagttggt gccgtcactt gagtagagac 960
   ggggtttcac cgcgttagcc aggatggtct ccatctcctg atcttgtgat ccgcccgcct 1020
   cggcctccca aagtgctgga attacaggtt gccctgaatc tcaagtccag aaatccacta 1080
   gaggacctgt tacggtggag agaagatcag tctccattaa ggttggcgat tgatcaggac 1140
   tatttatcaa gaaaatcaaa gacaaagaca gatcctagga ggttctcatt taaccaaatg 1200
   gatagaaatc agatcactgt tgaacatcta gttggaactg actttgccgc tctactcaaa 1260
   tggtgaaggc ttccttcttc caacagactg tgtggcagca tgaattatgg gcagggatct 1320
   gtgactgctc aacttttctc tggaggccct gctcaggggt tcagctgtcc tgttcctcag 1380
   tgtcacatct tccacaaagc cattcaccct ttaaggattc actgagcact catcctgtgt 1440
   caggtgctga gctgagcacc tgggatttgg aggacaggaa gacacagtcc cacaatcaga 1500
   agagaagcct ttccctagcc ttctctcaga gcactccccc agaatccctt agcctatgat 1560
   ctgcatctcc tgggcacctt tcctttccac cttcttttac ctttgtcttc tacttccagt 1620
   cctcttacca ccaggccatc tgtcccttga gggctgcctc agaatctccc acagcatgta 1680
   acagaatgag tggcacacag cagaagctca ataaatattt atggaatgaa aaa        1733
<210> 18
   <211> 1498
   <212> DNA
   <213> Homo sapiens
<220>
   <223> nbla20382
<400> 18
   atttcaaaat tggtacacct gcagtactgg agcttcaaag acaatgtctc cactgtcaat 60
   gattaaacac ttgtgcaagg gagtcagata tgcctggtgc tgataatacc atggtgggtt 120
   cagtgcagtc aggatggtgt gaatgaagaa cttctagaac actaaggaat atgtaaaata 180
   tacccttctt ctgaggaagt agagttgaca tttgagcttg aaggaccaat tggaataagg 240
   tttccgaaat atgttatgat gggtgggagt gtggaattgc aagcaaagca aagagtgtga 300
   ccaaactggc aacgttggaa actgatcata gactgtttga ggaatggcag gtcccctgat 360
   aaaagcagtg ccaggagaga gttgctaagc ctggaaagag ccttgcaaga gtattcaaag 420
   aataagggct ttgtttcaca ggcagtgagg aactgtcgtc atccttaagc tggacagtga 480
   tgtgttcaga ctgctgggtc tattcttcct ccgttctttc cttcctttct tccctcttga 540
   tgatttccat gctttgtgga ggttgtgtta gagtaaaaat aaaaaataca taaagcgtgg 600
   cactgtcatt ctctgctagt ggagatgcaa actgacacag ctcttctgga ggaaaaatag 660
   gtgatacata acaagaccaa cttttaactc aggatcttac tttcagtaat ttatgcaaaa 720
   gatctacctg caagaatatg aaaagacaag tggataagat tatttactgt agtattcttg 780
   gtaatagcaa aatattagat gttttgctat tacctaaata ttcacaccta agagaatggt 840
   tgaataaatg atagtgcagc tacacagtgg agtacaatgc aactgtaaaa tagagtgagg 900
   aaagttactg tgaattgatt gctattgaat aatgtccagg atatgctgta aagtggaaag 960
   gcaaagtgca gaagggttac tctgagatat tccttactta ataaaataaa aaggatatat 1020
   gaaaaataag catgcacctg ctaatttgta caagagaaat actggaaaga taaatcagaa 1080
   accagtgaaa taaattacct ataggaagtg gatgaggaag gagtagaagg aagaggaccg 1140
   aggtagtaga gatgaggaag aacagcactt ctcttatgcc ttagtttagc ttggccttta 1200
   ggaagtagag tagactgggc atggtggctc acgcctgtaa tcccagcatt ttgagaagcc 1260
   aaggtgggca catcacctga ggtcaggagt tcaagaccag cctggccaac atggcaaaac 1320
   gccatctcta ctaaaaatac aaaaattagc tggttgtggt ggcacgtgcc tgtagttcca 1380
   gctacttgga ggctgaggta ggagaatcac ttgaacccgg gaggtggagg ttgcagtgag 1440
   ctgagattgt gccactgcac tccagcctgg gcaacagaga gagactccca ctcaaaaa 1498
<210> 19
   <211> 2256
   <212> DNA
   <213> Homo sapiens
<220>
   <223> nbla20660
<400> 19
   ttaaaacttg tccgggcatg gtggtagctc aggagttcaa ggctacagtg aactatgatt 60
   gtgccactgc accccagctt gggtgacaga cagagtgaga ccctgtctct aagaaataaa 120
   taaaaataaa aaataagagg agcttttgga attcagcttc ttggaaggct gaggtgggag 180
   gatcacttga gcctgggcat ggaggttgta gtgagccatg atcacgccac tgcactccag 240
   cctgaatgac agagtgagac cctgtttcca aaaaaaaaaa aatgtgtgtg tgtgtgtgtt 300
   gtgtgtatat atataatata tatatatata tacacacaca cacaacacag acacaatttg 360
   tgtgtagcta ggggcagata ttgagatatt gaagtgataa gtaactgggg atggggaagt 420
   actggtcact taagagcata tagaaaaccg tcccagattg tcttttctaa tctatttttg 480
   gaggaggttt ttatatatcc catgttttat attatttctc ccaaaccgga ttagatatag 540
   tgaacaataa aataaatgca gtttccaaaa ccttggtgtt cagaaatgaa gggaaccatg 600
   aggggagtga aggggacttg ccctttgctc tgtgctgtat gcactgccca gggaacagcc 660
   ccaggacact tctatagttt ctttctgaga ctcacaaggt gttagcaatg ctctgagctc 720
   actcaattga cagatacgtt taaggttctc aaataaattt caaacttcta aatttttcct 780
   tttcattgtg tgcataatgt acagattagg aaaatgatct tctaattgag aagtatactt 840
   caaagtttgg aaataaaatc ataaaaatgt tttcctaaac atagcctttt tcaggagttt 900
   ttgtggatat ggtcaaaggc aatagctcta attatctggg gtcctcagga caggaaatga 960
   gctcacactc atgctctcaa actgtgtcac agcatttttg gaaatatttt catttctatt 1020
   caagaggagg aacaaggccc caagtgttca ccctaattgt tgaaaataaa cataaacatg 1080
   aaattcacaa aagaacaact ataaatggct gcaaatatgt gaaactatgt ttaacttccc 1140
   agggagtcaa aaaatactaa ttaatacaag aatcatcttt ggcccaccac attatgattt 1200
   tgtctgaata agcctcttca atgctggcaa atatgaggta aaatggctgc tccggctgct 1260
   ctttttggct ggtcttaagg ggcgcaaata gctccacccc atttggaaag cacttggcaa 1320
   tggctgctaa gactttagtg tttttcatag cttctaacct gctaagaagt agatacttgt 1380
   tcccattttg ctgctgtgca aacagacttg tagaggtcaa gtatctcgta caaggttaca 1440
   ttgatggtga ttgatggcgc caagatttga acttggttgt gagtccaaag tctaggtctc 1500
   ccattctacc catgtgattt tacacacatg cctgatataa ttagctcctc ctcctctcca 1560
   gagaagggca gctgaccttt gtttcccagt tcagaaatcc tggtgtgagt tatcagctgg 1620
   ggttgagggt ggatagattt gttccaactt tacacattgg acctgagaat gtactttcct 1680
   gaataaacag ttgtagaagc gagtgacagt tgtggttgaa gttgttcccc cagtgcagtg 1740
   ccccagaggg gttaatgacc tttcgtgggc tgtcccagga acttcatccg aggaaaaggt 1800
   gcttacctgc taacatttga cctgtttgga aattggggat tgtttttcct cattgaaatt 1860
   ggtgagggtt ggaagaatac gcaaacgaat gtttggtgtg gaagaacgct ggaggagtaa 1920
   acttactata ctcacaattt ggattacaac atagtttggt taacccagct ctggtgaacc 1980
   aaatgtacaa gtattatttc cttatggttc atcttataaa atattttata aattggttgc 2040
   tttctttaag ctctccacaa atgaaaaatc agtcccaaaa atctataaaa gactatttca 2100
   gcgttaattg accattaagg aaatacatac taggctgcat gtggtagctc acgcctgtaa 2160
   tccccacact ttgggaggct gaggcagaca gatagcctga gctcaggagt tcgagaccag 2220
   cctgggtaac ttggcaaaac cctgtctcta caaaaa 2256
<210> 20
   <211> 1411
   <212> DNA
   <213> Homo sapiens
<220>
   <223> nbla20666
<400> 20
   ttaaaaatta gccaggcatg gtgactggtg tctatagccc cacctgctca ggaggctgag 60
   gtgggaggac cacttgagcc caggagtgtg aggatgcagt gaatgccatg atcacaccat 120
   acactccagc ctgggtgaca gagtaaggcc ctgttaaaaa aaaaaaaaaa agtcctcctt 180
   aaagacatgg gctttctaga cagggttctt ctgctgaagc ggctttcctt ctgccagaat 240
   ctcaggaact cctggatctg cctttccaga accagcttct ctctccctgc tctgccttca 300
   gactgccctc tttctacctc tccctctaga actacatctc ttctggctgg gtttatagct 360
   tggggctggg ggaggcccag tgggactggc tgagtggagc cagccgtgtg acggaggcgg 420
   ccctcttcca gttgggcact gccaccctct cgtggtccaa gcagcacatg agcagaacca 480
   ggtgctcaac accaacagcc ggtacctgca tgacaacatc gtggactatg cgcagaggct 540
   gtcagagacc ctgccggagc agctctgtgt gttctatttc ctgaattctg ggtaagtgga 600
   ctgtggccag cccccgggaa gagggtgaga cggtaacaaa gacagtcact cacatgggcc 660
   cagtgtagtg tagctgactg agtgtggact cggagaggca gcccccactg caccaggctc 720
   ctgagattcc cggctgtagg ccctgatgct ttctctgttg gatccagttt ccttgtctct 780
   tattgaagga tgttattacc tcctttctag gatcattgct ggagcttagt gaggtaatat 840
   gttcctttat ttctgcctta cggatacagc caaaatccct gcctgtgggt tgctcagtaa 900
   ggaaggaaaa catcaagtga ttcttcaaag aaatacagaa ttgcaaggag ggctctggag 960
   gaagtgtaca gggtatcatg aggcctagaa aaagtgaggg gacctgacct gggggttcag 1020
   ggaaaccttc cctgaggaag ggctgttaag ctcagagctg actaggagat aactagaaga 1080
   ggaggaagga agggtgctgc cactgcatca gaagtctcgt caaggctggg cacgatggct 1140
   catgcctgta atcccagcac ttcgggagat cgaggtgggc ggatcacctg aggtcgggag 1200
   tttgagacca gcccggccaa cagggcgaaa ccccgtctct actaaaaata cacaaaattt 1260
   agctgggcgt ggtggtgggt gcctgtaatc ccaactactc aggaggctgg ggcaggagaa 1320
   tcgcttgaac cgggaggcgg aggttgcagt gagccaaaat tgcaccactg cactgcagtc 1380
   tgcaggacag agagaggctc tatctcaaaa a 1411
<210> 21
   <211> 1346
   <212> DNA
   <213> Homo sapiens
<220>
   <223> nbla21239
<400> 21
   attactacat tataaataaa ttcacagttg tataataaat tgctaactgt ctgtcataac 60
   tgatagactt tcagccccac cagggctgga aaaagtctgt cttctccact aagctatgtg 120
   tcttgcaagt atcagactgt cagcaaattg tgaaaataat aagtgaatta aataatgcat 180
   ttgatagtct agcaatagat ctggctactc agcagcgtct ctgacagcat ccactttaga 240
   aataggcata tgtttttccc actttcgcac tgtgtatcac tgtgatgcag gtccttaaag 300
   caattgacca gctaggtctc attcagaaaa gagcagtcct gtcaggcgcc cagcctatgt 360
   ctgtatcagg tcctactact tggtacattg tctgtcctga gaagcagcat catttggtcc 420
   atgcttatga cctctgccca gaatctcttg aaaaggagac cacaggaagc aggcatcatg 480
   aaggagtctt cagaagaggc agtgtaccag gaaggcacct tgtctggacc ccctgccggg 540
   tattcaaatt ttgctataca ttagaatcac ttgtcaaaac cccagtggcc agatgaatcc 600
   caataagttt taaatcagaa tttttggaag tcagacgcag acatcaatat tttctaggat 660
   tgccaggtga ttccagcatg tagccaagtt acagatgcca cactctagga ttttgtgact 720
   agtgctccag gaccagggac attggcatct gctgggagtg ttttaggagg gcaaaatcat 780
   agctctgtcc cagatctatg aaatcagaat ttgcatcata aagcaaatcc cttgtgtaga 840
   gttgtctgag ctccttatac attctgataa tcaatcctca ttgattgctg tgcatgttga 900
   ggtgtgagaa gcactgccct agcacagaga gcagtatcac accattaact tactcctggc 960
   cattttcttt ctcttttgtc cttctctctt ccacctgtct cttcactcta tataccagcc 1020
   atctagaact ccaattacct gaaatgcaac ctctttcttt cttagtaaag tgctgttagt 1080
   attacaaaac ctttaaacat ttagaaagtt caggggaaaa tgtgatgaaa ccctatgtat 1140
   gcaccattaa tatgtaacaa aaataaactt actatcattg agtcttttct tatttaaaaa 1200
   aaaaatgcta caaggccagg cccggtatct catgcctgta atcctagcac tttgggaggc 1260
   caagcggaga ggatcacttg aggccaggag tttgagacca gcctggccaa catggtgaaa 1320
   tcctgtctct aataaaaata caaaaa                                      1346
<210> 22
   <211> 2798
   <212> DNA
   <213> Homo sapiens
<220>
   <223> nbla21729
<400> 22
   caaaagatgc tgttttacat aaggctactc aataccctga taaattactg gtctactaag 60
   gtgaatctgt atctgaattt tattttcaaa gaggatgaaa agattgtttt aatacatact 120
   gttttgacat ttctaccaat ctgtgtgtct caaagagatt tgtgtgtttt tgttgaatat 180
   ggttttacct agtatttcct gacttcataa ttttattttg taattaagca atataagact 240
   ataaataaga gtgcttagag aaaacaaaga ctagtcagac ctaaaattct aaattgggta 300
   tatattttta agtattattc gaaccagaga aaagaagcac aagtgaaata gagcttaacc 360
   tcatcagagt cacttgatcc atggaaacca aggggtagaa atttcccctc cctgggcctt 420
   tctgaggtat cctggtcatt gattcttatt aaacccttgg gagtttagta tttaaaattc 480
   caaagcccat tctggcaaaa gtaatttcaa gaactaccta tttaatggga aagccaattg 540
   aataataaag gccatgaatt ataatatatt tagaatatat tcagggttcc tcccacgact 600
   ccccccgccc cccgagtata ttatagtgtc aaaaagcatg gctaatggga agtgctgcta 660
   aaaagaggtc ctgccagacc tgctttatct aatcctgagg aattaattca gaacttaata 720
   ggttttgcag ttgtggtttg tttttaaaat atcaataatt ctgagtagat tcaaggtctt 780
   ttttttgttt tgttttgttt tgttttgttt ttgagacgga gtctcactct gttgctaggc 840
   tagagtgcag tggcatgatc tcggctcact gtaacctccg tctcctgggt tcaagcaatt 900
   ctcctgcctc agccccctga gtagctagga ttacaggtgt gcgctaccat gcccagctaa 960
   ttttagtatt tttagtagag acaggatttt accatgttgg ccaagatggt ctcgatctct 1020
   taaccttctg atccacccac ctcggcctcc caaagtgctg ggattacagg catgagccac 1080
   cacacccggc ctcaattttt tttttttttt tttttttttt tttactaact tagtcttctc 1140
   ctctcctctg tctaccctta gcaatatata ggtaaacata tccagcttgt ctaacacatc 1200
   acagattatt agttaacaag gtgtagatta atgagcttat attgtattgc tggatctttt 1260
   gagttaataa caatggtaac ttgtccagaa ggcctatcat cattcctagt aggtgggcac 1320
   agagtaagag atattaagaa gcttcctgat gagtcatcat ctagcgaagg ccctgtgtag 1380
   ggctttatta taggagttac attgacttct ggggcattca aaggtctccc ctcttatcca 1440
   tatctctgtc attttgcttc tccagccacg acaacacact ttcctctcca actgctccct 1500
   ccccaccaaa aaagaagacc ctctaaaagg caaaggaata aatattctta gaagtaaatt 1560
   atcttcatcc catgctgcct ttttcaaaga ggtgttagga tatttatcct atttctgtat 1620
   ttcacagtag cttttcaggc tgtcctgctt atatataagc tgatttatat tgagaaaaat 1680
   cacttttgaa taaagaggat gaaatgactt tacaccccat taaatactca gtcaagctta 1740
   gccatgactc agtaactaaa aagttcaaaa aatccagtta tgtaatgtgc agagtaacaa 1800
   attgcaagaa aaacaactta atcttccagt gactaagtaa gaaaaactgt tgtcactatt 1860
   aaacatgtag gaaattgata attattacaa acaaagcaat actctaccct aaatctagac 1920
   aaatcactgg acagatgata agattttcag ctttctcctt taaagagctg tgccaatgta 1980
   cagatttttt tgtaaacatg caaagggaag gttacaaact ccttaaactt taaaaaacca 2040
   taaatccttt ctttgctact tatattctat gccaattata atattccaag acttaccttt 2100
   cttcagaatg cttacatatg gaaaggttta tttataaata tttgataggt aaatattcca 2160
   tatgtatttt ctagcccgtc tttctctgtc cctccctcaa ataacttcat taccctctcc 2220
   tttttaaacg aaatatcttg ataataagaa aacaaaatca tttttttgtg aaataataca 2280
   tatggacaaa aaatacaagt tgtattttac ttctggttca ttaaaatatt gtgtttagtt 2340
   ggattttttc ctcctttatt ttcagaaaca taaaagaaat tgttttattt cctaaaggat 2400
   aaaattggat atagcctctt tagtagacac tatcacagtt ctgttgtttg ctgtgttcat 2460
   ttgcttaatg aattgcgtga gaacagtcac tgtaatgaaa tatgtgtgct gggggtgggg 2520
   ggaagggcat gggaaatgtt ttatgaaaaa aagttataag cctaatacta tgaagtaaca 2580
   tctaatgcag ttctttttaa gtgcaatata tttatttctg ctagaaatat attatcaacc 2640
   ttatgtaata tttgaagcat tacatattat ttgtaaacag cttaaaatta tatattaccc 2700
   caattgtaca taagtacaaa tgtgtggata ttagtttctt tcattaaaag tggtgttttt 2760
   ttaaaaatac atttgcaccc atttacacct ttcaaaaa                        2798
<210> 23
   <211> 3322
   <212> DNA
   <213> Homo sapiens
<220>
   <223> nbla21831
<400> 23
   ctcattttct cttgctgccg ccatgattct gaggcctccc cagccatgtg gaactcttgt 60
   gttgtgtttt taatgggaga gttggtcagc gtctgctgga acagagctac gcctatggaa 120
   ccgtagactt gttcgtgctt tattgcaata ctttaaagac acaaagtctc aacaaccatc 180
   ttccgcttga cgagacagat cattctaatt tgagcagaag ctactatgtc ctgccctttg 240
   aacgcggcgg cccggacagc tgacaaggac acactgtgta tttccattcc aattctggga 300
   gtgctctgag gcctctgggg gagaaggacc catgaaatat tcaaaacata agtgaataaa 360
   atatctaggt gctagatatg ggccaggaag agccctcggc cctgcaaagt gtgtgtgatg 420
   gtgagaagct accggaagag atggtccctg tgcttggttc attccttgga catttatcaa 480
   gctgacgaat gtagcagagg tgcttcagtc ggctgtaatt ccacgggtgg agtgctggct 540
   ggagagttac ctggggctgt cacactgcat gggctccggg acactgtggc tgcccttatg 600
   tggtgtcccc ggagggccct gcaggtgtca caccgctgct ccacactgcc acctgctgtc 660
   agcatctgtg caacgtatcc aggtctctgg gggctagaat gaaaaacatg catctcgtaa 720
   ccaatgaaat cgggcttgtc ctgaagacct cgtgcattca tccattctca cactgctata 780
   aagacatacc taagactggg cacttcgtga agaagggagg tttaattggc tcacggttct 840
   gcgggcttta caggaagcat ggcagcttcc acttctaggg aggcctcagg aaacttatag 900
   tcatggtgga aggtgaaggc gggacaaggc gtctcacatg ggagcagtag agagagaaaa 960
   agaggggttg ccgcacactt tgaaacaacc agatctaacg ataactcact atcatgagaa 1020
   cagcaccaag aagctggcgc taacccactt gtgaaggacc accaccatga tccaatccct 1080
   tcccaccagg tcccacttcc aacgttgggg attacacttc acggtcacat ggagatggca 1140
   gagcacctgc acgtgcacct ggagaccctc tcaagcctcg tctcctggca ctgcctcctc 1200
   ctgacattgg aggctgctgg gagtaccagc ctgtaaccct cgttgtgatg gcacctgcct 1260
   ggtgctataa ttcagacatt tgtctcccca acctcatgtt gaaatttgaa ccccaatgtt 1320
   ggaggtggga cctgacagaa ggtgcctagg acatgagagc ttggtgctgt cctcgcggtc 1380
   atgaatgcat tcatgcttta ttccttctca caagaactga ttgttaaaaa cgcttggcac 1440
   ctcctctgcc cactctctct tgctccctct ctcaccatat ggtctgcatg cacctgctcc 1500
   catcgcctta gcatcgagtc ggccttgttg acctactgga ataattaggt ctaagtggag 1560
   ttttaaggtt actgatgact tacaaataat gggctctgat tgggcaatac tcatttgagt 1620
   tccttccatt tgacctaatt taactggtga aatttaaagt gaattcatgg gctcatcttt 1680
   aaagctttta ctaaaagatt ttcagctgaa tggaactcat tagctgtgtg catataaaaa 1740
   gatcacatca ggtggatgga gagacatttg atcccttgtt tgcttaataa attataaaat 1800
   gatggcttgg aaaagcaggc tagtctaacc atggtgctat tattaggctt gcttgttaca 1860
   cacacaggtc taagcctagt atgtcaataa agcaaatact tactgttttg tttctattaa 1920
   tgattcccaa accttgttgc aagtttttgc attggcatct ttggatttca gtcttgatgt 1980
   ttgttctatc agacttaacc ttttatttcc tgtccttcct tgaaattgct gattgttctg 2040
   ctccctctac agatatttat atcaattcct acagctttcc cctgccatcc ctgaactctt 2100
   tctagccctt ttagattttg gcactgtgaa acccctgctg gaaacctgag tgaccctccc 2160
   tccccaccaa gagtccacag acctttcatc tttcacgaac ttgatcctgt tagcaggtgg 2220
   taataccatg ggtgctgtga cactaacagt cattgagagg tgggaggaag tcccttttcc 2280
   ttggactggt atcttttcaa ctattgtttt atcctgtctt tgggggcaat gtgtcaaaag 2340
   tcccctcagg aattttcaga ggaaagaaca ttttatgagg ctttctctaa agtttccttt 2400
   gtataggagt atgctcactt aaatttacag aaagaggtga gctgtgttaa acctcagagt 2460
   ttaaaagcta ctgataaact gaagaaagtg tctatattgg aactagggtc atttgaaagc 2520
   ttcagtctcg gaacatgacc tttagtctgt ggactccatt taaaaatagg tatgaataag 2580
   atgactaaga atgtaatggg gaagaattgc cctgcctgcc catctcagag ccataaggtc 2640
   atctttgcta gagctatttt tacctatgta tttatcgttc ttgatcataa gccgcttatt 2700
   tatatcatgt atctctaagg acctaaaagc actttatgta gtttttaatt aatcttaaga 2760
   tctggttacg gtaactaaaa aagcctgtct gccaaatcca gtggaaacaa gtgcatagat 2820
   gtgaattggt ttttaggggc cccacttccc aattcattag gtatgactgt ggaaatacag 2880
   acaaggatct tagttgatat tttgggcttg gggcagtgag ggcttaggac accccaagtg 2940
   gtttgggaaa ggaggagggg agtggtgggt ttataggggg aggaggaggc aggtggtcta 3000
   agtgctgact ggctacgtag ttcgggcaaa tcctccaaaa gggaaaggga ggatttgctt 3060
   agaaggatgg cgctcccagt gactactttt tgacttctgt ttgtcttacg cttctctcag 3120
   ggaaaaacat gcagtcctct agtgtttcat gtacattctg tggggggtga acaccttggt 3180
   tctggttaaa cagctgtact tttgatagct gtgccaggaa gggttaggac caactacaaa 3240
   ttaatgttgg ttgtcaaatg tagtgtgttt ccctaacttt ctgtttttcc tgagaaaaaa 3300
   aaataaatct tttattcaaa aa 3322
<210> 24
   <211> 1823
   <212> DNA
   <213> Homo sapiens
<220>
   <223> nbla22826
<400> 24
   tgcatttaag caatccttcc ccttccttca gaatccccac ctaatagcca tgaagctgta 60
   gaaatggaaa taaatccaaa atagcaccat cagaataagt gccatcagca aaccagaaat 120
   ttagttgtgt tctggaaagc cgaaagtaat aaaaccctac tgaaaaatac ccctgaacag 180
   ggaaggtcgt gacacagcaa aggaagaatc agacaggaac aagttttagt ggtggtggga 240
   acagccccca ggagccccag gaaagaccac atttccactg gaccccaaga gagaacaagt 300
   gcgaattgct tgcagtgatg ggaacacctg gccatccttc aaccattacc cctccacccc 360
   catcctcacg gattcccaca cagagctttc aggatgattt tttctcaaaa acccccaaaa 420
   acaaaaagta ccataatatt tgctaaaaaa aaaaaaaaat tgaacagttc actcctcact 480
   gagaactaat accaaagaga gaaacagaat acattctaag atagtaccag accttaaaaa 540
   tagatgacat ggagtaatgg cagaagagtc aactatttct caagggaaat aaacaaaaat 600
   tctatacacc taaagtacag tgctttatat ttttcttaga ggagtgggtg gaggaagggc 660
   ttgggcttac agcttgcctg gaggcttctc ttctcttgag ccctaaatga atccttcaca 720
   tcagcatacc ctgcccactt acaaagagcc ataaatcagc tcttccctac aaaggatagg 780
   tgtgttagaa aaattgatcg gaatactgat acaggaaagc cacgccaact acctttgtta 840
   accaattttt tatttaaaaa tatgaatata taaccagtga cgccaaaaag aaagactagt 900
   cccaaaggaa atctaggaaa tctaattcaa ggtaaagaag aaaaaagttt caagtataat 960
   tgcagtcctt agaaagattt gaaattattt gtgttaaata aaaagagaac agattggtat 1020
   gaaaaagagg taattacaga acaaatgaac acttgagaat taaaaatatg attgacaaac 1080
   aatagaaggg atgataatag ctgaagtctg aaacgttgaa tataaagttg aaaacttttt 1140
   ttttctgagt ataaagcaaa acacagatgg aaaatatgaa agggattgaa gatacacagc 1200
   cagtcaaggt ggcagaaaaa gaaaatggag aggaatgaat aataacagaa atagagcact 1260
   aagggaaatg agcaacttac aatcagaaaa gaacccctta caaaaaagga aacgagacca 1320
   cgagcaagag caagaacaaa caggacagcg gagaatcaga ctcctaattc agaaactggg 1380
   gttatcaagc ctagaatgtg aaattagagc ccttgcttta atttctggaa ataaaagaga 1440
   ggattggaaa tgtggtaaag agcaagaaaa cttggaggag tgttaaacag aattctagga 1500
   ataaaataat ataataggaa ttagaatttc catgggcaga tgtaacagca cattagacat 1560
   agctgagaaa gaattaatga attggaaagt tgaatttaag aaattatcta gaatgcagcc 1620
   tagagagaca gaaatggaaa acaggaaatt agttaagaga catggagata aagtggggaa 1680
   gtctaacatg catctaacta gaatttcaga aagggaaagg gaagcgagac agtactgaag 1740
   atgattgatg gctgagaatt ttccagactt gaaagacatt aatccacaga gtcaagaaac 1800
   ccagtgaata ccaaggataa aaa                                         1823
<210> 25
   <211> 1751
   <212> DNA
   <213> Homo sapiens
<220>
   <223> nbla23899
<400> 25
   acaagatcca aggcatccgg agagtaggga ccatgcctcc aatttcttcc aggaggtctg 60
   gtgacgctga aggcagcctc taccttcctc tgcgcctgac tattccctgc tctctgagct 120
   acttctcatc tgggaaatgg aggcataacc ccagatgtac agggggattg ataacacaga 180
   tcaaacaatg agcgcgatgt caggcgcaca gcaggtcctc aggcagcact agctgaatat 240
   gtgaacaaat gagtggacag agggatggat ggaaggattc ttgaagcttc cactgcacag 300
   ggctgttgaa acaacacaac gcgggacctg gatgtagatt tcatctcgca gctgagccat 360
   gtgcttctct gccttgcatt tcatccaagc ccccagtatg agggggacac agggctggct 420
   cagagcaggc cccgctcagc aaaactcact gaactcccaa cagggcaaaa cctgcaggcc 480
   ccacagggag cttgggacct gactgagaag aatcagggtt cccaggggtc tcagtcacag 540
   ggaaggtcac atccatctct ctggggaaca ttatcactgg gttgaaatgg aagccaaagg 600
   gtaaaaagac acccgagtct gtgaagcagg aactggcaaa gcccatgtgg cagacatgca 660
   gcctcctata accctctgcc aaggccagcc tggacccacc ttctccacac agccctccca 720
   gacttcctct gtctggacac aacaggaccc actggggaaa acaatgatga cttgggagtc 780
   tgacaacctg ggctccattc ccaggtgtgg cacgtactgg atggatgaag ggccagcatt 840
   ccctctattt ttttattttt attttttttt ttgagacagt cttggctcac tgcagcctcc 900
   gcctcctggg ttgaagcaat tctcctgcct tagcctccca agcagctggc actgcaggca 960
   tgagccacca cgcccggcta atttttgtat tttcagtaga gatggggttt taccatgttg 1020
   tccaggctgc tctcgaactc ctagcctcaa gcaatctgcc ctcctaggcc tcccaaagtg 1080
   ctgggattac aggtgagaat ctggccccca actccccctc ctgatgcctc agtttcctgc 1140
   cctgcaaaat ggagatataa tgccaacttc aaaagattgc tgtgagtatt atatgcgata 1200
   atgcctggca agagcccagt gggaggcctg gctctaaaga gggtggcagt tttaatgaga 1260
   aggtgtcagc actcagggaa cgttgactgg tgacctatgt gactgaggcc actggggagg 1320
   agaacctgca ggtcccagga cagggaagag actggtctgt ccccaggaaa ctcctgggtt 1380
   tctgttcctc tggcctaagg gtcatagcaa ggcaaaaggc aggaaagggt gaagagccgt 1440
   gaaagtgata gaggctgctg ggcgtggtgg ctcgcgcctg caatcccagc actttgggag 1500
   gctgaggcat gtggatcacc tgaggtcgga agtttgagac cagcctggcc aacctggtga 1560
   aggcctgtct ctgctgaaaa tgcagggatt ggccgggcgt ggtggtgcat gcctgtagtc 1620
   cataatccct gctgccaggg aggctgaggc agaagaatcg ctattgaatc cgagaggcgg 1680
   aggttgcagt gagccgaggt cgcaccactg cacttcaggc tgggagacag agtgagactc 1740
   agtctcaaaa a 1751
<210> 26
   <211> 1264
   <212> DNA
   <213> Homo sapiens
<220>
   <223> nbla20578
<400> 26
   atgtgggatg taaaattgga tggggttaga gatgagtgca ggcaattcaa cgcattggta 60
   ggggtggaag ttctcagcag aaatcaccat ctgggttttt gctcccgtct caacctagtt 120
   gaggtctaga gtgattaagc tggagacttc tgaggagaga gaaatgaact aaagataaat 180
   acaactgatt taattttagc catagcagaa cagaacaaag aagcaaccac atttcatcta 240
   atatcaagca cctactaaag gatgcattct gcaggccagc tgcatctgca tccaaaccaa 300
   agtcactctg gttgctcttt tgctttgata acttaagagt ttagaaacaa gcggtttcta 360
   aaaaagccaa gataacacaa taaggaccaa attttaatcc cacatagaca aagagattaa 420
   agtgggtttt cctgaattgc ttatgttatg aacaggttac cttgtcataa tttggccttc 480
   ggcttgggat tctaactgtt ttaggccacc agttatgaca ctgacttact aatagctttg 540
   gactttgaaa ctgtgtgagg gtcatatagc ctcagcagtt ttcttgtagc ctgtgattgc 600
   attgagatta tataattttt aaagacatgg cctttggacc tctgtctact agttaatctc 660
   ttccatctac cattcaaatg tgctatatac aactatcata tcagcttctt agcaagcact 720
   tttctggacc tctgtcacac ccaccaagat gtctagttat gcctttcatt tgagagtttc 780
   cctttgctgt tttttttttt ttgttttgtt ttgttttgtt ttgttttgtt ttgtttttga 840
   gactgagtct cgctctgttg cctaggctgg agtgcagtgg cgtgatctcg gctcactgca 900
   atctccccct cctgggttaa agtgattttc ctgcctcagc ctccctagta gctgggatta 960
   caggcgcatg ccaccacacc tagctaattt ttgtattagt agagatcggg tttcaccatg 1020
   ttggccaggc tggtctcgaa cacctgacct caagttaatc cacccacctt ggcttcccta 1080
   agtgctgggt tttacaggca tgagccacca cgcccagcct ccctttgcat gttttttaaa 1140
   aaggcattaa gcatcttgca catgttcttt agtttcagtt tgcatgagtc aacctgtgtg 1200
   catcattttc cctttcacta tttcttgtct ttgctggtga aattttaaag cttcagttta 1260
   aaaa 1264
<210> 27
   <211> 1795
   <212> DNA
   <213> Homo sapiens
<220>
   <223> nbla21908
<400> 27
   acagttgttg caaagtgctc agcactaagg gagccagcgc acagcacagc caggaaggcg 60
   agcgagccca gccagcccag ccagcccagc cagcccggag gtaaggaaac ggtgctcggg 120
   cagcagctct gctcggaaag aaggcacggc ttctgctctt aagccaagtg gtcttttcaa 180
   aggccttctt taaaatcgct cagatgggtg cttttgagtc tgcgggtctg gtttctgaaa 240
   acccaggctg cacgcagctg cattgcaaag tgcttttgct aattcggagg gcttcacctt 300
   tctcttcaga aagcaaaggg cagttttctt aagtcacttg cagaaggaaa tttccatgtg 360
   tatttaggaa tctggtgttt atttgctgtg tggctattta agctccagta agcaggggaa 420
   ctttgcaaga acacagacta tccattctgc ctgaccaatt tggcatgggg attagcttgg 480
   cacccactgt ttacctgttt tgcttctagt atatcagttt ggaaacagat aaaattggca 540
   gtaaatacgt aattccagaa tgatgaacac tttattaaga ggcatcctta aatggagcag 600
   aaaactgctg agaatctttg tgagtccaag atgtatttga attcagtact ttgggggatt 660
   taccagagtc tgtaagtccg gaagctataa acgtgaatgt taaacacagc ccggtcttct 720
   cttctcttga tggcacgctt gctaatctaa tttgagtatt gttctcttag aaggtgttaa 780
   gtccaacttc aattggggtt gggggaagca cacacacaaa tctactattt tgcaatttaa 840
   atatactctt caggtaaaat gtggattttg ttcaattttg ttggcatgtg caaagattca 900
   aggagtgact gagagaactt tggagtgagg tcagggatgg gtggttagcc aagacttgta 960
   acttccaggg agaatgagaa gttgtaaaag tcagactggc tgtctctctt tctctcttcc 1020
   tctttctttc tttctttcct tttgctcaca acaggattac ttagtgtttc aaaagtggga 1080
   gagagcctcc ttaaatggtt tacagccctt tgaatgtatt tggtgcagtg acatcccctg 1140
   aaacttcagt ctgcaaagtc tcaacatggt aactttgttc ttttcttttt taaaggcaga 1200
   tgctgctttt agtgtccctt tatttattcc aggaaaaatg tggacatcag ctaggcacgc 1260
   ctagcaaaga aagtggaggc tgctggtttc tgtgctttaa ctttccatag attttaaatg 1320
   gataaactgc ttgcccttct ttcatcagaa tatgagcttt ccccagatgg aaagtctttt 1380
   ctaaagcaaa gttgcacatg ggagctctag cttggaaaca atttgctctt ttttccccag 1440
   tctctgccat aaacacttga atgtgcacac aactgcagag cttaatgcca caacctccag 1500
   gagattgggg ggaggggaaa gctgcccagg atgggggtgg gaaagcgaag gaagatggag 1560
   aaatggctgc agtttgctgc ccatcagctt ttctctttta aggggcagac attgcagacg 1620
   tagttttaaa aaagttccat aaagcatcgc caaggcagca tgcctgtgcg acacacgcag 1680
   ggctttgggg gtgtgttttc cccgtattaa cagcaagtcg ttgaagcgtt gagaaggtat 1740
   tatgatttct aatcaggccc agaacaggcc aagtataggc tttctgaatg aaaaa 1795
<210> 28
   <211> 1620
   <212> DNA
   <213> Homo sapiens
<220>
   <223> nbla22027
<400> 28
   ttgatgcata aatggttgga cagatggggg ggtgggtgga tgagtgggtc gatggatgga 60
   tggattaata ggtgagaaat atttggatgg atgaataaat gttttgatgc atagatggat 120
   ggacagatgg atgaatggac aggtggatgg atagataaat ggataagtgg atggatgggt 180
   agaaggatgc atggaaggat ggatggatga acagatggat ggatggatgg atagaaaaag 240
   aaatagagaa ttaaggacca ctgggggagg gatggattgg tgggtgactg gatcagttgg 300
   tggatggatc ttggtggact gcctgtctcc ttcaacccct atccatccaa ccacaatctc 360
   tttgctgttt tccctttcaa gtctgccctc ctctgaccat tcccctcctg ttcctcttgg 420
   gcatggcctt ctccctcata gtccctgatc tccatccttc ctgtttcggt tcatccccca 480
   cactgttctt tcaaacatga aagtctggct gtgtctccct cttgaacact ccatggctcc 540
   ccactacccc catcctgata aaacccaagc cttcctccca gacattgggg ccccttccca 600
   tctggtccct gctgactagt ccaaccacca ctcactcttc tcttcatgca tcagatatca 660
   tagccccatc aaaccaccca ggggtccctg tacaggctgt gggccctctt tcctatctgt 720
   ggaatgcctt gcccacctgt taagggaagg tgatctgtgg gtgggggcga gctgggccct 780
   ctctcagacc tgcccctcgt ccccagcctg accctctttg ccaaaatctg tgagaagact 840
   gtgctgaagc gagtgctgaa ggagctgtgg aagctggtta tgaacaccat ggagaaaacc 900
   atcgtcctgc cgcccctcac tgaccagacg gtgagacctg cagggggccc gaggggacat 960
   ttaggccacc tccctggcga gagcccagaa aacttggtgc ctagaggctg ggggtaagaa 1020
   caaaggcatc cggtctcaga gaggtcatcc aggctcaagg gccattcaag ggtcatggaa 1080
   gccaccagag gtcagtgggg ggccattcag aggtcagaga gttcacacag gggttaaaga 1140
   tcatcgaaga gttaaagagg tcattcagag tccattgtat tttctctggg gtcaaagaca 1200
   tcaggtagag tcaagagacc actaaagtca tagaggtcac atgtaggtca aaatagcttt 1260
   caaaggtcag aggtcatcta gaaaacaggt caattttggg atcaaggtta tccttgagcc 1320
   acggaaggca tagacattgg ccaggcaccg tggctcacgc ctgcaatccc agcactttgg 1380
   gaggctcgag gcgggcagat tgcttgaggt caggagttcg agaccagcct gggcaacatg 1440
   gtgaaatctc gtctctacta aaaatacaaa aattagctgg gtgtgatcct gtgatcctgg 1500
   cttcttggga aactgaggca cgaaaactgt ttgaacctaa gaggtagagg ctgcagtgag 1560
   ctgagatggc gccactgcac actccagcct gggcaacaga acgagaccct ttctcaaaaa 1620
<210> 29
   <211> 1426
   <212> DNA
   <213> Homo sapiens
<220>
   <223> nbla22082
<400> 29
   gagggcccat gtgctgaaaa tccgaagtgc cgcggaaagt ggaggtgagg gccgcccgcc 60
   ctagaggtgc ccgtccgaga ggcaggtgcg ggaagagcct atccttttcc ctggccatgg 120
   ctcagtcgcc tccccagggt ttatttgcac cggaagtttg gagcgggtgg gtgctgaaga 180
   cagctaggcc ttggcgatgt ctgggatgag gctggtgggg gaagcctttg gagccgtgac 240
   ctgagagggc agaccttcga ccccactaca ttgcactgcg ccttcagaac atgcagggaa 300
   aaccccactg cgggacgctc accagcagca tctccagatt gtgaagggaa agaagggaag 360
   gatctcgggg gcatgcaagc tgctctgggc tggggtggtt cagacctgga ttgactgagg 420
   tgaaggggct ccttgcagca atcacacaga aggctcgggt cttaagattg gccctgctcc 480
   tagtcaagct gtatgaacca gggtagtcac tccggctttc agggccttga tttccttgtc 540
   tgtaaaaggg actttacgat gcatctggca acctcacctt cctcactggg caatgtgaag 600
   accaaatgcc ggcaatgaaa ttcccagcat taggtttgtc atatagtagt cctctctaag 660
   catttgttga atactcacag gaacacttag gccagtcagc attaattgaa aataacaggt 720
   ggggtttttt tttttgtttg tttttgtttc tttttccgaa aataacatca ggcctttata 780
   ctgagaagta taaagaagaa aaatgagcca gtatctcact gttcagataa accgttaata 840
   catattttta aatgcacatg gttagaaaat gcaaacgtta cgggaaggaa caaaatggaa 900
   ttaacagacc tcccaaacag ttcctctccc cttaaacaag tactttggtt tcttgtttcc 960
   tttccataaa tataactgtg ctggaatata tatttgtata tttaccccac aggataaata 1020
   atacattatt ttgcaccttg ctttgttaaa atatttaaaa taatttaaat gacacccaca 1080
   accctgtaaa tgtttatgga tgatgaaact gaaattcaaa agttaaattg ctggatgggc 1140
   gtggtggctc acacctgtaa tcccagtact ctgggaggcc aaggcagatg gatcacctga 1200
   ggtcaggagt tcgagaccag cctggccaac atggtgaaac cctgtctcta ctaaaaatac 1260
   aaaaaaaaat ttagcgggtc atggtggcac atgcctgtaa tcctagctat tcaggaggct 1320
   gaggcaggag aatcgcttga acccaagacg cagaggtcgt ggtgagctga gatcatgcca 1380
   ctgcactcca gcctgggcga cagaacaaga ctccgtctca gaaaaa                 1426
<210> 30
   <211> 2062
   <212> DNA
   <213> Homo sapiens
<220>
   <223> nbla23303
<400> 30
   gagcttgagc tgagatggac tggtcttcat gggcgcccaa ggcgctgggt gcagctttcc 60
   ccgagacccc cagatggaaa ggagggaagg aggaacccca cacactcgcc ttttgcgaga 120
   agatcggcgc gcaccccaga gtgccccaag cctttggaat ctgcctgctg agcggagcgc 180
   gcgagcgtgg tggacaggtc ccgaacttgg ccagcgggct ttcttggcaa cttgctttgc 240
   gcagttctcc atggaaccct ggacccactg tgctcccggc gccttgcctt tttttttctt 300
   tttctttctc tcactgtctc tttttaaatt tatgaactcg aaatgaagcg gaaagcagat 360
   atgcgcgtca gcatactttg gcggtagttc ttcatgtggg ggatggtcag cgggagatgg 420
   cacttcataa gatctgcggt ggtcacccca gtcatcatcc gacgtgttgc accagtctgt 480
   ggcacttcat aaggtctgca gtggtcaccc cagtcatcat ccgatgtgtt gcaccagttt 540
   gtggcacttc ctaagttctg cggtggtcac cccagtcatc atccgacgtg ttgcaccagt 600
   gtgtgttgct gtttgagccg tgctgccgac cccttccagg gcatctgcca cgggcacctc 660
   ctccagcccg tgcactaaga ctcaagagag tcgaagaacc agggaatcgt tgtaataaca 720
   agcattctga attgcatcgt actgtgtact agacctttta aaaatggaac tgtcggctgc 780
   ggctggaagg cgcaggcagg cgccctggag agaattcaca gggaggcaca ggacagaacg 840
   ctcccaggaa cgaggaagca cccccagaaa ggagcgctct atgggctcca ggcagccgag 900
   gaaacgcgaa cgtgagcccc gtgactgcac tcccacgtgc accaacgctg ccagtgtgag 960
   cagaagcgga gcccgcagag cgccaggctg cgccgggaga tgcatcacga tgaaaaactg 1020
   cgccagagca tggcgggaac tttccgagag ggcgtgttgt ttccaggcgg ttccaccttc 1080
   taatatgaaa cagtcttggt tgattttcct tgatactact ttatgctcgg cctggttgtt 1140
   ggcaagtagc tgcccgcgtc tgtacgcgcc cttgattagt ttccactgca tgtgttttaa 1200
   cacagtcctc ctttttccac gtttatttgg gccaaccctg tctgcaaaga tccagtttaa 1260
   tacagatttg agtctacgtg ctatagcctg gaaatgtact aaagacacta caacatattg 1320
   ctgaaagaat agaatcttta ttctgaatgc aaagcggaca cctagtaaaa aattctggaa 1380
   taataaaaca agcaaggctt atgtgctcag ttttggggac gcttcaattt aaaggcttag 1440
   tcattgtcac ggtgtaaggt ttacccattg cccccatcac acagatgtgg gattgttgag 1500
   agctgagtgt cctatgacct cttctgctgc ccaagaactt ggggtgggtg gtaactggag 1560
   aaatcaaagt gatcagctgc aaagaacgct tccattgctg gagcttggtt gtgcgggatt 1620
   ctccacggag gtcttaaggc agagacaaaa acaaggactt tgggaggctc ctgtgagcag 1680
   ccaaaagggt ttagagtcag gcagcctcag gttacaaatc cagtcctgca ggctaggagt 1740
   tgtgtaagct taaaaaagtg actgcacttc caggaacatc atttccctac ctgctcctcc 1800
   ttctgacggg ttttctgagg acaatggaat ccacactctg tgtcgaacac ttttctaatt 1860
   agcgatgtgc agacactgtt tattttacag gaataaaaat gccagaagaa cccaagtcat 1920
   attcatttaa agcagggtga caagtacacc aaaatctgaa aaatcatcac taaagaactt 1980
   atccatgtaa ccaaaaacca ttgaaataaa agtaaactat ggaaacaaaa tttaaaagta 2040
   ataaaattta aaagtccaaa aa                                           2062
<210> 31
   <211> 1592
   <212> DNA
   <213> Homo sapiens
<220>
   <223> nbla20264
<400> 31
   ggtccttgga gcttggaaga tttatgcata taggagagtg agatctctgg tagtagaagc 60
   ataattaatt agatgcccac taaataacct aaacttttca tcaaagaaat gaacaatgct 120
   atacatttga gttcccctta ctcttgaggg atgaagaaag gcttaagttg accgttgggc 180
   agatgttagc ttgtgtctga gatctgtttc tctaaaaagg ataaggtctt ctctaccctc 240
   tcccttaatc atcagacaca ggactggctt catgggcatg tgacatgtgc agtcacacaa 300
   ggccccattc ttagaagggc ctcacacttg gtttaatgag ctgctgccac catcttgtaa 360
   ttcttaatca agttttttaa agggactctg tattttcatt ttgcactagt ccctccaatt 420
   atatgtttgg acctgacaga catatgttgc tgctaggact ggtgagaaag gaaatgaggc 480
   catcccacta actgtagtat ttatagatgg cagatcctgg tggttgtgaa aagtgggggc 540
   tttgtgcact tgtaagagca tttgcagtgc agtacatggt aacactcatc catgaaataa 600
   tgaccagttt gaaatgcttt ctagtataaa cgctacagtg atgtcagctg aaacatgaat 660
   gttagaaggt atctgttcat tcttcgtaac ccctaacgtg taaacctggg atgttccctc 720
   acctagcttt taactgaaag gtggttatat tttgaatccc taaatcaaga agtcccagag 780
   cagctttatt atcaaacttg gaatccagca ttcatcactg tgtttcactc ttctatgttg 840
   gaatattaac agcactggag tcccataaat tatgtatttg ttgctgaatg ttgctgccag 900
   ctatgagtgg caaagcagtt ccttatgtag cttattttgg ttttacaaga tcattgatgt 960
   gtatcaagat ggctcaacaa atgaaatgta gttcaaatca tagagttacg agtctgtgca 1020
   actagattga tttttcttgc ccttgagtgt cacagtggtg gcactctata ctttaaaaag 1080
   tgtgaaataa caaccaggag agatagggaa aacccaattg gcttttaaaa aaatgaatac 1140
   atgtcaaaga ttttatatta ggcattaatt aataattaat taactggcaa agtaagtggt 1200
   tactgcagtc caaaggaaaa tccaaagagt agacacatac ataggcaatg gagaatgtga 1260
   aaatgaattt gttagcagac gcacagctgg cttctcccat gggcagggtg gagtgtggga 1320
   ttaggtgtgt cttaactgga caagatttgt ttgcagtaat atcagtattc tttaagagtt 1380
   gtaaatagat tagtaaaaat actaaaaggt gtagtcccct gtagaatcag atagcccaga 1440
   aaagtgtgct agacaacacc tgaagttccg ctgaaaagat acccagtgat cactttttgc 1500
   ccatttcaaa tctttctcag tttatctgac tgtgcttccc ccctcctccc ctgtgatcgt 1560
   aataatctca gtgattatcc ttcatttaaa aa                                 1592
<210> 32
   <211> 859
   <212> DNA
   <213> Homo sapiens
<220>
   <223> nbla20269
<400> 32
   aaaaaggagg ggcgtacgcg ggcaagatgg aggcgactac ggctggtgtg ggccggctag 60
   aggaagaggc gttgcggcga aaggaacggc tgaaggccct acgggagaaa accgggcgca 120
   aggacaagga agatggggag ccaaagacca agcatctcag agaagaggag gaagaaggcg 180
   agaagcacag ggaacttagg ctgcggaact atgtcccgga ggatgaggac ctgaagaaga 240
   ggagggtgcc ccaggccaaa ccggttgcag tggaggagaa ggtgaaggag cagctggagg 300
   ccgccaagcc cgagcccgtc atcgaggagg tggacctggc caacctcgct cctcggaagc 360
   ctgactggga cctcaagaga gatgtggcca agaagctgga gaaactaaaa aagcggactc 420
   agagggccat tgccgagctg atccgtgaaa ggctgaaagg ccaggaagac agcctagcct 480
   ctgcagtgga tgctgccacc gaacaaaaga cctgtgactc cgactgaggc atgccctgcc 540
   ccaccactcg cccatcaggc ctgtcctgca ggggatggtc ttgggcaggg atgggggcta 600
   ggcttgccat cacctccagt ttggcttctg agcagagact ccctgcccat caagtctgaa 660
   acccccatgg atgaggtcag ctccttgtct gctgggtggc ccctgccatt ctgaatggag 720
   gcagaaccag caacaactct gggcgtgcct gtgtctgcac atgtggatgt acatatgtct 780
   gtatatatgt atatattttg aactttctaa aaaaaaaatc tggaaataga aacaagtaaa 840
   cccctgtgtg tggcaaaaa                                              859
<210> 33
   <211> 1800
   <212> DNA
   <213> Homo sapiens
<220>
   <223> nbla20406
<400> 33
   gattttgagc ttgcattaga aaactgtcca actcacagaa atgcaagaaa atacctctgc 60
   cagacacttg tagagagagg aggacagtta gaagaagaag aaaagttttt aaatgctgaa 120
   agttactata agaaagcttt ggctttggat gagactttta aagatgcaga ggatgctttg 180
   cagaaacttc ataaatatat gcaggtgatt ccttatttcc tcttagaaat ttagtgatat 240
   ttgaaataat gcccaaactt aattttctcc tgaggaaaac tattctacat tacttaagta 300
   aggcattatg aaaagtttct ttttaggtat agtttttcct aattgggttt gacattgctt 360
   catagtgcct ctgtttttgt ccataatcga aagtaaagat agctgtgaga aaactattac 420
   ctaaatttgg tatgttgttt tgagaaatgt ccttataggg agctcacctg gtggttttta 480
   aattattgtt gctactataa ttgagctaat tataaaaacc tttttgagac atattttaaa 540
   ttgtcttttc ctgtaatact gatgatgatg ttttctcatg cattttcttc tgaattggac 600
   cattgctgct gtgtctgtga catctggtgc tgctcatccc catccacaaa ctggaaaatg 660
   atttcctatg taatcatgca tccaactggg ctgtgctatt tttttaaatg gtttgtattt 720
   gaacatggtg attcctcctt cacttcacct taacggaatg tctttatttg aattttattt 780
   gtaaaatgtg tcctgtttaa atttttcaat ctttaaaaat aatttttatg tacttttttt 840
   ttttttttaa cctttcttgc actctgggtc atgggtacca ctgcaatggc ttcccctttt 900
   tttatgggat accaactgca atatggtcct caatgctgtt ctggccattt caatgactaa 960
   tgccaaacat ctgtatgact aattttttta tgttaaaaaa atactgttta atgctggctc 1020
   tatggtgatt tggttttact aaattgggtt tctcgttggg ggtggtcttt tgaatactgg 1080
   gttttatata ttctgctatt tttaacgtgt ggtttttttc gatatctggg ttctaaaaga 1140
   aatctttgga attaagagaa aaacaagctg aaaaggaaga aaagcagaaa acaaagaaaa 1200
   tagaaacaag tgcagaaaag ttgcgtaagc tcttaaaaga agagaagagg taaactataa 1260
   tattcagtat ttttaaactt aaggcaacta ctgaattgaa cccaaagtgc catactggag 1320
   gtaaagtaaa taaaaatatg aaagtatttc aagtgccaat cagtgactgt taagaatctt 1380
   tagcaaatat gtgttccatg tattttctta ttaaagagat gaagtggaat ttaaggctag 1440
   aattctacaa aaaaagagta tcttagaatt aaaatataga ataagttact ttaattatgt 1500
   tttaggaaga aatattttag aactagagca gtggttctca actaggggtg gatttattca 1560
   cccggggaca tttgacaaga tgtggagaca tttttgattg ccataactga tagggtgcta 1620
   ctgcatctag tgtataatgg tcagggatgc tcttaaacat attttaaagt tggacgccat 1680
   gtggatgcta tgaatgaata caataaagct ttggaaatag acaaacaaaa cgtggaagct 1740
   ttggtagctc gtggagcatt atatgcgaca aaaggaagtt tgaacaaagc aatagaaaaa 1800

<210> 34
   <211> 1716
   <212> DNA
   <213> Homo sapiens
<220>
   <223> nbla20949
<400> 34
   gttgtccaag atggagggcg ctccaccggg gtcgctcgcc ctccggctcc tgctgttcgt 60
   ggcgctaccc gcctccggct ggctgacgac gggcgccccc gagccgccgc cgctgtccgg 120
   agccccacag gacggcatca gaattaatgt aactacactg aaagatgatg gggacatatc 180
   taaacagcag gttgttctta acataaccta tgagagtgga caggtgtatg taaatgactt 240
   acctgtaaat agtggtgtaa cccgaataag ctgtcagact ttgatagtga agaatgaaaa 300
   tcttgaaaat ttggaggaaa aagaatattt tggaattgtc agtgtaagga ttttagttca 360
   tgagtggcct atgacatctg gttccagttt gcaactaatt gtcattcaag aagaggtagt 420
   agagattgat ggaaaacaag ttcagcaaaa ggatgtcact gaaattgata ttttagttaa 480
   gaaccgggga gtactcagac attcaaacta taccctccct ttggaagaaa gcatgctcta 540
   ctctatttct cgagacagtg acattttatt tacccttcct aacctctcca aaaaagaaag 600
   tgttagttca ctgcaaacca ctagccagta tcttatcagg aatgtggaaa ccactgtaga 660
   tgaagatgtt ttacctggca agttacctga aactcctctc agagcagagc cgccatcttc 720
   atataaggta atgtgttagt ggatggaaaa gtttagaaaa gatctgtgta ggttctggag 780
   caacgttttc ccagtattct ttcagttttt gaacatcatg gtggttggaa ttacaggagc 840
   agctgtggta ataaccatct taaaggtgtt tttcccagtt tctgaataca aaggaattct 900
   tcagttggat aaagtggacg tcatacctgt gacagctatc aacttatatc cagatggtcc 960
   agagaaaaga gctgaaaacc ttgaagataa aacatgtatt taaaacgcca tctcatatca 1020
   tggactccga agtagcctgt tgcctccaaa tttgccactt gaatataatt ttctttaaat 1080
   cgttaagaat cagtttatac actagagaaa ttgctaaact ctaagactgc ctgaaaattg 1140
   acctttacag tgccaagtta aagtttacct tattctcggc cgggtgcagt ggctcatgcc 1200
   tgtaatccca ggactttggg aggccaatgc gggcggatca cgaggtcaga tcaagaccat 1260
   cctgccaaca tggtgaaacc ctgtctctac taaaaaaaat aaaaaaaatt agctgggtgt 1320
   ggcggtgcac gcctgtagtc ccagctactt gggaggctga ggcaggagaa ttgcttgaac 1380
   ccgggaggcg gaggctgcag tgagccaaga tcacgccact gcactccagc ctgggtgaca 1440
   gagcgagact ctgtttcaaa aaaaaaaagt tgaccttatt ctctaaaagg gctggctatt 1500
   catatgatga attgttaagg aaaacttaaa gtggaagaga acacatgtga agagactttg 1560
   aaattatcaa aagaaaaaaa aaagaccaga caaaatctca tgtgccaata acttttcaag 1620
   gtgcctttgt taaggaaatt atatccactt aattactata atatataaga ctttatgaaa 1680
   agcactttat aaaattctaa tttaaaaggt caaaaa 1716
<210> 35
   <211> 2442
   <212> DNA
   <213> Homo sapiens
<220>
   <223> nbla21251
<400> 35
   ctcctgagct ccatcctagg ggtttttaat ttaaccaaca gccatgttga caaaagccaa 60
   caataagcat gtcttattct agccctgatc ccaacactga aagcgaagta ctttataaag 120
   aagccagcaa ttatgagggt ttctttatgt tagtagggga aaaaatggta ataaaagtac 180
   cagtgtagca agtgaagacc aaatttatag cactgtgcat tagatagcaa aatcaggttc 240
   ttaacaatga aaagtaaacc tcaagtttct aaatccatat gcagatggtt aggctgtccc 300
   tctcttagca aatctctcag cctccttctt tcccaagtgc caaggatccc tggagtaaag 360
   ctctggggtc tgtgctctct ttctgtgagg ggaaggctgc ggccctattt gcccctctct 420
   agcaaacacc cccaccaccc tgccgcttcc tgtggttatt gagccagcta ggagttactc 480
   atggactcta acctggtttt agtcccatgt acatcgttgt tttaggtttc atactgaaga 540
   gccaatggtt tatgtggttt tattctgtct taaatataag tttcaaggaa gggaaaacaa 600
   aagtgataaa atgatagaac agtctagagg ccactgtaaa gtcaccgcca ctttacgtgt 660
   atgtcagtct tggtgttctt gtatgagtaa aatggatgta aaatcataaa atcacagtga 720
   atgtttcagg ctacactgga aaaagtatgc acttagaatt aaaggaaatt gtataattca 780
   ccaagatttc tttgtgtaga tcaggggttg gcaactatga cccacaggct aagactggtc 840
   agcggtctgg tttttcacag ccatgagcta agttaccttt ttaaagggtt atataagtaa 900
   ttacatcata tttttgattt tgcctttggc ccacaccaca taaaatattc aatacctggc 960
   cttttttttt tttgagacag agtctcgctc tgtcacccag gatagagtgc actggtgcga 1020
   ttttggctca ctgcaacctc ttcctcctgg gttcaagcaa ttctccctgc ctcagcctct 1080
   aagtagctgg gactacaggc acccactacc atgccttgct aatttttgta tttttaatag 1140
   agatggggtt tcaccatgtt ggccaggctg gtctcgaact cctgacctca ggggatccgc 1200
   ccgccttggc ctcccaaact gctgggatta caggtgagcc actgcgccca gccaatacct 1260
   ggccttttaa gaagtttgct gactcctggt atggatgaca gaaaatggaa taacgttttg 1320
   tttctccagt ctaggaaaag caagtcaggt agtggataga ctgactggcg tccggggagc 1380
   ccagggtatg tgagggccac gtggatggaa gcaaatgcct cctgcatagc ccttggctct 1440
   ttgtcccact tgggaggagt ccatggatgt aatatttaca aaacaatttt ttccttacca 1500
   tttgcagaaa gcattgcata tatttccttt tagctcagga aactggcatg ccccaccctc 1560
   tgctactcca tcagatgtaa atacaatgac tataagccgt acaactcccc tctcttagaa 1620
   acctcagcag gaccacagag caagggagtc aaagctttct taattctctc cagtaaatga 1680
   ctcaactaat ttgatttttt taattaagtc aaaatatcaa gagaaaaatt gctactaaaa 1740
   cttacatttt gatccacact gatgtgcaac acaaaatgaa agttttcacc tccattccat 1800
   tttttaaaaa ttcacggtcc acactgaaac ttgctgggtt ttagcaggag acaaaggtgt 1860
   cacccacgct gtcctcatcc tgctctctct gtcccagtga cgctccagca tatgatcact 1920
   gcagccggtc cctggcccgt gccgattctg ccacctccca gccacacaca tttgcagacc 1980
   cacaagaaga actgtagcct tgataatttc agttcaggct ggaaaaatgc catgcaataa 2040
   tctggtttgc tttcagtaag taggcaacaa gtgaaaactg tataattttc atcacctatt 2100
   ctgctgttct atctaaaatg agtgtacctg tggtttgtga actgggccct tgtttgtgcc 2160
   agatccttca aagatgttcc ctgtcaggac acctgtggcc ctgcccctcc tcagacacct 2220
   tcccactggc attcacgttc cttatatgca gtgttagcca tctttggcct acgtggactt 2280
   tttttgtaaa ttacaccatt tccagacatt aaacttttta tattatgaaa tttaccatgt 2340
   aaaaagaact tcatattttt attgagattg ctaaggcact tggccttcct cctttgtgat 2400
   tttcagtgtc tattaaagca tgagttccct cagttttaaa aa                    2442
<210> 36
   <211> 1731
   <212> DNA
   <213> Homo sapiens
<220>
   <223> nbla21334
<400> 36
   attaaattca acaataaatt ttatatgaat gatttggtaa aatgaatatt ttaaaaaccc 60
   accaaaaaag taatagggaa ctctcatata tgctcacaca caagataaaa tgcagacagt 120
   ttttaaaata aaaagccaat accagcatgt tctaatatca tagagcagat taaatgaatt 180
   ctagcaaagt gcatttttga tttgaaattt ccaaaagctg ctagcatact tcaggtgcac 240
   acttatattg gctgggttat tcccttttaa tagctatcac acacacgaac acatttaaaa 300
   taacatatcc ataaagtgac attttggttc atgtttctta ggtttttgac acaagtagca 360
   agagaacatt gaactctact ttgcagagca cagaatatcc ttcctctttg ctaataaagt 420
   gagcactcac ataagttaaa cccaccagag ttatacattt ttcactaaaa aacttgcatg 480
   aaatgtctgc ttgaagagga gacagtaaat taatcattaa tttagatggt atttggaaac 540
   tctagttact gtatttcctc tgttcatttt cataataaag gatacctgac tatcgcagct 600
   aaagagaaat gatccctaga agtttttaga gataaacatg ggaattgctg ttatatatgt 660
   tatatatgtg tgtatatata ttacatctgt atatatgaat accactaaca taaataggct 720
   ggtatggaag caaatataaa cttttgcatg aaaaaagttc aggaaattga aggcatggat 780
   ttcaaaatag tgattttttt aatcttgcaa aaacttggaa ttatgcgaat ctttttgagg 840
   agctctaatg tagaatttgt ttgtttttat attttttaag ttctcataat cataatttct 900
   tgaaatactt atataactat gaatttttgc aatttaattc ttaaaagatt attggtttgt 960
   cttcctaagt gaaggatata gaataaatgc ttttaacaat catatttgaa gttgaattcc 1020
   aaacacaatc tagcaatatc atactgtgac cttcactgct taccattctt acttctcaca 1080
   ggagtaaaat caagctggag ccatcaagaa tgcagctctg gtgtttttta accagccaga 1140
   ggctcgtgcc accactttta cccaggttat ccaagcaagt tgtacatgta caatcacgtt 1200
   ctaaatgaat tttgactggc ctgcatgcta ctcagctatg ttccttcccc tgccatggca 1260
   aggaagtgct agacttgccc agctgctctc tgctgaatcg tgtgacacat cacagcatgg 1320
   tcaggcgaga tgggcaatcc caacatcata tttaattctg ctaatgagtt ttctaattta 1380
   gtctttagcc ttttaaaacc aattgcatgc tctataggat ttgtaatatc tattttaaaa 1440
   catgatagga atgtttatgg ttcaatatag tcagggatgt aggagggcat gcattttttt 1500
   gtttctctgc ttttatttca ttaaaataag accacaactt tttattgttg attcagcctt 1560
   tataagtaaa ttgtattacc aaaataagcc tcacagggtt tttttctgat agtactgcca 1620
   ctttcagatc attatattca gatctatgaa tataattttc agcctatcca attcatgtgc 1680
   tccagatgaa aatgtttgct ttcatgtttt gggggaaagg ttctgtaaaa a          1731
<210> 37
   <211> 3077
   <212> DNA
   <213> Homo sapiens
<220>
   <223> nbla21356
<400> 37
   gactcggtga aaaaaatgca ttttcccctg gctgtttgaa aatttactta tttgcagata 60
   agtctagatt tagtcttgga gatcaaagtc ttttatattt taaaaactta ttctttatat 120
   tgatcaaaca tggcatatgt tagagaacca cttcttctgt catgtttatg tattttggaa 180
   ttaagttgtt tgcattcact ttcaaaatct gcccatttct gtttatgtgc acttaccaca 240
   gatgtgtcgg gactttgcct caggggagag gtactttagc acctgtgtca ctgaggagat 300
   ggagtggttg acaagtactg ttgcgctgtg taacttgggg tttggccctg tggacaatat 360
   attagcagaa tgataccaca caaaagtatt acaggattaa ggcatgtaac ttctatggta 420
   gtccttatgt atcagcgtat acccaagttc agaaaccaca ggtgcatttt tagaccttta 480
   cttagagaac taaaggcagt tccaaccatc agcccatatg gcgggattaa tgcatgaaaa 540
   ccctcagagg gtgttgggac atcctacttc cctgtcctca cccagtggaa ctctggtgtg 600
   tgccttgagg ataaggaagt agagtggaaa ctcatcctat cattgagtat tctcaatatt 660
   ttggccttcc ctctggaatt atgagaaatt taacaaagtc tcaggaacct ttagaatcca 720
   ttgtccaaca ctgctagaaa aactgtagga ggtacatgga gaattcctat agttcttagg 780
   taagtgcaag acatggcaca gggatcccta tccacataaa ggggaatctg gatgctgcac 840
   acctcaattc tgagaaatcc ctgactgaac ttggaattat gacagtaaag ttttcgtcct 900
   ttagttttct agagcagctc acagaaattt taaaaagtaa aacaaggcca ggcgcagtgg 960
   ctcatgcctg taatcccagc tctttgggag gctgaggcgg gcagatcacg aggtgaggag 1020
   atcgagacca tcctggctaa cagggtgaaa ccccgtctct actgaaaata caaaaaatta 1080
   gctgggcatg gtggcgggcg cctgtagtcc cagatgctca ggaggctgag gcaggagaat 1140
   cgcttgaacc tgggaggcag aagattacag taagccaaga tcgccccact gcactccagc 1200
   ctgggcgaca gagtgagact ccgtctcaaa aaaaaaaaaa aaaaaaaaag taaaacaaaa 1260
   ataaagtcta tgcccattaa gacgtcttct aattcagttg tgattgtctg ctcctactta 1320
   aaaaaatatt taagcttgat gtttaattat tccctttcag caaatttgga tcagaaaatt 1380
   aaagtatgtg acaagatcag gtcaccttga atttccacac aatctcaaga cactgaatag 1440
   caaaaaagta acattacata gtaatgatta ggatatttcc ttagactttg ctggatcttt 1500
   ggtcttaagg taacatgtaa aagtagtgaa gcctttcctt tcatggccct gtgcaatgta 1560
   acggttttct gcctcctctt cagctggaag cgttagtggt agtatggcca cagaatatat 1620
   gtacactggc gatgctgacc atgcctccca ggtaccctgg ctctgggttc cttgacctag 1680
   ggaacaagat tggatgaggc agatctttga gcccatgtga ctatagaatt tgctgatgat 1740
   ataattttac aataacaatg gataggaatt ttacctctct ttttattagt ttaatattat 1800
   ttaatattat gtacataagt gttcactcgc ctaattaaaa acattgagta aaccaagttt 1860
   ttatatagac tacccttgcc atatgatgct ctttttctct aataatatgc agtttaaatc 1920
   ctgaggaatc aatgcccagc atttcaccac atctgaactc tgtgtgggca ttcttcactc 1980
   gcctacaagg ggtaaacaag gctaccagaa cttgaatttg acttataggg agctacccag 2040
   gaaggggaaa gcccttggga ctttttccaa aacaatcttc tatttgaact gttcatcagc 2100
   caaagtagtc cactgaggtg acaaagcttt cagaaataca aagatgggaa gataaaggta 2160
   acactggccc acttggggct ttgacattgg attgggtgga ctgaataaac acagcctagg 2220
   tggcctgggc ttgagcctca cttacttctc cttgatacat agttcctggt ctaccttctg 2280
   accctttttc taaaatagcc agtgtctatt tcactaggcc atttacttac aagttcccag 2340
   cttttaggga aaaaagaggg aggggggagc atctagtttt gaattagata tacatcttag 2400
   aagtaatgag ctattggcag ctgttaaatc agattcagcc acaaaccaga attctttctt 2460
   gttgaacaag accaatgagt tagatgactt taataattcc acttttctct ccctctcttc 2520
   tcctcttcct gaaatcagag agatgagaaa ctactctttg aaatacctcc agaggcgttt 2580
   tattgtgttc ctttcccctc caagcagctc cctttataca attttgctca ggcaaccaag 2640
   gacagagtat cggcagaaac atggagtgct tttgtatagg ccacctgtac ataaaagtgt 2700
   aattatttat ttaattttcc catttgtatc atattaaagc tttgtacagt gttttaagtt 2760
   ctgttttaaa attattttgt attttatttt tataacctag taataaaata ttcattccgc 2820
   atgcaaaatc tagttctgtt tgtgtgatgg tctggatttc aaaagtggaa aatatttttc 2880
   taatttaata aagttattga atacaccaga tgttacaaga tcaacgggga gcagatagtg 2940
   ttactgtaaa tgcagtacca catctagaag ttccctagaa aaagcagccc aggactgaat 3000
   agaagctagg tgttaagtgt ccctgcagtt aggagatgtt ttcctgtaat aaaattaaaa 3060
   tattaaaagc tcaaaaa 3077
<210> 38
   <211> 2043
   <212> DNA
   <213> Homo sapiens
<220>
   <223> nbla21418
<400> 38
   gcaagtaggg gcaaaaagac acaagcaaca taagtaagca tgtgtgcagt gtgttatgtg 60
   ataagtatta cataacaggg atgtgtgtca cagcggggaa gggggaagag ggtgagctgg 120
   gatatgtttg taattcagat gaggttcttg gggaagatct cactaaggag atgacattga 180
   ggaaagacct gaaggaggtg aggcagcaag ccatgaggaa gaacattcta ggcagaagga 240
   agaaagcaag tgcaaagact tcacctcgag ggaggagcgt ttgaatgatt ttgcagaaaa 300
   acagagaggc cggtatgact ggacagtctg agtaaagaga agaatgagat gggatggatt 360
   cagttgcaag tgattgaaat gaataacaag cattcatcga tccaaggatt caatgaccct 420
   aagtattctt aggtagaaag cagggtgaca ggcagggtga aataaaatct tcctctattc 480
   tgtagagctg tgacttaacc tttcagtctt gtgaaaatat gtatttattg gtactgctgg 540
   acagttttcc tgctggctgt ggagagagtc ttggtgaaca gagaggcctg cagcaaaaga 600
   gttaagagat actttctact ctagatgaat cagacagaaa tgagtcattt tttaaattac 660
   agaggtggac accactttac ttagcaactg tccttttgaa aattagcttt aatttttttt 720
   atttcagtca taatcacgga actataatta ctggaaagga ccttgtttgt catctaaccc 780
   agctctcatt ttatagtttc ttaagaaact aaggtatgaa gtgtagctga aatactatta 840
   caaataaatc tattcactat ttaaaacagt attctcataa ggaatctttt gaaaaatata 900
   tataatccct taaatttata gtttcaaaaa tgttttaaaa tatttatgaa gtccctacta 960
   tgtatttgac actattctgg catctgggaa ttcagccaca attaataagg tagatttcat 1020
   ccctactcag tcagcattta cattgtgctg tgaggtggga gtagggctag ggagagctgg 1080
   gagtagtatg tatagatgac aaaccagtat gttaatatat ggacaaaata atttcagaga 1140
   aagataagtg atataaagac aatcaaagca cagtgatgaa tcagaagaat tagaaagtac 1200
   cagagctgtg gccatgcagt gccgctctga gaaggtgaac tttgagcaga gaacagatcc 1260
   accttcagga gttagtggta tgggaatggc atggggaggg gaccaggttt tccagtcaga 1320
   gggtacagcc agcacaaagg cccgagcttg ctgtgttcaa agaacagaca aaaaaaccgc 1380
   atggttgaaa tgtaatggag gtgtgatatg taagatgggt gtggagaggt gcaaggtggc 1440
   cagcccacat ggggcctctt aaagactgtg gttagacagg tctacgaaaa tgtcagaaag 1500
   ctttcaacag ggaaatgttg acatcaggct tcatttttca gaagatctgg cttctgtgtg 1560
   gagaatggac tatgttggga caaaagacga agtgaggaga ttagatagat gccaatttta 1620
   ccagctccgg caagagaggt tgaggcttat gcttggttag cactggaagt gaagaagtag 1680
   gagcagactg gattcttttc tatcagattt ggagtaccat tagccgtata aatcattgtg 1740
   gggcggggaa tgcctggtgc cgtggctcgt gcctgtaatc ccagcacttt gggaggccaa 1800
   ggttgggagc attgcatgag gccaggagtt ccaaactagt ctgggcaaca cagcaagacc 1860
   ctgtttctac aaaaaaataa aattaaaaat taggtagacg tggtcacatg caccagtagt 1920
   cccagctact gggaaggcta aggctggagg atcttttgag cccaggattt tgaagctgca 1980
   ctgagccgtg atctcaccac ggcactccag cctgtgcaac acagtgagac cctgtctcaa 2040
   aaa                                                               2043
<210> 39
   <211> 1181
   <212> DNA
   <213> Homo sapiens
<220>
   <223> nbla21480
<400> 39
   atatgcaacg gtcagttcct ttagatatat tttactagct ttcctttttg gtcatgctgg 60
   aaagaattcc agttcttttg ggggggtggg agcagaacaa aatgaaaata actactattt 120
   agatttaaaa tgtttttacc atttcctgaa tccttggact gttttctgtt tggttgctcc 180
   acactatagg attcagtttg agtatttggg taccatccat ccctcccaga aggtaagctg 240
   gttgatgcaa cttttgtgga taataagtgg ctctgttctg gttgatggtg tttctgagaa 300
   gtatagacag agaagctgtc taaacataag gaacaaagtc agtatcagtg ttacatgaac 360
   tgtgaacatc atctggaagc caatgaatgg atccctattg tgaagtgagg cgctcaaaga 420
   gatgtatcat actttgatct taagtaaatg tgctggttcg ttccacattg ctctgccttt 480
   ggagcagtct gtgatgaagg tgacctaaaa agtgagcacc attagaactt gattgctgtc 540
   ccaaaccatc atatctttaa aaatcctatg atcttcttag ttatgcaggt aattgaatac 600
   cttgttaaat accaggaatg taaatggcca gaaacctaac agtgtaaaag agtgaaattt 660
   attagtagtt cctctcataa gactattttg taaagaaata actagagata tgtttgatat 720
   ttatagcaca ttcattgcaa ccattaaaaa tatcaaaatt gattatgtgg gaaaatgttt 780
   aatgggaatt gctcagtttt tttccataaa ggattataga atatgttcaa tatgatccca 840
   ctttttgaaa atactcagga aaaaaggatg tatacccagc tgggcacagt gactcacacc 900
   tgtaatccta acactttggg aggctcagtg ggaggatcac ttgaggccag gagtttgaga 960
   ccagcctggt caacatagtg ggactacatc tctaccaaaa aaaaaaaaat atatatatat 1020
   atattagctg ggcatggtgg tgcatacctg tagtcccagc tactcaggag atctgaggtg 1080
   agaggatttc ttgagcccag gagttggagg ctgcagtgag tgaggattgc accacttcac 1140
   tccagcctgg acgacagagc aagatcctgt ctcaggaaaa a 1181
<210> 40
   <211> 2312
   <212> DNA
   <213> Homo sapiens
<220>
   <223> nbla21509
<400> 40
   aacgatgacc tgaccaccaa gccaccatag gaaggagcca cggagctgcc tcctaggcca 60
   ggatccagaa cgagccaagg gaaggccgag atatccccag ggtacctctt ctcagcagca 120
   caaagaggag tttattttca aagacagtgg aagctggaaa agataaaagc cttgaaattg 180
   aaatgcaaac aggagagccc tgccagaaca aggctgtgtg tctttcaaac cccatctgag 240
   aaagagaggc tacctccaca gagctgcgtc agggcagggt ctggtcacct cctgggacaa 300
   acaggaggaa gctcgcatgg ggaccaccac ctagagtggc agcccaggcc tgggtccccg 360
   ccaccgaagg gtccgcagag cactcctggg catcctcagg tgcatgccaa gatttcagaa 420
   agcgttacag aagtgacgca tccttcacta cagccaagat acggaaataa tgtaactgtc 480
   tgttgatgga caaatagata aagaaaattg atgtatttac acaatggaat actttttggc 540
   catgaagaag aaggaaagtc tcccatttgt gccaacctcg atgaacccag aggacaggat 600
   gctaaatgaa atgacccagg cacaaaaaga tgcatatcac atggtcttcc ttatacatgg 660
   aactgaaaaa agctgaactc acagaagcag ggggtagact ggtggttgcc aggtgctggg 720
   agaaatgggg agatgttgtc aaagcatgca aacctccagt tgtaagctgg taagttctgg 780
   ggatctagca tggtgattat agctaatagt actgcagtgt ttacttgaga cttgctgaga 840
   gggtggacag taagtgtcct caccacacac atgcagaggg taaccatgct gggtgatgga 900
   tgtgttcatt agcttgactc agtagttatc ccgtcacaat gtctatgtct attgaatcat 960
   cacttgtaca tcttgaacat acagtttctg tgtgtcaatc atacctcagt aagctgcggg 1020
   ggagtgacac attcaccact ggccatcagt aagactggac aggaccacca aggcagacat 1080
   aggggggcta gaaacccaaa agtgcagatg gtgaccctac ttaccacata cagataacag 1140
   agactagaag aacaatttga tcctcttcat gatgcacttt ttttggaaga caagtctttt 1200
   caaagagaaa gatgacaata ataacgaaaa cgccccagag gacacaaatt tggaactacg 1260
   ggcctcaagg aagccacaac acctggtatt ctcagcattt cttggtccct gacagacctc 1320
   tttgaccaac tgcttcaaac tgacactttc tctttctgtc acctcagata aatcatttca 1380
   ccgccctaaa atgcaggctt cttcatttgc agaatgagag agggagactc tgtgcactcc 1440
   ttctgtgcct cgcctgtttc tcctagggat cctcaacacc cttcagcttg tggacagcag 1500
   cacacgagga cactgagcat tctgtttgag tccctctagt ggctgctgaa tggcgtagtg 1560
   actcatgtgg gcttagcgag ggcaggagct gtctcacggg agactgcccc ccacccgcct 1620
   tccacaaatg ggggagaagc aggaggcagc agcaggcatg tgcgtggtct atcacggccc 1680
   ttttaaaaac tgctgttaca gaaaatgtca aactgcacag gaatagagag gaggagcgtg 1740
   aaccagcgtg tgcccatcag ccagcttcag ccactgtccc ctctcagcca agcctccttc 1800
   cctcggcagc tgcccatgct cacacccttt atgctccact catattattt ttgaatcaaa 1860
   ccacagacat attaccattt catccgtgaa tgtttcagtg tacatctctc aaagatagga 1920
   tgactcattt ttataaatat aactataata ccattgtcac acctaaaaaa cttcacaatt 1980
   tattatgtta catttaccca ctcatgtccc taaggagcgg tcacacagct ttcatgagtg 2040
   aacacaacct cttctcattg ggaacatgag gagggaaggg gctgtgaaca cctaaagtga 2100
   gcagacacgc tgaaccaaag cttggatttt cttccgtgac aacagctggg tctctgcgct 2160
   ttgaacacac tcgtgatcag cagaggaaag tcaagttcag catgtctggc ttcatacttg 2220
   tggagaggag gtggggtaac aataatgatg ataatgctat taatagcaaa ggtggaggaa 2280
   ttaataaatg accactgtgc caggcgcaaa aa                               2312
<210> 41
   <211> 2764
   <212> DNA
   <213> Homo sapiens
<220>
   <223> nbla21527
<400> 41
   agtaaagaat ctaaagtagt aaattttatt aatatgacaa gctgaaaaat aattataagc 60
   tttattacta atttgcttga aaaagcaaac aatgaaatga ctattgatta tgatcttaag 120
   agatgagtat tgttttttct ctaggattta taaagatgca tcagagttca tctatagaag 180
   gacaggtagt gtttgggaag catctataat tctctttgtg aaacatcagt aagtctattg 240
   tagtttaaga aagtttcata ttactattat tttagttttt atctctaaaa ttctatgaaa 300
   cattttgaag tataaaataa atatttttaa aagaaagaga acagaagtag cttaaatgat 360
   catattttac tcttaatgca cttttaactt tctcaatact atatttctct ctccatctgg 420
   ggtacggtta aaaaagagcc ttcctaacac ctcaggaggg aaagggcaac acagggcatt 480
   ggactcccca tggaaatgaa agagtagctt cagcatttgt aggatgatta ggatgagact 540
   gtggggttga ctgaagaatc atcaattaga gagggctggt aaaacaaact tctagaaaga 600
   tttgggttaa ctttaaacca ttgtaacaat tatctaatca acgtgatgtt tttctagcga 660
   ttaaaatcaa gtggaaaaat ataactatca aatttcaaat tatttcagag tcatgcatat 720
   tgatcatcag cccatatttt caatctgctg gtgcttgttt tcaaccaaga tttaccatgg 780
   ggctaaccat gatgtcactt gctattagtt aacctctgta cttctttact tatagttgtt 840
   ttaaacaagc aaaagctcat agagtgattt aaattatatt ttaatgatgg aaattccaag 900
   agctctttca catactgtaa ttatctgcca taaagaagag taccccgttg gtgctctggg 960
   cttgcatccc aacaccacca cttactggct gtgtaatctt gggcaaatta tttaactctg 1020
   gttttccttt atctgtaaca agggcatgta atagttctac tcatttggtt gttatgaggt 1080
   ttctgcgcat tcatctacat aaagtgctga gaatcagacc aagcacatag aagtaccatg 1140
   aaagtgttca ttatggatga cggtgatgtc ggagtgacat tgtatagtta taagagttgc 1200
   tattatggct acataatatc cttcacaatc tttcaagtat ttctaacaat gttgtgccaa 1260
   aatatttgct aaacaaaact taattcactt ttgttgttga tgttgttgta tgtttctcgt 1320
   gtcctgtgcc actgagaagc aagtcaaagg aatggagcca agtaattgct tttaatggct 1380
   cagagatgag ataatggatc cagtcaatgt aaccacaggc agtctaaagc cagggtgtac 1440
   accacaggcg tgggtgccaa tatcagtgct gagacagaga tagaagggag agcgcaacaa 1500
   atgtttaaac agcaggctca gcaaggctca acagagaaac aaaatgtttc tagaaattac 1560
   aaaatcagag actccatcac ttggcccata catgtcaata gagtgtttga tttaattcag 1620
   aaataatttc caactatgct tttctctgca ggttaatgct agtaagaact actccatggc 1680
   taatttgttc ttcagagtaa actgaactaa tactttccaa gtgcaagctg cctcaagttg 1740
   ataaatgcct aaatttccaa aatactacaa ccaaaagcaa agttttccag ttctccagat 1800
   acaatttttt tatagatacc tcaacatgca caaaactttt ctttgttgct gttgtttttt 1860
   gagacagggt ctcgctctgt cacccgggcc agagtgtaat gatgtgaaca cagctcactg 1920
   cagcctcaac ctcctgggct caagcagtcc tccagcctca gcccccaact agctggtact 1980
   acaggcctgc accactattc ctagccaatt tttgtatttt ttatagagac ggggtcttac 2040
   tgtgttgccc aggctggtgt tgaactcctg ggttcaagca gtccaacttc cttggtctcc 2100
   caaagtgcta ggaatacagg catgaccacc atgcctggcc acagaaaact cttatataaa 2160
   aatttccaac aagtatgaaa gagtgtttaa atactctcta actcttcatt tactatttaa 2220
   aataacaaaa ttgtaacttg aaagttggat aaaaaaactc aaatgagaaa taatgtctca 2280
   acaaccgttt cttactatga aagaaaattc aatatgatct tttcacacca tataagacct 2340
   tattttgccc ttgtttataa cccactttct ttggggggcc acatgaataa acatatttga 2400
   catatatcca tagtctgaat taggacattt ctattcttgc ttgaagaatt tgatgtttag 2460
   aaaaatttct cagcactggc caggcacggt ggctcatgcc tgtaatccca gcactttagg 2520
   aggccgaggc aggcagatca gctgaggtca ggagtttgag accagcccaa ccaacatgga 2580
   gaaaccctgt ctctactaaa aatacaaaat tagccaggca tggtggcaca tgcctgtaat 2640
   cccagctact caggaggctg aggcaggaga atcgcttgaa cccaggaggc agaggttgca 2700
   gtgagccgag ttcgtgccat tgcactctag cttgggcaag aagagtgaaa ctccatctca 2760
   aaaa                                                              2764
<210> 42
   <211> 2141
   <212> DNA
   <213> Homo sapiens
<220>
   <223> nbla21551
<400> 42
   catatgaaaa aaccaaagtg ctttatttaa tcacccggtc tgcggattgt gttgaatcaa 60
   ggtgtcagtg attctaggtg gttctgtctc cccctaaact gagacagagc agatacttca 120
   ggaaaacgtg gaagttggtc cgtacttcta caatcctact ggcccagcct gacccccatg 180
   tgacagcttt gagagttttc atgcagttag agacaaacac aggtcaatga caacaactac 240
   agcatgtgat gtgtgcttta tgatctaagc actttcagag cctttcaaaa actcagggtc 300
   tgtgtgtctg ggcactgtga acttgaaaga aagccttcac cctgtccctg ataaccttgt 360
   gttgtcctca gatgagccca tgtctaaagc tcccatggcc aaagacagtt accagcttct 420
   cacctagccg gtcacctctg tctaacttgg tatgatcact gacaactttg gccaattaat 480
   gaagaggtgg cctcaaattg ttcaggaact cgaaaagcac atgtctgaag gggctaattg 540
   tagtgatagg aaactataaa agtaaggatg ttggattaga agttagctga tcatcaggag 600
   atcaagacca gcttggccaa catggtaaaa ctccatctct actaaacata caaaaattag 660
   ctgggtgtgg tggtgtgcac ctgtagtccc agctactcag gaggctgagg caggagaatg 720
   gcttgaacct ggaaggtgga ggttgcagtg agccgagatc tcaccactgc actccagcct 780
   gggtgacaga gcaagactcc gtctccagga aaaaaaaaag aagaaatcag ttgactgtac 840
   tacctttact ctcaatccag ggtcctatat tctagtccca cctacttatg tcttgctgtg 900
   ggaccaccag gaagtcttag cttcttaggg cccagggact tttcactgct aagtttaagt 960
   aacttgattc ggatccgttg tggttcccac agccttcaaa tactgtggaa gttttaattt 1020
   aaatcttcag ataaactctt aatttttgag aactccttga tttaaataaa acatgtcggc 1080
   tgggcgcgtt ggctcacacc tgtaatccca gcattttggg aggccaaagc gggcggatga 1140
   ggtcaagaga ttgagatcag cctggccaac atggtgaaac cccgtttcta ctaaaattac 1200
   aaaaattagc tgggcatggt ggcgcgcacc tgtagtctca gctactcagg aggctgaggc 1260
   aggagaattg cttgaacccg gaagccagag cttgcagtga gccaagatcg tgccactgca 1320
   ctccagcctc gtgacagagt gagaccccat ctcaaaaaaa aaaaaaaaaa gaggatgagt 1380
   ttcttaccta gcacaagatt aatttttcgt atgtgagaaa aatgtacctt catagatttc 1440
   caaacagaat tatggctttt gaacatacag gtactaaaat ttaaaaagga tttcattttt 1500
   ctcaatttgg attagatata ctgattgctc tcagggcgaa acgaatttta atttagttct 1560
   tctttttctt aagtgggagt aagcttttct acctaattta aaaaatgaga agacatttaa 1620
   tttacgcttt ctccttcact caaagatact aataaccata ctatttaaat tctaaatccc 1680
   ttctttaaag aacttcaaaa ccaaggagga aattaaaata ttttaattca tttcctgatc 1740
   tcactcatca taatagaaaa agattcttag attcagacaa gaaagataca aaccttagga 1800
   gaatttccac agtttatttc caaattttag gaaacttgat cctggaatgt tccttcattc 1860
   ttcacctata atttgtaaca atgtgaagtc acacttgttc cataaatcct gctcaaacca 1920
   ctctagtccc tagtaatctc tctgtccctc caaattcaaa caataaatgt agcccaaacc 1980
   tttcatttcc caaaccaaac agcatagatc ttctaaactg acatttgtct atagtgaaga 2040
   actagttcct cccctctccc tcccaattca ttgcagacca atacttttgt taaagaagga 2100
   aataatcaaa atgagttacc agaagaatga aacaggaaaa a 2141
<210> 43
   <211> 2761
   <212> DNA
   <213> Homo sapiens
<220>
   <223> nbla21735
<400> 43
   tagctggcgg cttccgagcg cctcttccaa agatggtcag aggggccgga ggcgtccccg 60
   ctcccgctcg ctactagccc gcgggccagc gccgcgtccc gagccccggc gggagccatg 120
   gctctaaaag gacaagaaga ttatatttat cttttcaagg attcaacaca tccagtggat 180
   tttctggatg cattcagaac attttacttg gatggattat ttactgatat tactcttcag 240
   tgtccttcag gcataatttt ccattgtcac cgagccgttt tagctgcttg cagcaattat 300
   tttaaggcaa tgttcacagc tgacatgaaa gaaaaattta aaaataaaat aaaactctct 360
   ggcatccacc atgatattct ggaaggcctt gtaaattatg catacacttc ccaaattgaa 420
   ataactaaaa gaaatgttca aagcctgctt gaggcagcgg atctgctaca gttcctttca 480
   gtaaagaagg cttgtgagcg gtttttggta aggcacttgg atattgataa ttgtattgga 540
   atgcactcct ttgcagaatt tcatgtgtgt ccagaactag agaaggaatc tcgaagaatt 600
   ctatgttcaa agtttaagga agtgtggcaa caagaagaat ttctggaaat cagccttgaa 660
   aagtttctct ttatcttgtc cagaaagaat ctcagtgttt ggaaagaaga agctatcata 720
   gagccagtta ttaagtggac tgctcatgat gtagaaaatc gaattgaatg cctctataat 780
   ctactgagct atatcaacat tgatatagat ccagtgtact taaaaacagc cttaggcctt 840
   caaagaagct gcctgctcac cgaaaataag atccgctccc taatatacaa tgccttgaat 900
   cccatgcata aagagatttc ccagaggtcc acagccacaa tgtatataat tggaggctat 960
   tactgcatcc tttatcagag gttcacatat gggatccttt gacaaatgtt tggattcagg 1020
   gagcagaaat accagattat accagggaga gctatggtgt tacatgttta ggacccaaca 1080
   tttatgtaac tgggggctac aggacggata acatagaagc tcttgacaca gtgtggatct 1140
   ataacagtga aagtgatgaa tggacagaag gtttgccaat gctcaatgcc aggtattacc 1200
   actgtgcagt caccttgggt ggctgtgtct atgctttagg tggttacaga aaaggggctc 1260
   cagcagaaga ggctgagttc tatgatcctt taaaagagaa atggattcct attgcaaaca 1320
   tgattaaagg tgtgggaaat gctactgcct gtgtcttaca tgatgttatc tacgtcattg 1380
   gtggccactg tggctacaga ggaagctgca cctatgacaa agttcagagc tacaattccg 1440
   atatcaacga atggagcctc atcacctcca gtccacatcc agaatatgga ttgtgctcag 1500
   ttccgtttga aaataagctc tatctagtcg gtggacaaac tacaatcaca gaatgctatg 1560
   accctgaaca aaatgaatgg agagagatag ctcccatgat ggaaaggagg atggagtgcg 1620
   gtgccgtcat catgaatgga tgtatttatg tcactggagg atactcctac tcaaagggaa 1680
   cgtatcttca gagcattgag aaatatgatc cagatcttaa taagtgggaa atagtgggta 1740
   atcttcccag tgccatgcgg tctcatgggt gtgtttgtgt gtataatgtc taattgaatc 1800
   tgcagaaatg accaagcaat cacttttttg gagtatagtt ttataaaaaa agaatgcagg 1860
   gtttgaagtt ccttacctga taattgtgtc tggcacatga taggggatca gtaaattgta 1920
   attcctaacc ctactgtact cccaaacatg gtgattcatg gtcaagaaaa atcttatata 1980
   tatgtataca cacacatata tatgtgttca tatatatgta tacatatatg tgtatatata 2040
   cgcatgtatg tatacatata tgtgtatata tacgcatgta tgtatacata tatgtgtata 2100
   tatacgtatg tatgtataca tatatgtgta tatatacgta tgtatgtata catatatgtg 2160
   tatatatacg tatgtatgta tacatatatg tgtatatata cgtatgtatg tatacatata 2220
   tgtgtatata tacgtatgta tgtatacata tatgtgtata tatacgtgtg tatgtataca 2280
   tatatgtgta tatatacgtg tgtatgtata catatatgtg tatatatgcg tgtgtatgta 2340
   tacatatatg tgtatatata cgtgtgtatg tatacatata tgtgtatata tacgtgtgta 2400
   tatatataca catatatacg tatatatgta tatatatata cacagttgaa tcagtgggat 2460
   taatacctat aatctctggt tttcaaaggt aatatggaat atttgacact tggtaaaagg 2520
   tgaactacct ttgtagtgaa tcttttcctc ttggtagcat caacactggg gataaatcag 2580
   aaccattctg tggaatgaaa tgtttctcaa gagcctataa tatagtagat agtgcatatt 2640
   aagatgtctg gctgggcatg gtggctcatg cctgtagtcc cagcactttg ggaggctgag 2700
   gcgggaggat cacttgagcc tagaagttgg agactaacct ggcgagaccc tgtctcaaaa 2760
   a 2761
<210> 44
   <211> 3851
   <212> DNA
   <213> Homo sapiens
<220>
   <223> nbla22247
<400> 44
   aatttaattt acaggcttga ctacctcagc agtttcacta agttctggta cataaatgga 60
   tgttttatta aagaagaaaa ggaaggggga agggaaggag gaggaagacg aagaagaaga 120
   aaagaagaga aggaaaggag gtgggaagga gaattcgtct ttttctgctc aatattatgt 180
   cagtgaaacc aaataatgtg tctcggttcc tccccctgag cattccaccc gggtaaaaaa 240
   ggaactaagc tcacctctgc tgagagaagc tgtgcatggc gagtggcccc cacacacctg 300
   gctggatgac atctgagggc tcggaggtcc ctgctgctca gtgtgccagg atggtcccac 360
   cgtcctcaca ttcacatttt tttatggatg atgcgtcatt ctgctaaggc agcaaaagtg 420
   aaaacaatca atagttctac ccaaatacct gttattttaa gaatggggcc agagtgattt 480
   cttggctatg agcagctcag gagacactat ttttcgttgt ttaaatacaa ttgattttcc 540
   ttgcttcaga acccagatca ctcacggagc tcctggtgtg tcgagcttgg atgaatttgt 600
   aatatgacac agtgatacct gtttgtttaa ggacaccttg tgtgtaatgt cagtgttgca 660
   ttactctgtg gttccaaaac ttcaagtcca cactggagaa ggtgggggca gcctgtggac 720
   agaggcagga aagaagggac attgttttga gtgcctgtta gcttgtaggc acagttccat 780
   gctcttttct gtaaaacagg ccaatgatat tggaagccaa gtttgtctgg ctatggagcc 840
   cctgtttctc cactctacca ataatcaaaa ctcacagtga gaggttaaac caatacatgc 900
   acacattacc aaaacaaggt ttcacaaaca atatttacct ttacacaggc aatttactct 960
   tattttacca gtcctactcc ataataattc aattctttaa aattatggtt gcaaccccac 1020
   taaattggcc tcataatcta ccattgtaac atggcccact gtttgataca cactggagta 1080
   caccttggta cccttcacat ttttaaatga tgctatattg gacttggtat ccatgtgatg 1140
   atcaagattg tatttgaaga tgttgcatag aaagtcccat cctatgattc agttttttca 1200
   tctaagaatt agaaattata acatttattc cccaaaattc tccagttgaa ttcactggag 1260
   gtattcattg cctctcagag agtctgttac ttaaaataaa gacaattaaa attaagatag 1320
   caagtatttt agcaacaaaa gccacaaaaa agaataataa ttataattgc tattgttagt 1380
   aataattgtt gaactcacaa cctgccggcc actgctctat gagctttcaa tataactcat 1440
   tcagttctca ctatcacttt atgaagtagg taaatattac tttcacttta cagccagaat 1500
   tcctttatct tctttctcat aaatttctca ttctaaactg ttaatataca tctcagaaaa 1560
   tgaatgagat tgtgactatg actcaagaaa tacgtatttc tatgcttggt ttaaataaaa 1620
   tacaaaagcc tgtatcatct aattggcttg ataaatctca ctagcttttt aataatcatg 1680
   aaatttaaat ttttttgtag aaaactttca gaatacttaa tgaaaaatca gtatgtattc 1740
   accttcaaaa aacacaaatt tccaggcata ataataatat ctgcaagtcc aaatgtaatc 1800
   acggtgccag ggctggttga ttcagcagct tatcactgtc actggggact cagattctct 1860
   ccacctttcc actctcccat ccctcatcag ctttgtccta tgagggctgc acagatccag 1920
   gtgtcacatc cagcatcata gtgacaagaa caaaggtctc tttctcagga gtctactaag 1980
   tgtcccttaa atcctgatta gcaaatttcc tttgctaaaa atgagttata tgccaattcc 2040
   taaactagtc actgttaggg tgtagaatca ctgtgattgg attagaaaag ctctgcctcc 2100
   tggaactagg aatgatgttg tttgccctga agcacatgga tctgtggtca ggaagagggg 2160
   tcttgcggga aatatatata ctgagtaagc agtgtctgat acaaagacga aaaatatttc 2220
   ttttgacagg aagatttggt aaaatataaa gtagtagaat tatttcccat tatttaatct 2280
   gtttaatgtt tcataaaaat tagcaaacgt aatgaggaaa cgtatctgta agaatccact 2340
   atgcatttgc tgtttgctct gaaatcaaca ggacccagtg ctttcattac tagaaagaag 2400
   aaaattaggt aggttaataa aacaaacatc tggaaagtat caacactcat aaaaataaat 2460
   ggaatcatcc tgtgtataca ctaaagccag gttggcattt gtcaacaact acagagaaaa 2520
   cactacagaa tttactactt ccaacttcct gggtgggttt gttctcattc atttaacata 2580
   ttttcctaag tgaaaattta gttttaggtt ttgaaataca atcatataag aatatgtaga 2640
   ctaatagtgt ttattaattt ttaataatgc ctacagtttc ccatatttgg ttgcattttt 2700
   cctgctatcc tattgcttct gagccacctg ttcccctctc aaaaacatgc aagctgggat 2760
   ttttttcttt tcttttaact agatatcttg ccaaaatttc agactcatag taaagagttt 2820
   ttatttttca ccaacctaat tattaaaaaa ggagtattta gaatagctct aagaattctc 2880
   atacagcctt catcctcatt ccccaaatgt taacatttta ctacatttgc tgtatctatc 2940
   tctttgtgtg tatatgcaca gacatacaca gaatgtctat gccttatata cacatgtata 3000
   tctctgtgta tatatgtatg tatatatgca catattttta atagattctg agttttctaa 3060
   tcctttgaga ataagttaca gtcatgaaac ccctttattt ttaaatactt gtgtatcttt 3120
   ctaataaaga agaaattccc caatgaaaca acaaaagatt accaaaatca gaaaactagc 3180
   attgctataa tactcttatc taatttatag actttattca gatttcaata ctatttttat 3240
   ggtcaaaaaa aatcaaatgt catgggtcat gccctgaatt cagctgtcat ttctctttag 3300
   tcttctttat tctgacagtc ctttagtgtt ttcctggcag aatgctgtta atatcagtct 3360
   tcagtaaaac atattaagag aggaaacatc atgccaaagc cagtggattt gtatggatgg 3420
   tggcttgagt gggattcgtc ctgcctttgc agccttcctc ctgcagggat aataggtgtg 3480
   agtacgtttc actattctct tagacatcct gacctgtacc acaaatgtga agggccaact 3540
   ggagaactag gtgatccaac agtttgttat taatcatctc atctcttgcc aatgaatagc 3600
   aacaagaaca tcccaaaaca tctgaaatat ttctaaatat tctaaacatt tgtaaaaatg 3660
   tgggacatta tagaaaaaaa cttacaaaaa catttgtttc aatcactgca tgcttagatg 3720
   caatctttaa aagtacttca agtaaataat tagaatgggg atgtttagaa ttggttaaag 3780
   gttcattatt tctgaaccaa tgtgcagaat ttggcttatg agtacaagaa taaagacatt 3840
   tggatcaaaa a 3851
<210> 45
   <211> 1863
   <212> DNA
   <213> Homo sapiens
<220>
   <223> nbla22477
<400> 45
   agctgcaggc tccgagctgg tttattctgc ggccgaggat tacatttatg cacgaacggg 60
   cttactggtt ccagattccc cacttgggca caggcatagg aggcttgttt tccaaattgc 120
   tggttttaat tgcacctgcc tttcagatta cctctgggaa tctgtgggag gagccgagag 180
   ggtggaaaat gtttcttagc tttgcaaaag gaagaaaact ttgtcaccca gcgggagacc 240
   tcagccacga gtaacccggg gagacaccag aaccgggacg ggctttgact gatttgccta 300
   cgagggttcc gtaggaaagg acgcttgaat tcggcgcttc ggcggcggcg gcggccgcgc 360
   gagttccctg ctcaccctcc ctctccgcgg aagtccccac gaggtggctt cagggtgtaa 420
   cagagcgcgc ggctccagtc cgaaggcagc ggccggggga gggaaggagg ggaccgaacc 480
   cccgaggagt tttgcagaat caacttctgg ttagagttat gggaagcgcg gttatggaca 540
   ccaagaagaa aaaagatgtt tccagccccg gcgggagcgg cggcaagaaa aatgccagcc 600
   agaagaggcg ttcgctgcgc gtgcacattc cggacctgag ctccttcgcc atgccgctcc 660
   tggacggaga cctggagggt tccggaaagc attcctctcg aaaggtggac agccccttcg 720
   gcccgggcag cccctccaaa gggttcttct ccagaggccc ccagccccgg ccctccagcc 780
   ccatgtctgc acctgtgagg cccaagacca gccccggctc tcccaaaacc gtgttcccgt 840
   tctcctacca ggagtccccg ccacgctccc ctcgacgcat gagcttcagt gggatcttcc 900
   gctcctcctc caaagagtct tcccccaact ccaaccctgc tacctcgccc gggggcatca 960
   ggtttttctc ccgctccaga aaaagtaaga ccttgatgct attgtttcag cctccggcct 1020
   ctcctcctct ccgtcaacac ccacccaagt gaccaagcag cacacgtttc ccctggaatc 1080
   ctataagcac gagcctgaac ggttagagaa tcgcatctat gcctcgtctt cccccccgga 1140
   acagggcaga ggttctgccc gtcttccttc cagagcccga ccaggcctcc actggcatca 1200
   ccgacacact atgctccctc caaagccgcg gcgctggcgg cggccctggg acccgcggaa 1260
   gccggcatgc tggagaagct ggagttcgag gacgaagcag tagaagactc agaaagtggt 1320
   gtttacatgc gattcatgag gtcacacaag tgttatgaca tcgttccaac cagttcaaag 1380
   cttgttgtct ttgatactac attacaagtt aaaaaggcct tctttgcttt ggtagccaac 1440
   ggtgtccgag cagcgccact gcgggagagt aaaaaacaaa gttttgtagg taagcagtgt 1500
   gggcctgagg aaaatcgaaa atggaaacct tgaaagcaga aagcctaaag tattttaata 1560
   gatgccggtt tggaattcaa cctagtaaac atgtttccaa gttaaagaac attcttgctg 1620
   gcagggtgca gtggcccatg cccgtaatct cagcactttg ggaggccaag gcagggagat 1680
   cgcttgagcc cagcagttcg aggccagcct gggcaacata gcaagacctc atctctacaa 1740
   aaacatgcaa aaattagcta ctcaggaggc tgaggtggga ggatcacttg agcccaggag 1800
   gtcaaggcca tgatcgctgc actgtactcc agcctgggtg acagagcgag accctgtcaa 1860
   aaa                                                               1863
<210> 46
   <211> 2680
   <212> DNA
   <213> Homo sapiens
<220>
   <223> nbla22639
<400> 46
   agacggacaa cttgagaaaa gcagtcaagt tccaaggaac tgacagcaac ctgcaaagag 60
   gaaaacagca tctcctcacc tgcgtaaaat tgtctcagct tctgttgttt ctcaactgag 120
   gttcgtaaac ccatcaggat aatccctgga gggaatagat ccttgcacat ccagggcaag 180
   aaacatgtcc aagttaccca gaccattgat aacagttgca tttaggttgc acctgggtaa 240
   tctggcataa aagatctctc taggcctcac tgttgcggtg tctatccctt cacctccatt 300
   gaaatcagca ttttggatct aggtcttcat ggaatccttg agaagagagg cctttacaat 360
   tacccagttc tgagggttca ggttcacgaa aagaaatgca acttgggata atcatgaaca 420
   ggttaaagat aagatttcaa gaagccatct aagaatacag aaccaaattg gatccatttt 480
   tttaaaaaaa tggttttgca tggaacctgg accaaggcaa atgtcttttc ttcgcagaat 540
   tgttttccag gatgccagtg gattcagata gcaatgcttg gagtagaatc cgttactaaa 600
   atagtttcaa agttgacaaa aaattttcaa agataaaagc agttttacat tgggggttgc 660
   tgaggtaggc acaagaaaaa gtcaggcata aagcacaagg cagactgttt gagtggattg 720
   gttgctgctc actaaagttg ttcccctgat ctctaaatat ggaggtcatt accaagaaat 780
   gctttggtat gaatgagagc cagatctcca ctgtgtgagc cagtgaatta tggctaattc 840
   ggctgttaca gccactggtt ggctggattt taaaccataa aacttgaaga ttacctacaa 900
   aagtaacagt gtggctataa gcctgagctt taatggatat acatcctcac agaaaagttg 960
   gaaataacca aaactgaagt cttaatttac cttcagttta atctgtggat ttgttcaaat 1020
   actaaagatc ctcaggtcca gaattccagc atcatttatt cttttaaaat ttttaagaac 1080
   ttgatccatt gtatcagtac ctcacaatca gagttggcaa atgatggatg agtgattcaa 1140
   gcagtgcacc cggtggaagc tgaaatccat ctgtgaatgg aactgaagtg aacgtgaata 1200
   tgctgactat atcctggaag catttttata ccatcttgaa atttcaacaa actggctttt 1260
   gccagttaat ccagctgtct ttcaagaata aaagttgggg ttttcaagga tcgcctcttc 1320
   tatattttaa atggattttc agtagaaatg atttttacta atcaagttaa tcccacccca 1380
   tcaaaaggta ttcctagaaa tgtcatagac ctaggtaact ttgaattgaa tgggagctaa 1440
   cgttctttcc aaagttttca ggtattcttt gtgtgacacc ttctcaacca ggaggcaagt 1500
   aaccccgcct ccacaatctt agtatttttt ttaaactgca tgcctgcccc ttatttgagc 1560
   tgccttttta atttattgca tatccttttt attatcttat tttggtatta ttcaatctat 1620
   acaatctttt tgtatttatt gggaaatgag taatatacaa aaaggttttc atgtatttgt 1680
   ggctgagagg gcgggaaata attgtgtaca taaaattagg ctttttaaaa aaaaatagat 1740
   tatgatgcag aatattgttg atcttagatt aaaaagtgga agagccacaa acattggtgc 1800
   ccttttcaga ctatttctct actctcatca tccacagtag aatttttaaa cagatttttt 1860
   taaagctttt cttttaaatt tttctccgtt gcaaagaatg tttcctaaat tgtatgggag 1920
   caatagtatt tttgatgttt taatgacatc cgtatacttg tactgtattt tgtactacaa 1980
   ggcagctgtt tttcaataat gtcctgctgt atttacctac gtgttttgag tgtctatttc 2040
   tttgctgcgg agaacaaatt cctaaatagt tttagtaaag gagctgagaa gctagcatta 2100
   ggtttgcaga aactatttaa gtttcaactc tgaggcagca atgaaaattt aagttgcagc 2160
   tattagttga ttgctgtaac tttttcattt tcaaaccatg tacaattctt gtatagacca 2220
   acttgttttc ttgcttcagt ggtggttctg ttgctcagct gcagtgagcc agttcaattt 2280
   tgcaaaggtg cagtacctct cctttttaag ggttggtttt attctttttt ctttttgttt 2340
   ggctgaattg cagtaactag ccttgccttt ctattctgta gaaatgacag ggtcttcaca 2400
   atccttcacc agtggctact aagctataat tagctgaata gaaagaatgt ggaagtggtc 2460
   tgaggcatat agagtatatg ccaagaacac taccatatat ggcatcagct ttggttacca 2520
   gagaaatttt cttagtcatt agaccatgta acagtaatat atcatatgta aatctttaga 2580
   tatcaatttg aaaatcctcc aaaaaaagga gcaaagaatg cataagctat gtgttggcaa 2640
   aagtaattta tattaaaatt ttgacctgcc tatgtaaaaa 2680
<210> 47
   <211> 1755
   <212> DNA
   <213> Homo sapiens
<220>
   <223> nbla23174
<400> 47
   atataaatgg agggatcacc aaaacaaaga ttatctcttt ggtagctatt taacctgaaa 60
   gcgtaggagt ctttccatta tagaagcccc tccgttccaa ggaactagcg atggggctag 120
   gtcaatcagc agagttgaca acagggcttc tttttgtgca ccagcattcc ccttcagaga 180
   gcataagatc ctgccagtgt gccaagtttg cagctgacca aacttctagg ttgtactgga 240
   attattctat gcaacactga tccttatatg aatgcgtttc ttctgaatga tgttgactac 300
   ccttcttaca acaaaactgt ttctttttta ttgcaaatag ggctcttggt gttttttact 360
   tttttgtaca tatcacagta catggttttt cactctttag tttatttcat tttattggaa 420
   ttaacttttt ttattctaat actgacagag tttgtaatct ctatataata cgtaattact 480
   ccaattacag cacttttacc ttgaagagca tctcagtttt tcccacaatt tcattgagtc 540
   atcagagact gatgttgctt cttggtttca aatttggtcc taaagaaact ttcggctgta 600
   gaaacaaaag cacagagtga attttttaca aaagacaggg aatatagaat agtcattaca 660
   gacacaaata accctagtag cacgaagttg gtgttttctc tgtttttact taagattaag 720
   aagatttttg gtgactctga actctttatt tatatttcag tttaaaatat caagactaag 780
   gggcatcagt tatctttact ctttaatatt gcccatattt taataaatta cactaattaa 840
   acgcatattt tcagcatacc agtggaatta attttgtgga tcacacacat ttaaatagtc 900
   atattgtggg aatattatag ctggtaacca gctgatattg attcttatta taggaatgac 960
   tgtaatgata gtggtggtag cagtagtgat attagcggtg gtggtgatgt gaagtaaaat 1020
   aaaagtatat attatattgt gcccaattta ttagaaatta tttgatcaat gcttcatttc 1080
   attaaaatat cataaagatg tttatagtat ttttttactt tattatttaa atcataacta 1140
   acaatatttt taaaaactta ttttcattgc tacaatgtca aatattccaa aatcagccaa 1200
   ctacagctat atatgtgtta tgtgtgacag aagtgatctt ccttccctct ttttgagctt 1260
   gacatgaaag tgaaagaaga ctcaatgaat aattatgagc tatttattta ataattactt 1320
   gccttgggtg taatacagta atgaatgagt gaaacaaata ttctcattga atatgataca 1380
   atgctgtttt ctgtatgttt catgttctat tattaaaggt atccattagg ccaaaattat 1440
   ttaatcaaat tctttatctg ataggtagat tgagagcatt ttcttaatgc attaccttgt 1500
   acataagtat acacttggta aagtagacga agttgaaata ttaatttcat ttggcattta 1560
   gcatgtgaat atgattattg tttgattgtg tctgtatatt tgtttggtga cgtgctcagg 1620
   tgctcccact actgattaat gtgtgtgcta atatcctaaa aacacatatg aggtttaaga 1680
   aaaaattttc ttgtctgaaa acataaacat cttaataaaa ctgattttga aataaaaact 1740
   aaagtacttg aaaaa                                                  1755
<210> 48
   <211> 1409
   <212> DNA
   <213> Homo sapiens
<220>
   <223> nbla23198
<400> 48
   caatgtagca gttattgata gagaaattga gaaaactgaa acgtgaccgg agtattggaa 60
   ataacgtagt acatcaccta gcacaatgac acatagtagg tgctcaataa atttatgctt 120
   ataatttttg tcacttctat ggcaggattt ttttattagg ttaaaattat cttttaaaca 180
   ccttccggaa ttttagaata ttcattaata atgtcttcaa acctttcaac tgaaataaat 240
   ttacagctga agtctgatga tttaaagtta gaaagtttaa tcttgaatat aaatgaacat 300
   tttctctccc acattttctt gggcattttg agaagtaaat gcgttattta ttggtccatg 360
   aaatgtgact gtaaatattc tttgctatac attatgtcta tatatctgca ttcatcctca 420
   atgccaaaac tagaatcatt agtcttaatg atcattttaa gtacaggcag tcctcgcttt 480
   ccttgatacc atgttaaccg aaacttgtgt atgtcaacac ggtgtccttg ctttgcttgg 540
   ttaagtgtga gttcttcctc ccttttttta agagttgtac aacgtttttc agtcgcctac 600
   cgaatcaggt catagactat ggaattgacc ccaccccacc aacattttta cagctaccct 660
   gatttctgac cagaaaggaa aaaaaaactt tccagctcta tcacacattt tacctactct 720
   taaacttagg aggtattaca aatagcattt tctcatgttc tctttctggc ctgtacctcc 780
   ctgctaagct tccttcagtg ttcatcctca cctcatagag agatgaagtg aagagacaaa 840
   cagaagtcat tttcttcctt actttagtgg tttctggttt agttagtttg ggccaaactg 900
   tggacaagta ccttttcagg taactttttt ttcttatttc tatgtcctca acacctagtg 960
   gagtacgtag ccaatagtag atgcttaata aacatttctt aaattaatat tgttgacctt 1020
   ttctgaccct gttcttgaca gtaaggtaca taatctgcct tcatcccttt agtccttagg 1080
   aacagataaa gtcatggata tgaaagtgat cactgtcatt aatatccaca ttaaaattgc 1140
   tcttgatttt agtttctcca taatcatttt ccctaaacaa tgaactctgt tcaccttttt 1200
   ttttaaaata tgcacagtga atattactgg tagcccaaat cttctaacat aaaatttcca 1260
   ttttgtaaaa gcttctgata agcatatatg ttatgaattg aatgtttgat tattatactt 1320
   taatattctt gaaaatattg atacctggac tggaaagaaa acagacaaaa gtaaatctca 1380
   gaataaatta ctgctttaaa catgaaaaa                                   1409
<210> 49
   <211> 2433
   <212> DNA
   <213> Homo sapiens
<220>
   <223> nbla23328
<400> 49
   tgtttctttt tttatttaaa attgtcattg tttggtttaa atttttcagc tagatgaaaa 60
   gagtatgaac tactttggaa aacttaacag ctcagagatg gccatgcctc cagcccctca 120
   cgtcatcttt gcaacagacg actgggctgc catggtccac ccctcagccc gggtcccggg 180
   tctggatgga acgggagcac tgctggtgcc cactggcgtg tgtgccccgg gtccctgtaa 240
   gtgccccctc accagcagca gcgtgacaca cacaagactc aagaccaccc tgtcagtgcc 300
   ccccagtgca cggcaaacgg gcaggtgccg ttcccccagt gacctgaggg taggggacaa 360
   ctgagcagta tctgaccagt gccacccagg agccagtctc ctggccacat gcagaaagtg 420
   tggcccctgc ttacctagat gttttgtgca cctccatggg cagagggtgt ggatattgcc 480
   tggattctgt gctgtcagcg ttgctgagta tggccccagg agaccaagga gagttttgta 540
   taggctggaa aacccctttt cagtctttcc aaaattagag ggtatggcaa gtttcctttt 600
   ttctctcctc ccttccttcc cctccttcct ttcctttacc cctcctttcc ttccttcctc 660
   ccttcctctc ttctttcctc cctccctccc tccttccctc ccttccttcc tctctttcct 720
   ccttccttcc ctcccttctt tccctccctc ccttcctctc tcccctattc cttcttcctt 780
   ttctcctcct ttttctgagt ggagggggaa atattctaaa ccaaaaatcc tagatgctct 840
   gcccaaagcc acttctgcat gagaatcgca acccacagtt ccccggatga gactcgccac 900
   agtggacagt gccacctcct tcccctcggc cccggagagg gcgaagtggg cgggaagcca 960
   ggatgtgagc actggaattt cttggaagag aagcgataaa tggagaccat ggccagcgct 1020
   gctttctgtg cactctgatg actgctctct gcagccatga ggatgtggct ttacatgcca 1080
   gggagagtgt tgagacgtct taggttgagg atgagcagat tcgagatatg tttgttgctc 1140
   tcgggttttc gatacaacat catgacactt ctgtttcaag ctcatgtttt ccgtctcccc 1200
   tccactctta gtaaaccttg atctgtacgg agcggcctgt ccgaggctac gccggcctcc 1260
   tggctgctgc tggactgtgc ttaggacagc gcccatgcct cggagggact ctgtcccatg 1320
   agaaccacct gtgcaaagga acagagctgg atgtttccag gtagattttg gcctcccaga 1380
   gcaatgcggc atttgagaag caacagttcc taactcctta tcttcaggga aggaaaagaa 1440
   aatcacagcc taggaagatg gaggttggat tttaatctcg gttttaaaaa gaggacaaac 1500
   aaaatgtctc taagccaggc tagatggaat gtgctcccgc tctctcctgc cgtgctgaaa 1560
   gtcatgcctt gcggatgcct catgacagca gtggctgagt ctccccaccc acccccaacg 1620
   tggctcattt cagattgctt cggccccacc ctgcaaggat gtggtcacgg agtggccagg 1680
   aggctccgtc tgagccacag ggatgggtgt gcagagctcc ctcctcctgg ggtgccaggg 1740
   cagagattcc aggcaggtga gcccagagag agctgccagg ccacaccccc tcggcctcct 1800
   gcacggccac cttctgggtg aatcggtcca gcccaagccc ctctccccag cctcgccttc 1860
   agcctctctc ccagcctgct tttataaggc gcacttcact caatgctgta gccaaaaaac 1920
   gaggggcccc agggagaggg gacccagatg gccacacacg gaacgcgcct ccacagcccc 1980
   gggaggtggc tcactctgta caggtcttcg gaggccgtgt ttgtatctaa ctgtgactgg 2040
   gctgaagcat gatgttgcct aatggttcgt agcatggttt ttatttctta cgcattcttg 2100
   gcacacagtg tagctatcct cctgacgagc aacccgtctg cgtacctaag tgtggctccc 2160
   cgtgggtcag cgtcctggta gcatggatcc agtctgaaag gtgaggacaa cgtggaaact 2220
   catgagctga gcctgcccgc tgggacacgt ctccttcccg cgtcaccttc tggtttaggg 2280
   agccgtcagg tccctaaacg ttccctacaa ctttttctga aattgtgcag aaaaacagat 2340
   ctcattaaaa gaaaaaaaga aacaacttgt aggaagacag agaggtgcta tgggtacaat 2400
   ttttaataaa aacattattt tgttccttaa aaa                              2433
<210> 50
   <211> 2201
   <212> DNA
   <213> Homo sapiens
<220>
   <223> nbla23420
<400> 50
<210> 51
   <211> 1806
   <212> DNA
   <213> Homo sapiens
<220>
   <223> nbla23483
<400> 51
   tttgtaactt aaactttgac caagaaattc ttcacttctc acttcttcac ttcttcccaa 60
   tatacagtaa gtacgtgagc cagtcatcca tacactaagg cctagttgag aaaaaccttt 120
   gattcaggat ggctgggtta ctaaccttga aatgtaagag atctggtttt gaatgtaaaa 180
   gttgcaacac acaaacggaa gtcttaaaaa ctttttgctc tggtcagtta caggtggatc 240
   cccaataatc tgtttttggt tttctgatgg aaataataga attaggggaa atcaaatctg 300
   gttggtaggt gtctacagta ttagaagagg gtataagggc actgtttaac actaagttct 360
   aatacttcca gaaactgtgc attccagatc tacatactaa atgctcttat cattttgaaa 420
   tgggctcttg attaatagac ccatattttt tagtggcttc tatgttgtat atttgtctaa 480
   aatgaaagct cttttgcgtt ctaaaactac aatatatgtc atcttatttt ccctgagtat 540
   ccaagtatag tgcagattct atgtaaaact actaaatgac actggaatat gtttagtaga 600
   ttagggggaa aaactataaa ggtttataca attgtttgta gttacattta ggatggactt 660
   atccctttgg agaagagtga agtttgtttt ttcgccatgt gatgaagacc actgtgattt 720
   tttaaaaaag tagataatac ttaaaatggc gtaataattc tgcacttgaa tttgtactgt 780
   taacagcaca tttggaagat tttaaaactt tttattgtct tataaatagc attcacttat 840
   tattttggat atttaagggt tccattaagt taacactgta tttggacaaa gtgtgaccaa 900
   attagccagt ctgttttctt ccatgtttaa ttagaagtga gaggtagaag tacttcaaat 960
   tcaacaggcc agcaagcaat cggcttaaaa ttccctttct taaatgttgt gctcttatgt 1020
   tctcggcttt ttaatgactt tatttttaca gtacttgttc agtcacttga gatgaaatgc 1080
   ttggggtagc ttttccatcc tcaaacttaa tgtttttact agttcatagt gtttggaaca 1140
   gtatatgcca atcactgaga ctgcatcaga gtttgcaatt ttgtatgttt cattgccaaa 1200
   gaaggcttag tggttgttga ctgtagtata agtcagcttt ctgtagcata agatttgatt 1260
   ttcccatact tacttcactt gttatacatc actgattatt tgggttaaac tggactcatt 1320
   tcaagcagtt tgcttttgtt caaatcgtga tgagaaacct aatactgtaa tttgatttga 1380
   gccataaaac acattttaat attagcttgt attatagtta ttaagcttgt ttttgtggaa 1440
   aaaaacttac taaaacctag gtaactctag attaggccag ttcaggtgta ttttgtatct 1500
   tagtaatgga tcatatcgta aaaatagaga taagttggga agatatattg attatgctgt 1560
   tctgttgagg gaaaggtcat gtatttagaa atttaaactt ttggttattg tgttcacatc 1620
   atagtattca agcatcattt atagtttggt tttgagaact tttctggtat tacgtttatg 1680
   gcaaatgtat aaaagaaaca agttttggtt atatttttat atttgtaaag taagtttggt 1740
   taaagtgatc actgttcttt ttttatttta ttgtcatttc aataaaaaat atttgaaaga 1800
   gaaaaa                                                            1806
<210> 52
   <211> 1659
   <212> DNA
   <213> Homo sapiens
<220>
   <223> nbla23808
<400> 52
   aagacttgat gctgctaagg atctactatg tgccaggcac tgctctgggc gctgggacct 60
   gcacctgggc tttttcgtca tggtgctttt atagcctagt gggagagttg gtgaagtaga 120
   tagtgattca gtgagatggg tgttatgatt ggtcaggggt ctgtgggagc accaaggaga 180
   cagacaagat tgatgtgcac ctactctgtg ccaggcgtgt gccaggcatt ggggatgtag 240
   tggtagttaa acaccatttg gtcttcagga gctttaattc tagtgtgttg ggtgcagggg 300
   ggtggaatgg ggacagagag acacctaatc caccctgtgg tggctttctg gagagggagg 360
   catctaagct gagctgtggc tgggtggagt gtgggtgggg atgagttccg ggcagcgaga 420
   gtggtggaca ccagtttctg gggatcagag aggatccaaa gaggttctgg aaggttcatg 480
   tggaatgtag caagagatag gagacatgga catggtgccg ggtctggttg ccaagaagtt 540
   tagattttat ccttaggcct tggggagcga cggatatgat ctgagaaagg gagttagtgg 600
   atttgagttt taggctggcc atttggcttt tccagcccag gtggaactca gaggagtttg 660
   caatggcctc tggccacatt ttagacaact gagcagaact ttttgaaact aggaagaccc 720
   tttggtccat cttttgataa acagaatcca tacatgtcta ccccagttgg aagtatctct 780
   gcaatgactg gaaagtaaag aggaccaagg tgaaaataaa ggctcggaag gggagcaatc 840
   ttgaaaacat gtcatcccat ggtggtggga agtccctgga gaagatcagg ggaaacacag 900
   tcataggctg caagtctata agataattcc attggggagg gagcccattt gtcatgcatg 960
   gctgcaaggg gcagatacaa gtgtggagta agcttgcaag agctgatcct ggtcccagag 1020
   agggaaaaat atgccttggt gggtaatgaa ccttttgttc ccagaggcag aaggattggg 1080
   actaggccaa catagagatt ggcgatggtt gtgagattct aagagtgtgt gtgcatcttg 1140
   acaatattag aggaggctga gcccaagcag gcacattctc ttcgacccct ccctcattca 1200
   gtctgctttg gagtctactg aacatcaagc ttgctatgag caggatctta gagctgagga 1260
   attggcctcc caatccgaac aggtgttata atcctttctt aataggttgt gctgtggacc 1320
   caatgtgagg gctgtgctgg tgtaaatggt gacatgttga gctgggggga tgctttcggg 1380
   gtggggggac tggttccatt ccatcaaagg ccctcttgag agtctatcca gggacccatt 1440
   gttttacttt aacagaccag aaaagatgtt tgttttccat gtcattaccc ccaggggata 1500
   ccgaatgtgt gggtagaaat ttctctgtag attaaaaatc agatttttac atggattcaa 1560
   caaaggagcg tcacttggat ttttgttttc atccatgaat gtagctgctt ctgtgtaaaa 1620
   tgccattttg ctattaaaaa tcaattcacg ctggaaaaa 1659
<210> 53
   <211> 1520
   <212> DNA
   <213> Homo sapiens
<220>
   <223> nbla23851
<400> 53
   aattaccaaa caaaattata gtcttgtact ctaagaagca gccatgtctt gagtgagaag 60
   gcttaggata tgaggactag atatcagcaa ggataccata ggtttggaaa gacattttaa 120
   tttaccctta gaatacacaa ctttactgat ttttaaggat gatcagccca tcatatagca 180
   ctttattttt tcttttaaag acaatcctgt ctcatttacc ttcacttgac acaggagttt 240
   gagaagtcct gggcaggtat acctggttat tttgtcattg gtagtctttt taacttttag 300
   aaaataatcc tagtaaacta aacctgagcc tctgaataag atgttgtctg cctttgtagc 360
   tatatgagaa gagtggcaga ccacagcttt tgacggggat ttttgaataa aataactaaa 420
   accaacaata cagcaaaagc tcatctggga aaaggacaaa gagtaaacta gtaaaatgta 480
   aggctgtaag gaaaggggta gaagatcaga ggaattctca tcaaaatatt gcaaattatc 540
   ccctgaacac aaactggtaa cggtggtttg cttaagggaa ggaaattcgg agattaggga 600
   tactgggtga aaggcagatt ctttttttta ccatatattc ctttgtatct tttaaatttt 660
   gtattacatt catttgtgat ctttcagaaa taaataaata aaaatgcagt agcttcctga 720
   tcagaaagag ggaataattg ctgtcacttg cgtttcagaa acatagcatc caaactgatg 780
   tgattatggt gacctgtccc acttagtttt gctgatgtac tataattact ttctccagtg 840
   aggctgactt cagaaacagt tgcagatgca gaattttaat ccagggtatg ctgtatataa 900
   gtaacttttg catttacaat ctaccatttg gcgttttatg gctaataatc cacaaatatc 960
   taaactaatt tataaaggca aaaactactg atttaatgta gtactctgct tctgtatccc 1020
   cgaggtgagt cagaaaaatt tcaagttgcc acgccttggc cagaccccac agtatattgg 1080
   ttattggtcc tgaagttagt tctttaaaat aacttgaaat gtttcatgct tagttctagg 1140
   atctatactt tctttgattt gactgggact gaaaggctca gaataactga atatccttgg 1200
   ctctaaataa gaagctgtaa ctttgggcca ggtgcagtgc ctcatgcctt tgggaggcca 1260
   aggcaggaag gtagcttgaa gtcaggaatt taagacagtc tgggcaacat agtgagaccc 1320
   ccatctctat aaatgctttt taaaagtagc agggcatggt ggcatgtgcc tgcaatctca 1380
   gctacttgga tgggtgagtt gggagcgtcg cttgagccca ggagttctga gctgcagtga 1440
   gctgtggttg cactactgag ctgtgattgc actcaaggct gggccacaga gtgagaccct 1500
   gtatttaaag aaaagaaaaa                                             1520
<210> 54
   <211> 2962
   <212> DNA
   <213> Homo sapiens
<220>
   <223> nbla24011
<400> 54
   aagaacactt gtggatcaag gcgtgggtgt ctttttcttt ttcatccaca gtacaggttt 60
   caaatggttg tatggaaagc tttggataac catacttagg gaacattaaa aatggtttta 120
   ttttggttgg ctcaatggtg atccaaagag ggggttgtgg tagtggtttc aataaaactt 180
   cacaaccaat gggaatcttt tttgagtttg tgtggagtgc ccttaatgct ggaaataatc 240
   ctgttggcta ggactccaga actgtacgga tgagaaaagg atgcaggaaa ttctgttgtt 300
   tacacatgtg gctgcaactg agacactgga gcagcccagc aagcccagag ggtcttaaaa 360
   ataaatatga atttagattc catacatcga ttaattgagg aaacacagat cttccagatg 420
   caacaatcat caattaagtc acgcggcgac atggtggccc ctgcctcacc ccccagggat 480
   acctgtaata cctgcttccc acttcatggg ctacaatctc atgctgctca caatttctgt 540
   gctcactcat ataacaccaa caaatgggat atttgtgaag aacttcgcct gcgggagctt 600
   gaagaagtca aggccagagc tgctcagatg gaaaagacca tgcggtggtg gtcggactgc 660
   actgccaact ggagagaaaa atggagtaaa gttcgagctg aaaggaacag tgccagggag 720
   gaaggaagac aactcagaat aaaactagag atggcgatga aagaattgag tacactgaaa 780
   aagaaacaga gtttgccacc tcagaaggag gcattagaag ctaatgttac ccaggatctg 840
   aagcttcctg gcttcgtaga agaatcctgt gaacatacag accaatttca attgagttca 900
   caaatgcatg agtctatcag agagtatttg gtaaaaagac aattttctac aaaggaggac 960
   acaaataata aggaacaagg tgtggttatt gattctctaa aattaagtga ggagatgaag 1020
   cccaatctag atggtgttga tttattcaac aatggtggtt ctggaaacgg tgaaacgaaa 1080
   actgggctga gactgaaagc aataaatctg cctttggaaa atgaagtaac tgaaatttca 1140
   gctttgcagg tgcatttgga tgaattccaa aaaatcttat ggaaggaaag agaaatgcgc 1200
   acagctttgg aaaaagaaat agagagactg gagtcggctt tgtctctgtg gaagtggaag 1260
   tatgaagaac tgaaagaatc aaagccaaaa aatgtgaaag agtttgacat tcttcttggt 1320
   caacataatg atgaaatgca agaactgtca ggcaatataa aggaagaatc caaatctcaa 1380
   aacagcaaag acagagtgat ttgtgagtta agagcagagc tagagagatt gcaagctgaa 1440
   aatacctcgg agtgggacaa gagggaaata cttgaaagag aaaagcaggg actggagaga 1500
   gaaaatagaa ggctgaagat ccaggtgaaa gaaatggaag agcttttgga taagaaaaat 1560
   agattaagtg caaactctca aagtcctgat ttcaagatgt cacaaattga tctgcaagaa 1620
   aaaaaccagg aattactgaa ccttcaacat gcctactata aactaaacag acaataccag 1680
   gcaaatattg cagaactgac tcatgcaaac aaccgagtgg atcaaaatga agcagaagta 1740
   aagaaactaa gattacgagt ggaagaacta aagcagggac tcaatcaaaa agaagatgag 1800
   cttgatgatt ccctgaatca gatccgtaag ctccagaggt ctctggatga agagaaagaa 1860
   agaaatgaaa acttagagac tgaactcagg cacttgcaaa actggtaatt ttttcacaaa 1920
   atatgctgaa ttaaagatta gggccttaaa gacatttcca tatccttttc ttaaatatca 1980
   gtaaaattgt ttttattaac tagaaatatt aatgaaaaaa acgtagacaa tacacaaatt 2040
   aatgggcttc ttcacttctt ctaatttttg cctaacagat actgcatatt ctcaaaaaga 2100
   caatttaaat gtcatttaaa aacaacttta attctaagat gtgtaaatat tttgaaagtc 2160
   aaaaagggct ttcagaatac tttttacata aaatctgaaa gagttataat atcggtaaga 2220
   aaaagtaagt tgaaaaccat acaagacgct gggtcattaa taagaaaacc attgacttta 2280
   agtataaagt actggtttgt ttaaataatt ggtaaacttt tatgtacgtg ttgtctatgt 2340
   ggtggggatg gcaggttgta ttaacaaaaa tgaatcattc tagaggtgta acaatacatt 2400
   tcttatataa ttttataagt catttctaat ctttgtataa aacagaagtg agcagatgaa 2460
   tcagaaaaaa gtgttttgta ttttaaagta acagataacc agtgattgaa tctaagacag 2520
   gctgtaagca tcgctgagaa actaaaagga cttttgactt ttatctggat agacatttct 2580
   acagtaaaat catggaaagg catcagcatt gcaaagtagc atctaggtag aaatcaggcc 2640
   aaaattaagc tgtggtttcc ctctgagtag tgggaataga gaaaattagg aaattgtggt 2700
   tatgtgaata tttctttaaa acttttatgt acattatagt ttattgcttc atatttaagt 2760
   ttagttttta aggtaaaatg ttattttgaa caaaaagaca cttataattt tccataccta 2820
   ttttcaactg aaggcaactt gtaagattta actcagtcaa taacatactg gttttactca 2880
   tctccccctc cattgattag ccaaaaaaaa aatgaaatct tactaattca ttattgaata 2940
   aagaccactt ttatcagaaa aa                                          2962
<210> 55
   <211> 1360
   <212> DNA
   <213> Homo sapiens
<220>
   <223> nbla24235
<400> 55
   tggcttaaga cctcttttcc tccttatcta ttgactggac tgcggcaaat gcctgctaat 60
   cgttattttt gtgttcattc atttagcaag ccaatttatt acgcgcttag ggtgctgcca 120
   gggctacaaa agctgttgag actgtacttg atatgaagaa gcttgctgat tatcatggga 180
   agactgacat aaggaagcac cataaaattc tgctgtatga gaggtataca cgggatactg 240
   ggggttaata attgagggta tggtccattc aacatgagtg agacagaaac aattcataaa 300
   ggagatgaaa tgtcttgagg aatgagctct tagaagaata gttttcaaat gagtgtgcat 360
   cacagtcacc tgtaagactt attaaaacag atcgctgggc cctacaccca gaggctgtgg 420
   ttcagtaggc tgtagtaaac cagtaatttg tatttctatg acgttcccag gttctaatgc 480
   tgttccccaa ggccacacct tggaaaccac cacattaaaa tacccagaag gcattaattc 540
   ccagtccttc ctctacacag ctgcaaaaca atggtcctga ccatttcatc tttgcactac 600
   atccttcact gtctctcttt tgcccatagg ataagtacaa actagatctg gttactgcct 660
   gccccaccag cctcagcatc tctcacaact aggactaact ttttcttctg acaactataa 720
   aatatttccc ttgccttctc aagtttgctc aaggtcaagt tatgcctttt gcctggaatg 780
   acttgacttc tcttttgttt tacttagctg gctgcttttc atcttgtagg ttaggtcaag 840
   gactccagga agtcttccct ggacaagtaa tgaagagggc ataatccaag ggccaactcc 900
   catgtttgga acctgactcc attttcaggc acgtaatatt gtcaaattcc ttttaaaagc 960
   acctgtctgt ctgttaacgt tggtgcagat actgctattc ccctcctcca taccattgct 1020
   gatggttact gagggtatgg gaagggccga ctagtccagc tgttcacaaa cagcccttaa 1080
   tgtcaaactg aatactgcca acgtagttcc agtttctgta tctaaagact cagcttggag 1140
   tcacttgtct ggactaaaag taacccctcc ttgtctggtt tgtgactttc tgtactctga 1200
   tgcccccagc tttctgcctt ctagaaattt gtcagaattt ccaaaattct tgggccttcc 1260
   ttcttgctct atatatggtt ttggattcat tccttttaaa aaatatttac tgtcatttca 1320
   gtagaatttt gacacaataa atataagcac atcagaaaaa 1360
<210> 56
   <211> 2049
   <212> DNA
   <213> Homo sapiens
<220>
   <223> nbla24556
<400> 56
   ggacaattaa ggtgaaaatt attctatttt aaaggggtag agttcttgag gaagaacacc 60
   tttgtgtgca tgtgtagttt attccttcta caaatattta ttggacaaca gtgttgcgac 120
   agtattctgt gcaggccact ggagatacag tggtggaaaa aactaaactt gttcccagtt 180
   ttaatggaat ttccagtcta gattgggaga taaacattaa gaaagtaatt ccactagtgc 240
   agaattatga tacatattat gcaagaaagt atatatgctc tgggggcttg taatgaagga 300
   acataagttg gtctccagga tgctctctga ggagggggaa attgcaccga gcacaaagga 360
   tgggtaggaa ttaacagggt gaagatggaa gggtgtagcc agctcctgag gtatccaggg 420
   cttttgcctt ttcacagatg gcagtgggtg tataaatgga ctccattttt tctttgtttc 480
   tgactttttg gctgcaatgc caagtggctg ttttctgtct gtgtgttctg tctgtctccc 540
   agaatctcca aagtgttctg ttcatggatg gtatttaata aatggacatt cactggtaga 600
   aagtatttga gagtctatta gaagttaaat ttgtttcaag gcaataaaat tctaaggcat 660
   ttaagagttt tctctgttta aatttttaaa caaattgtgt cttatttttt aacatcctac 720
   taaataatga cattattagg cagctacttt tagataaaat gtgataaata atactttctt 780
   cataaattct gctctaagaa tctgtttata ttttgattta aaatagaaat cttttatgta 840
   atttaaaacc tcattttgaa tggaagtgat atgaatagtt tatgcaattt ctgccaagga 900
   attaatatgg actttgtata aaccactgtc atttataatc aaaatgcttt taacttacat 960
   tgatgttggc attaacaagt attgctagat tggtagcata gaaggaaatt gcatttagac 1020
   ttactaggag ctcattgatg cctgaggttt tataatgctt tctttgggcc atttaactgc 1080
   tggcaacttt aattcacatg attcataatg ctggaaattc aaattcactc ttaactgaaa 1140
   agtgaagtta cttaaattct ttaaatgcta acctttggaa aaatatctga aaaataaagg 1200
   cactgccaaa agattatcat ttacataaat atctctttca gcagaagagt ttaatgtatt 1260
   gagctcagaa ggttagaata gagacttcaa tctggaagcc agcagtagcc tgttggcttg 1320
   tgaacagcag cattgttcat catactgaga acactgttgc attcaggcag aagcagagct 1380
   ggcattaaaa tgcagttaat ttgtttcatg tgacttgtca gctgtgtgtt tttatctaaa 1440
   tctttctagc ttctcttttt agtattttgt gttcaactcc tgcaatagat gaactaccta 1500
   tttaactgtt taagctctga ttttatcacc acttgcaacc attctccagg ttttccattt 1560
   cattttaaat atatttaata atcagtttga acacgatttt aatgtattaa aagtaacccc 1620
   atctcagagg gcttttctgt cttgtgcatg tgtctgtgtc tgtaaaacgg actttctgaa 1680
   gttaattaag ataaaattgc tacccttatt ttctccccag caccctattc tcttcttgtt 1740
   tgctaattgt gttctctggg tttttccctt agatgacttt caatatttgg ctactagcca 1800
   agtattgggt ctgagcagta aagtgctagt cccaaagaaa tgatataact gttactaaca 1860
   ttagaataag gttcccattt cactttttga agggcgtgaa aatcttactg ctcctctgca 1920
   actgtgctca cttagtataa tctaacagtt aatattcttg tttaattgga aggatatatc 1980
   cagtgatttt taaacaactt ttggaggtgt aattgacata caataaactg ccatatttaa 2040
   attgaaaaa 2049
<210> 57
   <211> 1373
   <212> DNA
   <213> Homo sapiens
<220>
   <223> nbla24800
<400> 57
   tgaatttgga tattgatgag gctgaaatgg gatagttcat acagggacct tggatttata 60
   ctgttgcttt tttatggcca tgttaaaagc atctactttc cccatgggag gaaggtgcat 120
   gctgagtgat cctgttgagc tgtcactgct ctgtcaggag attcttgttg atggacatat 180
   gtctgaccac ttgagaattg tgttggagtg aaatacactt gcataagtca attattaatg 240
   acagttcctt tagcaactcc cagagaaggt ggggcatgac ttcttccctg gagctgactt 300
   cagacaaatt cacagatgct aaaccctggc tttttttttt tttaacattt taatttcctc 360
   tcatagaatc atcacaaaat aagaaaacac ttctttatat cgtaatcata attccagtgt 420
   tttcagtttt atttcctttt tccactaaaa tcattcctgt gtttcaatca gtaaagtggg 480
   cttcttgatt tcatttggga tttgtatttg tgtttttgtt ttccattcgt ttatgtttct 540
   ttggttcgta gtgtcagaag acgatgtttt ttatgacaaa ctgccctcgt ttgaaaggcg 600
   ctgtgaaacg cctgcaggta tggtgctagc caagtgatct ctagagacct agattccaaa 660
   aatccaagcc attatccatc tgaatgctat aaacttcatg gacatgccct cacctcatga 720
   gtgtccagtg cctctcagat gcaccctgta tatttactgt tcatcgtgga actcgtgcca 780
   ctgaaaattt ttaagtgact atattcaaaa acagcaggtt gcatgacagt ttctcagtga 840
   agaggttcaa aaaaggtgag atgctattgc tttgtgaatt tacaaaggaa agaataattt 900
   aactgctcag aattacatgt ccggtcactg ctttttaatt taaaaaataa tagagcatca 960
   ttagtaatct tgttttctct ttgatacata ggtaaagggt gttttgtgtc tggatgccta 1020
   aggtgattcc aggggagggg atggaagata tgtgacatct tccctgaaat ttatattgat 1080
   atgcaatgct ttgtcattta aaacctaagc taatgttttc tacaatccat aactctgagt 1140
   ttatcttttt ggaaacatag aaggggatga cattgaagat gaaatggata cagcaattgc 1200
   tgaatgacag tttgcccaaa ttagtgcagt taaaatatgc tgatgcccct gcatggccag 1260
   gaagacttct gctccatgca cacaagcacc aagtatcaag cgaccaccaa cacattccca 1320
   ttcctttagg cctccatagc tttgcttttg ctttctgttt cctgaactaa aaa 1373
<210> 58
   <211> 2192
   <212> DNA
   <213> Homo sapiens
<220>
   <223> nbla20001
<400> 58
   ataataaaaa taaaacaatt tttacaaagt aatgggattc aaagaaagga aaaaaagatt 60
   tttctttttt gtcaaaatat cgatccaatc agattggtaa aaacccccac acaaattaaa 120
   gaggaataat aaaaattgca aaaataaaaa aaaacttttg caaatttttt tatttttcct 180
   tctttctttt atatcatgtg aactaaaaca gtcttctgtt aggggatggg ggcaaggggg 240
   atacctgatg acattaacaa tttaataaca ttaacattgt tgccaaagag gtggtctctt 300
   tgctgaaaat gggtttcaag aaaaatctat ttttataaaa tataaagaat ttttacaaga 360
   gaatctggat ttgagaaaaa aatattttga ctggctaatt taggggaaat tgacaacttt 420
   gtcgcgttca tactgcactg gtaacttttt agagatcaag atgtgtgttt taaactggat 480
   tcgtagactg ttttttgaag gatgggctat aaacagatga tcttcatatc ttttcatagc 540
   atgtaataat aattaaaaaa caattattaa ttactagggg aaaggagtgt tcgttctacc 600
   cagggtacca cagttcccca cagtcaaaac ccaaaagcaa ggagatgagt tgaaagacag 660
   tttttcttta agtcatcagt atgggatgtc agcagaacaa aaattaaaaa gattaatttt 720
   ccttttgatc taaaacttcc ttagtttgag cagtaggtgc tacaaaatta tttacatatc 780
   ttagtatcat agttaaatgt aatgtgttta ggagaggaaa acaaaagata catttgcttt 840
   aaattcatta agaaattttc aaattcactt tgtagcccat gctgatagaa ttgggctgtg 900
   ttggtacatt tgaaacactg tttatgttgc ttgaaacact tatttattta atcgccgatg 960
   tgatgatgcc tatggccgag atcaaatata gctagattgg ctagactact tatttgttta 1020
   cttaaactat gggaagaagc atattattgt gtcattctgt tgtgtgtgta tgtgtatata 1080
   caatataaat atatatatat aaagttattt tttctttggg ttaatttatt ataagttgta 1140
   acacttggct agttttgttt gtatatgtct taaaatgttt tcttatgata tttaagtgac 1200
   agttaaagag gtatcaaggt aacttgtgta gaactattct ttgatatatt gtcatgtttg 1260
   ttgtgaatat tttttcttac tgcacagtag aaaaataaaa acaactgagt ctttatttta 1320
   atgtaactca gattggggaa aacaaaacag agctaaggga acaaaatgac tgagggagca 1380
   ctctcccacg tccagtgcac tgatcatttt agtatgtttg tgctttgtac ggttatatat 1440
   ttaaaacgaa aacaaaacaa aaaaatacaa gggttcatgc tcttccctgg gtaatagaaa 1500
   cagttactcg ctatgcataa tctagttgat agttaaattt gctattgctt ttcttgtctt 1560
   gttatataaa atcttttcaa tacaagttta gtcttaatgg taataaaacg ttatggttat 1620
   ttataacttg tgcttatttt gtgcattttt tcccatgctg aacccactaa gtgcatgtag 1680
   acaggactgt tgttttcaca ctgaaaaggc aaactttgta gtagtcgttg tagtggtaga 1740
   cagataacga ataccaaggc tgcatcatag actcctcctt taaatttttt ttctgttttt 1800
   ttttcctctt ttcggttttg gatataacac cagatttcag ttcagagaac actcgttcaa 1860
   cattcaggga aagcttttta cgtcacctgc tatgaatgaa cgtagtttgc tggcaaagtt 1920
   ttgatgcatt tgctaagcat tagtgggaaa ggcatgccaa aatcttctct ataatgtgtt 1980
   caatcttggg ggaaaaaaaa aggaaaaaaa atcttaggac caggcagttg tatactttag 2040
   ttattaatga atgacttcat gttaatcttg ctagtttaga tgatttccaa gggaaagtat 2100
   tgtaaatgtt tttttttcat aatcttgttg tgtttgaatt atttgtactt tatctgtcca 2160
   gacaataaat gaaagtgtgt agaatggaaa aa                                2192
<210> 59
   <211> 1380
   <212> DNA
   <213> Homo sapiens
<220>
   <223> nbla20083
<400> 59
   atctacaaag ccagatgctc tgtcttcata tttgcagaca tctagacccc ttgctaaaaa 60
   cccactgaag tttttttttt atgttctttg acccacacca tcaacactac cctcaaatct 120
   aattgcccta cagcatattc tatcatgtgg actaggttcc tggaaagccg gaactcatga 180
   ttctttttca aactgccaga atagaaggga gagagaaaac atttctaccc tttgatcacc 240
   agtgtgaaca gaatccggaa tgcagtttca gcgtgacctg cagtcattca tgttcattgg 300
   atttgacaga tggaaaccca aggttatcga agattggaag gttatcattg tgaagaagta 360
   gctcaaagga ctccggtttc tgtctacaag tgtgatgtct ccatgaagaa gacttagtat 420
   ggatttgggt gggtaagaaa gcatttaaac gcccaggaaa ggacatgatt aaagttgacc 480
   ttttaatact gtagtacctt gctgttaagt aaccccacta ttgtatctgc atttatcttt 540
   tgttcatcta ctttcactta catacagtat tatataagta gagaaaaatg ggaaaatgca 600
   agcaaattca actttatttt atacattgta tatatgtaca ccctacacta ttcatttggg 660
   ttttattaaa gagatagtca caaagggctt acgaaaatca tttttgaatt gataattaga 720
   atattgaata agcaatccta tgatccacta atttgtttta tcagttaata atattaatca 780
   aagacattta ctgtatattc tagtcatttt gatttgagtt aaccccaaat ataaaattac 840
   ctgtagtgat gtctctctcc cagcccttat atgtggatat tttttaagtg gacttgtatg 900
   ctgataattc tagaccaaag taaatatggc agaatattta tacatgaaaa aataattttg 960
   caaatatttt ctataattgt attcatttaa aatgttgata gcttgtgtta gtttcaggga 1020
   ggggtgtata ttttgataaa aaaatacttg actttgtaat tctgtatatt ctatacaatt 1080
   tatagcagag ccgttttaag acagccttgt cacatttttt tgttaattgt gaaaatttta 1140
   ttgagtgatg tttaagtatg cattgagtac atgaccaact agaattaaag taagtgtaaa 1200
   cagtgaacat actgtatgct gtacaagata taatgtaact tgctgtttta gcatctgtat 1260
   tttggttaga agatattatt aaatgcagat gttaaggatt ggaaaagtct aattttattt 1320
   ttagaaataa tggatataaa tttgtttttg cttgattaaa atagcttatt cctacaaaaa 1380
<210> 60
   <211> 1833
   <212> DNA
   <213> Homo sapiens
<220>
   <223> nbla20182
<400> 60
   ttgtaaatgc tgggcctcct gtgatttgag tgaggccaac aggacatccc tccccagctc 60
   ccagggccca tgctgtggtg ggactggtgg gtgacccacc tcctctgggc ctctcagtgc 120
   tctgggacta taaaagctga atccccactg gagctggcct gagaggtggg aaatcagctc 180
   cccaccctgc cccagtgttg ggcatctggg acctccaaag gcagagtcca tacccaaagc 240
   accaggaaag gccactacgg tggtgtttgg gcgtggagga tgtgctgtct gggcttaacg 300
   gtcctgtcct cgggaaatga ctatagagca gagattccca gcctaggtca aattccacag 360
   ggatcggagc tactggaatc ctggaggccg acctgggcct gccccatttc ccctaggtgg 420
   tcccaccgcc cttggccact ccaggccctt ggccgagaga gcaggcagca accagggctc 480
   tgtcctccct gcttcctcca aagccaaaat gagagacagg caggtaccca ggcagtgccc 540
   ttggaggtgt ggattccccc gcgcgctcca cccagcttgg cctttgcact cccgaacccc 600
   catggggctc ctctgcccgc cgactcccat tcaggcggga gcaccctgag aagatcctca 660
   tcaggtgcag gggaggggtg cccagtgccc tcacccatcc gcatgcaggg aggtttccca 720
   gatccttggc tctgagccca ctccgaaggc aacccagctg ggcggagacg gaaggctctg 780
   gactctggct gggtgagcag caccagggag gcgggagagg ccgggtgggc ttctctttcc 840
   ctttctgtca gtgcctctcc cccaagagtc tttcgtggcc ttccgcccca ccttgcaact 900
   tgttggaaag ggaaaccggg gttctgagag gggcaggaat tctggagcac ggtggcactg 960
   aggctccccg gcgccctcct ccacccgcct gagggaggcc agcgggctac tcctgcgctg 1020
   gtgctgctgc tgttcctccc cgcctgtgca ctcatatgct tcataccctt cggccaccct 1080
   gcccttctgg tagccagagt gggcatgcct atccagggtc ccgctgggaa gtggggtccc 1140
   agccaccgga aattcgttcg ctgggcctcc tggactcgcc gatccccagg tccccaaggc 1200
   ggatcaccca atgaatgact gccctggagg gaaacggaga ggtggacacc ccttcatagg 1260
   tgggccggag aggggacagc cctgtcctca cagagctaag ctctgcgtgt catgcacgga 1320
   aggacacaca ggatcgggcg ccgagaacag ctaagtggtc gaagagccag cctcaccgcc 1380
   tggggagcaa acggccctcg ccacgttctg gagctgtggg gctgagtttt tgtttatttt 1440
   ttattacaaa agtaatagtg ctttttatta tctggacatt gcagtgaagt tcaaatggaa 1500
   atacgtctgc acttccaaca tcaaaagcca actgcctttg agtgtggatt tactgggaat 1560
   tgtaacttaa gccgtattgt tattttaaaa aaagttatta tcagtgaaaa tgcatttatg 1620
   tattcagtga aaatgtgtct gtgtttgctt tataataagg caacaaaaat aagttagtac 1680
   aaataaaagg aggccaatag agggaactag attggtcacg gtttaagaac tgtgggatag 1740
   gggtgggtac acgggaattc acttgaagcc tccctcgatt ttgttttata tttgaaaact 1800
   tccataataa aatgtttcaa aaagtgacaa aaa 1833
<210> 61
   <211> 1664
   <212> DNA
   <213> Homo sapiens
<220>
   <223> nbla20248
<400> 61
   tttcaagcac catatcagca tgatcagcaa tataagtagt atctcagtgc tttgttgttt 60
   agtcagagtt ttgtactcta tcacccattg taatgttcct atttgcaaaa ggtaatacat 120
   accctttaaa acatctttgc tttttctccc attatcgaga tgctagcagc ttcataaagc 180
   agaataacta agggcaaaca gattatataa agggttggag ctcaatgaag acaacaagaa 240
   cagcaaaggt tattgtaaaa ctggctgctt gcaggccaac aagcacatcc atatggaggc 300
   aatcagttta tgctacctct gtctgtttga tgggattcat aatattgact ttatccatta 360
   gatttggact accagggaat aaaataagca gatggagagt aaggatttgc taggaaataa 420
   ttcagccagt cactttgaaa gctgttcaag aaacagcttt caaagtgtct ctcaaactat 480
   gtttgcccat tatcccaata atttatttcc caataatttc atgggaaaag aaggaagttc 540
   tgtggtcaga taaatctgga aaacactggt ttaagcaaag ttcagtaggt ctgcttccct 600
   gcaggtcacc tcagagtctt tactctgcta acctaggaac tcatccaaca agtttaattt 660
   aacagctaca ctgtgtacgt cactttaaca gtcactgagc tgtgactctt gggggaaaga 720
   ttgtgcgtgt gtgtgtgtgt gtgtacacat gtgtgcacat gtgcagaatc taccaaatct 780
   taagagaaag gaacatgctg ggaaactgtc ctgtgaaaga gaatagaaac ctgaagattt 840
   gaggcagtga tagcatttat gaaagcagca gataaggact aatcaccaaa aggggtagct 900
   cttttgttgg ttggggaaaa caggaatttt tcccccaccc aatgtgctgc attttctaat 960
   tttctatgaa cacttcctaa gaaaaagctg aatgaagaac atttgcgatg caatcagctc 1020
   attaagaaac acgcactttt gtggagatac gtgctgtccc aggagatgct ctgcgaggag 1080
   ccgagtgttt ggactggagc tgctgaatgg tttctcacag ttctagaatg tttggggctg 1140
   caccctctaa gatgttgaac ccatcagtaa ttgctccaaa ccactttatg ggatataatg 1200
   ctgtgagttg acacctgagg ggattgtggt cctgttcatg agtaattact tttctgttgc 1260
   ctatagaagg gccagcaata gcagatgagt agctgaacag tggttttgag taataaaacg 1320
   ttctttttta aaaaaaagta atgctttctg ttaaactctg actatactct ctcctggtat 1380
   cacaacccag ctttcttttt gccttcttta ttgcagttac atatggggct gatgacttta 1440
   gggatttcca tgcaataatt cccaaatctt tctctctgtt ggaattgtga ctatcttctc 1500
   acacaagcgg ctacttggtc ttgatgcctt cccccgcaaa acagcaacca aactgttctg 1560
   ggccaatatc accaccttgt ggtcatgatg aagaattgcc ccctttgccc tcaacacctc 1620
   ttttcttctt gaaaattaaa aacaacccct ttcaccccca aaaa 1664
<210> 62
   <211> 1531
   <212> DNA
   <213> Homo sapiens
<220>
   <223> nbla20250
<400> 62
   ctttaaattt gactcaaatg gaaattgtgc actttcctgt ttataccctt ccccacgtta 60
   ttgtaaaaga gtttaacagc agcctgatat gtaagtttca gcaaaactta tacctgtata 120
   tgtttttatt tgactcaaaa attagatatt ttaccatata gtcataagaa tttgctcact 180
   ttgatgccag aagtacttaa gaagttacac ggcactaatt ttatgagttg tatgcctaat 240
   ttcaatttct aacctatttg acagtttctt ttaggtcagc ctttgttggt cttccatgta 300
   aatacaagtt ggtacaaatc aatagaaacc attttaccta cataggcaaa gtaaatgtgt 360
   gacttagaga ctgccagatt tatggtgcat ctaccttttt atccatttga gcttgctttt 420
   ttatgtttgt gtattggttg ctcctgacac tatacatttc aaaatttttt ataacttgaa 480
   aaacacttct gtgctaccac tcagttctga tcaaatcctt acattttgca acactcattt 540
   ctgaattttc agtaaagaaa tacacattac aaattaaagg ttaaaggccc cttttcatgc 600
   ccttccccag tctcctcttc ctcccccgga agtgtccatt ctgctgaatt caggttcatc 660
   attgccagac aaatgtagta agctagtgtt tcacatttcc aaaatcagcc ttctggcaga 720
   cttggaagta ctcttgagaa aagaagactc gtgaccaaat tctcccacag atttgtaata 780
   atgtacatat tgaaaggact gaaggctctc agactgggaa agaaacttac ccattttaaa 840
   attcagcatt gctcaactta cctgactgcc ggaccccttc acccatgatt ctatgcactg 900
   tattgttgga acatacattg tgaaaacact gccctgccta ggcatacccc ctttccagaa 960
   ttaactttcc atttaattct atagtttttc actgatgtaa ctttctagac tggacaacaa 1020
   agatgactaa tagtaatcac tccaagttga tgttgactgt tgggttgtgg tgaaatcatt 1080
   ttgcattaaa ggaaggtaaa atactaataa attgcatatt ccttgaccag agcacagatt 1140
   acttatgctt cttaattttt taaaatctta aatcctctgt ccaactggag tatctggcta 1200
   tgggccatgg gtactcatat accctttgtc ttaaactgat ctgttacatt ttatgttctt 1260
   gtggctagaa gtagcctgag tttgctgtta atgtttaaca cattttcttg agtaacagtt 1320
   ctgttaatat tgtacaagat ggtacttgaa ttctttgttt gccttttttc ttcctgtatt 1380
   agaaaatctt ggtgcttttt ataagttttg tataaaagaa ttttttttaa gatttgttca 1440
   taaaatggtc tgatccagga aaaataaaat gggaacatgg acaccatttc tgaccttcaa 1500
   ataaaactta ttatgtattg gttttcaaaa a 1531
<210> 63
   <211> 1871
   <212> DNA
   <213> Homo sapiens
<220>
   <223> nbla20330
<400> 63
   gaaatcagag gtatgttgag cagagtaacc tgatgatgga gaagaggaac aactcacttc 60
   agacagccac agaaaacaca caggccaagg tgacagagga gttagcagcg gccactgcac 120
   aggtctctca tctgcagctg aaaatgactg ctcaccaaaa aaaggaaaca gagctgcaga 180
   tgcagctgac agaaagcctg aaggagacag atcttctcag gggccagctc accaaagtgc 240
   aggcaaagct ctcagagctc caagaaacct ctgagcaagc acagtccaaa ttcaaaagtg 300
   aaaagcagaa ccggaaacaa ctggaactca aggtgacatc cctggaggag gaactgactg 360
   accttcgagt tgagaaggag tccttggaaa agaacctctc agaaaggaaa aagaagtcag 420
   ctcaagagcg ttctcaggcc gaggaggaga tagatgaaat tcgcaagtca taccaggagg 480
   aattggacaa acttcgacag ctcttgaaaa agactcgagt gtccacagac caagcagctg 540
   cagagcagct gtctttagta caggctgagc tacagaccca gtgggaagca aaatgtgaac 600
   atttgttggc ctccgccaag gatgagcacc tgcagcagta ccaggaggtg tgcgcacaga 660
   gagatgccta ccagcagaag ctggtacaac ttcaggaaaa gtgtttagcc ctccaggccc 720
   aaatcacagc tctcaccaag caaaatgaac agcacatcaa ggaactagag aagaacaagt 780
   cccagatgtc tggggttgaa gctgctgcat ctgacccctc agagaaggtc aagaagatca 840
   tgaaccaggt gttccagtcc ttacggagag agtttgagct ggaggaatct tacaatggca 900
   ggaccattct gggaaccatc atgaatacga tcaagatggt gactcttcag ctgttaaacc 960
   aacaggagca agagaaggaa gagagcagca gtgaagaaga agaagaaaaa gcagaagagc 1020
   ggccacgaag accttcccag gagcagtcag cctcagccag ttctgggcag cctcaagcac 1080
   ccctgaatag ggagaggcca gagtccccca tggtgccctc agagcaggtg gtcgaggaag 1140
   ctgtcccgtt gcctcctcag gccctcacca cttcccagga tggacacaga aggaaagggg 1200
   actcagaagc tgaggcactc tcagagataa aagatggttc ccttccaccc gaactgtctt 1260
   gcatcccatc ccacagagtt ctagggcccc cgacttcaat tccacctgag cccctaggcc 1320
   ctgtatccat ggactctgag tgtgaggagt cacttgctgc cagcccaatg gcagctaagc 1380
   ccgacaaccc atcaggaaag gtctgtgtca gggaagtagc accagatggc ccactacaag 1440
   aaagctccac aagactgtcc ctgacttcag accccgccag acctggtgaa gaggatcata 1500
   accctgtctt caagaacact gggatttcag cagcaagttg gaagaaggac tggtaggttc 1560
   ccctccaagc cagtcacctg taagagtcct gtcctctgcc agacttttta atctcttcat 1620
   taactctcag actgacctgg gagccctcct ctacctgaat ccagtgctca actgtgcccc 1680
   ggcaacaaga cctgggctga ggtctccctg gtagaactaa gggagattac accatctaaa 1740
   tcccagtgca gtcaacagcc tggcctatag tcctgggaca tgtatcttct tctttgcctt 1800
   aaatctgata caagaggtca atgactttga aaataaaact aaaataaatg tctataatga 1860
   aacttgaaaa a                                                       1871

<210> 64
   <211> 1474
   <212> DNA
   <213> Homo sapiens
<220>
   <223> nbla23983
<400> 64
   taaacattcc ttgtgtatct ttaagcatgc ttctcctgaa atttaactac attagtagtt 60
   gacatttgta tacatatatc ctaatacaag agtaggataa ggtggaaatg taatggcctg 120
   agggatggtg aagcattctt ttagtatttt tcatcatgtt gggctcctag attgtactgg 180
   ggttgcccat aaatcaaacc ccatactctt agaattcatt atattatggt gatatccgaa 240
   cctagtgaat ggtatgcttg ggtgttttcc attgagagtg gatggacctc tttataaagt 300
   tggttgctgc aaaatccagt tcttccaaaa gccactttat ttagggttta ttcacaagtc 360
   atatccattt tggtacagtg tttgtttcct aatatttatt aaccacctta taccaaatgt 420
   cttgcaaaga aatgttatta aaaccttgaa tttttacaaa tgtaaaaaac aaaaagtgta 480
   ttaatgtatt tgttcaggaa aagctacata ccgaagggct tttgtatatg aattctgtgg 540
   tggggagacc catttgtaat ctatatggca gttccatctg ggttttaagt ttagatttca 600
   ccgtgtctta gtgcttcatt ctattggttt attggaacat gtaataaata ggagtagtga 660
   tgtattaaaa cacaagtatt cattaatgtt ttatatcttc actaaaattc tatagttatg 720
   aaactatttt atcaatcaag gtgttatatt tcagtcagaa gtgaaaattt atgaagagta 780
   tttggaagtg tgtacagaaa taaactagac ttacaggtag gctagatcag aacgttaaca 840
   tatgaacctg cagaaatctg gtaagactta aattcagtgt gaggaataac tctagttctc 900
   tcctatgagc atttcctaaa agccatctga tttggcattc ttactggagc tgcagacaga 960
   aatctacaaa gacaaaagta aacaaaatta agttattatt ccactgttag gaatggaaat 1020
   aaacttgtga agtctgttta ttttgaagta ttggtgaact aggcttgcta attgataact 1080
   gcagcagttt gtgtttactc cagttcatca gcttaggtca tttgaaagat ataagagctt 1140
   aaggcaagaa agaaataaca tggaattcta tttgaaggac aacagaacat tcttggaaaa 1200
   gcagctccag ttggtttttc aactgtcaaa cttgaatgtg taagtcccca cagagcatgg 1260
   acagtcggtg cagagttcca aggaaacaat tattgcctga tgaccacttc cattttgtat 1320
   acactctttg gttcgtatag gccatattcc aactggcttt ttagtaatag aaatccagta 1380
   tataatgtat caaatacaat tgaggttcta acctagtgtg ttaatttatc tgaatttgga 1440
   tttttaaaaa gtaataaaaa gttaaatgta aaaa                             1474
<210> 65
   <211> 2167
   <212> DNA
   <213> Homo sapiens
<220>
   <223> nbla24111
<400> 65
   cttataaaaa ttttgaagcc catccccatg gatttattat aatacagctc tgatatatct 60
   taaagttaac ccgttttccg tagatgttaa gggctttact ggttgaggta acctatttca 120
   aatggtctgt tgggttttgt ggtaccttgt caagaattca ataagaattc tcaggctgtc 180
   tgtattttct tttaggactt tggagatctc tcaagttgta ctgttttcta gtattcctga 240
   gtatattcct ttggtaacgt gaaagtaaat agtttatatt tgatgatttt tttttttttt 300
   ttttgagacg gagtcttgct ctgtcaccca ggctggagta cagtggtgag atcacggctc 360
   actgcaagct ctgcctcccg ggttcacgcc attctcctgc ctaagcctcc tgagtagctg 420
   ggactacagg cgcccgccac cacacccggc gaattttttg tatttttagt agagatgggg 480
   tttcaccgtg ttagtccagg tggtctcaat ctcctgaacc ttgtgatcca cctaccttgg 540
   cctcccaaag tgctgggatt acaggcgtga gccacagtgc ccggcctgta tttggtgata 600
   ttttaaaaaa ttctactttg accttaagtg cttcaagaat tgtgttcagt tagtagtcct 660
   tttgtaagac taactttcat atgctatctt tgctccatga gctatcatag tactgttttc 720
   tttcattacc cgtaagagtg gctctatcac agcatttact gttaagggct acagttagac 780
   ctcttgttaa ctctactttt atttgtgatg gctgtgtttc acactacctt gatttataaa 840
   tgtagtaatg tgttaaataa ctatatgttg tggtccctta atacctcttt tgattggtga 900
   ggtaacagtg atgtggatga tgaaataaaa acgtttcccc aagtcactaa acacagtttt 960
   caattcattt tttttttaca tatttaattt acatctaact actgttaggt atgcagcccg 1020
   ttcctttttg ccttcagtag aatatagtta tataagtagt ctcatttaga ttcttgggac 1080
   agaacggcct gtgtattgat ctttctttaa tggcttggaa cagcttctat atattctgac 1140
   aggtcttgga agcatgttaa tatccgtgtg tttaattgtc atcttcctgc ctgggaaggc 1200
   agtagaagaa agaatctaca tttgtatagt ctgtagtaca ggctctgtgc tgattgcaag 1260
   gcactcttga gagaaattca ttcttatttt gcagaagaag aactgaaact tcattaagtc 1320
   attaagcaac ttgctcaggt ggtggaactg agctttaaat atggactttt tccagtctca 1380
   attcagcatt atactaggct gcctccatgt gtttttcaaa gccccattca agttttactt 1440
   ctatggtaaa ctaattttac atacacaaat cttttcattt tctgaacttc ctttatggct 1500
   ttactgtcac cccactagta tttgatgtct tagctattaa ctaattcctg attatttcac 1560
   ttgtcacatc aggaacccta tcctcttagt tctcccattg agatttcact gctggactaa 1620
   gattattctt gattcgtagt cattggtttc tgtttccatt cattttcagc actgattatg 1680
   ttaatcgtat tgcttgagtt ttttctttgt tcaatgttgt ttattacatt cattttgttt 1740
   catatacaca catttttttt tttttaactg gcattttgag gatattggtt taatggaagg 1800
   aaaaaggaat ggtgcaaagc acatggtatt tgaattccaa agaccttgac cctcagcatt 1860
   agcaagtcac ttgttttctg agcctcagtt ttcttactct caaatgaggt aatatccgaa 1920
   agtactttga caacacacta aagcctgatg cagatttcct ttttgaagta attgtgctgt 1980
   ttctattcat attggatatg gtattctatg gtattggcta tagatacata cattttaaaa 2040
   tgttatttaa cagcatgtaa atgttcattt catgccatgt gatcatgttc ccctttatga 2100
   ttttttaagg ctgtcttaca agcctaacag tgtactaagt cattaaaaga tatatttaaa 2160
   gtaaaaa                                                           2167
<210> 66
   <211> 1388
   <212> DNA
   <213> Homo sapiens
<220>
   <223> nbla24142
<400> 66
   gtgcttttta accaaataaa agaagaacca gctcttggga tatgtgactc tgcctctgta 60
   taaagtgact ggaattttgt taaaaccgtg tttccacttc tgaaccctgt taccattccc 120
   cctcacaaat ccccacccaa cacctggatt ttaaagatcc tccagtgtca agggaagcca 180
   cagagtctat taaagaggca gttctgaacc aattaatttt tgtccttata atttagagca 240
   ttaaatagct aatatattta atggcactaa ttgttgttca cggctttcat catactttta 300
   aacagaatcc aaagtattca aaggaaagta agcgaagtta tccaaagcca actttgtttc 360
   aggtgtgtcc cctgccccaa atagatttta gggcagaaat agaaaactga gtttacacag 420
   aactattttt ggaaaagctg cactggagta gatggattct tcttcagcat acttttttgt 480
   ttgtttgttt gagatggagt cttgctttgt cacccaggct ggagtgcagt ggtgtgatct 540
   ccactcactg caacctccac ctcccagctt caagtgattc tcctgcctca accttccaag 600
   tagcttggat tacaggcgtg cgccaccaca gctggctaat atttgtattg ttagtagaga 660
   cagggtttca ccatgttgtc caggcttgtc gaacttctga cctcacgtga tccacctgcc 720
   tcagcctccc aaagtgctag attataggcg tgaaccactg cgcccggcca gcatgcattt 780
   ttaaagtggc ttagatttag ttttaaatat tttggggtga aaggcaggaa cagttctgtt 840
   tttgacatac aggttttctt tgggattgtt ttcattttca agtatagatt catgtcagaa 900
   tggccaactt aacgtgggtt tctgtattcc ctggtgttgc tcttaacctg aactcataat 960
   cagttgccat actgaggcaa gagcactcag ggtgaacata gtcaagttac tttaaaagtg 1020
   ataaaagtgt ttttccatgg tgaaaccttc agtatttggc tgaatgtaaa gtatgttgaa 1080
   gtggtatatt gatggtaagt tgttaatcac taaccttgtt tgcacttttg tacaccactg 1140
   cttgcactag gatcttggtg tgaattttca attgttttac agtgtataca gattattaag 1200
   gataatttat ataaagatgt ttctgtttaa ctttgtgtgt tttacaacaa agagctataa 1260
   tagatggtta aacgtttttg aattgtgttt atatgttagt ttgattatgt tctattatct 1320
   tttcacctgc catgaatttg agtgttagga agggaaaaat aaaatactaa tctggtcttg 1380
   aagaaaaa                                                          1388
<210> 67
   <211> 2357
   <212> DNA
   <213> Homo sapiens
<220>
   <223> nbla24157
<400> 67
   aaaaaaaaaa atacttgtct gaagatgtat gcaagctaaa attagtttat acttcctgta 60
   ttctgggaac attcagtttc atggtatctc ttaatttcaa gtgtaatttt acttacaata 120
   aaatactcat tttctttgat accattctta ttttgtattt tataattatt ttcagttact 180
   aatggaaaat atgacctatg tgccataatt taatattata tttctacttt ctaatgttaa 240
   gaacttaaac atatctaaat ctaaatctat ctatctatct atctatctat ctatctatct 300
   atctattaga tatctgtcta tgtaatctag ctatctgggc atatattatt tgccttgaca 360
   ttgtttcaga aaaggacaaa attaatgttt agaagttttg acctggcttc tatatggagg 420
   caccttcaga taatttaatt aaattagata acatgtacta aatctatact gagcctagtt 480
   taacaactaa attgttctaa aactacattc tcatgtctcc atttgcttta cttgttcttc 540
   agatttatag cttgactaca tttactagtt aatcctttct aattatatgt ttgtgtttat 600
   catgtagcgc atggtagaaa gaaagcaagt aaaagaaaaa gcaaaaaata acatcagtaa 660
   tactttaaat gcatacagta gttaataaaa agattttatt gttaacttca cgtcataggt 720
   tagcagaata gactctggag gtataggttt ggatttgtat tttatcactt actaaattga 780
   tgaccttatt tacgttatat attacttatt gtaaaagaaa tgtaatctgg aaaactaaat 840
   agcataatta aattcgggat ggcagcagga tagaatactg ttaaataact gcactgcaac 900
   aatttagtga atctcacaag atctttataa tcccttttcc aagaaaaact gccatttaat 960
   aaatgttata catgatttta tttaataaat aaaaactgaa ggaaagataa cctaaatcta 1020
   tttttttaaa caccagcaat ctgtaacatc cctagaaaat tgtctagaac acagcattcc 1080
   taccttataa cgaaactgta tctcttgcaa gcaacaagaa atttctgttt ataattttct 1140
   aattcctagg gctcagaaca ttgcttatta tagagatatg caaaaaagta tttgttgttt 1200
   aatgaacagt cacttaaata ctgctatcct ctgcagtctg catgaaatca cataataaga 1260
   ccatgattgt tcttatgtcc aagtcaatac ttcattggtc taactgcatc agcttgtctg 1320
   caggggattt ctggaggttt gggggcttgt ttcatgtatt ttcaataacc aatttatcac 1380
   ttgttgttct actctggaac cctgttttct tggctatgtt gtgtttgcta tatgtgtgac 1440
   acaaagatgt cactgcttta ctaagcatgg cagttttaat gatgactgtc actctgaact 1500
   tagggcaatg gtgtaagtct tcctgtttta ttttgctttg tttgtttttc ttttgtttgt 1560
   ttgtttgttt gtttgtttgg cttcctctgt agcctaggct ggagtgcagt ggcacgatct 1620
   cggctggctt gctgcgacct ctgcctcccg ggttcaagca attctgcctc agcctcccaa 1680
   gtagctggga ttacaggcac ctatcaccac acccagctaa tttatttttt atttttattt 1740
   tttattttta tttttttttt agtgggggca gggtttcact gtgttggcca ggctggtttc 1800
   gaactcctga cctcaggtga tccacccgcc tcggcctctc agagtgctga gattaaaggc 1860
   gtgagccact gcacctggcc tttttgttgt ttttatgtca ttttcttgtg cacttaatta 1920
   atacatagtt tagttaaact gaattaaatt atctaaaact ggttaaggta attacctttt 1980
   ccataacttc taacagcaca accacaccca atctgtaact tttagcattg gttgaatgaa 2040
   aaatttagaa taatgcatgg ccaggcatgg tggctcatgc ctgtaatccc agcactttgg 2100
   gaggcagagg cgggtggatc acttgaggtc agttgttaag agatcagctt ggccaacaca 2160
   gtgaaaaccc atctccatta aaatacaaaa caaaacaaaa caaaaattag ccaggcatgc 2220
   tggcatactt gtggtcccag ctacttggga ggctgaggca ggaggatcgc ttgaacccgg 2280
   gaagcaaagg ttgcagtgag ccaagatctg ccactgcaca ccagcctggg tgacagagca 2340
   agactacatc tcaaaaa                                                2357
<210> 68
   <211> 1522
   <212> DNA
   <213> Homo sapiens
<220>
   <223> nbla24230
<400> 68
   tttgggcttt tgttgggcac tgtgtgtctc ccatgttccc catttgtctg ccacccaata 60
   agcatggtgt cgagggctga agtagaaatc agaggctaga atctgaaagc ttcattaggg 120
   ttctgctttt tgcagattag ggactttggc ccttagtgag ctgaggatct tggtttcctc 180
   ccagtgtgcg gtttcaggga tgtcggccac atgatgtgcc tgttgtggag gagggctggg 240
   tcgccagtgt gacaggagac agcagatccc ttttgtgaaa ggagaactgg tactttgcgt 300
   gatgttaaac ttcacaaacc gctgctcaga aatctgctat tttccttctc ttttaggact 360
   ttatggacag cagcctgcta accaagtcat cattcgagag cgctatcgag acaacgacag 420
   cgacctggca ctgggcatgc tggcaggagc agccacgggc atggccttag ggtctctatt 480
   ttgggtcttc taggggcctc aaggtcttga tgtgcatagc ttctgataac cctgtgtgca 540
   ataatatgat ttgcagggca tttctgtttg tgacaaaagt ttttaataat agttttaatc 600
   attcctttga aagtagtgat gtcataattg tactaatcca cataagtacc acagagaagg 660
   gtttgaactg tgctattttg ttcaaatgtt gactctccgg gggcactggc tcattccaag 720
   actgttcttg tgcaactctc agaatacctt atttgagcat acctgttttg aaaggcattt 780
   tctttttaga gttaggtgta gtgcttaagg gttaatttat tttcatgtta tgccagtaat 840
   atagtgttgt atgcctattg agtgattgtg gcaagaaaag ctacagcttc tttgcgttta 900
   actttttcaa accacagacc agaactggtt gcatgttact ttaggagttg tgggttggta 960
   agctcccagg tacttcccga ggctatggtg tgagagcccc cgtcctgccc tctggggctc 1020
   cacaggcccc tggcaaggcc gatggctcag gatgatgggg cacagcccgc ctttgaacaa 1080
   tcatgcttca gaaatctgcc tgaccctagc tgctgctgct gctcacttta ttcttgtatg 1140
   gctttggtag gcatacttgg agaacatatc ccacattagg aattgattta agcctgagag 1200
   tttgagggct ttaatccttt aaaacttgga gaagctggct gggcgcggtg gctcacgcct 1260
   gtaatcccag cactttgaga gaccgaggcg ggcggatcac gaggtcagga gatcgagacc 1320
   atcctggcta acacggtgaa accccatctc tactaaaaat acaaaaaatt agctgggcgt 1380
   ggtggcaggc gcctgtggtc ccagctactc gggaggctga ggcaggagaa tagtgtgaac 1440
   ccgggaggcg gagcttgcag tgagccaaga tagtgccact gcacttcagc ctgggtgaca 1500
   gagtgagact ctgtctcaaa aa                                          1522
<210> 69
   <211> 2098
   <212> DNA
   <213> Homo sapiens
<220>
   <223> nbla20541
<400> 69
   aaaaaagtaa gcaaaccaat acctggtgaa tctatggaca gtcatacaca tacatcaggg 60
   gaaaatgtgt gtgtacaacc caaatttaca gtatgattgt cattctttga ctttgttttg 120
   tatagcctga ctctgttgaa catgaaatta ttagtactct aggttttgga cagcttgagt 180
   tcatttgaat tccttcctta ggaataagtt tttatataca ctgctaaatg tgtgatgaga 240
   atcataaaac actaaccagc tgaggtagct gtgattcact ttccccccac cctaacttga 300
   gataaaatga aggactaggc aagtatttca tgttgtgtga gtggacttcg gttccttcag 360
   tattgtctag gttattgagt ctttctttgc ctaatagtgg attcccactc ttaagataac 420
   ttttattagt gataaatcag tttagggtat attctgtatg acaggcataa aatgttaagg 480
   gtgaatgctg gccttttcca agaaaaggcc accttaactt gtatgaggaa aaaatcctaa 540
   ctattctctt tttttgtatc tttttttccg taactgtttt gattgtatat tttaaagaaa 600
   ccacttaatt tgtgatgcac gtaatatttg tgtgaacctg agaatatgtc acaataggaa 660
   aaagcagaaa ttatacttag gggacatgtt aggggggtaa aaatatttaa gcctcgaatg 720
   ttttactgtc atctccacta actattttta cagaaaaagc taaaaactct gttgtaatta 780
   ttgtaagttt acttatttat acttttaaat taggcttttc atacttaaat ttttttgaca 840
   tttgctttta atatttgttt cttaatgtgg aaattgtgta ttttaataat caaattatta 900
   ggataataga tatattttta aacattcacc tcattaacaa atagatcttt gaatttttat 960
   taggtttttt ggctccagac aactgtttag ctttaatgat atttctaaat tcccagtgac 1020
   ttattaataa aaacaggaaa aatatttagg taatgtcata aaatttattt tacctttctc 1080
   attttctgag aaaataaatg aaaaaaaccc tagatattgc tttattacca acagtgtgta 1140
   ggtttttgta catatggaaa tttgacacaa aaaaataggg aatttgtata gagaagtttc 1200
   cctcttataa aaggactccc atttgattgt tcgaaactat aaaatgcact tttactttac 1260
   catatctgaa atgacaaaat atcgcccttt ggaaaacctg actctttgca cgtgtaattc 1320
   ccagagtcta cctcagttaa ccaggcttag ttttaggcag gaatgaattg aattaaattc 1380
   agttcatcat ctatgcagat ttgtttcttt taagcacatc cttccctcct gctgttgccc 1440
   tcctcccatt aacttttctt tttaatcttg aaattgttta aaatattcca tctttctttc 1500
   tctagcaaag tgtttgtatt ccaaataagg cctctgtgaa atgtctgaat tacttttccc 1560
   gtctttgtta tggtcagctt cattatttgg atgtattgca ttcaaagcag cagttccaaa 1620
   cataacacac atctattttc ttagagtttt gtaaatacag actaacctga tgacattaaa 1680
   aattgtggat cctacatgtt cctatgttca ttctctaaaa acctgagtaa ctttatgaaa 1740
   acacacaaac ctggaaaaac atcacatttt tgtcacattt ttactgacaa atgtatattc 1800
   atatgatggt acggcagcag ggagtggccc ccagttaaca tggctgtgag tggacacagt 1860
   gtctcgcagg atcactgcat gttatgatgg cttgtaagtg cgttgttaag acttttgttt 1920
   cagtgtttgt ctcccagtat ttgaacctaa tttaaagaaa aagacgtttc caagttgtat 1980
   ttattaaatg tgtttttcct taccttttgt gctgctactt tgctaatctc attagcttag 2040
   ctgtgtttgt gcataggtta tatttggtaa taaatttata gagtgttggt tgtaaaaa   2098
<210> 70
   <211> 1332
   <212> DNA
   <213> Homo sapiens
<220>
   <223> nbla20555
<400> 70
   tgggaacgac aggaattgcc ctctgcagta aatgacgttt attgctgaca ggcaagggga 60
   aacatctcgc ccaccatact ggcaaccaca ggcctactgg ctattaagta tgtgcgttcc 120
   gtggtcagct ggtcctggtt tctgctttct ggggacttca tagctgttag cccatgggca 180
   gttgatgtcc ccagtctgag ttttgtttac ttcctgtgta aagagtagtc cctctatatt 240
   aataccatga tgatgtttgt actcattacc catcccctag cacacactct ctccctttca 300
   gtcacttagc aagcactcaa taagttcagc aaatatttgc tgggtaccta ttgtgtgctg 360
   catacttttg tagggacaag gtatgcagtg attaataaaa tagagaattt ccagtattgt 420
   gttgtgatga aaacaaaact gatgtggtgg ggccagcata ctgagaggcc gaggtgggag 480
   ggtcgcttga ggcaaggaga ccagcctggg caacaaagtg agacctcatc tctacaaaaa 540
   aaaaaaaaaa ttaaaaattg gccatgagtg gtggcatgct agttgggagc ctgacccagg 600
   gggttcactg gagcccagtt caaggctgca gggagctatg atggtgccac tgcactccag 660
   cctgggtgac agagtgagac cccatctcca aacaaaaaac aacctaggct gggccgggcg 720
   cgggggctca cacctgtaat cccagcgctt tgggaggctg aggtgggtgg aacacttcag 780
   atcaggagta cgagaccatc ctggccaacg tgctgaaaca ctctctctac taaaaataca 840
   acaacagccg ggcgcagtgg ctcatgcctg taatcccagc actttgggaa gccgaggcgg 900
   gcggatcacg aggtcaggag atcgagacca tcctgactaa cccggtgaaa ccccgtctct 960
   actaaaaata caaaaaaatt agccgggtgt ggtggcgggc gcctgtggcc ccagctgctt 1020
   gggaggctga ggcaggagaa tggcgtgagc cattcgggag gtggagcttg ctgtgagccg 1080
   ggatcgcgcc actgcactcc aaaatccagc ctgggcgaca gagcaagact ctgtatcaaa 1140
   aaaacaaaca aacaaaacaa caacaacaac aacaaaatta gttagacgtg gtggtgcatg 1200
   cttgtagtcc tagctgcttg ggaggctgag gcaggagaat cacttgaacc tggaggtgga 1260
   ggttgcagtg agatggaggt gcagtggcac tgcacactcc agcctgggtg acagagcaag 1320
   actccaaaaa aa                                                      1332
<210> 71
   <211> 2014
   <212> DNA
   <213> Homo sapiens
<220>
   <223> nbla20645
<400> 71
   gtgcagacac acatgcaaga tacctgtgag gctgagcctc aagggggtct ccaggtacct 60
   agatgacagt tgcgtgactt ggcacagcgc tgaatatgga ggcaaagccc tgggttgact 120
   gagaacacca aaggcctttg cagctgttgc ctcacttact ctcatcccct tgttttctgg 180
   tgctggcctt ccttggagct tcttaactgg aattttattt ctgatgacca ctgggccagc 240
   tgcaccattg atcatataca ggctcccttg ctatatgcat cgtgtcacct ccaagaaagg 300
   ggccgggcag cagggcactg gggtatgttt ttagagcgta gcctttggtg tggggtggca 360
   ctaagggaac acaaaagtgt tgttgaggat gtatcccacc atggatcatg tcatcccata 420
   gggttcaggt tcaagacagc tcaagagcgg gtcctccctc cctcccactc tcaaggggat 480
   ttaagataca ggtgttcgtc ccggtgcctt gcattttgca aatagaaagc tcaggctgga 540
   ctctgcacgg gagcaggagg agtgcacaga gaagtttgag agcctgggtc tcttctagca 600
   tcatggtttc atgccatgtt cttcaaaacc cacggagaag gttctgcatg tttgccccta 660
   gtgtcacttt ttaaacttaa tttaactatt gtagaaactg ttaggaaaac ccgccttgct 720
   gtcaaccttt cactcatgtg ggtggcagaa aggagctttt gagtgtggtc ttggccaaat 780
   gggaacccct tgggggccac cggtgctttg cttcaggctg ctgggtagtt ttgtgctgat 840
   ctcaggctgc tgctgctgca tctgccttgt ccgcagtggt caagaactgg gaggaaactg 900
   ctctcctttg ctttctttat gcatgtaaca ggattttctc aacactgtgt caccaaagca 960
   aaacacagaa ataatttggt ggctaaggct gtaactagcc ttcataacct tatctgtaaa 1020
   actttgattc actcagtctc atttttggct ttttattggg tcaaagatac acattttaac 1080
   tcataaagga agagtatact aataacccat tactgctatc cgtttgacgt attgagatcc 1140
   acaagagatt taatttcgag agggagagga agggttctgc tgctaagtcg aaaaatcaaa 1200
   gaagttagaa aaacactgat ctaccgagta gagcactgtg ctcaggatta aagacctgga 1260
   ttctcaccta gttttgccag ggaccagctg tgtgatctta ggcaaatcac atcacttctc 1320
   tgggtctgta aaatggggag gttgaactgg taagatcttt tttaccttga aattctataa 1380
   atgtttctaa ctccatttcc ttcttacttg acttttccag cagcacttta tcctttaaag 1440
   atctgtggtc atcactgacc tcagagccct tgcctctaga ttatcttacc ctgaaatact 1500
   taggttttaa ctctgtggat ctggaacact tcaagagcca gattgtttga aactttaatg 1560
   gggtataccc ctgcttcagc ttaacattat tttcaaacca acaaacatgt cccgcaaaca 1620
   catatattta aatgacatga catctgtgtg ggctggagtg tttttcccgc ctcagcggca 1680
   gccatactac tacaccagtc cagatctgtt tgcagagctg ccgtgttgtg cagtccagag 1740
   gtgctgctgc tgttgtattc tgcatggagg tagtcaacaa gacagccctg cttaattatg 1800
   aaatgtctgt agcaccctgt gtacgaaggt gtatagaagt gtatagaaag cacccaaaag 1860
   agcagcagct tggctgggcg tggtggctca cacctgtaat ctcagcactt tgggaggcca 1920
   aggtgggcgg atcacttgag gtggacggat cacctgaggt caggagttcg agaccagcct 1980
   ggccaacatg gtgaaacccc gtctctacta aaaa                              2014
<210> 72
   <211> 1753
   <212> DNA
   <213> homo sapiens
<220>
   <223> nbla20713
<400> 72
   ttcagaagcc ttggaggaga ggcactgctg agctggaggc cgagagcctc tggccgagag 60
   gcccaggccg aaacagaggc tccttcgccc tatttttcct agatgtggat ctaggattgc 120
   taatgaaaac agagaaacca gacttagcgc cgactccagc tcccgcccct acatctggag 180
   taagagaaaa ggccccccgc tcctccataa acgactcgaa aacgggcggt tgtttataaa 240
   cttgtggatc cggttgttga gcgctgcagc gccgaggcct ccccgccggc tagggtagcg 300
   ctaaccttgg tagcttctct gcaggggctg ggactccccc atcgtatcct ttcctctctg 360
   gttcactgtc tcctccggcg caggaagctc cgggttggtg tggaaccagg tatcctctct 420
   gaatttctct ttccactttt ctcgccctcg cctttcctct gtccagaacg aaatcttgaa 480
   aagcacagtg agcagcaacg acaagaaaac caaaggccgg acgggctggc cgcagcacgt 540
   ctgggccctg gagctcaagc agtgaggagg aggagaagga ggaggaggag agcgcgagtg 600
   agcaggggcc aaggcgccag atgcagaccc aggactccgg aaaagccgtc cgcgctccgc 660
   tctgaggact ccttgcattt ggaatcatcc ggtttattta tgtgcaattt ccttcccctc 720
   tctttgaccc cctttgaggc atctgctccc cgtctccccc tccaaaaaaa agtggatatt 780
   tgaagaaaag cattccatat tttaatacga agaggacact cccgtgtggt aagggatccc 840
   gtcgtctcat agattctgtg tgcgtgaatg ttccctcttg gctgtgtaga caccagcgtt 900
   gccccccgcc aacctactca accccttcca gataaagaca gtgggcacta gtgcgtttgt 960
   gaagtgtatc tttaatactt ggcctttgga tataaatatt cctgggtatt ataaagtttt 1020
   atttcaaagc agaaaacagg gccgctaaca tttccgttgg ggtcggtatc tagtgctatc 1080
   cattcatctg tggtcgttcc ctctttgaag atgtttccaa cagccacttg ttttgtgcac 1140
   ttccgtcctc taaaactaaa tggaatttaa ttaatattga aggtgtaaac gttgtaagta 1200
   ttcaataaac cactgtgttt tttttttaca aaaaccttaa tcttttaatg gctgatacct 1260
   caaaagagtt ttgaaaacaa agctgttata cttgttttcg taatatttaa aatattcaga 1320
   agtaaactaa attatcatga ttgcctctaa ctttatttta aagactcagt ggttccaacc 1380
   agtcaccctg acctgcggcc tacgcaggag gaggaggtgc tcttaaagag aagtgtcctt 1440
   gttacaaatc ctgcaaatgg tctggggttt gtcggtgtgg tgtctccttc cctcttcccc 1500
   cagctggaga acgctgagta gtctctagaa ggaagatctg ggctggagaa cccagtccgg 1560
   cagttcgctc agaaggtgta aaggtgctct tgctttcctg aagtcaatca gaagccattt 1620
   cttgaggccg tcagtttttg tttggagagt gtttctggtg gaggagttgt gaggagaacc 1680
   ccggcattat tgctgcaacg ggaactagtc tggggtgttt aattcaaact atggggcttt 1740
   catccaagaa aaa                                                    1753
<210> 73
   <211> 1769
   <212> DNA
   <213> Homo sapiens
<220>
   <223> nbla24250
<400> 73
   ggggaggatt tttgaaattt tatctctaaa aacagttttc caattcagag tttttaaaac 60
   ccttttaaaa atatagttag ttttcagtgg tttcttttac ttttagtgtt tttacacttg 120
   gaagtcagat atctaaaaat agggaatgtt cttttgctat ttttagatct ctactaaaat 180
   gtaatctgta gtgttttctt gtttcagagc atatcttaaa agattcagac aagtggcatt 240
   tggggacctc ttccccatcc actggctttc actcaaagga aaataagact tcttggttct 300
   ggcagatact gtctctggca gaattggtct cactgttttc cttggggagc attttaggta 360
   gtatgttgaa agacagatat acatcagttg aagacaggat cagatgctat ctggttaata 420
   aagcttatga tcagggaagg ggcaaagaag acagatacca ctaccatttt gttctttctg 480
   gttttactaa tatgaccata atgagtcatt ttttatgcat ataggctatg tgtttcaggt 540
   tgcctttcct tttcctccta cagatctatt gagctttgtg ttctaaacaa gatagtgtgc 600
   ttatctgaat gtttcccatc tgtctttgat gaaaaagctc ccagttaaac taatttggat 660
   ttatttattt ttcctgtcta ttccagttct ctgctatgtg tgggcaagtg cctgttttat 720
   cttgaggggt agattttagc atttgaactc tctccctttt taaaatcacc ttgttactta 780
   cagatcatct cagtccagta acttttcttt ataaaggtta aaagattgtt tgctttcttc 840
   tcaggtagtc tcagtgttct cagccttgag agggaaaggg acatacttaa tattttcttg 900
   tcttgcttgc taagagctgt ttttccttcg tcatgtgttg ggcagggcta gccacccatc 960
   tgttggacca gctacttcat aaaactttca aaggatgata gtaggtgaaa tgaaattgac 1020
   aagagtgttg gatgcaggta gaatgaaggg tctgctgtag cgtgtatgtg gacttctttc 1080
   ttttgtttat gttcgtaaaa gtggagagac tctggatata gaaagggtaa tagcaaactg 1140
   atatctccag tacctgtctc ctatatgatc aaaaacatta acaatgtgtt ggttttgtaa 1200
   aattgctact gttttgttct gaagtgctgt agccattagc tggattgtaa cagtaatatg 1260
   acagctgtat agtaaaatac tgtctctctt tatgatagga aatgaaaaag catctgttat 1320
   gaagcctcag tgaactaaaa gccattctct gaaaagtcaa gacttttggg ctttatcagt 1380
   agataaacat gagccatagg ttttctagca atagaatatt ttaacctata tgaatatatg 1440
   ctttataggt gagactgcta tttaatgaga gttttaaagt aactaaacct tgttgacaga 1500
   attcaggatg gaaagtttta ccctaaataa aacttcagga tattgaatat gatagcaaag 1560
   ttccagggta tgttttatat ttatgaacaa ttttcatttg aatatttgga gcttgggggt 1620
   tttggtgaga catgttcatg tatgttatat acaaactttc aggcctggca tggtggctca 1680
   cacctgtaat cccaacactt caggaggctg aggcaggagg atcgcttaag cccaagagtt 1740
   caagacccat ctctacaaaa aattaaaaa 1769
<210> 74
   <211> 1819
   <212> DNA
   <213> Homo sapiens
<220>
   <223> nbla24254
<400> 74
   agctaaactt ggtgcctgaa gaagagaatg aattattgca gcttagttct tcatatacat 60
   tgaagaatga ttatgaaacc ttaagtttat cagcattttg gatgaaggta aaggaagact 120
   ttccattgtt aagtagaaag agtgtcctgc tattgctacc attcacaaca actagtttgt 180
   gtgaactagg gttttccatc ttaacgcagt taaaaacaaa ggaaagaaat gggctgaatt 240
   gtgcagcagt tatgcgggta gcattatctt cctgtgttcc agactggaat gaacttatga 300
   acaggcaagc acacccatca tagtaaataa aaatcttacc tagcttttgt ctttgtattt 360
   cttattttgt agtatttttc tatgttatat ttaaatggta ctataatact gtgatacttt 420
   tgttatgttt taatttttgt tatatttaat aaaattattt tatgttcatt gaacaaaaat 480
   ttaatgaatt tctgttagag gccaggaact attctagaga catttgggat acaaaagtga 540
   acaaaacagg taattcccta gtagagttta tatcctggca aggagaaatt gacaataaac 600
   ctaataaata aggtttataa tatttagaag ctattaagtg ctatggaaag agtagtaaga 660
   aggaaggtca gggaagtact ggggaaccaa accatgaagg gttctgtaga ccattattgg 720
   gcctctggct tttgtcagtg gactagagaa cagttgaagg gtttaagcga aggagagaaa 780
   tgatctgagc taggttttaa aagacactct ggtcactatt ttaaattctt agggtaagtc 840
   tgaattaaat gttactttcc cctcactggg catggtggct cagacctgta atccccgcac 900
   tttggcaggc catggcagaa ggctctgttg agcccaggag ttcaagacca tcctgggcaa 960
   catagtgaga ccttatttct actaaaaata ttttaaaaat aagtcaggtg tggtggtgca 1020
   cacctatagt cccagctact caggaggctg tggcaggagg gtcgcttgac ctcaggagtt 1080
   tgaagttgca gtcatctatg attgcaccac tgcagtccag cctgagcaac agagtgaaac 1140
   cctgtctcga gaaaattaaa tgttacttcc ctaaaaaaac cttttctaac caccctaggg 1200
   taaatcctcc attattcctt tatttctttg ttttccttgt atataatttg taataatttt 1260
   gattactgat tgtcattctg ccaccctgga gtatataatt tttaattatc tgattactgt 1320
   tattcttcca tagtagggga ggtgatatcc atttgcctga tacatagtat gtgttcaata 1380
   cacatttgct aaagaataaa tgaatcaata atacctaaca tctctaattt gcagtcattc 1440
   ccaagagtaa ttattaaata tgtggcaaat ttctttgcct ttttactttt aaaaatctaa 1500
   ttttgacata actgctgtaa ccatccagaa acggcattga tgttgcttca cgttgctgat 1560
   gcttaagcaa tgtatattgt gtaatataca atgtagtctt caaactaatt tcaacttctg 1620
   cctttctgtg tactccctta tcccactggg tgatattatt tggcatggtc attgtcatta 1680
   aaatcataca ggatagtaat tcctttccat ctgctaccat gcctagcctt atttaatttt 1740
   tcagattttc tgttctattg aaggtaattg attttttctt tttttttaat gcttgaaata 1800
   aagtgttgaa aaacaaaaa                                             1819
<210> 75
   <211> 2512
   <212> DNA
   <213> Homo sapiens
<220>
   <223> nbla24327
<400> 75
   atgctttaga tcaagggtta gcaaaccact gcttgtttgg gccacggcct gtttttgtat 60
   agtgtgggag ctaagagtgg gtggtgtaca tttttaaagg gttatgacaa cgacacagaa 120
   taatatgaga cacaaaccct atgtggccca taaaacctaa aattctactg tctagctctt 180
   cacagagaaa gtttgctgat ccccgcttta gataatgggg gtgctctact actccccttt 240
   tcatttatag tgttacataa gcctaaataa tcactgtagc tggtggcatc atgtttgtta 300
   cctactaagt aggtcaaagt gattgccaga catacacatg aaggccttga attagaaagc 360
   aaaggaactg atgatgacca atgtttaaca aaattcagac tgactttgtg cctgatcctt 420
   caaaggctag aggtgatatt tttggtacct gaaacgtaat ttccctgata agtactcttt 480
   gcccaattat tgcttatcag ctgagatatt aatgtctgaa ttattcagct catatatctt 540
   caagcactca actagttcat actttgaaat caattctaat agacaattct cataacacct 600
   ttatagtctt cccatttaaa aggtaaatgt tgttagggct ggaggggtaa gatgcaccct 660
   tggtatattg tctgatctca gcagaatcaa ctacttggta gtgtagtcca gagaaaatgg 720
   gtcaaatcta ttaattattt taggattttg aaattcataa ttgagactcg tgacttaata 780
   gtgaactgct catggtactt tacccagtct tcaagttgta tgccttttgt aggtaggcat 840
   ttagatggga tgcttttgaa agcataatta agaaacttta cttgaatttt gtttataatg 900
   ggctaatgtt attttcttat agtttgcagt gttgatgtgg gtatttacct atgttggtgc 960
   cttgtttaat ggtctgacac tactgatttt gggtaagtct acaaagccat tgggatgaaa 1020
   aattgctgga aagattgtgt gccaggagct tagacatttt agtggagaat attctcattg 1080
   tatgaaaagt aggggatgaa aatgtgggcc gggcgcggta gctcatgcct gtaatcccag 1140
   cactttggga ggccaaggtg ggcggatcac ctggggccag gagttcgaga ccagcctggc 1200
   taacatggtg aaaccccatt tctactaaaa atagaaaaaa ttagctgggc gtggtggcgc 1260
   acgcctgtaa tcccagctac tccggaggct gaggcaggag aatcacttga gcccaagagg 1320
   cagaggttgc agtgagcgga gatcgtgcca ttgcactcca ggttgggcaa caagagtgaa 1380
   actccatctc aaaataagtt tgaggttgta ttctctttaa ataagttggt gatactgctt 1440
   cccggtttat tgaaatgcta ccttagttgc tgaagacagc tcctactaac aaacagtgat 1500
   aaaccagata aagggtggct ttatatgatg gtgcagtcat aaatctaacc agggatacct 1560
   ttattttatg aaatctcact gtgatatgat ttgaagctag aaatggttcc tagctctaat 1620
   aactgcagcc tcacacagtt cattcattcc tctggagtgg ctcctcaaca gcagatgcat 1680
   ccagagatcc ttatgttttt attcattcat taggaacact gcttggttat cttgagttgc 1740
   cagtttaata gttttttgag tgtttattcc tcccaaatca ttccattctt tttgaaaagt 1800
   tgtatatttc ccttttcagc tctcatttca ctcttcagtg ttcctgttat ttatgaacgg 1860
   catcaggtaa tttcctaact aactgctgac ttcagaatag agcactcact ctattacatg 1920
   ggatttacgg atgtattagt gcccattttc aatgtcttac aaaaatgaga agtgtgatgg 1980
   tttcttaagc ctttagcttg acacatagta gtggttaata agcttcttta gcaacggtaa 2040
   taattccttt atacctctct ttcaggcaca gatagatcat tatctaggac ttgcaaataa 2100
   gaatgttaaa gatgctatgg ctaaaatcca agcaaaaatc cctggattga agcgcaaagc 2160
   tgaatgaaaa cgcccaaaat aattagtagg agttcatctt taaaggggat attcatttga 2220
   ttatacgggg gagggtcagg gaagaacgaa ccttgacgtt gcagtgcagt ttcacagatc 2280
   gttgttagat ctttattttt agccatgcac tgttgtgagg aaaaattacc tgtcttgact 2340
   gccatgtgtt catcatctta agtattgtaa gctgctatgt atggatttaa accgtaatca 2400
   tatctttttc ctatctgagg cactggtgga ataaaaaacc tgtatatttt actttgttgc 2460
   agatagtctt gccgcatctt ggcaagttgc agagatggtg gagctagaaa aa         2512
<210> 76
   <211> 1564
   <212> DNA
   <213> Homo sapiens
<220>
   <223> nbla24510
<400> 76
   ttatcgatac acagcctctc tgagctggag cgtctgaagc tgcaagagac tgcttaccac 60
   gaactcgtgg ccagacattt cctctccgaa ttcaaacctg acagagctct gcctattgac 120
   cgtccgaaca ccttggataa gtggtttctg attttgagag gacagcagag ggctgtatca 180
   cacaagacat ttggcattag cctggaagag gtcctggtga acgagtttac ccgccgcaag 240
   catcttgaac tgacagccac gatgcaggtt gaagaagcca ccggtcaggc tgcgggccgt 300
   cgtcggggaa acgtggtgcg aagggtgttt ggccgcatcc ggcgcttttt cagtcgcagg 360
   cggaatgagc ccaccttgcc ccgggagttc actcgccgtg ggcgtcgagg tgcagtgtct 420
   gtggatagtc tggctgagct ggaagacgga gccctgctgc tgcagaccct gcagctttca 480
   aaaatttcct ttccaattgg ccaacgactt ctgggatcca aaaggaagat gagtctcaat 540
   ccgattgcga aacaaatccc ccaggttgtt gaggcttgct gccaattcat tgaaaaacat 600
   ggcttaagcg cagtggggat ttttaccctt gaatactccg tgcagcgagt gcgtcagctc 660
   cgtgaagaat ttgatcaagg tctggatgta gtgctggatg acaatcagaa tgtgcatgat 720
   gtggctgcac tcctcaagga gtttttccgt gacatgaagg attctctgct gccagatgat 780
   ctgtacatgt cattcctcct gacagcaact ttaaagcccc aggatcagct ttctgccctg 840
   cagttgctgg tctacctgat gccaccctac cctcctccag agagctcagt tggaaaggcc 900
   ctcaagaggc atgctagaac gttaggtcag cctactgaca gctgacaaac aattaatgcg 960
   aaatcatgtc acaccaaccc atagccgtgt ccacgcagca actccaccac cttaggattt 1020
   ccccctccaa attattcaga ccaatggctt gccaaatggc ctctcccaaa attctgtaca 1080
   gttttgctca ggtcacgcca acagggaaac ctcaagtgta ggtctaatta gtgtttctgg 1140
   gatccaaagt tagaggaaaa tttagatttt attgcctgga tctgctttaa agacaattgg 1200
   tgtttacacc ctcttgtcag caaaacagct agttaggtaa ggacatatag ttccaagtag 1260
   gtaaagtcac ttgattacaa atgttcttaa ctatcgtctc tgtaattcct ttatacagga 1320
   cagtacaaaa ttgtgggaca tgctctggta acacacagat atgggttgca tatgatccag 1380
   aattacagct gatattatgg atgacaactg ctaaggtcca taaaatgaag actgtattgt 1440
   attgagggat agaaattgat catttaatgg gtaacaactg ctgagctcaa agatttgtga 1500
   ttgttaaaac ttctctggca tttaatcatt aataaacatc tgtattgtga cagcagcata 1560
   aaaa 1564
<210> 77
   <211> 1666
   <212> DNA
   <213> Homo sapiens
<220>
   <223> nbla24554
<400> 77
   aattttttat aatcctcaat tatgaaccac cttgtttata ggacaaaaaa atttaaccaa 60
   ttttattgaa acgaatttca ctgtgtaaaa gttggtttga ttcaaacatg tagagaagtt 120
   gtagattcaa gatatatgat ttctctatgg aaataaaaat atttgttagt gaattggttg 180
   agttttgatt cctctaactt ctcagaatga ttctttagaa ttctataatt catagcaatt 240
   tttgacaagt aagattgcaa aatagaaata tctataaaga ttccacagtt tgacattatg 300
   gcttgctatg cagatgtgaa aataggttaa ataatatgaa agatatggca gaatgtaaag 360
   tggaaaagat gacctaaaat tttgagttgt attaatagtt aaaaacattt gtgtcagatg 420
   acagggtggg cttttactgt caagacatga ataagaactg atctggctgc ctgatgagtg 480
   tttccacgca gccctgcata tttagtgacc aaggcatcaa ggacatcccg aaactggaaa 540
   ttcatatcca tctggtatga atatataact cagctggcaa atgaatgtgt ttgttgagat 600
   attacagtaa taaaacactt aagaacagga agattacatt tgttggcata cgaaacctta 660
   gtggctacag aagaaagttg accttgtgtc actatttatt ttatgccctg atcagactag 720
   caacttagat aagtgaaagt ttttctaaca tgccttaaaa atattatggt ttgatccaaa 780
   gacccacttt ttctttagct cttgtgataa gattttcttt tttttacttt tatacaaagg 840
   cagcatcttt gaattttttt ttcttttgat gttgcaactt ttgggttctt ttaaactgtg 900
   atagtgatgg taactgatgc ctttcatttt gttcaactta cacaaaacaa gccagcatct 960
   gatcaaaagt attacataaa atattttctt aaactattga aaggtgcttt gatgattttc 1020
   tcctttggtt tgtagaatta ggactgaact tttgactcaa attgctacag ttgccatcac 1080
   ctttctgtgg taatactact gatatttgct tttctatata aagaaatgtt gcctaaggct 1140
   gtctggtatt tcttttcaag ggttttccag tatgaatgtt aatgttgtca gtgtatgtat 1200
   gaatatgaaa gtgctttgtt ttgtttgttg ctgttttttg tttatgtgtg tgtttttaat 1260
   ttttttgttc ttatcagcag tcttgtgtta gcactgggta agctttaatt gtcccttagc 1320
   caatcaaaca ttaaggacta tggaggtctt tttttttttt tatttaacat gtcattgttc 1380
   atctattaaa tcttgatcag ggtttcaaga atgactgcag tgggttttgg aaacagactt 1440
   atcattattg atttgaggtt tcccagagat atagttcaca gttaattgtt gcgctctaat 1500
   acaactgacc atttaaaatt gaacaagttt attgttttgt aacaatgtca gttgttaaac 1560
   cttgacattt caattaaaac atgaattgta gttataactc aatgcaaatt caacagttgt 1620
   atttggagtt aaattatttt aacaaataaa tttatttaat gaaaaa 1666
<210> 78
   <211> 1374
   <212> DNA
   <213> Homo sapiens
<220>
   <223> nbla24604
<400> 78
   attagctgtt actgcagcac agacctgctt gtgtgtgcca ttccccagat ggtaggatat 60
   aagcttgact tgagaccagc gatggtcagt aacaggcttt gaagtggcag gattgcgtta 120
   ggtgtgctgc tgtgatctgg tgctgctttg accttgaaag caggataacg catgcactta 180
   cttacccccg cattacttgg gtaccttaag gactagtggt tcacaactta ctttaggagc 240
   ttttatttat tctgtaacac gtgagatctg gtaagacagt ggggggtaag gaaaacagac 300
   aagaccatga ctcttctttc cctcttcccc aaaacgtgcc tcttggaata atcttcagtg 360
   tgccctccag cagagccgaa atcaggcagg catagactcc ctcctctctc atcaaaccgc 420
   agaaatagag ttccttcatc ataaccgcaa agcttcctcc tcccctttgc accctgcctc 480
   agctgcattt tcttgtcgct tctacatggg agtgcttgct gttctgggaa gagtggggag 540
   aagcggtggg aatccttgag ccaattgaaa ctgaggtcat cttcaggaaa accatgtctt 600
   cctgaagttg aaagattcag gcacaccata cagtcctttc ctcatgaata atcttgttct 660
   ttactcatgg gaaattggga gaggttaacc cctcccaagt ttatgtttgc aaattcatgt 720
   ttatgggtcc aggtgaaaaa cttttctgaa cacagcatgc tacttctctt attacctctc 780
   tctatttaaa gaatggctag gctgagcatg gtggctcaca cctgtaatcc cagcactttg 840
   ggaggctgac atggcaggat tgcctgagcc cagcagttca tgactaagca acatatggag 900
   attctgtcta tataaaaaag taaaaaatta actgggtgtg gaagtgcata cgtctagtcc 960
   caagctactt gggaggctga ggcaggagga gttggaggct gcagtgagac gtgattgtgc 1020
   cgctgtatcc agcctgggtg acagaaaaag aagagaccct tcctttaaaa aaaaaaaaaa 1080
   aaaaaaagcc gggcgtggtg gctcacgtct gtaatcccag cactttggga ggccaaggcg 1140
   ggcggatcac ctgaggtcag gagttcttga gaccagcctg gccaacacgg caaaaccctg 1200
   tctctactaa aatacaaaaa ttaactgggc atggtggtgc acacctacaa tcccagctac 1260
   tctggaggct gagacaggag aatcgcttga acccaggagg caggggttgc agttaggtag 1320
   gatcgtacca ctgcactcca gcctgagtaa tagagtgaga ctccatctca aaaa 1374
<210> 79
   <211> 2478
   <212> DNA
   <213> Homo sapiens
<220>
   <223> nbla21037
<400> 79
   aaaaaaaaaa aaaaaaaaaa aaaaaaaaaa aaaaaaaaaa aaaagaagcg agggctcggg 60
   atcgacggcc gcggggcgcc gacgaggagt gcaggactca ggaagggcga gtgcgcggcg 120
   acagagcccg gggaaggagg cagggcaagg ccgggcttgg gggcaggtgg tccgggcatc 180
   cagccttgaa gatgcacaag aggaaaggac ccccgggacc cccgggcaga ggcgccgcgg 240
   ccgcccgcca gctgggcctg ctggttgacc tctccccaga tggcctgatg atccctgagg 300
   acggggctaa cgatgaagaa ctggaggctg agttcttggc tttggtcggg ggccagcccc 360
   cagccctgga gaagctcaaa ggcaaaggtc ccttgccgat ggaggccatt gagaagatgg 420
   ccagcctgtg catgagagac ccggatgagg atgaggagga ggggacggat gaggacgact 480
   tggaggctga tgatgacctg ctggcggagc taaatgaggt ccttggagag gagcagaagg 540
   cttcagagac cccacctcct gtggcccagc cgaagcctga ggcccctcat ccggggctgg 600
   agaccacctt gcaggagagg ctggcgctct atcagacagc aattgaaagc gccagacaag 660
   ctggagacag cgccaagatg cggcgctacg atcgggggct taaaacactg gaaaacctgc 720
   tcgcctccat ccgtaagggc aatgccattg acgaagcgga catcccgccg ccagtggcca 780
   taggaaaagg cccggcgtcc acgcctacct acagccctgc acccacccag ccggccccta 840
   gaatcgcgtc agccccagag cccagggtca ccctggaggg accttctgcc accgccccag 900
   cctcatctcc aggcttggct aagccccaga tgcccccagg tccctgcagc cctggccctc 960
   tggcccagtt gcagagccgc cagcgcgact acaagctggc tgccctccac gccaagcagc 1020
   agggagatac cactgctgcc gctagacact tccgcgtggc taagagcttt gatgctgtct 1080
   tggaggccct gagccggggt gagcccgtgg acctctcctg cctgccccct ccacccgacc 1140
   agctgccccc agacccaccg tcaccaccgt cgcagcctcc gacccccgct acggcgccct 1200
   ccacaacaga ggtgccccca cccccgagga ccctgctgga ggcgctggag cagcggatgg 1260
   agcggtacca ggtggccgca gcccaggcca agagcaaggg ggaccagcgg aaagctcgaa 1320
   tgcacgagcg catcgtcaag caataccaag atgccatccg agcccacaag gctggccgag 1380
   ccgtggatgt cgctgaattg cccgtgcccc caggtaggcc ttgcccctgt aggcctcgcc 1440
   ccagtaggcc ccgcccccgt aggccccgcc cccagaggcc ccgccgctgg caggctgtgc 1500
   cccaagctcc tgttcctcca gcctctgagc cttggcagat gctattactc cccatagcac 1560
   aggctcaggg agctgaatac aacatattca agggttttgt aaacttgtta atcagtggga 1620
   gcttgacatt ggacatgatg tgtctgcact gtagaaattg gcaaaccggc tggacgaggt 1680
   ggtcatgtct gtaatcccag cactttggga ggctgaggtg ggaaaatcac ttgaggccag 1740
   gagttcaaga ccagcttggg caacgtggca agaccccgtg gctacaagaa atttaaaaat 1800
   tagcctggtg tggtggtgca cacctgcagt cccactctag atcatgccac tgtactccag 1860
   cctgggcaac agagcgagat cctgtctcaa aaaaaaaaaa aaattaatta attaaaaaaa 1920
   gtaaaggccc aagactctat aggtgggaga ggaatctgca tctccaccat aatggtgtga 1980
   gttggtctcc atcctgacac acaataacca ggcctcgact ggccacccag gcttcccccc 2040
   aatccagggc ctggaggcca ccaagcccac ccagcagagt ctggtgggtg tcctggagac 2100
   tgccatgaag ctggccaacc aggatgaagg cccagaggat gaagaggatg aggtgcctaa 2160
   gaaggtttga gggttggggc cgggcgcagt ggctcacacc tgtagtccca gcactttggg 2220
   aatccaagat gggaggatcg cttgaggcca ggagtttgag accatcctgg gccacacagt 2280
   gagacccccg tctctacaaa aaaatttttt aaaattagcc aggcatggtg ggactcacct 2340
   gtagtccctg ctacttggga gactgaggtg ggaggatcac ctgaactaag gagttcaagg 2400
   ctgcagtgag ccatggtcat gccactgtac gccagtctgg gtgacagagc aagacctcat 2460
   ctccaagaca attaaaaa                                               2478
<210> 80
   <211> 1337
   <212> DNA
   <213> Homo sapiens
<220>
   <223> nbla21161
<400> 80
   taagggaaat tgtcattaat gagtcaagaa actgctcatt tatggtaaga ggaatacagc 60
   ggcgctggca gcccaacagt gctgggatat catttttagg ttgccttagc tgcttgagtg 120
   agacaagttt ctttctgtgg tggtggattg tggcagaaaa aaaaaaatca tgcatgactg 180
   ggagactcgc ctgcctgatt cttgagataa tatattgaga atctgttgct ttacaaatgt 240
   cacatcactg atgtagcggt cagcccctca ctctgaaaga tgaattgtac tattggaaat 300
   gcgataataa ggttgacttt tcccaacaat aggattctgc ctttgtcttt agagaaaagg 360
   cctctgagga catttgtgca tttgtttgag gattctgttg aaagacttta aagtggaggt 420
   ttgtggaaaa gtgatcaata tacaaaatgc atgaattttt agcctagcaa aaccagctag 480
   ttatttatac tgtatataca gctactattt tggaaaagtg gccagaatac cttttaatat 540
   acctaatgtt aatttatggt tcaataagtg tactgaggtt agtatggatg ggagaaaagg 600
   gtttttaaaa tttttatctt ttataacctc cagagaaatc taagtaaata ttttgttcca 660
   agtgagctgt ttttatttgt gtttgtcagc attgtcttaa tgtttacttt tcacaatatt 720
   ttaatattgg tgaaattgca ctcagagttt atgttgttga tttggggcac acatacctac 780
   tctgtgtata tatgctgaac catttagaac actttaacct gtgaattcac cctcagtaca 840
   cagttcaaca gatactgtag tactattgtg actcacagga cattttatac atttgctaaa 900
   gaaattactt taaaagttta cttaactgag tattgttcca ccttaaggaa ttatagtttt 960
   aacatttgta cttttctatt tcatgtattt tcatttctaa tagctgaacg tattcatact 1020
   caagtctaat ggattatgca gtgtacccaa cacatattgt tttatgatgt atctgtattt 1080
   tctgaagtgt gaatatatat gtatgtttat atgtgtgtgt tcatgaaaca gcatcttgga 1140
   cagaatagtt ttaatcttga aaaatgtaag gttattttct ttcctaataa tttttcacaa 1200
   acccatttat tcttggactt tgaaacccag aaatatagct tattttttgg tctgtatgtc 1260
   tactctgcct agttctgtct cactgtcaac tctagtcaaa gattaaagat tacattgaat 1320
   tttgtatttg gtaaaaa                                                1337
<210> 81
   <211> 3268
   <212> DNA
   <213> Homo sapiens
<220>
   <223> nbla21170
<400> 81
   atttgggtca gcagaaacgg cacgattgag cagcactgtg actataggat catggatcag 60
   aggctgcttc ctctttggtt ctgggcatca gcctcatgtc cactcaaagt aagtggcccc 120
   tctgattgga atcggaggtg cctgggtcat ctcacagagc caaacaaata caattagcta 180
   ttgcaaagcc ttttgggaat tattcccagt gtaaataaac acataaccat atagcaagag 240
   ccttgataaa gtccaaaaac atgcaaactt ggagtatcta agagaaaaga ccacaatgta 300
   aatgaaaaac caaataaact cgggcaaacc ataggatagg gccctgtctg tgatggcctg 360
   catatgatga gccatagaaa aaggatggtg aattctggat aataagaaat gtcaatgaga 420
   tggaagaacc acctgtttta tgtaaagctc caaataacca gatcacagtg gacagccact 480
   caaataatgc cttcataata cagagtatta ttgagaataa ctcaattcac agagagctta 540
   aggcagccaa tatttgatag cctgtcagaa aaaaacagaa cagtaattat agaaaagaat 600
   catatcctcg gaaaaacaaa aattaatcaa actaagtttg taaagtctat cttacagaca 660
   cattgtctgg actggtcctc tcaaaaatac ggtttttttt taatgccaat ttgtttagtt 720
   aatgattttt gtcttattac ttcaaaactg gaaatatcct atgactcata atatcttaca 780
   acctttctac tttcttaaag aatctcaagt ttataatcac aggggatcgg attatttttc 840
   aaaaattaaa tggtgatgta atgatttctg tgtctattgt agaaaagtca accttattac 900
   agctgcaaca atggcattaa gaaatatgag taattccaat caacttgaga taatgtctaa 960
   tcaaacacaa atacaactgg taaatttcat taaatagcat ggagattaaa ttaaaacact 1020
   attatgtaat aaaaaccttt agtggtacta aaattttaga atagttcaga tatacagaaa 1080
   aatttcaaag atacacagag ttcccatttt tttcctatta ctaacctctc atatttgtca 1140
   caactaatga atattcaata gagtattatt aactaaagcc tatacattta tttagatttc 1200
   cttagttttt agctaacatt ctttttcttt gttccaggat cccatccggg ccaccacatt 1260
   gaatttattt gtcattttag gtacctcttg gctgtgagtt tcttagactt tccttgtttt 1320
   tggtgaccct gacagtttga gggagtacta gtcagtcagt tatttttgca gaatgcccta 1380
   aatttgagtt tggctgatgt ttttcttagg gtttgactgg ggttatgggt tttggggagg 1440
   aagaccacag aggtgaagta ccattctcac caaattatat taaaggtaca taccatcagc 1500
   atgccttata ctattgatgt gaactttgat tgcctggctg tggtagtgtt tgtcatgttt 1560
   cttcactgta aagttactct tctcatcacc cacttttctg tactgtactc tttggaagaa 1620
   agtcactata tgcatcccaa atttaaggag tgggaagtta tgctccaccc atttgtaagc 1680
   agaaaatcta cataatttgt ttggcattct tctgcatagg aaaattatct cactctccca 1740
   gttatttatt tatttgatct ttttttatat cagtatggac tcatgggtat ttcttttata 1800
   ctttgggtta taatccaata ctaacacaat aaagaaattt ttaatggaga tgcattcaaa 1860
   ttcgttgcta aaatgggcct gacacctctt gaccttggct aaacagagat tctggatgga 1920
   gcaaagcact gtgacgtcat gtggactttg aaggttaaga aactacggat catcaggaca 1980
   tatttgctct tccatctcac agagaaaatg gggatatacc tcctcattcc aggaaacttt 2040
   cttcctatat ttctaatata tccaggataa aattcaatat atatagtcag tagcttcaaa 2100
   gttaagcata atttgtttac tagaattctt aaggcagatg ttggatcatt aactcattct 2160
   cttagaaata actttggtgc ttataagtag gcatcacata atctgataca ctgatattat 2220
   atatataatc gtgaaaaaca tatcggatta tatgatatca cataatctga tatatgtgat 2280
   atataatcag attatgtggt atcatataat ctgatatata aatgtttttc ataattatac 2340
   atatatttca agtataattg tgaaaaacat ttgccagttt aaagtttaat atgtagacag 2400
   aataatgcct ggaggtatag ggatataatt gggaattaga gtaataaaat aaatatttta 2460
   agtacttact acatattact cattaacaca aaagtaactt tacgtataaa atgcatgaca 2520
   agactccatt ataaagaagt gtctgaaagc tatagggcag aaaggtatag aacacagtat 2580
   agactagaag gagataaaga caatcagaag attttattca ttcatttatt caacaaaaat 2640
   ttacagagta cctccaatta tcagcagctg tgctgaagat taggtatatt acctacacag 2700
   ttacaaattt tgctttcatg tagtctgcag gaagagagac attaatcaaa gaatggcact 2760
   attgacactt gtgcaggaaa gggttacgtc aacaggcctg ggctgctcaa accttgcgta 2820
   ttcccagagt ctcaagactg gtcttggcct ggctcctggg aagattactt ctgagccctt 2880
   ggctgagata ggagtttatg ccaacagtgt gatttatggc aaacacctgt ttttgtatgc 2940
   ctgaggcttt ggatcatgct gtaccaattt gatctgaggc ctgaagactg gtagctaagg 3000
   tgctgcatgc ctacatgact gacctccagt aaaaaccctg gacacatgcc tcaagtgagt 3060
   ttcgttggtt ggcaacactt tacatatgtt gtcacacgtt gttgctgaga aaattaagtg 3120
   tactccatgt aatggcactg ggagaggaca actggaagct ggtgcttaat ttctcctcta 3180
   ctccacgcta tccacctttt cgcttcgctg agttttttct gtatcctttc aatgtaataa 3240
   actttaacca tgagtataac agcaaaaa                                     3268
<210> 82
   <211> 1304
   <212> DNA
   <213> Homo sapiens
<220>
   <223> nbla21198
<400> 82
   gataagcaga gctgtttcct ctggggaagg gagggaggtg gggtgcgggt gcggagggct 60
   cgcgctgctg ggcacccatg gacctcagcc acggcgggcc cagggacgga cctccaggag 120
   gcctgctggg ggaacaggtg cggggcatca ctggggctgg aggccggggt gctggggccc 180
   ccataccctt ggcctggatc aggcctcaga ggagccattc ctgtccatct gagcctgctc 240
   tgggcctccc gggacactgc ctttccacct tgctctgcag atccagcctc catcccacca 300
   cttctccccc gagcagcggg ccctgctcta cgaggacgca ctctacactg tcttgcaccg 360
   cctgggtcat cctgagccca accatgtgac ggaggcctct gagctgctgc gatacctgca 420
   ggaggccttc cacgtggagc ccgaggagca ccagcagaca ctgcagcggg tcagggagct 480
   tgagaagcca atattttgtc tgaaggcaac agtgaaacag gccaagggca ttctgggcaa 540
   agatgtcagt gggttcagcg acccctactg cctgctgggc attgagcagg gggtaggtgt 600
   gccagggggc agccccgggt cccggcatcg gcagaaggct gtggtgaggc acaccatccc 660
   cgaggaggag acccaccgca cgcaggtcat cacccagaca ctcaaccccg tctgggacga 720
   gaccttcatc ctggagtttg aggacatcac caatgcgagc tttcatctgg acatgtggga 780
   cctggacact gtggagtctg tccgacagaa gcttggggag ctcacggatc tgcatgggct 840
   tcgcaggatc tttaaagagg cccggaagga caaaggccag gacgactttc tggggaacgt 900
   ggttctgagg ctgcaggacc tgcgctgccg agaggaccag tggtaccccc tggaaccccg 960
   cactgagacc tacccagacc gaggccagtg ccacctccag ttccaactca tccataagcg 1020
   gagagccact tcggccagcc gctcgcagcc gagctacacc gtgcacctcc acctcctgca 1080
   gcagcttgtg tcccacgagg tcacccagca cgaggcggga agcacctcct gggacgggtc 1140
   gctgagtccc caggctgcca ccgtcctctt tctgcacgcc acacagaaag gacagtttgg 1200
   ctgctgtgtc tgctgcgcac gccccctccc cggacagcac ctgccaccta gaaactttct 1260
   tagcaaaaaa attaataaaa acaaatccat tgtcctctta aaaa 1304
<210> 83
   <211> 1656
   <212> DNA
   <213> Homo sapiens
<220>
   <223> nbla21298
<400> 83
   gatggacagt tggtcccagg caaccgtatg acttccacta acttggcctt ggtgtttgga 60
   tctgctctcc tgaaaaaagg aaagtttggc aagagagagt ccaggaaaac aaagctgggg 120
   attgatcact atgttgcttc tgtcaatgtg gtccgtgcca tgattgataa ctgggatgtc 180
   ctcttccagg tgcctcccca tattcagagg caggttgcta agcgcgtgtg gaagtccagc 240
   ccggaagcac ttgattttat cagacgcagg aacttgagga agatccagag tgcacgcata 300
   aagatggaag aggatgcact actttctgat ccagtggaaa cctctgctga agcccgggct 360
   gctgtccttg ctcaaagcaa gccttctgat gaaggttcct ctgaggagcc agctgtgcct 420
   tccggcactg cccgttccca tgacgatgag gaaggagcgg gtaaccctcc cattccggag 480
   caagaccgcc cattgctccg tgtgccccgg gagaaggagg ccaaaactgg cgtcagctac 540
   ttctttcctt agatgttttt ccttctataa ggtgccagac aggggaaaag ggtgggggta 600
   catctgggat gtcacaggaa acattaagga gagagttgaa ggtaaagatc tgaaggtaag 660
   aaggagttcc acctgatgct cgggtcagga tgagaattcc aaacacactg ccagcccctt 720
   cactggggat gcttggtctc ttctgctggt aaaagcagag atgtttctgt gtcatgccca 780
   agctccccgg tgctaccttg cctttctctt ttacccctga tcttggcttt ctctctctct 840
   ctgcagactt tcctttaatt gatgtgacat ttgtggtaaa cacctttccc agggaacctc 900
   acaaatcttg agatgctttc ccttccccag atgggattgc atgatttccc tgactttcct 960
   accctcctcc agagagctca gttggaaagg ccctcaagag gcatgctaga acgttaggtc 1020
   agcctactga cagctgacaa acaattaatg cgaaatcatg tcacaccaac ccatagccgt 1080
   gtccacgcag caactccacc accttaggat ttccccctcc aaattattca gaccaatggc 1140
   ttgccaaatg gcctctccca aaattctgta cagttttgct caggtcacgc caacagggaa 1200
   acctcaagtg taggtctaat tagtgtttct gggatccaaa gttagaggaa aatttagatt 1260
   ttattgcctg gatctgcttt aaagacaatt ggtgtttaca ccctcttgtc agcaaaacag 1320
   ctagttaggt aaggacatat agttccaagt aggtaaagtc acttgattac aaatgttctt 1380
   aactatcgtc tctgtaattc ctttatacag gacagtacaa aattgtggga catgctctgg 1440
   taacacacag atatgggttg catatgatcc agaattacag ctgatattat ggatgacaac 1500
   tgctaaggtc cataaaatga agactgtatt gtattgaggg atagaaattg atcatttaat 1560
   gggtaacaac tgctgagctc aaagatttgt gattgttaaa acttctctgg catttaatca 1620
   ttaataaaca tctgtattgt gacagcagca taaaaa 1656
<210> 84
   <211> 1800
   <212> DNA
   <213> Homo sapiens
<220>
   <223> nbla21379
<400> 84
   gcagctgcac cgtcctcctc cgccgccagt cgtccgccgc catggacgtg taccccccgc 60
   gccggcaggg gctgccccgc gctcggtccc ctggcggctc cagccgcggg tcaccctccg 120
   tcagctgcag tcgacttcgg caggttcaga gcatcctgac ccagagcagc aagtctcggc 180
   cggatgggat cctctgcatc ctaggaatcg atagcaggta caatgaaggc tgcagagagc 240
   tggcaaatta tcttctattt ggtttgtaca atcagaatac cagtgatttt gagaaaacgg 300
   gattttctga agaagtacta gatgatgtaa ttatattgat taaatcggat agcgtccatc 360
   tgtactgtaa tcctgtaaac tttcgctatc tcttacctta tgtggcacat tggagaaatc 420
   tgcatttcca ctgcatgacc gaaaatgagt atgaagatga agaagccgca gaagaattta 480
   aaattaccag ctttgtggac atggttcgag actgtagtag aattggcatt ccttacagct 540
   cccaaggtca cttgcagata tttgatatgt ttgtggtgga gaaatggcca attgtacagg 600
   cctttgcact tgagggcatt ggaggggatg gattttttac catgaaatat gagttgcagg 660
   atgtgagttt gaatctatgg aatgtctaca gcaagatgga tcctatgtct ctggagagtt 720
   tgctttcaga tgatttggtg gcttttgaac atcagtggac tagcttcttc gctaattttg 780
   acacagaaat tcctttcctg ctagaacttt cagaatctca ggcgggtgag ccattcagaa 840
   gttatttcag tcatggaatg atctctagcc atataactga aaacagccct aaccggcagc 900
   catttgttct ctttggtaat cactccacac gagaaaacct gaatgctggc aactttaact 960
   tcccttctga aggacatctg gtacgaagca ctggtcccgg cgggagcttt gccaagcaca 1020
   tggtagccca gtgtgtctca ccaaagggac ctcttgcttg ttcgagaaca tacttttttg 1080
   gagctactca tgttccttac ttgggtggtg acagcaagct gcccaagaaa actgaacaaa 1140
   tgtaagtctt catattttat tttttctttc tcaaagttga gttactcagt tgtgactgtc 1200
   ctgtgtactt ccttttgaga tcaacagtga ttaagacatc tgcttttgct gggtgcggtg 1260
   gcgcacactg taatcccaac attttcggaa gctgaggtgg gaggatcgct tgagaccagg 1320
   aattcgagac cagcctgggc aacataagca gaccctgtct ctacagaaaa taaaaaatta 1380
   gccaggcata gtggtgcaca cctgtggtcc cagctactca ggaggctgag gtgggaggat 1440
   cacttaagcc tgggaggtcg agatttcact gagctatatg attgcaccac tgcactcttg 1500
   gcaacagagg gagactgtgt aaaaaaaaaa gaagaagaag aagacatctg gtttatgaca 1560
   tgaacattac tgtgttgttt cccaagtttc tctcagcttg gaattcaggc cagagaacct 1620
   tgccagcttt gccatctgct cttctctcta gatttcagag acttcttacc tgcacaccca 1680
   tgcatttatg atgtaactct ccttgatatg ttttctatat aatgcatttt taaattaagg 1740
   gcttttctaa gaataaacca tcctgaaatc cattgggaga atcatgtgaa accccaaaaa 1800
<210> 85
   <211> 2150
   <212> DNA
   <213> Homo sapience
<220>
   <223> nbla24705
<400> 85
   agaaaaaaaa aaaaaaaaca aaaaaaacta aaggaaggaa aaagctgtaa aaatcactgg 60
   cattcgtggg gccactcccc acccaagctc cacgtgtgtc cgtctgtgct cctggcctct 120
   gggggaccag ctgggacatg aacttgtctg ccaggccccc gtcgcgtgct gaacggtgtt 180
   agtttgtagg taacgcacac accccacacc taaggtgtct gcatcctcct gccaacgcat 240
   gggctccacg tggtgtgctc gctggctgtc gtgactgtca gctgtctctt gggaggggct 300
   gtgggggccc gctgggctgc ctcctttccc gctagttgtg cctgagagtt gctgttgttc 360
   ctgctttccc ttcccttcct ttcatcccct gaagggctag gtgtgggttt tccgtgcccg 420
   gtatccccac acacccagca cggacaaccc ttcggcagag cccaggccgg cccctcaccc 480
   cctggagtat tgaaactgga gtcccgtccc caaggccttc agagatgccc ctacacaccc 540
   agggctccag ctctggtcct tctgggggag taaagtgcaa agaggggcac agcttagttt 600
   tgggcctctc gccgagcaag agacagcact gctggctaca gctccaacac agccagctgt 660
   ggcaagagga ctctgcctgg gctggccccc ctcctgtgtg aggtgtctgt cccttctctg 720
   ctggccagca gcagatgcac tggcagctcc caaccctgtt tccgcccctc ggccctcccc 780
   cagcctgttc ggcttctctg cagcccgcaa gggggagcag acttttgaca aaggactgcg 840
   ggcctcgctc aagtccctga gcccccagct gaagctggga ggggaggcca ggctttgtgt 900
   ctgggcatat tcgtctgctg atggggtttg gggaagcctg gggcttgggg tttggtcggg 960
   tggtgcagct agtggcagag cgggatcaga ggtggtggct gcccagcttc tgggctgaga 1020
   caagggtctg tgcaggggtt tactgaagtg ggagtgcctt tggaatctgg gccgggagca 1080
   gaagggagca aaagctacag tgggagccag cctagggcac atgggaggcg tgagggcagt 1140
   gctgcccgtg cagtgtcagg tgtgccagtg ccttggcggg ctgcagtgcg tgtgagggca 1200
   ccttctaggt gggccaggga tgcagctatg gagataaggc gggctgggga cagaaacagg 1260
   tgggcacagg gcccaggaca ccagcggatg gagggcaggg tctagccctg tgctcctgag 1320
   cgtcggctgc ctgggttcga ggcggtgggt ccccggcccc ttgtgatggt gtgtaccatg 1380
   ggggagctcg gggacagggc aagcccgagc atggtggggc tgcagggtgg gtctgaagcc 1440
   aggttgggtg ggggtggtca caagccctga ctgcagaggg tcaggggctc ctgccccagt 1500
   gcctgcccac tttcaattca cattgttttc aacaaggatt ttctttatct tcccctacaa 1560
   atcaagccaa gggaggggca cagaatgggg aacaggacac aggatcctaa actccaaggg 1620
   gactgtccac cgatgaacac tcagagtgga caccatcttc cgtccacgct gtgcccagga 1680
   cagctgtccc catccatgaa cacagggtaa acatctgccg ggctccgcac cagtggctcc 1740
   ctgggccatg ggacagcggc agggctcacc acggacagca cgtggcccag cagccggcca 1800
   ccctggcgtc ctggggcctc ctcccctcct ctccctctca ccttgtcacc tccacggagc 1860
   tgcctgtctg ggataatttg gggatttttt ttctggggga taattctttt gcatgacccc 1920
   taaagagcaa gccacaccgg tctgctagct aggtgtccgc ggtgtggtgg tggcggccgc 1980
   tggccagcgc tgcaaggggt cggctgccca cggtgctggc tggcctcccc tcctctctct 2040
   ttttgctgag tttcattgtc ttttctttct gagccttgta agtgtacaaa aattattctt 2100
   attttgttct gtctcgggaa actgcaaata aaagaaaaac aggacaaaaa            2150
<210> 86
   <211> 1732
   <212> DNA
   <213> Homo sapiens
<220>
   <223> nbla21385
<400> 86
   aaaacactta ctgtatgtta tttgagtcat ttgatattca cttaccctga gaggtaagtt 60
   ctgtttattt atgtggaacc tgaggcttag agaacttagg taacttgccc aaggtcccac 120
   agttagtgac agagctagta ttcaaatgtg gagcagtctg attccaagca tcgcaccttt 180
   aacctttaat ttcaacatca gccttattat gcactacttt tcatatactg ggttccagct 240
   aaactgcact ttcctttcgt atgctgttgc attgccattc ctcctctcca cactgcccct 300
   tctcttcatt tgtttgttga atgctataag aatcttcaga ttgatcatca ttgcttgctg 360
   aaaagtcgaa ataatagact ttgctgatac tcagtaaaag aagaatgtgc taaaattaac 420
   aggagacaca attacctaca aatttcacta gtttaggagc tttgataagc atggttcacg 480
   ttgtaagaac atgcttctta acaagagcca aaatgttctc ttctccattt gctgattctg 540
   ccttctctta gtttccatcg ctattgttct gggcttcaca tgtggcttga aattcaccct 600
   atcctgtatt gcagtcactt gcaggcatct cttcttcctt gttagattgt aagctctttc 660
   aagacaatca ctttttaaaa aatccttttg tattttctca aaacagtaga ttcttgtata 720
   gtaggttgtc aatgtttgtt aaaggatggt ttatttattc cactctgtaa gatttgagtg 780
   aatttttcat gaaagccaaa cagatctttg ttttgcagaa gagtatcttg tttctgaaga 840
   tgccaagaaa caaatttgat cctaagagtg gtcctttacg ataagtgatg tatataagat 900
   gacttttttt tttttgagac agtttctcac tctgtcacct aggctggagt gcagtggtgc 960
   catcagttcg ctgcagcctc gacttcccag gcccaaatga tcctcctacc ttagcctccc 1020
   gggtaagctc ggataacagg tgtgcaccac cgtgccttgt tttgttttgt tttgtttttt 1080
   gtggaaatgg ggtctccctt tggtcttgaa ttcctgggct caagcgatct tcccgccttg 1140
   cctcccaaag ggctgggatt acaggtgtga gccattatac ctggccacaa tgtgacattt 1200
   taaaattctt atacataatt agctttttat gtgttccaaa ttaaaaaata accatgattc 1260
   taataattaa gaagtgggaa gttttgttct tgtggggaaa gtagaagtta ttattgtaga 1320
   acctaagaag tgatatttcc tggtctaata cctgtatctg attcacttcc acataaatga 1380
   agttcaactc ttttgcccag gagttttgca tcccttgctt tggctgagaa gaggataaaa 1440
   cctagaaaga agtctaagca agaccgggtg tggtggctca ctcctgtaac cccagcactt 1500
   tgggaggcca aggtgagagg atggcttgag tccaggagtt caagaacagc ctgagcagca 1560
   tggcaaaacc ccatctctac acaaaataca aaaattagct ggacgtcgtg gtgcacacct 1620
   gtagtcccag ctactcggga gactgaggtg gatcactcaa gcctagggaa gtggaactgt 1680
   gattacacca ctgcactcca tcctgggcaa cagagtgaga ccctgtcaaa aa         1732
<210> 87
   <211> 2482
   <212> DNA
   <213> Homo sapiens
<220>
   <223> nbla21416-1
<400> 87
   gcttccggtc cgtcgcctcc ttctgttgct tcccgtctcc tcggcggctc ccctcccccg 60
   cccggctctc cgcgcccctt ctgggcggcg gggcggcgga gccgtcggcg tgcggccctc 120
   cttgcgttcg tgcgtgcgcc cgtggcccgg cgcacgtccc gcgacaccga ggccgagcgg 180
   ggcagggggc tgaccgccat gaccccccag agcccggcgt gagggggccg agatgcggtg 240
   acctgccagc acctgccgca gccttcgtcc gggagtcgcc ccatctctcc acgcatcggg 300
   gccctgtgcc ccttgctgct gcagccgggc accatgtcga cctcgtcctt gaggcgccag 360
   atgaagaaca tcgtccacaa ctactcagag gcggagatca aggttcgaga ggccacgagc 420
   aatgacccct ggggcccatc cagctccctc atgtcagaga ttgccgacct cacctacaac 480
   gttgtcgcct tctcggagat catgagcatg atctggaagc ggctcaatga ccatggcaag 540
   aactggcgtc acgtttacaa ggccatgacg ctgatggagt acctcatcaa gaccggctcg 600
   gagcgcgtgt cgcagcagtg caaggagaac atgtacgccg tgcagacgct gaaggacttc 660
   cagtacgtgg accgcgacgg caaggaccag ggcgtgaacg tgcgtgagaa agctaagcag 720
   ctggtggccc tgctgcgcga cgaggaccgg ctgcgggaag agcgggcgca cgcgctcaag 780
   accaaggaaa agctggcaca gaccgccacg gcctcatcag cagctgtggg ctcaggcccc 840
   cctcccgagg cggagcaggc gtggccgcag agcagcgggg aggaggagct gcagctccag 900
   ctggccctgg ccatgagcaa ggaggaggcc gaccagcccc cgtcctgcgg ccccgaggac 960
   gacgcccagc tccagctggc ccttagtttg agccgagaag agcatgataa ggaggagcgg 1020
   atccgtcgcg gggatgacct gcggctgcag atggcaatcg aggagagcaa gagggagact 1080
   gggggcaagg aggagtcgtc cctcatggac cttgctgacg tcttcacggc cccagctcct 1140
   gccccgacca cagacccctg gggggcccca gcacccatgg ctgctgccgt ccccacggct 1200
   gcccccacct cggacccctg gggcggcccc cctgtccctc cagctgctga tccctgggga 1260
   ggtccagccc ccacgccggc ctctggggac ccctggaggc ctgctgcccc tgcaggaccc 1320
   tcagttgacc cttggggtgg gaccccagcc cctgcagctg ggagggggcc cacgcctgat 1380
   ccatggggaa gttccgatgg tggggtcccg gtcagtgggc cctcagcctc cgatccctgg 1440
   acaccggccc cggccttctc agatccctgg ggagggtcac ctgccaagcc cagcaccaat 1500
   ggcacaacag ccgggggatt cgacacggag cccgacgagt tctctgactt tgaccgactc 1560
   cgcacggcac tgccgacctc cgggagcagc gcaggagagc tggagctgct ggcaggagag 1620
   gtgccggccc gaagccctgg ggcgtttgac atgagtgggg tcaggggatc tctggctgag 1680
   gctgtgggca gccccccacc tgcagccaca ccaactccca cgccccccac ccggaagacg 1740
   ccggagtcat tcctggggcc caatgcagcc ctcgtcgacc tggactcgct ggtgagccgg 1800
   ccgggcccca cgccgcctgg agccaaggcc tccaacccct tcctgccagg cggaggccca 1860
   gccactggcc cttccgtcac caaccccttc cagcccgcgc ctcccgcgac gctcaccctg 1920
   aaccagctcc gtctcagtcc tgtgcctccc gtccctggag cgccacccac gtacatctct 1980
   ccccttggcg ggggccctgg cctgcccccc atgatgcccc cgggcccccc ggcccccaac 2040
   actaatccct tcctcctata atccagggcg gaagggggcc tggctccatc cggctgcccc 2100
   attccggctc cctgggagat cagtgttgtg agtgcatgtg aaatggggga tccccacccc 2160
   cagtgccctt ccccttcctg gggcccactc acactacacc ctcttccttt cccaccccac 2220
   ctccccggag agaaactgga catggggcct ggggagggga gctggccaga ggaggacccc 2280
   tttcccgtgg cattagaagg gggaggggtg gctggggccc ccacccattc cccctccctc 2340
   caaactccca acccccagtc agtgtttgag cctcctcgtt cccctcacgc acccgctcac 2400
   gcaccctcgg tgaatccttg gtgatgattt tggcaacttt gggaataaat ggcaattccc 2460
   acgggcttgg cactcccaaa aa                                          2482
<210> 88
   <211> 1343
   <212> DNA
   <213> Homo sapiens
<220>
   <223> nbla21599
<400> 88
   gtaaaaagca agcatagaga ctagagagtt gggagatgta aggaaagata ggtataatca 60
   cagctaagtc atgatgaggt aactggtgac ttttttgaca tagtaggtac ttagtaagta 120
   tttgattgtt aaacagaaaa tgggatatct tgaagtttgt agttgtagtc ttaggtctgt 180
   ctctctattt ctaactctta ctgtattatg atacccaaaa cagggaacca tatcacattt 240
   ctttgatttt aacttgcaca gtttttaaat taacagactt tatttttaga acaattttag 300
   atttatagaa taattgagca gatactacag agaatttcca tatacctcat ataccaccct 360
   cattccaact caatctcccc attcatggtg ttctctgata ttaacatgca ttagtgtggt 420
   aagtttgtta cagttaatga acgaaaattg atacattgtt gttaactaat gttcataaca 480
   taataaggtt cactatttgt gttgaacaat tctatgtatt ttgacaaatg cgtaatgtca 540
   tgtatctacc attacagtat catgtggaat agtttcactg accgaaaaac caatatgtgt 600
   cacctgttta tccatacccc tgtcagccac tgatctgttt cctgtctctg tagtttttgc 660
   tttttccaaa atgtcatata tatagccatg tgttgcataa cgatgttaca ctcagtgaca 720
   attgtatata tgatggtggt cccaaaagat tataatggag ctgaaatact cctatagatt 780
   agggatgtta tagctgtcat aacatcatag catcttatag attagagatg ttatagctgt 840
   cataacatca tagcatctta tagattaggg atgttatagc tgtcataaca tcatagcatc 900
   ttatagatta gggatgttat agctgtcata acatcatagc atcttagtgc aatacattat 960
   tcacatgttt gtagtaatac tagtataaac taacctattg tgctaccagt tgtctaaaag 1020
   tatagcacat ataattgtgt acagtacata atatttgata atgataacaa atgactgtta 1080
   ctgtcatata tttattagaa tacacatttt attattttag agtttattcc ttctacttat 1140
   ttaagaaaaa cagcctcagg caggtccttc aggaaatatt ccagaaggca ttgttatcat 1200
   aggagatgat cactcagtgt gtgttactgt ccctgaagac cttctagtgg gacaagatct 1260
   agaggtggaa gacagtgaga ttgatgatcc tgatcctgtg taggcctagg ctaatgtgtg 1320
   tgactgtgtc ttagttttaa aaa 1343
<210> 89
   <211> 1484
   <212> DNA
   <213> Homo sapiens
<220>
   <223> nbla21681
<400> 89
   taggagcaat gactgttggg caggatggca gcagatgaaa gctcacagaa cactttgcgg 60
   ctccagttca aggcaatgca ggagatgcag cacaaatggt tacagaagca gatggagaaa 120
   aagagggaaa aagaactgag cctcaaaagc agagctgacg accaagagga gcccttggag 180
   gtttcagatg gcctcagcct tctccacgca ggggagccaa actcgaaaaa tagctttgag 240
   aagagggtgc ttgaagatga gattgaacac cttcgaaatg agctcaggga aacggtggac 300
   gagaacgggc gattgtataa gctgctgaag gaaagggact ttgaaatcaa acacctcaaa 360
   aagaaaatga ataggttact tgtgtattaa aggacccttt caaaggaaaa tgctcagact 420
   tgggacacag gcccagctgg ttcgttattt atttttattt acatagcgaa ttctctggca 480
   tttgtcttcc ctgctggaac cactcagact ggccaagatt tccaaaacag tgttctattg 540
   tggaaacaag tgccagagac ttggtacgct ggatcgggtt tctgtgacag gcttcagagg 600
   ggcccaggtc acaagctgga gcgtattgtt tctgcctcaa agccttgagg ttgggcctga 660
   gtgctgcact tcaacaaccg caaagctggg tccttcttgg accacagcac cccaactgac 720
   attcagtagc ccaccttttg ctgcactcag aggtccactt gtccgtgggt tttcacaaag 780
   gctagggtcc tgtggtgatg tacttcctat agccagaatt agctcagcac taggtgacag 840
   gtgagtgggt taaggaagca ggagttggtc agctttgtgg ttcagtcatc ccagaatatg 900
   ccaagccacc gagggcccag atgggagaca gagcattgct ggagacccca gaggtgaagg 960
   ccctgaccag gctgtcagcc aagggggcca ccgacgcagg agccaagcca ccgagggcca 1020
   gggacctgga ggggtcgggc tcaacaaatt cttgttttgc agagcaaggt gagtgagtca 1080
   tcagacttct cctggcctga acaaaggatt taaaacaccc cagaaagagc tgccctgacc 1140
   cccttagaga cctaagcaca cagtacccaa aaaaggcctt taggtctcac agtgcactcg 1200
   tgcggggttg ttgttttacc ttctcgccaa ccagcctgat tttaatttgt tatttaatga 1260
   acaagctctt atataacact tagcacatgc caggcactgg agcttaacaa atgccaacgc 1320
   ctttggtttg atttatttta ctccaggcat cttttttttt tcttagttta tgtagatttg 1380
   cgtgactgtt gtaattgtaa gctttttcca gttttgtcca gatgcttgta gtcttttgaa 1440
   agtttaatta cccaataaaa atttagcctt gtctccctca aaaa 1484
<210> 90
   <211> 1479
   <212> DNA
   <213> Homo sapiens
<220>
   <223> nbla21878
<400> 90
   taatcattgc agttaagaga aatggaaatt agttgtgtta atcttgcaga atgtttgcag 60
   gactgactat caaactggat gatttccatt tataccctac tgtgtcagtt caagcatcaa 120
   aataccttgc atctgagaca gacttcctac atcagggaca ggtatctgtg tgtcattata 180
   caaaacagtt ctagggggtt gaactacata gtaaaaaaat aaaataaata gtacttagtg 240
   taaaataatt ttataaatga tcttttgtac tttaggacat taaattgtac aacttttgta 300
   tatataaaag cttaggaact ttctgtttag caggaaggca acacattcct acacttttaa 360
   tgtatatgtt tgttataatg tccatgtaaa catgccctat gtttgtgcct tttaattagt 420
   ttgtctcaat aaacaaaatg tagagaaaaa tatgtagcta tgactttgtt acaactgttc 480
   ttatccacag tacaaaaatg gtttgttttt aatatgtaga gcattatgtg tggactactg 540
   gaaggactcg tgtggggaga gcccaagaat gaccttgctg aggcctggat tgggaggcac 600
   agtggccaca tttggaggaa gttcacattt cctggcatgc agacccaaaa ctgggttctg 660
   gctctgcctg ctgggatctg ttatctctgg tgggctggca gtcataattc acaattcaga 720
   cagcccaggc ttcctccaca gtggtccaag gagcagtcct cagtgggggc aggtgtgggc 780
   cctaccccta agctagaatg tggttgtcag aaccctgaaa gtattagttc taaaaaaaaa 840
   aaagatatat actagaagta attgttttat caattcattg tataataaac aggagtgaga 900
   cttcattgta tgacttcagt taaaatacta ttttgtatgc attctttatt cacttaagaa 960
   gcttgtctgc aataataaag ccacgtcatg tcttcttttg ggagggagag agtcgatggc 1020
   aggagggggt tttgggtggg ccactgaaaa ggggtaccga ataggttgtg tgatgaaatt 1080
   ctgtgtcttg gaactggaat tgagtttcga tgttgatgaa ctgattcaac caggtgttga 1140
   aggcacgaca gccactgctc tacgaaaagg cagagtacgt ttttcccttc tggttgtaac 1200
   ctggttgaga gcttcccctt tatcagattg gcagctaaac agttgtatta gataatcctt 1260
   aaatctgaca tccagcctgt tacgctctag ggctcgctgc ttggcctgcg tttgcttttt 1320
   attgtgtatc cgttcccctc ctacggtgtg ctcctgaatg aaggtttcta tgtaagcaga 1380
   tgatgatttt acctgtcaat accagcactg tattactaac atgcaaaata ctgcagattt 1440
   attttgaaaa ttaaagttaa ctggtcacaa atgtaaaaa                        1479
<210> 91
   <211> 1907
   <212> DNA
   <213> Homo sapiens
<220>
   <223> nbla21922
<400> 91
   aagctggatt aattgacaag tgattttttt tcccctctgc ttcttagaaa ctcaccaagc 60
   agtgtgccta aagcaaggtg gtttagtttt ttacaaagaa ttggacatga tgtattgaag 120
   agacttgtaa atgtaataat tagcactttt gaaaaaacaa aaaacctcct tttagctttt 180
   cagatatgta tttaaattga agtcatagga catttttatt ttatggaata gattttaatc 240
   tatttactac tattaaggta gattttctat ggcatgtcca ttagctattt catgatagat 300
   gattaggggt ttcctcaaaa cctgtgtgtg aggaaattgc acacagtagc aaaatttggg 360
   gaaatccata acattttcag accatgaatg aatgtttcca ttttttttct aatggaatgt 420
   gagagtttat ttttatttta ttctgaagga ctttaaggaa gggatacatg attttaaaaa 480
   agcctgtaag aggtgaaata tgtgatgttt gaagtctctt tatagacttt ttatatatat 540
   tttttaaaaa cactcatcta gatgaggtgc tttgagcagt tctgaaaaat gcagttccag 600
   gaaagcaact gctttggttc ctaaggaaga aattctaaat aatgcaaact tttaaaataa 660
   gcatctaggt ttttgataat tctgtctact tacaacaaac ttgttagtac ataaccacta 720
   ttttaataat tattttctct acacaaatgt gtaatatcat atttgacttt gcttatgcag 780
   gccataagtt ccaaaagata atttccctgc ccacaaaggc ataaacttga aaacacatga 840
   gattgaatca acatgcttta ataggaaaag atgtatggtc tatatatgta tcaatctggt 900
   gaatcctcgt tctaataaag gttctttttc ttttctatga tacacacagc cacgctgata 960
   atatgcaaat gaacattttc ctttatgtct ctccagataa tgtttattgt ctgaggtaaa 1020
   ttaaattccc accagggttt gctgtcagta ttttaacacc cacattagta tatgcgtcca 1080
   gggtcataac cccctaaaat ccatcatgca accttattaa tctgtcttgg gattccagtt 1140
   tagtgcttgg atttatttcc tgattacact acatagaaaa gtgagacatc tgccattccc 1200
   aactctggga aaaccaacta atatacaacc atataaatga aggccatctt gatggtctca 1260
   acactaattt ttatgatgca aatttataca ctgatttttg taaaggacaa agttttaaaa 1320
   gcgtatttaa cttgatgttt tctatcagca taaataaaat ggtcatgaat agtcattaaa 1380
   aacagttgcc agtgataatc tgcatgaagg aaaaagaacc ctgcaaatgg ctattgagtt 1440
   ggaagtattg tttttgatat gtaagagata ttcagaatgc tcacactgaa aatgcctcaa 1500
   ctttttaaag tgtaagaaac caccatgagt ggtgtctaga tttctaatga agaatcatga 1560
   tacagtttgg attaagtatc ttggactggt tttaaacagt gctttgtacc ggatctgctg 1620
   aagcatctgt ccagctggta tcctgtgaaa gtttgttatt ttctgagtag acattcttat 1680
   agagtattgt ctttaaaatc agattgtctc ttctatattg aaagcatttt tatgttttct 1740
   aatttaaaaa ttaatatttt cttatagata ttgtgcaata aagctgaagt agaatgtgtg 1800
   gtttttgcaa atgctttaac agctgataaa aattttacat ttgtaaaatt aatatattgt 1860
   actggtacaa aatagtttta aattatattt taaaaagctt ccaaaaa              1907
<210> 92
   <211> 1402
   <212> DNA
   <213> Homo sapiens
<220>
   <223> nbla22004-2
<400> 92
   aacatggcga tgcacaacaa gacggacacc cggcgggagc tggcggagct cgtgaagcgg 60
   aagcaggagc tggcggaaac attggcaaat ttggagcgac agatctatgc ttttgaggga 120
   agctacctgg aagacactca gatgtatggc aatattattc gtggctggga tcggtatctg 180
   accaaccaaa aaaactccaa tagcaaaaat gatcgaagga accggaagtt taaggaagct 240
   gagcggctct tcagtaaatc ctcggttacc tcagcagctg cagtaagtgc attggcagga 300
   gttcaggacc agctcattga aaagagggag ccaggaagtg ggacggaaag tgacacttct 360
   ccagacttcc acaatcagga aaatgagccc agccaggagg accctgagga tctggatgga 420
   tctgtgcagg gagtgaaacc tcagaaggct gcttctttta cttcctcagg gagtcaccac 480
   agcagccata aaaagcgaaa gaataaaaac cggcacagcc cgtctggcat gtttgattat 540
   gactttgaga ttgatctgaa gttaaacaaa aaaccacgag ctgactatta gaagacacat 600
   tagtgcagaa gcttccaggc tgtagagccc tgcttccctt ctctgacctc acaaagataa 660
   acatccttca cctgagttcg tggccatcca cctctgctct cccagaccca gtgcctgtga 720
   ctttgagtag tttgttctaa atgtggtgac aaacaagtca tttctgtaag acattgggtc 780
   ttactttatg tcatttttag taacagaact gcaggaagat caagacaatg ttgtaatccc 840
   ggcaagttgc taactgtgcg tttctccctt cttagaatga atgtctcccc caaaactggc 900
   tggcaccagc ttcatctgtg atacccttca agaaatgttc tctggttttg ttttatgctg 960
   aaagtagaac acaagtcaca tttcagatgg aggctgtaaa tatctggcat tttcttatat 1020
   tgttttatgt tttcttgttt ttctcttgtt gtttttatct tattttcttt ggggtttttt 1080
   tgtaatgcct ttgtacagct catactttcc tgctgacata tctgatcatc tctttcatgc 1140
   agttgccaat attcataact gaaaataatc tggtttatca taagtaaaat gggaaacttg 1200
   cctctgtttt ttgcaagggg aggtaaagag tgtttagtaa ttacctatct taaatctttc 1260
   tgagttggta gtagattcat gttcaaggaa caggaaaaat ggaaaaacat aagtttaaat 1320
   cagttctttt taaataactt tttattcttt tgtataaata aaatttcaca ggcttcaaat 1380
   tctcatgctt tacttttaaa aa                                          1402
<210> 93
   <211> 1577
   <212> DNA
   <213> Homo sapiens
<220>
   <223> nbla22004-1
<400> 93
   gaagttggca ttaaacatca agagatacca ttcattcaac atatctatca gaagggcacg 60
   tccaccatca gcacaatgag atctcatact caagaggatc cttttctatg caatgactta 120
   ggagaagatt tcactcaaca tatagcattg actcaaaatg tgattaccta catgagaacg 180
   aaacactttg taagcaaaaa gtttgggaaa atcttcagtg actggttatc ctttaatcaa 240
   cacaaggaaa ttcacaccaa atgtaaatca tatggaagtc atctatttga ttatgccttt 300
   atccaaaact ctgcccttag accacacagt gtgactcaca ctagagagat aacattggaa 360
   tgtcgtgtgt gtgggaaaac ctttagcaaa aattctaatc ttaggcgaca tgagatgatt 420
   cacactggag agaaaccaca cggatgtcat ctatgtggga aagcctttac tcattgctct 480
   gatcttcgaa aacatgagag aactcacact ggagagaagc catatggatg tcatctatgt 540
   gggaaagcct tcagtaaaag ttctaacctt agacgacatg agatgattca cactagagaa 600
   aaagcacaga tatgccatct atgtgggaaa gccttcactc attgctctga ccttagaaaa 660
   catgagagaa ctcacttagg agataaacca tatggatgtc tcctatgtgg gaaggctttc 720
   agtaaatgtt cttaccttag acaacatgaa agaactcaca atggagagaa accatatgaa 780
   tgtcatctat gtggaaaagc cttctctcat tgttctcacc ttagacaaca tgagcgaagt 840
   cacaatggag agaaaccaca tggatgtcat ctatgtggga aagcattcac tgaatcttct 900
   gtgcttaaac gacatgagag aattcacact ggagagaaac catatgagtg ccatgtatgt 960
   gggaaagcct tcactgaatc ttctgacctc agacgacatg agagaactca cactggagaa 1020
   aaaccatatg aatgccatct atgcggaaaa gccttcaatc actcttctgt ccttagacga 1080
   catgagagaa ctcacactgg agagaaacca tatgaatgca atatatgtgg taaagccttc 1140
   aatagaagtt acaactttag acttcataga agagttcaca ctggagagaa accatatgta 1200
   tgtcctctat gtgggaaagc ctttagtaaa ttttttaacc ttagacaaca tgagagaact 1260
   cacactaaaa aagcaatgaa tatgtaagaa tcatcagctg tagcgttaac actaaataca 1320
   ccaaggacaa acatactaca ggaatattat gtctgtaatc agtgtggaaa agcctttatt 1380
   tatatttacc actttgctca acctaaatga attcaaggta gagagaatcc agatgtattt 1440
   aatgtttatg gcacaaactt cagactctag gctgaccata tacaacgtga gagaatgaaa 1500
   ctatagatca aaggaatgtg gaggagtctt catccacagc tctgttaaat aaatgggaga 1560
   aatcacatca cgaaaaa                                                1577
<210> 94
   <211> 1945
   <212> DNA
   <213> Homo sapiens
<220>
   <223> nbla22085
<400> 94
   gtaaattatg caggtgataa catggtttgg aactgtttat tgggctcttt aactgaattt 60
   tcaaatgaaa tgaactatgc ttattgctgg cacattgatc ccatttctgg aacatttttc 120
   ctatttccag agttacatat gttcttttgt cattacccaa tttaacctcc ctttctctga 180
   tatgccttgt agccaaagta ttaaaggctg atgaacatag acaagggaaa tgcatttctt 240
   agaaatccgt gaaccctcag ttgtatgctt tcagtactcg tgttaatatg tttctatggc 300
   aactctgagg tcagtggttt agaaatgaga taccagtgtt aatgaaaagt gtgtgctctt 360
   tgcttttgca tggcttggct tagtatccaa ggtatattag ggccacttga aagcatgaag 420
   accagttata tagggaacag gtttctctca gtggcacatt ttgctttttc tgagccccaa 480
   atacattgcc tgggcatgaa cattgttacc gtaaattgca catggtcatg gactgaatta 540
   tgtgacttta aaggatgtaa ctgcccaaca tttgcagatt ctgggtggtc tatgtgacca 600
   tttgtctcgt atccaaaaac cccggggcta ttggaaccct tccaacactt tttcctttgt 660
   catagacaag tttatatata acttaccaag atgttggctg tcctggtgta ttgccagaca 720
   gctgtctttt ggttcccatt ccaaatgtgc tgctgtcctt ctttgcattt cacaatatca 780
   aagaaaccac cacccttctt cctaacagca ttttatgcct tttattccac attaaatggg 840
   aattgtgcct acttaggagt gcccctccaa ttaattacat gtgtccaaga ataatccaag 900
   ctagagacac aaggtgggaa aacatttcaa aaaaaaaagt cctcttaagg ccagtaattt 960
   atctgaaaag gtattttatc acaccttgac accttatata tgagcctatt aggagctgca 1020
   ggtggtttca tagggtaaaa tccaagaaaa gagaaggatg tgtggggttt ctattagaag 1080
   ataattttgt tctcatttta ccttttcttt tatgatcctt ctctgctaga acaggttaat 1140
   tctccaaatt tgttttgttt tgttttgtta ttttttaggg aactcttttg caaaagcaat 1200
   ggtcggatgt aaataacatt taaagtatag tgcacataac ttccccggac tgttccaatc 1260
   tgataatttg taaatgcttt agagtttttt taattaacac ttgtgttgct aaattctatt 1320
   tatgtaagtc tgctaaagtt ttttagccca cttaaaactt aagacaacca tttaaaataa 1380
   tggatgggtt actatgagca atttcgcttt cagaaccccc ttgttttagt atatgaaaaa 1440
   gcctaatgcg cattaatgag gttgaagaga ctatgagaaa tatgtatagt gtatatttta 1500
   aaacagcttt gcttgtattg tgaagattta aaaacaaact tgagattttt aacgtaacta 1560
   ttaacacagt tttaacataa gttatcccac tgggtttaag agcatcttga atgtataatc 1620
   ctttttgtaa cccaggttgg tttctacttt taccagtcac ccaaacatat ttatgttttt 1680
   agttttatgt actcatttcc ctttgttttc ctcaaacagc atgatttttt tgcacatgta 1740
   gaaatttttt aaaagaaaga aattagtaca tcattttctc tggattttct tcacttccct 1800
   cttcctttct actaactcct tccttaaagg ccatatcact ccatttgcat tatttgtgca 1860
   aatgccaggg ttggttttta tttttatttt tgctatttac ctaaaaaaag aaaatgcttc 1920
   agtcaattgc ttttttattt aaaaa                                        1945
<210> 95
   <211> 1551
   <212> DNA
   <213> Homo sapiens
<220>
   <223> nbla22119
<400> 95
   ttttgcatca gtaaaatgat ttttttaaaa ccaataaatc atcaattatt agaaatagtt 60
   gtctcacagt gatactggtt tttcttttgt gctgttatga tttaacattg acaggaacac 120
   tattttaaat ccttacgttc aggtgtttgt aacttggcct tataattagg ctgaattatg 180
   gcttcaaggt ctacaattta tgtgtatggt tcacagccta gcttctattt acatttgaaa 240
   atacagattt ttaccaactt tggattcttt tttagttata tgtttgtctt tcctttttaa 300
   attgttcaaa actatttttt aatggtcaag ttactaacac ttgaaaatca gatactgcac 360
   caaatacagt gtttttccgt agtgttttta atgagtgcac ctattactac tgtgcgagaa 420
   ttcatgtttt accagtcatt gttatattac aaacagactt gcatgattaa ccagttgtta 480
   cacttacttt ttcaagttgg agtatatatg actcagtgca gactggtctc tcttatgtga 540
   atgcacacat gcagaaatgc agagtcaatt ttacatgccc ataaagacat ttgtaaagaa 600
   ttcagctctt atggtctgtt gtataaatgt gtatctaggc actttggaat ttgacctcac 660
   agatgttaca acttgatcag tcgtttgacc taatttgtgg tagctatctg tatgttttgc 720
   aatcttaata cagacatgct ttccaaaaag attaatacag aaccatcctg ccgttttgga 780
   taagtctatc cagctgtgga aagggcaacc tgtggtttct ctgtactggt gtttaatggg 840
   ggaagaatat gaacagcttt aaagagctgt gtattgtggt tactactatt aaaaaataag 900
   atctgcacga gtctgactgg cctttgggtg gcctttgtgg acggctcgta gctggaaagt 960
   gttgatctgg gttttctggc attcttttaa gttaaaaagt taacatcggg acatgggttt 1020
   gatcttttgt tgtacctgat gacagtgcag agattctcca cagctggata aaaatgtcac 1080
   aaagctactt actgtacatg ggcagtatca gatttcaaat cctaatattt cagctgtgct 1140
   tttaatactc aaaatattag gggatggggt gttgaagctt tccctttttt gcttttaaca 1200
   atttatagaa tttaacagat gtactgtctt tcatgtggcc tcacatttaa agttatgaga 1260
   acatacacat ggtttacaac ttttactata tacctttcct tggccaccaa gtattttaaa 1320
   agtgtgccac cttttaacct ttactttttt taagttgaag gtgatacttt ttctatatat 1380
   gatgaaactc atgtcaactg aagtgagtgt aatctcagat accaacatta ttatatttta 1440
   aaatcacgct atggaaatat cacctgaatt ctgtcatttg tcagatttac agtacctttt 1500
   tttctttaac ttttagcatt aaataaaaat aaaattggga gcactgaaaa a          1551
<210> 96
   <211> 2151
   <212> DNA
   <213> Homo sapiens
<220>
   <223> nbla22149
<400> 96
   aaaaaaaaaa aaaaaaagaa gaagaaatct cagcaggctg agatggaact cattcttctc 60
   atgaagaacg tggcaagcat tatacagagg ggccatagtc tggaaagcag gagatgctta 120
   cagacatata agttgtttcc agtgttttgc tcttggtact catggttcca ctatttacat 180
   caaccttttg agaaacatat ttatacactg tcttatactt ccctcctttg ctacagaatg 240
   aatctacttg taacctacca aaaatttacc ctgtcacatt tccccagctg ctggtttaaa 300
   aataaatatc ctggatttaa agccaattgt gtctaacagg tgccaccatc caagtgagga 360
   tttcactgtt cacaggcatt tgagacacac cagcggccgg cggttctcac tgctcttcat 420
   atggaggcaa ccatatatgg gtaagtcatt tagtctctta ggtaggcgaa ctgaggccaa 480
   tctccccact tttagggctg tgaaactgtt ctgtatgata caataatggt ggatatgcgt 540
   cactatacat tcgtccaaat ccacagaatg tacaacacca agagtgaacc ctactgtaaa 600
   ctatggactc tgagtgacaa tgatgcatca aataggttca tcagttgtaa taaatgcact 660
   gctctggtgc agaatgttga tgatggagga gacaggggta catgggaatc tccgtacctt 720
   ccattcaatt ttgctaaaac tactctaaaa aataaaatta aagaaaaaaa aaaagctccc 780
   ctctttcccc agttttacga tttatttatg ctttgtgaaa tggagtctca ctcttgactc 840
   ccaggctgga gtgcagtgat ctcagctcaa tgcaacctcc acctcccggg ttcaagagat 900
   tctcctgctt cagcctcctg agaggctggg attacaggcg catggcacca tgcccggcta 960
   atttttgtat ttttagtaga gatggggttt cactatgttg gccaggccag tctcgagctc 1020
   ctgaactcaa gtgatctacc gtacccggcc cccaatgtta gtttttaaat aaacgactat 1080
   gtttaattca catgctaaca ggcacctaga gaatactttc aagtaaaaag attaatgaac 1140
   ccacttcgca ttgagttagc tggttgtttt ctgccaacca ggtgtccctg cctggtccac 1200
   agttgaccaa ggatccctgc atctgcctct agcaacaccc aacactgtat gaagggctga 1260
   gggggtctga cagttcacgt cactgacatc ctctcactgg tatttcgaat gccaagccag 1320
   ccctcaaatc aagttcactg gcctcgactg agctgccaag tatttcatac atggggaggg 1380
   gggttggggg gggggagggt atggggatca cacaggtgcc aggcaatgag taagattatc 1440
   ccagcaactt ctccatgcag agagaaatgt ctgcagctgc aacactattt ctactccagc 1500
   cttctagact ccatgtagtt tgcctttgtt tgaatgtttc tatttatctg aaataaccag 1560
   aaatcatttt tattattata tattactcca gtttattaaa taaatgaaac aaggcttatg 1620
   ccacatattc caacaatgtt taaataaaga gcttgaaata taaaggctta tgaaaacttc 1680
   atactcttta tataatgcat actatttcta gcacatgaat aaatataaag gacaggagcc 1740
   actttttata ttatgaatcc acaacattaa gcatcaatga ttacacaaat ccataagcac 1800
   acaaacaaaa aaacccattg gttataaaaa ctagaattcc ttttggcata tttaagaaaa 1860
   cccaaaggtg gggaggtact tatagccaga accctgacaa cgaggggacc aagtctccca 1920
   attccttaag ttgtttcttg gttagaagct tcaacaattg cattaactct ttcaaaaaaa 1980
   cagaaaaagc aggttaagat cctgttcaat aaggcactta ataagtctac actgaagaaa 2040
   tactatgctt ttatcttaaa tcgtgcttaa gttttaccat gaggtttgaa tttctttcca 2100
   ccttggtagg aacatgtatg taatttgaat aaactggtaa taatacaaaa a          2151

<210> 97
   <211> 1790
   <212> DNA
   <213> Homo sapiens
<220>
   <223> nbla22161
<400> 97
   gttgactacc cttcttacaa caaaactgtt tcttttttat tgcaaatagg gctcttggtg 60
   ttttttactt ttttgtacat atcacagtac atggtttttc actctttagt ttatttcatt 120
   ttattggaat taactttttt ttattctaat actgacagag tttgtaatct ctatataata 180
   cgtaattact ccaattacag cacttttacc ttgaagagca tctcagtttt tcccacaatt 240
   tcattgagtc atcagagact gatgttgctt cttggtttca aatttggtcc taaagaaact 300
   ttcggctgta gaaacaaaag cacagagtga attttttaca aaagacaggg aatatagaat 360
   agtcattaca gacacaaata accctagtag cacgaagttg gtgttttctc tgtttttact 420
   taagattaag aagatttttg gtgactctga actctttatt tatatttcag tttaaaatat 480
   caagactaag gggcatcagt tatctttact ctttaatatt gcccatattt taataaatta 540
   cactaattaa acgcatattt tcagcatacc agtggaatta attttgtgga tcacacacat 600
   ttaaatagtc atattgtggg aatattatag ctggtaacca gctgatattg attcttatta 660
   taggaatgac tgtaatgata gtggtggtag cagtagtgat attagcggtg gtggtgatgt 720
   gaagtaaaat aaaagtatat attatattgt gcccaattta ttagaaatta tttgatcaat 780
   gcttcatttc attaaaatat cataaagatg tttatagtat ttttttactt tattatttaa 840
   atcataacta acaatatttt taaaaactta ttttcattgc tacaatgtca aatattccaa 900
   aatcagccaa ctacagctat atatgtgtta tgtgtgacag aagtgatctt ccttccctct 960
   ttttgagctt gacatgaaag tgaaagaaga ctcaatgaat aattatgagc tatttattta 1020
   ataattactt gccttgggtg taatacagta atgaatgagt gaaacaaata ttctcattga 1080
   atatgataca atgctgtttt ctgtatgttt catgttctat tattaaaggt atccattagg 1140
   ccaaaattat ttaatcaaat tctttatctg ataggtagat tgagagcatt ttcttaatgc 1200
   attaccttgt acataagtat acacttggta aagtagacga agttgaaata ttaatttcat 1260
   ttggcattta gcatgtgaat atgattattg tttgattgtg tctgtatatt tgtttggtga 1320
   cgtgctcagg tgctcccact actgattaat gtgtgtgcta atatcctaaa aacacatatg 1380
   aggtttaaga aaaaattttc ttgtctgaaa acataaacat cttaataaaa ctgattttga 1440
   aataaaaact aaagtacttg aagatatgtc ttgtttctaa ctatatgttg catgccatgt 1500
   tggtgatttg ctaatgtgtt tttttgtttg tttgttttac ccaaatccct ttggaaaatc 1560
   taatggacaa atgcaaattc ttggactaag gactgtataa attgacctga aaatacatga 1620
   gagttgcatt taaaaaaaaa tgcttgtaaa tccgtcttga gttttactct atgtaaaata 1680
   tgtcttggtt ttgtgattgt atacaagatg tatcttgata acttatgtaa actgtgccgt 1740
   ataaaggctg ttgcctcagc cttactaata aatactgaaa atatcaaaaa            1790
<210> 98
   <211> 1955
   <212> DNA
   <213> Homo sapiens
<220>
   <223> nbla22252
<400> 98
   aatgcaaccg gtgagagtgg ggaggctaag ctgtcgatta gtcccggcac gtggatgaga 60
   aagacaacga ggagggagca gctagagggg tggaaatggg atcacgtgac cttgcgagaa 120
   gcagggagag agaacactgc gtctgctccc ttttagaaca gctcaatata gggaatccct 180
   aacagaggac ttccaggata tcctagggac agcagagcct caagatccag ggaggatcct 240
   ggatacctga gtcaccactt ggaggggaat ctcctggaag aactgattga tcagcaacat 300
   ctacattcaa cttgagggtc ttcttgcttg gtgagcctgg tggttggcca acagctctgg 360
   cattgtggga cccacaccag ccaggttagc ctcccatccg ctggacatca tgggagtact 420
   gagcatcagt tcctccttag tcttgcaaca ggatggaacg gttcccaggg cgctggcact 480
   tccattggca gcagcagaag aaccaaaata ggacacacca aatggatcta attttgcctg 540
   aacctcggtc tgcaaggatc atgatttgcc atctgggcac aagcttaggg aagctctggg 600
   aacagctcta ctcccagaaa gctgggtgaa aatcaactag acccagcagg gaagtctccg 660
   cgttgatcag tggggccttg ctgggctgcc ctcccagtcc ccacaggtgt tccaaggagg 720
   ggcctgaaca ccaggctctg gaaaacctga ggatgatgtt gctggagttg gtgccggggc 780
   tcgctctagg acaggcgtgg gctcctcctc tccactggtg tgcctttggg aagggtatcc 840
   tccacccact gtgcacccac ccgacctgtg gcttggagca ggccctccct ggccagcagc 900
   tctgcttctg ctgagtgaag aggaaggagc acttggctct ccctccagga ggtgcatgaa 960
   gattaattag aaacttacaa atccacagaa agtttgaaga agaaagtgaa aaaacttcct 1020
   acccccatca cctcaagata ttcactgtgg gtgtgttggt ggactcctga tggacacccc 1080
   agcttctcaa tacctgggag tgcaggcaca aaccttgacc actctgtaat gccactatca 1140
   tgctcagttg tcctgctgta gctgaaatca tttctgcagc aacctcttgg aattaccttg 1200
   aagaagcagc ccagacagat cctctgaaca ttctctaaga atatagcggt gaatgtggtg 1260
   tttccctgag ttctgtgagc tgctctagca gattaatcga accctagaag aggattgtgg 1320
   gaagccaagt ttacagccag cagaaaaatg aaaccatcaa tgccagcgac agggtgctga 1380
   ccaggcggag gcagcacggg ggagcacaga ggctgggtgt ttacttagct tcctccctct 1440
   gtactctctc cacccggccc ctcagcccac cgctcttctc ttcctggggc agttccctct 1500
   gctgagcggg ctggatggag attttccaag caggaagagg agtagagcct cggtagatta 1560
   agttcagctg tctccttcat tgtactggct cagggctggc cgggatcctc tctgctaggg 1620
   gcttgaggtg gaggcaggac ggctcaggag gacccactga ggatcattct gcagtctctg 1680
   caggtgctgg tcaggttctc agcgctcagg ctgcaggtag ctgggcttcc acaagggggc 1740
   aggtgctctg cggggtgcac ccctggatca cccgtgccct ggcaataatt catgctcctg 1800
   agataccttt ccaatcggta tcttccagcc ttcccctgct cccaggctcc gtgtgggcag 1860
   gagctgagtc ttcttcaact tgattctctc tgcatctagt ccagtgcttg gaacaacata 1920
   agcaggaaat aaatattgga tgaatgaatg aaaaa                            1955
<210> 99
   <211> 2059
   <212> DNA
   <213> Homo sapiens
<220>
   <223> nbla22347
<400> 99
   gatttccagg catcttaatt cttcttttgc tgtgctttca aatgggttat tttgtgggtc 60
   tcaaatatat ttccttaaat atttggtgaa tccttggagt tagaagagaa aggaatatta 120
   ccatcatttt attagtgctg gtcaattctg atgggggtaa aaattaaaga agctgatatg 180
   gtaaagacga agaaaaaata aaaatatggg gagactgacc ctggctttca ttggcgtagt 240
   tcatttctgc ccttcctttc tatagattta aataaagaca agtatttatt ttgactaaat 300
   cacagacata taaggcattt tcgggggtag attgcagagg tagtaaaata aactatagta 360
   tttcttggat ttgcttattt cttgtagcag tgtctatatt aatgcatctt gaattttatg 420
   cagtgtaatt actgtttagt gaaatttaaa aaaggttttt taagagacat ggtcttactc 480
   tgtcactcaa gctgttgtgc agtggcacta tcatggctca ctcactgcag ctggggactc 540
   ctgggctcaa gtgatcctcc cacctcagcc tcctgagtgg ctgggactgc aggcatgtgc 600
   cacctcacct ggctaatttt aaaatttttg tagagatggg gtctcactgt gttgttcagg 660
   ctggtcttga actcctgtgc tcaagagatt ctcccacttt ggcctcccaa agtgctggga 720
   ttacaggtgt gagccaccac gtccagcctt aatgaataat ttttttaaat tgaaaagtca 780
   caaaacttat tacgaacaag gtaaaaggtg tacagtttga cttagctctt tgctcaaaaa 840
   tactgataac ataataagta gggtaagcct ccccagtgcc tcaaaatacc agataccgtg 900
   ttcatcattc tctcagacat gagtgattaa agtaagatta tttcattttt ttatgatacc 960
   tgctgtgctc ttgaagaaga ctgtcttatt ttcacttact agtaaaagtg aaagaggaac 1020
   attgttttaa cattttaaaa ataaaaatta ttttttaatt attgttgatt tgaaataatc 1080
   agtttcctaa tatgttggtt caggtttcct gagatgcaag gaaataataa ttgtaccaga 1140
   atggggggaa aaggagggaa gaaaaagggg aagagaggag aaaccagttg caatgaatta 1200
   tagtccttat catgttactt tctgagaaat aaaatgggct tctgattcta aaaaatatac 1260
   tgtatctgca agagtaaaag tcgtaatctt tcccatattt cctataggca aattaagtta 1320
   ctttagtggc aaagtacatt taaaggccca tttatttctt caatcacatg atagtaaaag 1380
   ttttgtcagg aggtctgctg aactgagaat acagaatcag tggcagtgac agaacatcta 1440
   aaaatttcca gtcaccatct cctttagaca tactggtcct tgcattagtc cttaagccaa 1500
   cataaatgat ctttaatgta aaattgtaac aagtacataa agcaggctaa cgtagatatt 1560
   gcgtatctca aagcagttgg atttaaaata agtgatagtt aacgaaatcc aatactgtaa 1620
   tgaacttttg agaaaaaaat agttgattat gctttttaat tgtgtgtttg gggttttggc 1680
   ttttattatt actgttaatt tggccataag ctcattatgt taatcagttt taacagtgtt 1740
   tctccatttg ctggataaga atttggctga ttggccgggt gcggtgttgc atgcctgtaa 1800
   tcccagcact ttgggagact gaggcgggtg gatcagttca gctcaggagt ttgagaccag 1860
   tctgggcagc atgatgagac cccatctcta caaaaaatag aaaaattagc cagtgtgttg 1920
   gcacatgcct gttgtcccag ctacttggga gtcttgaggt gagaggatca cttgagcctg 1980
   ggaagcagag attgcagtga gccgagatca tgctactgca ctccagcctg ggcaacagag 2040
   tgagagcctg tctcaaaaa                                               2059
<210> 100
   <211> 1773
   <212> DNA
   <213> Homo sapiens
<220>
   <223> nbla22352
<400> 100
   gtaaatagta gaatgtgaat ctggttttct tttgcttgca aattgccatt cttttttttt 60
   ttcaaattta aaattacaca tgctgttttt ttctttgatg gggagaaaga actcattccc 120
   tgagttcatt catttttgtt gatgtcatcg gtaatcttca agacttattg aagtagagtt 180
   gtatttgggg aagatacatt ttatattcac tttttttttt ctttctgtag tctacctctt 240
   ttactcaaac tgtataagga aatagtgact gattgttcag gtttggcatt ttcattgcta 300
   cctgcctgca gaattaatgc cctcttcctt gtctaagata ttactgtgtt aagtgtcctg 360
   ttaattataa atagttcaaa atggacagac tgtcaacttg aaatttactt atgtaaaaag 420
   cttaggtgat tcttagggtt tccatgttca taactttaca aagctttata aaaataaaat 480
   tgcaacttaa tagagctaat taacttgtat ttgtataaaa agaaaaaaga attgcagctc 540
   gatattgtga agtttttcaa taacttcatt aaaccatatt tatgatggga gggaccagac 600
   attctatagt aataatgtat agtgctgtgt ataattccat ggtttcttca acatcttatc 660
   aaccaagtaa aattaataca agatacgcaa aagatagtaa aataagaatc taattatagg 720
   tgcaagggga ctcaggctta tgctggaaga atctgacaag tggtatagtt tgtttttcta 780
   ggaagaattt actgatgagt cacataactt gcatgtaata ttaggttctc attttttagc 840
   ttcgaaactg tgtccatgca aagactctat aactgttaag acttgtgtgg ttgaattttg 900
   acttctttga tattcagcat ttagtgcata cattttgcaa ctagggaatt tgattttcta 960
   tacccacaat aatatttatg gctaacattt attaggcact tactatgtgc taggcactgt 1020
   aagcacttta catgcataat ctcggtattc cctgtgagta cagggttaat tatttacctc 1080
   tatttcacaa atgagataat gaagtgggat gaagtgcgag gttaagcaac ttgcttgaag 1140
   tcataggtag taaatcgtgg ggccaatttt aacccagaca gaccactgac tccagttcat 1200
   gcttttgctg cctcactttt ttaagtggta tttttaatta ggaagaccat gctaaagata 1260
   ctttcaagga taaatgatta ttttctcact tcaattgttg gtttaaaatt agcataaata 1320
   ggtaaaacca gcatgctcaa acactgagct caaacattaa cattactaat aaaaaaaaaa 1380
   aagagtgact ttaaaagttt ctttctatcc agggtttctc ttgggatact catatggtat 1440
   attactggct tatatttcaa aattatttta ttcaacatga ttgactttgg ccttttataa 1500
   tttacataaa acataatttt cctcagttct gtaatccaga ttttccccat tgagtaaata 1560
   atacaattaa atttacatat ggtaatttag acatttaata ggatattgca taggtagaat 1620
   actttgtcag tacttagtta ctacctatat gtatttttgt gttacttttc agtgatttaa 1680
   agaaatctaa cagaaatctg cttaaatttg ttttaaatag tgaatatcct gcttgctatg 1740
   gaatgaataa acaggtaaat ttgatatgaa aaa 1773
<210> 101
   <211> 1641
   <212> DNA
   <213> Homo sapiens
<220>
   <223> nbla22394
<400> 101
   aaaaaaaaat gattagttaa gtgcatacat tatgaaactt acagaataaa acttattata 60
   catctctttc ttaaattaat atctttacac attttcaact ggctccccaa gtctgataag 120
   gaaggattaa aagaaaaaag aaatgtatta gttgggtggc caaggagttt cctttgtaat 180
   gttgagagac ttccgctttc tgaatttcgc tggttctcta aggtaaaaga gttaaatagt 240
   acccttgttc accaaggaaa gtgatccaaa ctatatatct agtgcagata tttcctttgc 300
   attatttagt cttctctgga gagaaaatac agtttcccct tcctctttct cttcacattt 360
   actcttttca acccaaaata agagacatag aaagcaaacc acagccagtt tggcatcttc 420
   tcagtgctac tagtataggc acatacacat acacagtctc agcaaggtta taaagaaccc 480
   tgtcaggtcc acttgcaaca tggccttgct acttggatta gctcctttaa gcctgaaaat 540
   aactttcctg gtcatggaag aactggacgc atcttttaac ttatgaaata gaagttgaac 600
   ttgaaaactc tttttaaaaa atcctggttt tgcaggacag ctacataatg aatgtatata 660
   ttaagactgt agctgaattg cacatgaaat cagattgcca acttcttgac tttcaatgtt 720
   agacatttat ccttaagttg tgagcgatat atgtagcatg ctgtgaaatg tctgttatag 780
   ctctttaatt catcagtatt aatacagaat tatcatttgc gtttcttggt actttttatt 840
   caatgtaatc agaagctgtg atgttttgcc tttgtagtcc tgtgctttgt tactgtaatt 900
   tttttttttt tttacgaagc acgtgactgg actaatgtaa ggcagatgac gtgatcttta 960
   agactgctat atatatcagt ctcttactct ataaggtttt aaattagaat aagcttttat 1020
   caaatagata attgatgcaa tttaggattc acgcaagttt cagtgtcaaa tggcggtctt 1080
   atagtttcaa ttctgaaaat agcaaactta ataaacagcc actttaaact tgttctggca 1140
   aaccagaccc tgctgtagat atagtctaag gtagttaacc atataagcct tttcaactct 1200
   taatgccctc cacatgaatc agcagttaag aaggttctag aacccatgaa agcttttgta 1260
   tgtattacta ggttttgttt ttcttatgtt tgctgatttt acagttctga ctaaagctga 1320
   cctaaatgga tcagtttatg tgtaatattc tagtgcttta atgactcttt ttttctttgg 1380
   agggagggta acattatttg gacagatgca gaaggaactg ttagtgagtc aagacaaaca 1440
   catctgaaat aaaggaactg tgtattaaca tgttaacaat tcataactgc actttttatg 1500
   acattttgaa aatctattta taggtacaga acaatgggtt ttgttaaact gtatcacatt 1560
   tatacttgca gaaatttatt tcattgttat tagtaggaat tttattggtt caataaaatt 1620
   ggcaaaactg aacaccaaaa a 1641
<210> 102
   <211> 2960
   <212> DNA
   <213> Homo sapiens
<220>
   <223> nbla22423
<400> 102
   ttggggcata tcgctgcatc agagaatcca cagagcaatg caaatagaga aaaaacaaag 60
   ttagaagaag gaaatatgcc aaccacttga ctagagagga aaaagaaaat ttattcaggg 120
   aagaaagcca cagaagtgtc cctttgtgct tttctagttc ctttaggaga ttttgtctct 180
   cacacattca tcatgtttgg gccaagccca ctgggtgcag cggtgcagct cgggaagcat 240
   cggggtgagc ttcaaggaca gagtttcttc cagtcctaag ttgtctgata tgtttgttca 300
   taaaactgcc ctttctctga cttttcaggc cacgaccccc agccagaaat tatcgttttc 360
   cccactcttt atattataat gacaataaga tttttcagtg ggggagcatc acatatgcaa 420
   tcaggtggca gaaaaagttc ctgcaatatg aatttagaga tttgattacc cagcacatgt 480
   ttctgtcctg tctctaacag tctctggaat ctggtagacc ttcctgaata ttttgctttg 540
   tctgatgatg actttaacat attgctgctg gtgtgcatcc gtgtgtatac tggacagcag 600
   gaaactagcc tgtgccactg cccagctcag cagcagaaca agaggtcttt gatgaccgta 660
   agtttaagaa atataaatat gttctgcacc acagaatata cagaacaaga ttcatcctag 720
   ctagaaatat atcataatct tgaatgtgct ttttaaagcc actgcaccaa gccataaacc 780
   tcttcttttt aagtttattg ggtagtcagt ttctagcttc ggtcactgct aaggaagaca 840
   aaggaggata ctgtcagatt cttcctgctc aaaatgttct ccatcctggc agtatatcag 900
   agcaggtcaa caactcaaca gcttgcatct cagaactact gggcttttct aggtgccctg 960
   ctctctcccc tcccccgtcc tttgttcttc aaggtctttc catgcctacc acctgaggtt 1020
   ggagccctcg ggcatttttt agttctgcca aagcacatag tcattgaaag acctgcgtga 1080
   tccccgtaac tggcaagcca caacctcttc tctcaaatga cctccttctg aaagttttca 1140
   gaggaaagag gattgaacag agagggacag atgatcacag atatcttgaa attgccaaag 1200
   ggagtagact tgttatgaaa tgctgtgagc cagacacgaa gggaaaaaac caggacagct 1260
   catttgggca gagagcaaag acaaagcctt caatcctatt caggagctga gccctgcagg 1320
   aaacccactg cctctagcca cagtggagag gtgcaggcac agtgtggttg gctactcatc 1380
   ggaggtgatg cgggggttgt ctgagaatgg agggtaggaa tgatctttat ctgagtccct 1440
   tctacctgag aacagaacag aacacacacg cacacacaca cacttttgta taaaaagata 1500
   gataggaatt taattttcat aatgaaacat atcaaatctt ttgatatgtt cactattatt 1560
   gcttagtggt gcacctttaa atacattcat tttaattaaa aagtggatca agttaagcaa 1620
   actaaatggt agagtttata caaacagagt tgcaatgcaa ggactaaggt tcttagatct 1680
   acagagtctc tcatacttgg aagtgaagct atagatgttt tttgaggtgg aatctcgctc 1740
   tgtcgcccag gctggagcac agtagcacga tctcagctca cttgcaacct ccgcttccag 1800
   ggttcaaggg actcttcaac ctcagcctcc tgagtaactg ggattacagg cactcgccac 1860
   catgcccagc taatccatgt attttagtag agatggggtt tcgccatgtt ggccaggctg 1920
   gtctcaaact cctgacttca agagatccac ctgccttggc ctcccaaagt gctgggatta 1980
   caggtgtgac ccaccatgac tgacccctga agctataggt tttatgaggc tagaagttga 2040
   ccaaggagtg gaaaacaagc attgcttaac tgaaccaaga catctgttgg ttgaccttct 2100
   cagaaagaga ccaaaaagta tagcatttga tcaaaagata actattaata ttacaaatga 2160
   aaagagggag agaaagaaat tataatgaac tgttaaaaag aattgacaaa cggatagaaa 2220
   ctggaataac atagtgaggt gtgacaatgg taagagcaga gagaaagagt gagaggatat 2280
   agagtataat gttaaccttg ttccttttta ttaagaacat cctaagcgtc ctaacattag 2340
   acgcaaccat gagggccgcc tagcaaatat gtcttgagat tccagtgcat ttttatacca 2400
   ttcctaaatt ctgtataaca agtttctggt taacaccatg gctaaacaca attatttctg 2460
   aattcctgtc actctgccac ccatatgttt taaaacaaag aggtatcctc atttcactga 2520
   tgtttaaact caggaatgag atgtgtcagt agctttggga acatgtaaag ctggaaagta 2580
   ggaattcttt aaataaaaac tcctagtctt tcttcctgag accttgcttt cagtgtgagg 2640
   tggctgagga ttggcatttg acttgccgtc cccagtcacc atagtggaga cctcagtcca 2700
   ccaagaaatc aggcgaatgc tgtgtttgca atgggagaga caagatgttg agtgttttac 2760
   ctgtattacg tcatctctcc tcaccacagc ccttgaaaca aggaatctta cctctatttt 2820
   tctgttgttc cagaagagaa acttttttgg gagacatagc ctccctgtat cacccagaag 2880
   gcagaggtta gagtgagccg agatcatgtc actgcactcc agccgaggtg acagagcaag 2940
   actctgtctc aaaagaaaaa 2960
<210> 103
   <211> 2920
   <212> DNA
   <213> Homo sapiens
<220>
   <223> nbla22439
<400> 103
<210> 104
   <211> 1522
   <212> DNA
   <213> Homo sapiens
<220>
   <223> nbla22633
<400> 104
   tcaaggctct cccaggagtc cccctctgcc ggccccccaa tgccccagct ccctcctact 60
   cgctggagat ccagtggtgg tatgtacgga gccaccggga ctggaccgac aagcaggcgt 120
   gggcctcgaa ccagctaaaa gcatctcagc aggaagacgc agggaaggag gcaaccaaaa 180
   taagtgtggt caaggtggtg ggcagcaaca tctcccacaa gctgcgcctg tcccgggtga 240
   agcccacgga cgaaggcacc tacgagtgcc gcgtcatcga cttcagcgac ggcaaggccc 300
   ggcaccacaa ggtcaaggcc tacctgcggg tgcagccagg ggagaactcc gtcctgcatc 360
   tgcccgaagc ccctcccgcc gcgcccgccc cgccgccccc caagccaggc aaggagctga 420
   ggaagcgctc ggtggaccag gaggcctgca gcctctagac tgatgcccct gcccccgccc 480
   atccgccccc acgctgtaca gagtgcatga ggagccgccg gaccaccggg gaccgactgc 540
   ctgcgtccag ccgcgcccca tccccgaggc cgcctgtggc caccatgtcg gccctctttc 600
   caccacccct tgctcagcat gtaagcccca cccacccctg ccctttcaga cccctgcggt 660
   gacctggctc ggagaaggtg gccctgggca ccaaggggcc aaccgccctg aacactgggg 720
   cagggaccat gctggggccc ggggccaccc ccttcctgtc accagcttct gtggagtcca 780
   gtgttttgct ttgcttgctt gtcccccatc ctgtcctgag ccggggcccc ccagcctcgc 840
   ctccctcctc ctaccatccc tcacttggac ctgggggtgt ggacagtgac ccctccctga 900
   atatggactt gaatcttctg agcagaacta gggcctctcc cctggtgaag acccagggaa 960
   cccaggaggg cccttctggg gcagtggctc tgcagggtca ctcatggagg cctaggggaa 1020
   cagcgagatg ccccaccacc tcctggcgag tccttcctgt tcagctccct gtgcgaccct 1080
   ccagggatgc aggggatcca ggattctctg ccctgtcaca cggcgagtca gaagggaggg 1140
   gcctttccct cggacccatg gccccaggca gagttttgca ccagcaggac ccctttgagg 1200
   gccttcaagg ctctcccagg agtccctctt gggtcctgtg ccaagtccgc cccagggcct 1260
   ggggctgttg ggagccaagg gccccctggt actcagttcc ctcacgattc ccgatcacgg 1320
   gcacacctgc cccctggtta tttgtaaata tttctattgg acccaattct cctcggaatt 1380
   ggctggcacc tctggctgcc gcagctcagt gatgacgtgg gggaggtggg agaggccgag 1440
   ggctttgcct aggggtgggt tgccctgtat acatgatcca gtctgtgact accagccaac 1500
   ctgaataaag cggttttaaa aa                                          1522
<210> 105
   <211> 2914
   <212> DNA
   <213> Homo sapiens
<220>
   <223> nbla22698
<400> 105
   gttttaagaa actgactgtg gctccagagt atgttggaga agtgaaaatg gagactagga 60
   ataacaggtg ggagactatt agtctaatta agatgtaatt ataaatctaa gctaggaacg 120
   taaaatgaga atgcaaagta agaaacaaat atggggaaaa ttatatgtaa aagtaatagg 180
   acttggcatc ttactgatgt gattgattat gagaaaaatg aagcatgtgg aggagtccac 240
   tggacagtag gaaattcagc ctaagacttg ggtaagagtt ctgtggagtt gtgaattcag 300
   aggccagaga tgtgatattt aaaattttgg ttcaagattt cccaggtata agaaagcaag 360
   aggattaaag cattgtaatt aaactttaag cagtgcatat ttatgttata gataagataa 420
   acaagaaatc tagggatcaa ataggattaa aattagtagt gatcattcag tacagtagtt 480
   acgtactgtt attcacaaga gtatataaat caaattacaa ggaattaagg atataaacgt 540
   gataagaaag tatgcactgt actctttgag gaagtttgcc atagaaagga agaagaaata 600
   ggatggtaga tcagaagtaa agcaggaccc agtgggggga gtgtttgcag tgaggcagta 660
   tgtataatca tttaaaacat gggtttggag tcctctcagg ttccatgttt gtaatggaca 720
   taatgataat aatccctttc atttaaggct gttgtgagga ttaaatgtgt taatgtgcaa 780
   ataactttac acagtgcctg gtatataata aatgcttgct acctattaac tagtatttgt 840
   ttctaaggct aatttaagtc ctagaattga ttgcaaggat tagatcagga gtatagtgga 900
   catgttggga tttaaatatt taaatataga gatgcttttt aggaccattg ttagaaccag 960
   aagagatttt ttaccaagtt cacacagaaa tgtaggtgca ttggctgggc atggtggctc 1020
   acacctgcaa tcccagcact tgggaaggct gaggcagaag aactgcttga ggccaacatt 1080
   ttgagaccag cctgggcaac atattaagac cccgtctcca ccaaaaaaaa aaaaaaaaag 1140
   aagtaggtgc agagctggaa gcagaaccga aatcatcagt gttacagtca ttattctttc 1200
   ctgtcaccat tatatgtctt tatgaagcaa gggagaaaga agaacagatg aaagaagtga 1260
   ggattttgaa gttggttgaa agatttgatt gaattctgat ctaaaaatta taaggcactt 1320
   gtttaacaag ttgaaagtag gaaagtagac ataagactct actagatttg gggaaactct 1380
   caaaaatgga ctggaaattc agctaaaagt ggataacaaa atatttctag aattagcatt 1440
   tgtggggtgt gtgtgttttc actctagtat ttgtcaagcc cagatgaaag catagacaga 1500
   atgtaagact ggatttatct aagtctggaa ttgtgtaaca ttaaaggaat agtagcaaat 1560
   gagcagagtg ttggctcaag cctaagcttg agcctaagct tgactctatg gtaaagtcaa 1620
   gtcaagggag aatagaaagg gggtcaccat aaaggtcaaa agtgggttta gtggttgtgt 1680
   gggaataggc agatcaagaa aagaatgaag ttaggaaagg agatataagt gttgaatgac 1740
   cattacaaaa agagacagag gaaagaaaaa tgaagatgta tcaaaagaag ttgctaatat 1800
   ggatggcaaa gtagatgttt ttaagaaatc atgagaccag agtcttggaa aagtcatagg 1860
   atgatgcagg gaatggagaa gagggaaata aagccaggtg ctgaagtctt tatgtaatgg 1920
   gaggagatgt tccagtaatc caatggctat tttgatggga aagagtgtgg tatgattggg 1980
   tggcattgac atcggaagcc atcctcattg atggtggtgg aacagcagtt tgaaagtaac 2040
   attgtgcggt gaggtagagt ggcacatgat gcatccttat tcttaccttt ggagaaaagt 2100
   tgagggagac caaaaatgac tttttgaggg aattgtagaa gtttcattag aagaaaagta 2160
   agtttttaat taaaaagtta atctgaggaa caggtagaat aaaagtgtag ttgttagtgg 2220
   tagaagagaa tggattccat agggcaaaat aagaactcaa gggaaggttg gtggaagagg 2280
   aagaggattg aattgtttca agaaagaata gcagttgtca tccttatgaa aagtaaaatt 2340
   tttattttca aatcaggaaa tgtaaaatgt gccttccaga ccccttggtg gtatacatgg 2400
   gagattggtt ctaggacaca cacagtccca tcccccaccc tctgacccca tacaccccct 2460
   ggatactcaa atccactgat gctcaagttc cttgcataaa atggtatagt gtttgcatgt 2520
   gacctataca caacctctta tgtgtacttt aaatcatctc tagattactt atattaccca 2580
   gtacaatata aatgttatgt aaatggttgt tatagtgtat tgtttaggga ataatgacaa 2640
   gaacaacttt ctatacattt gcagtacacc attgttttac cccaaatatt ttttgatcca 2700
   aggttggttg aatcggaacc cagagataca gagggctgac tatactttaa gaattagaat 2760
   tagctgggtg tggtggtggg tgcctgtagt cccagctact cgggaggctg acgcaggaga 2820
   aaggcgtgaa cccgggaggt ggagcttgca gtgagccgag atcgtgccac tgcactccag 2880
   cctgggcgac agagcgagac tctgtctcta aaaa 2914
<210> 106
   <211> 1696
   <212> DNA
   <213> Homo sapiens
<220>
   <223> nbla22896
<400> 106
   catgtagcaa atctgagaat tgaaaactgc agataaccgg ccgggtatgg tgactcatgc 60
   ctgtaatcct agcactttgg gaggccgagg tgggtggacc acctgaggtt aggacttcaa 120
   gaccagcctg gccaacatgg tgaaacccca tctgtactaa aaatacaaaa atttgcctgg 180
   tgtggtggtg catgcctcta gtcctagcta ctcgggaggc tgaggcacga gaatcacttg 240
   aacctgggag gcggaggttg cagtgagtcg agataacact actgcattcc agcctgggtg 300
   acagagtgag actccacctc aaaaaaaaaa aaaaaaaaaa aaacagaaag aaagaaaaag 360
   aaaactgcag ataaccctat acattaatac tggtatctcg aggtgactct tctgaccaag 420
   ggtggttaag tgacacatag aacttttcta agagaagaca gacaagttga caggcatgcc 480
   ttgtactcag ctgtgttcat gtggtggtct gtggaaagaa aagaagactc atttggaaat 540
   gaagctgtcc ctttccaagc agtctctggt gcttttcttc tctcaaaatg gatccgataa 600
   atatttgaat agagcagatt gtagaatgtc gtgctgtcac cagaaagctg ctgttttggg 660
   ttctgcattg agccaaatat gtagaggacc taccaagccc actgagggac taggttttca 720
   tgtctctagt catacctaga atgttctgag ccgtctgagg gccttcatgc cggcagcagc 780
   tagcaaagcc agaaagcaag tctaacagga tctaagatga ccatcaggag aaggagtttg 840
   agactgtgta tgcaaccccc aatagacccc cttttactct gatctggaga atgtatctgg 900
   cttcatattt tcaagtcaca tgtctctcag acccctggat tcagaaccca aggccacaaa 960
   tcataggcat gaagcacttt cttaagactg acctaacgct ggattatttc ccgtccaatg 1020
   cctgcatgct gcttgaattg ctccacccac acctccatga ccaagggcgc cagagtgctg 1080
   caactggggc gtgggccgct ctctgctttt cctgtctgac tctgacaagt cctccctcac 1140
   tgaatgtaga atcgttgcca agtttctgag aagtgtcgat tccctgttaa catggatatc 1200
   agttctgcct cacatttccc acttgaggtt gaggcgtact ggagacaaca cctcagacca 1260
   tctgaacccc atcagtggat gaaaatgggg ctgttaatat actctaaaag ccatactaaa 1320
   aatgctctga gggaactggc taagaatagt gggcctggtg attgtctatc acgcaaggct 1380
   ttgttttgta ctgttcagaa atctgtcacc tttctgcctg cccttgtttc ctgaatgaaa 1440
   tgcttctggg gttatttatg aaaggagtga tcctggggca ggcaggaggc agtgggcttc 1500
   atggctcctt gaagttatta ctgatcttga ccttctcttt ggctaccttt agacaaagaa 1560
   tacgccaatc aatacttggg gctctaagtt ttacaattga tatttatttg tatcatctct 1620
   ttgtctagga atgtaaaagt gattctaaac taagatgtgt aataaaaatc aatcagattt 1680
   attgtaccta caaaaa                                                  1696
<210> 107
   <211> 1742
   <212> DNA
   <213> Homo sapiens
<220>
   <223> nbla23167
<400> 107
   gagcatacac agggaggctt cactgggaga ccacattgac ccatggggcc tggaccacga 60
   gtgggacagg gctcaacagc ctctgaaaat cattccccat tctgcaggat ccgttcccct 120
   ggcagcagaa ggtcaggttt gccaaaggaa tcgcctccgg aatggtgagt cccaccaaca 180
   aacctgccag cagggcgaga gtagggagag gtgtgagaat tgtgggcttc actggaaggt 240
   agagacccct tcctatgcaa cttgtgtggg ctgggtcagc agctattcat tgagtttgtc 300
   tgtgtcactg aaactgaccc cagccaactg ttctcagttc acagccctgt tttcaaagaa 360
   ttacacatct ctaaaggcaa acagggcacg gacaaggcaa actggagagg caaactgtag 420
   cctgagatgg cctgggcttg ccatcacagg tattcaggtg ctgagggccc ttagaccaac 480
   tagagcacct cactgcctag gaaatcaatg aaggggaaat gagttctagc ggagccctga 540
   aggatcagaa ttggataaag ttcttattgg cagagaggca ccaggattga agtgacagga 600
   gcaaagacct gggaggaaag aggagaaaat catctatttc acctggaaac aaatgattcc 660
   aagcatagaa ataataacag ctgacaagta ctgagtgccc tctatatgct aggcactggg 720
   ctgagggatt aacatgcatg tgcatgttta ttcctcatga caaccttggt ttccagataa 780
   gctggactgg aaagggacag agctgggatc ctgggctaat cagtctggtc gccaagcctg 840
   agactttagc cactgccctt cacatggggg tccatgaaaa tagtagtagt ctggaacagt 900
   ttgggggtac atcaaggtcg ctgtgtttta agctatggag tctggactat aggagacaaa 960
   tgtaaaagag ttttttggtt gactggcttt ttggtttttt tgtttgtttg tttgtttgtt 1020
   tttttgtttt tttttcctgt ttctggggct tgaatcagga aggaggtttt tttgttgttg 1080
   ttgttttgag aaaggatatt gctctgttgc ccagactgga gtgcagtggc acgatcatgg 1140
   ctcactacag cttcgacctc ctgggctcaa gcaatcctcc tgccttagcc tcccaagtag 1200
   ctggactaca ggtgtgtacc accacaccta attttttgaa tttttttttc tttttttttt 1260
   tttttttttt tggtagagac aggttctcac tttgttgccc aggctgatct caaactcctg 1320
   ggctcaagca ttcctcctgc ctcgccctcc caaagtgttg ggattacagt tgtgagccac 1380
   catgcccggc aggaaaagat ttttaagcaa gaaagcttaa gagctgtggt ttttccaaaa 1440
   tgagtctggg ctggcacagt ggctcatgcc tgtaatccca gcactttttt gggaggccga 1500
   ggtgagtgga tcacttgagg tcaggagttt gagaccagcc tggccaactg gtgaaaccct 1560
   gtttctacta aagaaaaaaa tgcaaaaatt agctgggcgt ggtggtgcac gcctgtagtc 1620
   ccagctactc aggaggccga ggcaggagaa tagcttgaac ctgggaggca gaagttgcag 1680
   tgagccaaga tcacaccact gcattccagc ctgggtgaca gagtgagact tcatctcaaa 1740
   aa                                                                1742
<210> 108
   <211> 1416
   <212> DNA
   <213> Homo sapiens
<220>
   <223> nbla23339
<400> 108
   tttgctagag ttacatggat tatatatttc ttaaagggaa aaatttgaga gtatcatgga 60
   ctaccaccag cattattatt acagtagtta ctcagatttg gttaaggaag cccaagcaat 120
   gtatagtgaa aggattatta tctctctgct aagattcaga tattgtttca gaaatctcag 180
   ctccagtaat tccacaacat ctaaaaacaa atgtttgtga tcatgtgtaa gcatgaaatt 240
   gttccaagta agtgaggata ttttagttat gtgaaagaca gtttcatgga aggtatttgt 300
   tttataccag tggctgggat ggtggaattg gggttatttc tacaattatt cttagacgat 360
   tactaaactg ttaagaaatg ccccatatca tttttgtatc taggaaagaa aaaaatcagt 420
   ttcatactgt tgtcatctgt cagaaatgct cattttattt tgaattaaat gtggcttttg 480
   aagtacctag ttaccttgaa ttcctggtga ccacatgttt ttatctggaa aacctggaga 540
   aagttatctg tcccatctcc cctgcttgtt tttttttttt ttttttggtt ggagctgctg 600
   tttagatgat gcttttacta tgcaggagag agtttttgtt aaggatatat ttgaagattg 660
   gcttttccat attgtccttc attctttgac catggcaaag tgtacagtag attttcatga 720
   tcattgcata tttcttgtca ttgaaatgta tcttttatgt ttttaaatgc attcatttta 780
   cacttgtgag tttatcattg actttaagag gtagaaatga aaaatgaaaa ttaaagctaa 840
   agccttttta tctattaatg cagatatatt agaataagaa tattttgggt ttgtgtttat 900
   tttttaatga atttatgttt acttgatatg gaaaattacg ctttataggt ggaaaagtag 960
   caaataaaga ttaagtaaaa gtaagtgaaa atgatgggga atatagtatt ggaattttat 1020
   agctagttaa aacaataagt atcatctaat ttgggtgttt attttgcaga tgagaaaaca 1080
   gacctagaac cgtggcatgt tttgcctgaa acatacagtg agttagagac agggcctaag 1140
   atagcttcta gcatcagatc aatcccaaga atccatcagc aacctcagac caacccaaga 1200
   agataattta aatctatact gcttattggt caatatattt ggttctagta ttaataaaga 1260
   aaaatgttat taaaatagca tacatagtag taaaataaaa tacaaaaagt gtgttgattt 1320
   atagctgttt gagatgataa aagtgaagca aagcctgtta aatcattgga agacttggaa 1380
   aattatttta aataaacaat tacatgtaat taaaaa 1416
<210> 109
   <211> 1549
   <212> DNA
   <213> Homo sapiens
<220>
   <223> nbla23352
<400> 109
   gggattggga ggcccacgcc ctgctgcgag aagggcgcgt tctagctcct gaggaaggtg 60
   ggagtcaatc attttgacaa gtctcctgaa aggaacagct agcaggaact gaaacctttt 120
   tccatttggt ctcgtggcaa aggcagagat tgctccagca gctccacaca aaatgatgtg 180
   ctcacgggtg ccctctgaac agtcttctgg tacctctctc ttgcctaaag acggtgcccc 240
   attttcttgg gattccttgg atgaggatgg attggatgac tccttgctgg agctgtcaga 300
   gggagaagaa gatgatggtg atgtaaatta cacagaggaa gagattgatg cactgttgaa 360
   ggaagatgac ccatcatatg agcagtcttc tggggaagat gatggtgggc atgttgagaa 420
   gggagaaaga gggagtcaaa ttctacttga tactccccga gagaaaaatt catcgtacag 480
   cctgggacca gtagctgaga ctcctgacct cttcaaacta cctcagctaa gtacatcaag 540
   tggtcatgga ccagctcata ctaaaccatt aaacagacgc tctgtactag aaaagaatct 600
   tataaaagta actgttgcac catttaatcc aacagtttgt gatgctctgc ttgataagga 660
   cgagactgat tcgtccaaag atactgaaaa actctcttcc cttggagaag agatgagaga 720
   agatggtctt agcccaaatg aaagcaaact ttgtactgaa tctgaaggga tcagccccaa 780
   taactctgcc tggaatgggc cccagctctc ttcttcaaac aataactttc aacagactgt 840
   ctctgataaa aatatgcctg acagtgagaa ccctacgtct gtattctctc ggatctcaga 900
   ccattcagag actcctaata tggagttatc ctgcagaaat ggtggttcac acaagtcaag 960
   ttgtgaaatg agatctctgg ttgtttccac ctcatcaaac aaacaggatg ttcttaacaa 1020
   ggattctggg aagatgaaag gccatgagag aagactaggc aaagtcattc ctgttctaca 1080
   aactaagacc aggactaatg ttccgacgtt ttcacagtca aatctagaac agcagaagca 1140
   gctttatctc aggagtgtca ttgctcatat agaagaccca gaggacacta accaaggtat 1200
   ctcgggggag ctttgtgcct tgatggatca agttcatcat atgcagcact caaaatggca 1260
   gcatccttcg gacctcacca cgcgaaacta cgcccgccga cagaaacatc tgcaaagata 1320
   cagtctgact cagtgggttg acaggaacat gcgaagccac catcggttcc agcgtctccc 1380
   agacttctcg tacagttaat ttgtgtcatc ccatcagcaa tgaaggtccc tatccagggt 1440
   cctgcttgga gcagcatttc atgttctttt gctgttttgt gctttgccga ttttggattt 1500
   tatttttcac aaaattttta tttaaaaaac tcgtcacctt ttggaaaaa 1549
<210> 110
   <211> 1797
   <212> DNA
   <213> Homo sapiens
<220>
   <223> nbla23575
<400> 110
   gaggatgatt aaaataatgt gcatatatgt tgaagggcag aggatggtat tgcacaatat 60
   agatgaaata gtcattggtt tgttttacat tctatgcatt tttaatgagc aaattcccat 120
   ttacaggaat taaatgttcc agatattgat ttcagaggga caatatataa tatgaaaaca 180
   aaattcagta acattatgtg atgattacat gatgtgtaat tcaatatagc tagaaccctg 240
   gaaagtgaat aatataacca ttcctataaa atatttcaga aaatcaaatt tattccctga 300
   agtacattat aataaaacgg aaacagtgtt acttgattta tagtcctcta attcaggctt 360
   ttaaagctat tttcatgtca aaaataaggg attctttctc cccttgtccc cagtcttgtg 420
   catagtttat aatgacaaga aaagctacaa aagaaacatt acaaagcaga tgtgctccca 480
   agtttgttcc agtttaaact tcagctttaa gcatcttgtg gctatgaaat attcatgtaa 540
   attatgtaag tgcatctagt ttagatccca gtcactcatg ggttttctca caaagtaaaa 600
   taccatactt gatcctgtct atttctagag agtgaatgct cacctggtgg atttgtacca 660
   accccttagg gcatcagggg gacaatcaat taggttcact gggtgtttta cctgacagat 720
   actctcctaa atactttcaa atgccctctc attttgttct cacaggacct gaagaagtag 780
   gtgtcatttt catccacact ttgcaggagg aaacaaatga ggctcagtaa ggttttagta 840
   acttactggt tgtcatacat gaacagccag gtttcaaact caggaatcaa cagggctgcc 900
   ctgactactg ggctactctc cctacattag atgcctagaa ggtatgcaag tggctggagt 960
   aggggcaccg acttccatga atggttagga gtttggtgta tgagcccctg acccatgctg 1020
   aagtgactca ggaaaagcct agtcctggga aacttacgtt ttgtattttt tttctcttta 1080
   acagttggta ctgaaggatt aaaattatct taaggttaaa aacaggaatg gttgagcatt 1140
   gcaaaaagct tttgctgtta gaatagatga catctgctgc ctggctacaa gtcattttaa 1200
   gatgacacaa aatgatgcta tggagaccac agagcttttg taagaaagca gaaacgcttg 1260
   gtcacttttc cgctaagtga cttcccttta ttggaagctg tactgaatct ggaatgctta 1320
   taaatggttg caagggcaga tcatttcaga gtaagagata tttaaaaaca aagggctaag 1380
   ggaaacctca attgaaacta gagcaataca aaataaaatc tcctactgaa ccctaaaaga 1440
   ctcctactga ctgacccctc aaaagcaccc catatgtctt tctcttctcc tctgaaaagg 1500
   taactcaggg cccggcgtgg tggctcacac ctgtaatccc agcactttgg gaggccgagg 1560
   cgggcggatc acgaggtcag gagatcaaga ccttcctggc taacatggtg aaaccccgtc 1620
   tctactaaaa atacaaaaaa ttagccgggt gtggtatcag gcgcctgtag tcccagctac 1680
   tcgggaggct gaggcagggg aatggcgtga acccgggagg cggagcttgc aatgagccga 1740
   gatcgcacca ctgcactcca gactgggcaa aggagcgaaa ctcagtctca acaaaaa 1797
<210> 111
   <211> 1957
   <212> DNA
   <213> Homo sapiens
<220>
   <223> nbla23592
<400> 111
   ctaaacacat cgagttgcac acagatggaa ataacattta tgttaaattc tacaagtgtc 60
   ctctttgcac ttatgaaact cgtcggaaac gtgatgtgat acgacatata actgtggttc 120
   ataaaaagtc atctcgttat cttgggaaaa taacagccag tttagagatc agagctataa 180
   aaaagcctat tgattttgtt ctaaataaag tggcaaaaag aggcccttcg agggatgaag 240
   caaaacatag tgattcaaaa catgatggca cttctaactc tcctagtaaa aagtatgaag 300
   tagctgacgt cggtattgaa gtaaaagtca caaaaaactt ttctcttcac agatgcaata 360
   aatgtggaaa ggcatttgcc aaaaagactt accttgaaca tcataagaaa actcataagg 420
   caaatgcttc caattcacct gaaggaaaca aaaccaaagg ccgaagtaca agatctaagg 480
   ctcttgtctg ataacttcaa gtgatgtacg aaaaggtttg gagttcattt ttgtggaaag 540
   actttaaatt ggtgttagaa ccactaaaca tcttcaaatg gtactatgag gaaaaaaaga 600
   aaaacatttt tctaaatatt caactataac tgctgttttc tgactaaaat aaccatctaa 660
   ccacttgttt ctaaggcact gcctattcca gcactttcaa gtagctgtga tattacatgt 720
   tgtcatcaca gtccatcagc tatccaccct tgaccttgtg catttggtcg acagtttcta 780
   caaaaatgtt acaaattttg ttttctaaac aatttgttga ttaagtgatc aacaacctga 840
   agaaaatatc aatttttaat tgacaaagac tttatatctt agtgatttta gttttgtttc 900
   tctttatttg gcaacatttt catctgaatt gtatagatat atgattttct agtgagtgta 960
   tgttaggaac aaaagacaaa atagtatcaa cacattataa atatttagct tactaaatat 1020
   ttgtaattat ttttacatcc atttatttct agcttgttct ccagcacttc agtgtttgaa 1080
   agtttcatcc taaaatatat actacaggaa agctgcagtt cattttcatg catggatcat 1140
   tacatttttc acttgtaaat gtaggttttt atgaaaatta aacattcccc tatttttctt 1200
   taaattttat acaaagcact ttaatgatag atgcaacctt atttttcagt tcctattttt 1260
   ttaaagacca cacatttact aatgttaata tgaaggtaat aaatagctta ctgatatttt 1320
   atggatgcag acaatccatg cacaaccact tcttatgata ctagtttatt tccttaaata 1380
   ttgctacaaa aggaagatgc gggtgtaagc cctgattttt ttttctccca agaaaaatct 1440
   taaaggacca ctttagataa tatttgattc ctactgtaaa atttagaaaa tgatgaattc 1500
   ttgtccattt ttgtaatcaa gattttagga aaaacagaag tacatctatc tttatgaaat 1560
   tttgggcagg tttttgtgta tcaatatttt gtacttttag ggaatatttt attttttagt 1620
   tatttgtgtc aaattataat tataaaaggt acagcagaaa atataccatg tttttatata 1680
   ggttcacacc tgtacttagg agggaccctg tccatctata tactttttgt ataaaatttt 1740
   aaaatgttaa agatccacaa ggtcttaata aaatgattct atagctagaa aaacatttac 1800
   cttcccagtg ctttgcacta aaatatactg tgaaaggaaa ctagaaagac tgtaactatt 1860
   gctggaaatg ttctatattg aatgtacatg ctcttgttgg aaaaatgtac tatatgtgat 1920
   ggaaataaac cagaatcgaa gttatttcag ctaaaaa                          1957
<210> 112
   <211> 1674
   <212> DNA
   <213> Homo sapiens
<220>
   <223> nbla23601
<400> 112
   gagattactt cctgctgcac tcctgtcttg ccatgcacgt cttgccccct cacttttgct 60
   cagcctagca gtctacttca ctttattgcc gtgtaagtgt caggcctcct gggtgctctg 120
   gaaaagacag ggagccaggc cctctcaccc ctactggtaa caggtcattg ctgggtgcac 180
   aagagggagg tgatttgcat catggtcatg ctgcatgggc ttcactggga tgctgttaaa 240
   caccagagga gccaacctat cagaatccca gcagcaaagg aaaactcaga ttttagaggc 300
   tttttacaat aaagtagcgt aactctaggt catgattgat ttcaaatgcc tgccatgaat 360
   gatttgtaag tatttatgta ggatccatca aagcagtatt gtaggctttt gaattgtccc 420
   agtggatccg ggaccccatt tcactgtctc tcttgatcgt gttaatgatg caatcagagt 480
   tcaagacagg ccccatgaag tctgactgca ctgggatgga gaaatgaatt tcttcccact 540
   gaaggaaact ctttctcatt cgcagccaag acgggagtgc cactgttcct ctcttcactc 600
   ctgagatact gcttctggaa gcgggtgtca cttcctctct agtacctctt ctcttctctg 660
   aagtgtgtga ctatctccta gtgtttaaat ttggcagtta ctcgccatgt atgtcagcat 720
   agaaaaggaa atgtttttac cttatctcct gtatgtatga tagaacttaa aagaaatgtg 780
   catttgtttt catagcccca gcagagaaaa tcctcttcat agattaaatg tgctgctgtg 840
   gacaggaggg aaaaaaaaaa ccctctacat attgaaaggc accaaatgta atatctgaca 900
   ctgttaagat gcccaaaaga gcaaagttgt agtggagatg cagggtcatt tccccatgcc 960
   atccacagtg tttgttagtg agtccacggc tgacttgcag tgataaagaa aagcatggag 1020
   ctgtgtctgc agacaatggt ggctgcatct gtaagtggct tcagaggcag cagccctggg 1080
   gaaattgatg ggtgtggcag tggacctgtg aagagggaga atctagcctt cagcctgtcc 1140
   agtgttaacc actagagaaa ctgagcttta tatccttttt taatgcctgt gaattttagc 1200
   atattgaaac attagagcaa atactcaggg gatttttcat taaacatccc tcagataatt 1260
   taggtatata tcattagaaa gggaaagcta tcatttttat tttaaaacta aacaaggcca 1320
   tcttataaac tgtcaccaaa gtcttccctt ttttattgca tgtgtgcctt gaatttcata 1380
   aaacattaat tcacaatggg ggtcagaatg tactcttgtt gaaacacttc ttgtaccatt 1440
   ttatgttcat attatgtttg agagggtaaa aatgtatgag cagcttaact gaagtagaac 1500
   tattcatgat gcttttcaca cattgtggca taagatgtaa agttaagttt gtaattaatg 1560
   ttaatttctg tgcattttaa tattctttta taattattaa tgttaatttc tgtgcatttt 1620
   aatattcttt tataattatg agcattttaa taaattcatt tttacaaaca aaaa 1674
<210> 113
   <211> 1490
   <212> DNA
   <213> Homo sapiens
<220>
   <223> nbla23630
<400> 113
   actcagtatg taagtagatg agatttgttg attttgcccc taaaacgggc tttagtcatt 60
   ttaggagtga gttgcacaaa aggacctaaa atgcattgtt ttttgccttc ttttaagaga 120
   tggggtctcg ctctattgcc caggctggag tgcagtgtgc tatcatacat agctcactgc 180
   agtctccaac tcctcatacc agaggcatgt gtcaccatgt cttactccta aaatgcattt 240
   ttaaaaagcg aatttttaga ttaaagtgcc tagtttctga ttaataaata gaagatgaaa 300
   aaagtgggcg ggaaaagcat aatcttttaa gatttgtaat tttctgtatg tgccacattt 360
   atgtaaatta actataaaat atggaattca ggatcatgct gttttgcatg tactttatag 420
   gttatatagc atgaaacata caaattatca ctgttcttta gtatatagct ccttgccttt 480
   tcttacatag atgcttaatt taacaattac ctatttatag ttcttattat tgacgggaat 540
   atgattagaa gtaccaaaac taaaaattcc attatgtact gtttactttt tatttaatat 600
   tacatgtttt taccttgttg cggtatcttt ggccttcaca cacacatgtg tgcgtgcacg 660
   tgcatttcat taccatgtag acaagacagt tattgcttat agtaatttac ccatttgagg 720
   gctaagtgtt ttaagctgtg gttttataag caaagctgta agtaaatgta atttatttta 780
   gaaagatatt atttgaaatc aattttgaag aattgcacta tttgataatg ctgctactac 840
   atgagataac tctggggaat taattttatg agataagatg aatggctttc tagaaggtgt 900
   tgctttttgt tttttctttt tcttttttac atttcatctt agaaaaagtt gcttatattc 960
   agcaggttgg tttgtcaaat tcagtgtttg agtttgtttc tggtcagttc agtagctgct 1020
   actttagcaa gatgtggcct ttcacaaaag aggtaagagt gaccaaatag aattttagga 1080
   caataagtat aggaaatatc tctttatcgt aagataagaa acttgaactt tttaaaggaa 1140
   atgtcctctt gaaaagaaca tttctgactg catgcagaag ggtacttaag acatatataa 1200
   caggccagga gcagtggctc acgcctgtaa tcccagcact ttgggaggcc caagtgggca 1260
   gataacctga ggtcaggagt ttgagaccag cctgaccaac atggtgaaac cccatctcta 1320
   ctaaaaatac aaaaattagc caggcatggt ggcgcatgcc tgtaatccca gctactcgag 1380
   aggctgaggc aggagaatcg cttgaacccg ggaggcggag gttgcagtta gccgagatcg 1440
   tgccattgca ctccagcctg ggcaacaaga gtaaaactct gtctcaaaaa            1490
<210> 114
   <211> 3442
   <212> DNA
   <213> Homo sapiens
<220>
   <223> nbla23754
<400> 114
   cttacaaatg tagcagttgt gaaagagtct tcagtcgtag tgtccacctt actcaacatc 60
   agaaaattca caaagagatg ccctgtaagt gtactgtatg tggcagtgac ttctgccata 120
   cttcatacct acttgaacat cagagggtcc atcatgaaga gaaagcctat gagtatgatg 180
   aatatgggtt ggcctatatt aaacaacaag gaattcattt cagagaaaag ccctatacgt 240
   gtagtgaatg tggaaaagac ttcagattga attcacatct tattcagcat caaagaattc 300
   acacaggaga gaaagcacat gaatgtaatg aatgtggaaa agctttcagt caaacctcat 360
   gccttattca gcatcacaaa atgcatagga aagagaaatc gtatgaatgt aatgagtatg 420
   agggcagttt cagtcatagc tcagatctta tcctgcaaca agaagtcctc accagacaga 480
   aagcctttga ttgtgatgta tgggaaaaga actccagtca gagagcacat ctagttcaac 540
   atcagagcat tcataccaaa gagaactcat gaatgtaatg aagatgggaa gatatttatc 600
   aaattcaggc ttcattcagc atctgagagt tcacaccagg gagaaatcat gtatgtactg 660
   catgtggtaa agccttcagt catagctcag ccattgctca gcatcagata attcacacca 720
   gagagaaacc ctctgaatgt gacgaatgaa gaaaaggtat tagtgttaaa ctcttaatcg 780
   actcctgcaa atctatacca gtgagaaatc ttacaaatgt attgaatgtg gcaaattttt 840
   catgctatta gtattttcat accttagtca catttggaga attcacatgg gaataaaatt 900
   ccattgctgc aatgaatgtg aaaaagccat cagtcaaaga aactaccttg tttagtatca 960
   aattcacgcc atgcaaaaag attataaatg taataagcat gtatgtgtgt gaggagattc 1020
   agtcataacc caacgctcat tcaacatcaa agaatttata cctaagagaa cttatttggg 1080
   tgtagtaaat ggcagatctt tcaataggag tttaactagt ctttgtcata tcagaatatc 1140
   catagtagac aagaatttga tgtaacgcaa atggaaaaac tcgacaccac atttcaggct 1200
   ttacccaaca tcgaaataat ggagagaaaa ttgttgatta tttgtttatg aaattgttaa 1260
   tacatagtcc caatcttttt cattgcacaa aaatctaggg ttgacttggt aaatgcagtg 1320
   acattttctc atggagttcc tttatttaat atgtattcta agtaggtacg tttattttta 1380
   cttttttatt ataattttga tattaaaaag aacagagatg gggtcttgct ttgttgccca 1440
   ggctggtctt gaactcctgg cctcaagcga tcctcccgcc tgtcctccca gagtgctggg 1500
   gttacaggcg tgtgtcactg tgctgggcct attttattta tagaactcat ttaagctgtt 1560
   tttattttaa tatgccctat aaacattttt atattttttg aaattggttc ttagtgttca 1620
   caacttccat aagatactgc taatgcacca gtattaaaac acatcgacgt aagtagctca 1680
   tttagctttt tctgctgttc ttggcccaag ttctttccaa aaccaactct taggcctgct 1740
   ctttactagg gatcttatgt cgtattgctt tacagccaca acacttggat tcctgttgat 1800
   taacttctcc attctcttaa gcacctttag aagatttaga agtttcctag ttttaagtgt 1860
   ttcaccagca agtattccat acctacttga tgttgctggt ctggtgtctt atttcctaaa 1920
   gtgaagcatc tttttttaaa aaagaatttg attgacaata tatccagtcc aatataagta 1980
   tgaaggattc tctctcctga gattgtagca ggcagccaaa cattttcaaa tgatgcccaa 2040
   ggttttagct gtcttgtgtg catccacagt ctgcgaagaa gacatgataa ggacatcagg 2100
   gagccaacaa gactcctaat agcctcacta cattcatcca gtgcctattc tgcatgccta 2160
   agcttagagt tcttttatat acctctacgg ccagcaaaat gctcaggtct gctcttggta 2220
   gggtaaacat aaagaagata cacaggccgg gcatggtggc tcacgcctgt aatcccagca 2280
   ctttgggagg ctgaggcgga tggatcacga ggtcaggcag tcgagaccat cctggccaac 2340
   atggtgaaac cccgtctcta ctaaaaatac aaaagttagc cgggtgtggt ggcacgcgcc 2400
   tgtaatccca gctactcagg aggctgaggc gggagaactg cttgaacctg ggaggcggag 2460
   gttgcagtga gccgagattg caccactgca ctccagcctg ggcgacagag caggactctc 2520
   tctcaaaaaa acacaaaaaa acaaaaacaa aaaaccatac acacacacac acacacacaa 2580
   atcagcatca taagggaatg tagccttcca acagagatga tgctgttcgt atgttaatct 2640
   cagagacagt atttcaagag agtggcaggt ctgttcctgg taaaatttta accattagga 2700
   ttgcagataa atgtttgaat tctgctcctc tctcatcaat ccaggacagt atttgaagtg 2760
   tgagggcttt gtgtatagtt gtttatccat taccacattt ttgtatttta atagtctaca 2820
   ggctatataa aagaacatgg ctttttgact gataaaagtg attacagatg ttggctcaag 2880
   ttcagggcca ccatcatata cctaacaaga gttcatgatt ctttaggtaa tgtcaaaaca 2940
   ttttgtattt ttccatctta agctttataa cattttgtga gtaagacaaa tgttatttaa 3000
   aattcttgtt gtcagtccag caattgaggc tttcatagtt cagtgttata atattcagta 3060
   gggaccctca acaaatacat aaaaatatgt tgctcactct ataatcctcc tatggctaac 3120
   ctctaggata gttctgccac tatattttac ttctttgcca tcagcaagag taggatttca 3180
   tcaaggcaag gtaggaatct aaatgaaatt gatatataaa tgaattgatc taaatgtaaa 3240
   agcaaatgaa aaatgcatgt gttttttcct gtcaaacatg tataccctta tgtatagaga 3300
   ccagtagtca cgtatggtga ctgaaacagg attatgtaat ccctaaaaag cagaatatgt 3360
   aaaaatcaca tgtatgcgtt tggtttagga atgtgctttt gtacttccac ttgaataaag 3420
   gtgtgtttgg tattctgaaa aa 3442
<210> 115
   <211> 2384
   <212> DNA
   <213> Homo sapiens
<220>
   <223> nbla23892
<400> 115
   atttaagttg aatgcttttg ttctttctta aagacttata aattgcttgc aataaaataa 60
   tgcaaatgaa aacacatgag ggtaaaataa taaaataaga taaaaattat ttaaagcaga 120
   agccgctagt cagggttagt aaataagctt agtggagttc atgaacccac tggaattcca 180
   tgtatatttt tgcatatctg ttttatggag gtggtccttc atacggccaa tcaattcatt 240
   gattctgagt gatttgatta tgatttgctt gcctaaaaga ataatgttta gatgattttg 300
   agcatctaag aaaacctgat agttataatt ttgaactggt ttgccttaaa gttcttgaat 360
   ataatttagg aaggtatgtt aagacacaca tatgtgtggg tgtgtacagg gggagtacaa 420
   aaaaaaccac atttttaagt tcagaaaaaa aatcattgca atttgttgta aacagcatgg 480
   actaatgata caggatgatg ttggttgaat tttcaggact agcaatgtaa ctttgcaatg 540
   gatacgtaga tgccattcaa ataagtgatt ctgttattta tcctgttttt ttaaagtaaa 600
   aatattaaac ataacttagt ttgtataaga aaaaataatt gcaggaggta aatgtaacct 660
   gtctgagata acacacaaaa ctctgatgat tgtattttgg agttaagact atgaagctaa 720
   aaaatgtgtg tgcacataat ttcaaatatt aggcccaagt aattttattt tcggaactgc 780
   tcattaatta tgggagcact cagtgtttca ggaagtgtta agacttcagg gtttcagcaa 840
   tgaaattgat aaggctcttc cctagatcta agaagagaca gacaataaac attcaaaagc 900
   aagaacataa gatactgata aattctaaga agaaaaccca gtaggatgat atacaggggt 960
   gtgactagga ggtcagagga ggttgctctg aggaggtgat gtttatgcaa atctgaatga 1020
   taggaagccc agcaagagat ctgggagcag agccttccag ggaaagggaa ggacctgtgc 1080
   aaaaccccag aggcgaagtc catctaggct tgctcaaaga caagaaagag gacaagaaca 1140
   ttaagtgtgg ggagagtggc aagaggcaag atcatcaggc aagggcgcct cagacaagac 1200
   cacgccaagg ggagagcaca gggcagagca ggactgtgtg gaaattccaa cgtgaatgac 1260
   ttccaaaatc aggacacagg ctctctcccc agcctgacct cttctggtgc ttaactaact 1320
   tgttagcaaa actccttggg gcacagcact gagtcctcca gccaggctgc ccctttgtat 1380
   tgacatggca gggatacagg aggcacgaga gactgtaact ttctagagtt agaatgtctc 1440
   tagtaactct agagacattt tagtgctaac ttacaattga tctggcaaag aaagataggc 1500
   agagctatta aagtgttcaa tttccttcca gagagattct tccattttct ctcattacaa 1560
   aaccagaaga tcagctgtgt ggggccatca gctcccagcc taaggtccta taacctgaag 1620
   cttgaaggca atcagtacct ctgctttata attgatcact ttgaggagcc aaaggaaaga 1680
   gtgaaagatt gggactgctt tgagtggaga tggcactgaa ctcgttgtaa taactacaaa 1740
   tgcaatttaa agtaaaagca tgagtatata aattgaaagg gcaggtggac agaaagaaga 1800
   gactgactcc tagacaggtg ctgagaaagc agtgtaatta aaaagataag gaagggaaag 1860
   gagctacaac atataccaca cacacacaca cacacacaca cacacacgtt atcagacatt 1920
   caaaaaatta gatcttagac tccacaatac aaatcccaga ggacaatgga ttacagtgtt 1980
   gacaggggag aaatattgtc ataaaatcat tgcatactta gttatgtttt cattgttaaa 2040
   gaaataaaca gaccattttg aggtagttaa acctcagaga agaatagcat gtatttactc 2100
   ttcttgaaat ctatgttggc tttatgcccc agctgagata ggaatcaaag gtgaggttga 2160
   aaataaatag ggataatata aaccgtccac cagattgtgt taaatctaaa gaatcgttca 2220
   gtattttatt gtatctcact gtatgtgaaa agaaacaagt ttcaccaaac aatacttagc 2280
   cttattttgt atatgcagtg cattataata ttttctattt tgttctgtct ctttttttgt 2340
   tcatgcttga cacaaaacat taaattggtt ttgcaaccta aaaa                   2384
<210> 116
   <211> 2971
   <212> DNA
   <213> Homo sapiens
<220>
   <223> nbla23956
<400> 116
   atccagataa tatttatata atgaatttct aatgggagac ctcatgctag attctgagga 60
   tttaaaaaaa taagttaaaa catagttctt tgtttttcca tggagaaaga attaactctc 120
   cctgactgag gcttcagttc catttcaaaa agacataacc tttaaaatca ttggttaact 180
   ctttgtcaat gtccctaact tacttaatca attgcacttc aatcgtggtt cttcctgatt 240
   gtttgcttac ttttttccaa ggtattgaag tgtaaaatca cacatttctg tcttcattga 300
   tgctactata tatctatatc tcagcttggc caaaactttc tctgaactct gctacagtct 360
   aacactcttg ctatgtaact ctccttcctt atccttctag gtgtaggagg tcaggcctgt 420
   atccaatgtc tattcctgtt ttctcctctt tatacttcac aagcgtttcc tctaatattt 480
   tcctctaggt ttaatcctgt tttgggatct gactcttgaa gaaccaaaat taaccaaatc 540
   ttcatcctga aaagtgaaca aatataaaat gcatttcagt tcatagccaa cacataaata 600
   atctctgctc tactgcctac tccctctttt tctcaagtct caactagctt caaaataatt 660
   tttaaaaagt cagcctcctc agctctgtga attcctgtac atgccagtct cctccattta 720
   cagccgaatt gtaaagatta acttttactt aaaaacctca agttcagtgt tgctatatcc 780
   ctggggcagc tactcacttg tattcatgtg atggtaggaa gaaggtgaag aaagatactc 840
   cagagagcta aatgcatata ttcctaggtg catctagaca cctaggaata atctggttta 900
   atttgtttta atgtacagtt gaacaaggct aggagaaaat ccagaggcta tacttcatta 960
   gtatgtgctg attacctcca atgaagtact tatacccacc tgagtttgcc agtcgtttac 1020
   ttcttctttc caatagattg ggagtttcta aaggatacat tgggtctatt gtatctgtaa 1080
   ttctagatct taatgcattg ataaagagtt caattaatac tagttgaaag aaataaaaga 1140
   ctaaaacaga aagacggaaa aaaggaagca cagaagtata gttttgtgat aaaaagagga 1200
   catagaagaa tgctaaagag tgatagttcc aaaggagata taaagataag atttttcagt 1260
   ggaagaggtt atagatgttt aagggtaaat agagaagatt ttctggtgga aacagaactt 1320
   aacccaaatt ttgtcggaat agtaagactg ctataaagga tacacaagat ggaaggctct 1380
   tcaggcaagg cagagaccga agactcatat gtaaattgca aacttaaaaa caacaatagc 1440
   aacaatagtc aagacctgac acaacagaag aattatattg tgcaatacaa atctttgttt 1500
   tgataaggat actaaaatta tcttggccaa agtgccaacc aagcaagcag taccagcagg 1560
   ggagcataac catttcatat tcttagatac agatttgtta tttgtctgtt tatttatttt 1620
   ctctgaaaag gtgatctact cagacggttt agaacctgca tatgcatggg gcaggtggat 1680
   gataatctct ccctaaccat ttggattagc tgaatcaaca cggatattca aaagactgaa 1740
   gaggttgtgg ccagatcact ttcaaataga attctacaat gttcaaaaga attcctttca 1800
   ctgtgaattt gagaaaagac aaaacagtct ctgtttgtac acttgcaaat gacagaaaag 1860
   ctgttacaag cttcctgtca tattttgaaa gagtgttagc attgggagag aagaaaattt 1920
   ggggatgtga aggaaaaatt ctcttggtag aacagaaata atattttcaa aatcacttaa 1980
   gaaagataaa ataataaaga tgccaggact ttgaaattac atttctaaat atttggtatg 2040
   cttgagaaca gtgagttaga ttgctagaat cttgccttgc tacatctcaa tattaaagta 2100
   tgagaaaaaa atcaaagaaa tccctttcac taatattgat gttcttacac ctgatataac 2160
   ttaaatattt ttcctagtag atgttgactt gaggcttttc tatcaatgca gccacataac 2220
   tgtgttgtta cacaggctat agaaactaac agattattat aattattatt gattattaat 2280
   ttatagtgag ccctcaaaaa taccaatatc ttagtttgtt cttggagtct acaaaataaa 2340
   tttaatttgt tttctatatg acagtgttta aatatttcaa gagagtatgt gccatctaag 2400
   tcttttcttc tttaggcaga acatacatcc tcctaattct aataacactt gaattgataa 2460
   ccttctattt attattagat ttttaaagca gcactcccag ccccttcaag ttaaaacaat 2520
   tctccaggta aattctggtc aatacagtat aaaaagtaaa gttctttttc tggactcaaa 2580
   attctataaa ctctttatgg acagcttgat tttgtaatga gcttaataaa cttagaaaat 2640
   ccatttaaat cctatactta ataaataaaa caaagcaaaa catgaattgc tgttcagcta 2700
   gcttttcctc acctgtacat ttgtaatgat atttttctca tccacatggg ggccttcata 2760
   tttattctta ctatcatttt taattttctt tttctgtgtt gtatactaaa acacatctga 2820
   acaatgattc cttgccctat ttagtgaatt ggaagcaaat acagttttca tcaacttgta 2880
   cagctgaatt ccatgaacaa tttcagggat gcagtttgca ggattagttg aagaggagaa 2940
   aacctggagg caaaagtaaa ctctttaaaa a                                2971
<210> 117
   <211> 1745
   <212> DNA
   <213> Homo sapiens
<220>
   <223> nbla20365
<400> 117
   ctttccctca ttcaccacct tccagggttt catagaaaat aacttgttac aaaatcagtt 60
   caattctaat gtggacatag tggcatgttc ataattagac ccatataggg gacactgagc 120
   tttaaatcgt tgattctaaa ctctatacat taaaaaaatt cagcccaggc ccctcaaagc 180
   ctgagaaaat ttaatttgct cttaatttaa tgttccaaaa ctcactcttg gaaaaatgcc 240
   tgttggaaaa ctacaggtgg gtcacatgtg ggggctgtct ccgtgacact caggattcca 300
   gtcagaacct aatcctcata tctattgcct acaaaaatag accaagaatg ttgctgctct 360
   tttataatcc tttaaatatt taacattcaa gttttctttg tcttaaattc agcctcttcc 420
   taaaagcaaa aaagaaaaaa aaaacctcac agaattgtgt tgagatccac cgctcacacg 480
   ccgtacacca cccagtggct tcattctggc ttagccgcag aggcaagaaa gggaccccac 540
   ttgctcccat gcccacctca agaaaaaaca taaaacaatt ttttttaaaa aagaaaagaa 600
   atctacctca gttgacagga ttccaccttt agggtttctt caacttttaa gtcttacctg 660
   ttgagtgtaa cttttgtagc atcttgcttt tccaagcaag ctagtgaggc atgacagagc 720
   agaagtctgt aaatgtccct gtgatggacc tctttctagc atgttgcagt tttattttta 780
   ataaattggt aagtgaaatg aacgtaaagg taattgtgta cgttttagac atgacaatga 840
   aaatttaaaa tgtagcttcc atacttgtgc ataattccaa agtattttat tttttatcaa 900
   tcagtgttaa atagcttttt gtacaggctt caatccattt ttcgaagtgt gctgtttttt 960
   aatgaaagta actataatct tttcacatcc catggaactg ccgtttacac attgcaactt 1020
   tttaaactta accatatttt tcaaaattaa cgtttttgga gggagaaaaa tccccgcttg 1080
   ctaaatgata ctaaaccgtt gtttgggctc ttataattag gtcctgagat tttataaaaa 1140
   tttagtctgt agctttttag gttcttcact agagttggtt gtacataaaa ataataaaga 1200
   atataaagta tcccaaaatt cttttaaagt ctggattttt ccgctaatat gtactttaga 1260
   gaatattttg ttcatgcata cttccacgtt aaattgaaaa tgtcttcagc ttctcttggt 1320
   aaatgtgaac catttgtttt ttattgtgct tgggggagag ggtattttaa tataattttt 1380
   gcctaaatca agaagtcccc tctgaatgtt aatttttaaa tgtcaaaata tgatgaacga 1440
   tatatcttga aagtgagatt gcaatatgct taaacttaag tggtatttca aaaacgagaa 1500
   aattctggaa tttgtcattt gaagctccat aagagaaatt gataggactt cgtttttgat 1560
   cagtctgaat agataccaat gtcattgtgt gggaattttt tttaacttgt ttatgtatta 1620
   ttttgatcca tttttctgtg gcatttggtg caataaaact tttgaattta tcttgaacat 1680
   tttcctggtg ctgcatgcga tttgttatag ttaataaaat gtagaggtct catttctaat 1740
   aaaaa                                                             1745
<210> 118
   <211> 929
   <212> DNA
   <213> Home sapiens
<220>
   <223> nbla20378
<400> 118
   gtaacaaatt gcaagaaaaa caacttaatc ttccagtgac taagtaagaa aaactgttgt 60
   cactattaaa catgtaggaa attgataatt attacaaaca aagcaatact ctaccctaaa 120
   tctagacaaa tcactggaca gatgataaga ttttcagctt tctcctttaa agagctgtgc 180
   caatgtacag atttttttgt aaacatgcaa agggaaggtt acaaactcct taaactttaa 240
   aaaaccataa atcctttctt tgctacttat attctatgcc aattataata ttccaagact 300
   tacctttctt cagaatgctt acatatggaa aggtttattt ataaatattt gataggtaaa 360
   tattccatat gtattttcta gcccgtcttt ctctgtccct ccctcaaata acttcattac 420
   cctctccttt ttaaacgaaa tatcttgata ataagaaaac aaaatcattt ttttgtgaaa 480
   taatacatat ggacaaaaaa tacaagttgt attttacttc tggttcatta aaatattgtg 540
   tttagttgga ttttttcctc ctttattttc agaaacataa aagaaattgt tttatttcct 600
   aaaggataaa attggatata gcctctttag tagacactat cacagttctg ttgtttgctg 660
   tgttcatttg cttaatgaat tgcgtgagaa cagtcactgt aatgaaatat gtgtgctggg 720
   ggtgggggga agggcatggg aaatgtttta tgaaaaaaag ttataagcct aatactatga 780
   agtaacatct aatgcagttc tttttaagtg caatatattt atttctgcta gaaatatatt 840
   atcaacctta tgtaatattt gaagcattac atattatttg taaacagctt aaaattatat 900
   attaccccaa ttgtacataa gtacaaaaa                                  929
<210> 119
   <211> 1972
   <212> DNA
   <213> Homo sapiens
<220>
   <223> nbla20511
<400> 119
   atgtacacgt ctctcaaact atgaagagaa gatttgggag gagtatgaga aaatcctcaa 60
   taccaaacta gcagagcaat acgagtcgtt tgtgaaattc acacatgagc agattatgcg 120
   accatgtggg acaagcccaa caaactatgt gtcttgaagc tttttgttgc agatctcggt 180
   accaggtttg acctcaaggc atggttgcta tacatttttt gcaactgttt gatatcacat 240
   ttcagctcca actttgcatc ctgagaacat tccaacgttt ctgcaggtcc attttatacg 300
   acttgaaaga ccttaaaact ttctggttgc cacaggtata tctttctttt ctgttcatcc 360
   agtaaatagt cataccctac tgtgacagat ttttccaaac aaaaatacct ggagcagcag 420
   tgtagcaaaa tatgccttca gtggcactca acaaatggag tttccccaag cacagttctg 480
   taagaagtgt gtgtgagagt gtgtatgtgt ctgtacatgt actttagatt atggtttgta 540
   ttgtgcaaat ttttttgatc ttggggattc tggctgtgga tttgatgcag aaaattatgg 600
   ttaaaaacta tggtctacag aagatactta atgctttgtg actatataaa ttgtaacagt 660
   ggattgtttt atgtgtaggt attattgtta aatatgggga ctgttcacca ggcacaaaat 720
   aggaatcata aattaggatg caggctgggt atggtggctc atgcctgtaa tcccagcact 780
   ttgggaggag gccgagctgg gcggatcgct tgaggagagt tcgtgatcag cctggccaac 840
   gtggagaaac cctgtcccta ctaaaaatac aaaaattagg tggacatggt ggcgagcacc 900
   tgtaatccca gcttctcggg aggctgaggc aggagaatca cttgaaccag ggaggcagag 960
   gttgcagtga gcccagattg tgccactgca ctctagcctc ggtgacagag taagattcca 1020
   tctcaaagaa aaaaaaaaaa aaagtgaaga tggccattgg ctgtggttat gacaatacag 1080
   tgaaagtctg ttgtcttaga tatacaaata catagtgaga aattagaaca aactggagac 1140
   tggcctttga cacatggact ctgcctagct gtgttagaaa aatatttaac tccaagcctt 1200
   aaaattccca aatggagttg gtgcttacct cattcacaca atccaagagt tcactgggtc 1260
   ctgaacctct aaagggaaaa ggtctcccct ggagcaggag catcagagtt tgctcggggg 1320
   cataaggtag gtgagtgctg ggccgaggca ggctcccctg gcactgctag ttgcaggagc 1380
   actttacctt tgtatcagtt actaaaaaca aaatttgaat cctttggtca ggttccccca 1440
   aattattttg aggtagccat gtttaagtgc ttgagctttt gtgttggcaa acccctgccc 1500
   aaggttgcta atagggtatt ctgccccttg tttccacagc tgaggcacag aaagtagcct 1560
   cttttgtgag gagttgggag ttaagtatac atttattttt ttaccatgat ttgttcagga 1620
   ccacatttta caagatacct tgtttccttt attattgttt ctggaaagtc ctattcatat 1680
   tattttattt gaatatagaa tatagttttt ttaaatgagg gcttattttg aaaaattctg 1740
   agcttaattc aaatttatgc caataccttc ccaaataagg taatagtcaa agacagatgt 1800
   tctgatcaaa tggcttagag atagtcctgg aatattcata ttcaaagatt ccttattaat 1860
   gaatgtcttt aacttaaatc tacccaataa ttgcaacatg gttctttgta cattttcatt 1920
   atatggtgtt aacaagcttc actgcaaaca aataaattac ttaagttaaa aa         1972
<210> 120
   <211> 1806
   <212> DNA
   <213> Homo sapiens
<220>
   <223> nbla21039
<400> 120
   ttggagcggt ttctgaattt gttgtttgtt taattttttt atatcctcta ttccttaacc 60
   atgctgttaa ttattgccat tttatcattt gctttactgt gtgttaagtt tcaatggggg 120
   taaatgttac tcttcttttt taaatatttt tggctatatt ttttcatgta ttctcgcaga 180
   taagcttcag ttatgttttg gtcacattcc aaaagaaatt ctattgccat tttgtttgga 240
   attgctttaa caatatagac taatttgggg agaattacca actttgtaat attaaatttc 300
   ttattcagga acttgtcatg tgttaaggtt actgaagaca atctccaaat gttctttagt 360
   aattttgtat gttttgcata tttttattaa ttgtattgtt agatattaca tttctttgtt 420
   gcatttgaat gggatcattt ttccatcatt ttttattagt ggctactcct tctggggtca 480
   agctatttat ttttgtgttt gataactttt ttgaactctt tcattaatac tccctagttg 540
   attctcttct aagtagccaa tcattcttaa gtataattaa tggtgatttt tctctcttcc 600
   tttataatat ttatacctca tgtgttaaat aaatgatggt aataactttg cccttattcc 660
   tgatttgtta ggtatgccat taaattttac cagcttttaa atgttagctc ttagactgaa 720
   atacaggtct ttatcatgtt aaagatgtgg tttgatggtt caaatatact aagcatttta 780
   ttagaagttg ttcaattata ttaagcatac tctaggcaac tagaaatgat catgtgattt 840
   tacttctctg gcctattatc acaataaatt atgttagtag tttctcaata ctggattatt 900
   tctgcatgag atgtatcctg ttaaataatg gtggtttttc cccttttttt cttgtaacaa 960
   tgttgaactc aatttcttac attttatttg ggattgcatt tatatttatg aataagattg 1020
   ggttgtagtt ttacattttt gaattctacc tttatcagct taaaaaccta gattggtaca 1080
   tagcatatga attgcctatt ctttggggtc tggagaaact cacccataac attgtttagg 1140
   cctcaatctc tttaagggag gatgagtaga agacaattct ctgaaaactt aaaaaacctt 1200
   tttctatggt tttactgtca cttcttgagt atcaatttaa aaaatcatat ttttaaagaa 1260
   aaacatgcat tttcagagaa ttttaaaatt tgttgtctat atataattat aattaaaaat 1320
   atttttctct gtatctctgg ttattgcttc tttctcattt ctggtccttt tagttacttt 1380
   tttcctttct tttattagac ttgccagtct ttttaaggaa ccagcacttg agtatttcat 1440
   cagttctatt ttttctattt gttataatat taatataact tttcatcttt aattccttcc 1500
   ttatttttaa tgtttatttt gttgctttat gaatgttttg tactaattga tggcttagtt 1560
   catttatttt cattttttaa tttaataata ggacacgtaa gactataact ttgcctttgg 1620
   gcacagcttt gactgcatct caaaagtttt ggtatgtagt ctcttaattg ttgctattta 1680
   aaaataatgg attatattac tgtttttatt ttatttttga tgtattattt aggatagtat 1740
   tgtgaggttt ttgttatttg ttaatcctac tttcttttta ataaagaata aacttattaa 1800
   taaaaa                                                            1806
<210> 121
   <211> 2614
   <212> DNA
   <213> Homo sapiens
<220>
   <223> nbla21107
<400> 121
   gggtccagat ttttcccctt tataagctgt tttcacaggc actgctgttt gtttttcctg 60
   aaccagtgta ttcatctttc ctgtgtccag aaagttactt tatctgtgat cagttctgga 120
   gtaaaggatg tcatgtcata gtctgtgctc ttgcccacgt tggaggagct cctagcataa 180
   tttaagctta atgtcaaaat gcttggggtt gcaatctagg ttctgccatt tgctggttgt 240
   gctgctttgg gtagagcagt tgtataattc aatgagatat tgaatggcta gcgctcgaca 300
   tatagtaata actcagtcaa aataattaat attttttatt atctggaagg gtgcagtggc 360
   tcattcctat aatcccagta ctttggagct gaggcaggtg gatcacttga ggtcagaagt 420
   tcaagaccag cctggccaac acggtgaaac cccatctctg ctaataatac aaaaattagc 480
   cggatgtggt ggcgtgtacc tgtagtccta gccactcagg aggctgaggc aagagaattg 540
   cttgaacctg ggaggcggag gttgcagtga gccaagactc tctagactgc actctagact 600
   gggcaacaga gtgagattca atctcaaaat aaattaataa ataaataata aaataaaata 660
   aaataattag tattttctag cccgccactt acccagttag gtatcccagg actttgttag 720
   tagcaagtag catacaagaa aacaacagca gcaacaacag agttctgtga gcacacgagt 780
   taggaaaaca tcaggatgaa aagctcacat agactccttt atggcaggac ttagtctcta 840
   aaatgttaca taatgtgttt tgtagagaag agtggaataa acgctaatta ccaaactatt 900
   tggccttaga accccttttg ttttagggtg gcatggtaga gagagtgatg ttccttagaa 960
   tcccattagg aaagaaattc cagggtggtc cacttccctt aggaattcta aggtattctg 1020
   aggagcatca cggtctctat cctgccatcc ttgaaaacag tatttgaggc caggcacagt 1080
   ggctcatacc tgcagtccca ggactttggg aggccgaggt agacagatta cttgaggtca 1140
   ggagttcgag accagcctgg ccaatatggt gaaaccttgt ttctactaaa aatacaaaaa 1200
   ttctctgggt gcggtggcac atgtctgtaa tcccagctac ttggggggct tagccaggag 1260
   agttgcttga acttgggagg tggaggttgc agtgagccaa aatcatgcca ctgcacttca 1320
   gcctgggcag cagagcaaga ctctgtcaat caatgtatca ataaggtctt gctaaagatg 1380
   ataaagcaaa ttagatgtgg aacaacgtta gaagtgcagg ttcctctctg cttcctcctg 1440
   cacgtgcact tctcaaagtc tgatctttga tacacctctg tcagcatcac ctggggaggg 1500
   gatgggtagg aacacagatc acagacacag ggcatcagaa tctcctgtct cagagcccag 1560
   gaatctgcat ggtggcaagt cttctgggta attttctagt aagctaaatt ccggaaacca 1620
   ctggactgga ccacccatct ctgtagctat attgtgtggg cagaactgag gttgctgctc 1680
   cttccaaaaa ctctggtgac tttggaaaaa tggttgatga tggctcctca ccacctctct 1740
   gcctgcccca tgaccctgga ggaggtgtgt atcttgggag aatgctggag gccttcctgg 1800
   gctttcacag gccagcccgt catgcagagt ctctccagag accgctccct gccctccatg 1860
   gtcactgtgg gagctatgtg tccctacgat ccctggtaat gctcctccag ggaaacctgt 1920
   gtgtgcggtg caggggagat tagttcgaaa tggagagaca cgtacttggg gccttgccaa 1980
   gtcgctctgg agagagcatg gcgatgcttc ggtttccatg gaaaccaggt gactgtaagc 2040
   tcacctttgg cccttgaaac agcctccagc ttctgggaac aactgcaagg ctgctgctta 2100
   ctatgagagg ggagagcagc cacagagaag agaaaaccaa ctgctgattg gaaaacaggc 2160
   tcagttgtct gttttgaact gcaagaaaag ttagaagagt gctccaatcc aaagatacag 2220
   aaggtcagat gtggggcagg caactagccc actgtcccga tctgtattaa gagacaccac 2280
   catcaaggtg gctcccttct ctaggttttc tactcaaaaa gccttttttg gctttttgag 2340
   tcgaaattta tgaacatcac aggcttagac agtttttttg actgttcctt tattccctgc 2400
   taaaatcgat attccatgat atccagacat tgccatgctg gcttcaattc ccactttgtg 2460
   tgtgttcttc ctctttctca tatgtgagca gctgtggata gcaccgcgcc cccagttttg 2520
   taaagtaagc tttccaaagt ggaaggatca cttgcagggc aggagtttaa gaccaggctg 2580
   ggcatcctag ggagaatcct gtctcttcca aaaa 2614
<210> 122
   <211> 1779
   <212> DNA
   <213> Homo sapiens
<220>
   <223> nbla21367
<400> 122
   aaaaaaaagt tttcaggttc ctgtgtcttc ccgttgagtt gctcgctcca cggcatggag 60
   gactaggaat caggagtcag tggccgtatg ctggaggctg gagccgcggg agcgcggctc 120
   gcctcgctgc ggttgttggc agcgacggag ataggagtgc ggctggagcg atgtgccagg 180
   tggtgccagt gccagctaat cacctccctg ttggcagcac cgtgaacact gtgcacctgt 240
   cttcagatgg cacttaaagc agagaagcct gctgtgtggc tgtgggagtc atcagagagg 300
   tggcagtgga ggtttgttat cgacccaatc attacatcgc tacccaaggc tcatctggaa 360
   cttctggact caagtgatct gcctgccttg gcctcccaaa gtgctgagat tatagggatc 420
   atggcccaag taacaatgtc cgccctggcc gttgaagatg aggagtcctc agcaggatgg 480
   tggtgacatt cctcatgtca gctcttgagt ccatgatggg gtttcacaat gttggtgagg 540
   cttgtcttca actgctgagc tcaagtgatc tgtctgcctt ggactcccaa attgctggga 600
   ttgcaggatt gttttagagt gtggatagtg aagcaagaat gtacgctcat ttactcaagg 660
   tgttgaagaa gaagaaaagg ttgcagagat gcattaaata aagtctacaa cccagggaaa 720
   gctttacaaa aacccacaga ggtaactgta ccatatgaga agatgctaca agaccagtca 780
   gctttgatag tacaggggct tccagaaggt gttgccttta aacaccctga gaattactat 840
   cttgcaaccc tgaaatggat tttggagaac acagccggga tttcattcat tattaagaga 900
   gatggggttt cctcatattg tccaggctgg tgtcacactc cttggctcaa gcaatccacc 960
   tgcctaggct tcccaaagtg cttggattat aggcatttcc ctggaagtgg cagctgtgac 1020
   agtaaaggaa gaatcagaag atcctgatta tgatttatat cacattcaag gagccagctg 1080
   aggaggtgtc atgcaacttg ctgccccagc cctccccttc taaggaactc ccacacctgc 1140
   cctgtcttcc tgctacagtc tctgagaaaa gccctttcat ctgtcaagaa ctacaaaagt 1200
   cattctttaa gcagtgctaa ataaacaatg aaacagacac aaagttaaag ttacctgatc 1260
   caaaaaggag tgaagcctag accccagccc actgactcag tctctaagtc ccctcactca 1320
   aatcttcaat caacagtgga gatggctgtg agctcttctg attccagatg acaatactcc 1380
   tgcctttaat tccttgatat gtttaattat gtaagtaaat ttaatataaa aatcattgca 1440
   ttagagttcg tggtttttat acaagattca gtgtgagatc aatgtcatac ttccaatttg 1500
   tcacacttat agagaactga gaagagtcac attatttaaa atcttagcaa atgtgcataa 1560
   ttcctttgga taattttaaa gtgataggat tggatcacat atgatgcaat ttcctgggtc 1620
   tcttttgttt ttagatgttt ttatctctcg tattgtggat ctcatattta tgtgaataat 1680
   tatcagaaga ttttatttct attatgcata tttagtataa aatgatcata cagtgaagag 1740
   tgtgtaaaat caaaataaaa tgccattcat caccaaaaa                        1779
<210> 123
   <211> 2942
   <212> DNA
   <213> Homo sapiens
<220>
   <223> nbla21790
<400> 123
   gatattttgt tacaagatta tcctagtttt tagagagctc aataatacta acacctcatg 60
   tatttcaggc tgcttcaggt ctaattcaac cataaattta aatggttgaa attctgatta 120
   attgacaagt taagttggga ggtaaggaac aactaggata ccatctacta tggtgtgtag 180
   gcaagtgctc cagattgtga tttctttgag tatctggggt cgtagggaat aatcactgtg 240
   cctgggtaag tcaggaagag atcataaatg acaaagaagt cacccttgta aggattgagt 300
   atgtattagc agagatggat tcatctgaaa atggtactag atggtgggtg cctgcatcag 360
   tattatttca gtatattttc acatcaagat agttgagtaa gaaatgtcat gtgattaaat 420
   ccaagaggta tttattgacc tgtaatttgc cagacactgc taatcccttg tgctacaaaa 480
   atgagtaaga accacttatt ggtgcacaca gtctgtggaa gaaacagaac tgcaaacctg 540
   tcattctaaa ataataggta ctaagggact gaatcagtag aagttcttct tgtgtacacc 600
   agagaaaagt gacaattgag ttgatgcttg aaaagtgaca attgagttga tgcttgaaaa 660
   gtgacataat ggaaaagtga caattttgaa gaatcactaa attggggagt aaatggaaag 720
   agaaggatta ataatagctt tagtgaaaaa agaggataaa gtgaagttga tttgtttatg 780
   attttagata gggataggag ccaatagaga aacaaagatt ggaaatccag agaagagagt 840
   aattggtaat gcagtgttca acaggagtca ggagggagtg gaattaggag tactggtttg 900
   aagaagagag gaattatctt ctgagactgg agagaagaga gtaaatattt cattctcaaa 960
   aactccttgg gataattggg ttttttcttg tgcccacttt ttaagagtaa cacttgaagt 1020
   aaatcttttt gtttagtaag gcactaaggg aaaagtcaaa ttatgaacct tcagaggaaa 1080
   tagaatgatg acctaatctt gcatattctt aggggtagag aagatgaagt ccatgtcaca 1140
   gcttgcagtt ttgtcaagac ggtggaagcc ttcagagatg aagttggatc ccttccagga 1200
   ggttgtattg gaaagcagta gtgtggacga attgcgagag aagcttagtg aaatcagtgg 1260
   gattcctttg gatgatattg aatttgctaa gggtagagga acatttccct gtgatatttc 1320
   tgtccttgat attcatcagg atttagactg gaatcctaaa gtttctaccc tgaatgtctg 1380
   gcctctttat atctgtgatg atggtgcggt catattttat agggataaaa cagaagaatt 1440
   aatggaattg acagatgagc aaagaaatga actgatgaaa aaagaaagca gtcgactcca 1500
   gaagactgga catcgtgtaa catactcacc tcgtaaagag aaagcactaa aaatatatct 1560
   ggatggagca ccaaataaag atctgactca agactgactc tgatagtgta gcattttccc 1620
   tgggggagtt ttggttttaa ttagatggtt cactaccact gggtagtgcc attttggccg 1680
   gacatggttg gggtaaccca gtgacaccag cactgattgg actgccctac accaatcaga 1740
   agctcagtgc ccaatgggcc actgttttga ctcggaatca tgttgtgcac tatagtcaaa 1800
   tgtactgtaa agtgaaaagg gatgtgcaaa aaaataaaaa aaaacaacaa aaaaagctaa 1860
   ccttctatta gaaaagggga caggggaatg agtaaacttc ttttattgcg gacaaatgtg 1920
   cacatagccg ctagtaaaac tagcctcaaa caggatgctc atagcttaat aataaaagct 1980
   gtgcaaaggc catgaatgaa tgaattttct gtttatttca ctgatgcaca cattacctca 2040
   ttgacaattc agaagtaaat ccaacgtgtg ttgactcttg gaaagcagca aaaacaggag 2100
   ctgaagaaaa gaaattcttg gaaccagccg taacccagta aggaattgtg aagttgtgtt 2160
   tttattttgt ttcatttttt gcagagtatt aagaacatta ttctggaaca tcagaacgtt 2220
   tcccttagac cgatcccagc aggtggcagc tcagattgct gcagtgttgt aattataact 2280
   gattgtactt aagttatgga tgtagagaat atgtttcatt catttattca gcatgtaaat 2340
   aaaattgatc ctgttgagtt atcataattg cagttcaact atctgccatg attattcttt 2400
   tcacgtatca ttcattctgt acatttgtgt acattgagaa gtatagcaat ctatgtaaat 2460
   gtaatcctca gtgaggttcc tcagtgctag gtcccatagg attgtcgttg cccttgttaa 2520
   tgaggtttct ctgttcagcg gcttcaattt ttttctcttt gtacatctag ttttgaagat 2580
   ttacttcaag tttgaatctt ctagaatgct tgtaagtcca gttttaattt ttagagtcaa 2640
   tttgtagtta catgtagttt aacttttggg aaacgtctta acattgttct gaataaactt 2700
   gctaatgagg tcaggtcatg gtacagactg atgcagtcaa catgatttca ttgcagagtt 2760
   tattagtatc agcaagtttt tgctttgcta aataaaagta cccaatgaac acaattctac 2820
   ataaattttg acataccatc taatttataa aaatcaataa aaaaggtttt ggtaaaactt 2880
   tttcatgcca gatgctgttt acaacaatga acatgccaat aaaacatttg ttcattcaaa 2940
   aa 2942
<210> 124
   <211> 1679
   <212> DNA
   <213> Homo sapiens
<220>
   <223> nbla22253
<400> 124
   ccgtttgatg ttcaggagac tggcgaaggc tcagcaggag cttcaggagc cccagagaag 60
   gtccctgaaa atgatggcta catggagccc tatgaggctc aaaagatgat ggccgagatc 120
   cggggctcca aggagacagc aactcagccc ttgcctctgt atgacacacc ctatgagcca 180
   gaggaggatg gggccacccc ggaaggtgag ggggccccct ggccccggga gtcccgcctg 240
   ttagaggatg atgagaggcc ccctgaggag tatgaccagc cctgggagtg gaagaaggag 300
   cggatttcca aagcctttgc agttgacatt aaggtcatca aagacctacc ttggcctcca 360
   cctgtgggac agctggacag cagcccctcc ctgcctgatg gggacaggga catctccggt 420
   ccagcctcgc ccctccctga gcccagcctg gaggacagca gcgcccagtt tgaaggaccg 480
   gagaagagct gcctgtcacc tggccgggag gagaaggggc ggctacctcc ccgactctct 540
   gcagggaacc ccaagtcagc caaaccccta agcatggagc ccagcagccc cctgggggag 600
   tggacagatc cagcactgcc tctggaaaac caggtctggt atcacggggc catcagccga 660
   accgacgccg agaacctgct ccggctgtgc aaagaggcca gctacctggt gcgcaacagt 720
   gagaccagca agaatgactt ctccctctcc ctcaagagca gccagggatt catgcacatg 780
   aagctgtccc gaaccaagga acacaaatat gtgctgggcc agaacagccc gcccttcagc 840
   agcgtccctg aaattgtgca ccactatgcc agccgcaagc tacccattaa gggagccgaa 900
   cacatgtccc tgctctaccc tgtggccatc cggactcttt agatgtgaag ccagggcact 960
   gtgatagacc tgtacccagc cctgtgccca tcacctggct gagggctgtg gctcttgcca 1020
   gggacgtgat ctttcaaacc tttcttctcc tgggatccag tagaagctgg agattcctta 1080
   atttattcta aagggaaagg gctcctgggg ccttggagta aggggttgtc tggagctggg 1140
   gaaagaggaa tccctggaga gaaaggatag cccctggagg aagggggttc cagagctact 1200
   gggatggtag ggagtttcag actggcagct ccggctcctt tccgacctta gggcagaggt 1260
   ggtgacctcc accaccacca ccctctcccc actgggtccg tgcgaggtag tgcagaattc 1320
   ggccccttgg ggcgccttac cacctctctg cctccgtccc cgacttccac cccagaccgt 1380
   cggagggctc cgcccagagt ctggtaagag gtttggggaa gacaggcccc tgggaagcag 1440
   ccggctttgg ggggtgggga gagaagggga ggggctcggg cagagggaac tgtgcagtcc 1500
   ccaggccgcc ccggctccgg gccagaggca ataaataaac ccgatcctgc cgggcacagc 1560
   cgcgcccgcg cctccggcgc cgtccccggg ctgacggggg agggagcgga gaagcgagcg 1620
   cagattctgc gtataaatca gctctggagc agacacagcc cggctgtgaa aagcaaaaa  1679
<210> 125
   <211> 3886
   <212> DNA
   <213> Homo sapiens
<220>
   <223> nbla22355
<400> 125
   acaggaagga accagctgat tccatatgct tctgtctgtc ttctcgtcgc agggtagatt 60
   ctctgtccct ttctctgctg ctcttcctcc tggagccgtg gaggcagctg ggcgtgggtg 120
   ccttccccgt ggaaagccgc tggcagggag tctacagccc cttccgggac tttgtgtgtg 180
   ctggctgccc cagggacctg caggaggccc tgctgggctt cgacgtgcag agctccaggg 240
   agctgcgtag gtctcaggat tacctgtcct gcgagaggac ccaccctgag gacagtgtgg 300
   gcagtatgga agacatcctg gaggagctgc tgcagcaccg ggagcccaag gccctgcagc 360
   tgtacctcag gaaggctctg agcaactcac tgcaccccct gggaaagctg ctccggacac 420
   tgatgctgac cttccaggct acctacgcag gtgtcggggc caacaagcac ctgcaggagc 480
   tggcccagga ggaggtgaag cagcatgccc aggaactctg ggctgcctac aggggtctgc 540
   tgcgagttgc cttagagcgc aagggccagg ccctggagga ggatgaagac acagagacaa 600
   ggtgactggc gcaggtctcc ttggggcctg ccgtgtccag ggaggcctca tgcgtctgct 660
   cctaggacct cccttgggga aagaggtgct tctggggaag tgctgggcat tcactctatt 720
   gaccaaacat tgtgcattga tcgtttgtgg attagaatga cccatgacct ctgttctgtg 780
   aggaaccagg gagggggcac tgctacaatg cattgaatgc atctttgttc taaatgtatg 840
   atcccaatct catctttcgc atgcagaagg tgagtagctc cccgaggcac cctcctctcc 900
   ctgcacacag atggggaaac cgagggctgg tagggatgag cctgaggtta tacaggagtt 960
   aggtgggcat gaaatttgtt tcccccagtc cctggagcaa accttacaat ttgcctttag 1020
   attctagacc tgaaagtgtt cctgatcaga gaggccttcc tgtcactgcc ttgcaggagg 1080
   caagggaaat ggggttagac attagggagg attccccgcc cggagtccta gcacagcaaa 1140
   ccaggaggtg gaactgaatc agcctggaat ggctgctgag agctcagctg caagttgctg 1200
   gtccatctgg ggccctggtt ttgctttcag tcaaatgggg atccaactcc tgccccacct 1260
   gccatcttgg ttgtcaaagt caaaggaggg aatgaagtta tgaattgaat tgggcaaatg 1320
   atgactgaga acaggcttgg aaaaggtttt ctggggagga ggaggctgga ggccaggaca 1380
   ctgtttgttg tggaactagg agctctttga gacgagactc caagtagtaa tcccagaccc 1440
   caccttgctc atcccaacct gttccggtct ccccatcagg gacctccagg tgcatggatt 1500
   ggtgctgccc ctcatgctgc ccagcttcta ctcagagctc ttcacgctct acctgctgct 1560
   tcatgagcgg gaggacagct tctacagcca gggcattgcc aacttgagcc tctttcctga 1620
   tacccaactg ctcgagttcc tggatgtgca gaagcacttg tggcccctca aggacctcac 1680
   gctgacgagc aatcagaggt actccctggt cagggacaag tgtttcctgt cagccaccga 1740
   gtgcctgcag aagatcatga ccacggtgga cccacgggag aagctggagg tgctggagag 1800
   gacatacggg gaaattgagg gcaccgtgtc gagggtattg ggccgggagt acaagctgcc 1860
   catggacgac ctgctgccac ttctcatcta cgtggtgtcg cgcgcccgaa ttcagcacct 1920
   gggagccgag atccacctga tccgtgacat gatggacccc aaccacacag gaggcctgta 1980
   tgacttcctg ctcacagccc tggagtcctg ttacgagcac atccagaaag aagacatgag 2040
   gctgcaccgc ttacctggcc actggcactc cagggagctc tggtagcctg gcctttcctg 2100
   gacagactga agagctgagc agggcactgc cagcctgtcc ctcattaccc aaggcaaggg 2160
   gcaggacagg ccctcagaag cagctcttgg aggagatgag cattttgttt tgcacaggaa 2220
   gatgctgctg ctgccctgac tgggatgagg gtgaggggtg acgggtgtgg ccctggatgt 2280
   ggtggttttc ccttggccac tagcccatct tcaatgaccc cttaatctgc agcagctcac 2340
   aggctggggg tgaggagtcc ctggcttctc ttagcctgag cctttctccc aagttccaga 2400
   gcctctccgg gcctcagtgc tgccatctgt acaatggtgg agtgagtacg ctgtaaagga 2460
   ccttccattc attttgctga attccagagt ccttttggaa aactgacttt agtctgctgg 2520
   gctgtattga cctctggcag gctcgaagcc tcactgggta tgcagtcaac aggatgggcc 2580
   tggagatccg tgaactgcag gccacgtacc catgacgtaa acggcggcac tggagcaagc 2640
   tggggcgggg ggtgggtaaa ccctcactgc cagcaggccc caagtggctt gtaaatcatt 2700
   ctcctgtgac gtctgtgggc ctgcgtgggg acaacagggg cacatgacat ctacctgggc 2760
   cctgaccaat aaaccctcag acccaggacc caggaccctg ctgtagttgg ggagcaggag 2820
   tacctttggg aggggaggac tttatttaaa cagtggttct agtgtgggac caagagaggc 2880
   aggagctggg tcttggggca gctttattcc tgttgggcct cagtttctct tccccacaca 2940
   gtttatcttc cgtcacattg tgccgggtga cgtgcacggt ctccctctgc cctagcagga 3000
   gatgcatgat gacaggcagt gtgatgtgtt ctgaaagtgt ccagggcaaa gcgtagggag 3060
   agggtggatt tgtgcagggt gcagctctgg agaagaagct ggatcactct tggtcccatt 3120
   ccctaggccc tgagcaagtc aggctcctgg ctctgggtgt ggctccccca aacgaagtac 3180
   tgacttcagc ctgtgagggg agggttgagg gaggctctgg aaagcccagc cacacctgag 3240
   tccctggcag tagccttggg gcagagggca cccgcagagt cccagagatg atgtgggcag 3300
   tgggcagaga gagccttggc gcctctgttt gccaccactt ccccaggaag gagggacagc 3360
   atttctctgg ctggttccac taaatgtgcc agcccaaatg cagggcatgg gctctggttc 3420
   tgccgggagc ctgtgacacc cccaggaagg gggtggaact gaggaagagc gaggatatgc 3480
   aggcactcat gcttaccggg actggggcag ctcactagga ttctatcctt tccaatcggc 3540
   atcagccagc tcttgtcccc tgataagtga ggacagcctg accctggcct caaatgcagc 3600
   catccctgag ttcatgcgat gctgacggga ccccagcaca cttccctgcc tcctttgaga 3660
   tctgcgagcc cttgctgcag ttcagattca acaaggccct ctgcccaccc tctcactagg 3720
   cctcacccaa caccagtgga actggagcct ctggctgggc acagtggctc actttgggag 3780
   gctgaggcag gaaggctgct ggaaactgag agttcaagac cagcctgggc aacatagtga 3840
   gaccctgtct ctacaaatac aaaataaaat aattagctgg gaaaaa                3886
<210> 126
   <211> 2024
   <212> DNA
   <213> Homo sapiens
<220>
   <223> nbla22832
<400> 126
   agcgcgggca cacggcggcc agagcgccga ggcggtacct tcagcctgca atgagaggaa 60
   cccgggagag cccccgggag ccagcgaaga gcttggctgc tgcgtccagg gctgctgctg 120
   ccgccgcggc tgcttgaaac tcctcaaagt tgagagccgg ctagagggtg ccgcccgccg 180
   ggagccggag ggaaaggaag tcggaaggtg caagagtgac agacacggac agacggacgc 240
   gcagaccttc ggaaggcact gcgtaggcag cctccccgga gcccacgagg ctccccagca 300
   ccgttcactg gtgggaggct gagccggtgg aaaagacacc gggaagagac tcagaggcga 360
   ccataatgtc gttacgtgta cacactctgc ccaccctgct tggagccgtc gtcagaccgg 420
   gctgcaggga gctgctgtgt ttgctgatga tcacagtgac tgtgggccct ggtgcctctg 480
   gggtgtgccc caccgcttgc atctgtgcca ctgacatcgt cagctgcacc aacaaaaacc 540
   tgtccaaggt gcctgggaac cttttcagac tgattaagag actggacctg agttataaca 600
   gaattgggct tctggattct gagtggattc cagtatcgtt tgcaaagctg aacaccctaa 660
   ttcttcgtca taacaacatc accagcattt ccacgggcag tttttccaca actccaaatt 720
   tgaagtgtct tgacttatcg tccaataagc tgaagacggt gaaaaatgct gtattccaag 780
   agttgaaggt tctggaagtg cttctgcttt acaacaatca catatcctat ctcgatcctt 840
   cagcgtttgg agggctctcc cagttgcaga aactctactt aagtggaaat tttctcacac 900
   agtttccgat ggatttgtat gttggaaggt tcaagctggc agaactgatg tttttagatg 960
   tttcttataa ccgaattcct tccatgccaa tgcaccacat aaatttagtg ccaggaaaac 1020
   agctgagagg catctacctt catggaaacc catttgtctg tgactgttcc ctgtactcct 1080
   tgctggtctt ttggtatcgt aggcacttta gctcagtgat ggattttaag aacgattaca 1140
   cctgtcgcct gtggtctgac tccaggcact cgcgtcaggt acttctgctc caggatagct 1200
   ttatgaattg ctctgacagc atcatcaatg gttcctttcg tgcgcttggc tttattcatg 1260
   aggctcaggt cggggaaaga ctgatggtcc actgtgacag caagacaggt aatgcaaata 1320
   cggatttcat ctgggtgggt ccagataaca gactgctaga gccggataaa gagatggaaa 1380
   acttttacgt gtttcacaat ggaagtctgg ttatagaaag ccctcgtttt gaggatgctg 1440
   gagtgtattc ttgtatcgca atgaataagc aacgtctgtt aaatgaaact gtggacgtca 1500
   caataaatgt gagcaatttc actgtaagca gatcccatgc tcatgaggca tttaacacag 1560
   cttttaccac tcttgctgct tgcgtggcca gtatcgtttt ggtacttttg tacctctatc 1620
   tgactccatg cccctgcaag tgtaaaacca agagacagaa aaatatgcta caccaaagca 1680
   atgcccattc atcgattctc agtcctggcc ccgctagtga tgcctccgct gatgaacgga 1740
   aggcaggtgc aggtaaaaga gtggtgtttt tggaacccct gaaggatact gcagcagggc 1800
   agaacgggaa agtcaggctc tttcccagcg aggcagtgat agctgagggc atcctaaagt 1860
   ccacgagggg gaaatctgac tcagattcag tcaattcagt gttttctgac acaccttttg 1920
   tggcgtccac ttaatttgtg cctatatttg tatgatgtca taatttaatc tgttcatatt 1980
   taactttgtg tgtggtctgc aaaataaaca gcaggacaga aaaa                  2024

<210> 127
   <211> 2106
   <212> DNA
   <213> Homo sapiens
<220>
   <223> nbla23755
<400> 127
   tttctctgat caaaattgtg gctgttttcc ttatgaacca taatataatc atttgtgtga 60
   tgtacattgt gccatttttg atgactaaat gtctatattt ctgccattcc tgtaagagag 120
   ggagtttttt actgatagta gcaaatgttc acttcagtca aacttgggtg ttcagtggta 180
   aaccatatag tatttagact ggtaaaaata gtttgcacac aggaatagcc tctgattttt 240
   agctctcttg taatccaagt atcattgttc atggaattct ctaggtcatt tttattgtgt 300
   tgttctaaca agacagatta ttgctacaac aatagttaca agatatttct aaaatatcct 360
   ttgattttta ctctaagtat ggtagagtaa gaggctaaac aagaagctgt ttccttgaag 420
   acattgcttt cagtcaccat acatgtctaa ataatttagc ttatcattca ttctatgtag 480
   gaatgagata agaaaggata tgatggcagg aaaagaaatg ctattcattt tttatacttt 540
   agttttattt tcttaggatc tatatcctat atatatatat tttttaaagc actaatttat 600
   tgcagtcttt attttagaaa aatgtgaagc atttttttct cccctaaaat gaatatattt 660
   agatgacaag tctttagtgc tggtagagga actaattgat tttgtactat agtaggaaag 720
   tgtttatatg tttcaccaga aataaaatat gtagggtttg tatgtaatct tctgtgttta 780
   tcctatgttg atttacctta aatttgcaac atacatatcc acataaatat tcatgacttt 840
   cttatatttc attaaaatgt tttatggctt cttaaaatca tcactgtgct tctaaatatt 900
   tttacgtaaa atcattgtat aatgctatac tgtgatatac atgaaagttt atcttgaaca 960
   gtgctcttta acaatattaa atttaaattt atcttggttt tgctatgctt atgggtaatt 1020
   catagaaaac agaaaaaata ctgttcccaa aaggcagtta tatatttcag tttaatatca 1080
   cctataagta tgagaaaggt ttccatgtct cctacccctc actgcactta ggaaaattct 1140
   tatttatgaa taaagtaaga taagtaaatc taattgccta gtcgtttttt taacacatat 1200
   acatgcaatg tatctggatg aatagaaggc tgaattgaag ctttctttat atttaagagg 1260
   taaaaagaaa tattaatact tttaaaatat actaacaacc aaaaagtgtt cagaattttg 1320
   ctataataat aatttgtatt aaaatagtac ctagaaaaat tcagtctatg gaataggtaa 1380
   aattttaaaa ttttaatttg ctctcagagt tctgtctgat aaaataattg aactataatt 1440
   ggcatgatga atattcccag gttttacttc agtatataaa tttaactctc agccacatgg 1500
   gctttccaga cttttcaata catatgatgt tgcaggaatt gcaatatttg caaacatgtg 1560
   ccacaacagt gttcttggtg atgtttctaa aacagttttt attctattaa tgttaaattt 1620
   tctaacataa acatttaatt gattaatgta aaattttagg aaggaacatc tttaattttc 1680
   aatatgagat ggttgcaacc tttaaagtag tacatatttg atttttttta aaaggcaata 1740
   tttttttttc taggaaaact attcattatg gttatttaac tgcatgtttt ttaaattttt 1800
   ccctcttgga acaacatgta ctggggccta tcaaagggtg gagggtggga ggagggagag 1860
   gaacaggaaa aataactaat gggtactagg cttaatacct gggtgatgaa ataatctgta 1920
   taataaatcc ccatgacaca aaagtttacc tgtgtaacaa acctgcatat gtacccctaa 1980
   acttaaattc ttccctcttt ttgtcttggg cacaagtttt tgggatatgg aaaagtttat 2040
   tgtatccctt ttgaattttc ttctaagatg aactttttaa ataaaagata ttactgcttt 2100
   taaaaa                                                            2106
<210> 128
   <211> 2147
   <212> DNA
   <213> Homo sapiens
<220>
   <223> nbla24549
<400> 128
   aagtcacggg tgtggaattt ggagcaatta tacccatcat cttctagaag actcccatat 60
   caaggggctt ctggtgacct ataagagttc ccctttcttt ctgtcacctc atgtaatgtt 120
   ctcacgcggg tggggctttc agtttttcaa aaggcatctt acatatgtga atcatagagc 180
   agaccctgcc agtagtgggc tgttgcctcc tggaaactga aggctgtgaa tgccaatttt 240
   cagcctcctg gagacctggc agttttgggg gaagacccca gggtagacac cagtgttcct 300
   ctaagtgtgc ccacccgtgg actggggctt ggggcctggg gctggggcta tgtctgagtg 360
   aggctgccac acatccacag ccaggcctac cttttgggca gtgctgggac tgtcgatggg 420
   accagtatgt cccggggcct gccacatctc cgtctcaggg ccctctccag ctctggattt 480
   atcccaaacc ccatggagcc caggtgagcc ctcagtaact accaatagaa gattcgattt 540
   gacggttggt ggcgtagggc taaattagtc actgccccca ttaaaaatac agcggggggt 600
   ttaagagctt ttcacgccat gtgggaatca gcagcgaagc cggctgatgc cttgggtaag 660
   gagagaggcg gcctagggga ccgtgaggta atgaggttta tggcggtgac aaggcagcca 720
   gggaacccca ccgactcccc cctcaccccg gccgcattgt tctccggctg gctctgtccc 780
   tgctgctacg gctgagagcc cctcgtgact ttgtgtgggg agggggctgg cagtggggac 840
   cctgaggccc ttcctgggac tggcattctt taccatcagg tggtattagg gttggggagc 900
   agtgtagggt tataaacctg tgcctcggag aactcactaa ccccttccag aggaaaggtc 960
   tggagctggg atgagacact tgcctttcaa ctgtgagggg ccttagaggg tctctgggag 1020
   gcttgtatga agtgatgcct gacaaagggc tgcacacaga gacctgtaag cagcatggtc 1080
   atcaatgatg gtgccaggca tcctgcagga gggcaccttt tcagccagga gggcgcgatg 1140
   gaatcagcct ctcatttgga tttggctttg gggagtgggc gaggtgactg aaagcctaag 1200
   gttcattgct gctgtattta gatgtacaaa ttgtcagttt ctagcggctc tctgggcaga 1260
   ggaattttgc ccaggctggg aaatggacca ggaccaggac aggccacagg cccctgtcat 1320
   ggaacacctg cccagagtgc ccagaagcag gcaggtaagg gtttcagtct cagtggagaa 1380
   actgtcatgg gagaaatctt ctcgagttcc ccagccttaa agaacgctcc ttttaaattc 1440
   acagttgtgt aatattgaac ttttcacctg tttttcttcc ctcctaaggt gtgtgttcct 1500
   agggatggaa cctgtaccta taaatattca gtaaatagga ccaaaactca aatccatccc 1560
   ttcctctact catccattca gcaagtattt actgagctgc ccccaggtgc caggcactgc 1620
   acagggcact ggggataagg agatgaccag cagatgtggt ccctggcctc atggaggcca 1680
   cagtggcaca ggcaagcatg ccagtaaatg catagccaca cctggtcatg agtgccgtga 1740
   tggcaaagcc agaagctgcc agggattaat ggagggaccc gtgtatgaga cacaggggtg 1800
   cattaaggaa ggccttgctg aggcaggggc ttgagacctg aagaaataag ggaggtgggc 1860
   agtccaagag cagtgggaag agaacactgc aaatgcagaa gccgtgagct ggaaacgagc 1920
   cagaagtacc cttagagagc tgtattccta tggagtttca attctaggtg gcttaaaaca 1980
   atcccagaga agttcagaat cttgtccaag atcataccat tcaggataga cttgcgacta 2040
   taacttgggt gcctcacctt ccagcctggg gtgtctgact ccattgttaa ttttattata 2100
   acaatcatga tgacaacgat gaataaagtg aaattgtact gtaaaaa               2147
<210> 129
   <211> 2353
   <212> DNA
   <213> Homo sapiens
<220>
   <223> nbla20084
<400> 129
   aagcagagtg aggactccct ggccagtgtt ctctaccatc tcttctgcct accttctttt 60
   ctctcatgga agtaagaaaa gaatccattt catcaaaggt tgaacattcc acttcatccc 120
   tgaattctct cttgctttga gttcttaggt acatctatat tagatatcac tttctcctct 180
   gcatccccaa tgcccttttc cctcctcagc atacctgatc ctctgtcctt gctgaccttt 240
   gtatgtgtgt gttttctccc ttgatgacat atccctcttc agctattgct ctatttatat 300
   tcagaatccc aggcaagcaa cataaataga tgtctccaag gagtaagtga tttaattagc 360
   ttgaagtatt atatatattt tcacacacac agacataaac atatatatgt atgtatgcat 420
   ataaaacaaa taagataaat aactggaaaa tatatgcaat gaagtcagtg aattagcaga 480
   tgagataaac atcccagatt ggtatggtat tgtacggtac ggtgtggtat ggtatggttt 540
   ggtaccacaa ggtctagtgt ttagagctgg ctctgccagt cactatccct ttgatactga 600
   tcaaatcata caaaatcagt ttcttcatcc ataataaagc cttcccttgt tactcagagt 660
   tgctgttcca gtcaaaaata aaatgttaat ttatgataca gaccttgata agctgtatga 720
   accttacatg atactaatgt agtaagatgc accacggttc attcagataa gtgtcccagt 780
   gagtcctcag ttttcacaaa gtcatttcct atccccagtt ttgtttattt gtgcacctct 840
   gcatttacct agaacaagaa ttgttatatt ctaagtaatt gccaagaagt atggtaacaa 900
   attcactact acttgattct tcagtggaga aaattatata catatatata tatatagatg 960
   cttgcctaaa tgatatgcca ttcttccata cttttaaata ctgtaacttg tgttattgaa 1020
   ttaagccagc cagtcaaaag cttgaaatta aacatagtat tttcctatga aatatatttt 1080
   ttaacattat aaaataaaat ttggaataaa agcattatgt atatatatat atatatatat 1140
   acatatatat atatactcat aactcttcat tcatttttgt gaatcagtct cattcgtagt 1200
   tttattgtac ttccaaatct tcatttttct ttggatcatt ttcctttgcc agcattacgt 1260
   gtgtgtatgt gtgtgtatgt gtgttttaaa agggccataa gaggaaaaca gcaagaagtc 1320
   tgtctcctaa ctttgaaacc taaattttag ttttctttca cacaggaagt catcttgtgg 1380
   atctaataat attatgaagt tattctcttt ttgatggaca tttaggaatg ttttgatgtt 1440
   tcgtacaata attcaagttg caagaaataa gcacacacat tttgcaagta catctatggg 1500
   atatgttcta aaaaattaaa gtgcattctt tgtcccttaa cctatgatac gggcattctt 1560
   tattttgata gatgctgcag aattgtcttc taataagtca taggaattta cgttttcagt 1620
   taaaatgtat aacagttctt gttatataaa atgtataaca gaaaggactt ttgatttcta 1680
   tccatctgaa tgatgaaaaa tagtaagtta tttattaaat tattattacc ttcacatatg 1740
   tatagtggaa cactgagcat atgtttaaaa gctacttgag tttttaaaat ctgtaatctg 1800
   tgctttactc attttcttta ttggctgttg gttatttttt actgattttt gatgccccta 1860
   tcttgttaag aaaataatat ttttataata tatcacattt atcacaagtt actgtttatc 1920
   tttgagtttt cttattgaat tttgacatac acaaatggct catatttact ctttttatca 1980
   gcagtgtctt tgttagttcc tggatttggg atatgcttag aaaagtttat cctaaacaaa 2040
   gacttgtgta cttttaattt attgtcatgt ttaaatcgtt gctaaatctg gaatttattc 2100
   tggagtaaga agtgaagtag agttctaact ttactttgat tttgttttgt tcttattgtt 2160
   gtttttcttc cagatttgtt atctagttgt ataaaaacca atgattaaaa aaaaaagttt 2220
   tttcttgctg ggcaaggtgg cttacgactg taatcccagc actttttggg aggccggggc 2280
   aggtggatca cttgaggtca ggagttcaag acaagcctgg ccaataggtg aaaccccatc 2340
   tctactttaa aaa                                                    2353
<210> 130
   <211> 2194
   <212> DNA
   <213> Homo sapiens
<220>
   <223> nbla21081
<400> 130
   aaatttctca acaccacagt cagctaagtc acctactgcc accttcgaaa aacacggaga 60
   gcacctaccc agaggagaag gtagatttgg agtaagccgc cgtcgacata attcctctga 120
   tggttttttt aacaatggtc ccctacgaac tgcaggagat tcttggcacc agccctccct 180
   gttccgccat gattctgtgg actctggtgt ctctaaggga gcatatgctg gaatcacagg 240
   gaacccatct ggttggcata gctcttcccg aggtcatgat ggcatgagcc aacgtagtgg 300
   aggtggcaca gggaaccatc gccattggaa tggcagcttc cactcccgga aagggtgtgc 360
   ttttcaggaa aagccaccta tggagattag ggaagaaaag aaagaagaca aggtggaaaa 420
   gttgcagttt gaagaggagg actttccttc cttgaatcca gaagctggca aacagcatca 480
   gccatgcaga cctattggga caccttctgg agtatgggaa aacccgccta gtgccaagca 540
   accctccaag atgctagtta tcaaaaaagt ttccaaagag gatcctgctg ctgccttctc 600
   tgctgcattc acctcaccag gatctcacca tgcaaatggg aacaaattgt catccgtggt 660
   tccaagtgtc tataagaacc tggttcctaa gcctgtacca cctccttcca agcctaatgc 720
   atggaaagct aacaggatgg agcacaagtc aggatccctt tcctctagcc gggagtctgc 780
   ttttaccagt ccaatctctg ttaccaaacc agtggtactg gctagtggtg cagctctgag 840
   ttctcccaaa gaggagcaac ctgtttgtgg tatttgccca gagtccctcc agcaccaccc 900
   ctccaattga gatcagctcc tctcgtctga ccaagttgac ccgccgaacc accgacagga 960
   agagtgagtt cctgaaaact ctgaaggatg accggaatgg agacttctca gagaatagag 1020
   actgtgacaa gctggaagat ttggaggaca acagcacacc tgaaccaaag gaaaatgggg 1080
   aggaaggctg tcatcaaaat ggtcttgccc tccctgtagt ggaagaaggg gaggttctct 1140
   cacactctct agaagcagag cacaggttat tgaaagctat gggttggcag gaatatcctg 1200
   aaaatgatga gaattgcctt cccctcacag aggatgagct caaagagttc cacatgaaga 1260
   cagagcagct gagaagaaat ggctttggaa agaatggctt cttgcagagc cgcagttcca 1320
   gtctgttctc cccttggaga agcacttgca aagcagagtt tgaggactca gacaccgaaa 1380
   ccagtagcag tgaaacatca gatgacgatg cctggaagta ggcatataaa tgctcacagt 1440
   taaatctgac ccagtaaact ctgtgtgttt agggagtata caaaagaaat cgttcttttc 1500
   cttttcttat gttgttgaat acttcattca caagggaaat aatcatatcc caaagagaga 1560
   gcaattggct tgttttgctt ttgttattgt tcttccctgt tatctgcttt atagagagaa 1620
   gtttgtgtgg tgggacagat tttttaaaca cactcacaca cacacacaca tacacaccca 1680
   gtatatatgg ggcgatgcac aggtaggagc tggcagtgca gggaagagga gacactggtc 1740
   tgcagcaaca gcttctacta ccagcccttg gggcactcac ccctgtgatc aagcaatcat 1800
   tgtcaatgac aaagtgacta ttgaagttat aattgtatta aattaatgct aataatttgg 1860
   atattttatt ttatttttgg ctgctcgggt aactttagcc cttaaccaag catatgtggg 1920
   tttttttggt tgtttttttt tgtttttttt ttctttttcc tttttgggta cagctgtaaa 1980
   atatttggat ataggaaatg ttgtgttatt cttgcagcct tgatattcag ggtggattgt 2040
   aaaatataaa tttttgtgag atttcaaaga ttaagattat tttgataaca ttatttacag 2100
   attttaaaag atgtggttat cacaagtctc gagggggaaa ctactgcata aaataactaa 2160
   cttggaataa atattttgca tcagtttgga aaaa                            2194
<210> 131
   <211> 4042
   <212> DNA
   <213> Homo sapiens
<220>
   <223> nbla21420
<400> 131
   cgaacttggg atccgtctcc tctttcgcct cctccacttt gggagccccg ggctactctt 60
   tcacagcccc tgttgccctg tgatctgtag gtccttgggg acgcatagtt aaggtgccag 120
   gacatcctgg aagctgggaa atggtgagta tacggagttc ggcatcccga gaggggagag 180
   caggctgtga aaccggcagg accggcctcc ccacggttag ctccgagtct cccgcagctt 240
   ggccctcagt cccctgtggc tgcaagatgg ccgctgggcc agcagcgagg acccccacgt 300
   cccgtccggc ccatccggtc ctgtccctgg gcagcgccct gctctgcgcc cacagccatg 360
   agtatttccc agattgttca gggaggcctg gtgggtcatc agggaaaaac cgcgactggg 420
   tgtttgcgtg ggaggagctg cggcccgtgg ggtccccagt ctctcttgtt aaaaattaac 480
   gggagtctat gttaaacgtt aaccagttta tctgaacaaa cagtgattgg tgaaatggaa 540
   agcacccagc catgatttct ggtccaccag aggggcataa aggaaaggct ttcataagat 600
   gcatgagaaa gcagcccaaa ttcaaaaatt ggttccagtt atgtagtcac cttatttgaa 660
   ctatccagat ggaaatgtcc tggttacata ttcagaggtt aattgcatgt ttgccattgg 720
   ttaaacgtgc attttgtttc aggctaagat aatggtttat aggaaatgta tttgagttag 780
   gttttagttt tttttttttt taacctatga acccaggaca ctagagccac tttagtctaa 840
   ttttctgctc tttaattatt ttaacactcc agaggaggac tggttttctc ctgtgttttt 900
   ttaatatatg gcaagtggaa cctctaatcg accaccctgt ttttcagcct aactcaggct 960
   tgtggtaaaa ttatcagttc ccactttctt tgctgcattc tcaaatgcaa cacaggagaa 1020
   cagttttccc ttgcaaattc acaaagctgt taactatttg tcctttatta tacatttcat 1080
   taaagttttc tattattgga tttctttcta cttctcccta cagttctgcc catatttgct 1140
   ttttatattt agaagcctcc cttttgggtg cataaatata tatagctata ttcacttgac 1200
   aaattaacct ctattattat tgtatggtaa actcatttca tgcttgtgag agacattgct 1260
   agaaagtcta ttttgtctaa tttaagcata actaccattg aactcttttg gctattattt 1320
   gcatggaata tcattttcta tcctttcact attagcctat gctcttaatt cataattgag 1380
   tctcttgtaa gcagcatatt acgaggttta aaagtttcat ttatccactc tgtctgcttt 1440
   agtctctttt ggctgttaga atatcacaga ctagtaatta ataaggaaca gaattttatt 1500
   tgactcatga ttctggaggc tgggaaggta aaagaacatg ttactggtat ctgttgaagg 1560
   tctagttgct ggataataac atggccaaag atgtgaggga gagagagctt tttttttttt 1620
   aatatataac agatccattc ttgttaaaat tagcccattc ccataataag aacattaatc 1680
   cattcatgag ggcagagtgc ttatagctta attaattttt aaaggttcca cctcttaatt 1740
   ctatcacatt ggtcatttta tcctaaattt tggagatgac attcagtcta cagaagtatc 1800
   tgtttagtag ataatttaat ctttttattt gtaaggtagt gataggtaag cagttactat 1860
   tgtacatttg tagttttctg tccattttaa gtttgcttct tttttttctg gttctgtctt 1920
   tcctgtggta ttgttcattt ttgttgagac aaagttatgc tttcttgctc agactgaagt 1980
   tcagtggcat atcacagctc actgtagcct caatctcctg ggctcaagca atcctccccc 2040
   cttagccacc caagtagctt ggactacttg gacacgtacc acaacaccca aggagcttat 2100
   gattcttcca ccttggcctc caaaagtgtt ggaattataa gcaggagcca ctgtatccaa 2160
   tgtgtaattt ttgttgtttg tgtatgcttt aattactttc tctttttctt tactatgttt 2220
   tttttttccc cagtggttat catgagactt atgtaaaacc tcttgtattt taatagtcta 2280
   gtttaagatg ataacaattt agagtattct gaatttcagt atgtatttac catttttagt 2340
   gacatttata ctttagtatt tttcatattg ttagttagct tttcgtcata tcaatgtgaa 2400
   gatttcttcc agaccatggc tggagaagga aagaaggtgt gttttgcctg attcagggac 2460
   tatagagaga accaagttct gcaggcctgt cacctaagtc tcagatgagt atgaattctc 2520
   ttgtgttttt cacagatttt tgcagtggca ggaccaagtt caaatgagtc atagccaagt 2580
   ctacagtaag atgtggtagt attctgtttt gaaccgagga ccatgattgg caagcttgcc 2640
   acttggtcaa gtgcttaccc tctaaagatg tcttccttgg tctttgcctc cagctgggtg 2700
   tcacaaactc tgaactggat tctaaggctt tcatgaatgc acttatgttt cctgtggcag 2760
   ctgcattatg ttgtggggga tgtgcatgcc gaacctccca ttctgtcatc ttgcttatgt 2820
   tactctcctt tatgtttcac tttctcaaat gaatgtcaag ctggtgattt ttagattcaa 2880
   aaattctaaa ataaattgct caaatttcca cattatgtaa gctattaata aaatgtcttg 2940
   taggtgctac atatttatta aaatttttgg ttgtaatttt aagctcactg caggcagaaa 3000
   ggaatcatta acatttatat tctttttttt tagtctgtat ctaaatgatg gcatatttta 3060
   attccagata tttactttat actgcagtaa tgctcgtcat attttgcaaa atttatgttg 3120
   ttcttttatt tggaaatata aggctttttt agctcctgaa atctatatta tagtcatata 3180
   attttattat gttttgtggt aagaagtgca gcaacatatt gagaacataa taaaattatc 3240
   ctgtattttt aatgattatt tattaaattc ctctcattag agcctgttat taatgattgt 3300
   aatgtatttt ctgtataatt ttactgcaat ttattaaatt ctaatgactt aaattgtctg 3360
   cttttcatga gtgcacacag ttgaatgctg tagatatcta aagaattatt tttcggccgg 3420
   ttgtggtggc tcatgcctgt attcccagca ccttgggagg ccaaggcggg tggatcacga 3480
   ggtcaggaga tcgagacaac cctgactaac atggtgaaac cccgtctcta ctaaatatac 3540
   aaaaaattag ccgggcatag tggcaggcgc ctgtatcccc agctactcag gaggcccagg 3600
   ctggagtgcg gtagcacgat cacggctcac tgaagcctca aatccctagc cttaagtgat 3660
   ctacctatct cagcctcctg agtagctggg actaccgacc tgcaccacca agcctggcta 3720
   attttttaaa atttttgtag aggttgagga gggaggggct ctgttgccca ggctagtctc 3780
   gaactcctgg actcaagtga tccacctgcc tcggcactgg gattacaagt gtgagccatc 3840
   acacccagct tccctgagcc tttatacaga actcgccttt gagttaggtt ctgttgtata 3900
   ttctagttag ggcattatat tgatttttta aattactatc attctgaatt aataacaaat 3960
   tgtggtacat tcatacagtg gaatagaact cagcaataaa aagtaatgag gggaggtggg 4020
   gatggttaat gggtaccaaa aa                                          4042
<210> 132
   <211> 1898
   <212> DNA
   <213> Homo sapiens
<220>
   <223> nbla22452
<400> 132
   aactataaag tgtggttcct gtatgataca ttattacagt tgtcttcttc cttaaagact 60
   catcttgaat ataaagaaaa agacaatgta agttcagtgc acagcatcgg gaacattaga 120
   catgctcagc gtatgccctt cctgccaaca ttgtacctct tcctcctccg ggttggatgg 180
   gcactaactc gtcctcagtc ctgcagctag atctgcaact ttgcttaatg atgcaggtta 240
   aaattgaaat agaattatgt attattattt ttcacattca tttttgcctg agacaggagg 300
   tggagggtgg taaattaaga aacccgggaa gctcagtgct tgagagaacc catagaagct 360
   acagtctagc ccatttggct tcttactttg ttagattaga taatcatacc tgctgctcca 420
   ggcgtgacta gcccagtggg agtcaggaag gaaattattt ccctctgttg ataccggttt 480
   acaattgccg actgtcgcca agggctttca gttttaatat ttcctctttg gtcctcagaa 540
   gtatcaggta ttagtctctg ccggaagcaa agcattggtc acttccgtca gaggtgaatg 600
   tcttggctgt ctataattcc tcagtcaggt gctttctggg catgtgtgag catttgctca 660
   gctagctttt attgcttgta tgttatttgc ttcaaaaatt acaagaggat ttgtcgggtc 720
   tgagcagtga cctatccagt cccctgaaac tctatggttc ttcgtgtaac ccagggatgt 780
   cttgtaggag gtatgtttgc tgtccacgaa agtaaaaagt agtgatatct ctttctctct 840
   tttgcttcct tcctcctaat tccacattct cctatttctt ggcttctggc acagtggaga 900
   taccgctact ctacattagg catggcctta ggggatccga attctcaggt cttcctcaat 960
   gagttgctgt gtggtagaca gcatctgaag tttgaatgga tagagagacc tttgtagatt 1020
   gtggccaaat atttacacct ggttcataga gtatgtgttt gctgccctga tctcagtgtt 1080
   ggtctgggtg ttagtgaacc tcatgatctt taggaaacta tgtgaattag gcttagtccc 1140
   tgaccctgag aagcttatag ttagggaaaa agacaaacat ataaaggaga aatacacatt 1200
   agaaacatat tctttttttt ttttgagatg gagtctcatt ccgttgccag gccagagtgc 1260
   ggtggtgcga tcttggctca ctgcaacctc cgcctccagg attcaagaga ttctcttgcc 1320
   tcagctgccc gagtagctgg gactacaggt gcgtgccgcc acgcccagct aatttttgta 1380
   tttttagtag agacggggtt tcaccacatt ggccagaatg gtcttgatct cctgacctca 1440
   tgatccatcc accttggccc tccaaagtgc tgggattaca agaaacatat tatttatggt 1500
   acacatttat taatcaccag atatgtttca ggccttacgc tgagtgcttg ggaaattgag 1560
   ataaattata gtctcagatc tcatggggcg tggatgaaga gttgggagaa agaaaaaaat 1620
   aggccaggcg tagtggctta tacctgtaat cccagcactt tgggaggctg aggcaggcag 1680
   atcacctgag gtcgggagtt caagaccaga ctgaccaaaa tggagaagcc tcatctctgc 1740
   tagaaataca aaattagccg ggcatgcctc tagtcccagc tactcgggag gctgaggcag 1800
   gagaattgct tgaacccagg aggcggaggt tgcggtgagc cgagatcgca ccattgcact 1860
   ccagcctggg caacaacagc aaaactctgt ctcaaaaa                        1898
<210> 133
   <211> 1798
   <212> DNA
   <213> Homo sapiens
<220>
   <223> nbla22595
<400> 133
   aagtacaaat ccatagggca catgagaact acaatgtcta tctacagtaa atacagtttg 60
   atgaataaaa tgaaaggcaa ttgacctaag gtgaaaaaaa aaaacaaaaa acaatcaaag 120
   catgggtact atgtgtcatc tgtaagagca tttggttaag aataacaaac aaaccagtat 180
   tatcgtttta atagccgaaa ttggcaaaat ttccagtttt tctttcataa gaatgttctt 240
   tgcaagaaaa aattttcata tagtgagagc aaaaatggca accatttgca agtaaatgtc 300
   ccatgaaatt aagtagcaga tatcaagctc atgaccttca gatagttacc cctaactcaa 360
   tcacttacat agcaagtgca gataattttc atagctccct attaaaatta tatttcaatg 420
   cccttacaaa ttgtgactgt ttttaaataa agttgaccaa ctaaaatttt gtatatgaca 480
   tatgataaat tccccttcaa gtcaccttac atttacttaa ttttattagg cagtgtctgt 540
   ctaccaccca ataatacttg aggattctcc ctccatttgc acagacatca tagctgggaa 600
   acagggattc acaagaccca ggctgttccc tacatatgtt tcctcctccg acatcagttc 660
   atcagtcaat caagccatgt gagagtggag gccttgtatt ccctattatt cttgggcact 720
   ctactccaag taggaaaagg ccaggaggtc ctgttaaagg atgcactcag agcccgggct 780
   ccctaacgta tgagagtgct aaccagcagg tgtagacttt tcaggagtga agaatgaggc 840
   aggcattcca aacctggacc ttcatcacct tttgtttcat ctcaagacaa ttctgaggga 900
   ctgttttgga gcgtgtctgg aaggtgaacg ttgaagaaga gtgtgggctt tgatgtgact 960
   cagttgagat ctttcatggg gaggcaggaa ttcaatgccc agaatctggg ctggtgtctt 1020
   tgaggtcagt aggttgcgtc tttgtatcca agtccattgt tactaggttg gaggctggag 1080
   attctaaatg gcttccagac catctctctg attctctttg ggagatgggg tctgaaagac 1140
   aatgtcagta gttttgggaa attctagaaa gtgtgcttgg aaacgtggga agagctcttg 1200
   cctagtgcct aaacgctcca tttgcagctc tagccaagta gatacttggt aggtatagag 1260
   ccgggtttgc atttatatca gcaaaaccta tgtcagaatt gaagaagtag tcaggaaaaa 1320
   gtgtcttggt cgcaggccgg ggaacatctt aaaagcaaac ttctagcctg ctgactcttg 1380
   gcaatgagtg ttggatcctg gctaaattgc cttgaatgca gcatgaggcc aatctatgaa 1440
   tccaacttct catggagaaa tgttaatatt ttttcagttt gaatcaatca gggtgaaact 1500
   accatgctat tggtttgctt actttttatt atttcatata aaatctaaga caaaatacat 1560
   taaatgctta ttgacatatg tatttattct tcaccgggct gataatatct gcctgatttt 1620
   aaactttctt ccattgtgta ggtttcaact tattctattg taagatactg ttaaatctaa 1680
   tagaggcatt gtcactttta tgtataattt tattttattt catatatttc ctattggctt 1740
   tttacattta aattatggag cacttcatca tataaaaagc ttcaattata tttaaaaa   1798
<210> 134
   <211> 1528
   <212> DNA
   <213> Homo sapiens
<220>
   <223> nbla22676
<400> 134
   ctatgtatgt gagtccatgt atgagtgtac caccccatat ctatgagtgt gtgagtatct 60
   gtgagtccat gtgtctgtgt ttgtgagcgt gtgtttatga gagtctaggt atgtgagggt 120
   gggtgtccga gtgcatgtgc ataagtgcta gagcctctct gtgtgtttgt gtgtgtgtgt 180
   gtgtgtgccc gtgtgtgcac gtggggtggt atacacacag ggctccaggg ctggcatcag 240
   gggcgaggcc agtggttttt ggtggttgga gtcagtggag tcaggaacag gacagagtcc 300
   cagagataac aggaaataga agaattgctg caatcgaacg tgcaaagctc tctcaacttt 360
   tctgctgaca aaccgcaaac tgcccgcgtc cacccccact cgtccccctt ccttcctgcc 420
   acagtagaag ggtggggctg gcgtggctat cctggctgcg cccacgccct cctgctgccc 480
   agcaaccgcc ccgggtgtgg attccatcgc tccctgggct tccagtccct cccaccagcc 540
   cctgccccgc tgtgcagaat atgctcggac ctcctagggc cacataaaac caccccctca 600
   gccagaccag ttcctggtca tcctggcctt agggctgggc actgggtcag cttctgagca 660
   ggcaggagct ctgctcatgt ggacctgaca cacattgcat gagcagacgg gaggaaaaga 720
   agccagttcc tgggagggag tgcactggcg aaggagtgtg tggcgtgggc agagagcaga 780
   ggtcaggggc ctccctgaga agggcagtgc gactggcatc tgaggggtga ggagaaaggc 840
   ctggccagag tcccagcttt atgaccattg cagggcagct tctgggctgt gcagctcaca 900
   cacaccttcc cctccttccc ctccttcccc tcctctctgc cctggggcca gcctccctcc 960
   tccactcccc tgaaatggct cccagccata attagcacag gacagaaaca gcaaatgctg 1020
   gtcggtgtgg taggctcacg cctgtaatcc cagcactttg gaaggccgag gccggtggat 1080
   cacttgaagt caggagttcg agaccagcct ggccaacatg gtgaaatccc atcccactaa 1140
   aaatacaaaa attagttgga tgtggtggtg cgcacctgta atcccagctg cttgggaggc 1200
   tgaggcagga gaatcgcttg aacccgagag gtggaggttg cagtgagctg agatcatgcc 1260
   actgcactcc agcctgggtg acagagcgag actctatctc aaaaaaaaaa aaagtcctta 1320
   gaacaaccaa ggcctttcta agagtgtgcc ctaagcaagg ctgtgtgctg aatgctttga 1380
   atcatctcat ttgatataaa caccctgcta ggcacgatgg ctcatgccta taatcccaca 1440
   ctttgggagg ccaaggtggg aggacctcct gagaccggga gtttgagacc agcctgggca 1500
   acataggaag gtaccatttc tacaaaaa 1528
<210> 135
   <211> 1132
   <212> DNA
   <213> Homo sapiens
<220>
   <223> nbla22909
<400> 135
   gttgcttata ggttttcaga gtaaaagcag ttatgatctg atttcaaaaa taattgttgt 60
   aggaataatg acctatctaa acttttattt aaatttctgt ttaaacttct atttaaattt 120
   gtgataagtt cctcatctga aatgagctgt ctttgttgct tttgttctct ttttattaac 180
   tatgctcaga ctttaaagta tatacaaatc acctgaagat ctttttaaaa tctagaatct 240
   gattcagtaa ttttggggtg gggcctgaga tttttcattt tttgcaagct cctaggtgat 300
   gctaaatgct gtttgttcat ggaccatatt ttgagtacaa aggatctaaa ggaagatatt 360
   ttatattgct ctaatgtaac atttttaaac ataaacaact ttagattctg tgaaccttaa 420
   agtgatccgc ctcaatctaa gagaataaca attttgggag acacttataa aaataatgtg 480
   atgttagctt aaacattaca cggacattac aaccttacaa cttaggtgag agaggctttg 540
   gttatgctga gttgcctatg tgctagtgat aacactaccc ctttcttcta agtaaaatat 600
   ctcaggatac aagtgaaaaa taatagtact gttatcgagt tctctttggt ggtcaccatg 660
   atgtgtgttg aggagcagag tgaacaaagg caacctgatc cctgtctctg tagagcttag 720
   tctttattca ctgccagtat tttatttttg cttcatagct aattgagaca cattgatacc 780
   tgatgattgg gaggaactgt tctaatgcga tttgtaaaag gagaattcaa attggaagta 840
   ccagctaggc acggtggctc acacatataa ttccagcact ttgagaggct gaggtaggag 900
   gatcacactt gagcccagga gtttgagacc agcctgggaa acttagggaa acccgatctc 960
   tattaaaaat ttaaaaatta gccacgcttg gtggcaggca catatagtcc caggtaactt 1020
   gagagattga ggtgggatga tcacttgagt ctgggaagtc aagactgcag tgagccatga 1080
   tcatgggact acactgtagc ctgggtgaca cagcaagact ctgtctcaaa aa 1132
<210> 136
   <211> 2160
   <212> DNA
   <213> Homo sapiens
<220>
   <223> nbla24435
<400> 136
   aggagaaact gtttttgtac actgtacatc cttagtattt ttacacgtat atgataggga 60
   tgaacatgat tttccttcgt acagacagct taaataaagc actatgtcaa tctgctactt 120
   ctctgtttat tgttgttgga tgtggttcta taatcccccc aaattaaatc ttctttaatg 180
   aaaacatgat ttttaatagc cccagctggt attaacctac cttgtataaa atgtgacagg 240
   aaaatataga aataattcct tgtagctcac acacacacac ataggggatc atttttactt 300
   cagtgaaatg gcagtagcgc ggttgtgaaa actttgatga acggctgctt ctgaggggaa 360
   acgctgacct ctcagcactg gatttaggat ggatgtactg tgaagccagg gatgaaggag 420
   gtctcagacc ctggggacat tcagacccga atcatctata caacacacgg tttggaccca 480
   gaatctgaag gaatgtagct tttcattaac gtcttcctga taatgtactg ctctgcatat 540
   ttcctttctt agagtgtatt tctaacaaca tgtcatggca aattaacaaa cttagacgtg 600
   ggtgatgtag atgggtagga tggctggact gcagtctgac ttcacgttga atcattctgg 660
   atggggcctt tttctgattt tacctcataa agctactatt gtagaaactt ggctttgctc 720
   ctgtgacgaa gccagacaga ggaatggctt ttgggaccag agtgagtcaa gcatgtatgt 780
   gtatgtcaca cggccaaatt tgagggcatt ctcacatgtg ctcttctctc aaaaccactg 840
   gggttgacag atccaggagg ctaaaaaaaa gtgacctcta taattcttta aaggtgctat 900
   ttttagaata ttgtataatt tattcacagt atatctaaaa cagaattaag gacaattaaa 960
   atatcttatg tgacagcctt tatgtctagc acatttgatg aaataaaaaa cttctgaatc 1020
   tgaatagaag ttctactgtt tcaggcttga accttttaca tgctcaagag attcaaatgg 1080
   tctctgtgtg tagatcatgc caccgcctcc aaagcctaat ccacatcact tctgagaggc 1140
   aaggctgagc atatggtgac atcagctctg tgttgagatg gtgatgagga tgatggctcg 1200
   ctggccaggc agggcagccg aaggtcaggg acctgtccta actaactgca gccttgcctt 1260
   tagtgtttgt cattctcaga tacaacacgg tatgtccagt gtccgttttt attactttaa 1320
   agcatttgag ggcttaattg tgtatagtag aaatactatt ttagacaaat aattatctgt 1380
   gtacagatat ttgatatact ctaagtaaat tttctaattt cactaagtac gtttttaggc 1440
   tcctctcaaa tactgcgtat tgaagaaaaa aatctgacac caccgagcca aagatgcttt 1500
   tttgtctgtt ttcgttgttt aacagaatgg aaagagtaat gcatagtgct tcctggtgtc 1560
   tcctgattga ttgattgtgc acaaagtagg acgataaata aataaaatgg agtctgatgg 1620
   gacattgatt aaaggtgaag gatgattgat atatagatca tgaaaagaaa aatgaatggc 1680
   aggaaaaaaa gtttggtcct taatatactt tggcctagtt aaaatatgtg cctttttggt 1740
   gtgttttgtt catcactaca agataaaaag gaaacattac aactcaagtc tttaaaaagt 1800
   tcatttattg aaaatcatat gtataaccta gcatacgaat gagcagattt aaacacataa 1860
   cttcaagcca tttctgaaaa catacaccag gagctctgct cagctagagt cagactccag 1920
   ctccagcccg actgcgtgcg gggacagcgc ccgcgttgat gaggaccagc cccactgcag 1980
   gctgaggcgg tgtcaccctg ggaaggtcgt ggtgcgttgt ggcatattaa gtctaaacca 2040
   gatgaatgta aatatctctt tgtaaatcat ttatttcact ctgttccatc caggtcagca 2100
   atcagattgt ggcatgctgg gtaactggaa aaaataataa aaagtaagtt tcaataaaaa 2160
<210> 137
   <211> 1766
   <212> DNA
   <213> Homo sapiens
<220>
   <223> nbla20146
<400> 137
   aaaaaagaaa acagccagtc tgaagtatcc attactcaag tcccaaggtg acctctctct 60
   cctcagattt ccttcttggc cctgtgccct gcactttctt cactgtgttc aagtgtcata 120
   gctatcaggc cactatcatg gatatcatgt atccttcctg gtgctcacac acctgtcacc 180
   ttgtaaaaca cggacattag tgtgaacaca ggacagcttc gctctttctc ttcctgcctt 240
   tcctctatca gagaagttga tccattaagt aattatgttt ggtctattgt aattacagat 300
   gggaccactc aggggcaaag gtctgactct tcctggtagg tgtaacagat agttcacctg 360
   tgaacgaaca tcagcttaca gatgatgagg acttaaggtt gcaagaatga agatttcaga 420
   ctccaagatc ccttattctt tgggccttga gcaggttagt agtcccctgg tgagaagaga 480
   acattttgtt tgtggggcta atgggcccag aggagggtaa gactctgctg tctaagctga 540
   agcctcttcc tcgcagcgag ggtcttccta ggaacattga tgctgcctca gacatcctct 600
   tttctccaga gtagggaaga ctcccactga tctgagaatg agcccagagg cttgttgggg 660
   gactgtttta ctctgatact acctggatat ctagcttcct ttacccctgt tctgcttaac 720
   agaactgcca agcccagaag tacctttgca ctcctggttt tcagtggaca gaggaagctt 780
   tagatagaga ctttagaccc tgccctgcag agtcaagact tgaggccatt gaagctgcag 840
   gaagccctgc ccagggatgg tcctgccatg aggaggctgc aaccctataa gagggctcaa 900
   gattgtgaat tctgctcctg ccatgaggag ctcagaaggc caggaagcca gcaatagggg 960
   agagaatctg tgtgcttatg gacagtcctt acctaaagct gtttctgaat gttgcaccct 1020
   ttgagaaatt tcttctcaga accataaatt gaaacaaatg aggactgatc ttgtatacaa 1080
   agtgccaact caagagggaa gttggagtat gtctgttgca gagaaccaat atagcagtgc 1140
   ccaggggtag agaccatgtg ttccatactc ggatatttgg gtctttttga gagagctggg 1200
   gaaagtagca gcaactagat taagactggg aggattttga ccaaactaaa ggccttttct 1260
   ccttactgca tctgacgtgt gtcttcttga gacaagatag cacccatgaa ttacatcatg 1320
   aggtatgtgt gaattcagtt tacatgtaag acctgagagt tcgaagaggg cacattccca 1380
   aagacattcc cagtcatgaa atgtagaaga ctggaaaatt aagacattat gtaaaggtag 1440
   atatggcttt tagagttaca ttatgcttgg catgaataag gtgccaggaa aacagtttaa 1500
   aattatacat cagcatacag actgctgtta gaaggtatgg gatcatatta agataatctg 1560
   tcagctacta ctaggcattt attgttaatt gagttacaga aagtcattca agactgagtt 1620
   tatagaaagc atattgcatc tatctctgtg tagaacattt gattcacatt gtgaagaatg 1680
   cagtttaaaa tatactgaat gcaatctaga tgtattgtac acgaaaggtg aaaaataaca 1740
   ggtgctcttt actgtttaga taaaaa                                      1766
<210> 138
   <211> 2470
   <212> DNA
   <213> Homo sapiens
<220>
   <223> nbla20170
<400> 138
   agctttaggc acgttttagt gatgtgtagg actttgacct atatttggtg tggcttctat 60
   cctatgaaaa gggaattgag tgttttgact cgtcgtttcc cacctgttgg gcctgtctgt 120
   aggtatacct tctaaaatca actgacatct ccattttgct acagagtagc aaaaatcaac 180
   aatttttaag catactaatg gtgtgcattt gatctgaatc ttcttgatgc tatcatgttt 240
   cagctgtgaa tatagcctgt cagatgctta gaacaatcag ttgaactggt atgagtggct 300
   gcattagggc tttacaaatc gttaggactg aattttggtg ggtttagaga gtgcattttt 360
   atagctgagt tgaatgtgat gagttcacta caggcttttg gcaaaggagg ggagctgcag 420
   tgagtagctc ataagattca ttttataaat agaaacataa ggattttgta taaggcctca 480
   cctgtttata atctacctaa gattcttttt gggaattaaa gttagaatta taaatggctg 540
   gttgggtaaa atgtaatact atgggctttc tttagatttt tcagagtatg tgggtaacat 600
   tttggtttat attcttccta aagacagatt gttaggtaat gtgtaaaatc taatttgacc 660
   ttatgttctc acaattaaag gtttatattc tagataacag gtagctgata gctcctgggt 720
   tctcagctgg tgtaattaac ataattatga aagccccaac tttttctttt tttaagttct 780
   tagaggtaga acacagaaca atgagccaaa aaccctgtaa tttataagat tttgaaaaac 840
   aaaggataaa agtttagtca tgttgagtag ctcaatagta ttttgtttaa aagaatgttg 900
   aaattgtgta taggaacagt taaaccctga tgccctttta gttttttatt tggagtaata 960
   ctcttagtaa ctggtctatt ataaatggaa tgagaaaaag tgtaggctgc tgtgtttgca 1020
   tacctgaggg gtctgctatt taggcacata tgtttctatt gaaaacttct atctccagaa 1080
   ttacctaaaa ctagatggga atagtgaagt cactcactgc tttattgcag ttactttagc 1140
   ttcgtgtttc actgttcggg aagtgtctaa aacatggaat tacagcaaag tgtctgcact 1200
   tttcaaagac ctaagggaaa agatggactg atgaaggtgg gtggggtttg ttcatttagt 1260
   ttgcaacaat atagaatagt actgagaacg taattgtctc tggttatata gtgatggctc 1320
   ggaaggtagt gtgcctgtga gaatttggca acataagttt ttttgatcaa gttactgtgc 1380
   cggttaagtg actaaatcta tagtcttatg ctttttcttt ttgtagtctg gtagcatttt 1440
   attaaaactt tcaacctttt aagatttctg caacttagca gatgtgtctt aagatcttga 1500
   aaagcacaag gtttcttaag cagcacatgc cactaactgg tgagtaggtc tttgtcactt 1560
   cattgagtga attgaatctc tggttgggct tgttaggctt acttggaaat taaatttccg 1620
   ttcagacgtt gaaagtgaga gtttgcaagt ttttcagtgg gttaatctga tgtgaaattt 1680
   cttagaactc attttggaat ggattttcac atctgcacta attcttaaat tttttagcac 1740
   tacagggaag atctgttctt tgaaacaggt gtatgagaat ggctcaagtg ggaacatacc 1800
   acaaggcatg tattaccgta aactaatttt caaattaccc ttttttcctt tctatgttcc 1860
   cggtacctgt ggatcgactc attggtgatt gtatcgacga acgttgacta cggaaccttc 1920
   taaaatattt acttaacaca catggacatc aactacttat aatgaactgt taattactgt 1980
   tccaatagcg tactgagcgc tttgggcagg gaggtgcggg gcctgtgggt ggacagggtc 2040
   ctagaggaat ggggcctgga actccagcag gatatggtag agggagagaa gagtacgaag 2100
   gcccaaacaa aaaaccccga ttttagatgt gatatttagg ctttcattcc agtttgtttt 2160
   gtttttttgt ttagatacca atcttttaaa ttcttgcatt ttagtaagaa agctatcttt 2220
   ttatggatgt tagcagttta ttgacctaat atttgtaaat ggtctgtttg ggcaggtaaa 2280
   attatgtaat gcagtgtttg gaacaggaga attttttttt cctttttatt tctttatttt 2340
   ttctttttta ctgtataatg tccctcaagt tatggcagtg taccttgtgc cactgaattt 2400
   ccaaagtgta ccaatttttt tttttttact gtgcttcaaa taaatagaaa aatagttata 2460
   atattaaaaa                                                         2470
<210> 139
   <211> 1992
   <212> DNA
   <213> Homo sapiens
<220>
   <223> nbla20216
<400> 139
   tagttataca aagtattttc ataatattag tctttcttta atctgtgtag aaatacaaaa 60
   ctgtgtgctt cagataagtc tcatttccaa tttgataaca tttatgtgtg tcctataatg 120
   tataatttga gtatgtataa ggagaatcta tgtcctaaca actttgtaga accctcttaa 180
   aataaaatgt aatttgaaat cctcaggttt tagcaattca gttacccaat ttttcttctc 240
   aaaatatgtt tggggctata gcggttttcc taaatttcat tcccatctct ccattagccc 300
   agaagttata tttaacaggt aggactgata ggcaagttct atgaaccttt tttggtgttt 360
   ctgctctttt ggccatgctg tttctatgac tcagtttata tttcttagca tggtttatcc 420
   aaaactaaat gtattaattc attagtagca accaattggg atttcagtct tagcttatcc 480
   atctcctctc tcttttttgg ttgcaatggc aagatttaca gcatttaaac tttcttgcta 540
   ctaaaccctc ctcaccctac tcctcgcttc taaaatgatt cttttggcca atcactttgt 600
   tgtcagtata gttaccatca tagaaaataa ggatttgatt tcagaaagtt tagaaataca 660
   aagctcggct tctaggtatg taaaatttga tgcttcagac catcagcaag atcaatgaat 720
   ttgatacatt gatcatctcc tctgcctggg agcttgggat atatttggtg tgtgctggat 780
   tggggagacc ttctaaacac atttctgtgt tcgtgttttt gaatatacta tttacgttaa 840
   atattttaag cttctagtag tcaagggctt cggtagtgtt atacagactt gtttttaaat 900
   tttatttgca tataatgcaa aaaggaaatg aaagcatttg aacaatgtga acaattgcct 960
   ttactttttt ttctaaaaga aaataataac aatagtagac ttgttcagag agagcatccc 1020
   attcatctgc gctccagtct cctcatctga aaatgagggg gtaggagtag ataacctttg 1080
   aaaaatcttt gagatgaagt tcatcagagg catttggaaa gtcagtatca gttttctgtt 1140
   acaaagaaaa gccctgtccc acaaatttct gatttctcaa tggactgtga aaggttagag 1200
   taaatactgt tttcctgaat tcccaggggt ctagaacagc attaaacgaa atcttccagt 1260
   gtatctgggg cgacattgtt ttcctcgctc tgaaggattt ttttctaggt ggaatgtagt 1320
   aatctccagc tggatgatca ttgactaaat tgtaagccca ttcaacccag agagaaataa 1380
   gcctccagtg cttttggata tagtaattct accttgcatt gtgtgtgtgt gtgtgttttc 1440
   atatgtgcac tcatatttgt gtattcagag tgagtctaac taaaaatgaa acatctttca 1500
   tgaccctaaa taacaccttt aggatcacgc aatctcagct gaggctaaag aatcacaaga 1560
   agcgagaata tgatgtgttt gccaaattaa agtagttgat catgactcaa ctagagaaag 1620
   ataggggaag ggtggtggag atgtggctgc aggcattggc aatgacatat tcttgaaagc 1680
   cttggacact actttaacaa agttgaggtt aggaaagtga aacgtcatta aagagctcat 1740
   caaaacagag atatgattga tttgtttttc tctaaaatga cactgcttga agtatttaaa 1800
   attatctgga aagagggaag actgaaaaga aggagtcacg gtgagtaact gaggtacaag 1860
   gtgatggctt ccaaagttaa tgtcagtgtg gtaggcaagg aggggatgga gtagataaat 1920
   attaaagagc agaatgtatt ggtctggttg gttgaatatg tgtggtggtg gtggtagtag 1980
   gttggccaaa aa 1992
<210> 140
   <211> 1603
   <212> DNA
   <213> Homo sapiens
<220>
   <223> nbla20657
<400> 140
   aagcattctc tctgtgcaga ttgctctgaa aagtcgattt ctgtaatatt tgcgtgtttt 60
   cctctaatgc tggccttttt gcttcccaca gtgttttacg acgacgactt gactgatgct 120
   gtgtttaaaa cgctctcccg actcgcccac agattgaaaa atgcctgcac agccatactg 180
   tcggtggaga agaggtgagc tttgcgccac gggaaccgtg ctgacgtccc gagtgtcagc 240
   ggaactctca cctcctaatt gtgtccttgt tagtgtcatt atgattgtta ctcagtgcca 300
   cttattgagc acctactatg tgccaggtct gtgctcatcc tttgtgtacg ttactgcact 360
   gaatctgcat cctagccctg tgtgcaggcg ctgctgtccc acttgactga tgaagagagg 420
   aaggctggaa agcatcaggg gccttggcca gggcacggct agttagtgat agacaaggac 480
   ttgaatgcag actgtactgg aacctcaact cttggccagc acacactgtc gagagcttct 540
   cttcctgaat gttctctctg tggtgccgtc tgtctcttca gctcccccag gtctctttct 600
   cttgctgaat cggacagctc ctcacccaac agcctcaccc agacatttcc actagaatat 660
   cctgaaatgt taggttccat ttattgagtg cccaccttgt gatagctaca cacattctcc 720
   tgtaatactt aacagtagtc cacagctttt ctgaagatcg tttggaatcc acagcaaaag 780
   ctgtaaaacg aaacagactt cttcacccag caattcagca tctggaaatt cacattcagg 840
   gttgtgtaca aagctgtatg tacttgcata tttattgcag tgttacttat accaataata 900
   ccgagggctt gctttgaggc acacactgag caatagcaat gtacagacct catttggatc 960
   ctgatttcat aaactgtaaa ggaaaaacat caggacagtt gggaaaagtt gaatactgaa 1020
   tatttgatgt taaagggtga ttgttaaact ttagttgaag aggtctccat cttcttgaga 1080
   cacacactga catttccaac ttcacagagg aaatgggttg gtgtctggca tttgcttttt 1140
   aataactcag tgagggcagg gggccccggg aagagccaag gtggcagagt ggctggaagt 1200
   ggacagtggc tgaagctggt aatgggttca ttagacagtt ttgttttttt tgtttttttg 1260
   aggcagagtc ttgccctgtc ggcccaggct ggagtgtgat ggtgcgatct cggctcagca 1320
   caagctccac ctcccgggtt cacaccattc tcctgtctca gcctcccaag tagctgggat 1380
   tacaggcatg cgtcaccaca cctggctaat ttttgtattt ttagtagaaa cggggtttca 1440
   ccatgttgtc caggctggtc tcaaactcct gacctcaggt gatctgccct cctcggcctc 1500
   ccacagtact gagattacag gtgggagcca ccacgcccag catagactgt tcttactcct 1560
   gttgcatgtc tggaattttt cttgataaaa aaatttggaa aaa 1603
<210> 141
   <211> 2235
   <212> DNA
   <213> Homo sapiens
<220>
   <223> nbla20688
<400> 141
   aagtggtgca catgtatttg ttaaataagg tcacccatgc tttgtcttta gattccccag 60
   gcaggattct gtaccttttt aaaaaaatat atttaatttt atttacttat ttatttattt 120
   atttatttac ttatttattt gacagagtct cgctttgcca cccagtctgg agtgcagcgg 180
   tgcaatccca gctcactgca acctccgcct tctgggctca agcagttctc ctgccttagc 240
   ctcctgagtg gctgcgactg caggctcgtg ccaccgcgcc cagctaattt tgtaattttt 300
   gtagggacgg gatttcccca tgttgcccag gacggtctcc agctcctgag ctcaggtgac 360
   ccgcctgcct cggcctccca aagttctggg attactggcc tgagccaccg tccctggcct 420
   tcagtcaggg ttctgtctgt tgactctcca acctcgaaag cagcagcggt attgtttctg 480
   agaaagtttg tttgcattgc ttaggaaccg taacaagcct ctcttcataa ggataggaag 540
   aagcccaagg gcattagtgg gaggcggata agggagccta acttcccagt ttggctatca 600
   ttctttgcaa aatcacttct aatctccaaa gaggaggggg tttctcctct ttcaagttgc 660
   ttagaagggc acccacagat ctgcttattt ctcacagcat ctctctgccc ttgcaatctt 720
   tcctctccac ctcaccatcc acttttagtg caattagtga attcttttct gtttttcaca 780
   caatcccctt ttgtcttatg ttgggaggtt gctgaaatcc ctttagaaac aggtcactgt 840
   tattctgaca ggtggccagc cttaagcctg ccttcatctc catcatttaa gtaaataaat 900
   accgtgacct aggtcttaag tagggagaaa cggaagctgg gaggatttgg gatttgtcaa 960
   ttgcagataa aacacttgct gtgtctcaga ataatgcccc attccccact ctcatccagc 1020
   aaggatgtgc agctttggca gaatcaacat ccagatatta ttttgcttcc tagtctcttt 1080
   tcatgctcta ttcccacttt cctgaaagtt ttaagatgct ttctgtgtaa ttattaaaca 1140
   aaagtgaatt aagatctact tttaaggatt tggccatgag gtgaggcatt tggaaacact 1200
   gctaggtatg gggcaggaac aattgcttgt ggggaaggtt ccaggatggg atggtcctaa 1260
   tgtgtggttt cacggaaggc cccaggacca cttctggatg tcaggttctt agcacaaaac 1320
   atttttgttg ttgtttgctt ctgtgtttgt ttgtttgttt tattttgttt tctcctatct 1380
   tgcattcaat agcaggatgt gtcggccttc tagcatggct cttccagaag tttagagcta 1440
   cttttccctc ccttttctaa gtgtcccctc taccttcctc ctcttacttt gcttttccat 1500
   gggagagaaa aacactgatt cagaaaactc cctaagaagc tccaatcttc cctggtgccc 1560
   cagtaaagtc agcctctgga gatcaggaga ggttcagaga ggatcagtgg tatcaccatg 1620
   gtcacagagc aattcaaaga taatgcccca ctttggcatt tggacattcc attttgagca 1680
   tgaactgatt tttcagcttg acattcagaa ataatcaaag atggagagat cagttttggc 1740
   ctgacatagt gtgattttgt agcacaggac cagctgccaa tctgtgaaga gaaaacaaga 1800
   ttatttgaaa gaaacctcag aatctgaggt ttcccatgaa tgttcccatg aggattcatt 1860
   ttccttttct tcaacccgtc cacctgcaac aattccaata ggcttccaat tcctccttct 1920
   acaagagaga tgggtgctca gtttctacct tttctacctc agaacatgat ggctgtttgt 1980
   catgcgtttt gacatacatg tgtatgtcag gtctggaagc tgttgggtgt tggtaagagc 2040
   ccccaacttt ggaatcagac atgctgggta gccttggatg tgctctttta tttctctcag 2100
   cctcagattc cacacttgta gaaaaggaat cattcccatc tcacagtgga tttgtcagaa 2160
   ttgatacatt aatatcgaca ggaccctggg tggaggattt ttattctgtc aattgtaatt 2220
   tcctaaagag aaaaa                                                  2235
<210> 142
   <211> 1952
   <212> DNA
   <213> Homo sapiens
<220>
   <223> nbla20755
<400> 142
   tttgaaaccc agtgaactgc aggagtatgg ctttggaaaa tcttggaatc taatttgctt 60
   tgtaaaatag ggaatatttc atttgtgtct tcaggcaaga ggttaatagt tgatttcttg 120
   tgatctttgt cagttctgag ctgttgagta gtttagaaat gaagcttaaa ctagacctga 180
   tagcctacta cagtgttaaa atacatatga aaagtcaagc atagagtcta atgaatattc 240
   ctgcctctta caaaggtaga aatgatactg cctatggtat ttttttttgt ttgagtgcaa 300
   atccaattca tgaatttgtg cattttagtt gaccagtgtt taatatttag gaatagttag 360
   tacctaattc atgatgacct cttgttctag catattgaag gccagctatc attaaagcag 420
   tgcttttcac agaatggttt tgctgacctc ctaaatagaa gtgtggatgg cagaagcatc 480
   aaagaggatg atcacaagtg gggaaggcag aaattttaaa agaactgact gaagtaactc 540
   ctctactaat gtgacaccat ctctatcccc cacaaaccct tggaaatact agttttggga 600
   gaagagagga gtatggtgac tagaaagtag ctataacctg ttgatcattg tatactttat 660
   aaggcagtga gtcagaagat atgtttaaga aatggaaggt tgttggagta gctctgatga 720
   cagatgctta tcataaggca aacttaatat atgttccaca gtgttcagaa taccacttgg 780
   tcggtggact tttaaatgtg tgcatactta atttttaata aaccgtagac atggtatatt 840
   taaacatact gtttcattta agactaactt ttaagaaatt tgctatcacg tggttcacat 900
   atgatgtaca agtgtatagt tgcatgagat aaagctggaa gatgacatga aaaatttaat 960
   tgtggtagtc tcagagtaag agtaattggg gagctttaaa ttttaatttt gtctgtgttt 1020
   tcagatttaa gtattaatgt aattgcacaa attacaaatg tttaaaaagt gaagtgaatt 1080
   tatacaatct agaagtggtt tgtttctttc tggaatgagc aaaataaaat tagctatcgc 1140
   ctgcagcatt gggaatctaa gtgttgacat ctaaggtgag tgatataaca atgctgggcg 1200
   cagggtgaaa tggtagataa accaaaatgc taacattttt cttgaaagtg acttgagttt 1260
   catgatagtt ccagaagagg ataacaaatt cccatttcat aacaagtaaa ttaaaatatt 1320
   tccttatgaa cttgcaactt agtggttgca gttacatact aatctctttc ctgctttcat 1380
   ttcctgttag aataccagag taaaagtggt ctgattctag tcacttttga aaagcaaaga 1440
   gttgtaggtt acagctgaat tttgaggctt tacagtaaga gaaacagagt gagtctgaca 1500
   aattttaagc tcatatattt tccttttaga aatgtaggaa ctctgcacaa ataatgtaga 1560
   aacaaattac caatttcaat acaaaaaatt ttgcaggata gtggaatttg taagcttgtc 1620
   ataccttgat tttttgaatt caccttttcc caaaagaaag caactgttgg ccaggcacag 1680
   tggctcatgc ctgtaatcct aacactttgg gaggctgagg tgggcggatc atgaggtcag 1740
   gagatcgaga ccaacctggc caacatggtg aaaccccgtc tctactaaaa atacaaaaat 1800
   tagctgggcc tggtggcaca tgcctgtaat tccagctact tgggaggctg aggcagaaga 1860
   atcgcttgaa ccagggagtc ggaggttgca gtgagccgag atcatgctgc tgtactccag 1920
   cctagcgaca gagcgagact ccgtctcaaa aa                                1952
<210> 143
   <211> 1605
   <212> DNA
   <213> Homo sapiens
<220>
   <223> nbla21013
<400> 143
   aaatccagta ctcggttaca ccagaagact ctgatctttg cccccgaaaa ctgtcctact 60
   ttatccttat acctgaaatc actgcatacc tgaaatcact gcagccctac tgttttaccc 120
   ataccattaa tttaaaaagg catctatttc tttatagaaa gaaacattca cagtgaggtc 180
   ttagtttgtg aacctcaaaa tccagatatt aatccacttt agttattact ttgtaattgc 240
   ttctcagtca ttggctgata atgcaatggt gtgataaatt tgacttatct ccacatacaa 300
   aagtcgatca gaagggatag ttctcttcct ttttttcccc tcctactggc tcttactgtt 360
   ttctaatctc cagtgtaaat ggaatgaaca catctatagt taaggtaaat gccaccaatc 420
   agaagattga gtgatttact gcttgtaaag caactgtctt tgaatcttat gaaataggtg 480
   gtgttgctac cacagaagcc aaaaaggtct taaaattgga aatagatgtc tttattgtac 540
   ttcagccaac agcaagccag gggaaggaac atacataaat atgacaggtc atatatgaaa 600
   tttggctctc ctcctatcaa agtagcctag gagcttggag gaagcctaat taactaaaac 660
   aggaaaaaag catactcatc tgatgtaaaa actcatcagc tgtaaattac caacattaaa 720
   ccagaagtca ttaccagtta aaatgtgtgg ttttcatctt attcttaaat aggagaggtg 780
   gacagtagtg taagtaacat tgctttaaag acataaagct tgtcctggta aacatggtct 840
   aaatgagaaa tgcctccatc ttttcaggta gaaccagatt tcaggcatag ctcagctaca 900
   tctgtatttg aaatacaata aaaatatttc ttatgtctct gtattctctt ttaaaaagaa 960
   ctgctgactg gctcctgtct cttcagtaac actgattttt ttttaaagaa gtgatatgtt 1020
   ggactctgtt gtagaagaat gagcactagt attcagcaac aagtgcaatt tctccatgtt 1080
   atgttgagct ctgttggagc ctatggtgag tatttgatgt gaaaaccttg ctgtgggaat 1140
   tttttattct tccttttccc cccacgccag ttcgttttgg taagtctttt atttgaacac 1200
   aagacgcatg cttttttaaa cctctagttt ttgaagtaac tgtagaagag aatctttaaa 1260
   aaaaaatgga gggcagaatg cttgttagca atctgaaaat caaagctgaa caagctgctt 1320
   aaagtttctg attaagaagt ttaaaaagaa aaattaattg ctactgcttt ccaggtaatt 1380
   gtattattag tttctgtata aaagaaacat tattgctgtt gtataaataa aattttcctg 1440
   tggtacaatt aagtattgat ttttcagaaa ctgtccctat aaatcttttc acatatttcc 1500
   atgtgctgtc caaaacaaaa attattgaaa tgtctaatct gtgagattat atactcctgg 1560
   taaaatattt ttgtatatat aaagaaatat ttactattgg aaaaa 1605
<210> 144
   <211> 1534
   <212> DNA
   <213> Homo sapiens
<220>
   <223> nbla21172
<400> 144
   ctataaatat ttcaatcctt accttcaaat gtatattatt gtgcacttca cggagttaga 60
   gtgagaatgc tatgttcagc agggtgtctt aagttaaaca ttcagactta gaaaaccgtt 120
   agtccacatt tggcatattc acttagaaaa atacaggata ggatgcagca agtagggcag 180
   tgccaggcat tccacaggga tccttgtagc agttcacgca gcaatacaac ttaggtctga 240
   gatgtgagat ccacatcacg caagtgcaca agacacctgg ttttaaaagt tttatgacct 300
   gttacccaca ggcatagctt ctaagcttcc tgagacatat gcctcttatg tcattgcact 360
   taagatgtag ggtctccatt ggatacttta gtttctccca gtgaagacgc aatttaccag 420
   tcaaatcatt tttaccacaa gcaatgttgt aacacagttg acatactagc cttatcaggg 480
   tgccagagaa acaactagaa atttaatgaa aggccaaatt cccacacaga aggggaaagt 540
   tcttattaaa cagtttatag tagtccctac aagatttggg gctgggggcg gggagttcaa 600
   tgaaatagta ccaaaggtca catggaagaa tgtacttaga aatgaataaa caatcaggaa 660
   tagagtccag actagatcca agtacctatg aaaacttaca tgggctgggc gtggtggctc 720
   atgcctgtaa tcccagcact ttgggaggct aacgcaagag gatcacgtga gcccaggagt 780
   tcaagactag cctggacaac atactgagat ccccatctct acaaaaaata aaaaattacc 840
   tgggtttggt ggtgcatacc tgtagtctta gctacttagg aggatgaggt gtaagttgag 900
   cctgggagat ccaggctgca gtgagccatg gttgtgccac tgtactccag cctagctgac 960
   agaatgagac cttgtctcaa aaaaggaaag aaaacataca tacttaaatg ataaaggtag 1020
   cattttattt ttatgggaaa atgacagatc agtaaagaat ggtatatggc tatttggaag 1080
   aaaatagatt tagactcttg cttcatacaa tattacaaca atacaaatta taggtgggtt 1140
   aatatataaa tgtaaaaaaa ctatatgtta tttggcaacc atgataataa tagttgataa 1200
   ggcaagactc tgattggtac taaaactagt acataaaaat ttcaggaata ggccaggcgt 1260
   ggtggctcac acctgtaatc ccagcacttt gggaggccga ggccagtgga tcacctgagg 1320
   tgaggagttc aagaccagcc tagccaacat ggtgaaaccc cgtctctact aaaaataaaa 1380
   aattagccgg gtgtggtggc acacacctat agtcccagct acttgggagg ctgaggcagg 1440
   agaatcgctt gaacctggga ggcggaggtt gcagtgagcc aagatcgtgc cactgcacta 1500
   caacctgggc aagagtgaga ctccgtctca aaaa                            1534
<210> 145
   <211> 3171
   <212> DNA
   <213> Homo sapiens
<220>
   <223> nbla21200
<400> 145
   gacagagtgc aaacaactaa agtataccac gggagaaggg gaaggaagtg ctgcattaga 60
   agtgcaagca aactgcaatg gaagcaaaga agtgatgaaa ttctaaagag aacagtcagg 120
   actgcaaatt cacattgtta caccatgagg aaaacaactg gagcaagaaa catcccagag 180
   aagtaactag ggttagataa aggataatgc catgggctac caagaagcaa caagacgggg 240
   atatttttct tcaagcacgc catgtgagtc acagataata gagtcgggac attgggctca 300
   gccagtgcaa actcactgct caacagaacc tgtctttttt ttttttcctt tttctactat 360
   ttttctttct tgtgttaagg taaactacta ggtactgttt ttaatttagt ttttaattat 420
   gatctaagga tcagtactat ggaaacacac ataattatat aagaaagtat tgcacatata 480
   aagcattatt tattttataa tattaaataa atggcaacaa tctaatgttc aatagtaggg 540
   gaaaatttac aaaactttac tgtctgtact taacaggata ttctccagct actaatgggt 600
   gtttatgcgg aattagaaca ggaaaaaatg cccatatttt aatgttaggt gagaaaactg 660
   ggatgcaaaa tttaccatag agtgtgatca aaagcaaaaa gctagtgcat tttttagcaa 720
   caaaatgtat cagtggctgt ctttgtgtag ggggaaagag gaggctagaa aatagtattt 780
   gttgagtcca accaactaat ttgttcaatg tttctttctg tcgtaaaggt ttttatttgc 840
   attttaatat atgttttgac cagatgtggt ggctcagggc tgtaatccca gcactttggg 900
   aggctgaggc aggtggagta cttgaggtca ggagtttgaa accagcctgg ccaacgtggt 960
   gaaaccccgt ctgtactaaa atacaaaaat tagctaggtg tagtggcgca tgcctgtaat 1020
   cccagctact tgggaggctg aagcatgaga atcgcttgaa cctgggaggt ggaggttgca 1080
   gtgagccaag atcacgccac tgcactccag cctgggccac agagtgagat tccgtctcaa 1140
   aaatatatat aagtaagtaa aataaaaatt taaagatgta tatatgtgta tatgcacaca 1200
   gacacacaca cacacatata tatgttttga tgagcctcta ataaggcact taagggaagt 1260
   ttaatgattt agttatatgg ttattttctt ggaaaaaaaa atcgaggttc ctaatcatta 1320
   agggatatta gttgtcttga agattgacat atgttaagca cacctggaat aacaaacaaa 1380
   tttggctgtt aggtataacc caatgagtaa aagacaagga tgtgcattat gacatagcca 1440
   cagtgatcag ggaggagctg cccatgcaca caaactcaca cattcctgca cacaggcata 1500
   cctcagtaat gaaaccacgt acccctaagg actgagagcc aatccatggg agaggttttt 1560
   aaacgccaaa acacataagg tgggcagaga tccgagactc attttatgta gtatttttca 1620
   atcgcggttg agagcattgg gtagaaggac acttctagat gaagtcgaaa gtggcaacag 1680
   tatatctaga gctgacagct ggtgttgtaa aatcttcctg aaacaatgtt ggcaccgtgg 1740
   ctgtgtttct cttgtcttcc tgtctgtctc tggtccaggt tgccctatgc tcttcccttt 1800
   atttcttatt ctttttcctg gcctcagtcc taggggaagt gaactgtgta cccaggtgtg 1860
   tatctggcat ttctctagca ggtttttaaa taattttatc tatcataatt attttcatca 1920
   ggacagaaat ctttccatat tctttatcaa gatactctat catgaaaatt gtcaaatata 1980
   tgcaaaaaca aagagaatga cccttcatat accattactc agatacactg agtaccaaga 2040
   ttttgtcata ctcagttcat ctgtcgtctc cctctttttt gtcaaagtaa aaatctcaga 2100
   tgtgtcattt cacccttatt tactttaggt tatttctcag aaaaatggag agttctcata 2160
   taacaatgat gctattatca agcctaacaa tattagtatc atctaatacc taacccataa 2220
   tcaaattaac tcaattgtcc caaaacagcc ttttccaagt aggtttgtgt caatcaggat 2280
   cccgacaaag tccacacatt acattggttg ttatatctct tgagtctttt taatctgtct 2340
   ctgcttcctc actctccccc attaacacat tagggaacat gttttgaata atttggaaac 2400
   atagccatcg agtactctta ggaaagagta atggggttga ggatggttaa tttagcccat 2460
   cctaacttct gtgagatttt tttcagaata ttttggatgg ttctctcact tttgttatta 2520
   agcatttggg aagaagattc tgcagcctac tcaggtgagc caatctcatg gcattgaaca 2580
   gagaagatat gttttcacgt ctctaaccag tgtttttcat agtgtaagtc aggcctttct 2640
   cctttgatct aagtggaacc aagaggttag atactccctt ttctttagtt atattatggg 2700
   cttcatgtaa ctccaaattg tatttcttcc tcagctattt atatatattt tttggtggtg 2760
   gttctattgt tttacaaatt taagcaagag gttgaatagc agagtgatta agagcaaaga 2820
   ctgctggagt caaatcttga ctctgggccg ggctcagtgg cttatgcctg taatcccagc 2880
   acacgcctgt aatcccagca cacgcttgta atccagcact ttggggagcc aaggtgggaa 2940
   gattgccaga agccaggtgt ttgagaccag tctgggcaac aaagtgaggc acccatctct 3000
   gttaaaaatt taaaaattag ccaggcacag tgatgtgcac ctatagtccc agctactcca 3060
   gaggctgaga cagggagatc atttgagccc aggagtttga ggctgcagtg agctgtgata 3120
   gcaccactgc actccagcct aggcgacgga gcaagacact gtctctaaaa a          3171
<210> 146
   <211> 2002
   <212> DNA
   <213> Homo sapiens
<220>
   <223> nbla21255
<400> 146
   atgttttggt ggattaaaag tggaacagat tcaagggtat tagctcaatt ctgagctgtt 60
   ttgagtttca ctcagcaaag gtgggtaaga aggaggctac ctcctgagct gtatgttaat 120
   acttcttatc cttatttatc caagttcctg aggtctccaa ttgtcccaga ttaggaaggg 180
   ctgcctgtgt ttttatgtta tttgcagggt ggatgaaaaa actaaaacca aatattttca 240
   tgtgagcagg gattagaggt acctgggatt tagggaaggt gaacgcagta caagtgaaaa 300
   tttttcctta aacttcattg cttctagacc agcctgaagc ccctgtgtat ctgttaattt 360
   agtctggtgc ttttgttgct cctgatttag ggacattaga tgagaagcag taggcctaag 420
   aaaggggagg taggtggcat ccatgtgtgg tctgtagttc aggacaggaa agggaatatg 480
   tttgtgcctg ttgagggtca tcagaaagga gacttcagga gagaatttgg cttttggggc 540
   ctctctctgg agtgagacta ttcttcattg atgatggtca gattgtgggt gtctccccta 600
   ctcccagtgg ctcctgacac tatcaacaat catgtgaaga cttgtcgaga agagcagaag 660
   aatctacact tctttgcacc agagtatgga gaagtcacta atgtgacaac agcagtggac 720
   atctactcct ttggcatgtg tgcactggag atggcagtgc tggagattca gggcaatgga 780
   gagtcctcat atgtgccaca ggaagccatc agcagtgcca tccagcttct agaagaccca 840
   ttacagaggg agttcattca aaagtgcctg cagtctgagc ctgctcgcag accaacagcc 900
   agagaacttc tgttccaccc agcattgttt gaagtgccct cgctcaaact ccttgcggcc 960
   cactgcattg tgggacacca acacatgatc ccagagaacg ctctagagga gatcaccaaa 1020
   aacatggata ctagtgccgt actggctgaa atccctgcag gaccaggaag agaaccagtt 1080
   cagactttgt actctcagtc accagctctg gaattagata aattccttga agatgtcagg 1140
   aatgggatct atcctctgac agcctttggg ctgcctcggc cccagcagcc acagcaggag 1200
   gaggtgacat cacctgtcgt gcccccctct gtcaagactc cgacacctga accagctgag 1260
   gtggagactc gcaaggtggt gctgatgcag tgcaacattg agtcggtgga ggagggagtc 1320
   aaacaccacc tgacacttct gctgaagttg gaggacaaac tgaaccggca cctgagctgt 1380
   gacctgatgc caaatgagaa tatccccgag ttggcggctg agctggtgca gctgggcttc 1440
   attagtgagg ctgaccagag ccggttgact tctctgctag aagagacctt gaacaagttc 1500
   aattttgcca ggaacagtac cctcaactca gccgctgtca ccgtctcctc ttagagctca 1560
   ctcgggccag gccctgatct gcgctgtggc tgtccctgga cgtgctgcag ccctcctgtc 1620
   ccttcccccc agtcagtatt accctgtgaa gccccttccc tcctttatta ttcaggaggg 1680
   ctgggggggc tccctggttc tgagcatcat cctttcccct cccctctctt cctcccctct 1740
   gcactttgtt tacttgtttt gcacagacgt gggcctgggc cttctcagca gccgccttct 1800
   agttgggggc tagtcgctga tctgccggct cccgcccagc ctgtgtggaa aggaggccca 1860
   cgggcactag gggagccgaa ttctacaatc ccgctggggc ggccggggcg ggagagaaag 1920
   gtggtgctgc agtggtggcc ctggggggcc attcgattcg cctcagttgc tgctgtaata 1980
   aaagtctact ttttgctaaa aa 2002
<210> 147
   <211> 3112
   <212> DNA
   <213> Homo sapiens
<220>
   <223> nbla21345
<400> 147
   agatttttag caaatacccc ggctcgcact acccggagat cgtgcgctcg ccgtgcaaac 60
   cccctctaaa ctatgaaact gccccgctcc agggaaacta cgtcgccttc ccctcggacc 120
   ctgcttattt tcggagcctg ctgtgcagca aacacccggc ggccgccgcg ggggccactt 180
   gcctggagag gtttcatctg gtcaacggct tctgcccgcc tccgcaccac caccaccacc 240
   accaccatca ccaccaccac caccaccacc gggcccagcc gccgcagcag agtcaccacc 300
   cccctcacca ccaccggccg cagccccatc tgggcagctt tcccgagagc tgcagcagcg 360
   actccgagtc cagctcctac tcggaccacg cggccaacga ctcggatttt ggctccagtt 420
   tgtccagctc cagcaattct gtgtcctcag aggaagagga ggaggaggga gaggaggagg 480
   aggaggaaga ggaggaggag gaggaggggg gcagcggggc ctcggattcc agtgaagtca 540
   gctcggagga ggaggactcg tccaccgagt cggactccag ctccggctcc agccaagtgt 600
   cagtgcagag catccgattc aggcgcacca gcttctgcaa gcctcccagc gtgcaggcgc 660
   aggccaactt cttgtaccat ctggcctccg ccgccgctgc aaccaaaccc gctgctttcg 720
   aggatgccgg cagacttccc gacctcaaga gtagtgtcaa agcggagtcg ccggcggagt 780
   ggaatctgca gagctgggcc cccaaagcat ctccggtgta ctgcccggcc agcctgggga 840
   gttgtttcgc tgagataagg aacgataggg tatctgagat tacattccca cactctgaaa 900
   tttccaatgc tgtaaagaga aaggcggtag tggcggaaga ggttcggcgg ctgatggcgg 960
   atcaggatcg gaagcctgcg taactttctc ccttgatccg ggagtctttc cactggattc 1020
   acaatgacat cctttcaaga agtcccattg cagacttcca actttgccca tgtcatcttt 1080
   caaaatgtgg ccaagagtta ccttcctaat gcacacctgg aatgtcatta caccttaact 1140
   ccatatattc atccacatcc aaaagattgg gttggtatat tcaaggttgg atggagtact 1200
   gctcgtgatt attacacgtt tttatggtcc cctatgcctg aacattatgt ggaaggatca 1260
   acagtcaatt gtgtactagc attccaagga tattaccttc caaatgatga tggagaattt 1320
   tatcagttct gttacgttac ccataagggt gaaattcgtg gagcaagtac acctttccag 1380
   tttcgagctt cttctccagt tgaagagctg cttactatgg aagatgaagg aaattctgac 1440
   atgttagtgg tgaccacaaa agcaggcctt cttgagttga aaattgagaa aaccatgaaa 1500
   gaaaaagaag aactgttaaa gttaattgcc gttctggaaa aagaaacagc acaacttcga 1560
   gaacaagttg ggagaatgga aagagaactt aaccatgaga aagaaagatg tgaccaactg 1620
   caagcagaac aaaagggtct tactgaagta acacaaagct taaaaatgga aaatgaagag 1680
   tttaagaaga ggttcagtga tgctacatcc aaagcccatc agcttgagga agatattgtg 1740
   tcagtaacac ataaagcaat tgaaaaagaa accgaattag acagtttaaa ggacaaactc 1800
   aagaaggcac aacatgaaag agaacaactt gaatgtcagt tgaagacaga gaaggatgaa 1860
   aaggaacttt ataaggtaca tttgaagaat acagaaatag aaaataccaa gcttatgtca 1920
   gaggtccaga ctttaaaaaa tttagatggg aacaaagaaa gcgtgattac tcatttcaaa 1980
   gaagagattg gcaggctgca gttatgtttg gctgaaaagg aaaatctgca aagaactttc 2040
   ctgcttacaa cctcaagtaa agaagatact tgttttttaa aggagcaact tcgtaaagca 2100
   gaggaacagg ttcaggcaac tcggcaagaa gttgtctttc tggctaaaga actcagtgat 2160
   gctgtcaacg tacgagacag aacgatggca gacctgcata ctgcacgctt ggaaaacgag 2220
   aaagtgaaaa agcagttagc tgatgcagtg gcagaactta aactaaatgc tatgaaaaaa 2280
   gatcaggaca agactgatac actggaacac gaactaagaa gagaagttga agatctgaaa 2340
   ctccgtcttc agatggctgc agaccattat aaagaaaaat ttaaggaatg ccaaaggctc 2400
   caaaaacaaa taaacaaact ttcagatcaa tcagctaata ataataatgt cttcacaaag 2460
   aaaacgggga atcagcagaa agtgaatgat gcttcagtaa acacagaccc agccacttct 2520
   gcctctactg tagatgtaaa gccatcacct tctgcagcag aggcagattt tgacatagta 2580
   acaaaggggc aagtctgtga aatgaccaaa gaaattgctg acaaaacaga aaagtataat 2640
   aaatgtaaac aactcttgca ggatgagaaa gcaaaatgca ataaatatgc tgatgaactt 2700
   gcaaaaatgg agctgaaatg gaaagaacaa gtgaaaattg ctgaaaatgt aaaacttgaa 2760
   ctagctgaag tacaggacaa ttataaagaa gatgagaatg tgcctactgc tcctgatcct 2820
   ccaagtcaac atttacgtgg gcatgggaca ggcttttgct ttgattccag ctttgatgtt 2880
   cacaagaagt gtcccctctg tgagttaatg tttcctccta actatgatca gagcaaattt 2940
   gaagaacatg ttgaaagtca ctggaaggtg tgcccgatgt gcagcgagca gttccctcct 3000
   gactatgacc agcaggtgtt tgaaaggcat gtgcagaccc attttgatca gaatgttcta 3060
   aattttgact agttactttt tattatgagt taatatagtt tagcagtaaa aa 3112
<210> 148
   <211> 1921
   <212> DNA
   <213> Homo sapiens
<220>
   <223> nbla21410
<400> 148
   atacattttt tttttcttta agaaaaggtt agctttttat cttgcaggct tttcaccctg 60
   gttttgataa tggtcttcat tccttaaaat aagtatccct aaacaccaaa gggaaggaaa 120
   taattattga gagtttttag agaccatttt tcatttttaa aaatgatatc agagtattga 180
   gaatagctag ttttcttaga tgctgtttag aagatagaga tggagaagaa tattattcca 240
   agcatacatt aatgtcacca catttagttt ctttaaatgc ctttgtttaa acttctgatg 300
   tttgatttaa aaatactttg aaactgctgg atgacatata aataacattt cttaatcatt 360
   acatattctc aaaaattccc caaattagcc aactacatta gagtgatttt tgataagaac 420
   atctgaggcc aggcgcattg gctcattcct gtaatcctag cactttggga ggccgagatg 480
   gtgtatcgct tgagctcaag agtttgagac cagcctgggc aacatggtga aaccccatct 540
   ctacaaaata taaaaaaatt agacatagtg gcttgtacct gtagtcccag ctacttggga 600
   ggctgaggca gcccagctac ttgagctcag gaggtgaagg ttgcagtgtg agattgtgcc 660
   cctgcacttc agccaaaaaa aaaaacatct gtagtgagca gccaaatgta ctataaaatt 720
   tggtatttta tcctacatga tttttctgtc attgaaaaat agtattttgc agtaggatgt 780
   tcagtgacta cttattaaat gtatagaaga taacatagct aaggaagaaa actaccattt 840
   ttggcaggga gaagtggaat ttaatagaaa tcattgattt tcatgttaat agtatatact 900
   tatgaattat accaagaatt gacctattta gagatacttg gttgaaatac tcaggattta 960
   atgtgtagat aagttcttta taatgtgagt tatttttagt cttggtggtt ttgttttgtt 1020
   ttcagttttt atttattttg atttgggaat gggagctggg gacatcaaag ccatatagtt 1080
   tagaaaattt cacattactg aaataatctg tatccacaat agtaagcatt tcttcttttc 1140
   ttgctgtaat ttcatgctcc acctacaata tggcttttac tattttttta ttttttattt 1200
   tttttaccca aggaataaat tatcctgaca gtctttaatt ttgggtatgg attagttaaa 1260
   tgtaaggatt gttgatttga tttagtaatg tgagacacaa tgtttatgtc ctcattatct 1320
   acagtagatg gatagttttt tctcctggtc tctaagaata gtatttctta atgtgtggcc 1380
   catgattggc attaggcgtt tttgcttgac cacttgttaa acatgatttt tttctaggta 1440
   gtgtttgcca tttgaatgtc tttgtggaaa cagactcctt aatagcttag ctataatttt 1500
   ctaagttaac atctttacct gccttgtttt tttaattctc ctaatcttac taatacctta 1560
   gcattagttt tgcttccatt atcagtgctt ccaacttctt gttttatgtg ctttaaaatg 1620
   attatatatg ggctgagcat ggtggctcac tcctgtaatc ccagcacttt gggaggctga 1680
   ggtgggtgga tcacttgagg ccaggagttc cagactagtc tagccaacat ggggaaaccc 1740
   tgtctctaca aagaatacaa aaaacattag ccaggcatgg tggtgcatgc ctgtagtccc 1800
   agctacttgg gaggctgagg caggagaatc gcctgaaccc agaaggcaga ggttgcagtg 1860
   agccgagatc gcgctactgc acttccagcc tgggcgacag agtgagactc cctctcaaaa 1920
   a                                                                 1921
<210> 149
   <211> 2099
   <212> DNA
   <213> Homo sapiens
<220>
   <223> nbla21522
<400> 149
   tttaaattca gcttgtgact ttgcacttca ggattctgag tgttctctgt cttcttcctg 60
   cattgttttt cttataccat acaggttttt cattggcctt gactttttgt tgttaactca 120
   ttcttctttg ggtttatttt catttgtttc tgctgaatat tatttgtttc aaactaaaaa 180
   taacattcca cattttaatt gatgtgcgga ctcttaatct acttaaaatg tgggctgaag 240
   ttccatgatt ccagctagtc tggaataggt catttaactg gatgttaatt cacctacatt 300
   gttccctaag tgacatgtgg gtcccattct gctgacatat ttgtgggtcc tggtaacaac 360
   catttgggta gatttgctga ttcctctttt ctccttagtg gaagagaaag ccaataccca 420
   cctcctcttg ggcatgtgct tagacgcctg tgctcgctac cttctgttct ccaagcagcc 480
   gtcacaggca caaaggatgt atgaaaaagc tctgcagatt tctgaagaaa tacaaggagt 540
   gacctggcta ctaccctgga tgcacagggc cgctttgatg aggcctatat ttatatgcaa 600
   agggcatcag atctggcaag acagataaat catcctgagc tacacatggt actcagtaat 660
   ctagctgcag ttttgatgca cagagaacga tatacacaag caaaagagat ctaccaggaa 720
   gcactgaagc aagcaaagct gaaaaaagat gaaatttctg tacaacacat cagggaagag 780
   ttggctgagc tgtcaaagaa aagtagacct ttgacaaatt ctgtcaagct ctaaatccat 840
   ttttgtgtag ggagaataat gtctagtaat gtggaagaat agctatcatt cctgtctctg 900
   tggcacccga tcaatggctt aaatctgtcg tttttgatat tcaggtttcc tcaatttagc 960
   cttagtgaag gaggggttgt acacactgcc atttttgtat tttaaaggaa aaatgacttt 1020
   cattcccaac tgattatgac ctttcaggat gtcgtcaagt gatgctttca gttgtaacac 1080
   gtgacttggt gctgtccctg ctggtctaag tagaactgta gattcatatg ggctggtgtt 1140
   cctgtgcgct gtgggtgtgg tgattcagcc tggcatttct accataagtt tttggtctgc 1200
   tgatttgctg ccctgtcttc tcttacttta ctttatcaat acctggcaaa ctgaccagaa 1260
   ttaccttcct catggcaaag gggattatgg gtgaattgtt gttcttatag tctgtttcat 1320
   gaagcacaag tggaatttaa tacataaaag agaaaaatat cttagtttgc taccagcatc 1380
   cagcatgaag ttgtaaagtg gggattaggc acgtgacagt atagcaccca tttgaattta 1440
   aataaaagtg aaccatattt atctggttat ataaaactaa aaatgggggt gtttatataa 1500
   aactaaaaac taagaatgat gtaacctttt gtctgtgtta tctgaacact ctacttcctt 1560
   tgcagcctta gtcacacaac tgagtcatct caagtactct ttaaggacac acagcccagg 1620
   ctgttctgag tcagaatagg cccctacagg tatattttaa aactcttcgt aattctaatg 1680
   tgtactgctg gtatagctga actactgacc tggatcttag tcctagcctt tttgcttttg 1740
   caatttcagt atcttcatct ctaaactagg gaaacactgg gattctttct tagctgtggg 1800
   ggaaggtatt tggttagatg actttgaatg aatagactgc tgtgctgaaa gagctttatc 1860
   acactgtctc aaagtatgta aagatacata ggtggatgct cttactgcag cagtcatgaa 1920
   tacattttta gccatttacc taaggaaaaa gacagttttt ctaggtacca tgaaggaaga 1980
   ttgaccctgt tggtatgcct gtgggggtgg gatgtgagtg ggactgataa actgatactt 2040
   ttggttcgta tgtacatact ggaagaatct tcataataaa tgagactaca caacaaaaa 2099
<210> 150
   <211> 2471
   <212> DNA
   <213> Homo sapiens
<220>
   <223> nbla21631
<400> 150
   gaacggccct gcggggctgg ccggacggct gcaagaacat gctgagccca aagatcaggc 60
   aggccaggag gggtaagtcc aactttctgg ggtttctctg ggcaccgcat gtgcctcttg 120
   gcaaacgtgc gcggacactt ggccagccgt cacgccatgt gtcacatggg gcgggcgttc 180
   tgggaccatt gccgctgaat agtgagcatc ctcctgagga agtgcccttc ctcgtgaaac 240
   tcctgggctg ggtggggaca cgacctgaag ttgcaaaagg gcggtggccg gcttagtgcc 300
   ccagtgggtg tgcacacttc gccccacatt ccacatttta cagaggccct cggtcgctcc 360
   aggtgacctg gtggcaactt taaggaaact ttgcttcttt actaaaaagg aaatgcccaa 420
   gatttgccct gtggccaaca cagaagcacc ccttaccagg gaaggccatg ccctggcttc 480
   tagagacagc tgggtgcaag cgagggtctt cgttcccgct gctttgcaga cagtatttcc 540
   tcaagcaggc caggggcagg caggctttcc tgccagaaca ctcagaaagc tgcagggtct 600
   gggggcagga cgggtggatg cgggagcaga ctcagaccag caagagatgg gggtcaggag 660
   agtccaggac tgggctagcc agcctgtgtc cagccagcga cccagcacag tgacctgaag 720
   acttggccac tgtatggggc tagagacagc atctccatgg acaacaactt cttagccacg 780
   gaaagtgtca ttttgaatga gaacatctgt cttttacaaa aatagaatgt gtcttttcag 840
   gtggccagta tctgggaggg ctgagctcct tttgtaaaca atgaagtgga ggatgggtct 900
   ttggaggtgg atggagcatt tgcctgggag cttggaaaca gtttgtgtct caccaggtgt 960
   ttgcagcggg gggcctccag cctcctgtgg attcacaggg aacacaccca tcttattagc 1020
   acactgcaag cacttggatg attttcctgg atgggaccag ccttccagtg tgttccacag 1080
   acgtcaggac ccctctgtgg ggtgctttcg catgggctga accctgtgta ccccaatggg 1140
   caaaggagga acttgcatgg ctctgctgag gagggggcaa gtctagtgat gacccaaggg 1200
   ataggacaag ccagatacct ctgcgagagc ttagttccac cctcccactc ctgtgtaatg 1260
   agctggccac tggccacatg tggctactaa gcacttggca tgtcactagt ccaaattgag 1320
   aaaaagacac accaaatttt gatgatttag tacaaaaaaa gaatgtacaa tatctcaata 1380
   attattttac tgaaatgaca gtattttgga tatattgggt taaataaaat ctattattaa 1440
   aactaatttt acctgtttta cattctttta ctatagctac tagaacattt acaagtacat 1500
   atgtgactca cattatattt tttatatttc tattggacag tgctagtaag agaccagtgc 1560
   ttcagcaaag gggcttacag gcagcctgtc tttgaaatcc aggatttctc ataaatgttt 1620
   gttttaagtc aatggttctc aaccaggagc aatttgcccc actagagaac atttggcatt 1680
   gtttggagta tttttggtta ttccaactga ggggtgctac tggcatctag tgcgtatagg 1740
   ccagccatac agccctcttc cagtcctcag tgttccatga ggcttccacc atagggcttt 1800
   tgcacatcgt tctttcccct gaaatgcctc ccacattcac atgtgcgcac atgcatgcct 1860
   gtatgtgtgt gcacatgcgt tcatgcatgc aaacatacac acacacctta attcctattc 1920
   accctccagt tatagtatag ttcaagtgtt gccagccagg gaagtcttcc cacacacccc 1980
   agtccaggct ggatcctctg ctccatctct ccttttcttt atggtattta ccataggttg 2040
   cagttgtata ctcctgagtg tgactgattg gtgaatatct gtcacttgca ttgctccatg 2100
   agcttcgtga aagcaggaac catttctgtt cggggacatc attatacccc caatgccagg 2160
   tacctggtgg acactcaacc tgtgtttttt gagtgagtgg atgaatagct ggatagagga 2220
   gaaagcattt gcctgggtgg ctggagcact gtctctaccc aagctggccc ggtacttagg 2280
   aaatttggcc tcatctttca ctgactcata tgttgcaaat attttcccaa tttgttgctt 2340
   gccctttatt tttatattag agtgggtttt ttttctagat ttacctgttt ttaactcgta 2400
   tatattttcc ttttagaatt tctgtctttg tttgcaatat ttcaaaataa aattgttgat 2460
   gctattaaaa a                                                      2471
<210> 151
   <211> 2669
   <212> DNA
   <213> Homo sapiens
<220>
   <223> nbla21788
<400> 151
   aaagcaaact cctacctacc ccggcctcgt ggagccttcc tgcggtcctg ccatttgccc 60
   catcctgtag acagggctgc aggaagcagc ccagccagca accagtgtgg agggagaggg 120
   agtccaaggc ccaggccggc ccctccccat ctggggctgc cctgcaaccc tcagtggtaa 180
   cttaggacag ctcctatttc ccctttggct aaaagggtct acaccagtgt gtcaccactc 240
   ccaaacagtc cccttcctgg gcctttgcca ctttgttgaa tgaagacctc acctgcagtt 300
   aagcaaaata ttaacatgtg agatgccttt caagatgcaa aaggatattt tccttctaaa 360
   atcacatggg caggaaggct ctgaagatgt tagagcccca gtggactgga gaaagccagg 420
   aagaaagcag tgtgggtcct gcagtagccc ctgccgcttc tcctgcctcc tgctctccca 480
   ggacgcccgg ggccgacccg ggccgaccct tgtcatgctc cttccgttcg cctgggcctc 540
   catgctttca gctaccttct gcatcttcag gtggagccca gtgacagata ctcgcaggaa 600
   ggagaaagca ttcaaatggc ttaggttgat ggaaagtgac actgattaca gccaccatgg 660
   tagatgcttc acgtgtacct taccaaggaa gggcacccag ccacgatcat aggcgactct 720
   acaaacccag ccccttactg aactccaata ggccaggctg gcttcttcca gagtcaggct 780
   ggcccttggc acagtgcccg tgctatgtat ccagaggcct gggcccacat cctgaccctg 840
   tttctccctt attggaggcc ctggcatttc cgaacccact cacctctaag aattggattc 900
   tgtacagtta aaggaacagt gtcccttccc cgagaggtgg agaaaaggtg gccaggaggg 960
   agagggtcct gggaggagca tttatgcgcg atgctgagag atgggattct acggagggag 1020
   gcagcattgg ctctcagctc agcaggggct gtgccccagc ccaggacggg tgtcctgctc 1080
   ctgctgtctg gcaggcgtct gcccgcaccc ccacactttg cttttgtctt cagtacaccc 1140
   tgcctgcccc agcaggaaga gccgaggaag acgactgggg ttggtcagat ggggcctgag 1200
   cagtcccttt gccatgctct agtaccatgg ccttggataa gtccagtctg ctctccaagc 1260
   ctcagtttct ttctgtgtaa tgtgagcagc tcctatctga aaggtttatt gggcggattt 1320
   ttgcaggtca tgggtgtgaa gcccctagca cagtgctgga ctgtggtcag aactcagtat 1380
   cactggcccg catcttcact gtgagcccag gacaggccac acgtcacacg tcacctccca 1440
   caaagcccgg cagagggtgc ccagggaact cttgttatgc ccagagctca gtgacccagg 1500
   ggagcacttc ttgctgtccc cttcccttga gttctccaaa gcaggccatg gccatgatca 1560
   caggctgagg agccaggccg ccaggggcca tcctggctct gcctcttcca tgggagcact 1620
   ttttcctctg caaagcgggg agcagtcgga cacctgccgg cgatatgaag tctgagcgag 1680
   tcaggacagg gggaggccca gatcccaggc gaagatcagt gctctgtccc gccttggttg 1740
   ctgggagccc tcctgtcccc tcttcctcag ggactggacc caaaccaggc caggccggaa 1800
   gactagttgt gtgtttcaga tgtcacttgg agttgtgaag cttttatcaa agctgagaca 1860
   atccctgtta actaaaatcc ctaggacaat gaactgttgt cctttattca cttcctaatt 1920
   atagaagtgt cctgccatgt agtaagtact cagtaaatgt tagcatggta gcagataaag 1980
   tagaaaatct cttttccccc atgaccctcc ttgtgaagag gtttctaaaa gccagtggtc 2040
   ccttctccct gagtaaagag ggtgtggtaa cttccagaaa cgtttcttgc cctttgagga 2100
   tatgtggcac tgagtagtca ccacacaagc tcatcccccg gtgcggagat atggctactt 2160
   caggaattgg gaggaccccg cgctgcgccc ggaatgtgct ctggcaatgg tttgcctttc 2220
   ttttctgtca tttcctttat ttttgtgtgt ttccattcat ctcttgttcc tcaaagctgc 2280
   acacagcccg cccttctgct ggccaaggtc tggttagcaa agggcctgtc tccggcggat 2340
   ctggctttcc tcgctgtcag ccttcaggtg ccctgaaagc tggcgaaggt ttctgagtca 2400
   atgctggggt tgagtgggag tttagaacat cactgcggtg ccgcagtcac tcttggacgt 2460
   ccacgtcctc ttggaagttt gaggcaggct cagctcagcc gttcgctttg gtatcctcat 2520
   aatcaggtag aaagtctggg ccggggccag gggcagtggt gcacgcctgt aatcccagca 2580
   cttttgggag gtcgaggcag gaggattact tgagcttagg ggttcgggac cagcttgggc 2640
   agcatggtga gaccccatct ctacaaaaa 2669
<210> 152
   <211> 1969
   <212> DNA
   <213> Homo sapiens
<220>
   <223> nbla21897
<400> 152
   gagatttgca aaggcatttt aaagaaacgg tgcctagagg ctgggcgcag tgtctcacgc 60
   ctgtaatccc agcactttgg gaggccgagg cgggcggatc acaacgtcag gagatcgaga 120
   ccatcctggc taacacagtg aaaccccatc tctactaaaa atacaaaaat tagccgggcg 180
   tgatggcagg tgccttgaga agtctgaggc ctccttgaga atgccttaag gaaaatacgg 240
   tcagaagggg gttgtcaaca gtgaagttgg gaaaacagcc ttctggaggt gtggctcgga 300
   ggcagagcat cgggctgtgc tggtcagatg ccattccccg ttggcgctgt ggaccagctt 360
   taccagtggg gatgccgtgc tttccaagag caagccctta cgaaggtgga ggtgggcagg 420
   tagggaggag ggaagattta ggaaggaaga ggagcttcaa gaaggcagcc tttgtcttct 480
   aaccagagcc actgagactc taggccatcc tctgctgtgc cccatggtgg ctattttggg 540
   tacttaccac ttcctgtccc cctcctggca tctcacaggt attcaggcag ctttgcatcc 600
   tgggcttccg ttattcctgc tgttgatacc acccacgctc actggtgtca gcagccaccg 660
   ttgtacttgc tcatacgcta gtgggttaga aatggggagc atctgccgag ggatctgtct 720
   tgtggcctga cctgggcgtt gatggctgtg gtcccccagg gcttcgtggg tgtcccatct 780
   gagaaggctg gaagttggcc aggggcttca tgggggtcct gcagggacag tcccaaggtg 840
   acagctgctg cacctcgagt gcggcctgaa ctggagaggc acctgcacct ctgacatggc 900
   tttggatgct gcacagcatc gtcacacctg ctgtgttctg ttggttccag gccagtcgcc 960
   agagctcgtg cagatttggt gggggcctcc ctctcaatgg caggtgtcca aagaacctgt 1020
   ggacatggtc atagccaccc cagacgttca ctcccttcca atccactggt agtttccgca 1080
   gccttccccc atctgaatgt actgaagaac tgacacccac catctggttt taaaatgttt 1140
   agaatttgta ataatttacg tattttctag agagtgatgt aacatccata aaaacacaga 1200
   ttttctagga agttactgtg aaatctacaa aagcaataaa acatttcctc ccaggtgctg 1260
   agctgtgagg agagcatcag ggtttgggct ctgctgcctt tccccgaaga actcactcgg 1320
   caagccgtca gaagataatt ctgaaacaaa tgcctgccac tctttgatta caaaaatgac 1380
   ggatgagctg tatcaccata tgcctgagaa tcgttgtgtg ttaaaggact tggatcgtct 1440
   tcctactgag acgtggcccc agcttctccg tgagctctgc agcacacctg ttcccaccct 1500
   gttctgcccc aggattgtgc tggaagtgct ggttgtgctc cgaagcatca gcgaacagtg 1560
   ccgccgtgtg tccagccagg tcaccgttgc ctcagagctg agacacaggc agtgggtgga 1620
   aaggacgctg cggtctcgcc agcggcagaa ctacctgcgt atgtggatat cagactactg 1680
   tcccctgtgc tcagcctgat actgttactc attgcgctgg agttggtcaa cattcatgct 1740
   gtttgtggga agaatgcgca tgagtatcag cagtacctaa agtttgtaaa gtcgatcttg 1800
   cagtacacgg agaacctggt ggcttacacc agttacgaaa agaacaagtg gaatgaaact 1860
   atcaatctta cacatacagc tttgttgaaa atgtggactt ttagtgagaa gaaacaaatg 1920
   ttaatacatt tagccaagaa atccacaagt aaagtactct tatgaaaaa 1969
<210> 153
   <211> 2573
   <212> DNA
   <213> Homo sapiens
<220>
   <223> nbla22116
<400> 153
   gatatgctgc ttagtttcac taaaagcaga ccctatacct agagaagtca ctggcttttt 60
   attggtcatt ctcaatacag aaatacttag gggagtctta accctgccat ccccggttga 120
   atctcttggt ctttatctaa gctacttgca gttaatattc agttaagcaa aggtatggcc 180
   agtagtgcaa gtatctccca gtctctgagc tctgaacaag aggactgaaa ttcagcattt 240
   gtaaactgac agtttgatgg gcctgggatt tgaagtgaac tcagcacaca attctgaacg 300
   tgtatttgca tgtggactgg gaaggaaata aatgggaact tggaaataat ggaatatttc 360
   tcctatgaaa gaatttttcg tagaagattt gtttttgata taatctttct gttggttagc 420
   ttttagtgtt ttcattcctt ttctgatcca cactccttta agtgaccaaa tgaatataac 480
   ccaacatgca ttgggaatgt gtttaatatt aaacaatgtc taactgaatc tgcaaatgcg 540
   ggaactgaga tatcacctcc atgtgcacac ctgtgtgtac gagtattcta tacaacttgt 600
   agcatttact gccacttaat tgggttgaac ttgcaagata aacttttgga aactgcttag 660
   tgccatcgga gtctccttta gaagctgcca tcaggcaaat gctatcccat aataccagca 720
   gtaagcctgg caacatgttc aacagattta gtacccaaga ggaaatcaac agcgatagta 780
   gagaatgagt cagatgtagt gggataaata ctagcctagg aagaaggagc cccggagtct 840
   aatatgagct ttattactaa attgctatgt gacgctaggc aagtcactta acctctccat 900
   ggctgtttcc tcatctgtaa aataagtgta ttggactaga tgatccttag ggtctttcca 960
   aaagtctaac attctatggc attataggtt gccttgcaaa ttcagcctgc tatagtgatg 1020
   gcaaatatca cgtttaagtc tgagtctctt atgttgcagt taaataaaag aactatgtaa 1080
   gatgattttt aaaattcaag caaatgggcc gggtgcggtg gctcatacct gtaatcccag 1140
   cactttggga ggccaaggca ggcggatcac ctgaggtcag gagttcgaga ccagcctgac 1200
   caacatagag aaaccccatc tctactaaaa atacaaaatt agccgggtgt ggtggcgggc 1260
   gcctgtaatc ccagctactt gggaggctga ggtgggagaa tcgcttgaac ccaggaggcg 1320
   gaggttgtgg tgagctgaga tcatgccatt gcactccagc ctcggcaaca agagtgaaac 1380
   ttcgtctcca aaaaaaaaaa ctcaagcaaa tgaagttcat aataataggg gatgttgata 1440
   aaacttgtgg cagccttcca attcatttac agttgtttcg ttttgttttt gttttaatgt 1500
   ccattttctg ttgactgttc ccagttttca ttttccatac agtctgtatg taaagtctgg 1560
   ttttcattaa gctgtggcca gtatttgcca ctacaacaga aacacactgt cacacttgct 1620
   agaatataac tgtacttggg cttctccttt cctgtgaagt agtgctgggc tttctagagt 1680
   ttaattctca agtggcacaa gatagcagag cccatgcatt ttaatggctg agactgctaa 1740
   gagtgaacct aaacacttac aagttgcaga gagaaatgaa aaagtaatta catgctatta 1800
   gcattgagaa atgttgacaa attaatttgt tgggaaccaa agatagcatt tctgatgaca 1860
   actcccacag tgattggcca gttgtatgat gagtacactg ctggaaagag ggtaaactgg 1920
   gagttagtgg atggtcccaa tgccctgcct acagcagagt gccaaccagc cctgagtgca 1980
   aaattcaagt tcaatgtgtg tgcttgtgtg tggtgtgctt tatggacccg caaataccat 2040
   attcattatt gatgataaga tcttcacaga atcctgtagc tactaatgca ttgagttttt 2100
   aatctcagta catcagccag gaggagccag atcacagggt agtgatgtct actgggatta 2160
   tactcataac atctacacaa aacaagttga gaaggatcca cgttttcatt gtttatcaga 2220
   attgtatctc atttggctga gcattacttt tgtcagaatg tgttatctgt aaaccatgtg 2280
   tagtgaaatt cttctgtaac tttggattaa aggtatttat ggtctttttg tttgtttgat 2340
   ttttaagtaa gttatttctt ttgtagacct gctgatggta tggttccatc cttctgacct 2400
   cagcatccaa tctttttaag gatttttgtt ttcaatattg ttattttaaa ttgtggttga 2460
   agcaatagaa aattgaaata tggattgtgc atgactgtgt cttgagtgta aaaatattgc 2520
   agtttgaaac ttggacctaa agtattgcaa ataaaaatga caaacatcaa aaa 2573
<210> 154
   <211> 3324
   <212> DNA
   <213> Homo sapiens
<220>
   <223> nbla22223
<400> 154
   caaacacagg ctgaaaaccc atgctgctgt tatacacaat ggcagtatta acaagcattt 60
   taaacctttg cacatgatat tgaacctgtt cagtttacaa tgacaatatt aatactgttt 120
   atagctagaa gtttgatttc tgaattcttt gagattttag caaaacagtt tattatacac 180
   tgtacatttt tttcacagca attggaaaaa aacaaccact tgcaatcatt caataaccct 240
   gaagaatttg gttcctgagt gtacaaactc agagcccgga agccaagaag ggtccttggc 300
   ctgcacggtc tgtagttgac tccaagtctc tgtgagcagt gacttgaacc aaacacacca 360
   ggaataatcc attctttggg gcctctttcc aactcgaggt tgttttcttt caagatactc 420
   taatcagcca tagaatttag tgtaaatatt tttttttcca aatagatatc atattcaaaa 480
   aaggcagcat tcaaattata tagaatctag tttttaaaat cagcacagat cttcttaaaa 540
   actgtgaact atgttttgaa atactcgtta ctaaagctgt ttataaacca caggtgccat 600
   aagatcccca aacggactaa agttatctct gctcttccat ggtcttgttc ctctcgtttt 660
   ggctttagga agcatgtctt taacagcacc gctcgttcac aagttccccc atcaagttgt 720
   ttggaggcct tcagctttaa atgtacaggc ttaaagtgcg cttgcaaacg tttgctctcc 780
   tttttttctg aatgttgatt gccttagctg gccacctggt gttctgcatg tagccttctg 840
   tggtcatgtg aaaggagaca ggctcttcta agttgagttg ggatttttgc actcagtgaa 900
   aagctgaagt gcaaaagagc tatcaaagac aagaggataa aagactggga tagtcttttc 960
   caaggaccct ctttagaggg ccctaaagac ctcctttggg aattctgggg aaaaagaaaa 1020
   agtaatcttc tacttgcttc aagatttgat ttttttaaaa aagcctgcga cctattcaat 1080
   acattatgct taaattagca gtttctctgg aattcctgtc tctcctttaa aagaaaggag 1140
   agaacatttt agaacaatag ttctcaaagt gtgttccccg gacaagcagc atctgcaaca 1200
   cttaggaagg tcttcgaaat actaatttgt aagccccacc tcaggcctac tgaatcagaa 1260
   gctctggggg ttgggtccag aagtctgttt tagtcaaccc tctaggtgat tctgatgctc 1320
   gctaaaggtt gagaactact gctttagaat gaagtcgtat aataaagtct ctgaaaaggc 1380
   cttattcaga ataagcaaga aaggttctgt gattcacttt tgcttctggg gctggcaaaa 1440
   accttctctg aacccacaca ccaagttcgt agttggtagg tgcccagcca agtcctgaca 1500
   tcttcatgcc ccctctgcag agggcggctg tacgatgttc acatgtctgc gtttggtcag 1560
   acatcatctc cttggctgcc ctttgaaacc aaatcacttg ccttggggat aaagtgctca 1620
   attggcatta gtgagaagcc catcctatcc cttgacatac ttaatcatat atctctccag 1680
   agaactcacc tgacaaatgt ctctgagcac aggctgacac caaagtggca caactgcaca 1740
   gttctcagat ttctttgcac agattgattt ttattgcggg ttttgttggg gtgtcttaat 1800
   gttcatctct tttccactgc ccatcctctg tgaacccata cctctctaga tggagcaggt 1860
   ggccactggt gcctcatact cagattgaaa accactacat cccagctacc tataatgctg 1920
   tcagctcaaa atcatagcca ggtagttctt gaactcagaa cttaaatcct gcacgtggca 1980
   ctccaccact gactggaccg agctggcata tgttgtttct ttgtgtttct acatcaaaat 2040
   gttcgtctaa gatttgaact gttctgctga taaccttccc cgttgtcata gctatttcat 2100
   tgccaaccaa ctccatcaca tggttgttga tatcgtcata taaagccatt gcaaggactc 2160
   tggaaactgc cgccaatgac caatttctga ctaaccagcc accttttctc tctcttagct 2220
   ccacgtcagc actgagacca gactcgagca cccctgtcct gtaagcgaga caaaatggcg 2280
   tgtgttattt tggggttttg tgttttttgg tgggtttctt tccttggctc tccagattta 2340
   cttttggggc ctgttctaag tgcaaaccca gcaagtttca cttgtcctgt ccattagata 2400
   caactacatc ttgcgggggt tgtttctttc ttgttccaca atgaattgca catccatctc 2460
   catcagagct gatagcctgt taataagcac tggtctaaca cagccaaccc tcctccacag 2520
   cgccatatta atggaggagg ggaggaaggt gaaatctact gcatgggatt caggaaacag 2580
   ttgtggttgg tcaggacgga agttggggta agtttggttg gtcagaggga gttgtgctgg 2640
   agattgtgaa aaatgggttc ttgaatgatc tactataagg cagggaaggt tcatttgtaa 2700
   gtagtaatgt gaactgaatt gcattaagag tgtgtggcct ttgttgtgat atactatgta 2760
   ttttcttata tgcatgagcc aaactgttgc atcataattt agcactgatg tctgctttta 2820
   ttttgatcat ctttgtccac ccttattagt tcttggctgt taaccgtaga tagatcttgt 2880
   aaatccagca acctttggtt gctgcattcc ccttggttcg attccacgca aggagccaca 2940
   agtgagaact ccactgtcct tagaagaaag ggcattttta cttttgaacc aaaaagagaa 3000
   aaaaaaatca gaagtgttgc atcttgaggc gaattaactg taagacattt ttaattatga 3060
   ctactgcaat ttgacaccat ttgaaataat caattcagag acactaaaga tttcacaata 3120
   ttcattggta ttgtaaaaaa aaaatactat tgtatggatt tttgtattgc tgttaagtat 3180
   tgttttgtgt gtgtgtgtgt gtgtgtgtgt tggaacctcc tggggacatg ttatattttg 3240
   aagtgattaa actatttaat tgtgtgtcta tattttggag tggaataatt tcttcattaa 3300
   aaaatgtttt taaaaacaca aaaa 3324

<210> 155
   <211> 1618
   <212> DNA
   <213> Homo sapiens
<220>
   <223> nbla22344
<400> 155
   atacatcatt agataataat gtagcaataa attgtagctt tcactacata tgaataggca 60
   catgaatata cacttgtatt agtaaactct agtaaagatt tttactctgc ctatacaaat 120
   tatgaattac atatacttta atttctatca tattttgttt gtatccattt aattttcaca 180
   tagcttaaac acgaagtgaa gagagctgtt taggatctgg gaaataataa aaatgaattc 240
   ttttaaaatt tatttctggt gaattcgaaa tgcagaacat gtctttcaag agacaactcc 300
   ccctttttct caaaaatgtc aagatcagac tagaaaaatt ttcatccaag gcaatgtgtt 360
   atttttattg tctgaaggaa caggggagac tttcatggaa gagagagcat ggtttagtga 420
   aagcccaggc tgagagccct tactcctgaa cttgaatccc acctttctgc tgggctggcc 480
   ctgtgtgcaa gtcaaccagg ctcagtacct acatctgcaa catggagcta agggtatctg 540
   ctccttcctt gcccattaga ctgtaaggag ggaaacatta gtattagctg gagagttctt 600
   tggtttctta gcgaaattgg tactaaatga tgcactgtgg ctttctaaga aaatgctttc 660
   tatgcagtgt cagcccccag gaccatgcgc aacactgcat gcagcagata gaatgcaaca 720
   taaaattata tgcataactt tattttgaat atcaccctgg aaagtattgg gttttcattg 780
   ctgtaaaatc atgttaccag gagtcacttc acaaaatact tgataataga aggatcactt 840
   gcattctaat caccaaacag tacaattttt ttaaaggaag cacaaaaata aaattataac 900
   aaatatattg gccaaagcag actgatgtag atttggactt atattttaaa atcttaaatt 960
   attataagaa taataagttt tactatttgg tttaatattt taataaaaat aaaaaatgaa 1020
   aagtttgacc attcaaacat catttgtaag ttaaggatta gctataaaag tcagacatag 1080
   acatttgcaa cctgtttttg gaagctacta tgaattgctg aattgttttt catttatggc 1140
   ctgaaatttg aaagctaagt actgttatgt gaacagcgaa ttggaaaagg gaataaaata 1200
   ttgtgtactc agtggtgatt atgcaccagg cacaccacat tccttacctg tttttcatcc 1260
   ctacaactgc acaaagtagg tattaatagt tccacctcag agatgaggaa cctagaattg 1320
   tacaaaatta gaggccaggc acggtggctc acacctgtaa tcccagcact ttgggaggcc 1380
   gaggtgggcg gatcacaagg tcaggagatc gagaccatcc tggctaacac ggtgaaaccc 1440
   cgtctctact aaaaatacaa aaaataagcc gggcgtagtg gcggacgcct gtagtcccag 1500
   ctactcggga ggctgaggca ggagaatggc gtgaacccgg gaggcggagc ttgcagtgag 1560
   cggagatggc accaccgcac tccagcctgg gcgacagagc gagactctgt ctcaaaaa   1618
<210> 156
   <211> 2274
   <212> DNA
   <213> Homo sapiens
<220>
   <223> nbla22939
<400> 156
   ggacaaaaag tagctattgc aagcaccatt ctctggtttc ctggagattt cacgaggctc 60
   tgctaggtct agcggaaggc caagcaggct gaccactgac ttcttacctt cttggatttt 120
   atcttttttc tttattggat ttcatagaat attttattgc tcttgttgtt ttttcaatcc 180
   cactatttaa agtcactgtt cctcagcatg gagtatggag gtgtggaggg tggaaacatg 240
   ccagggtgtg ccgtttgtac ttactttagt gagtaagcca tcaaaggtct gggaagccat 300
   caagaccttt gaacagaagt gtgactgatt cagagcattc cttgaaaaag atgagtgtaa 360
   ggagcaagga ggattgagta gggcacatct cctattctgc atcttttcac cctaacacat 420
   ccattgaaca gatatttacc gagtgcctgc ctacgctggg ccaagcaatg ttgtcaacat 480
   aggggacaga gtctctgccc tcataaactg ctattgctgg taaaagccac tttctgaatc 540
   gtatgctggt gaaaattctc tgaagaaaag gctgccactg ccaacttatc tcagggcatt 600
   tgatggtcct gactggcctt ttcctaccca aaatgttgag ctttggtgtt tggtgaatgg 660
   gggtagcaca tggcagagtc acacatgact agttgtatgg gagaatgatc aaattccaga 720
   aacaagagtt gtagtcatcc taatagccaa gccactgaca aatgtcaact gagtagaaag 780
   taaccactga atatcgtttt aaaaagattc actgatttat ttcatctaat cagaccatgg 840
   agcctgttta ggtagcagac tgaacttcat cagccactac ttgttccctt tgagtttaga 900
   aattaaaaac aactaagccg gatattccat actgaagtct gggtttgaag ggatgtggcc 960
   aacttgtcta tccttcatga tgcaaaattt gcttttatag cataagcagc ctttgaatga 1020
   acactatctt taggtttggt gtatccgaac acagtgcctt ttttagtccg gagaccttgc 1080
   tctgttgaac aggagagcac tggaggtcaa gctagacctg gaactaaccc tatttctccc 1140
   attcttcaat tctggaggcc attcacattt cactcttttt cttccttcca tacttctcct 1200
   ccatctgtgt ctggttttta tttaactgat tattgcatta tgctctaatg atggttcaga 1260
   tcattttgga agataatgaa tgttcccacc acaaagaaac gataaatgat tgaaatgatg 1320
   gatatgttaa ttacccagat ctgatcacta aatagtttag agctgggacc aagctgaaat 1380
   attgagatca aaaagtgggt aattagctga gactggtttg gccagctggc ttggccagag 1440
   aaactgaata cagcaaaggc atccaaaggt ccttggattt atagctccat gtgggaaggg 1500
   aagtcaattc ctgataacca tgatatgtta atcccactgg taaaaactcc agatgacaaa 1560
   aaataatgca aagttgggaa gaactgaaaa atgtttccaa ttcatgtttg tagttttttc 1620
   tataactagg agtttcggaa gcaggactaa gactcctggg aagaagggct ggcaaaaggg 1680
   aggtatattt tggggaccca gatatgcaca ctgagattta aagaagaacc ccttgcagta 1740
   taggtatgtg taacacaaag tcaccaaaga aaaaaatatc catttccaaa taaaagccca 1800
   atcttagcct ggaccaattt ggagagagtg agaaaattct ttgacttcca accattgtag 1860
   aaatctttcc tgttagtttt gatagtaggg tctttcggct atataattcc aagcctgatc 1920
   aactggcatt attaagtttt ctgtcatggc tagttcagca actggagtag atatagattt 1980
   atatgtggat aattagctcc agtttgataa gtaaacaaag ataatgtcat gggctgatgg 2040
   aataactgag ttttggaaac ttttgctata ttgagtttgg ctatgctggt cataacgcat 2100
   tagagctggc ggtgtccaca ggagcacagt cactcagggc tcgattttct tatgcaaaag 2160
   acaaacgtgt caacgggaac agcaattgtg ataaggaagt aaaatatggg agggatctgt 2220
   ttcctgttgg tgattgctcc tacgttacct ttagctacct gattaaaaga aaaa       2274
<210> 157
   <211> 2653
   <212> DNA
   <213> Homo sapiens
<220>
   <223> nbla23084
<400> 157
   ttgaacataa aggacactca ataatttttt tcaaaaatta agaaattgaa agaaagggaa 60
   atggcatttt taattaagaa aaaaggcata tcctttaatg cactgtttgc taaagtgtgc 120
   cccataattt tctagaatac ttgttcaaaa attcagattc ctggatgcct ccaggcctgc 180
   tgaaccaaaa tctcctaggc ttagtaacca taaatattaa catactctcc agggacttgt 240
   tatgaacact aagtttgaag accactggtt aatatcagtg gaaatttcac atctattatt 300
   cttcctctac atgcatttca tttcatttgg tacttcaaag tgtgtacggc aaaacaacat 360
   cttaaggctt aagacagatt atcatggcac tcgatgacta ccaaaaagtc acattttatt 420
   ataaatataa ccaaaactat ttttgaatat gtattattgc cataaaatgc actaagctca 480
   taaaactatt gaagacacta cctgtacaga acttagagtc aaggtaaaag aaaagacaca 540
   aaaatataaa gtgtattgaa caagcaaaat actaaaagat acccgaagtg tcatatggtt 600
   gcacatattt gccattagcc aacctactca ttatcctgtc tcccaaggac aacaaccttt 660
   taaggtaatt aaaataattc catatgcaga catggcaggg agacaaaaag agaatggggc 720
   tgtacaatga gaagctgggt gtcacgccac tcacattcaa taagtagatg tttattggaa 780
   caaggttctt attttattta caaaattctc tagcgttgta tacccccttc tcctccccag 840
   ggctaaattt tattcacatc ttggaatagc ctagcaggtg ttaccaagca cccacataaa 900
   aggaattttt gtctggtcac agtggcttat gcctgtaatc ccaacaattt ggaaggccaa 960
   ggcaggagga ttgcttgagg ccaggagttc aaagccagcc tgggcaacat agtgagagct 1020
   tgcctctaca aaaaaaaaat ttgaacaatt agctgggcat ggtgacacct gtctataatc 1080
   ccagctactc aggtggctga ggtaggagga tcacttgagg ccatgagttt tatacctgcc 1140
   tgggcaaaat agagagactc caactctacg aaaaaaaaat taatttaacc aggtgcaaag 1200
   gcacacccct gtagtcctag ctactctgga ggctgaggca ggaagatagc ttgaactcag 1260
   gagttggagc tatgatcaca ccactgtatg ccagcctggg tgacagaaca aaacaatgtc 1320
   tctaaataat aataataata ataaaaggaa ttctaactct atgagatgga gggtatttgg 1380
   gggtgaagga attatagagc actgtggagt ggtagccctg ggaagccaga tggcatgagc 1440
   accgaatgcc ttaggaaaaa ggaacaggtc agaagagtga agttggtcac agaatgaaag 1500
   tggagaatgg tgtcacacac agagcaccta atatgcgatt ttgtaattcc taaaaatggc 1560
   ccaagtaaca ctgcaaaaat cactgccata taaaaggcca tatataaatt gccacataaa 1620
   aactgatata aactttggtt aagtccacaa cctttagctt cccctaagtg gaacctatga 1680
   tccctaagct gggttgatgc aagtcctccc aaatgtcagc ccacacaagt ctcttcccta 1740
   cccacttctt acttcttctt tcctccccta gaaagttgca ggccagcaat aaagggggaa 1800
   aggggcagga actagtgacg ttgatagggg ccgcctctcc tgttgagttg tctcaggatc 1860
   tccttattct agaccttgat ggcacatcct ttgaggatgc tgatagcctg ctgagcaaga 1920
   taagcagtaa cagctaagtg gtaagatact caagagtttc tggacattta gctgaggagg 1980
   gaaagaaagc attgaaatac tggaaaggaa gatctgaggc atttctaggc aaggagaata 2040
   ctgttggcaa aattagaaga ctgggaaatg catgaggcac agtgatgcaa ttgagcagcc 2100
   cagccagctg gaggctagag tttgagttta gaaggagaga agagtggaaa aatggtatgg 2160
   gtccagactc caacagccct caaagagtga ttataatttt tacaaggaat actaattctt 2220
   attaatccgt tacattgccc catctgcaga gatctagaca tccttattct tagttctgta 2280
   ttaaaggaaa acaaaaacaa ttatttttaa atgatacact ataataccag aaactcttta 2340
   gataacaact gtgatcacta ttgacaacaa acttttaata agtatacatt tcatgggatt 2400
   tagtggctag gttagaaaaa aagtcaaaat attttgaagt aggcttttgg ttttgctgat 2460
   acacttctaa aaactgagct ctgatttatt ataattcaac cattgctcat gataatacat 2520
   aacaagtgac acaatcttta taaagataac gtatgaattt aaagaactaa gaaaatagct 2580
   gtttctaaag atctccaatt ttccaactga tttctgagca aatattctcc taagaaattc 2640
   tatttcctaa aaa                                                     2653
<210> 158
<211> 1909
   <212> DNA
   <213> Homo sapiens
<220>
   <223> nbla23103
<400> 158
   cacttgttct tgtagaaaag ggagaaatct ctactcaagt tttagaagaa gataaaatat 60
   gggtaaggtg acagttgtta ctgccatgca ggaagaaaat attggggctt gatagataag 120
   caaataaaca agataccttt gtgataaagg tctccacttt tagcactctt cttagccagt 180
   atgaccctca ataattcctt taccatctcc aaagcttcag gtacttcagc tctcaaaagg 240
   aaagtgactg gataggttgc acctaaaaca catttgggaa aattaagtgt gatttcctca 300
   aatataaccg tatagcctcc taaaataaga ctatgctgtt aaacctcttc ttttagattc 360
   tttacttacc caacccatta ttaattagtg cctcatctat cccagacggc ttttgcgttt 420
   gtttgattgt aagccttcaa agtgtcaggt attataatac ccatttgttt taattggtta 480
   aagtgataaa catagtgcct gtgcatgtac acattgaagg tatggctgtt tgacagaaat 540
   aatcttccta ccttctcctt cccagcccta acttctgaag ggtgagagaa tgagtgttta 600
   aaaaaatttt cttttcagcc caatgttatc ttttagcagt ctttacatct tcatcacctt 660
   tatgcatggt aatcagcaga acaggtctcc ctactgcagc agaactctgc atgaacccag 720
   taatttctca aatctgatag gtacagaaaa gtgtgtggcc tttcacttcc tgtcccttcc 780
   tccaacccca aaccatagag aagcatgctt tctggtgaca ttttattcac atagacattc 840
   tcacagctct ttattctgta agaaagatta tgtggagtat gaggagtgtt gttccgtgtc 900
   attttataac tgcctactcg tttgattttg caaatttgga aataaattat gaacgctcag 960
   gaaaatcctt ctatgagaga gttattactt ctgtccagtt ttgaaagtca ggtttgcagc 1020
   tatctgtgct atatcatttt aggaaggtgc ctgatgtgat cttcacacgt atcacctagg 1080
   attattcagg aaaggataat tcagattgtg gagctacaat atggagtttc cagtggttca 1140
   gtatgagtgc agtgagcaag acaataggga ccagaatggg gaaggccact taaaaatcca 1200
   agttcatggc tggccacagt gggtcacaag gtcaggagtt tgagaccagc ctggccaaca 1260
   cgctgaaacc ccatctctaa taaaaataca taaattagct aggcgtggtg gtgggcacct 1320
   gtaatgccag ctactcggga ggctgaggca ggagaatcgc ctgaacccag gaggcagaag 1380
   ttgcagtgag ccgagatcgt gccactgcac tctagcctgg gcgacagagc aagactctgt 1440
   ctcaaaaaaa gaaaaaaatc caagttcgtt actgactttt attgtactcc acgagataaa 1500
   aaacatagag attcatcagt ttagctctac ttgctcaata aaccacaact ttaactcttt 1560
   atatatattt ttctgttgac agaatacaaa ctggtgactt ccaaaattat gggtacctta 1620
   cttctgaggt ttagtcaaga gtttgtacag ctctaaatcc ttggatagaa ggttttaata 1680
   aaaatgccaa cttttaatta aaaatctctc tcttgattca gttatcttgc ccaaacttgg 1740
   aaactcttct tactactgta tataataatt cctgttaacc agatgttgtt tgatagctca 1800
   gtaataacaa atggagggta cttgtcctaa cctgatttac attctttcct tttgatgtgt 1860
   agcatatgtg gagcagtcag ctaaataaag gtcttatcaa taagaaaaa            1909
<210> 159
   <211> 1989
   <212> DNA
   <213> Homo sapiens
<220>
   <223> nbla23234
<400> 159
   aatttgatgc tggggacatt acctgacttt gtatattgca ttttgatctg catcatgttt 60
   gagaaatatc tgcaaatgac tctaagggag aggactttga aggattctat cgaacaaata 120
   tgtacatatg tttagtgccg tgctgggcag aggagtgtgg gaatgtacca gcgtgtatat 180
   aagacagtgt gcatcttacc taataatctt tatggccaga ttgagaataa atttttcgaa 240
   attttctttc ttccgcattt ccaactgacc cttatttaaa agtcattaat gttgagctct 300
   ctcatggtat ctttatccat ttttctaaag ctgcggtttc tcaggttatc aagtttataa 360
   cccttgtgag caagtcacgg atggtggagg aagcatgatg gagtatctgc atgagacggg 420
   gggctgagtg tgggaaactt gtgggatctt ctcatcctcc ctttctcaga gcacccagag 480
   tttgacagcg ctttgtgagt gtttatcaag agcctcccaa aagaggccgt ggggcgattt 540
   gcgaagtgca caaggcaaaa agtcaatagc ctgttttctt gtgctggctg ggcttcttgc 600
   cattaattag ttgtgtgatt tggggctagt cctttaaccc atctgcactt ccatctctgt 660
   gtgtgtaaaa tgaggtgatt gtaccaggcg atctctaaac acccttcctg ccctgatgtt 720
   ccagaaagcc tggttcggga gagagagaga cagacacaga aaggcgtgtg gcccaatctc 780
   tgctctcaag tatttcaacc ataggagcga ttaatattca ctacacagat tcaaaatcgg 840
   ggctactcca gggctggggt gccctcctgt ccgtttcttt tctcctctaa taaactcaaa 900
   ttgcctacaa cttttctttt tattattatt attatacttt aagttctagg gtacatgtgc 960
   acaatgtgca gatttcttac atatgtatac atgtgccatg ttggtgtgct gacatgcaca 1020
   catatgttta ttgcggcact attcacaata gcaaagactt ggaaccatcc caaatgtcta 1080
   tcaatgccta caacttttca acatgatttt attcttctca gcattgcctt ccacacaatg 1140
   ctcttttcta tataacttct tcctgtgagg ttctgtaata tttgctctgt gcctttcttt 1200
   tctcacattc attatctttc aggtagaaac acccaagagt gtttccactt gaactttcct 1260
   ctttctcagg acagcctctt tgccaaaccc atcttgacgc atgtactctc ttccttgagc 1320
   gtatgtgctt gcaaacactg tgtatggtag aatcatatgt tgccacattg aagacatata 1380
   agatgcctcc agtttctatg ttcaccattg tgatcattga tcacatatat gtgcccagtt 1440
   acatactgta ctgaaccaac catcctatgc cagacgttta caaacaaaac attcagaaaa 1500
   cagatggggc atagaggatg ataataaggc agagtggatg gcaggaatca gcagagtgaa 1560
   taatagggat gtagactaga ccaaaggaga aaaaaaatcc tgggagtttt ggttgcaaat 1620
   ttggaatgaa gagaatctat tctttttttc tgtttattgg gctttaggac tgtgtaaaca 1680
   aatttaggct ggctaggcca ggcatagtgg ctcacagctg tactcccagc aagcactttg 1740
   ggaggctaag gcaggcggaa cacttgaggt cgggagtttg aaaccaccct ggccaacatg 1800
   gtgaaactcc atctctacta aaaataaaaa aattagctga gcaccgtggc acatccctgt 1860
   aatcccagct actcaggagg ctgaggaaag agactcactt gaacccggga ggcggaggtt 1920
   gcagtgagct gagatcattc cactgcactc cagcctgggt gacagagcaa gactctgtct 1980
   caggaaaaa 1989
<210> 160
   <211> 1715
   <212> DNA
   <213> Homo sapiens
<220>
   <223> nbla23300
<400> 160
   aaatatgtaa caatacgttc tgggaattaa atgtgatgtt ttaaaaaaag caaacccaca 60
   ctgcattagc ccagtgccaa gcacataagt gtttaataaa gagtaatgat attattaaca 120
   cacttgaaat gtatcacccc ttcagttaat agaatggtaa acatgttagg atgcctgcct 180
   agaggattta gcagaaaatg ctttgtaaat gttctatatc tgaaagtaca tgttgatgtc 240
   caggttagca agatgaacaa agatgcgcag atgagagcag cgattaacca aaagttgata 300
   gaaactggag aaagagaacg gtaagtaata gattgtgtta ataaattaca tttcaccgcc 360
   tttaatagtt agcttgtaag aatctaaaca agaaatgaaa catgtcactg gaaagaatta 420
   caattgagat tataaaagtt tctattccga acatctggaa aaaataattt aggtttgtta 480
   agtattgcag gacttggagg gaagtggtga tgtagaaaga tgagcaagat atggctctta 540
   aagagtaatt tgatggagaa gaaaaagtac atacctgaat tacaactgga gacataatgt 600
   tacctgggtt ttaacaggga gacaatgttg tggaaccagc ttcatttacc aagctctcaa 660
   ccttggagca gcaagtgctg tttccactct ctaaaacatt cttccttctc ttccttttta 720
   gctaattcta gctcaactat caggtctaat ttatatattg tctcttcaag gaatgttttt 780
   aagtcccaaa taccttgttc catcatagca tttaatcaac attgtgttct aacaatctac 840
   ttggctagtt tgtatcctcc agtataatct aagttcttta acaccaaaaa caacattgta 900
   tacccagcat ctaatgcttt gtttggttca taattggcca cgtaaatatt tgcagaatca 960
   acaggtttag aagtacaaag aaggggctga ggaatcaaga aaggctccca gaggcagggt 1020
   tataaagtga gtaagacaaa tatgtataag gaagaggcaa atatgtataa ggaagagggg 1080
   gaatctttca actcaacccg ggcattcagg tgatcatgta agatctcaca caataagaaa 1140
   aagaggtgca tctgttgctg actttatttt tggatgatgg gagtcattta aagttttaag 1200
   aaaggaaatg actagatcac atttacactt taggaatctt actctgttgg tgtggagctt 1260
   ggacttgaag gggacaagat agatggcaga aaaatgaggt agaagattat atagggttga 1320
   aaatggaaaa ctccaaaaat tggaaggaga ccttagaatt ttaataaaat gtagaaacag 1380
   caaccctcaa aatgaggaag gaggcatcga taactgcctt gggtagcttt agaggatagt 1440
   actgctggta aggagtacgg attgtatgtt gttttttttt ttgttgtttg tttttgattc 1500
   atgcagcttc aagttactga gtttctatca tatgccatgc cctgttaagg tgttggaggt 1560
   aacagtagta gacaaaaatg gagactttgt tttcacagag cttgcattct aatgggagga 1620
   gacagataaa actgtgtaat aatgtcagat ggtgatgagc actagaggaa caataaagca 1680
   gaaaataaag aggtgtaata ttttagatag aaaaa                             1715
<210> 161
   <211> 2585
   <212> DNA
   <213> Homo sapiens
<220>
   <223> nbla23369
<400> 161
   atacaaatat tccagcccca aatgagaaat caaacatatt aaaattgttc aagaaaattt 60
   ctttgaacac ttttgaaagt ttttggaaac ttagaaaaga gggaaaaaaa tccagtgtta 120
   ctagtaattt ccatggtaat acagataaaa tacattcttt taattctggg aaattagaaa 180
   aagtggggtg atctttccag gaaaaacatg tgtaacatct gcttatcact ccagctccct 240
   cctcctcctc ctctccacgt tcccttgagt aaatgtctgg gaaagcatga agcttgatgc 300
   aagaaccctg ttgtactggc gttttcctcc cctgtgaaaa cgtaactact gttgggagtg 360
   aattgaggat gtagaaaggt ggtggaacca aattgtggtc aatggaaata ggagaatatg 420
   gttctcactc ttgagaaaaa aacctaagat tagcccaggt agttgcctgt aacttcagtt 480
   tttctgcctg ggtttgatat agtttagggt tggggttaga ttaagatcta aattacatca 540
   ggacaaagag acagactatt aactccacag ttaattaagg acgtatgttc catgtttatt 600
   tgttaaagca gtgtgaatag ccttcaagca tgtgaataat cttccatctt ccccgccaca 660
   catacacaca cacacttttt gtttctttca ggtagacacc ttttaaaatg caaaactaac 720
   tgaggcattt cagtaacttt gctttcaaat caataaagtc aaatgtatgg aaacattttg 780
   tgccctactc tccatacccc gtgtactcaa attctctact gtatgaatta tgctttaagt 840
   agaattcagt gccaaggaga acttggtgaa ataaattatt ttaatttttt ttttatcctt 900
   tacaaagcca tggattttat ttggttgatg tgtgctctgt acacaagcca tttcaatagg 960
   atggagctgt taattatttt ccaaagagta atagacatgc aaaagtttca ataaaaactg 1020
   ggccattaac aaataaatta ataaactaat aagcattccc ttctaggttt ttgccaaact 1080
   gcctatccaa taacaaattt gagaatcgtt gaaaaagcta gttatatttc agagaaatga 1140
   ttttcattat tgaaactgtt ctccctagca ggccattttc cctttttcct gggagtttag 1200
   caagtttagg agagaatagt catgaaaaga aagggaagaa aggggagaag ggaagaggtt 1260
   aaaaagtaag tgctcagacc tatgaacgta atccctttgc tagaaatatt taagagcagc 1320
   tcagcttggt tgaaactgag ttttgtcatc ttccatattt gcaggaaggt attttctgac 1380
   ttgcaatgca gctagatgta aaattttatt ttatcatact agaaagcctt gactagaaaa 1440
   atgaataaat attgagggtt tcctgtccat atctggcttg catgtgccag aaagcagaga 1500
   atagaaaatg taatctccaa catccaagca tcgaaaccca aggggtaggc aattctatgt 1560
   aggttttgga catgaagttt ggtgcatctt ggtttatgct ggctcaactg ctattaaacc 1620
   tctctggctt atagtctctt cattctatta gacaagcacg tatcgaacac ttgcttcgca 1680
   caaggctctt tagttaacaa tttagcagct actgtttgtg ttaaacacac ttttcaccaa 1740
   ataggttctg aggcaaacga gagcaatgac tatttaaaga aaggctttcc cagcatcact 1800
   tacacatccc aaaactaaaa agatcaactc ttccaactga gaaaagactc ctggctttga 1860
   atggaaactt acagcagaga gtcacaggcc acggcaacaa caacgacaac aacaaacatt 1920
   tggaatatta ttctcaactc acgttttaat aatacatctt attatttttc tagtagagaa 1980
   actacaaatc agcctcttca acatttatat acagtttaat aagcctcttg caagttactt 2040
   gttctctcac ctgaggtatt tttttcctcc ccaccttgcc cctgttcctc ccttcctctt 2100
   ctccctttgc aagaggaaat atttaacata tttgggtcca acttcaataa tgtaataatt 2160
   aatacattaa aagcatttaa cttcctttct agaaaaatgc acaggctaag gcatagacaa 2220
   aacaaagaga aatgctgaga aatttgccac tggagacaag caatctgaat aaatatttgc 2280
   caaaagttct ttttatgtca tatagtgtca ggatttgaag gagctatttt tttttaatgt 2340
   tgcaactagc aactcatctt cggaagacac agccaggaga atgaagtaga agtgaaaggt 2400
   ttataaatcc atttgtaagc atttatccca tatattttaa attcaagaaa aattgtgttt 2460
   atctttagaa ttttgtattc aatactttat gtactatgtg actcatgctt ctggataaat 2520
   aaagcaccaa atatgtatct gtaaccacaa tcacacatat tatattaaat atatatctat 2580
   aaaaa                                                             2585
<210> 162
   <211> 2027
   <212> DNA
   <213> Homo sapiens
<220>
   <223> nbla23436
<400> 162
   gacgctacgg cggatatggc tgcagagcgg ccggctggga tcttagatag gaggggtgga 60
   tttgcaaggc ctagaatagc tggggagtgg tttccccgcg gaatcggcct ccctgccgct 120
   cctgctttgt actgtgacgc tcagcctgtg atgactggtg tggaatccgc tgagccacct 180
   tggcctaagg agactttacc actctgagat tgtaaatctg taaaatagag atgtaggatt 240
   agcccatacg gtagttgtgg taaatactgt gagacaataa ggggcctggg acacagcatt 300
   caaatgggaa taatgaaggt caagactgtg attcctgtat ctttgacgct ctcggtataa 360
   gcaccgtcgt gggcacaggg cagtggcctt tatgcaggag tttaagaggg aatgaaggaa 420
   tgaatgggca aactctggag ttcccaagta ttctctccag gagctgtttc cattcttttc 480
   gtttccagca ggttggtaaa ttcattaatt tattcattga tctaattaaa atatactaag 540
   tgcccctcac ctgtgctagg ccaatgtgat acaatgagca gaacagtcat gggccctccc 600
   tgggaagccc tcactagccc aaggactcct tgtagacatt taagtgtcca caggctctgg 660
   agttccaacc ttgagtgcaa tttagcagct gtggaccttg ggcaagtcat tacatctaag 720
   cctgttttct cttctgcaaa atggttaagg attcaataag ataaaactgt aggcaatgaa 780
   aaccgtacct ggtaacagta ggtgctgaag aagtgttagc tattaatttt tgcttaattt 840
   ttctctctct gctctatgtg atgaaaagat tcaagaggca attgttggaa tgtaaaaaga 900
   gcacgggact tggagtcaaa tacttaagtc taccatcaag tagttgttaa gaattaaaca 960
   acaatttttg tgtacccagt taaatgtggg ctgcttagga atgatgactg tgtcttaatg 1020
   atctctgtat tcttagtgac atgtagaatc attgtgcctg acacatagta tgtactcagg 1080
   aaagaaatgg aaaatgtggt tttagcattg aaggccggga gagagggtct aacagactac 1140
   aagccctgcc aggagcagag taagggaaac agaggagaaa agtgttttta gtctgtgcct 1200
   gaatgtattt acatctgttt gtagcccaaa agccaaaagc gtacatacgc ttggcttttc 1260
   tgtagctatg tttatggctt tacagcagat tttatggagc tgcaattact ttgatcatga 1320
   gggactgatg ctagtggatt tacttcacca aatggaactc actttgtggc ttctgaagaa 1380
   gggacctttg tggactgtca tggagtagtt aagagtgcag gctctgattt agtgatcaga 1440
   gtctgcattg tcaggaatgg gacaaagtga agttatgtgg cacttgatag gatgccctga 1500
   gaagattgca acatcacccc tgtgatattc ctgctgaaga tccataacct ggatgtaatc 1560
   atgaggatat atcagacaaa cccacgtaaa gagacatgct gtatacaaaa ctgtaatctt 1620
   agaaagtgcc aaggtcatga aaatcaaaga tagaccctgg aactgttcca aactggaggg 1680
   gaccaaagag gcatgacaac taaacacaac acatgattct gaactggatc tttttgcttg 1740
   aaaggaagtt acagggacag ttggaaaagt ttaaatgggg cctacaatgc cgtggtaatg 1800
   atgtgtccgt gttaatttcc tgattttcat ggttgcctgt taagttacat cagaggatgt 1860
   tcttgtttgc tggaaagtaa atcaatgtat ttggcagggg ataaggcatc aaatggtcac 1920
   cttaatttca aattattaca gggaaaatgt ttctctctgt acttaataac ttttttgcaa 1980
   tttcttaaaa tgaaagctct ggagtaaaaa cttcaaggat ccaaaaa 2027
<210> 163
   <211> 2400
   <212> DNA
   <213> Homo sapiens
<220>
   <223> nbla23511
<400> 163
   tttcctggct aactccatcc agatgaatta ttcagtattt tttctcctat cttaatgaag 60
   ttaatttgaa tgctaatttc ctataaccaa gaaaacagtt gaattaaata acccttatct 120
   tttaaactta aagcttatac tactaataat catttaacat tcacttcctt ttttctgact 180
   taattggtag gtaaataaaa tacttcaaat ttgattggca aattggaaaa tcacttagaa 240
   caatctgcta gtatttttta ttccctttgt tttttccttt acacatttgt actgcaaaat 300
   aaatcaagga caaagactca cactgaattg atcaacttgt gtttggtctt catgggaatt 360
   acatcttttt tcccctcaac atttattaaa ggaacataca gaatttcaga ctatagcaaa 420
   ctaatacctt tagcttgact aagagttgat tttcgttaag gaacagaact tgtaatttat 480
   ttcgacatac tttaatgtat gactcatccc tgttaaagtt gtgagactca aaactacgcc 540
   caaatcactt aattttatgt ccttccctgt ttactgtgtc tgaccttcaa gatttcgtga 600
   ctgatgctga aatggaagcc aaccactgca gaaatttggg ggaaaatgag atctgaagaa 660
   tacaagggga agtaggaatt catttctagc atttccaaac ctgcttaatc gtgtctgctc 720
   caccacagtc agaggaaaag actgagttca tggaaattac cagctaagcc ttacatctgt 780
   ctttaatgtt tttaggaagt atactgaaaa ggtaagtgag atgtctgttt tgaagaaaga 840
   ctcttactgg gtaccttaaa acccgttgtt tcctattagt aaagatgggc agcttcttta 900
   ttcctagctt caaaaagcct tgcccctgtt tggtgtgttt ctcagtattg tggagaaggt 960
   agtttctgag caaggtggtg cttttcctct gcttctcagc agctaagaca gaaattgcac 1020
   cgaagtgtac aaagggccaa tttttgttgt cctgttgtgc tcaaatcctt ttttttaaaa 1080
   aagttatttc aatcaagtct tagttttatt cctcactata taggaaaaaa atctttaatg 1140
   cctcaaaagt tccattcagc attacatttg cattactctt atttgcagca aatatgagta 1200
   aaattatagg tttttaaagg tctctaataa catccactta tattggtttt gtagataatc 1260
   cataaattac cagaaataaa ttattccaca tttattacac acccatgtaa tagatgtcgt 1320
   gccaggccct ggaatatact aatggcatca cctcatgtgg taaaaagaca cattccgcca 1380
   tcctggagta tacaaaggta gactagcata tagttcatgt gctcaaggag ttcattttta 1440
   ttgacatgat acagatagaa ttgtagttta gggaatcaaa atctaataaa atgaggctaa 1500
   ttccattttc ccattaacac taataactag tgtgtaaatc tgaatatgac acattctata 1560
   tgaaagaagc tctgtgtgca tctacactaa atactcgtgt gtgccaggta ctgttttaaa 1620
   ctacgtatat ttttttaatt ctcataactg ttctctgagg tatgtactaa tactaaagct 1680
   tattgttaaa ggaaggcaga aaaattaagt aacttggcct aagtttgcat aactgtgatc 1740
   tgggatcaat atttgaaccc atacaggctg attgcagagc ctgcactctt aatttgagtg 1800
   tgatatttat gtgcagtacc tggctataag tacccacaaa acgtttcaaa ttctttataa 1860
   aatttgctta gttaaaaaag taccaattgc ataatatggt tataagtctg gtagaagtta 1920
   ggctttttac aagacatgct gcttactgca ccaaggaggc aagaaggctt tttagagagc 1980
   ccagaatttc ctttcctcaa ctcctgcttc caagacagtc attttgcatg ataaccgttt 2040
   ccccaaaaaa cacagacaca aaatttaaag aactggaaca gaggaagcag agcttatcat 2100
   agtatatatg tttagtaccc tgtcacttag gtccacccct ctttctttgt ggattgtgga 2160
   cattttgttt aactgctaaa tcatgagaat atatgactgc tgagactttt ccaaggattt 2220
   tttaaaaaac acattaggct ttgtgcagaa gtaaagaaaa agtgctgtga gaaccccagg 2280
   taggtaattt actttctatt gtactcatag tttgtttgaa acctcttcac ctctatccct 2340
   tattgtttta tactctgtaa atctgatttt acctttaata aacttttctg aagtgaaaaa 2400
<210> 164
   <211> 2954
   <212> DNA
   <213> Homo sapiens
<220>
   <223> nbla23664
<400> 164
   cattaattta atagacttta tattaagcag aataaattgt aatattgctt atgactaact 60
   tcaaatctaa tattttaatt tcaactaatc atttaactac tgacatcaag aaattactaa 120
   agctgttgag atttctatct catgtcttga tgttctctca gaatgtttat tggtctcatg 180
   acttttggtg actttcattt ctcctgctgt ccccatttct tcataaaagc tcatgtaaat 240
   acctaatatt taactttaaa tttcagtaat ggcaatcact gtttattttc tctgtcagca 300
   caatacaaga agctgattta cagctgttta aggaaataca aatgagtgga agaaaaggaa 360
   agctttttct gggaattaaa gagtaaatca ggttttgttt tattttgctt tgttttaaga 420
   gttctataca atataaatag aaaatgggtg agtccccata gtcacttgtt tggctctaaa 480
   tcttatccat tctattatta ctcctgagaa agctttgtag ttgtcatgtt actcatgttt 540
   taatgactga gaagagtttt ttcattggta cttttaaaaa atttaaataa atacaaattg 600
   atttttgtgt ttggtaaact atgttttcta gggtggtgtt tttaaatgta gtttaatttt 660
   taactctgtt ttaatttgta ttctcaacca ctagttagca gaaaataaaa tatctgtaag 720
   tcagataata aaaaacttaa atgaactgta aaaacctgaa gttatgaaga aagagtgacc 780
   taatataggt actagtttgt ttgttttttc attcattcat tctggcccac tgtgttcagt 840
   cttgtacttg aataaaaatg tcagaaacac cacacttttt tctttagttt ttcatgcttt 900
   tttgtctttt cccctccccc agcaaacgtg ttattgtgtg tcagcatttt ctgcaaactt 960
   cattttttct actagcattt aaatatttcc tgtgtcctag ggattgctct gtggattgca 1020
   ggataaaaga gggaagggac cctagtgccc ctccaggagg ctgtgtatct tgtagtggag 1080
   gagtccaatc actgaacaga tacttacatt tagaatgatg agtgctctgg tgaaggggta 1140
   cagagtacta caggacacca gcgtgaagat taaagggaaa gtgtttcaga ctagaatact 1200
   ccctgtcttt ttctgtataa aatagaaaac attttgctaa cattagtagg attatagtta 1260
   cttttcgtat cgttctcttc gaacctgcct aacattgcag agcaagtagg gtgagttgga 1320
   aagatttttc aggttctcat attgactatt ttgcttttca tttttattcc tttctcctaa 1380
   caacaaaata aaggaattca gacaaacatg tcatgtgata attatatagc cttgggtaat 1440
   acattattat tttttagttt taaagtactt taaaaattgg cagagtattt ttagtatact 1500
   aagatttgaa cagtttaacc agtagtgtcg ggatttgatt acgctgataa agatatgcaa 1560
   gaaataaagt aataaaagac aaaatgtagg tttggaaaat tcaaattgta gttttatcca 1620
   ttaatcatat actttacttt gtgcttgtca ttgtgataat tacataaaga taaataaaat 1680
   aacacaccta gcccttaaag tagtagttct ttacttttta aaggtcaggg gtcccttgag 1740
   aatctgaaaa attgagaatc tcttcctaag aaagtgcaca tacacataaa attttaggga 1800
   atattctagt tgtcttttca tccttgaaac cccaattaaa aattcatgtc ttaaagaact 1860
   gagatgatga tcatgctata tgagctagtt aattattaat gctgatgtgg atattcgttt 1920
   aaataaagcg aaattttaga aatcagaagt taaatttata gaaggaaaaa gtatattttc 1980
   tgttgttagg aaagcatttt ccagtaattt gatttttctg gcaccctaac taagggaagt 2040
   tggctttttt aaattttact ttgttgcaga agattaaatt taaggttgag ttccactttg 2100
   tttgcaatag tttgaaaaag aatagttaat gcagattttt tttttaaatt tttttccttt 2160
   taagctttgt gtcttgtaca atgtgagttt gccaaatttt cttcatctgc tacagattag 2220
   gtatgccatt gttgctgcca tgtggcggcg caccccgtgc ttcttaaacg cactgactgg 2280
   aggtttatcg catcacttgt tcacatgcac ggagcctggt aacagcctca tctgtatctt 2340
   gttagcttca ttttcttatt tttaaaattt cattatttat aaactcaaca tagcatttaa 2400
   aaataaaggc tagttttaat taattaatgt tactacaaaa agtcattgct aaaattttca 2460
   tagtgaaaca gattttaact tttgttaaaa tgtgctatgc tttaattaaa ttgtatttac 2520
   tctacaagca gggatgtttt acctgccatt ttaactgtat ttgccaaatt ctaaatataa 2580
   ttttgaaaat tgaaattgaa gcttatgttt atgtggcaaa agtaagcttc aggactgggc 2640
   tgtgtatttt tattggcatg taacagttaa tatgagctct acaagaattt gtttttaagg 2700
   agctaaagct atcaacagct gcagatttaa aaaattatat attaaaactg ttaggttagc 2760
   tcagttgtac aacttagtga atcttgtatc ctgagtttct gaaggctggt ggataggtat 2820
   ctctgaaatc attgtgtttt agtcttttta ctgatagttt tgtataggga attcatcttc 2880
   tcttttaaaa taactttttt cctttaattt atttctatta cttattgtac ataaatttta 2940
   aaataaaaaa aaaa 2954
<210> 165
   <211> 1996
   <212> DNA
   <213> Homo sapiens
<220>
   <223> nbla23860
<400> 165
   tatcaaaaag attttatctg tcccaaactt tctaactgta gccccagcac caacctcttc 60
   tttacatttg caccattacc tctctttgaa tactgtcctt ttttaacttg attctgttta 120
   tgatgatgcc aaccaataaa ttcaattagg aatataatga tgagcaaagc agacattgac 180
   tctgtcctca gtctggggga gaaaatgaga cattaattga ataatcacac aaataaatat 240
   aaatctgtta ctgtgtcaag ctctgtgaaa aaaaaagggg ggactgtgat gctctgagta 300
   cctataatag ggcatctgac tttgtcgggg tggtcaggga ggtcatggaa ggctcttatc 360
   tgaatgacca atagaccttg actaggcaaa gaaaaggtca ttatcaatgg ctgcacaatg 420
   attacaaatc tgtctgagtg tatgactgag cagagcacag atgagaacaa catgaactca 480
   gtagtgcttt ccatttagaa atttataata aggaggctga ctcatggttg actcactgtc 540
   tcctcctaag aggctgcctg atggggtctt ccacttgctt atcagagctc tgtggtctcg 600
   acatagacat gattttctaa atcccatggc tgaccagttc tgctgttcct tcggttttat 660
   gtttatgtgt ttgtttgcct atttatctac ctgtgtgcca gaattatgag atcgttcatt 720
   gccactgctg catctttcct tctcctctac cggttcctcc cttggcccct tttattttct 780
   gtattttctc cctttcccct cccttctcta cagaaacttt ctcctcctcc tttctcttag 840
   tcttaatttg ccattcattt tctttttttt ctcttttatt cttgtctttt ttttcttcgc 900
   tgttcaccat gaagatacca ggcttatgtt tgcatagtgc aatataattt acaaaggcat 960
   ctcagggaca ttattccatt tgatcctaat agcagctttg taaggtggtt gggtaagagt 1020
   catttatcct gtctacagat atgacagagg accagtgact tccccaaggt catgtgtctg 1080
   ggaagggaag gattcttgac tgcaacctag atggctgtct cctgcactac tagaccatcc 1140
   tgccttaaca gaaatgtcac atacattcca atcacgtctt ttagtctgac tgacaaaagt 1200
   ccttttccgt cttgtcttta tctttcatga aaataagtct agacaaaagt cgtggtcaga 1260
   ggggttttct ggtggctcat ccatcacatg agtagaaaca gccttagtct tatctgatga 1320
   atatttgcgg gacaataaat ttgaccttgg attgaactgc ttataaataa tgattttcat 1380
   tctgttggta ccttgcctgg ctgtgacctg gaaggtggca tggctaacaa gaaccaaaaa 1440
   caaagaggat tgcctcaggt atcatttgtc agccttcatt atattactca tcttgagaca 1500
   tctatcttta tatatccaaa tgaaatctgg tttttttttc tgcatatatt tcaatccctc 1560
   agagactctt aaattccatc aggatttctg tttacttcct tcttctgacc aattataaga 1620
   gagtttaaag aaagagcacg tctgtatcct atgccacaga ccagatgccc ctttattgcc 1680
   agggaaacag ccagcgatgt ttatccttta tttaatctct ctgctgactt tcagtgctgg 1740
   taaatgttta ttccaccgaa gtatgctttt aagatgtcag tcagcaacct ttattgacca 1800
   atggatcaca tttggtaaag gctcctgctt attacataga gaattagact gctcaaagag 1860
   gattttgcag gggacaggca ccatttattc attcagtcat tgatttgatt gattaacttc 1920
   ttatgcattt gttcaactaa gcatttactg aatgtctagt atgtgccaag cactctggtg 1980
   agatatttga gaaaaa                                                1996
<210> 166
   <211> 1481
   <212> DNA
   <213> Homo sapiens
<220>
   <223> nbla23877
<400> 166
   gttcaagagg aaatcttgtg ttacttcttt atgaaggact ccagcctggt ggagatgaat 60
   gagtcctgaa gatggaatcg aagctgtttg ggcacaaaca aggtttcact cttgttgccc 120
   aggctggagt gcaatggtgt gatctaggct cactgcaacc tccacctccc gggttcaagt 180
   gattctcctg tctcaggctc ccaagtaggt gggattacag tactttaatc agcatttaat 240
   gacccagtcg aaaattcatt gtttggaccc aagcactggt gggaaaggca ggaggggagg 300
   cctgccttcc ttcctccctc ccgagcccta cagcaggcca tggagtggtg agcgagttcg 360
   tacagtgcca accacattcc cagaaacttc cagcagaggt taatcctgct cctctcaggt 420
   gggcttggcc cattctctag actttggaag gtaatgttct atagaggcct gttctgaagc 480
   tttaccaggt caaaccggag aagaacccaa caagtaactc atcccagcct aactattctt 540
   caagggcaat caacctacag catccaagca cagagaaatc aaatccatgg agaatcttca 600
   aattaggctc agaatccatt tgggtcaatg aatttactgt tattaagatc ttagttgtgt 660
   tcaaccatga tttgacatac cttagagtga gaagatattc ttcctggcct cagactagtt 720
   gaaggtagag agagagacag gcccttgggt gtggggagac ctctcctggg ataatacaca 780
   caaaaaacca agagctgctc actgtggtgc aggagacagc agggcctgaa gccagaggct 840
   ctgtgtcctt gaatacaatg ttttactcct ctgacccctg ttactgtgat ttggagaggc 900
   agacaatata ggatgggctt tgcaggcagg gaggtccagt tataatccca gctcttacta 960
   agttgggtaa gactcactct gagacttagt ttcttctgtc atctctcaat agaatcataa 1020
   aggtactttc ctcttagtgt tgttttaaaa ttcagtaaaa taatgcaggc ttagcacagg 1080
   gtctgatgta aattttcaat gaattatcgt tgtcaatatt gttctggaaa acaagagggc 1140
   atattagaag atcaaaagta ctgccaagca ttgaagtgcc aattctagat ccagtctcag 1200
   ccctctgaga atggatatca ttgttttcaa gccattcaga aaccaatgtg aattgaacac 1260
   ctagtatgag ctctctgagg gaagagccaa gtcatgcatt ttttatctta aggggtcttc 1320
   aatacctcta gcccaaaaca gtatctccat caggattctt ctctgatagt gttcatttct 1380
   tttttctcaa tggatgcctt aaaaaaaaaa tcctacaagg aaacctgtac tcctcaaata 1440
   caccactcag gtgaccatta aatcatttac attgttaaaa a                     1481
<210> 167
   <211> 2056
   <212> DNA
   <213> Homo sapiens
<220>
   <223> nbla23998
<400> 167
   ctttgtgtgt ttggcttatt tcacttaaca taatgttctc caggttcatc catgttattg 60
   caaatgacag gattgcattc tttttttatg gctgaataat ttgtgtatat atagcacatt 120
   ttctttattc atctattgat agatgcttaa gttgttttca tagcttggct attgtgaata 180
   atgctgcaaa taaacatgca agtgcagata cctgtttgag atatgatttc attttctttg 240
   gatatatacc cagtaatgag attgctgaat ctacaaaact ttttactgag ataatcacta 300
   gactcattaa aagtaccaaa ataaattatg tgcctaaagg aagttatctg tatcctgctg 360
   ttcatggtag ataatatccg tatggccatt aaggtctctt ttataatttg agcaagcttc 420
   agacttcaaa gacttcacca agctacgact ttttgcttta atctccatag ttcagctata 480
   ttcactctgg ctacaaaagt ttcatgttcc tatttacttt gacttttgtt ggatatgggc 540
   tttctaaata ttttaaagaa aaatattggg actattcttt ggcactgtaa ctctgaaaca 600
   gctgctccct tagcacagaa ccatgcactt gtcagacaca tggtgaagac ttgcagagtg 660
   aattgtaaag ccctgtattc tcgatcgggt aagcacttgg gcagcccctc ccattttgca 720
   gacagagaac tagaaaatct aggaaatctg agacgtgcat gtgagaacca ggatcactcc 780
   acaactgtgc tgttgcagca gctgtgatag aaccaggctc agctggttgc ctcagtgagc 840
   cacatctgtt ttctctgcct caccacctag cattgcattt cttcagcctg tttttctggt 900
   cctcacaaag gggatgtaat tgtcacatag gatactgtgg ttcacaaagt ccatggagtg 960
   gccatctgag ttaattaaag ctctgtggta gttgctgaaa gcatttctgc ctgaagtgat 1020
   tctgtcctgt tgctttctcc tgcagggtgt ggttggcggt gttatgatag tgactcctaa 1080
   caacatcatg tttgaccctc ataaatctga tcctctggtt attgaaaatg ggtgtgagga 1140
   gtatggtctc atctgcccca tggaagaggt tgtttccatt gcgctctaca atgacatttc 1200
   tcacatgaag atcaaagatg ccttgccatc gtaagacatt tatttgttta ccaggaaaaa 1260
   aggggtgttg agagagctaa atgtagctta aaaatgaggg catttgcatg attgagggat 1320
   tgtgtagagg tgattttgaa gatggaagac ttgtgcactg aagaaaatga gaaaaatgag 1380
   aagaaatgaa aagaataaaa tcaatgatgg gaaaagttga acatataaag attaaaggag 1440
   aaaaacaaag aagccgtcat gtaaaaatag tatttgttgg gcttattttt ctaaaaagca 1500
   gtgcacgttc ttaatgaaat tatgaaggaa gaaaggcagt tctctgaaag aagtttatcc 1560
   aattatcaat aagagaataa tgttttcttc tgggtttaat taaggagagt tatgtttgtc 1620
   ttcatttaac ttctaggaaa agcagtctcc ctgattcatg tcctccctca gtcctgcatg 1680
   gagagaggtt tgggtctaca gtgtagtggt agccaccttc tcatgctgtg aagagggagt 1740
   aataccagtt tgctttttcc ctgaaataca gatgaatata acttcagtcc tgattacttt 1800
   tgccttataa tgctggattt attgtaaaaa agagagggaa gctccccagg aaaaaagaga 1860
   aagcattaag aaagctcagg aaattgatta actgatacag ataatctgat ttttactgtc 1920
   ctttcgctct actgtgtctg tttctctata aaagccagca gtaaaaaact ttaaaaacct 1980
   tcagtgatgg gaagaggcaa agcagtaggt cctaacagta aagagggaaa ctagcccttg 2040
   gggcttatat gaaaaa                                                  2056
<210> 168
   <211> 2564
   <212> DNA
   <213> Homo sapiens
<220>
   <223> nbla24043-1
<400> 168
   atttcatgac tggtgcgttc ctaaactctg aaatcagcct tgcacaagta cttgagaata 60
   aatgagcatt ttttaaaatg tgtgagcatg tgctttccca gatgctttat gaatgtcttt 120
   tcacttatat caaaacctta cagctttgtt gcaacccctt cttcctgcgc cttatttttt 180
   cctttcttct ccaattgaga aaactaggag aagcatagta tgcaggcaag tctccttctg 240
   ttagaagact aaacatacgt acccaccatg aatgtatgat acatgaaatt tggccttcaa 300
   ttttaatagc agttttattt tattttttct cctatgactg gagctttgtg ttctctttac 360
   agttgagtca tggaatgtag gtgtctgctt cacatctttt agtaggtata gcttgtcaaa 420
   gatggtgatc tggaacatga aaataattta ctaatgaaaa tatgtttaaa tttatactgt 480
   gatttgacac ttgcatcatg tttagatagc ttaagaacaa tggaagtcac agtacttagt 540
   ggatctataa ataagaaagt ccatagtttt gataaatatt ctctttaatt gagatgtaca 600
   gagagtttct tgctgggtca ataggatagt atcattttgg tgaaaaccat gtctctgaaa 660
   ttgatgtttt agtttcagtg ttccctatcc ctcattctcc atctcctttt gaagctcttt 720
   tgaatgttga attgttcata agctaaaatc caagaaattt cagctgacaa cttcgaaaat 780
   tataatatgg tatattgccc tcctggtgtg tggctgcaca cattttatca gggaaagttt 840
   tttgatctag gatttattgc taactaactg aaaagagaag aaaaaatatc ttttatttat 900
   gattataaaa tagctttttc ttcgatataa cagatttttt aagtcattat tttgtgccaa 960
   tcagttttct gaagtttccc ttacacaaaa ggatagcttt attttaaaat ctaaagtttc 1020
   ttttaatagt taaaaatgtt tcagaagaat tataaaactt taaaactgca agggatgttg 1080
   gagtttagta ctactccctc aagatttaaa aagctaaata ttttaagact gaacatttat 1140
   gttaattatt accagtgtgt ttgtcatatt ttccatggat atttgttcat tacctttttc 1200
   cattgaaaag ttacattaaa cttttcatac acttgaattg atgagctacc taatataaaa 1260
   atgagaaaac caatatgcat tttaaagttt taactttaga gtttataaag ttcatatata 1320
   ccctagttaa agcacttaag aaaatatggc atgtttgact tttagttcct agagagtttt 1380
   tgtttttgtt tttgtttttt tttgagacgg agtcttgcta tgtctcccag gctggagggc 1440
   agtggcatga tctcggctca ctacaacttc cacctcccgg gttcaagcaa ttctcctgcc 1500
   tcagcctcca gagtagctga gattacaggc gcccaccacc acacccggca gatttttgta 1560
   tttttggtag agacgcggtt tcatcatgtt tggccaggct ggtctcgaac tcctgacctc 1620
   aggtgatccg cctgccttgg cctcccaaag tgttgggatt acaggcatga gccactgcgc 1680
   ctggccagct agagagtttt taaagcagag ctgagcacac actggatgcg tttgaatgtg 1740
   tttgtgtagt ttgttgtgaa attgttacat ttagcaggca gatccagaag cactagtgaa 1800
   ctgtcatctt gttggggttg gcttaaattt aattgactgt ttagattcca tttcttaatt 1860
   gattggccag tatgaaaaga tgccagtgca agtaaccata gtatcaaaaa agttaaaaat 1920
   tattcaaagc tatagtttat acatcaggta ctgccattta ctgtaaacca cctgcaagaa 1980
   agtcaggaac aactaaattc acaagaactg tcctgctaag aagtgtatta aagatttcca 2040
   ttttgtttta ctaattggga acatcttaat gtttaatatt taaactattg gtatcatttt 2100
   tctaatgtat aatttgtatt actgggatca agtatgtaca gtggtgatgc tagtagaagt 2160
   ttaagccttg gaaataccac tttcatattt tcagatgtca tggatttaat gagtaattta 2220
   tgtttttaaa attcagaata gttaatctct gatctaaaac catcaatcta tgttttttac 2280
   ggtaatcatg taaatatttc agtaatataa actgtttgaa aaggctgctg caggtaaact 2340
   ctatactagg atcttggcca aataatttac aattcacaga atattttatt taaggtggtg 2400
   cttttttttt tgtccttaaa acttgatttt tcttaacttt attcatgatg ccaaagtaaa 2460
   tgaggaaaaa aactcaaaac cagttgagta tcattgcaga caaaactacc agtagtccat 2520
   attgtttaat attaagttga ataaaataaa ttttatttca aaaa 2564
<210> 169
   <211> 1945
   <212> DNA
   <213> Homo sapiens
<220>
   <223> nb1a24402
<400> 169
   agaaacatgg atacggtcaa cctattaggc ctgagccttg gaccacaagg cctaacacct 60
   acaggtctaa ggagatccct ggaacaaaga cactacacac actctttcag gtacctttgt 120
   tatgggcact tgaatggtgc tgcttcacag aggctgcacc accagtcatg aggatctcag 180
   accagagctc caggaagttc tgctgttggt ctgataccaa gagtaccttc agattctgga 240
   aaggattttc acggggttgc ctatgaagga gacaggaaag gaccttagca tgacaagtaa 300
   tatccaacaa actgcctttc tgcaaaggga ctcatgtaca tctgaatgct ttcaaaaata 360
   aatgccccat cagacatagt gtctcaagcc tgtaatccca gcactttggg aggctgtcgt 420
   ggttggatct cttgggcctg ggagttcgag accagcctgg gcaatgtggt gagaccccat 480
   ctctacaaaa gacaacaaaa aaattagctg ggtgtggtgg cgagtgcctg tagtcccagc 540
   agcttgggag gctgaggtag ggggatcact tcagcctggg aggttgaggc tgcagtaagt 600
   cgtcactgcg ccactgtact ccagcctagg tgacagagca agacttcatc ttaaaaaact 660
   aagccctata ttagggtccc ccttctcttc cttctttcta tgaatgatct gtattccttg 720
   cattcctggc tttctaattt ccatgtttgt tctggggctg agaataatcc aaatcatgct 780
   cctgagccta tatattttta atgcttgctt aaaacttagt tctctgactt tacaggttga 840
   gaatattgaa cctatataca aatcttcaca catttgcaaa aggttcctag ccaatgtaac 900
   ctagggaaat aaactagata aactcctgaa gtcatttcaa acccactcaa atttatccca 960
   cagacattcc aatttctaga aagctttact ctctcaccta gattctcttc cctccaaagc 1020
   ttgctgtcct cctgcctata caattctgga tgggcttcaa atacttacca gtccagaatt 1080
   ctttgctcct caaggctgta cccagctggc aacagataat tacggtagtt ctggagctgg 1140
   ttggcatggc aactatcatg gacccagaca tgagacacac aaggaatccc actggcaagg 1200
   cacaggaagt acttccgggt tcgacaatgc tgatccgcaa ttagaagaca ctggtaagct 1260
   gtgttacact gcaagaaaag aagcagagcc aatgggtttg gtgacttctg tggaaagctc 1320
   ctaagcagca gccataatga gccatgaaga gcagatctga agactcccaa ctactaccca 1380
   aaatgtgatt tagtctatcc tgcccaaggc cactcttctc actggaaggc ccaagtaatt 1440
   tccatagatg ttctctctgc ctcacctgca gcatactgag gacctaaatc ctcaacggac 1500
   aaccaaaacc tatgaactca gcctttcagg ctaaaaatca gcaaccctaa taggggtttc 1560
   tactactaaa cataaacatc aatcttcttt tgtcccagca acagaaccat agccattaac 1620
   taacccaagg tcctaccttc tcttccctat acacaacaaa aattctattt catgcaaaaa 1680
   cattttggca gtttctcagt tcctgaaatc tctggctact ttatccaggt tccccaaccc 1740
   ctcccaggcc tcttctcaac acagcaagtt ggctcttatc attgccacta tattaggtta 1800
   cacaaagaaa ctcctcacct gggcttcatt gaaatcttca aggatatagc cagctcctgc 1860
   tcgaagctgg gattctgtat actgcttgtt gaaaggagga atttccaaaa attctatatt 1920
   aaaaaaaaaa ccaagataat aaaaa 1945
<210> 170
   <211> 1559
   <212> DNA
   <213> Homo sapiens
<220>
   <223> nbla24821
<400> 170
   atatttaatg taattactga tatatgtggt tgcattcctc ctcttttacc tcatttttac 60
   tctttatttt acttgactat tgtttgtgca tgcatctgtg tgtgtgtgtg tgtgtgtgtg 120
   tgtgtgtgtg tgtacacatg tatttcccta aagtgattgg ctggtcaaaa ctgtacagta 180
   ccacataccc catccccaag gccccatatt tacccattta gcaactttat aagatgaaat 240
   ccttatactt catttatttc tccacgttct ctgtttttgc cttgtcaggc cacaggtctt 300
   tcctttctgc cttctctgat acttcctcaa aacctgtgcc aatcatacct gtagctgtgg 360
   actttgctga gagagtctag tatttttagc acaagctgta atgagagtgt cattgacagg 420
   gtgttgcttc tctttcagta atccatacca ccagctgtgt gatttgcctg tcatctatct 480
   tcacccactc atatgaactc actctcttac tgtcctctct ctcctcccct ttgtctccat 540
   ttttgcgttt ttgtctttag atctctgttc tcatttagat tttggttata ggaccttttc 600
   aaatgggtta cgtaggttgt atattctttg acacccatca tgacaaaact attaatacct 660
   ttctttctga aatgtgagtc atattttgcc tagctttctg actcatatca gagttctttt 720
   ctctccgaca tatagaagtt attctacagt tttctaagtt ctggttttgc aaatgagaat 780
   tcaacttact ttccattgta aactttacat ttctcattct ggaagagcat ttgattttca 840
   gtttatcctt gaaagtaaaa aatttgaaaa ggatacgtct tgttgtatgt gtgtgttcct 900
   attaatcaca ctcagtgagc cctctaagtc tagaggactc aaatctagtg attgtaatat 960
   gggagcaaaa tgatgtactg gcttctccac ctgcagcatt tattttctat attagtagta 1020
   ttattttatt tatgtatatt cagaatttat aaatttaaaa ctagtaaaat atttaagaat 1080
   ttcaattaca aacatttaaa cctaaatgat taagtattta caaagataaa ctttaaacat 1140
   attattcaaa tatgttatta gcagattaat taaaataaaa tatcaaaata agcattacct 1200
   aaaatgaaaa accttaatct ggaaaaaaag gtaaagtaat actatttttt ctttttaaaa 1260
   aggtataatt aggccgggca cagtagctca cgcctgtaat cccagcactt tgggaggcca 1320
   aggcgggcgg atcacctgag gttgggagtt cgagaccagc ctgatgaaca tggagaaacc 1380
   ccgcctctac taaaaaatac aaaattagtt gggcgtggtg gcaggcacct gtaatcccag 1440
   ctactcggga ggctgaggca ggagaatccc ttgagcctag gaggcggagg ttgcggtgag 1500
   ccaagatcgc gccattgcac tctagcctgg gcaaaaagag caaaactcca tctcaaaaa 1559
<210> 171
   <211> 3106
   <212> DNA
   <213> Homo sapiens
<220>
   <223> nbla20026
<400> 171
   ttttcttgta cattttgcct cacctacctc aaggctagag cagttttgcc agctctgtgt 60
   gcttcacccc agctgcttgc agccagtgag agaagctttc cattcttcct ggaccttctg 120
   ggcttgggga agctgtgggg ccatctatgg ctccttaggc cacctgttcc acatgcatgt 180
   ctttagcatc aaatgtgctg gttgtgggag gaccatggga ccttactggc ttcctacact 240
   gcttggagaa cagaaggtgc aagtgatccc tgttttcagg ttcattaaac ctattgtggg 300
   gttcttctgt tcccctccca gggatgagtg atgaggactc agggctcctt cccacagatg 360
   cttgtcccag acacagctgg gtctggctgc ttggcttccc ccgagaactc tccctgagcc 420
   ctctgcttat gacattgctt cacttttgtg acatcgctta atttttgtga tgttgcttca 480
   cttttgtcat attttattca tcagaaagaa ggcaccaggt ctaacccaca ctcctgaaaa 540
   ggggattgca cagaggcaca aagacctctg gtgtttccag tccgggtaga ctggctgtca 600
   ccactggggc actggtgggt acctgtgagc tgatgagtgg gaccaaacgg ctctggccac 660
   cttggacccc attcctccca ggctttgtct ctccctgagc cctgcgcttg agaacattaa 720
   aagccatgcc ttggaccccc ttgttctgag tcctgccatg ggccgtgagg acagccggcc 780
   actcttcctg gtgagcagat tgtcacttgg ctccagctgc acgtccagct cttccgctgt 840
   tttgctcacg gtaaatgcgt cactggagaa gggaaggtgg atttttgcgg ttccacgtgc 900
   ctggcacaag gatatcattt ggtaaggaaa cttgttggag aatgtgtgaa ggcccagggt 960
   ttgttctttc ctctcttcca gctgtgctta ctggctggag agaagggttt ggattcgtct 1020
   cgttactctt ggctgctggg cccttcttcc tttgtcggct gttcagaagt gggaaaatat 1080
   atattttttt atccctctcc ttctttgtct ctttgtctgt gtctgtctgt ctgtctctct 1140
   cacgcacaca ccctccatcc tctgatccca ttctagcttc cctgctttat ttcccactga 1200
   tttctttaat gccccaatca catataaact aaaccatttt ctgttccttg cgttctggct 1260
   cttgggtggt cctagttaac cagctttcac agggcagcgt ttcccctttg gtgtgattca 1320
   cattaaaggt gagacttaga cgctgtctga agtgcaggca atttactctg gcagcaatct 1380
   cacaacacgg acagcaggag caggctggtg gccaaacaca aggtccagat gaccacccga 1440
   ctgggaaggg tctccatctg gcgaccgttc tcggagtttg agggattctt cctcctttct 1500
   tacacctgta ctcagtccag gtcagttccc aggtgtttct ttcataatgg agctttaagc 1560
   tattctggta agggtgagct ttgttttaag gtttgtgaaa gttgtgtctg tgctagatgg 1620
   ccttatctct agggcaacta ggattttggg atccagttga catagagacc cagtaatccc 1680
   tgggccaggg ctggaaatcc caggccaggt tgcatcatac tgctaagtgt gtaggtcctg 1740
   tgagatgttt gagtgggcgt atggctgtca ttaatcttat agccatggta tctcatagta 1800
   tactacagtg tgtctttgtt tgtgttagtc tactggaaat gaccttctct tatgactcta 1860
   acatttaccc cattccttaa aaaaatctgc tgtaaagcaa tatttacaat cagaaacctg 1920
   gaaaatatac aaatatatat ctctacattt gtagaatgat ttctatgcat atatatatat 1980
   aagaaatacg gaaatgtata aagtagaaag caaaacccca taactttatc acctggctgt 2040
   aatcattccg attcattctt ttagattatt tttcttcttt ctttcttttt cttttctttc 2100
   ttgcaactcc ctgatatgat gagagatcct tgaggcccac ttcaagtgca agtctcctca 2160
   gacacctttt tatatcatta ttcctagcca aaagagatgg tgtctttctc agtaccccta 2220
   gaatgttagt gctcctgctc gtcactgtgt gttcggggtc attgtattag ttatctattg 2280
   tattgcaaat tacccccaaa attatctatt gttgtattgc aaaaattact attgcaaaat 2340
   agtggcttaa aacagcagcc atttactatt acacagtttc tctgggtcag gagtctgtat 2400
   ccagctttac taggttctct gtccaggatc tctgacaggc tgcactcaag gtgtcagcgg 2460
   actgcagtct cacctgaagg ctcggctagg ggggaactgc atccaggctt acgcatggtc 2520
   tgagggcttc caggccttgc tggctccctc agacctttgc cacatgggcc tctctgttga 2580
   gcagctcact gcatggcagc tggcttccag cagagtgacc aggggagaca gcaagagagc 2640
   ctttttgtaa tctgatcttg gaggtgacat tgcttcactt ctgtcatatt ttattcatta 2700
   ggaagaagtc accaggtcta acccacactc atgggaagag ggttgcacaa aggcataaag 2760
   accaggaggc agggaccact ggggtccatc caagaagttg cctgccgcag acaatcctgc 2820
   ttatgagcct gtgctggact gcatgccatc ttgggcagag ccctgcctta tctttatatg 2880
   tctaatgaga tcgtgtatct tgtgcctgat gggcactcag aaacccactt tgctgttccc 2940
   tctttcgtct ctcatagcag gcgtggtggc atacgcctgt ggtctcagct gcttgggagg 3000
   ctgaggcagg agaattgctt gaacttgcga ggtggaggtt gcagtgagcc gggatcacgt 3060
   ggctgcactc cagcctggtc aacagggcaa gactctgtct caaaaa                3106
<210> 172
   <211> 1668
   <212> DNA
   <213> Homo sapiens
<220>
   <223> nbla20421
<400> 172
   ctttctgcgc tagtttatta catttagtac atttgtattg tatgaaaagc aacagcccag 60
   attatttgat ccccgtctgt gttaatcttt ccttcctgcc tctccctttt ttttttttgc 120
   ggcggcgggg gcggttggcc tttctttgtt tttgtttttt tttctatgtt cctgtccctt 180
   atttttaaaa atctctttta gcaacaggga tatcatcacc acgctggtat cctcacatgt 240
   gtgggttttg ctgagctagt agaaaatgat ccaaagatga ttggtgacca aatgtctgat 300
   tgcaacattt cgttttcctc cgtggtacat agctccaggc tgccagtctc ctatttgtgg 360
   ataatcccgt gggcactggg ttcagttatg tgaatggtag tggtgcctat gccaaggacc 420
   tggctatggt ggcttcagac atgatggttc tcctgaagac cttcttcagt tgccacaaag 480
   aattccaggt aagcaaagac tcaggaacag ctaagtaaag ggctggcaat atcaactcta 540
   catccatcag cataaacctg aactgcctcc agagtttaat gcctagctga tttcagagaa 600
   aactttttaa ttcccaagat tgggttgtgg acttttgttt ctgtcatctc taaagttgat 660
   atttaacttg aaagaatgac cttggagtga gcattctaat cagacgcaat aatcagatat 720
   ggagtggtgg gggaggaaga caaagcagat ttgttttttt ctggtcatta cgtgcaatag 780
   aaatttgaaa ttaatttgtg tgactcagaa agcaatcaag gtagttaatt ctgtgtaaat 840
   tccttttctt gctagacagt tccattctac attttctcag agtcctatgg aggaaaaatg 900
   gcagctggca ttggtctaga gctttataag gtaatggaaa ataactttgt tgttatggtt 960
   ttggacagaa aatcaattat gttactttta tgtactcacg tgctattaaa tatactttga 1020
   atagggccat gtacatgcag agtacgatta aatctgtagt aataaccata aaaagttttt 1080
   aaaagaagaa tgaagattgc cctgctagat ctggaacaag atataaagca tgagtgagta 1140
   aaagaatgtg gtactaacat agcaatagac aaataggtta attgcaacag gatacagaat 1200
   ccagaaacac acacacatat atatgtatgt gtatcatata tttgtatttt atataaatat 1260
   atatgatcat atataaatat aagataacgt ttcaaatcat tggggcatgg atataatgtc 1320
   gataaatgtt atggagacaa atacctatca ctttggaaaa tagaaaactt gtattcctgc 1380
   cttgtataaa atattaattc tggatggatt aaaatctaaa cataaaaata aaaataatgg 1440
   agacaaatac ctatcacttt ggaaaataga aaacttgtat tcttgccttg tataaaatat 1500
   taattctgga tggattaaaa tctaaacata aaaataaaaa ttaggacaga atgcagtggc 1560
   ccacgcctat aatcccagca ctttgggaag cccaggcagg aggactgctt gtgaccagga 1620
   gttccagacc agcctgggca acatagcgac accctgtttc tacaaaaa 1668
<210> 173
   <211> 1559
   <212> DNA
   <213> Homo sapiens
<220>
   <223> nbla22298
<400> 173
   gctgaaaagg gaaaaatcgt gggcaattct gacgccagtg agcattgcca gttcttcctt 60
   caggcactgt tctataggga aggaggttag aaactcagat tcatggatgt tgctaagagc 120
   aacccggaac tcagacattt ttcactgtgc tttccttggc atgccaactc gaaggagaaa 180
   tgttagcaat ggggcacagg gagaaaccgt gccagtaggt atggtattgt taggtaaaat 240
   ggagcagcct tgcttgtttg gggaaccttt cagtctcccc aactatggac tatcgggttc 300
   ctgattttcc aagtccctgc tgagggtggg atgttgtgtg gatgatgtct ttcccctctg 360
   cagtggttgt ggcacacaca gacgtgtgaa ccttgaccac aggctcgaca caccctggtg 420
   tcatcgggtg ggttgtgttc cagtggccct gagccaagca agaccccagg aaagactctg 480
   gaaaactgaa gggggctgga tgtcacccac agtacatacc ttgtgcctgt aacgaagcag 540
   gcactggttt catttaggaa aggtattgtg tccgaagccc catttttaga ctgttaaaag 600
   tatacaaaca gaaacgaaca ccattgcctt aggtgcaaag cacacttttt tattttaata 660
   gaagcccagg cttgcacaac accaccttca tgaagattgg tcatttctga ggatgacaaa 720
   accacaaagt ttattgagat tgctccttca ttgacagtct ctaagcactt cagaagcaat 780
   gacaaggcaa actctgtggg atgatgacaa gggtccctcg cgctgcggca gtggagagtg 840
   tgtctgagcc aggctgtctg tggggagacc ccaccccacc cctgagacct gggtgacttg 900
   gcacctgtcc acggctctgc ttctccatcc acaaatggga aatatcacag gccctgcctt 960
   gtgtgttatt gatataagaa cttggaaaag agcttgctgt aatagtaagc atggtagatg 1020
   ttggctgcaa taaataattg tgttgctggg aactcagcaa cattaggata atattaaaaa 1080
   taaaatttaa agatttttct gggatatgtg ttaattgcaa cggttaaata aggtaaactt 1140
   catgaagaca tgtatagaat tttagttatc tataggtaaa ctacttattt taattcatca 1200
   tggactaagg ggacaaaact gcacccacac acacacatac acacacacaa acgtacacac 1260
   agtaaatatt ttcatgatat cgtctaggga tgtcaaatta acaaaaatta acataaaaac 1320
   agatgcattt tcaatgagat tatcatcaga tattatttat gaacagctta aatagaatga 1380
   agacttggaa ggatttgggg gaaggctcgc atgtgagtgt gtgtgtttgt ttgtgtgtgt 1440
   gagtgtgtgt gtgtttgccc ttttttccct ttgttttcag gatagttcca tttagaaaaa 1500
   aaagctttcc taccaaattt gcagatatct gcaaataata ttctgccaag aagcaaaaa 1559
<210> 174
   <211> 1557
   <212> DNA
   <213> Homo sapiens
<220>
   <223> nbla22549
<400> 174
   gaaaaaacta aagtaccttg aaaggttaca cattcagcaa accatgaaga taatagctat 60
   tctttattaa acactgtgtg ccaagcaata gactaggcaa tttttagata cgttacctgc 120
   aacctgtaca acatttctac actttatgga tgggaaacgg agacatggga agtgtggctg 180
   agttgttcat ggatgtagaa atagtaaacg gcagagtagg aaagtgaaac cgcctatctc 240
   tgacctggag gtctgcctgt atctttccca ctccaccaca ctgcacgtgg gtgtcccgaa 300
   accaccttcc cagattcctg actctcagta attttattat ggacaacatg catgagtagt 360
   catcatattt ttcaagtgaa atatcgggac atgatataac acatgactta acaatggtac 420
   tgaatatttg aaatcaggcc tttcccggaa aatcatgcat gaaggatcat tataaacaaa 480
   catagcaacc agttgtctcc ccgaacttgt cacttttctc ataaatgtct ggcctggagc 540
   tccaaaatca tccaaatact tagtagcatt ttagcctgag tacactttct cagttcctca 600
   actctttgta tacctttcca ccaatataga cattctagaa tctgcttcag atgcatttga 660
   aattttcacc cccatggaac tagtgattaa tatcagagcc cactcttgca gttggtaatg 720
   gggtggcaat caaacgttca gatgatgata aaggagagat aatggataat tctttttcag 780
   agttctcact taacagctct gttgtggaat gttttaaata gtcttataaa taatttgttt 840
   atagtattgt tgttagttta attgaatttt atgtaagaag ctgtccaaca tcagagaaat 900
   gaaattcctc ccactttctg tgtagaacaa ggtctctgac agtattgatt catggaagta 960
   ctaatggact tagaaaacat taagagaatg tcatttctca tagtgtttct gtttctgaaa 1020
   atgaatctcc tgaattatta tctttctccc tgttacttgg ctggggaaag agatagaagc 1080
   tgtataaaca aattctcttc catgctcaaa gcaagtgttc catgtgcaca acctgctgca 1140
   gactggggcc cttctcagtt aattgggttt cacaagcaat aatttctcca caacaaaaac 1200
   cacaacttga agtgagttga aaagagatca atagtggaaa cagtcgcctc agtacttttt 1260
   ctttctggat ttcatctcta gaaatttgaa gtgtttgaga cagagtccac cctttgtgca 1320
   aggcgagaac caatgaatgg actccttgtg tgaattattg catcttcttc caaagcaggt 1380
   tcatcaagac tttcacagag attcattttt gttgagaagt aagggttaat aggaggatag 1440
   aatttggatc caaatctagt gataaaagtg tccaagcaat cataaagtaa gatattttag 1500
   ggacatacca acatcttccc tttctgctaa tttcatgctc caaagatatg gcaaaaa 1557
<210> 175
   <211> 20
   <212> DNA
   <213> Artificial sequence
<220>
   <223> synthetic Primer: nbla22420-1-1f
<400> 175
   gcctactgga atggaaacac 20
<210> 176
   <211> 20
   <212> DNA
   <213> Artificial Sequence
<220>
   <223> Synthetic Primer: nbla22420-1r
<400> 176
   caaaggctat ccaaaagcaa 20
<210> 177
   <211> 19
   <212> DNA
   <213> Artificial Sequence
<220>
   <223> Synthetic Primer: nbla22689-1f
<400> 177
   cggattctgg tgggttctt 19
<210> 178
   <211> 21
   <212> DNA
   <213> Artificial sequence
<220>
   <223> synthetic Primer: nbla22689-1r
<400> 178
   agagtgaggg gaacaaagtg g 21
<210> 179
   <211> 21
   <212> DNA
   <213> Artificial sequence
<220>
   <223> synthetic Primer: nbla24135-1f
<400> 179
   gaggacacca gcgtagaaga g 21
<210> 180
   <211> 20
   <212> DNA
   <213> Artificial Sequence
<220>
   <223> synthetic Primer: nbla24135-1r
<400> 180
   ggaagaaact gaggcagagg 20
<210> 181
   <211> 19
   <212> DNA
   <213> Artificial Sequence
<220>
   <223> Synthetic Primer: nbla24350-1f
<400> 181
   tcccaggaga aatgaatgg 19
<210> 182
   <211> 18
   <212> DNA
   <213> Artificial Sequence
<220>
   <223> synthetic Primer: nbla24350-1r
<400> 182
   gtgtttggcc ctttggag 18
<210> 183
   <211> 20
   <212> DNA
   <213> Artificial sequence
<220>
   <223> Synthetic Primer: nbla23701-1f
<400> 183
   agccctcacc ccaagtaaag 20
<210> 184
   <211> 20
   <212> DNA
   <213> Artificial Sequence
<220>
   <223> synthetic Primer: nbla23701-1r
<400> 184
   cagcgagcta gagtgaacga 20
<210> 185
   <211> 19
   <212> DNA
   <213> Artificial sequence
<220>
   <223> synthetic Primer: nbla23890-1f
<400> 185
   tggaaaagac accgggaag 19
<210> 186
   <211> 21
   <212> DNA
   <213> Artificial sequence
<220>
   <223> synthetic Primer: nbla23890-1r
<400> 186
   ccttggacag gtttttgttg g 21
<210> 187
   <211> 20
   <212> DNA
   <213> Artificial sequence
<220>
   <223> Synthetic Primer: nbla21650-1f
<400> 187
   cagttttctc cacggtccaa 20
<210> 188
   <211> 19
   <212> DNA
   <213> Artificial Sequence
<220>
   <223> synthetic Primer: nbla21650-1r
<400> 188
   atgggtggct gagatgagg 19
<210> 189
   <211> 20
   <212> DNA
   <213> Artificial sequence
<220>
   <223> synthetic Primer: nbla22094-1f
<400> 189
   ggtcaggatt tccccttttc 20
<210> 190
   <211> 20
   <212> DNA
   <213> Artificial Sequence
<220>
   <223> synthetic Primer: nbla22094-1r
<400> 190
   tcctagaagg ctgggctaca 20
<210> 191
   <211> 21
   <212> DNA
   <213> Artificial sequence
<220>
   <223> synthetic Primer: nbla22739-1f
<400> 191
   cgacgaatct ctgcaatctc t 21
<210> 192
   <211> 20
   <212> DNA
   <213> Artificial sequence
<220>
   <223> Synthetic Primer: nbla22739-1r
<400> 192
   tgcccatgaa tctcctaacc 20
<210> 193
   <211> 21
   <212> DNA
   <213> Artificial sequence
<220>
   <223> Synthetic Primer: nbla23525-1f
<400> 193
   tctgccatca acttctttcc t 21
<210> 194
   <211> 23
   <212> DNA
   <213> Artificial sequence
<220>
   <223> Synthetic Primer: nbla23525-1r
<400> 194
   ccatctcttt ctttcttgca ctc 23
<210> 195
   <211> 21
   <212> DNA
   <213> Artificial sequence
<220>
   <223> synthetic Primer: nbla20226r1-1f
<400> 195
   caagcaacaa tgacgaatga g 21
<210> 196
   <211> 22
   <212> DNA
   <213> Artificial sequence
<220>
   <223> Synthetic Primer: nbla20226r1-1r
<400> 196
   ggaggaatga gaatgaggtt tg 22
<210> 197
   <211> 21
   <212> DNA
   <213> Artificial sequence
<220>
   <223> Synthetic Primer: nbla22182-1f
<400> 197
   ttggaagcag gacatggata g 21
<210> 198
   <211> 20
   <212> DNA
   <213> Artificial sequence
<220>
   <223> synthetic Primer: nbla22182-1r
<400> 198
   tggacacatg gtggtgaaag 20
<210> 199
   <211> 18
   <212> DNA
   <213> Artificial sequence
<220>
   <223> synthetic Primer: nbla23256-1f
<400> 199
   ttgggggcag gagattac 18
<210> 200
   <211> 22
   <212> DNA
   <213> Artificial Sequence
<220>
   <223> Synthetic Primer: nbla23256-1r
<400> 200
   cctggctaca tagagaaacc aa 22
<210> 201
   <211> 23
   <212> DNA
   <213> Artificial Sequence
<220>
   <223> synthetic Primer: nbla21297-1f
<400> 201
   acaacgctag tcccacttac aac 23
<210> 202
   <211> 20
   <212> DNA
   <213> Artificial Sequence
<220>
   <223> Synthetic Primer: nbla21297-1r
<400> 202
   gctcctctgg ctcaacaatc 20
<210> 203
   <211> 25
   <212> DNA
   <213> Artificial sequence
<220>
   <223> Synthetic Primer: nbla20787-1f
<400> 203
   gagataggtt ctcttctgag tttgt 25
<210> 204
   <211> 21
   <212> DNA
   <213> Artificial sequence
<220>
   <223> synthetic Primer: nbla20787-1r
<400> 204
   caggtaagtt tgtcctccat c 21
<210> 205
   <211> 19
   <212> DNA
   <213> Artificial Sequence
<220>
   <223> synthetic Primer: nbla22284-1f
<400> 205
   ctaccgatcc ccagacaca 19
<210> 206
   <211> 18
   <212> DNA
   <213> Artificial sequence
<220>
   <223> Synthetic Primer: nbla22284-1r
<400> 206
   cagcaacagc cagaacca 18
<210> 207
   <211> 20
   <212> DNA
   <213> Artificial Sequence
<220>
   <223> Synthetic Primer: nbla20123-1f
<400> 207
   cgagagccat gcaaaaacac 20
<210> 208
   <211> 20
   <212> DNA
   <213> Artificial sequence
<220>
   <223> Synthetic Primer: nbla20123-1r
<400> 208
   gcacagaaaa tggaggcaga 20
<210> 209
   <211> 20
   <212> DNA
   <213> Artificial Sequence
<220>
   <223> synthetic Primer: nbla20382-1f
<400> 209
   gttcagtgca gtcaggatgg 20
<210> 210
   <211> 22
   <212> DNA
   <213> Artificial sequence
<220>
   <223> synthetic Primer: nbla20382-1r
<400> 210
   gtcacactct ttgctttgct tg 22
<210> 211
   <211> 19
   <212> DNA
   <213> Artificial sequence
<220>
   <223> synthetic Primer: nbla20660r1-1f
<400> 211
   gcgttcttcc acaccaaac 19
<210> 212
   <211> 20
   <212> DNA
   <213> Artificial Sequence
<220>
   <223> synthetic Primer: nbla20660r1-1f

<400> 212
   tccgaggaaa aggtgcttac 20
<210> 213
   <211> 21
   <212> DNA
   <213> Artificial Sequence
<220>
   <223> synthetic Primer: nbla20666-1f
<400> 213
   tctggctggg tttatagctt g 21
<210> 214
   <211> 18
   <212> DNA
   <213> Artificial Sequence
<220>
   <223> synthetic Primer: nbla20666-1r
<400> 214
   taccggctgt tggtgttg 18
<210> 215
   <211> 20
   <212> DNA
   <213> Artificial sequence
<220>
   <223> synthetic Primer: nbla21239-1f
<400> 215
   gcccagccta tgtctgtatc 20
<210> 216
   <211> 20
   <212> DNA
   <213> Artificial sequence
<220>
   <223> Synthetic Primer: nbla21239-1r
<400> 216
   tcctggtaca ctgcctcttc 20
<210> 217
   <211> 25
   <212> DNA
   <213> Artificial sequence
<220>
   <223> synthetic Primer: nbla21729-1f
<400> 217
   gacatttcta ccaatctgtg tgtct 25
<210> 218
   <211> 22
   <212> DNA
   <213> Artificial sequence
<220>
   <223> synthetic Primer: nbla21729-1r
<400> 218
   cacttgtgct tcttttctct gg 22
<210> 219
   <211> 21
   <212> DNA
   <213> Artificial sequence
<220>
   <223> Synthetic Primer: nbla21831-1f
<400> 219
   ggaaccgtag acttgttcgt g 21
<210> 220
   <211> 20
   <212> DNA
   <213> Artificial Sequence
<220>
   <223> Synthetic Primer: nbla21831-1r
<400> 220
   actcccagaa ttggaatgga 20
<210> 221
   <211> 19
   <212> DNA
   <213> Artificial sequence
<220>
   <223> Synthetic Primer: nbla22826-1f
<400> 221
   gcaatccttc cccttcctt 19
<210> 222
   <211> 20
   <212> DNA
   <213> Artificial sequence
<220>
   <223> synthetic Primer: nbla22826-1r
<400> 222
   tgtcacgacc ttccctgttc 20
<210> 223
   <211> 21
   <212> DNA
   <213> Artificial sequence
<220>
   <223> Synthetic Primer: nbla23899-1f
<400> 223
   cagggggatt gataacacag a 21
<210> 224
   <211> 20
   <212> DNA
   <213> Artificial Sequence
<220>
   <223> Synthetic Primer: nbla23899-1r
<400> 224
   ggatgaaatg caaggcagag 20
<210> 225
   <211> 20
   <212> DNA
   <213> Artificial sequence
<220>
   <223> synthetic Primer: nbla20578-1f
<400> 225
   catctgcatc caaaccaaag 20
<210> 226
   <211> 21
   <212> DNA
   <213> Artificial Sequence
<220>
   <223> synthetic Primer: nbla20578-1r
<400> 226
   agttagaatc ccaagccgaa g 21
<210> 227
   <211> 20
   <212> DNA
   <213> Artificial Sequence
<220>
   <223> synthetic Primer: nbla21908-1f
<400> 227
   agtctgcggg tctggtttct 20
<210> 228
   <211> 20
   <212> DNA
   <213> Artificial Sequence
<220>
   <223> synthetic Primer: nbla21908-1r
<400> 228
   tgcaaagttc ccctgcttac 20
<210> 229
   <211> 20
   <212> DNA
   <213> Artificial Sequence
<220>
   <223> Synthetic Primer: nbla22027-1f
<400> 229
   agttggtgga tggatcttgg 20
<210> 230
   <211> 20
   <212> DNA
   <213> Artificial Sequence
<220>
   <223> Synthetic Primer: nbla22027-1r
<400> 230
   gatgaaccga aacaggaagg 20
<210> 231
   <211> 20
   <212> DNA
   <213> Artificial sequence
<220>
   <223> synthetic Primer: nbla22082-1f
<400> 231
   tgtgctgaaa atccgaagtg 20
<210> 232
   <211> 20
   <212> DNA
   <213> Artificial sequence
<220>
   <223> synthetic Primer: nbla22082-1r
<400> 232
   gcaatgtagt ggggtcgaag 20
<210> 233
   <211> 20
   <212> DNA
   <213> Artificial Sequence
<220>
   <223> Synthetic Primer: nbla23303-1f
<400> 233
   cttgagctga gatggactgg 20
<210> 234
   <211> 19
   <212> DNA
   <213> Artificial Sequence
<220>
   <223> Synthetic Primer: nbla23303-1r
<400> 234
   cagcaggcag attccaaag 19
<210> 235
   <211> 25
   <212> DNA
   <213> Artificial Sequence
<220>
   <223> Synthetic Primer: nbla20264-1f
<400> 235
   gtcttctcta ccctctccct taatc 25
<210> 236
   <211> 20
   <212> DNA
   <213> Artificial Sequence
<220>
   <223> synthetic Primer: nbla20264-1r
<400> 236
   caccagtcct agcagcaaca 20
<210> 237
   <211> 19
   <212> DNA
   <213> Artificial sequence
<220>
   <223> synthetic Primer: nbla20269r1-1f
<400> 237
   agccaaactg gaggtgatg 19
<210> 238
   <211> 18
   <212> DNA
   <213> Artificial sequence
<220>
   <223> synthetic Primer: nbla20269r1-1r
<400> 238
   ccgtgaaagg ctgaaagg 18
<210> 239
   <211> 21
   <212> DNA
   <213> Artificial Sequence
<220>
   <223> synthetic Primer: nbla204O6-1f
   <400> 239
   tccaactcac agaaatgcaa g 21
<210> 240
   <211> 21
   <212> DNA
   <213> Artificial Sequence
<220>
   <223> Synthetic Primer: nbla20406-1r
<400> 240
   aagtctcatc caaagccaaa g 21
<210> 241
   <211> 22
   <212> DNA
   <213> Artificial Sequence
<220>
   <223> Synthetic Primer: nbla20949-1f
<400> 241
   ttcaaactat accctccctt tg 22
<210> 242
   <211> 20
   <212> DNA
   <213> Artificial Sequence
<220>
   <223> Synthetic Primer: nbla20949-1r
<400> 242
   cagttggttt ccacattcct 20
<210> 243
   <211> 20
   <212> DNA
   <213> Artificial Sequence
<220>
   <223> synthetic Primer: nbla21251-1f
<400> 243
   cttctttccc aagtgccaag 20
<210> 244
   <211> 21
   <212> DNA
   <213> Artificial sequence
<220>
   <223> synthetic Primer: nbla21251-1r
<400> 244
   tggctcaata accacaggaa g 21
<210> 245
   <211> 19
   <212> DNA
   <213> Artificial sequence
<220>
   <223> Synthetic Primer: nbla21334-1f
<400> 245
   tggctgggtt attcccttt 19
<210> 246
   <211> 25
   <212> DNA
   <213> Artificial Sequence
<220>
   <223> Synthetic Primer: nbla21334-1r
<400> 246
   gttcaatgtt ctcttgctac ttgtg 25
<210> 247
   <211> 21
   <212> DNA
   <213> Artificial sequence
<220>
   <223> synthetic Primer: nbla21356-1f
<400> 247
   actgaggaga tggagtggtt g 21
<210> 248
   <211> 19
   <212> DNA
   <213> Artificial Sequence
<220>
   <223> Synthetic Primer: nbla21356-1r
<400> 248
   atatgggctg atggttgga 19
<210> 249
   <211> 21
   <212> DNA
   <213> Artificial Sequence
<220>
   <223> Synthetic Primer: nbla21418-1f
<400> 249
   gagggtgagc tgggatatgt t 21
<210> 250
   <211> 20
   <212> DNA
   <213> Artificial Sequence
<220>
   <223> Synthetic Primer: nbla21418-1r
<400> 250
   accggcctct ctgtttttct 20
<210> 251
   <211> 20
   <212> DNA
   <213> Artificial sequence
<220>
   <223> Synthetic Primer: nbla21480-1f
<400> 251
   tgggagcaga acaaaatgaa 20
<210> 252
   <211> 22
   <212> DNA
   <213> Artificial Sequence
<220>
   <223> Synthetic Primer: nbla21480-1r
<400> 252
   aacaccatca accagaacag ag 22
<210> 253
   <211> 20
   <212> DNA
   <213> Artificial sequence
<220>
   <223> synthetic Primer: nbla21509-1f
<400> 253
   caaagacagt ggaagctgga 20
<210> 254
   <211> 19
   <212> DNA
   <213> Artificial Sequence
<220>
   <223> Synthetic Primer: nbla21509-1r
<400> 254
   ctgtttgtcc caggaggtg 19
<210> 255
   <211> 21
   <212> DNA
   <213> Artificial Sequence
<220>
   <223> synthetic Primer: nbla21527-1f
<400> 255
   ggacaggtag tgtttgggaa g 21
<210> 256
   <211> 19
   <212> DNA
   <213> Artificial sequence
<220>
   <223> Synthetic Primer: nbla21527-1r
<400> 256
   cgtaccccag atggagaga 19
<210> 257
   <211> 20
   <212> DNA
   <213> Artificial Sequence
<220>
   <223> synthetic Primer: nbla21551-1f
<400> 257
   caggaaaacg tggaagttgg 20
<210> 258
   <211> 20
   <212> DNA
   <213> Artificial sequence
<220>
   <223> Synthetic Primer: nbla21551-1r
<400> 258 20
   acagtgccca gacacacaga 20
<210> 259
   <211> 23
   <212> DNA
   <213> Artificial Sequence
<220>
   <223> synthetic Primer: nbla21735-1f
<400> 259
   catggctcta aaaggacaag aag 23
<210> 260
   <211> 20
   <212> DNA
   <213> Artificial Sequence
<220>
   <223> synthetic Primer: nbla21735-1r
<400> 260
   tgcctgaagg acactgaaga 20
<210> 261
   <211> 19
   <212> DNA
   <213> Artificial Sequence
<220>
   <223> Synthetic Primer: nbla22247-1-1f
<400> 261
   caccgtcctc acattcaca 19
<210> 262
   <211> 20
   <212> DNA
   <213> Artificial Sequence
<220>
   <223> synthetic Primer: nbla22247-1-1r
<400> 262
   ttcatccaag ctcgacacac 20
<210> 263
   <211> 20
   <212> DNA
   <213> Artificial sequence
<220>
   <223> synthetic Primer: nbla22477-1f
<400> 263
   cataggaggc ttgttttcca 20
<210> 264
   <211> 21
   <212> DNA
   <213> Artificial Sequence
<220>
   <223> Synthetic Primer: nbla22477-1r
<400> 264
   tcgtaggcaa atcagtcaaa g 21
<210> 265
   <211> 20
   <212> DNA
   <213> Artificial Sequence
<220>
   <223> Synthetic Primer: nbla22639-1f
<400> 265
   tgacagcaac ctgcaaagag 20
<210> 266
   <211> 20
   <212> DNA
   <213> Artificial Sequence
<220>
   <223> synthetic Primer: nbla22639-1r
<400> 266
   aagggataga caccgcaaca 20
<210> 267
   <211> 21
   <212> DNA
   <213> Artificial Sequence
<220>
   <223> Synthetic Primer: nbla23174-1f
<400> 267
   ggagggatca ccaaaacaaa g 21
<210> 268
   <211> 22
   <212> DNA
   <213> Artificial sequence
<220>
   <223> synthetic Primer: nbla23174-1r
<400> 268
   ttatgctctc tgaaggggaa tg 22
<210> 269
   <211> 19
   <212> DNA
   <213> Artificial sequence
<220>
   <223> Synthetic Primer: nbla23198-1f
<400> 269
   acaggcagtc ctcgctttc 19
<210> 270
   <211> 24
   <212> DNA
   <213> Artificial Sequence
<220>
   <223> Synthetic Primer: nbla23198-1r
<400> 270
   cagggtagct gtaaaaatgt tggt 24
<210> 271
   <211> 23
   <212> DNA
   <213> Artificial Sequence
<220>
   <223> synthetic Primer: nbla23328-1f
<400> 271
   tgacacacac aagactcaag acc 23
<210> 272
   <211> 20
   <212> DNA
   <213> Artificial sequence
<220>
   <223> synthetic Primer: nbla23328-1r
<400> 272
   atccaggcaa tatccacacc 20
<210> 273
   <211> 19
   <212> DNA
   <213> Artificial sequence
<220>
   <223> Synthetic Primer: nbla23420-1f
<400> 273
   ggagcacagg ccatcaaag 19
<210> 274
   <211> 21
   <212> DNA
   <213> Artificial Sequence
<220>
   <223> synthetic Primer: nbla23420-1r
<400> 274
   aggggacgaa ctctgaaaca a 21
<210> 275
   <211> 23
   <212> DNA
   <213> Artificial Sequence
<220>
   <223> synthetic Primer: nbla23483-1f
<400> 275
   gtaagtacgt gagccagtca tcc 23
<210> 276
   <211> 22
   <212> DNA
   <213> Artificial sequence
<220>
   <223> synthetic Primer: nbla23483-1r
<400> 276
   cacctgtaac tgaccagagc aa 22
<210> 277
   <211> 22
   <212> DNA
   <213> Artificial sequence
<220>
   <223> synthetic Primer: nbla23808-1f
<400> 277
   tgttatgatt ggtcaggggt ct 22
<210> 278
   <211> 22
   <212> DNA
   <213> Artificial Sequence
<220>
   <223> synthetic Primer: nbla23808-1r
<400> 278
   cagggtggat taggtgtctc tc 22
<210> 279
   <211> 19
   <212> DNA
   <213> Artificial sequence
<220>
   <223> synthetic Primer: nbla23851-1f
<400> 279
   cttttgacgg ggatttttg 19
<210> 280
   <211> 20
   <212> DNA
   <213> Artificial Sequence
<220>
   <223> Synthetic Primer: nbla23851-1r
<400> 280
   accaccgtta ccagtttgtg 20
<210> 281
   <211> 20
   <212> DNA
   <213> Artificial Sequence
<220>
   <223> Synthetic Primer: nbla24011-1f
<400> 281
   gctgcaactg agacactgga 20
<210> 282
   <211> 20
   <212> DNA
   <213> Artificial Sequence
<220>
   <223> synthetic Primer: nbla24011-1r
<400> 282
   gtagcccatg aagtgggaag 20
<210> 283
   <211> 25
   <212> DNA
   <213> Artificial Sequence
<220>
   <223> Synthetic Primer: nbla24235-1f
<400> 283
   gagatgaaat gtcttgagga atgag 25
<210> 284
   <211> 21
   <212> DNA
   <213> Artificial sequence
<220>
   <223> synthetic Primer: nbla24235-1r
<400> 284
   tgcaaagatg aaatggtcag g 21
<210> 285
   <211> 20
   <212> DNA
   <213> Artificial Sequence
<220>
   <223> synthetic Primer: nbla24556-1f
<400> 285
   gagcacaaag gatgggtagg 20
<210> 286
   <211> 21
   <212> DNA
   <213> Artificial Sequence
<220>
   <223> Synthetic Primer: nbla24556-1r
<400> 286
   ctgggagaca gacagaacac a 21
<210> 287
   <211> 20
   <212> DNA
   <213> Artificial Sequence
<220>
   <223> Synthetic Primer: nbla24800-1f
<400> 287
   tgctgagtga tcctgttgag 20
<210> 288
   <211> 19
   <212> DNA
   <213> Artificial sequence
<220>
   <223> Synthetic Primer: nbla24800-1r
<400> 288
   gccagggttt agcatctgt 19
<210> 289
   <211> 22
   <212> DNA
   <213> Artificial sequence
<220>
   <223> Synthetic Primer: nbla20001-1f
<400> 289
   acagtcttct gttaggggat gg 22
<210> 290
   <211> 20
   <212> DNA
   <213> Artificial Sequence
<220>
   <223> Synthetic Primer: nbla20001-1r
<400> 290
   gcagtatgaa cgcgacaaag 20
<210> 291
   <211> 22
   <212> DNA
   <213> Artificial sequence
<220>
   <223> Synthetic Primer: nbla20083-1f
<400> 291
   gccagaatag aagggagaga ga 22
<210> 292
   <211> 22
   <212> DNA
   <213> Artificial sequence
<220>
   <223> Synthetic Primer: nbla20083-1r
<400> 292
   tcttacccac ccaaatccat ac 22
<210> 293
   <211> 20
   <212> DNA
   <213> Artificial Sequence
<220>
   <223> synthetic Primer: nbla20182-1f
<400> 293
   atttgagtga ggccaacagg 20
<210> 294
   <211> 19
   <212> DNA
   <213> Artificial Sequence
<220>
   <223> synthetic Primer: nbla20182-1r
<400> 294
   ctggtgcttt gggtatgga 19
<210> 295
   <211> 22
   <212> DNA
   <213> Artificial sequence
<220>
   <223> Synthetic Primer: nbla20248-1f
<400> 295
   gcagaataac taagggcaaa ca 22
<210> 296
   <211> 22
   <212> DNA
   <213> Artificial sequence
<220>
   <223> Synthetic Primer: nbla20248-1r
<400> 296
   gaatcccatc aaacagacag ag 22
<210> 297
   <211> 20
   <212> DNA
   <213> Artificial sequence
<220>
   <223> Synthetic Primer: nbla20250r1-1f
<400> 297
   ggcccatagc cagatactcc 20
<210> 298
   <211> 19
   <212> DNA
   <213> Artificial sequence
<220>
   <223> synthetic Primer: nbla20250r1-1r
<400> 298
   taggcatacc ccctttcca 19
<210> 299
   <211> 20
   <212> DNA
   <213> Artificial sequence
<220>
   <223> synthetic Primer: nbla20330-1f
<400> 299
   gccaaggtga cagaggagtt 20
<210> 300
   <211> 20
   <212> DNA
   <213> Artificial Sequence
<220>
   <223> synthetic Primer: nbla20330-1r
<400> 300
   gttccagttg tttccggttc 20
<210> 301
   <211> 23
   <212> DNA
   <213> Artificial Sequence
<220>
   <223> Synthetic Primer: nbla23983-1f
<400> 301
   gctcctagat tgtactgggg ttg 23
<210> 302
   <211> 20
   <212> DNA
   <213> Artificial Sequence
<220>
   <223> synthetic Primer: nbla23983-1r
<400> 302
   tggcttttgg aagaactgga 20
<210> 303
   <211> 22
   <212> DNA
   <213> Artificial Sequence
<220>
   <223> Synthetic Primer: nbla24111r1-1f
<400> 303
   tctgcatcag gctttagtgt gt 22
<210> 304
   <211> 21
   <212> DNA
   <213> Artificial sequence
<220>
   <223> Synthetic Primer: nbla24111r1-1r
<400> 304
   ctggcatttt gaggatattg g 21
<210> 305
   <211> 21
   <212> DNA
   <213> Artificial Sequence
<220>
   <223> synthetic Primer: nbla24142-1f
<400> 305
   tctgaaccct gttaccattc c 21
<210> 306
   <211> 20
   <212> DNA
   <213> Artificial Sequence
<220>
   <223> Synthetic Primer: nbla24142-1r
<400> 306
   tgatgaaagc cgtgaacaac 20
<210> 307
   <211> 22
   <212> DNA
   <213> Artificial Sequence
<220>
   <223> Synthetic Primer: nbla24157-1f
<400> 307
   cattctcatg tctccatttg ct 22
<210> 308
   <211> 22
   <212> DNA
   <213> Artificial Sequence
<220>
   <223> synthetic Primer: nbla24157-1r
<400> 308
   ctttctttct accatgcgct ac 22
<210> 309
   <211> 19
   <212> DNA
   <213> Artificial Sequence
<220>
   <223> Synthetic Primer: nbla24230-1f
<400> 309
   gtctgccacc caataagca 19
<210> 310
   <211> 19
   <212> DNA
   <213> Artificial sequence
<220>
   <223> Synthetic Primer: nbla24230-1r
<400> 310
   cctccacaac aggcacatc 19
<210> 311
   <211> 21
   <212> DNA
   <213> Artificial sequence
<220>
   <223> synthetic Primer: nbla20541-1f
<400> 311
   tgagtggact tcggttcctt c 21
<210> 312
   <211> 21
   <212> DNA
   <213> Artificial sequence
<220>
   <223> Synthetic Primer: nbla20541-1r
<400> 312
   aggcagcatt cacccttaac a 21
<210> 313
   <211> 19
   <212> DNA
   <213> Artificial Sequence
<220>
   <223> synthetic Primer: nbla20555-1f
<400> 313
   agtatgtgcg ttccgtggt 19
<210> 314
   <211> 20
   <212> DNA
   <213> Artificial sequence
<220>
   <223> Synthetic Primer: nbla20555-1r
<400> 314
   gtgctagggg atgggtaatg 20
<210> 315
   <211> 20
   <212> DNA
   <213> Artificial Sequence
<220>
   <223> synthetic Primer: nbla20645-1f
<400> 315
   cgctgaatat ggaggcaaag 20
<210> 316
   <211> 18
   <212> DNA
   <213> Artificial Sequence
<220>
   <223> synthetic Primer: nbla20645-1r
<400> 316
   gcccctttct tggaggtg 18
<210> 317
   <211> 20
   <212> DNA
   <213> Artificial sequence
<220>
   <223> Synthetic Primer: nbla20713-1f
<400> 317
   ctcccccatc gtatcctttc 20
<210> 318
   <211> 18
   <212> DNA
   <213> Artificial Sequence
<220>
   <223> synthetic Primer: nbla20713-1r
<400> 318
   gtccggcctt tggttttc 18
<210> 319
   <211> 19
   <212> DNA
   <213> Artificial Sequence
<220>
   <223> Synthetic Primer: nbla24250-1f
<400> 319
   ggcatttggg gacctcttc 19
<210> 320
   <211> 20
   <212> DNA
   <213> Artificial Sequence
<220>
   <223> Synthetic Primer: nbla24250-1r
<400> 320
   ctgtcttctt tgccccttcc 20
<210> 321
   <211> 21
   <212> DNA
   <213> Artificial Sequence
<220>
   <223> Synthetic Primer: nbla24254-1f
<400> 321
   acttggtgcc tgaagaagag a 21
<210> 322
   <211> 21
   <212> DNA
   <213> Artificial Sequence
<220>
   <223> synthetic Primer: nbla24254-1r
<400> 322
   actgcgttaa gatggaaaac c 21
<210> 323
   <211> 23
   <212> DNA
   <213> Artificial Sequence
<220>
   <223> synthetic Primer: nbla24327-1f
<400> 323
   ggtgctctac tactcccctt ttc 23
<210> 324
   <211> 22
   <212> DNA
   <213> Artificial Sequence
<220>
   <223> Synthetic Primer: nbla24327-1r
<400> 324
   ggtcatcatc agttcctttg ct 22
<210> 325
   <211> 20
   <212> DNA
   <213> Artificial Sequence
<220>
   <223> synthetic Primer: nbla24510-1f
<400> 325
   ggcattagcc tggaagaggt 20
<210> 326
   <211> 18
   <212> DNA
   <213> Artificial Sequence
<220>
   <223> Synthetic Primer: nbla24510-1r
<400> 326
   cgcctgcgac tgaaaaag 18
<210> 327
   <211> 20
   <212> DNA
   <213> Artificial Sequence
<220>
   <223> Synthetic Primer: nbla24554-1f

<400> 327
   atgacagggt gggcttttac 20
<210> 328
   <211> 19
   <212> DNA
   <213> Artificial Sequence
<220>
   <223> synthetic Primer: nbla24554-1r
<400> 328
   ccagtttcgg gatgtcctt 19
<210> 329
   <211> 20
   <212> DNA
   <213> Artificial sequence
<220>
   <223> Synthetic Primer: nbla24604-1f
<400> 329
   ctttccctct tccccaaaac 20
<210> 330
   <211> 19
   <212> DNA
   <213> Artificial Sequence
<220>
   <223> synthetic Primer: nbla24604-1r
<400> 330
   cttcccagaa cagcaagca 19
<210> 331
   <211> 19
   <212> DNA
   <213> Artificial Sequence
<220>
   <223> synthetic Primer: nbla21037-1f
<400> 331
   cctgctggtt gacctctcc 19
<210> 332
   <211> 19
   <212> DNA
   <213> Artificial sequence
<220>
   <223> synthetic Primer: nbla21037-1r
<400> 332
   ctcatcctca tccgggtct 19
<210> 333
   <211> 19
   <212> DNA
   <213> Artificial sequence
<220>
   <223> synthetic Primer: nbla21161-1f
<400> 333
   actcgcctgc ctgattctt 19
<210> 334
   <211> 21
   <212> DNA
   <213> Artificial Sequence
<220>
   <223> Synthetic Primer: nbla21161-1r
<400> 334
   cacttttcca caaacctcca c 21
<210> 335
   <211> 20
   <212> DNA
   <213> Artificial Sequence
<220>
   <223> Synthetic Primer: nbla21170-1f
<400> 335
   gctgcttcct ctttggttct 20
<210> 336
   <211> 20
   <212> DNA
   <213> Artificial sequence
<220>
   <223> Synthetic Primer: nbla21170-1r
<400> 336
   ccaagtttgc atgtttttgg 20
<210> 337
   <211> 18
   <212> DNA
   <213> Artificial Sequence
<220>
   <223> synthetic Primer: nbla21198-1f
<400> 337
   ctgcctttcc accttgct 18
<210> 338
   <211> 19
   <212> DNA
   <213> Artificial sequence
<220>
   <223> Synthetic Primer: nbla21198-1r
<400> 338
   gtgtctgctg gtgctcctc 19
<210> 339
   <211> 20
   <212> DNA
   <213> Artificial sequence
<220>
   <223> Synthetic Primer: nbla21298-1f
<400> 339
   taacttggcc ttggtgtttg 20
<210> 340
   <211> 20
   <212> DNA
   <213> Artificial Sequence
<220>
   <223> Synthetic Primer: nbla21298-1r
<400> 340
   caacctgcct ctgaatatgg 20
<210> 341
   <211> 21
   <212> DNA
   <213> Artificial Sequence
<220>
   <223> synthetic Primer: nbla21379-1f
<400> 341
   cgatagcagg tacaatgaag g 21
<210> 342
   <211> 25
   <212> DNA
   <213> Artificial Sequence
<220>
   <223> Synthetic Primer: nbla21379-1r
<400> 342
   cacataaggt aagagatagc gaaag 25
<210> 343
   <211> 20
   <212> DNA
   <213> Artificial Sequence
<220>
   <223> Synthetic Primer: nbla24705-1f
<400> 343
   agggctaggt gtgggttttc 20
<210> 344
   <211> 21
   <212> DNA
   <213> Artificial Sequence
<220>
   <223> Synthetic Primer: nbla24705-1r
<400> 344
   gcccctcttt gcactttact c 21
<210> 345
   <211> 20
   <212> DNA
   <213> Artificial Sequence
<220>
   <223> synthetic Primer: nbla21385-1f
<400> 345
   tgcttgctga aaagtcgaaa 20
<210> 346
   <211> 23
   <212> DNA
   <213> Artificial Sequence
<220>
   <223> Synthetic Primer: nbla21385-1r
<400> 346
   tagcgatgga aactaagaga agg 23
<210> 347
   <211> 20
   <212> DNA
   <213> Artificial Sequence
<220>
   <223> Synthetic Primer: nbla21416-1r1-1f
<400> 347
   gccaaaatca tcaccaagga 20
<210> 348
   <211> 18
   <212> DNA
   <213> Artificial sequence
<220>
   <223> Synthetic Primer: nbla21416-1r1-1f
<400> 348
   attccccctc cctccaaa 18
<210> 349
   <211> 24
   <212> DNA
   <213> Artificial Sequence
<220>
   <223> Synthetic Primer: nbla21599-1f
<400> 349
   gagagttggg agatgtaagg aaag 24
<210> 350
   <211> 22
   <212> DNA
   <213> Artificial Sequence
<220>
   <223> Synthetic Primer: nbla21599-1r
<400> 350
   gtgatatggt tccctgtttt gg 22
<210> 351
   <211> 21
   <212> DNA
   <213> Artificial sequence
<220>
   <223> Synthetic Primer: nbla21681-1f
<400> 351
   ggtaggagca atgactgttg g 21
<210> 352
   <211> 20
   <212> DNA
   <213> Artificial sequence
<220>
   <223> synthetic Primer: nbla21681-1r
<400> 352
   tcgtcagctc tgcttttgag 20
<210> 353
   <211> 22
   <212> DNA
   <213> Artificial sequence
<220>
   <223> Synthetic Primer: nbla21878-1f
<400> 353
   ggaaggcaac acattcctac ac 22
<210> 354
   <211> 21
   <212> DNA
   <213> Artificial sequence
<220>
   <223> synthetic Primer: nbla21878-1r
<400> 354
   caaggtcatt cttgggctct c 21
<210> 355
   <211> 20
   <212> DNA
   <213> Artificial sequence
<220>
   <223> Synthetic Primer: nbla21922-1f
<400> 355
   caccaagcag tgtgcctaaa 20
<210> 356
   <211> 24
   <212> DNA
   <213> Artificial Sequence
<220>
   <223> Synthetic Primer: nbla21922-1r
<400> 356
   tgaggaaacc cctaatcatc tatc 24
<210> 357
   <211> 20
   <212> DNA
   <213> Artificial Sequence
<220>
   <223> synthetic Primer: nbla22004-1f
<400> 357
   ttggaatgtc gtgtgtgtgg 20
<210> 358
   <211> 22
   <212> DNA
   <213> Artificial Sequence
<220>
   <223> synthetic Primer: nbla22004-1r
<400> 358
   aggtcagagc aatgagtgaa gg 22
<210> 359
   <211> 20
   <212> DNA
   <213> Artificial Sequence
<220>
   <223> synthetic Primer: nbla22004-2-1f
<400> 359 -
   cagtaagtgc attggcagga 20
<210> 360
   <211> 20
   <212> DNA
   <213> Artificial Sequence
<220>
   <223> synthetic Primer: nbla22004-2-1r
<400> 360
   gctttttatg gctgctgtgg 20
<210> 361
   <211> 22
   <212> DNA
   <213> Artificial Sequence
<220>
   <223> Synthetic Primer: nbla22085-1f
<400> 361
   acccaattta acctcccttt ct 22
<210> 362
   <211> 20
   <212> DNA
   <213> Artificial sequence
<220>
   <223> synthetic Primer: nbla22085-1r
<400> 362
   tgcaaaagca aagagcacac 20
<210> 363
   <211> 18
   <212> DNA
   <213> Artificial sequence
<220>
   <223> Synthetic Primer: nbla22119r1-1f
<400> 363
   gaggccacat gaaagaca 18
<210> 364
   <211> 18
   <212> DNA
   <213> Artificial sequence
<220>
   <223> Synthetic Primer: nbla22119r1-1r
<400> 364
   ctgatgacag ggcagaga 18
<210> 365
   <211> 19
   <212> DNA
   <213> Artificial sequence
<220>
   <223> Synthetic Primer: nbla22149-1f
<400> 365
   ccagtgtttt gctcttggt 19
<210> 366
   <211> 20
   <212> DNA
   <213> Artificial Sequence
<220>
   <223> synthetic Primer: nbla22149-1r
<400> 366
   gaaatcctca cttggatggt 20
<210> 367
   <211> 22
   <212> DNA
   <213> Artificial Sequence
<220>
   <223> Synthetic Primer: nbla22161-1f
<400> 367
   cgaagttggt gttttctctg tt 22
<210> 368
   <211> 22
   <212> DNA
   <213> Artificial sequence
<220>
   <223> synthetic Primer: nbla22161-1r
<400> 368
   taactgatgc cccttagtct tg 22
<210> 369
   <211> 20
   <212> DNA
   <213> Artificial sequence
<220>
   <223> synthetic Primer: nbla22252-1f
<400> 369
   tgagggtctt cttgcttggt 20
<210> 370
   <211> 22
   <212> DNA
   <213> Artificial Sequence
<220>
   <223> synthetic Primer: nbla22252-1r
<400> 370
   ccatttggtg tgtcctattt tg 22
<210> 371
   <211> 23
   <212> DNA
   <213> Artificial sequence
<220>
   <223> synthetic Primer: nbla22347-1f
<400> 371
   ccttggagtt agaagagaaa gga 23
<210> 372
   <211> 20
   <212> DNA
   <213> Artificial sequence
<220>
   <223> Synthetic Primer: nbla22347-1r
<400> 372
   agaaaggaag ggcagaaatg 20
<210> 373
   <211> 20
   <212> DNA
   <213> Artificial Sequence
<220>
   <223> Synthetic Primer: nbla22352-1f
<400> 373
   tggcattttc attgctacct 20
<210> 374
   <211> 21
   <212> DNA
   <213> Artificial Sequence
<220>
   <223> Synthetic Primer: nbla22352-1r
<400> 374
   tggaaaccct aagaatcacc t 21
<210> 375
   <211> 21
   <212> DNA
   <213> Artificial Sequence
<220>
   <223> Synthetic Primer: nbla22394-1f
<400> 375
   tgttgagaga cttccgcttt c 21
<210> 376
   <211> 20
   <212> DNA
   <213> Artificial Sequence
<220>
   <223> Synthetic Primer: nbla22394-1r
<400> 376
   ctggctgtgg tttgctttct 20
<210> 377
   <211> 21
   <212> DNA
   <213> Artificial Sequence
<220>
   <223> Synthetic Primer: nbla22423-1f
<400> 377
   cagggaagaa agccacagaa g 21
<210> 378
   <211> 22
   <212> DNA
   <213> Artificial Sequence
<220>
   <223> Synthetic Primer: nbla22423-1r
<400> 378
   ggcctgaaaa gtcagagaaa gg 22
<210> 379
   <211> 20
   <212> DNA
   <213> Artificial Sequence
<220>
   <223> Synthetic Primer: nbla22439r1-1f
<400> 379
   ccatttgttc ccctccttgt 20
<210> 380
   <211> 20
   <212> DNA
   <213> Artificial Sequence
<220>
   <223> Synthetic Primer: nbla22439r1-1r
<400> 380
   ctttgagagg cgctttgatg 20
<210> 381
   <211> 18
   <212> DNA
   <213> Artificial Sequence
<220>
   <223> Synthetic Primer: nbla22633-1f
<400> 381
   caggaagacg cagggaag 18
<210> 382
   <211> 18
   <212> DNA
   <213> Artificial Sequence
<220>
   <223> Synthetic Primer: nbla22633-1r
<400> 382
   ggccttgacc ttgtggtg 18
<210> 383
   <211> 25
   <212> DNA
   <213> Artificial Sequence
<220>
   <223> Synthetic Primer: nbla22698-1f
<400> 383
   acttggcatc ttactgatgt gattg 25
<210> 384
   <211> 25
   <212> DNA
   <213> Artificial Sequence
<220>
   <223> Synthetic Primer: nbla22698-1r
<400> 384
   gctttcttat acctgggaaa tcttg 25
<210> 385
   <211> 20
   <212> DNA
   <213> Artificial Sequence
<220>
   <223> Synthetic Primer: nbla22896-1f
<400> 385
   tcgaggtgac tcttctgacc 20
<210> 386
   <211> 19
   <212> DNA
   <213> Artificial Sequence
<220>
   <223> Synthetic Primer: nbla22896-1r
<400> 386
   agggacagct tcatttcca 19
<210> 387
   <211> 22
   <212> DNA
   <213> Artificial Sequence
<220>
   <223> Synthetic Primer: nbla23167-1-1f
<400> 387
   tagagacccc ttcctatgca ac 22
<210> 388
   <211> 20
   <212> DNA
   <213> Artificial Sequence
<220>
   <223> Synthetic Primer: nbla23167-1-1r
<400> 388
   ggctacagtt tgcctctcca 20
<210> 389
   <211> 22
   <212> DNA
   <213> Artificial Sequence
<220>
   <223> Synthetic Primer: nbla23339-1f
<400> 389
   tctcagctcc agtaattcca ca 22
<210> 390
   <211> 21
   <212> DNA
   <213> Artificial Sequence
<220>
   <223> Synthetic Primer: nbla23339-1r
<400> 390
   gaaataaccc caattccacc a 21
<210> 391
   <211> 20
   <212> DNA
   <213> Artificial Sequence
<220>
   <223> Synthetic Primer: nbla23352-1f
<400> 391
   ggattggatg actccttgct 20
<210> 392
   <211> 22
   <212> DNA
   <213> Artificial Sequence
<220>
   <223> Synthetic Primer: nbla23352-1r
<400> 392
   gactccctct ttctcccttc tc 22
<210> 393
   <211> 24
   <212> DNA
   <213> Artificial Sequence
<220>
   <223> Synthetic Primer: nbla23575-1f
<400> 393
   ccagatattg atttcagagg gaca 24
<210> 394
   <211> 19
   <212> DNA
   <213> Artificial Sequence
<220>
   <223> Synthetic Primer: nbla23575-1r
<400> 394
   tggggacaag gggagaaag 19
<210> 395
   <211> 22
   <212> DNA
   <213> Artificial Sequence
<220>
   <223> Synthetic Primer: nbla23592-1f
<400> 395
   tgatggcact tctaactctc ct 22
<210> 396
   <211> 21
   <212> DNA
   <213> Artificial sequence
<220>
   <223> Synthetic Primer: nbla23592-1r
<400> 396
   gatcttgtac ttcggccttt g 21
<210> 397
   <211> 20
   <212> DNA
   <213> Artificial Sequence
<220>
   <223> Synthetic Primer: nbla23601-1f
<400> 397
   ccagcagcaa aggaaaactc 20
<210> 398
   <211> 22
   <212> DNA
   <213> Artificial Sequence
<220>
   <223> Synthetic Primer: nbla23601-1r
<400> 398
   ctgggacaat tcaaaagcct ac 22
<210> 399
   <211> 25
   <212> DNA
   <213> Artificial Sequence
<220>
   <223> Synthetic Primer: nbla23630-1f
<400> 399
   aaacgggctt tagtcatttt aggag 25
<210> 400
   <211> 18
   <212> DNA
   <213> Artificial Sequence
<220>
   <223> Synthetic Primer: nbla23630-1r
<400> 400
   gcttttcccg cccacttt 18
<210> 401
   <211> 24
   <212> DNA
   <213> Artificial sequence
<220>
   <223> Synthetic Primer: nbla23754-1f
<400> 401
   tcagtcgtag tgtccacctt actc 24
<210> 402
   <211> 22
   <212> DNA
   <213> Artificial Sequence
<220>
   <223> Synthetic Primer: nbla23754-1r
<400> 402
   ggccaaccca tattcatcat ac 22
<210> 403
   <211> 19
   <212> DNA
   <213> Artificial Sequence
<220>
   <223> Synthetic Primer: nbla23892-1f
<400> 403
   gtccttcata cggccaatc 19
<210> 404
   <211> 23
   <212> DNA
   <213> Artificial Sequence
<220>
   <223> Synthetic Primer: nbla23892-1r
<400> 404
   cctgtatcat tagtccatgc tgt 23
<210> 405
   <211> 22
   <212> DNA
   <213> Artificial Sequence
<220>
   <223> Synthetic Primer: nbla23956-1f
<400> 405
   cttctaggtg taggaggtca gg 22
<210> 406
   <211> 22
   <212> DNA
   <213> Artificial Sequence
<220>
   <223> Synthetic Primer: nbla23956-1r
<400> 406
   ggagtaggca gtagagcaga ga 22
<210> 407
   <211> 21
   <212> DNA
   <213> Artificial Sequence
<220>
   <223> Synthetic Primer: nbla20365r1-1f
<400> 407
   tcagagggga cttcttgatt t 21
<210> 408
   <211> 24
   <212> DNA
   <213> Artificial Sequence
<220>
   <223> Synthetic Primer: nbla20365r1-1r
<400> 408
   aggttcttca ctagagttgg ttgt 24
<210> 409
   <211> 21
   <212> DNA
   <213> Artificial Sequence
<220>
   <223> Synthetic Primer: nbla20378-1f
<400> 409
   tgtaaacatg caaagggaag g 21
<210> 410
   <211> 22
   <212> DNA
   <213> Artificial Sequence
<220>
   <223> Synthetic Primer: nbla20378-1r
<400> 410
   agttatttga gggagggaca ga 22
<210> 411
   <211> 19
   <212> DNA
   <213> Artificial Sequence
<220>
   <223> Synthetic Primer: nbla20511-1f
<400> 411
   acctcaaggc atggttgct 19
<210> 412
   <211> 22
   <212> DNA
   <213> Artificial Sequence
<220>
   <223> Synthetic Primer: nbla20511-1r
<400> 412
   ctgctgctcc aggtattttt gt 22
<210> 413
   <211> 25
   <212> DNA
   <213> Artificial Sequence
<220>
   <223> Synthetic Primer: nbla21039r1-1f
<400> 413
   agaagcaata accagagata cagag 25
<210> 414
   <211> 22
   <212> DNA
   <213> Artificial Sequence
<220>
   <223> Synthetic Primer: nbla21039r1-1r
<400> 414
   aagggaggat gagtagaaga ca 22
<210> 415
   <211> 22
   <212> DNA
   <213> Artificial Sequence
<220>
   <223> Synthetic Primer: nbla21107r1-1f
<400> 415
   cgattttagc agggaataaa gg 22
<210> 416
   <211> 24
   <212> DNA
   <213> Artificial Sequence
<220>
   <223> Synthetic Primer: nbla21107r1-1r
<400> 416
   ctccaatcca aagatacaga aggt 24
<210> 417
   <211> 18
   <212> DNA
   <213> Artificial Sequence
<220>
   <223> Synthetic Primer: nbla21367-1f
<400> 417
   cggcatggag gactagga 18
<210> 418
   <211> 20
   <212> DNA
   <213> Artificial Sequence
<220>
   <223> Synthetic Primer: nbla21367-1r
<400> 418
   gccaacaggg aggtgattag 20
<210> 419
   <211> 24
   <212> DNA
   <213> Artificial Sequence
<220>
   <223> Synthetic Primer: nbla21790-1f
<400> 419
   atttctttga gtatctgggg tcgt 24
<210> 420
   <211> 24
   <212> DNA
   <213> Artificial Sequence
<220>
   <223> Synthetic Primer: nbla21790-1r
<400> 420
   cacccaccat ctagtaccat tttc 24
<210> 421
   <211> 20
   <212> DNA
   <213> Artificial Sequence
<220>
   <223> Synthetic Primer: nbla22253-1f
<400> 421
   tatgagccag aggaggatgg 20
<210> 422
   <211> 21
   <212> DNA
   <213> Artificial Sequence
<220>
   <223> Synthetic Primer: nbla22253-1r
<400> 422
   ggccaaggta ggtctttgat g 21
<210> 423
   <211> 20
   <212> DNA
   <213> Artificial Sequence
<220>
   <223> Synthetic Primer: nbla22355-1f
<400> 423
   atgctgacct tccaggctac 20
<210> 424
   <211> 20
   <212> DNA
   <213> Artificial Sequence
<220>
   <223> Synthetic Primer: nbla22355-1r
<400> 424
   tgtgtcttca tcctcctcca 20
<210> 425
   <211> 18
   <212> DNA
   <213> Artificial Sequence
<220>
   <223> Synthetic Primer: nbla22832-1f
<400> 425
   cggctgcttg aaactcct 18
<210> 426
   <211> 19
   <212> DNA
   <213> Artificial Sequence
<220>
   <223> Synthetic Primer: nbla22832-1r
<400> 426
   tcttcccggt gtcttttcc 19
<210> 427
   <211> 23
   <212> DNA
   <213> Artificial sequence
<220>
   <223> Synthetic Primer: nbla23755-1f
<400> 427
   gcctctgatt tttagctctc ttg 23
<210> 428
   <211> 21
   <212> DNA
   <213> Artificial Sequence
<220>
   <223> Synthetic Primer: nbla23755-1r
<400> 428
   tcctgccatc atatcctttc t 21
<210> 429
   <211> 20
   <212> DNA
   <213> Artificial Sequence
<220>
   <223> Synthetic Primer: nbla24549-1f
<400> 429
   catatcaagg ggcttctggt 20
<210> 430
   <211> 21
   <212> DNA
   <213> Artificial sequence
<220>
   <223> Synthetic Primer: nbla24549-1r
<400> 430
   gcattcacag ccttcagttt c 21
<210> 431
   <211> 22
   <212> DNA
   <213> Artificial Sequence
<220>
   <223> Synthetic Primer: nbla20084-1f
<400> 431
   ggccagtgtt ctctaccatc tc 22
<210> 432
   <211> 22
   <212> DNA
   <213> Artificial Sequence
<220>
   <223> Synthetic Primer: nbla20084-1r
<400> 432
   cacacacata caaaggtcag ca 22
<210> 433
   <211> 19
   <212> DNA
   <213> Artificial sequence
<220>
   <223> Synthetic Primer: nbla21081-1f
<400> 433
   tcgaaaaaca cggagagca 19
<210> 434
   <211> 19
   <212> DNA
   <213> Artificial Sequence
<220>
   <223> Synthetic Primer: nbla21081-1r
<400> 434
   cacagaatca tggcggaac 19
<210> 435
   <211> 19
   <212> DNA
   <213> Artificial Sequence
<220>
   <223> Synthetic Primer: nbla21420-1f
<400> 435
   gaagctggga aatggtgag 19
<210> 436
   <211> 20
   <212> DNA
   <213> Artificial Sequence
<220>
   <223> Synthetic Primer: nbla21420-1r
<400> 436
   ggaaatactc atggctgtgg 20
<210> 437
   <211> 19
   <212> DNA
   <213> Artificial Sequence
<220>
   <223> Synthetic Primer: nbla22452-1f
<400> 437
   cagtgggagt caggaagga 19
<210> 438
   <211> 19
   <212> DNA
   <213> Artificial Sequence
<220>
   <223> Synthetic Primer: nbla22452-1r
<400> 438
   acacatgccc agaaagcac 19
<210> 439
   <211> 22
   <212> DNA
   <213> Artificial Sequence
<220>
   <223> Synthetic Primer: nbla22595-1f
<400> 439
   catgaccttc agatagttac cc 22
<210> 440
   <211> 22
   <212> DNA
   <213> Artificial Sequence
<220>
   <223> Synthetic Primer: nbla22595-1r
<400> 440
   attattgggt ggtagacaga ca 22
<210> 441
   <211> 20
   <212> DNA
   <213> Artificial Sequence
<220>
   <223> Synthetic Primer: nbla22676-1f
<400> 441
   gtggtttttg gtggttggag 20
<210> 442
   <211> 20
   <212> DNA
   <213> Artificial Sequence
<220>
   <223> Synthetic Primer: nbla22676-1r

<400> 442
   tactgtggca ggaaggaagg 20
<210> 443
   <211> 22
   <212> DNA
   <213> Artificial Sequence
<220>
   <223> Synthetic Primer: nbla22909-1f
<400> 443
   acacggacat tacaacctta ca 22
<210> 444
   <211> 22
   <212> DNA
   <213> Artificial Sequence
<220>
   <223> Synthetic Primer: nbla22909-1r
<400> 444
   caccaaagag aactcgataa ca 22
<210> 445
   <211> 21
   <212> DNA
   <213> Artificial Sequence
<220>
   <223> Synthetic Primer: nbla24435-1f
<400> 445
   tcagcactgg atttaggatg g 21
<210> 446
   <211> 24
   <212> DNA
   <213> Artificial Sequence
<220>
   <223> Synthetic Primer: nbla24435-1r
<400> 446
   gcagagcagt acattatcag gaag 24
<210> 447
   <211> 22
   <212> DNA
   <213> Artificial Sequence
<220>
   <223> Synthetic Primer: nbla20146-1f
<400> 447
   tccattactc aagtcccaag gt 22
<210> 448
   <211> 20
   <212> DNA
   <213> Artificial Sequence
<220>
   <223> Synthetic Primer: nbla20146-1r
<400> 448
   agcgaagctg tcctgtgttc 20
<210> 449
   <211> 19
   <212> DNA
   <213> Artificial Sequence
<220>
   <223> Synthetic Primer: nbla20170-1f
<400> 449
   gactcgtcgt ttcccacct 19
<210> 450
   <211> 21
   <212> DNA
   <213> Artificial sequence
<220>
   <223> Synthetic Primer: nbla20170-1r
<400> 450
   cctaatgcag ccactcatac c 21
<210> 451
   <211> 22
   <212> DNA
   <213> Artificial Sequence
<220>
   <223> Synthetic Primer: nbla20216-1f
<400> 451
   catctctcca ttagcccaga ag 22
<210> 452
   <211> 21
   <212> DNA
   <213> Artificial Sequence
<220>
   <223> Synthetic Primer: nbla20216-1r
<400> 452
   agaagcgagg agtagggtga g 21
<210> 453
   <211> 21
   <212> DNA
   <213> Artificial Sequence
<220>
   <223> Synthetic Primer: nbla20657-1f
<400> 453
   gacgacttga ctgatgctgt g 21
<210> 454
   <211> 23
   <212> DNA
   <213> Artificial Sequence
<220>
   <223> Synthetic Primer: nbla20657-1r
<400> 454
   caaggacaca attaggaggt gag 23
<210> 455
   <211> 23
   <212> DNA
   <213> Artificial Sequence
<220>
   <223> Synthetic Primer: nbla20688-1f
<400> 455
   ctgtctgttg actctccaac ctc 23
<210> 456
   <211> 21
   <212> DNA
   <213> Artificial Sequence
<220>
   <223> Synthetic Primer: nbla20688-1r
<400> 456
   ccttgggctt ctttcctatc c 21
<210> 457
   <211> 20
   <212> DNA
   <213> Artificial Sequence
<220>
   <223> Synthetic Primer: nbla20755-1f
<400> 457
   ggatggcaga agcatcaaag 20
<210> 458
   <211> 20
   <212> DNA
   <213> Artificial Sequence
<220>
   <223> Synthetic Primer: nbla20755-1r
   <400> 458
   agggtttgtg ggggatagag 20
<210> 459
   <211> 20
   <212> DNA
   <213> Artificial Sequence
<220>
   <223> Synthetic Primer: nbla21013-1f
<400> 459
   tggctgataa tgcaatggtg 20
<210> 460
   <211> 20
   <212> DNA
   <213> Artificial Sequence
<220>
   <223> Synthetic Primer: nbla21013-1r
<400> 460
   gacctttttg gcttctgtgg 20
<210> 461
   <211> 22
   <212> DNA
   <213> Artificial Sequence
<220>
   <223> Synthetic Primer: nbla21172-1f
<400> 461
   aatgctatgt tcagcagggt gt 22
<210> 462
   <211> 18
   <212> DNA
   <213> Artificial Sequence
<220>
   <223> Synthetic Primer: nbla21172-1r
<400> 462
   tgcacttgcg tgatgtgg 18
<210> 463
   <211> 21
   <212> DNA
   <213> Artificial Sequence
<220>
   <223> Synthetic Primer: nbla21200-1f
<400> 463
   accatgagga aaacaactgg a 21
<210> 464
   <211> 24
   <212> DNA
   <213> Artificial Sequence
<220>
   <223> Synthetic Primer: nbla21200-1r
<400> 464
   aatgtcccga ctctattatc tgtg 24
<210> 465
   <211> 20
   <212> DNA
   <213> Artificial sequence
<220>
   <223> Synthetic Primer: nbla21255-1f
<400> 465
   cctgaagccc ctgtgtatct 20
<210> 466
   <211> 20
   <212> DNA
   <213> Artificial Sequence
<220>
   <223> Synthetic Primer: nbla21255-1r
<400> 466
   ccaaaagcca aattctctcc 20
<210> 467
   <211> 19
   <212> DNA
   <213> Artificial Sequence
<220>
   <223> Synthetic Primer: nbla21345-1f
<400> 467
   gtgcaaaccc cctctaaac 19
<210> 468
   <211> 20
   <212> DNA
   <213> Artificial Sequence
<220>
   <223> Synthetic Primer: nbla21345-1r
<400> 468
   tgaccagatg aaacctctcc 20
<210> 469
   <211> 20
   <212> DNA
   <213> Artificial Sequence
<220>
   <223> Synthetic Primer: nbla21410-1f
<400> 469
   cctaaacacc aaagggaagg 20
<210> 470
   <211> 25
   <212> DNA
   <213> Artificial Sequence
<220>
   <223> Synthetic Primer: nbla21410-1r
<400> 470
   ctccatctct atcttctaaa cagca 25
<210> 471
   <211> 22
   <212> DNA
   <213> Artificial Sequence
<220>
   <223> Synthetic Primer: nbla21522-1f
<400> 471
   ttgatgtgcg gactcttaat ct 22
<210> 472
   <211> 21
   <212> DNA
   <213> Artificial Sequence
<220>
   <223> Synthetic Primer: nbla21522-1r
<400> 472
   aggtgggtat tggctttctc t 21
<210> 473
   <211> 20
   <212> DNA
   <213> Artificial Sequence
<220>
   <223> Synthetic Primer: nbla21631-1f
<400> 473
   actttctggg gtttctctgg 20
<210> 474
   <211> 21
   <212> DNA
   <213> Artificial Sequence
<220>
   <223> Synthetic Primer: nbla21631-1r
<400> 474
   gcctctgtaa aatgtggaat g 21
<210> 475
   <211> 20
   <212> DNA
   <213> Artificial Sequence
<220>
   <223> Synthetic Primer: nbla21788-1f
<400> 475
   actcccaaac agtccccttc 20
<210> 476
   <211> 19
   <212> DNA
   <213> Artificial Sequence
<220>
   <223> Synthetic Primer: nbla21788-1r
<400> 476
   tcctggcttt ctccagtcc 19
<210> 477
   <211> 22
   <212> DNA
   <213> Artificial Sequence
<220>
   <223> Synthetic Primer: nbla21897-1f
<400> 477
   caacagtgaa gttgggaaaa ca 22
<210> 478
   <211> 22
   <212> DNA
   <213> Artificial Sequence
<220>
   <223> Synthetic Primer: nbla21897-1r
<400> 478
   ggctctggtt agaagacaaa gg 22
<210> 479
   <211> 19
   <212> DNA
   <213> Artificial Sequence
<220>
   <223> Synthetic Primer: nbla22116-1f
<400> 479
   catccccggt tgaatctct 19
<210> 480
   <211> 21
   <212> DNA
   <213> Artificial Sequence
<220>
   <223> Synthetic Primer: nbla22116-1r
<400> 480
   tcccagtcca catgcaaata c 21
<210> 481
   <211> 20
   <212> DNA
   <213> Artificial Sequence
<220>
   <223> Synthetic Primer: nbla22223-1f
<400> 481
   cattctttgg ggcctctttc 20
<210> 482
   <211> 19
   <212> DNA
   <213> Artificial Sequence
<220>
   <223> Synthetic Primer: nbla22223-1r
   <400> 482
   tggggatctt atggcacct 19
<210> 483
   <211> 20
   <212> DNA
   <213> Artificial Sequence
<220>
   <223> Synthetic Primer: nbla22344-1f
<400> 483
   gtctgaagga acaggggaga 20
<210> 484
   <211> 21
   <212> DNA
   <213> Artificial Sequence
<220>
   <223> Synthetic Primer: nbla22344-1r
<400> 484
   gtctaatggg caaggaagga g 21
<210> 485
   <211> 20
   <212> DNA
   <213> Artificial Sequence
<220>
   <223> Synthetic Primer: nbla22939-1f
<400> 485
   gcaccattct ctggtttcct 20
<210> 486
   <211> 21
   <212> DNA
   <213> Artificial Sequence
<220>
   <223> Synthetic Primer: nbla22939-1r
<400> 486
   cacacctcca tactccatgc t 21
<210> 487
   <211> 22
   <212> DNA
   <213> Artificial Sequence
<220>
   <223> Synthetic Primer: nbla23084-1f
<400> 487
   gcactcgatg actaccaaaa ag 22
<210> 488
   <211> 24
   <212> DNA
   <213> Artificial Sequence
<220>
   <223> Synthetic Primer: nbla23084-1r
<400> 488
   ggataatgag taggttggct aatg 24
<210> 489
   <211> 18
   <212> DNA
   <213> Artificial Sequence
<220>
   <223> Synthetic Primer: nbla23103-1f
<400> 489
   agacggcttt tgcgtttg 18
<210> 490
   <211> 20
   <212> DNA
   <213> Artificial Sequence
<220>
   <223> Synthetic Primer: nbla23103-1r
<400> 490
   agaagttagg gctgggaagg 20
<210> 491
   <211> 18
   <212> DNA
   <213> Artificial Sequence
<220>
   <223> Synthetic Primer: nbla23234-1f
<400> 491
   ccgcatttcc aactgacc 18
<210> 492
   <211> 20
   <212> DNA
   <213> Artificial Sequence
<220>
   <223> Synthetic Primer: nbla23234-1r
<400> 492
   gatcccacaa gtttcccaca 20
<210> 493
   <211> 20
   <212> DNA
   <213> Artificial Sequence
<220>
   <223> Synthetic Primer: nbla23369-1f
<400> 493
   agccccaaat gagaaatcaa 20
<210> 494
   <211> 21
   <212> DNA
   <213> Artificial Sequence
<220>
   <223> Synthetic Primer: nbla23369-1r
<400> 494
   ggagctggag tgataagcag a 21
<210> 495
   <211> 21
   <212> DNA
   <213> Artificial Sequence
<220>
   <223> Synthetic Primer: nbla23436-1f
<400> 495
   cctagaatag ctggggagtg 21
<210> 496
   <211> 21
   <212> DNA
   <213> Artificial Sequence
<220>
   <223> Synthetic Primer: nbla23436-1r
<400> 496
   cgagagcgtc aaagatacag g 21
<210> 497
   <211> 22
   <212> DNA
   <213> Artificial Sequence
<220>
   <223> Synthetic Primer: nbla23511-1f
<400> 497
   aatcaaggac aaagactcac ac 22
<210> 498
   <211> 22
   <212> DNA
   <213> Artificial Sequence
<220>
   <223> Synthetic Primer: nbla23511-1r
<400> 498
   agacacagta aacagggaag ga 22
<210> 499
   <211> 19
   <212> DNA
   <213> Artificial Sequence
<220>
   <223> Synthetic Primer: nbla23860-1f
<400> 499
   gtcagggagg tcatggaag 19
<210> 500
   <211> 22
   <212> DNA
   <213> Artificial Sequence
<220>
   <223> Synthetic Primer: nbla23860-1r
<400> 500
   gctctgataa gcaagtggaa ga 22
<210> 501
   <211> 18
   <212> DNA
   <213> Artificial Sequence
<220>
   <223> Synthetic Primer: nbla23877-1f
<400> 501
   tcctctcagg tgggcttg 18
<210> 502
   <211> 20
   <212> DNA
   <213> Artificial Sequence
<220>
   <223> Synthetic Primer: nbla23877-1r
<400> 502
   ctgtgcttgg atgctgtagg 20
<210> 503
   <211> 23
   <212> DNA
   <213> Artificial Sequence
<220>
   <223> Synthetic Primer: nbla23998-1f
<400> 503
   ctgtatcctg ctgttcatgg tag 23
<210> 504
   <211> 21
   <212> DNA
   <213> Artificial Sequence
<220>
   <223> Synthetic Primer: nbla23998-1r
<400> 504
   agcaaaaagt cgtagcttgg t 21
<210> 505
   <211> 22
   <212> DNA
   <213> Artificial sequence
<220>
   <223> Synthetic Primer: nbla24043-1-1f
<400> 505
   agatggtgat ctggaacatg aa 22
<210> 506
   <211> 21
   <212> DNA
   <213> Artificial Sequence
<220>
   <223> Synthetic Primer: nbla24043-1-1r
<400> 506
   cctattgacc cagcaagaaa c 21
<210> 507
   <211> 21
   <212> DNA
   <213> Artificial Sequence
<220>
   <223> Synthetic Primer: nbla24402-1f
<400> 507
   tgttatgggc acttgaatgg t 21
<210> 508
   <211> 20
   <212> DNA
   <213> Artificial Sequence
<220>
   <223> Synthetic Primer: nbla24402-1r
<400> 508
   tgcagaaagg cagtttgttg 20
<210> 509
   <211> 21
   <212> DNA
   <213> Artificial Sequence
<220>
   <223> Synthetic Primer: nbla24821-1f
<400> 509
   tccctaaagt gattggctgg t 21
<210> 510
   <211> 20
   <212> DNA
   <213> Artificial Sequence
<220>
   <223> Synthetic Primer: nbla24821-1r
<400> 510
   gattggcaca ggttttgagg 20
<210> 511
   <211> 20
   <212> DNA
   <213> Artificial Sequence
<220>
   <223> Synthetic Primer: nbla20026-1f
<400> 511
   atcaaatgtg ctggttgtgg 20
<210> 512
   <211> 19
   <212> DNA
   <213> Artificial Sequence
<220>
   <223> Synthetic Primer: nbla20026-1r
<400> 512
   caagcatctg tgggaagga 19
<210> 513
   <211> 20
   <212> DNA
   <213> Artificial Sequence
<220>
   <223> Synthetic Primer: nbla20421-1f
<400> 513
   tgcaacattt cgttttcctc 20
<210> 514
   <211> 23
   <212> DNA
   <213> Artificial Sequence
<220>
   <223> Synthetic Primer: nbla20421-1r
<400> 514
   gctgttcctg agtctttgct tac 23
<210> 515
   <211> 22
   <212> DNA
   <213> Artificial Sequence
<220>
   <223> Synthetic Primer: nbla22298-1f
<400> 515
   ccaactatgg actatcgggt tc 22
<210> 516
   <211> 20
   <212> DNA
   <213> Artificial Sequence
<220>
   <223> Synthetic Primer: nbla22298-1r
<400> 516
   gtctttcctg gggtcttgct 20
<210> 517
   <211> 20
   <212> DNA
   <213> Artificial Sequence
<220>
   <223> Synthetic Primer: nbla22549-1f
   <400> 517
   atctttccca ctccaccaca 20
<210> 518
   <211> 20
   <212> DNA
   <213> Artificial Sequence
<220>
   <223> Synthetic Primer: nbla22549-1r
<400> 518
   gacaagttcg gggagacaac 20
<210> 519
   <211> 20
   <212> DNA
   <213> Artificial Sequence
<220>
   <223> Synthetic Primer: nbla22256-1f
<400> 519
   gcagccctct tcgtagttcc 20
<210> 520
   <211> 19
   <212> DNA
   <213> Artificial Sequence
<220>
   <223> Synthetic Primer: nbla22256-1r
<400> 520
   ctcgccctgg tctctgtct 19
<210> 521
   <211> 20
   <212> DNA
   <213> Artificial Sequence
<220>
   <223> Synthetic Primer: nbla22968-1f
<400> 521
   cagtgcattt gggagatgtg 20
<210> 522
   <211> 20
   <212> DNA
   <213> Artificial Sequence
<220>
   <223> Synthetic Primer: nbla22968-1r
<400> 522
   ctcaaaacgc caggaaagag 20
<210> 523
   <211> 20
   <212> DNA
   <213> Artificial Sequence
<220>
   <223> Synthetic Primer: nbla24079-1f
<400> 523
   gcctactgga aaagccactc 20
<210> 524
   <211> 19
   <212> DNA
   <213> Artificial Sequence
<220>
   <223> Synthetic Primer: nbla24079-1r
<400> 524
   ctgtgtgcaa atccctgct 19
<210> 525
   <211> 19
   <212> DNA
   <213> Artificial Sequence
<220>
   <223> Synthetic Primer: nbla20211-1f
<400> 525
   acaacatggg caaccacct 19
<210> 526
   <211> 20
   <212> DNA
   <213> Artificial Sequence
<220>
   <223> Synthetic Primer: nbla20211-1r
<400> 526
   gtcgtcatcg tgcaaagtcc 20
<210> 527
   <211> 21
   <212> DNA
   <213> Artificial Sequence
<220>
   <223> Synthetic Primer: nbla20469-1f
<400> 527
   gctcttcacc tcaaatgctc t 21
<210> 528
   <211> 22
   <212> DNA
   <213> Artificial Sequence
<220>
   <223> Synthetic Primer: nbla20469-1r
<400> 528
   gagttagtcc tgctcatggt tc 22
<210> 529
   <211> 20
   <212> DNA
   <213> Artificial Sequence
<220>
   <223> Synthetic Primer: nbla21250-1f
<400> 529
   tcgcctctgc actagctctc 20
<210> 530
   <211> 21
   <212> DNA
   <213> Artificial Sequence
<220>
   <223> Synthetic Primer: nbla21250-1r
<400> 530
   gtgtaaaccc acatgcctcc t 21
<210> 531
   <211> 18
   <212> DNA
   <213> Artificial Sequence
<220>
   <223> Synthetic Primer: nbla22761-1f
<400> 531
   gatgagaacg ccaaagca 18
<210> 532
   <211> 19
   <212> DNA
   <213> Artificial Sequence
<220>
   <223> Synthetic Primer: nbla22761-1r
<400> 532
   aattcggtcc aactcagca 19
<210> 533
   <211> 20
   <212> DNA
   <213> Artificial Sequence
<220>
   <223> Synthetic Primer: nbla23631-1f
<400> 533
   gcctagagca atgtcgtgaa 20
<210> 534
   <211> 21
   <212> DNA
   <213> Artificial sequence
<220>
   <223> Synthetic Primer: nbla23631-1r
<400> 534
   cgcaggaaga taagtgtgag g 21
<210> 535
   <211> 22
   <212> DNA
   <213> Artificial Sequence
<220>
   <223> Synthetic Primer: nbla23711-1f
<400> 535
   gaccctagac cacggacatt ac 22
<210> 536
   <211> 18
   <212> DNA
   <213> Artificial Sequence
<220>
   <223> Synthetic Primer: nbla23711-1r
<400> 536
   cgctcaccac catcaaca 18
<210> 537
   <211> 20
   <212> DNA
   <213> Artificial Sequence
<220>
   <223> Synthetic Primer: nbla24532-1f
<400> 537
   agggctcagt catggatagg 20
<210> 538
   <211> 20
   <212> DNA
   <213> Artificial Sequence
<220>
   <223> Synthetic Primer: nbla24532-1r
<400> 538
   gctgggcaca cacagtaaag 20
<210> 539
   <211> 20
   <212> DNA
   <213> Artificial Sequence
<220>
   <223> Synthetic Primer: nbla24951-1f
<400> 539
   tgttttctgc atcaggcttc 20
<210> 540
   <211> 21
   <212> DNA
   <213> Artificial Sequence
<220>
   <223> Synthetic Primer: nbla24951-1r
<400> 540
   catttggttc ccacttcttg t 21
<210> 541
   <211> 26
   <212> DNA
   <213> Artificial Sequence
<220>
   <223> Synthetic Primer: nbla24348-1f
<400> 541
   gacagagtag aagaggaaca tgaaga 26
<210> 542
   <211> 21
   <212> DNA
   <213> Artificial Sequence
<220>
   <223> Synthetic Primer: nbla24348-1r
<400> 542
   catcagtttg tgggaaggtt g 21
<210> 543
   <211> 18
   <212> DNA
   <213> Artificial Sequence
<220>
   <223> Synthetic Primer: nbla24686-1f
<400> 543
   tcgaaaagcc tgcggtgt 18
<210> 544
   <211> 20
   <212> DNA
   <213> Artificial Sequence
<220>
   <223> Synthetic Primer: nbla24686-1r
<400> 544
   taggcggggc tgagtgtatc 20
<210> 545
   <211> 20
   <212> DNA
   <213> Artificial Sequence
<220>
   <223> Synthetic Primer: nbla24756-1f
<400> 545
   ttgactgtgc ttgagaggtg 20
<210> 546
   <211> 20
   <212> DNA
   <213> Artificial Sequence
<220>
   <223> Synthetic Primer: nbla24756-1r
<400> 546
   cttgttggtg gagaaactgg 20
<210> 547
   <211> 20
   <212> DNA
   <213> Artificial Sequence
<220>
   <223> Synthetic Primer: nbla24521-1f
<400> 547
   gccaaaatgc aaaggagaag 20
<210> 548
   <211> 20
   <212> DNA
   <213> Artificial Sequence
<220>
   <223> Synthetic Primer: nbla24521-1r
<400> 548
   tatggtccca aaggtggatg 20
<210> 549
   <211> 20
   <212> DNA
   <213> Artificial Sequence
<220>
   <223> Synthetic Primer: nbla24526-1f
<400> 549
   tgaaatggca gagaatggaa 20
<210> 550
   <211> 22
   <212> DNA
   <213> Artificial Sequence
<220>
   <223> Synthetic Primer: nbla24526-1r
<400> 550
   tccagagaaa aatactgcaa gg 22
<210> 551
   <211> 19
   <212> DNA
   <213> Artificial Sequence
<220>
   <223> Synthetic Primer: nbla21212-1f
<400> 551
   ctggggattt tcgttgttg 19
<210> 552
   <211> 21
   <212> DNA
   <213> Artificial Sequence
<220>
   <223> Synthetic Primer: nbla21212-1r
<400> 552
   tgtttctggg ctgtttatcc t 21
<210> 553
   <211> 21
   <212> DNA
   <213> Artificial Sequence
<220>
   <223> Synthetic Primer: nbla20604-1f
<400> 553
   atcgtcttca gatggagctt g 21
<210> 554
   <211> 19
   <212> DNA
   <213> Artificial Sequence
<220>
   <223> Synthetic Primer: nbla20604-1r
<400> 554
   atgtgacccg acgttgatg 19
<210> 555
   <211> 20
   <212> DNA
   <213> Artificial Sequence
<220>
   <223> Synthetic Primer: nbla21226-1f
<400> 555
   gcctcagtgg atggtaaatg 20
<210> 556
   <211> 20
   <212> DNA
   <213> Artificial Sequence
<220>
   <223> Synthetic Primer: nbla21226-1r
<400> 556
   ccaagaagca gaaaagcaag 20
<210> 557
   <211> 20
   <212> DNA
   <213> Artificial Sequence
<220>
   <223> Synthetic Primer: nbla21928-1f
<400> 557
   ctcaggtttt ctgcatagtt 20

<210> 558
   <211> 20
   <212> DNA
   <213> Artificial Sequence
<220>
   <223> Synthetic Primer: nbla21928-1r
<400> 558
   tgatagtttc caaggtaagg 20
<210> 559
   <211> 24
   <212> DNA
   <213> Artificial Sequence
<220>
   <223> Synthetic Primer: nbla22643-1f
<400> 559
   ctggtttata ttggatgaga gtgg 24
<210> 560
   <211> 22
   <212> DNA
   <213> Artificial Sequence
<220>
   <223> Synthetic Primer: nbla22643-1r
<400> 560
   agatgaaatg gaagctcaca ag 22
<210> 561
   <211> 20
   <212> DNA
   <213> Artificial Sequence
<220>
   <223> Synthetic Primer: nbla23649-1f
<400> 561
   tgtatccagt tgcccaaggt 20
<210> 562
   <211> 21
   <212> DNA
   <213> Artificial Sequence
<220>
   <223> Synthetic Primer: nbla23649-1r
<400> 562
   cacagcagaa gccaaagaaa g 21
<210> 563
   <211> 20
   <212> DNA
   <213> Artificial Sequence
<220>
   <223> Synthetic Primer: nbla24468-1f
<400> 563
   cgacacaggt tctgcttcct 20
<210> 564
   <211> 20
   <212> DNA
   <213> Artificial Sequence
<220>
   <223> Synthetic Primer: nbla24468-1r
<400> 564
   gccttctctc ctccatcctt 20
<210> 565
   <211> 23
   <212> DNA
   <213> Artificial Sequence
<220>
   <223> Synthetic Primer: nbla20874r1-1f
<400> 565
   acccagctct tatcccttaa tct 23
<210> 566
   <211> 21
   <212> DNA
   <213> Artificial Sequence
<220>
   <223> Synthetic Primer: nbla20874r1-1r
<400> 566
   gccttcacaa caaagttctc c 21
<210> 567
   <211> 20
   <212> DNA
   <213> Artificial Sequence
<220>
   <223> Synthetic Primer: nbla20134-1f
<400> 567
   gtaactaggg ggccacattc 20
<210> 568
   <211> 20
   <212> DNA
   <213> Artificial Sequence
<220>
   <223> Synthetic Primer: nbla20134-1r
<400> 568
   gacaacacgt ctgcaccttc 20
<210> 569
   <211> 22
   <212> DNA
   <213> Artificial Sequence
<220>
   <223> Synthetic Primer: nbla20181-1f
<400> 569
   cgtgtaaaga aacccaaagg ag 22
<210> 570
   <211> 20
   <212> DNA
   <213> Artificial Sequence
<220>
   <223> Synthetic Primer: nbla20181-1r
<400> 570
   tctacccagc ggagtttgag 20
<210> 571
   <211> 22
   <212> DNA
   <213> Artificial Sequence
<220>
   <223> Synthetic Primer: nbla20276-1f
<400> 571
   ctatctccca ggattttgct ct 22
<210> 572
   <211> 19
   <212> DNA
   <213> Artificial sequence
<220>
   <223> Synthetic Primer: nbla20276-1r
<400> 572
   ccaggaagct ggaacctct 19
<210> 573
   <211> 21
   <212> DNA
   <213> Artificial Sequence
<220>
   <223> Synthetic Primer: nbla20709-1f
<400> 573
   gattagttgg gacctgcctt g 21
<210> 574
   <211> 20
   <212> DNA
   <213> Artificial Sequence
<220>
   <223> Synthetic Primer: nbla20709-1r
<400> 574
   caatgctttt tcggaggaga 20
<210> 575
   <211> 23
   <212> DNA
   <213> Artificial Sequence
<220>
   <223> Synthetic Primer: nbla20782-1f
<400> 575
   caaagatggg aacaaccagt atc 23
<210> 576
   <211> 24
   <212> DNA
   <213> Artificial Sequence
<220>
   <223> Synthetic Primer: nbla20782-1r
<400> 576
   actgtctatg aagtaaggca agca 24
<210> 577
   <211> 21
   <212> DNA
   <213> Artificial Sequence
<220>
   <223> Synthetic Primer: nbla20788-1f
<400> 577
   ctggactcag gagaggagac a 21
<210> 578
   <211> 19
   <212> DNA
   <213> Artificial Sequence
<220>
   <223> Synthetic Primer: nbla20788-1r
<400> 578
   gaaagccacc caaaccaag 19
<210> 579
   <211> 21
   <212> DNA
   <213> Artificial Sequence
<220>
   <223> Synthetic primer: nbla21046-1f
<400> 579
   tcttggaggt gtgcagagat g 21
<210> 580
   <211> 20
   <212> DNA
   <213> Artificial Sequence
<220>
   <223> Synthetic Primer: nbla21046-1r
<400> 580
   tctgtttcgg gctggtagtg 20
<210> 581
   <211> 24
   <212> DNA
   <213> Artificial Sequence
<220>
   <223> Synthetic Primer: nbla21122-1f
<400> 581
   ctagaagctc catattccct cttc 24
<210> 582
   <211> 21
   <212> DNA
   <213> Artificial Sequence
<220>
   <223> Synthetic Primer: nbla21122-1r
<400> 582
   ggttaagaac gtgatgcctg t 21
<210> 583
   <211> 20
   <212> DNA
   <213> Artificial Sequence
<220>
   <223> Synthetic Primer: nbla21211r1-1f
<400> 583
   cttcagctcc tttcccaatc 20
<210> 584
   <211> 20
   <212> DNA
   <213> Artificial Sequence
<220>
   <223> Synthetic Primer: nbla21211r1-1f
<400> 584
   accatgtctt gtggtggtgt 20
<210> 585
   <211> 19
   <212> DNA
   <213> Artificial Sequence
<220>
   <223> Synthetic Primer: nbla21233d-1f
<400> 585
   atggggaatg gtctgcttc 19
<210> 586
   <211> 21
   <212> DNA
   <213> Artificial Sequence
<220>
   <223> Synthetic Primer: nbla21233d-1r
<400> 586
   ctccctcttc caaggatgtc t 21
<210> 587
   <211> 21
   <212> DNA
   <213> Artificial Sequence
<220>
   <223> Synthetic Primer: nbla21375-1f
<400> 587
   ctttgccatc ctgaaagaga g 21
<210> 588
   <211> 20
   <212> DNA
   <213> Artificial Sequence
<220>
   <223> Synthetic Primer: nbla21375-1r
<400> 588
   gtagcagacg atgtggtgga 20
<210> 589
   <211> 20
   <212> DNA
   <213> Artificial Sequence
<220>
   <223> Synthetic Primer: nbla21524-1f
<400> 589
   cctcgaaaga tccctgattg 20
<210> 590
   <211> 21
   <212> DNA
   <213> Artificial Sequence
<220>
   <223> Synthetic Primer: nbla21524-1r
<400> 590
   tcccagctcc agaacttacc t 21
<210> 591
   <211> 20
   <212> DNA
   <213> Artificial Sequence
<220>
   <223> Synthetic Primer: nbla21843-1f
<400> 591
   ccatattggg agacaccatc 20
<210> 592
   <211> 18
   <212> DNA
   <213> Artificial Sequence
<220>
   <223> Synthetic Primer: nbla21843-1r
<400> 592
   atcctgaccc tgcacctt 18
<210> 593
   <211> 21
   <212> DNA
   <213> Artificial Sequence
<220>
   <223> Synthetic Primer: nbla21934-1f
<400> 593
   gattttcagg tgggagattt g 21
<210> 594
   <211> 20
   <212> DNA
   <213> Artificial Sequence
<220>
   <223> Synthetic Primer: nbla21934-1r
<400> 594
   tctgttttgt gcctttttgg 20
<210> 595
   <211> 23
   <212> DNA
   <213> Artificial Sequence
<220>
   <223> Synthetic Primer: nbla22153-1f
<400> 595
   gctgctgaag aaatagtgga ttg 23
<210> 596
   <211> 20
   <212> DNA
   <213> Artificial Sequence
<220>
   <223> synthetic Primer: nbla22153-1r
<400> 596
   acgataggtg gcattgaggt 20
<210> 597
   <211> 25
   <212> DNA
   <213> Artificial Sequence
<220>
   <223> Synthetic Primer: nbla22382-1f
<400> 597
   gtgcctgtga tattgagttt aagga 25
<210> 598
<211> 24
   <212> DNA
   <213> Artificial Sequence
<220>
   <223> Synthetic Primer: nbla22382-1r
<400> 598
   tagtggagat gggactacaa aagg 24
<210> 599
   <211> 18
   <212> DNA
   <213> Artificial Sequence
<220>
   <223> Synthetic Primer: nbla22571-1f
<400> 599
   gtcatagtgc ccaccaca 18
<210> 600
   <211> 18
   <212> DNA
   <213> Artificial Sequence
<220>
   <223> Synthetic Primer: nbla22571-1r
<400> 600
   ttgcacagga gaaatgga 18
<210> 601
   <211> 20
   <212> DNA
   <213> Artificial Sequence
<220>
   <223> Synthetic Primer: nbla22789-1f
<400> 601
   gctaagggga tgaagcaaac 20
<210> 602
   <211> 19
   <212> DNA
   <213> Artificial Sequence
<220>
   <223> Synthetic Primer: nbla22789-1r
<400> 602
   agcagagcca ctccacaga 19
<210> 603
   <211> 20
   <212> DNA
   <213> Artificial Sequence
<220>
   <223> Synthetic Primer: nbla23060-1f
<400> 603
   catgcgggag agagaatgag 20
<210> 604
   <211> 22
   <212> DNA
   <213> Artificial Sequence
<220>
   <223> Synthetic Primer: nbla23060-1r
<400> 604
   tcacctttag gcaatgaaga gg 22
<210> 605
   <211> 21
   <212> DNA
   <213> Artificial Sequence
<220>
   <223> Synthetic Primer: nbla23218-1f
<400> 605
   ccttgactct ctctcccctt c 21
<210> 606
   <211> 18
   <212> DNA
   <213> Artificial Sequence
<220>
   <223> Synthetic Primer: nbla23218-1r
<400> 606
   gacacggttc tgcctgct 18
<210> 607
   <211> 20
   <212> DNA
   <213> Artificial Sequence
<220>
   <223> Synthetic Primer: nbla23545-1f
<400> 607
   cattcactcc tttggcctct 20
<210> 608
   <211> 20
   <212> DNA
   <213> Artificial Sequence
<220>
   <223> Synthetic Primer: nbla23545-1r
<400> 608
   agcctcatgt tcgcatttct 20
<210> 609
   <211> 21
   <212> DNA
   <213> Artificial Sequence
<220>
   <223> Synthetic Primer: nbla23653-1f
<400> 609
   acccaaagct agggaatcaa c 21
<210> 610
   <211> 19
   <212> DNA
   <213> Artificial Sequence
<220>
   <223> Synthetic Primer: nbla23653-1r
<400> 610
   tcagaaacac ggccaaaac 19
<210> 611
   <211> 20
   <212> DNA
   <213> Artificial Sequence
<220>
   <223> Synthetic Primer: nbla23666-1f
<400> 611
   cgtggtggtg tgtattttgg 20
<210> 612
   <211> 21
   <212> DNA
   <213> Artificial Sequence
<220>
   <223> Synthetic Primer: nbla23666-1r
<400> 612
   gtatcgcggt gacataaaag g 21
<210> 613
   <211> 20
   <212> DNA
   <213> Artificial Sequence
<220>
   <223> Synthetic Primer: nbla23760-1f
<400> 613
   attgaggcga aagtcaaacc 20
<210> 614
   <211> 21
   <212> DNA
   <213> Artificial Sequence
<220>
   <223> Synthetic Primer: nbla23760-1r
<400> 614
   acaggactga aagaaccagc a 21
<210> 615
   <211> 21
   <212> DNA
   <213> Artificial Sequence
<220>
   <223> Synthetic Primer: nbla23830-1f
<400> 615
   tatagtgacg ggagggacag a 21
<210> 616
   <211> 19
   <212> DNA
   <213> Artificial sequence
<220>
   <223> Synthetic Primer: nbla23830-1r
<400> 616
   cggatggaag tcatggaag 19
<210> 617
   <211> 21
   <212> DNA
   <213> Artificial Sequence
<220>
   <223> Synthetic Primer: nbla23942-1f
<400> 617
   cgaagaagag ccagaatgag a 21
<210> 618
   <211> 20
   <212> DNA
   <213> Artificial Sequence
<220>
   <223> Synthetic Primer: nbla23942-1r
<400> 618
   tggggaaaga ttttgtgagg 20
<210> 619
   <211> 21
   <212> DNA
   <213> Artificial Sequence
<220>
   <223> Synthetic Primer: nbla24131-1f
<400> 619
   ggcacataac cagtttccaa g 21
<210> 620
   <211> 21
   <212> DNA
   <213> Artificial Sequence
<220>
   <223> Synthetic Primer: nbla24131-1r
<400> 620
   gccaccaaaa tgtagcaaaa g 21
<210> 621
   <211> 18
   <212> DNA
   <213> Artificial Sequence
<220>
   <223> Synthetic Primer: nbla24908-1f
<400> 621
   acaaggccat cctgcaac 18
<210> 622
   <211> 20
   <212> DNA
   <213> Artificial sequence
<220>
   <223> Synthetic Primer: nbla24908-1r
<400> 622
   ctgatctggt tctccgtcct 20
<210> 623
   <211> 21
   <212> DNA
   <213> Artificial Sequence
<220>
   <223> Synthetic Primer: nbla20125-1f
<400> 623
   tctcccttcg ccttcttcta c 21
<210> 624
   <211> 20
   <212> DNA
   <213> Artificial Sequence
<220>
   <223> Synthetic Primer: nbla20125-1r
<400> 624
   actggttccg atgtgttgct 20
<210> 625
   <211> 20
   <212> DNA
   <213> Artificial Sequence
<220>
   <223> synthetic Primer: nbla20231d-1f
<400> 625
   tagggtgctg gatggtagag 20
<210> 626
   <211> 20
   <212> DNA
   <213> Artificial sequence
<220>
   <223> Synthetic Primer: nbla20231-1r
<400> 626
   catcaacttc tgcaaggaca 20
<210> 627
   <211> 20
   <212> DNA
   <213> Artificial Sequence
<220>
   <223> Synthetic Primer: nbla20268-1f
<400> 627
   atcaggacag atggggaaca 20
<210> 628
   <211> 23
   <212> DNA
   <213> Artificial Sequence
<220>
   <223> Synthetic Primer: nbla20268-1r
<400> 628
   tcagagagaa ggatttggat gag 23
<210> 629
   <211> 22
   <212> DNA
   <213> Artificial Sequence
<220>
   <223> Synthetic Primer: nbla20395-1f
<400> 629
   tttcctgagt gtgtgagatg aa 22
<210> 630
   <211> 19
   <212> DNA
   <213> Artificial Sequence
<220>
   <223> Synthetic Primer: nbla20395-1r
<400> 630
   taggccaggg acagaaatg 19
<210> 631
   <211> 20
   <212> DNA
   <213> Artificial Sequence
<220>
   <223> Synthetic Primer: nbla23973-1f
<400> 631
   agaaaagaaa cggcaacgag 20
<210> 632
   <211> 20
   <212> DNA
   <213> Artificial Sequence
<220>
   <223> Synthetic Primer: nbla23973-1r
<400> 632
   ggtgggtgag aagatgatgg 20
<210> 633
   <211> 21
   <212> DNA
   <213> Artificial Sequence
<220>
   <223> Synthetic Primer: nbla24041-1f
<400> 633
   cagtaaaggc aagggaagag g 21
<210> 634
   <211> 22
   <212> DNA
   <213> Artificial Sequence
<220>
   <223> Synthetic Primer: nbla24041-1r
<400> 634
   cttgggaaac aaaagtccag ag 22
<210> 635
   <211> 20
   <212> DNA
   <213> Artificial Sequence
<220>
   <223> Synthetic Primer: nbla24082-1f
<400> 635
   cgcaatactc atttgctgtg 20
<210> 636
   <211> 24
   <212> DNA
   <213> Artificial Sequence
<220>
   <223> Synthetic Primer: nbla24082-1r
<400> 636
   tgtagacttc tggtaacaat ctgg 24
<210> 637
   <211> 21
   <212> DNA
   <213> Artificial Sequence
<220>
   <223> Synthetic Primer: nbla24239-1f
<400> 637
   gaaggaattg agagcacagc a 21
<210> 638
   <211> 19
   <212> DNA
   <213> Artificial Sequence
<220>
   <223> Synthetic Primer: nbla24239-1r
<400> 638
   atccctgcat caccacctc 19
<210> 639
   <211> 25
   <212> DNA
   <213> Artificial Sequence
<220>
   <223> Synthetic Primer: nbla20638-1f
<400> 639
   gtctgtcaac aaatacacca aaacc 25
<210> 640
   <211> 20
   <212> DNA
   <213> Artificial Sequence
<220>
   <223> Synthetic Primer: nbla20638-1r
<400> 640
   ttatccaact ccccaaagca 20
<210> 641
   <211> 21
   <212> DNA
   <213> Artificial Sequence
<220>
   <223> Synthetic Primer: nbla20765-1f
<400> 641
   tgaaagcgtc tgttgttacc c 21
<210> 642
   <211> 23
   <212> DNA
   <213> Artificial Sequence
<220>
   <223> Synthetic Primer: nbla20765-1r
<400> 642
   tgtcggaact catctacctc aac 23
<210> 643
<211> 21
<212> DNA
   <213> Artificial Sequence
<220>
   <223> Synthetic Primer: nbla20789-1f
<400> 643
   tgtcctgctt cttgtttgtg g 21
<210> 644
   <211> 20
   <212> DNA
   <213> Artificial Sequence
<220>
   <223> Synthetic Primer: nbla20789-1r
<400> 644
   ggcgctcctt gtgtagtgaa 20
<210> 645
   <211> 21
   <212> DNA
   <213> Artificial Sequence
<220>
   <223> Synthetic Primer: nbla20792-1f
<400> 645
   ctttgtaccc ctgcctaatc c 21
<210> 646
   <211> 20
   <212> DNA
   <213> Artificial Sequence
<220>
   <223> Synthetic Primer: nbla20792-1r
<400> 646
   aatacccaac ccacccttgt 20
<210> 647
   <211> 19
   <212> DNA
   <213> Artificial Sequence
<220>
   <223> Synthetic Primer: nbla20798-1f
<400> 647
   gctgcctcag aacatttgg 19
<210> 648
   <211> 20
   <212> DNA
   <213> Artificial Sequence
<220>
   <223> Synthetic Primer: nbla20798-1r
<400> 648
   ggccctccac cataaataga 20
<210> 649
   <211> 20
   <212> DNA
   <213> Artificial Sequence
<220>
   <223> Synthetic Primer: nbla21024-1f
<400> 649
   tgccacatac atggaacacc 20
<210> 650
   <211> 21
   <212> DNA
   <213> Artificial Sequence
<220>
   <223> Synthetic Primer: nbla21024-1r
<400> 650
   catgctacac gggacctact c 21
<210> 651
   <211> 20
   <212> DNA
   <213> Artificial Sequence
<220>
   <223> Synthetic Primer: nbla24363-1f
<400> 651
   caaatggttg ctggtctcct 20
<210> 652
   <211> 22
   <212> DNA
   <213> Artificial Sequence
<220>
   <223> Synthetic Primer: nbla24363-1r
<400> 652
   cttccctcct cttgctacct ct 22
<210> 653
   <211> 19
   <212> DNA
   <213> Artificial Sequence
<220>
   <223> Synthetic Primer: nbla24622-1f
<400> 653
   tgccagggaa cagagagtg 19
<210> 654
   <211> 22
   <212> DNA
   <213> Artificial Sequence
<220>
   <223> Synthetic Primer: nbla24622-1r
<400> 654
   tgtaaaaggg acctgagagg ag 22
<210> 655
   <211> 20
   <212> DNA
   <213> Artificial sequence
<220>
   <223> Synthetic Primer: nbla24646-1f
<400> 655
   tgcaggcgta caactaacaa 20
<210> 656
   <211> 20
   <212> DNA
   <213> Artificial Sequence
<220>
   <223> Synthetic Primer: nbla24646-1r
<400> 656
   tggtctgcga gaaatcaaac 20
<210> 657
   <211> 20
   <212> DNA
   <213> Artificial sequence
<220>
   <223> Synthetic Primer: nbla24672-1f
<400> 657
   ccagcctctg tggtctttgt 20
<210> 658
   <211> 19
   <212> DNA
   <213> Artificial sequence
<220>
   <223> Synthetic Primer: nbla24672-1r
<400> 658
   cacctaacgc cacgtcttc 19
<210> 659
   <211> 21
   <212> DNA
   <213> Artificial Sequence
<220>
   <223> Synthetic Primer: nbla21077-1f
<400> 659
   tgaaggatgt accccagaga g 21
<210> 660
   <211> 20
   <212> DNA
   <213> Artificial Sequence
<220>
   <223> Synthetic Primer: nbla21077-1r
<400> 660
   gataaggcca cagcaaaagg 20
<210> 661
   <211> 22
   <212> DNA
   <213> Artificial Sequence
<220> Synthetic Primer: nbla21089-1f
   <223> Synthetic Primer: nbla21089-1f
<400> 661
   cacgctcaag ttcattagca ca 22
<210> 662
   <211> 20
   <212> DNA
   <213> Artificial Sequence
<220>
   <223> Synthetic Primer: nbla21089-1r
<400> 662
   tgtccaatca ccgcagtttc 20
<210> 663
   <211> 21
   <212> DNA
   <213> Artificial Sequence
<220>
   <223> Synthetic Primer: nbla21130-1f
<400> 663
   agcttgacct ctccagaaca c 21
<210> 664
   <211> 24
   <212> DNA
   <213> Artificial Sequence
<220>
   <223> Synthetic Primer: nbla21130-1r
<400> 664
   ggttgtctct ttaattgtcc cttc 24
<210> 665
   <211> 22
   <212> DNA
   <213> Artificial Sequence
<220>
   <223> Synthetic Primer: nbla21266-1f
<400> 665
   gacagagtgc tcagattgtt gg 22
<210> 666
   <211> 20
   <212> DNA
   <213> Artificial Sequence
<220>
   <223> Synthetic Primer: nbla21266-1r
<400> 666
   cctagaggaa ggtgggctgt 20
<210> 667
   <211> 20
   <212> DNA
   <213> Artificial Sequence
<220>
   <223> Synthetic Primer: nbla24709-1f
<400> 667
   cagcctccca actcattttc 20
<210> 668
   <211> 18
   <212> DNA
   <213> Artificial Sequence
<220>
   <223> Synthetic Primer: nbla24709-1r
<400> 668
   tgggctcctt ctgcaatc 18
<210> 669
   <211> 20
   <212> DNA
   <213> Artificial Sequence
<220>
   <223> Synthetic Primer: nbla24748-1f
<400> 669
   cggtttgccc tgtttttatg 20
<210> 670
   <211> 27
   <212> DNA
   <213> Artificial Sequence
<220>
   <223> Synthetic Primer: nbla24748-1r
<400> 670
   gctcaactac tatcttggga tctcttt 27
<210> 671
   <211> 22
   <212> DNA
   <213> Artificial Sequence
<220>
   <223> Synthetic Primer: nbla24831-1f
<400> 671
   gcagtttctt catcaaaggt gt 22
<210> 672
   <211> 22
   <212> DNA
   <213> Artificial Sequence
<220>
   <223> Synthetic Primer: nbla24831-1r
<400> 672
   tctatcccat gtgttgtgtt tg 22

<210> 673
   <211> 22
   <212> DNA
   <213> Artificial Sequence
<220>
   <223> Synthetic Primer: nbla24972-1f
<400> 673
   ggtattttca accaccagga ac 22
<210> 674
   <211> 22
   <212> DNA
   <213> Artificial Sequence
<220>
   <223> Synthetic Primer: nbla24972-1r
<400> 674
   aggatagcac cattcatcac ct 22
<210> 675
   <211> 20
   <212> DNA
   <213> Artificial Sequence
<220>
   <223> Synthetic Primer: nbla21413-1f
<400> 675
   tgctggggag tatgaagaca 20
<210> 676
   <211> 21
   <212> DNA
   <213> Artificial Sequence
<220>
   <223> Synthetic Primer: nbla21413-1r
<400> 676
   ctttatttgc agccattcca c 21
<210> 677
   <211> 22
   <212> DNA
   <213> Artificial Sequence
<220>
   <223> Synthetic Primer: nbla21520-1f
<400> 677
   tggaacctac gtctttccct ac 22
<210> 678
   <211> 20
   <212> DNA
   <213> Artificial Sequence
<220>
   <223> Synthetic Primer: nbla21520-1r
<400> 678
   acagctcatg tctgcctcct 20
<210> 679
   <211> 24
   <212> DNA
   <213> Artificial Sequence
<220>
   <223> Synthetic Primer: nbla21936-1f
<400> 679
   ccacaggaag ctatcaaaga aaag 24
<210> 680
   <211> 22
   <212> DNA
   <213> Artificial Sequence
<220>
   <223> Synthetic Primer: nbla21936-1r
<400> 680
   tacactggtg gagaggaaca ga 22
<210> 681
   <211> 21
   <212> DNA
   <213> Artificial Sequence
<220>
   <223> Synthetic Primer: nbla22028-1f
<400> 681
   tgtagggacc agaacacgag a 21
<210> 682
   <211> 20
   <212> DNA
   <213> Artificial Sequence
<220>
   <223> Synthetic Primer: nbla22028-1r
<400> 682
   cagaagcaga gacccttcca 20
<210> 683
   <211> 22
   <212> DNA
   <213> Artificial Sequence
<220>
   <223> Synthetic Primer: nbla22093-1d-1f
<400> 683
   agacactatc acgagaccca ga 22
<210> 684
   <211> 22
   <212> DNA
   <213> Artificial Sequence
<220>
   <223> Synthetic Primer: nbla22093-1d-1r
<400> 684
   agacactatc acgagaccca ga 22
<210> 685
   <211> 22
   <212> DNA
   <213> Artificial Sequence
<220>
   <223> Synthetic Primer: nbla22218-1f
<400> 685
   ggctcaggaa gagaagaaga tg 22
<210> 686
   <211> 20
   <212> DNA
   <213> Artificial Sequence
<220>
   <223> Synthetic Primer: nbla22218-1r
<400> 686
   atccaaaagg ggccatagag 20
<210> 687
   <211> 23
   <212> DNA
   <213> Artificial Sequence
<220>
   <223> Synthetic Primer: nbla22451-1f
<400> 687
   tcctcaataa taagcctgtg tcc 23
<210> 688
   <211> 20
   <212> DNA
   <213> Artificial Sequence
<220>
   <223> Synthetic Primer: nbla22451-1r
<400> 688
   tccctgtgtt tgcttttcac 20
<210> 689
   <211> 21
   <212> DNA
   <213> Artificial Sequence
<220>
   <223> Synthetic Primer: nbla22455d-1f
<400> 689
   caatggtgga aaccagtaag g 21
<210> 690
   <211> 20
   <212> DNA
   <213> Artificial Sequence
<220>
   <223> Synthetic Primer: nbla22455d-1r
<400> 690
   agtttgggga acagtgcaag 20
<210> 691
   <211> 20
   <212> DNA
   <213> Artificial Sequence
<220>
   <223> Synthetic Primer: nbla22464-1f
<400> 691
   ggacaaggca gaggtgaatg 20
<210> 692
   <211> 20
   <212> DNA
   <213> Artificial Sequence
<220>
   <223> Synthetic Primer: nbla22464-1r
<400> 692
   cgtgtaagga cggtgattgg 20
<210> 693
   <211> 22
   <212> DNA
   <213> Artificial Sequence
<220>
   <223> Synthetic Primer: nbla22465-1f
<400> 693
   gtcactttgc ttttgctcgt ct 22
<210> 694
   <211> 20
   <212> DNA
   <213> Artificial Sequence
<220>
   <223> Synthetic Primer: nbla22465-1r
<400> 694
   tgggaacttg aaccaccatc 20
<210> 695
   <211> 18
   <212> DNA
   <213> Artificial Sequence
<220>
   <223> Synthetic Primer: nbla22487-1f
<400> 695
   aacgcctcgt cctgctct 18
<210> 696
   <211> 18
   <212> DNA
   <213> Artificial Sequence
<220>
   <223> Synthetic Primer: nbla22487-1r
<400> 696
   ccggtgggct aaaatggt 18
<210> 697
   <211> 19
   <212> DNA
   <213> Artificial Sequence
<220>
   <223> Synthetic Primer: nbla22669-1f
<400> 697
   ccgaggaaga agagcaagg 19
<210> 698
   <211> 18
   <212> DNA
   <213> Artificial Sequence
<220>
   <223> Synthetic Primer: nbla22669-1r
<400> 698
   ccaagcagat ggcacaca 18
<210> 699
   <211> 19
   <212> DNA
   <213> Artificial Sequence
<220>
   <223> Synthetic Primer: nbla22726-1f
<400> 699
   gcccagcaac aagacagag 19
<210> 700
   <211> 19
   <212> DNA
   <213> Artificial Sequence
<220>
   <223> Synthetic Primer: nbla22726-1r
<400> 700
   ctgcaaaatg ggagactgg 19
<210> 701
   <211> 20
   <212> DNA
   <213> Artificial Sequence
<220>
   <223> Synthetic Primer: nbla22886-1f
<400> 701
   gcacagggaa ccatcagaac 20
<210> 702
   <211> 20
   <212> DNA
   <213> Artificial Sequence
<220>
   <223> Synthetic Primer: nbla22886-1r
<400> 702
   caccaccaac gtcattcctc 20
<210> 703
   <211> 20
   <212> DNA
   <213> Artificial Sequence
<220>
   <223> synthetic Primer: nbla23012-1f
<400> 703
   aggagaaaca ggagcgagag 20
<210> 704
   <211> 18
   <212> DNA
   <213> Artificial Sequence
<220>
   <223> Synthetic Primer: nbla23012-1r
<400> 704
   ttgctgagat gcgtggag 18
<210> 705
   <211> 20
   <212> DNA
   <213> Artificial Sequence
<220>
   <223> Synthetic Primer: nbla23038-1f
<400> 705
   gaaacctcag catggagaca 20
<210> 706
   <211> 23
   <212> DNA
   <213> Artificial Sequence
<220>
   <223> Synthetic Primer: nbla23038-1r
<400> 706
   ccaatcactc actcacaaaa gag 23
<210> 707
   <211> 20
   <212> DNA
   <213> Artificial Sequence
<220>
   <223> Synthetic Primer: nbla23718-1f
<400> 707
   atggaaaact tgcctgctct 20
<210> 708
   <211> 22
   <212> DNA
   <213> Artificial Sequence
<220>
   <223> Synthetic Primer: nbla23718-1r
<400> 708
   tcacccacac tttatctcca ac 22
<210> 709
   <211> 22
   <212> DNA
   <213> Artificial Sequence
<220>
   <223> Synthetic Primer: nbla23719-1f
<400> 709
   ctgaacagaa aagcacaacc tc 22
<210> 710
   <211> 20
   <212> DNA
   <213> Artificial Sequence
<220>
   <223> Synthetic Primer: nbla23719-1r
<400> 710
   acaggcgggt caaatctatc 20
<210> 711
   <211> 20
   <212> DNA
   <213> Artificial Sequence
<220>
   <223> Synthetic Primer: nbla23951-1f
<400> 711
   cctgctgttc tggttccttg 20
<210> 712
   <211> 20
   <212> DNA
   <213> Artificial Sequence
<220>
   <223> Synthetic Primer: nbla23951-1r
<400> 712
   agcctgggtc tttcatctgg 20
<210> 713
   <211> 20
   <212> DNA
   <213> Artificial Sequence
<220>
   <223> Synthetic Primer: nbla21855-1f
<400> 713
   atgaaggggg aaggggttct 20
<210> 714
   <211> 21
   <212> DNA
   <213> Artificial Sequence
<220>
   <223> Synthetic Primer: nbla21855-1r
<400> 714
   gaacatggtg ctcctttgtg g 21
<210> 715
   <211> 19
   <212> DNA
   <213> Artificial Sequence
<220>
   <223> Synthetic Primer: nbla22704-1f
<400> 715
   tcacaaatca gcaggcaca 19
<210> 716
   <211> 21
   <212> DNA
   <213> Artificial Sequence
<220>
   <223> Synthetic Primer: nbla22704-1r
<400> 716
   tgctaccaac ccctctacat c 21
<210> 717
   <211> 21
   <212> DNA
   <213> Artificial Sequence
<220>
   <223> Synthetic Primer: nbla23394-1f
<400> 717
   ttcctgagag actgggagtt g 21
<210> 718
   <211> 19
   <212> DNA
   <213> Artificial Sequence
<220>
   <223> Synthetic Primer: nbla23394-1r
<400> 718
   atagctgagg gagccgttg 19
<210> 719
   <211> 21
   <212> DNA
   <213> Artificial Sequence
<220>
   <223> Synthetic Primer: nbla23512-1f
<400> 719
   actgtcccac cacaactgaa c 21
<210> 720
   <211> 23
   <212> DNA
   <213> Artificial Sequence
<220>
   <223> Synthetic Primer: nbla23512-1r
<400> 720
   ctcataatct cgtctttgca cct 23
<210> 721
   <211> 22
   <212> DNA
   <213> Artificial Sequence
<220>
   <223> Synthetic Primer: nbla24084-1f
<400> 721
   ttagcagaga catgcaacaa ca 22
<210> 722
   <211> 22
   <212> DNA
   <213> Artificial Sequence
<220>
   <223> Synthetic Primer: nbla24084-1r
<400> 722
   cgtgatccaa cagaagattg ag 22
<210> 723
   <211> 21
   <212> DNA
   <213> Artificial Sequence
<220>
   <223> Synthetic Primer: nbla24376-1f
<400> 723
   aacaagccta gaggaatgaa c 21
<210> 724
   <211> 18
   <212> DNA
   <213> Artificial Sequence
<220>
   <223> Synthetic Primer: nbla24376-1r
<400> 724
   tacaagaagc gcaacacc 18
<210> 725
   <211> 20
   <212> DNA
   <213> Artificial Sequence
<220>
   <223> Synthetic Primer: nbla21761-1f
<400> 725
   cttcgccaga caaaaccatc 20
<210> 726
   <211> 21
   <212> DNA
   <213> Artificial Sequence
<220>
   <223> Synthetic Primer: nbla21761-1r
<400> 726
   gatctccccc ttcttctcct c 21
<210> 727
   <211> 20
   <212> DNA
   <213> Artificial Sequence
<220>
   <223> Synthetic Primer: nbla23456-1f
<400> 727
   ccattgcttt agtcgttgct 20
<210> 728
   <211> 20
   <212> DNA
   <213> Artificial Sequence
<220>
   <223> Synthetic Primer: nbla23456-1r
<400> 728
   aattagctcc tcctcgctgt 20
<210> 729
   <211> 22
   <212> DNA
   <213> Artificial Sequence
<220>
   <223> Synthetic Primer: nbla24297-1f
<400> 729
   acaaccattc cctaactcca tc 22
<210> 730
   <211> 21
   <212> DNA
   <213> Artificial Sequence
<220>
   <223> Synthetic Primer: nbla24297-1r
<400> 730
   ctgttactgt tgctgcttcc a 21
<210> 731
   <211> 19
   <212> DNA
   <213> Artificial Sequence
<220>
   <223> Synthetic Primer: nbla24719-1f
<400> 731
   tcgttacacc gctttgtcc 19
<210> 732
   <211> 20
   <212> DNA
   <213> Artificial Sequence
<220>
   <223> Synthetic Primer: nbla24719-1r
<400> 732
   ggcttggaaa acacacacac 20
<210> 733
   <211> 20
   <212> DNA
   <213> Artificial Sequence
<220>
   <223> Synthetic Primer: nbla20113-1f
<400> 733
   gcccaaaggg tatttccaag 20
<210> 734
   <211> 19
   <212> DNA
   <213> Artificial Sequence
<220>
   <223> Synthetic Primer: nbla20113-1r
<400> 734
   cacaaggggt ggactgatg 19
<210> 735
   <211> 19
   <212> DNA
   <213> Artificial Sequence
<220>
   <223> Synthetic Primer: nbla20253r1-1f
<400> 735
   accagggata agggggaac 19
<210> 736
   <211> 20
   <212> DNA
   <213> Artificial Sequence
<220>
   <223> Synthetic Primer: nbla20253r1-1r
<400> 736
   tgctttgccc acactaaaga 20
<210> 737
   <211> 19
   <212> DNA
   <213> Artificial Sequence
<220>
   <223> Synthetic Primer: nbla20549-1f
<400> 737
   gtgcttgtct gatgggatg 19
<210> 738
   <211> 19
   <212> DNA
   <213> Artificial Sequence
<220>
   <223> Synthetic Primer: nbla20549-1r
<400> 738
   caatgaagac gctcacagg 19
<210> 739
   <211> 21
   <212> DNA
   <213> Artificial Sequence
<220>
   <223> Synthetic Primer: nbla20835-1f
<400> 739
   aaggtgacag cataggtgga g 21
<210> 740
   <211> 23
   <212> DNA
   <213> Artificial Sequence
<220>
   <223> Synthetic Primer: nbla20835-1r
<400> 740
   tgatagggat tcttgctaac tgg 23
<210> 741
   <211> 18
   <212> DNA
   <213> Artificial Sequence
<220>
   <223> Synthetic Primer: nbla20968-1f
<400> 741
   agcctggtgg ctcacatc 18
<210> 742
   <211> 22
   <212> DNA
   <213> Artificial Sequence
<220>
   <223> Synthetic Primer: nbla20968-1r
<400> 742
   gacacttgcc tcaatagggt tc 22
<210> 743
   <211> 25
   <212> DNA
   <213> Artificial Sequence
<220>
   <223> Synthetic Primer: nbla21087-1f
<400> 743
   gtgtctctcc tagtgattga ttttg 25
<210> 744
   <211> 22
   <212> DNA
   <213> Artificial Sequence
<220>
   <223> Synthetic Primer: nbla21087-1r
<400> 744
   taaaaggggt tgttctcttg ct 22
<210> 745
   <211> 19
   <212> DNA
   <213> Artificial Sequence
<220>
   <223> Synthetic Primer: nbla21189-1f
<400> 745
   catcctacag gtggaagca 19
<210> 746
   <211> 20
   <212> DNA
   <213> Artificial Sequence
<220>
   <223> Synthetic Primer: nbla21189-1r
<400> 746
   agttcttggg tgtggtgaag 20
<210> 747
   <211> 20
   <212> DNA
   <213> Artificial Sequence
<220>
   <223> Synthetic Primer: nbla21214-1f
<400> 747
   aggggtaagt cagggaagga 20
<210> 748
   <211> 20
   <212> DNA
   <213> Artificial Sequence
<220>
   <223> Synthetic Primer: nbla21214-1r
<400> 748
   cctaccaggc aaagtccaag 20
<210> 749
   <211> 20
   <212> DNA
   <213> Artificial Sequence
<220>
   <223> Synthetic Primer: nbla21337-1f
<400> 749
   atttcagccg catctcacac 20
<210> 750
   <211> 21
   <212> DNA
   <213> Artificial Sequence
<220>
   <223> Synthetic Primer: nbla21337-1r
<400> 750
   gcttcgccaa cactcattac a 21
<210> 751
   <211> 22
   <212> DNA
   <213> Artificial Sequence
<220>
   <223> Synthetic Primer: nbla21344r1-1f
<400> 751
   ccattttgct gattttctct gg 22
<210> 752
   <211> 20
   <212> DNA
   <213> Artificial Sequence
<220>
   <223> Synthetic Primer: nbla21344r1-1r
<400> 752
   attcttcccc ctccctctgt 20
<210> 753
   <211> 19
   <212> DNA
   <213> Artificial Sequence
<220>
   <223> Synthetic Primer: nbla21956-2-1f
<400> 753
   ggacttgggg ctctcctct 19
<210> 754
   <211> 20
   <212> DNA
   <213> Artificial Sequence
<220>
   <223> Synthetic Primer: nbla21956-2-1r
<400> 754
   gctagggcac ctgatttgtg 20
<210> 755
   <211> 20
   <212> DNA
   <213> Artificial Sequence
<220>
   <223> Synthetic Primer: nbla22228-1f
<400> 755
   gtatgttgga gcagcgaaag 20
<210> 756
   <211> 20
   <212> DNA
   <213> Artificial Sequence
<220>
   <223> Synthetic Primer: nbla22228-1r
<400> 756
   gtccccaaag aagagttcca 20
<210> 757
   <211> 22
   <212> DNA
   <213> Artificial Sequence
<220>
   <223> Synthetic Primer: nbla22351-1f
<400> 757
   ggtgagttag ctttgaggtg tg 22
<210> 758
   <211> 20
   <212> DNA
   <213> Artificial Sequence
<220>
   <223> Synthetic Primer: nbla22351-1r
<400> 758
   ggccagacga gtggaaatag 20
<210> 759
   <211> 20
   <212> DNA
   <213> Artificial Sequence
<220>
   <223> Synthetic Primer: nbla22361-1f
<400> 759
   ccctacggat caagggctac 20
<210> 760
   <211> 19
   <212> DNA
   <213> Artificial Sequence
<220>
   <223> Synthetic Primer: nbla22361-1r
<400> 760
   ctgtctcagg ggctccaac 19
<210> 761
   <211> 20
   <212> DNA
   <213> Artificial Sequence
<220>
   <223> Synthetic Primer: nbla22474-1f
<400> 761
   gaagatgctg ccctaattcc 20
<210> 762
   <211> 21
   <212> DNA
   <213> Artificial Sequence
<220>
   <223> Synthetic Primer: nbla22474-1r
<400> 762
   ccacattcct tttctttgtc c 21
<210> 763
   <211> 21
   <212> DNA
   <213> Artificial Sequence
<220>
   <223> Synthetic Primer: nbla22939-1f
<400> 763
   ggacagcagc aactcaaaaa g 21
<210> 764
   <211> 20
   <212> DNA
   <213> Artificial Sequence
<220>
   <223> Synthetic Primer: nbla22939-1r
<400> 764
   tatctatccc catgcctcca 20
<210> 765
   <211> 20
   <212> DNA
   <213> Artificial Sequence
<220>
   <223> Synthetic Primer: nbla23775-1f
<400> 765
   tgagcaatac cctgcctaca 20
<210> 766
   <211> 22
   <212> DNA
   <213> Artificial Sequence
<220>
   <223> Synthetic Primer: nbla23775-1r
<400> 766
   gtccccagtg ctaatcctac tc 22
<210> 767
   <211> 18
   <212> DNA
   <213> Artificial Sequence
<220>
   <223> Synthetic Primer: nbla24182-1f
<400> 767
   ctgacgggag aggaggaa 18
<210> 768
   <211> 20
   <212> DNA
   <213> Artificial Sequence
<220>
   <223> Synthetic Primer: nbla24182-1r
<400> 768
   gaaaaggcac cgaacagaac 20
<210> 769
   <211> 20
   <212> DNA
   <213> Artificial Sequence
<220>
   <223> Synthetic Primer: nbla24285-1f
<400> 769
   tcagacggtg aggatgatgt 20
<210> 770
   <211> 18
   <212> DNA
   <213> Artificial Sequence
<220>
   <223> Synthetic Primer: nbla24285-1r
<400> 770
   cgctgtcctt ttgcctgt 18
<210> 771
   <211> 20
   <212> DNA
   <213> Artificial Sequence
<220>
   <223> Synthetic Primer: nbla24434-1f
<400> 771
   cagaggctga gaatggtgtg 20
<210> 772
   <211> 20
   <212> DNA
   <213> Artificial Sequence
<220>
   <223> Synthetic Primer: nbla24434-1r
<400> 772
   gccttgtact ggctggaaga 20
<210> 773
   <211> 21
   <212> DNA
   <213> Artificial Sequence
<220>
   <223> Synthetic Primer: nbla24460d-1f
<400> 773
   tctctgaaaa gtgccagtcc a 21
<210> 774
   <211> 20
   <212> DNA
   <213> Artificial Sequence
<220>
   <223> Synthetic Primer: nbla24460d-1r
<400> 774
   tcatgccctg ccttagaaac 20
<210> 775
   <211> 25
   <212> DNA
   <213> Artificial Sequence
<220>
   <223> Synthetic Primer: nbla24762-1f
<400> 775
   agctactctg aagacctccc tatgt 25
<210> 776
   <211> 20
   <212> DNA
   <213> Artificial Sequence
<220>
   <223> Synthetic Primer: nbla24762-1r
<400> 776
   tgcatccaca cgttctcttg 20
<210> 777
   <211> 20
   <212> DNA
   <213> Artificial Sequence
<220>
   <223> Synthetic Primer: nbla24893-1f
<400> 777
   agatggattt ttgccccttc 20
<210> 778
   <211> 22
   <212> DNA
   <213> Artificial Sequence
<220>
   <223> Synthetic Primer: nbla24893-1r
<400> 778
   tacaggtaga aacaagccca ca 22
<210> 779
   <211> 18
   <212> DNA
   <213> Artificial Sequence
<220>
   <223> Synthetic Primer: nbla24973-1f
<400> 779
   tccctggagg caaacaca 18
<210> 780
   <211> 20
   <212> DNA
   <213> Artificial Sequence
<220>
   <223> Synthetic Primer: nbla24973-1r
<400> 780
   atgtgacgca gtggcctatc 20
<210> 781
   <211> 19
   <212> DNA
   <213> Artificial Sequence
<220>
   <223> Synthetic Primer: nbla24986-1-1f
<400> 781
   atggaacacc acagccaga 19
<210> 782
   <211> 21
   <212> DNA
   <213> Artificial Sequence
<220>
   <223> Synthetic Primer: nbla24986-1-1r
<400> 782
   ccagagtcag cccattaaac a 21
<210> 783
   <211> 21
   <212> DNA
   <213> Artificial Sequence
<220>
   <223> Synthetic Primer: nbla23020-1f
<400> 783
   tcaggatgag gaaatgacag g 21
<210> 784
   <211> 19
   <212> DNA
   <213> Artificial Sequence
<220>
   <223> Synthetic Primer: nbla23020-1r
<400> 784
   agtcacgctg ggaggaaag 19
<210> 785
   <211> 20
   <212> DNA
   <213> Artificial Sequence
<220>
   <223> Synthetic Primer: nbla20087d-1f(k)
<400> 785
   ccagctctcc agttttcagg 20
<210> 786
   <211> 21
   <212> DNA
   <213> Artificial Sequence
<220>
   <223> Synthetic Primer: nbla20087d-1r
<400> 786
   gttccctttc ggtagttgag g 21
<210> 787
   <211> 22
   <212> DNA
   <213> Artificial Sequence
<220>
   <223> Synthetic Primer: nbla21750d-1f(k)
<400> 787
   gatgaattgc ctccattgtc tc 22

<210> 788
   <211> 20
   <212> DNA
   <213> Artificial Sequence
<220>
   <223> Synthetic Primer: nbla21750d-1r
<400> 788
   ggtttgctgc ttctggatgt 20
<210> 789
   <211> 21
   <212> DNA
   <213> Artificial Sequence
<220>
   <223> Synthetic Primer: nbla22129-1f(k)
<400> 789
   cagatgggga gtgttctgat g 21
<210> 790
   <211> 21
   <212> DNA
   <213> Artificial Sequence
<220>
   <223> Synthetic Primer: nbla22129-1r
<400> 790
   tctagggggt ggtaaagatg g 21
<210> 791
   <211> 22
   <212> DNA
   <213> Artificial Sequence
<220>
   <223> Synthetic Primer: nbla22808-1f(k)
<400> 791
   ggaccaagat atggttttgg ag 22
<210> 792
   <211> 20
   <212> DNA
   <213> Artificial Sequence
<220>
   <223> Synthetic Primer: nbla22808-1r
<400> 792
   gcatgtattt gcctcccttg 20
<210> 793
   <211> 21
   <212> DNA
   <213> Artificial Sequence
<220>
   <223> Synthetic Primer: nbla23064-1f(k)
<400> 793
   catgaaccct tccctatgtc c 21
<210> 794
   <211> 19
   <212> DNA
   <213> Artificial Sequence
<220>
   <223> Synthetic Primer: nbla23064-1r
<400> 794
   tctttgcatc catcgcatc 19
<210> 795
   <211> 19
   <212> DNA
   <213> Artificial Sequence
<220>
   <223> Synthetic Primer: nbla23358d-1f(k)
<400> 795
   gctctcccaa atcgcctac 19
<210> 796
   <211> 19
   <212> DNA
   <213> Artificial Sequence
<220>
   <223> Synthetic Primer: nbla23358-d-1r
<400> 796
   cctcatcatc cccttccac 19
<210> 797
   <211> 20
   <212> DNA
   <213> Artificial Sequence
<220>
   <223> Synthetic Primer: nbla22443-1f(k)
<400> 797
   atccttggtg gccttgtatg 20
<210> 798
   <211> 20
   <212> DNA
   <213> Artificial Sequence
<220>
   <223> Synthetic Primer: nbla22443-1r
<400> 798
   tcagagtgat tgctggcttg 20
<210> 799
   <211> 22
   <212> DNA
   <213> Artificial Sequence
<220>
   <223> Synthetic Primer: nbla20235-1f(k)
<400> 799
   tccttacacg ggccataaat ac 22
<210> 800
   <211> 20
   <212> DNA
   <213> Artificial Sequence
<220>
   <223> Synthetic Primer: nbla20235-1r
<400> 800
   accgtctcaa atcgaaccac 20
<210> 801
   <211> 19
   <212> DNA
   <213> Artificial Sequence
<220>
   <223> Synthetic Primer: nbla22607-1f(k)
<400> 801
   acacatgcct agcagacca 19
<210> 802
   <211> 22
   <212> DNA
   <213> Artificial Sequence
<220>
   <223> Synthetic Primer: nbla22607-1r
<400> 802 -
   tgcacttcat ttagacttca cc 22
<210> 803
   <211> 19
   <212> DNA
   <213> Artificial Sequence
<220>
   <223> Synthetic Primer: nbla22305-1f(k)
<400> 803
   gcagttccaa tgaaggaca 19
<210> 804
   <211> 22
   <212> DNA
   <213> Artificial Sequence
<220>
   <223> Synthetic Primer: nbla22305-1r
<400> 804
   tcatctgctt ggtgtatgaa ag 22
<210> 805
   <211> 21
   <212> DNA
   <213> Artificial Sequence
<220>
   <223> Synthetic Primer: nbla22031-1f(k)
<400> 805
   tccctctgta ttttgggttg g 21
<210> 806
   <211> 20
   <212> DNA
   <213> Artificial Sequence
<220>
   <223> Synthetic Primer: nbla22031-1r
<400> 806
   ggtggatgtt ccttgagtgg 20
<210> 807
   <211> 20
   <212> DNA
   <213> Artificial Sequence
<220>
   <223> Synthetic Primer: nbla23478d-1f(k)
<400> 807
   agcacaacag caaggacaga 20
<210> 808
   <211> 19
   <212> DNA
   <213> Artificial Sequence
<220>
   <223> Synthetic Primer: nbla23478d-1r
<400> 808
   cgttaccaaa cagcccaga 19
<210> 809
   <211> 20
   <212> DNA
   <213> Artificial Sequence
<220>
   <223> Synthetic Primer: nbla23896-1f(k)
<400> 809
   tcccattaca ggctctttcc 20
<210> 810
   <211> 22
   <212> DNA
   <213> Artificial Sequence
<220>
   <223> Synthetic Primer: nbla23896-1r
<400> 810
   gctccttcca agatttatcc ac 22
<210> 811
<211> 19
<212> DNA
   <213> Artificial Sequence
<220>
   <223> Synthetic Primer: nbla24920-1f(k)
<400> 811
   gcaactccat ccaccgtct 19
<210> 812
   <211> 19
   <212> DNA
   <213> Artificial Sequence
<220>
   <223> Synthetic Primer: nbla24920-1r
<400> 812
   ccgtttctgg gctctcttg 19
<210> 813
   <211> 24
   <212> DNA
   <213> Artificial Sequence
<220>
   <223> Synthetic Primer: nbla20141-1f(k)
<400> 813
   ctgtgttacc ctgtttttct acct 24
<210> 814
   <211> 23
   <212> DNA
   <213> Artificial Sequence
<220>
   <223> Synthetic Primer: nbla20141-1r
<400> 814
   cgggctatgt atctaaggtt ttc 23
<210> 815
   <211> 20
   <212> DNA
   <213> Artificial Sequence
<220>
   <223> Synthetic Primer: nbla20446-1f(k)
<400> 815
   tagccctctt tggtcctcct 20
<210> 816
   <211> 22
   <212> DNA
   <213> Artificial Sequence
<220>
   <223> Synthetic Primer: nbla20446-1r
<400> 816
   ttacagtcat gttgccagtt cc 22
<210> 817
   <211> 22
   <212> DNA
   <213> Artificial Sequence
<220>
   <223> Synthetic Primer: nbla21538-1f(k)
<400> 817
   ggagagaagt ttgaagaaac ca 22
<210> 818
   <211> 20
   <212> DNA
   <213> Artificial Sequence
<220>
   <223> Synthetic Primer: nbla21538-1r
<400> 818
   tccaccacta atttcccatc 20
<210> 819
   <211> 18
   <212> DNA
   <213> Artificial Sequence
<220>
   <223> Synthetic Primer: nbla22558-1f(k)
<400> 819
   cgggccacca gtttctct 18
<210> 820
<211> 19
   <212> DNA
   <213> Artificial Sequence
<220>
   <223> Synthetic Primer: nbla22558-1r
<400> 820
   tcgatactcg gcctcgaac 19
<210> 821
   <211> 20
   <212> DNA
   <213> Artificial Sequence
<220>
   <223> Synthetic Primer: nbla21623-1f(k)
<400> 821
   ggaagaaaag ttccgaggtg 20
<210> 822
   <211> 19
   <212> DNA
   <213> Artificial Sequence
<220>
   <223> Synthetic Primer: nbla21623-1r
<400> 822
   ttgacagtgc tgcttgtgg 19
<210> 823
   <211> 21
   <212> DNA
   <213> Artificial Sequence
<220>
   <223> Synthetic Primer: nbla21969-1f(k)
<400> 823
   caaaagcgtc ctgctctaca c 21
<210> 824
   <211> 19
   <212> DNA
   <213> Artificial Sequence
<220>
   <223> Synthetic Primer: nbla21969-1r
<400> 824
   acgagactga ccacccaga 19
<210> 825
   <211> 21
   <212> DNA
   <213> Artificial Sequence
<220>
   <223> synthetic Primer: nbla22219-1f(k)
<400> 825
   tgtggttcat agtgaggtgg a 21
<210> 826
   <211> 20
   <212> DNA
   <213> Artificial Sequence
<220>
   <223> Synthetic Primer: nbla22219-1r
<400> 826
   gagcaagttt tggctttgtg 20
<210> 827
   <211> 21
   <212> DNA
   <213> Artificial Sequence
<220>
   <223> Synthetic Primer: nbla23272-1f(k)
<400> 827
   ctagggacag gaagatggtt g 21
<210> 828
   <211> 20
   <212> DNA
   <213> Artificial Sequence
<220>
   <223> Synthetic Primer: nbla23272-1r
<400> 828
   gatacaggtc atgggcagag 20
<210> 829
   <211> 19
   <212> DNA
   <213> Artificial Sequence
<220>
   <223> Synthetic Primer: nbla23307-1-1f(k)
<400> 829
   atccctcaga acccatgct 9
<210> 830
   <211> 20
   <212> DNA
   <213> Artificial Sequence
<220>
   <223> Synthetic Primer: nbla23307-1-1r
<400> 830
   cgctcaactt ccacttctcc 20
<210> 831
   <211> 20
   <212> DNA
   <213> Artificial Sequence
<220>
   <223> Synthetic Primer: nbla24117-1f(k)
<400> 831
   gtcctgaagg cagagggaag 20
<210> 832
   <211> 20
   <212> DNA
   <213> Artificial Sequence
<220>
   <223> Synthetic Primer: nbla24117-1r
<400> 832
   cagggttggg gtaagagagg 20
<210> 833
   <211> 19
   <212> DNA
   <213> Artificial Sequence
<220>
   <223> Synthetic Primer: nbla23262-1f(k)
<400> 833
   ggacaagagc caggaagaa 19
<210> 834
   <211> 21
   <212> DNA
   <213> Artificial Sequence
<220>
   <223> Synthetic Primer: nbla23262-1r
<400> 834
   ggtggaaagg tttggagtat g 21
<210> 835
   <211> 21
   <212> DNA
   <213> Artificial Sequence
<220>
   <223> Synthetic Primer: nbla20133d-1f(k)
<400> 835
   gctacgtgga agtgaatgga g 21
<210> 836
   <211> 20
   <212> DNA
   <213> Artificial Sequence
<220>
   <223> Synthetic Primer: nbla20133d-1r
<400> 836
   ccagaaacag accccaagag 20
<210> 837
   <211> 18
   <212> DNA
   <213> Artificial Sequence
<220>
   <223> Synthetic Primer: nbla20263r1-1f(k)
<400> 837
   tgggggaaaa gttcttgg 18
<210> 838
   <211> 19
   <212> DNA
   <213> Artificial Sequence
<220>
   <223> Synthetic Primer: nbla20263r1-1r
<400> 838
   gcctgtcctg tagctggtt 19
<210> 839
   <211> 18
   <212> DNA
   <213> Artificial Sequence
<220>
   <223> Synthetic Primer: nbla20723-1f(k)
<400> 839
   agatgccaaa cgcagaac 18
<210> 840
   <211> 20
   <212> DNA
   <213> Artificial Sequence
<220>
   <223> Synthetic Primer: nbla20723-1r
<400> 840
   ttgaagcaaa cactcaccaa 20
<210> 841
   <211> 20
   <212> DNA
   <213> Artificial Sequence
<220>
   <223> Synthetic Primer: nbla20748-1f(k)
<400> 841
   catccatctc acagcaccac 20
<210> 842
   <211> 20
   <212> DNA
   <213> Artificial Sequence
<220>
   <223> Synthetic Primer: nbla20748-1r
<400> 842
   tctcacgcag caactcaatc 20
<210> 843
   <211> 21
   <212> DNA
   <213> Artificial Sequence
<220>
   <223> Synthetic Primer: nbla20915-1f(k)
<400> 843
   ggatcagaga gggctacctt g 21
<210> 844
   <211> 20
   <212> DNA
   <213> Artificial Sequence
<220>
   <223> Synthetic Primer: nbla20915-1r
<400> 844
   cctgctgttt ggtcgtagtg 20
<210> 845
   <211> 21
   <212> DNA
   <213> Artificial Sequence
<220>
   <223> Synthetic Primer: nbla21016-1f(k)
<400> 845
   agtttactct tgcccactcc a 21
<210> 846
   <211> 20
   <212> DNA
   <213> Artificial Sequence
<220>
   <223> Synthetic Primer: nbla21016-1r
<400> 846
   ctggattttt gccctgtctc 20
<210> 847
   <211> 20
   <212> DNA
   <213> Artificial Sequence
<220>
   <223> Synthetic Primer: nbla21034r1-1f(k)
<400> 847
   caatcaccag ttgctgtcct 20
<210> 848
   <211> 21
   <212> DNA
   <213> Artificial Sequence
<220>
   <223> Synthetic Primer: nbla21034r1-1r
<400> 848
   atttcccagt ctcccctatg t 21
<210> 849
   <211> 20
   <212> DNA
   <213> Artificial Sequence
<220>
   <223> Synthetic Primer: nbla21067-1f(k)
<400> 849
   tgagaagagg agtgcaagga 20
<210> 850
   <211> 18
   <212> DNA
   <213> Artificial Sequence
<220>
   <223> Synthetic Primer: nbla21067-1r
<400> 850
   tgcatggatt tgggtttg 18
<210> 851
   <211> 20
   <212> DNA
   <213> Artificial Sequence
<220>
   <223> Synthetic Primer: nbla21167-1f(k)
<400> 851
   ttcttctctg tccccaaaca 20
<210> 852
   <211> 22
   <212> DNA
   <213> Artificial Sequence
<220>
   <223> Synthetic Primer: nbla21167-1r
<400> 852
   gagctgtcaa tacaacactg ga 22
<210> 853
   <211> 19
   <212> DNA
   <213> Artificial Sequence
<220>
   <223> Synthetic Primer: nbla21319-1f(k)
<400> 853
   ttggggttca tcctccttc 19
<210> 854
   <211> 20
   <212> DNA
   <213> Artificial Sequence
<220>
   <223> Synthetic Primer: nbla21319-1r
<400> 854
   gttgaggtcg ttctccgtgt 20
<210> 855
   <211> 23
   <212> DNA
   <213> Artificial Sequence
<220>
   <223> Synthetic Primer: nbla21331-1f(k)
<400> 855
   tggcaggttt tcttctactt gtg 23
<210> 856
   <211> 22
   <212> DNA
   <213> Artificial Sequence
<220>
   <223> Synthetic Primer: nbla21331-1r
<400> 856
   tcccagctaa catggttgat tt 22
<210> 857
   <211> 20
   <212> DNA
   <213> Artificial Sequence
<220>
   <223> Synthetic Primer: nbla21516-1f(k)
<400> 857
   gcaggaagcg atggttaaga 20
<210> 858
   <211> 21
   <212> DNA
   <213> Artificial Sequence
<220>
   <223> Synthetic Primer: nbla21516-1r
<400> 858
   gcccaagtag gaatctgtgt g 21
<210> 859
   <211> 20
   <212> DNA
   <213> Artificial Sequence
<220>
   <223> Synthetic Primer: nbla21682d-1f(k)
<400> 859
   aatctacgct tcccaaacca 20
<210> 860
   <211> 20
   <212> DNA
   <213> Artificial Sequence
<220>
   <223> Synthetic Primer: nbla21682-1r
<400> 860
   taggcactgg gcaatgatac 20
<210> 861
   <211> 18
   <212> DNA
   <213> Artificial Sequence
<220>
   <223> Synthetic Primer: nbla21691-1f(k)
<400> 861
   gcaggtgaat gccttggt 18
<210> 862
   <211> 19
   <212> DNA
   <213> Artificial Sequence
<220>
   <223> Synthetic Primer: nbla21691-1r
<400> 862
   gcacgaattg cttggagag 19
<210> 863
   <211> 21
   <212> DNA
   <213> Artificial Sequence
<220>
   <223> Synthetic Primer: nbla21822-1f(k)
<400> 863
   gcagaggatg gaaagttgat g 21
<210> 864
   <211> 20
   <212> DNA
   <213> Artificial Sequence
<220>
   <223> Synthetic Primer: nbla21822-1r
<400> 864
   gtggcagcac aaagaaaaga 20
   <210> 865
   <211> 19
   <212> DNA
   <213> Artificial Sequence
<220>
   <223> Synthetic Primer: nbla21976-2-1f(k)
<400> 865
   agtgctgggc ctaaaggag 19
<210> 866
   <211> 22
   <212> DNA
   <213> Artificial Sequence
<220>
   <223> Synthetic Primer: nbla21976-2-1r
<400> 866
   gactccctga ctgttgatgt tg 22
<210> 867
   <211> 22
   <212> DNA
   <213> Artificial Sequence
<220>
   <223> Synthetic Primer: nbla21977-1f(k)
<400> 867
   gcctaccatt tcacagaggt tt 22
<210> 868
   <211> 22
   <212> DNA
   <213> Artificial Sequence
<220>
   <223> Synthetic Primer: nbla21977-1r
<400> 868
   tgtttttata tgctgccctt cc 22
<210> 869
   <211> 20
   <212> DNA
   <213> Artificial Sequence
<220>
   <223> Synthetic Primer: nbla22159-1f(k)
<400> 869
   tggcacatca gaaaggaatg 20
<210> 870
   <211> 20
   <212> DNA
   <213> Artificial Sequence
<220>
   <223> Synthetic Primer: nbla22159-1r
<400> 870
   aatgggagcc aaggaaagag 20
<210> 871
   <211> 20
   <212> DNA
   <213> Artificial Sequence
<220>
   <223> Synthetic Primer: nbla22168-1f(k)
<400> 871
   tactgggtcg ggtgtttgtg 20
<210> 872
   <211> 19
   <212> DNA
   <213> Artificial Sequence
<220>
   <223> Synthetic Primer: nbla22168-1r
<400> 872
   ccgatggtgc tcttgctct 19
<210> 873
   <211> 21
   <212> DNA
   <213> Artificial Sequence
<220>
   <223> Synthetic Primer: nbla22215-1-1f(k)
<400> 873
   gccctctcct gacttgtatt g 21
<210> 874
   <211> 21
   <212> DNA
   <213> Artificial Sequence
<220>
   <223> Synthetic Primer: nbla22215-1-1r
<400> 874
   cctgaagttt gctgttttgt g 21
<210> 875
   <211> 22
   <212> DNA
   <213> Artificial Sequence
<220>
   <223> synthetic Primer: nbla22244-1f(k)
<400> 875
   agagaatcgg aagtggatga ga 22
<210> 876
   <211> 19
   <212> DNA
   <213> Artificial Sequence
<220>
   <223> Synthetic Primer: nbla22244-1r
<400> 876
   atgcttgctg ctttgcttg 19
<210> 877
   <211> 20
   <212> DNA
   <213> Artificial Sequence
<220>
   <223> Synthetic Primer: nbla22263-1f(k)
<400> 877
   aagattggaa gacccgtttg 20
<210> 878
   <211> 20
   <212> DNA
   <213> Artificial Sequence
<220>
   <223> Synthetic Primer: nbla22263-1r
<400> 878
   acagcttttg gggtgatttg 20
<210> 879
   <211> 19
   <212> DNA
   <213> Artificial Sequence
<220>
   <223> Synthetic Primer: nbla22548-1f(k)
<400> 879
   atcccaacca cctcccttg 19
<210> 880
   <211> 20
   <212> DNA
   <213> Artificial Sequence
<220>
   <223> Synthetic Primer: nbla22548-1r
<400> 880
   ctgctgtccc cactcctctt 20
<210> 881
   <211> 20
   <212> DNA
   <213> Artificial Sequence
<220>
   <223> Synthetic Primer: nbla23033-1f(k)
<400> 881
   tctagtggtg gcagggaaga 20
<210> 882
   <211> 21
   <212> DNA
   <213> Artificial Sequence
<220>
   <223> Synthetic Primer: nbla23033-1r
<400> 882
   agcatggagg aaacagacag a 21
<210> 883
   <211> 20
   <212> DNA
   <213> Artificial Sequence
<220>
   <223> Synthetic Primer: nbla23231-1f(k)
<400> 883
   aggctctccc tcagttacca 20
<210> 884
   <211> 18
   <212> DNA
   <213> Artificial Sequence
<220>
   <223> Synthetic Primer: nbla23231-1r
<400> 884
   caaaaccgtc ccgaagag 18
<210> 885
   <211> 22
   <212> DNA
   <213> Artificial Sequence
<220>
   <223> Synthetic Primer: nbla23284-1f(k)
<400> 885
   gtgatgctgt cttgaattgt cc 22
<210> 886
   <211> 20
   <212> DNA
   <213> Artificial Sequence
<220>
   <223> Synthetic Primer: nbla23284-1r
<400> 886
   cttatggacc cgccttttct 20
<210> 887
   <211> 20
   <212> DNA
   <213> Artificial Sequence
<220>
   <223> Synthetic Primer: nbla23329-1d-1f(k)
<400> 887
   gcatggacag ttgtttggag 20
<210> 888
   <211> 19
   <212> DNA
   <213> Artificial Sequence
<220>
   <223> Synthetic Primer: nbla23329-1d-1r
<400> 888
   ggaagaaccg gaggacttg 19
<210> 889
   <211> 19
   <212> DNA
   <213> Artificial Sequence
<220>
   <223> Synthetic Primer: nbla23384-1f(k)
<400> 889
   ttagccagcg cacctttac 19
<210> 890
<211> 20
   <212> DNA
   <213> Artificial Sequence
<220>
   <223> Synthetic Primer: nbla23384-1r
<400> 890
   tacccaccac atctccttcc 20
   <210> 891
<211> 20
   <212> DNA
   <213> Artificial Sequence
<220>
   <223> Synthetic Primer: nbla23556-1f(k)
<400> 891
   ggaagtcctt tccacctctc 20
<210> 892
   <211> 20
   <212> DNA
   <213> Artificial Sequence
<220>
   <223> Synthetic Primer: nbla23556-1r
<400> 892
   agtcctatgc acgactccaa 20
<210> 893
   <211> 19
   <212> DNA
   <213> Artificial Sequence
<220>
   <223> Synthetic Primer: nbla23674r1-1f(k)
<400> 893
   tgttcttctt ggccttgct 19
<210> 894
   <211> 20
   <212> DNA
   <213> Artificial Sequence
<220>
   <223> Synthetic Primer: nbla23674r1-1r
<400> 894
   ctgcatcctc atcctcctct 20
<210> 895
   <211> 23
   <212> DNA
   <213> Artificial Sequence
<220>
   <223> Synthetic Primer: nbla23879-2-1f(k)
<400> 895
   cattctgttt gatcttcggt ctc 23
<210> 896
   <211> 21
   <212> DNA
   <213> Artificial Sequence
<220>
   <223> Synthetic Primer: nbla23879-2-1r
<400> 896
   agctgtagca gtggatgctt t 21
<210> 897
   <211> 20
   <212> DNA
   <213> Artificial Sequence
<220>
   <223> Synthetic Primer: nbla24098r1-1f(k)
<400> 897
   tagggcttca tgtgggaaac 20
<210> 898
   <211> 18
   <212> DNA
   <213> Artificial Sequence
<220>
   <223> Synthetic Primer: nbla24098r1-1r
<400> 898
   agccgcgaaa ctgagaac 18
<210> 899
   <211> 20
   <212> DNA
   <213> Artificial Sequence
<220>
   <223> Synthetic Primer: nbla24329-1f(k)
<400> 899
   aggtggaggc tgatgacttg 20
<210> 900
   <211> 21
   <212> DNA
   <213> Artificial Sequence
<220>
   <223> Synthetic Primer: nbla24329-1r
<400> 900
   tctctgaata gtgccccgta g 21
<210> 901
   <211> 20
   <212> DNA
   <213> Artificial Sequence
<220>
   <223> Synthetic Primer: nbla24334-1f(k)
<400> 901
   tgggtaaagg acgaggaaga 20
<210> 902
   <211> 21
   <212> DNA
   <213> Artificial Sequence
<220>
   <223> Synthetic Primer: nbla24334-1r
<400> 902
   caggccatct atcaaccaca c 21

<210> 903
   <211> 18
   <212> DNA
   <213> Artificial Sequence
<220>
   <223> Synthetic Primer: nbla24439-1-1f(k)
<400> 903
   ggcggtgcag atccagtt 18
<210> 904
   <211> 18
   <212> DNA
   <213> Artificial Sequence
<220>
   <223> Synthetic Primer: nbla24439-1r
<400> 904
   gtcacgttgc cgtccttg 18
<210> 905
   <211> 21
   <212> DNA
   <213> Artificial Sequence
<220>
   <223> Synthetic Primer: nbla24507-1f(k)
<400> 905
   aacccgcatg gaattatctg t 21
<210> 906
   <211> 19
   <212> DNA
   <213> Artificial Sequence
<220>
   <223> Synthetic Primer: nbla24507-1r
<400> 906
   ctttggtgaa gggcatggt 19
<210> 907
   <211> 20
   <212> DNA
   <213> Artificial Sequence
<220>
   <223> Synthetic Primer: nbla24836-1f(k)
<400> 907
   cacgttgaca ggtttgcttg 20
<210> 908
   <211> 21
   <212> DNA
   <213> Artificial Sequence
<220>
   <223> Synthetic Primer: nbla24836-1r
<400> 908
   ccttgctctg ttgacattcc t 21
<210> 909
   <211> 20
   <212> DNA
   <213> Artificial Sequence
<220>
   <223> Synthetic Primer: nbla24958-1f(k)
<400> 909
   tggagcagtt ggctaaagag 20
<210> 910
   <211> 22
   <212> DNA
   <213> Artificial Sequence
<220>
   <223> Synthetic Primer: nbla24958-1r
<400> 910
   agtgatggta ctggatgtct gg 22
<210> 911
   <211> 20
   <212> DNA
   <213> Artificial Sequence
<220>
   <223> Synthetic Primer: nbla24989-1f(k)
<400> 911
   tggaaatcta tcgccctcac 20
<210> 912
   <211> 21
   <212> DNA
   <213> Artificial Sequence
<220>
   <223> Synthetic Primer: nbla24989-1r
<400> 912
   acagaactca aacaggccat c 21
<210> 913
   <211> 20
   <212> DNA
   <213> Artificial Sequence
<220>
   <223> Synthetic Primer: nbla20393d-1f(k)
<400> 913
   agtgcagaaa accgacgaag 20
<210> 914
   <211> 20
   <212> DNA
   <213> Artificial Sequence
<220>
   <223> Synthetic Primer: nbla20393d-1r
<400> 914
   ggtcaggcca ttgaagagag 20
<210> 915
   <211> 20
   <212> DNA
   <213> Artificial Sequence
<220>
   <223> Synthetic Primer: nbla20423d-1f(k)
<400> 915
   tggtctatca ccccagcttc 20
<210> 916
   <211> 22
   <212> DNA
   <213> Artificial Sequence
<220>
   <223> Synthetic Primer: nbla20423d-1r
<400> 916
   gttcttcacc ttctccaaca cc 22
<210> 917
   <211> 20
   <212> DNA
   <213> Artificial Sequence
<220>
   <223> Synthetic Primer: nbla20510-1f(k)
<400> 917
   gttcactggt gctcattcca 20
<210> 918
   <211> 22
   <212> DNA
   <213> Artificial Sequence
<220>
   <223> Synthetic Primer: nbla20510-1r
<400> 918
   tgatctcctc cctcttatcc ac 22
<210> 919
   <211> 19
   <212> DNA
   <213> Artificial Sequence
<220>
   <223> Synthetic Primer: nbla20833d-1f(k)
<400> 919
   gctaatcaaa gcggcaaca 19
<210> 920
   <211> 22
   <212> DNA
   <213> Artificial Sequence
<220>
   <223> Synthetic Primer: nbla20833d-1r
<400> 920
   tccatcagtc tcttcccata cc 22
<210> 921
   <211> 20
   <212> DNA
   <213> Artificial Sequence
<220>
   <223> Synthetic Primer: nbla20931-1f(k)
<400> 921
   tagcagggaa gccaaagatg 20
<210> 922
   <211> 23
   <212> DNA
   <213> Artificial Sequence
<220>
   <223> Synthetic Primer: nbla20931-1r
<400> 922
   cagtacacag gctccagaag aag 23
<210> 923
   <211> 20
   <212> DNA
   <213> Artificial Sequence
<220>
   <223> Synthetic Primer: nbla20943-1f(k)
<400> 923
   tctaggctgc ttggttcgtg 20
<210> 924
   <211> 19
   <212> DNA
   <213> Artificial Sequence
<220>
   <223> Synthetic Primer: nbla20943-1r
<400> 924
   gatcttcctg tggggcttg 19
<210> 925
   <211> 19
   <212> DNA
   <213> Artificial Sequence
<220>
   <223> Synthetic Primer: nbla21258r1-1f(k)
<400> 925
   ttaaggcggg tctctgttc 19
<210> 926
   <211> 21
   <212> DNA
   <213> Artificial Sequence
<220>
   <223> Synthetic Primer: nbla21258-1r
<400> 926
   tggaaacctc aaggaaaact c 21
<210> 927
   <211> 19
   <212> DNA
   <213> Artificial Sequence
<220>
   <223> Synthetic Primer: nbla21268-1f(k)
<400> 927
   cctagagggc agatgcaga 19
<210> 928
   <211> 18
   <212> DNA
   <213> Artificial Sequence
<220>
   <223> Synthetic Primer: nbla21268-1r
<400> 928
   gcctgagagg gaaaccac 18
<210> 929
   <211> 20
   <212> DNA
   <213> Artificial Sequence
<220>
   <223> synthetic Primer: nbla21273-1f(k)
<400> 929
   agagccttcc tcacccaaac 20
<210> 930
   <211> 20
   <212> DNA
   <213> Artificial Sequence
<220>
   <223> Synthetic Primer: nbla21273-1r
<400> 930
   agctccttca cctcctcaca 20
<210> 931
   <211> 20
   <212> DNA
   <213> Artificial Sequence
<220>
   <223> Synthetic Primer: nbla21412-1f(k)
<400> 931
   ttgaacagga gaagcaagca 20
<210> 932
   <211> 19
   <212> DNA
   <213> Artificial Sequence
<220>
   <223> Synthetic Primer: nbla21412-1r
<400> 932
   cggccttcgt tgtcagtag 19
<210> 933
   <211> 20
   <212> DNA
   <213> Artificial Sequence
<220>
   <223> Synthetic Primer: nbla21578-1f(k)
<400> 933
   ctcctcctgt tgctgatcct 20
<210> 934
   <211> 20
   <212> DNA
   <213> Artificial Sequence
<220>
   <223> Synthetic Primer: nbla21578-1r
<400> 934
   tggtgtcagt gctgttcctc 20
<210> 935
   <211> 19
   <212> DNA
   <213> Artificial Sequence
<220>
   <223> Synthetic Primer: nbla21614-1f(k)
<400> 935
   tggtatgagc caatgcaga 19
<210> 936
   <211> 22
   <212> DNA
   <213> Artificial Sequence
<220>
   <223> Synthetic Primer: nbla21614-1r
<400> 936
   ctgtaaacca tgaagatgca ga 22
<210> 937
   <211> 21
   <212> DNA
   <213> Artificial Sequence
<220>
   <223> Synthetic Primer: nbla21624-1f(k)
<400> 937
   tgggaacata cgatgatgga g 21
<210> 938
   <211> 20
   <212> DNA
   <213> Artificial Sequence
<220>
   <223> Synthetic Primer: nbla21624-1r
<400> 938
   agtcttgctt ctgggggatg 20
<210> 939
   <211> 19
   <212> DNA
   <213> Artificial Sequence
<220>
   <223> Synthetic Primer: nbla21655-1f(k)
<400> 939
   tgtcattgtg ctggctgtg 19
<210> 940
   <211> 20
   <212> DNA
   <213> Artificial Sequence
<220>
   <223> Synthetic Primer: nbla21655-1r
<400> 940
   acctccacct tccctgttgt 20
<210> 941
   <211> 20
   <212> DNA
   <213> Artificial Sequence
<220>
   <223> Synthetic Primer: nbla21670-1f(k)
<400> 941
   gtctttgaac gccattaccc 20
<210> 942
   <211> 21
   <212> DNA
   <213> Artificial Sequence
<220>
   <223> Synthetic Primer: nbla21670-1r
<400> 942
   ttgttcccct atctacccac a 21
<210> 943
   <211> 23
   <212> DNA
   <213> Artificial Sequence
<220>
   <223> Synthetic Primer: nbla21787-1f(k)
<400> 943
   agccctctca ctatatgcta tcc 23
<210> 944
   <211> 23
   <212> DNA
   <213> Artificial Sequence
<220>
   <223> Synthetic Primer: nbla21787-1r
<400> 944
   gggtgtatat ttcctttgtg tcc 23
<210> 945
   <211> 22
   <212> DNA
   <213> Artificial Sequence
<220>
   <223> Synthetic Primer: nbla21954-1f(k)
<400> 945
   ccagcttcct acaacaccat ct 22
<210> 946
   <211> 20
   <212> DNA
   <213> Artificial Sequence
<220>
   <223> Synthetic Primer: nbla21954-1r
<400> 946
tacaagccaa cgctttctcc 20
   <210> 947
<211> 21
   <212> DNA
   <213> Artificial Sequence
<220>
   <223> Synthetic Primer: nbla21979-1f(k)
<400> 947
   catgtagtgg gttcggagat g 21
<210> 948
   <211> 20
   <212> DNA
   <213> Artificial Sequence
<220>
   <223> Synthetic Primer: nbla21979-1r
<400> 948
   cgtagccatc agtgcaagag 20
<210> 949
   <211> 19
   <212> DNA
   <213> Artificial Sequence
<220>
   <223> Synthetic Primer: nbla22043-1f(k)
<400> 949
   ggcccagaac aactgctac 19
<210> 950
   <211> 18
   <212> DNA
   <213> Artificial Sequence
<220>
   <223> Synthetic Primer: nbla22043-1r
<400> 950
   aggccaccct ccttcttc 18
<210> 951
   <211> 18
   <212> DNA
   <213> Artificial Sequence
<220>
   <223> Synthetic Primer: nbla22137r1-1f(k)
<400> 951
   aggcattaag ggcacacc 18
<210> 952
   <211> 21
   <212> DNA
   <213> Artificial Sequence
<220>
   <223> Synthetic Primer: nbla22137r1-1r
<400> 952
   ctgcaagtaa ataggcccag a 21
<210> 953
   <211> 20
   <212> DNA
   <213> Artificial Sequence
<220>
   <223> Synthetic Primer: nbla22192-1f(k)
<400> 953
   cgttatggtg gtcatgttgg 20
<210> 954
   <211> 20
   <212> DNA
   <213> Artificial Sequence
<220>
   <223> Synthetic Primer: nbla22192-1r
<400> 954
   tgccttcttc ctgctgttct 20
<210> 955
   <211> 20
   <212> DNA
   <213> Artificial Sequence
<220>
   <223> Synthetic Primer: nbla22325d-1f(k)
<400> 955
   ccattgtact gcccgtctct 20
<210> 956
   <211> 19
   <212> DNA
   <213> Artificial Sequence
<220>
   <223> Synthetic Primer: nbla22325d-1r
<400> 956
   gtccccactt tccatcacc 19
<210> 957
   <211> 20
   <212> DNA
   <213> Artificial Sequence
<220>
   <223> Synthetic Primer: nbla22327-1f(k)
<400> 957
   tgtttgcttc ttgccatcac 20
<210> 958
   <211> 22
   <212> DNA
   <213> Artificial Sequence
<220>
   <223> Synthetic Primer: nbla22327-1r
<400> 958
   tgcctcttta tcacctacca ca 22
<210> 959
   <211> 21
   <212> DNA
   <213> Artificial Sequence
<220>
   <223> Synthetic Primer: nbla22337-1f(k)
<400> 959
   ggctgttctt accatctcct t 21
<210> 960
   <211> 23
   <212> DNA
   <213> Artificial Sequence
<220>
   <223> Synthetic Primer: nbla22337-1r
<400> 960
   agctcctgct aaattctaac ctc 23
<210> 961
   <211> 20
   <212> DNA
   <213> Artificial Sequence
<220>
   <223> Synthetic Primer: nbla22482-1f(k)
<400> 961
   gctgcgtctc atacaaacca 20
<210> 962
   <211> 22
   <212> DNA
   <213> Artificial Sequence
<220>
   <223> Synthetic Primer: nbla22482-1r
<400> 962
   catccacagc aactttcaca tc 22
<210> 963
   <211> 20
   <212> DNA
   <213> Artificial Sequence
<220>
   <223> Synthetic Primer: nbla22763-1f(k)
<400> 963
   cagcacagca actcaggaac 20
<210> 964
   <211> 18
   <212> DNA
   <213> Artificial Sequence
<220>
   <223> Synthetic Primer: nbla22763-1r
<400> 964
   tggcaaactt gaggcaga 18
<210> 965
   <211> 19
   <212> DNA
   <213> Artificial sequence
<220>
   <223> synthetic Primer: nbla22788-1f(k)
<400> 965
   ctggatcagg tttcccaca 19
<210> 966
   <211> 20
   <212> DNA
   <213> Artificial Sequence
<220>
   <223> Synthetic Primer: nbla22788-1r
<400> 966
   aggcagctca aatccttcac 20
<210> 967
   <211> 19
   <212> DNA
   <213> Artificial Sequence
<220>
   <223> Synthetic Primer: nbla22839-1f(k)
<400> 967
   tgtcatcacg cttcccttc 19
<210> 968
   <211> 20
   <212> DNA
   <213> Artificial Sequence
<220>
   <223> Synthetic Primer: nbla22839-1r
<400> 968
   gacgccaaca tagaccacct 20
<210> 969
   <211> 20
   <212> DNA
   <213> Artificial Sequence
<220>
   <223> synthetic Primer: nbla22851-1f(k)
<400> 969
   atgcctctgc ctcatctcac 20
<210> 970
   <211> 20
   <212> DNA
   <213> Artificial Sequence
<220>
   <223> synthetic Primer: nbla22851-1r
<400> 970
   gctctgcctg ctgactctct 20
<210> 971
   <211> 22
   <212> DNA
   <213> Artificial Sequence
<220>
   <223> Synthetic Primer: nbla22935-1f(k)
<400> 971
   tgactaacgc tcacataact gg 22
<210> 972
   <211> 22
   <212> DNA
   <213> Artificial Sequence
<220>
   <223> Synthetic Primer: nbla22935-1r
<400> 972
   tgcttacctt cttgcttaat gg 22
<210> 973
   <211> 20
   <212> DNA
   <213> Artificial Sequence
<220>
   <223> Synthetic Primer: nbla22937-1f(k)
<400> 973
   gcagtttgag ggtgttttgg 20
<210> 974
   <211> 21
   <212> DNA
   <213> Artificial Sequence
<220>
   <223> Synthetic Primer: nbla22937-1r
<400> 974
   atttctactg gggagggagg a 21
<210> 975
   <211> 23
   <212> DNA
   <213> Artificial Sequence
<220>
   <223> Synthetic Primer: nbla23238-1f(k)
<400> 975
   gccactcctt ctcagtcttc atc 23
<210> 976
   <211> 20
   <212> DNA
   <213> Artificial Sequence
<220>
   <223> Synthetic Primer: nbla23238-1r
<400> 976
   gttccatcaa ctcccaagca 20
<210> 977
   <211> 22
   <212> DNA
   <213> Artificial Sequence
<220>
   <223> Synthetic Primer: nbla23327-1f(k)
<400> 977
   gaagggctac tctatggtga gg 22
<210> 978
   <211> 20
   <212> DNA
   <213> Artificial Sequence
<220>
   <223> Synthetic Primer: nbla23327-1r
<400> 978
   aatggactgg tggaacttgg 20
<210> 979
   <211> 19
   <212> DNA
   <213> Artificial Sequence
<220>
   <223> synthetic Primer: nbla23360-1f(k)
<400> 979
   gacgtgctca aggaagtgg 19
<210> 980
   <211> 21
   <212> DNA
   <213> Artificial Sequence
<220>
   <223> Synthetic Primer: nbla23360-1r
<400> 980
   tgatgaactc gacccagaga g 21
<210> 981
   <211> 20
   <212> DNA
   <213> Artificial Sequence
<220>
   <223> Synthetic Primer: nbla23519-1f(k)
<400> 981
   gaacaggatt tcccctagca 20
<210> 982
   <211> 20
   <212> DNA
   <213> Artificial Sequence
<220>
   <223> Synthetic Primer: nbla23519-1r
<400> 982
   ctctgaaaga cccccacatc 20
<210> 983
   <211> 20
   <212> DNA
   <213> Artificial Sequence
<220>
   <223> synthetic Primer: nbla23553-1f(k)
<400> 983
   cagagggagg gtgttacgag 20
<210> 984
   <211> 18
   <212> DNA
   <213> Artificial Sequence
<220>
   <223> synthetic Primer: nbla23553-1r
<400> 984
   ggcacgatat tgggatgg 18
<210> 985
<211> 20
   <212> DNA
   <213> Artificial Sequence
<220>
   <223> Synthetic Primer: nbla23554-1f(k)
<400> 985
   gccaaagtgt atgggatgct 20
<210> 986
   <211> 21
   <212> DNA
   <213> Artificial Sequence
<220>
   <223> synthetic Primer: nbla23554-1r
<400> 986
   ctggacctgt gtgaactgat g 21
<210> 987
   <211> 20
   <212> DNA
   <213> Artificial Sequence
<220>
   <223> Synthetic Primer: nbla23683-1f(k)
<400> 987
   tctgtgacca gggttttgtg 20
<210> 988
   <211> 20
   <212> DNA
   <213> Artificial Sequence
<220>
   <223> Synthetic Primer: nbla23683-1r
<400> 988
   cacacgagaa gtggatggtg 20
<210> 989
   <211> 19
   <212> DNA
   <213> Artificial Sequence
<220>
   <223> Synthetic Primer: nbla23812-1f(k)
<400> 989
   ctgcacacag ccacgattt 19
<210> 990
   <211> 22
   <212> DNA
   <213> Artificial Sequence
<220>
   <223> Synthetic Primer: nbla23812-1r
<400> 990
   tggcaggtta aatgtcttct cc 22
<210> 991
   <211> 21
   <212> DNA
   <213> Artificial Sequence
<220>
   <223> Synthetic Primer: nbla23823-1f(k)
<400> 991
   gccagagtcc cagctttcta c 21
<210> 992
   <211> 20
   <212> DNA
   <213> Artificial Sequence
<220>
   <223> Synthetic Primer: nbla23823-1r
<400> 992
   agttgtccct tcctcgcttc 20
<210> 993
   <211> 19
   <212> DNA
   <213> Artificial Sequence
<220>
   <223> Synthetic Primer: nbla23849-1f(k)
<400> 993
   agcaacacgc aaacgagag 19
<210> 994
   <211> 21
   <212> DNA
   <213> Artificial Sequence
<220>
   <223> Synthetic Primer: nbla23849-1r
<400> 994
   gcatctcctg ccttgattag a 21
<210> 995
   <211> 20
   <212> DNA
   <213> Artificial Sequence
<220>
   <223> Synthetic Primer: nbla23882-1f(k)
<400> 995
   tgctactggg agctgatgtg 20
<210> 996
   <211> 20
   <212> DNA
   <213> Artificial Sequence
<220>
   <223> Synthetic Primer: nbla23882-1r
<400> 996
   cggatggcaa acttctctgt 20
<210> 997
   <211> 21
   <212> DNA
   <213> Artificial Sequence
<220>
   <223> Synthetic Primer: nbla23910rl-1f(k)
<400> 997
   catggaaaca acgaaggaac a 21
<210> 998
   <211> 19
   <212> DNA
   <213> Artificial Sequence
<220>
   <223> Synthetic Primer: nbla23910r1-1r
<400> 998
   gacttggggt tggaacagg 19
<210> 999
   <211> 18
   <212> DNA
   <213> Artificial Sequence
<220>
   <223> Synthetic Primer: nbla24064-1f(k)
<400> 999
   cggaggagaa acggaggt 18
<210> 1000
   <211> 19
   <212> DNA
   <213> Artificial Sequence
<220>
   <223> Synthetic Primer: nbla24064-1r
<400> 1000
   gctattgacc cgtgggaag 19
<210> 1001
   <211> 20
   <212> DNA
   <213> Artificial Sequence
<220>
   <223> synthetic Primer: nbla24405-1f(k)
<400> 1001
   agccagtaca cgcaggaaac 20
<210> 1002
   <211> 21
   <212> DNA
   <213> Artificial Sequence
<220>
   <223> Synthetic Primer: nbla24405-1r
<400> 1002
   catcaaacca cctccacaag a 21
<210> 1003
   <211> 20
   <212> DNA
   <213> Artificial Sequence
<220>
   <223> synthetic Primer: nbla24897-1f(k)
<400> 1003
   aggagtttgc tgctgctctc 20
<210> 1004
   <211> 20
   <212> DNA
   <213> Artificial Sequence
<220>
   <223> Synthetic Primer: nbla24897-1r
<400> 1004
   tcagtccctg cttccctatc 20
<210> 1005
   <211> 20
   <212> DNA
   <213> Artificial Sequence
<220>
   <223> Synthetic Primer: nbla24913-1f(k)
<400> 1005
   atcaggtggt ggaagatgga 20
<210> 1006
   <211> 20
   <212> DNA
   <213> Artificial Sequence
<220>
   <223> Synthetic Primer: nbla24913-1r
<400> 1006
   cggattagct gttcgaggtg 20
<210> 1007
   <211> 19
   <212> DNA
   <213> Artificial Sequence
<220>
   <223> Synthetic Primer: nbla20624d-1f(k)
<400> 1007
   ttctggtgcg agttttgga 19
<210> 1008
   <211> 20
   <212> DNA
   <213> Artificial Sequence
<220>
   <223> Synthetic Primer: nbla20624d-1r
<400> 1008
   tctgaatggg caagaaggag 20
<210> 1009
   <211> 21
   <212> DNA
   <213> Artificial Sequence
<220>
   <223> Synthetic Primer: nbla22029-1f(k)
<400> 1009
   cagggacagg aaagatagga g 21
<210> 1010
   <211> 20
   <212> DNA
   <213> Artificial Sequence
<220>
   <223> Synthetic Primer: nbla22029-1r
<400> 1010
   gctgaactct ggatgtctgg 20
<210> 1011
   <211> 21
   <212> DNA
   <213> Artificial Sequence
<220>
   <223> Synthetic Primer: nbla22424r1d-1f(k)
<400> 1011
   tgcaccagct ctttcttctg t 21
<210> 1012
   <211> 21
   <212> DNA
   <213> Artificial Sequence
<220>
   <223> Synthetic Primer: nbla22424r1s-1r
<400> 1012
   catgatcctc tcctgcatct c 21
<210> 1013
   <211> 24
   <212> DNA
   <213> Artificial Sequence
<220>
   <223> Synthetic Primer: nbla22594-1f(k)
<400> 1013
   cacgatattc agaccttgac tttg 24
<210> 1014
   <211> 20
   <212> DNA
   <213> Artificial Sequence
<220>
   <223> Synthetic Primer: nbla22594-1r
<400> 1014
   agcatccttt gcctctgtgt 20
<210> 1015
   <211> 19
   <212> DNA
   <213> Artificial Sequence
<220>
   <223> Synthetic Primer: nbla22622-1f(k)
<400> 1015
   gcaagggggt cttcttcct 19
<210> 1016
   <211> 19
   <212> DNA
   <213> Artificial Sequence
<220>
   <223> Synthetic Primer: nbla22622-1r
<400> 1016
   ggctggcaag ttcattcct 19
<210> 1017
   <211> 20
   <212> DNA
   <213> Artificial Sequence
<220>
   <223> Synthetic Primer: nbla20117d-1f(k)
<400> 1017
   tggaccttgt ggttgagttg 20

<210> 1018
   <211> 20
   <212> DNA
   <213> Artificial Sequence
<220>
   <223> Synthetic Primer: nbla20117-1r
<400> 1018
   ctcttttgga ttgctgcttg 20
<210> 1019
   <211> 18
   <212> DNA
   <213> Artificial Sequence
<220>
   <223> Synthetic Primer: nbla20238-1f(k)
<400> 1019
   cgtggggatg tagcagga 18
<210> 1020
   <211> 20
   <212> DNA
   <213> Artificial Sequence
<220>
   <223> Synthetic Primer: nbla20238-1r
<400> 1020
   ctggaaagat ggggaaggag 20
<210> 1021
   <211> 21
   <212> DNA
   <213> Artificial Sequence
<220>
   <223> Synthetic Primer: nbla20904-1f(k)
<400> 1021
   acgtggattt atggtctgtg g 21
<210> 1022
   <211> 20
   <212> DNA
   <213> Artificial Sequence
<220>
   <223> Synthetic Primer: nbla20904-1r
<400> 1022
   tgggaaaagg acatcaggaa 20
<210> 1023
   <211> 20
   <212> DNA
   <213> Artificial Sequence
<220>
   <223> synthetic Primer: nbla23293-1f(k)
<400> 1023
   tgatgctggg caactacaga 20
<210> 1024
   <211> 20
   <212> DNA
   <213> Artificial Sequence
<220>
   <223> Synthetic Primer: nbla23293-1r
<400> 1024
   tccaaaacta gccaggagga 20
<210> 1025
   <211> 22
   <212> DNA
   <213> Artificial Sequence
<220>
   <223> Synthetic Primer: nbla23297d-1f(k)
<400> 1025
   acaagaaagc agtggagagg ag 22
<210> 1026
   <211> 21
   <212> DNA
   <213> Artificial Sequence
<220>
   <223> synthetic Primer: nbla23297d-1r
<400> 1026
   gttttgctgt tggtcacttg g 21
<210> 1027
   <211> 21
   <212> DNA
   <213> Artificial Sequence
<220>
   <223> Synthetic Primer: nbla23311-1f(k)
<400> 1027
   tctccgttgg tctcactgtc t 21
<210> 1028
   <211> 21
   <212> DNA
   <213> Artificial Sequence
<220>
   <223> Synthetic Primer: nbla23311-1r
<400> 1028
   ggccacaatt tccatatcct c 21
<210> 1029
   <211> 22
   <212> DNA
   <213> Artificial Sequence
<220>
   <223> Synthetic Primer: nbla23589-1f(k)
<400> 1029
   gaagcatgag cccgtattta tc 22
<210> 1030
   <211> 22
   <212> DNA
   <213> Artificial Sequence
<220>
   <223> Synthetic Primer: nbla23589-1r
<400> 1030
   tccacaactt cataatccca ca 22
<210> 1031
   <211> 19
   <212> DNA
   <213> Artificial Sequence
<220>
   <223> Synthetic Primer: nbla23629r1-1f(k)
<400> 1031
   gtggtcgcac ctccattct 19
<210> 1032
   <211> 19
   <212> DNA
   <213> Artificial Sequence
<220>
   <223> Synthetic Primer: nbla23629r1-1r
<400> 1032
   acatgcggtg gatttttgg 19
<210> 1033
   <211> 20
   <212> DNA
   <213> Artificial Sequence
<220>
   <223> Synthetic Primer: nbla23862d-1df(k)
<400> 1033
   gctcctgtga tctggatgga 20
<210> 1034
   <211> 20
   <212> DNA
   <213> Artificial Sequence
<220>
   <223> Synthetic Primer: nbla23862d-1dr
<400> 1034
   ccaagtggga caaggtgaag 20
<210> 1035
   <211> 20
   <212> DNA
   <213> Artificial Sequence
<220>
   <223> Synthetic Primer: nbla24133r1-1f(k)
<400> 1035
   ccataagcca ccccacttac 20
<210> 1036
   <211> 19
   <212> DNA
   <213> Artificial Sequence
<220>
   <223> Synthetic Primer: nbla24133r1-1r
<400> 1036
   gagccttggg tcatttgct 19
<210> 1037
   <211> 18
   <212> DNA
   <213> Artificial Sequence
<220>
   <223> Synthetic Primer: nbla24761-1f(k)
<400> 1037
   atggagccac gaacaacc 18
<210> 1038
   <211> 23
   <212> DNA
   <213> Artificial Sequence
<220>
   <223> Synthetic Primer: nbla24761-1r
<400> 1038
   ggtctgggaa gtgtagttga aga 23
<210> 1039
   <211> 19
   <212> DNA
   <213> Artificial Sequence
<220>
   <223> Synthetic Primer: nbla20279-1f(k)
<400> 1039
   cctatggaca ccccaatcc 19
<210> 1040
   <211> 19
   <212> DNA
   <213> Artificial Sequence
<220>
   <223> Synthetic Primer: nbla20279-1r
<400> 1040
   ggcctgcttt agctccttc 19
<210> 1041
   <211> 19
   <212> DNA
   <213> Artificial Sequence
<220>
   <223> Synthetic Primer: nbla20687-1f(k)
<400> 1041
   ggcagacctc cagaccaac 19
<210> 1042
   <211> 20
   <212> DNA
   <213> Artificial Sequence
<220>
   <223> Synthetic Primer: nbla20687-1r
<400> 1042
   tgccacttcc actacccaga 20
<210> 1043
   <211> 19
   <212> DNA
   <213> Artificial Sequence
<220>
   <223> Synthetic Primer: nbla20924d-1f(k)
<400> 1043
   gcagcctcag ctcatacca 19
<210> 1044
   <211> 20
   <212> DNA
   <213> Artificial Sequence
<220>
   <223> Synthetic Primer: nbla20924d-1r
<400> 1044
   tccaaatctt ccaccaaacc 20
<210> 1045
   <211> 18
   <212> DNA
   <213> Artificial Sequence <220>
   <223> Synthetic Primer: nbla21168-1f(k)
<400> 1045
   caactccgtc agctcgtt 18
<210> 1046
   <211> 20
   <212> DNA
   <213> Artificial Sequence
<220>
   <223> Synthetic Primer: nbla21168-1r
<400> 1046
   ccagagcctt ttcattcttg 20
<210> 1047
   <211> 20
   <212> DNA
   <213> Artificial Sequence
<220>
   <223> Synthetic Primer: nbla21303-1f(k)
<400> 1047
   gttggctacc agaggaaatg 20
<210> 1048
   <211> 20
   <212> DNA
   <213> Artificial Sequence
<220>
   <223> Synthetic Primer: nbla21303-1r
<400> 1048
   tccacttaga aacggaagga 20
<210> 1049
   <211> 21
   <212> DNA
   <213> Artificial Sequence
<220>
   <223> Synthetic Primer: nbla21483-1f(k)
<400> 1049
   cacagcagaa aggaaaatgg a 21
<210> 1050
   <211> 20
   <212> DNA
   <213> Artificial Sequence
<220>
   <223> Synthetic Primer: nbla21483-1r
<400> 1050
   tgataagcag cactggatgg 20
<210> 1051
   <211> 23
   <212> DNA
   <213> Artificial Sequence
<220>
   <223> Synthetic Primer: nbla21838-1f(k)
<400> 1051
   ctagaatagg gaggtggaga atg 23
<210> 1052
   <211> 19
   <212> DNA
   <213> Artificial Sequence
<220>
   <223> Synthetic Primer: nbla21838-1r
<400> 1052
   ctgcgggttg gtaattgag 19
<210> 1053
   <211> 20
   <212> DNA
   <213> Artificial Sequence
<220>
   <223> Synthetic Primer: nbla21917-1f(k)
<400> 1053
   tgagttctgg attgcctgtg 20
<210> 1054
   <211> 20
   <212> DNA
   <213> Artificial Sequence
<220>
   <223> Synthetic Primer: nbla21917-1r
<400> 1054
   cagggcatgg attcttttct 20
<210> 1055
   <211> 20
   <212> DNA
   <213> Artificial Sequence
<220>
   <223> Synthetic Primer: nbla22099-1f(k)
<400> 1055
   ctggttccca cgcaagtaag 20
<210> 1056
   <211> 20
   <212> DNA
   <213> Artificial Sequence
<220>
   <223> Synthetic Primer: nbla22099-1r
<400> 1056
   ggttcatggc tctggaatgt 20
<210> 1057
   <211> 20
   <212> DNA
   <213> Artificial Sequence
<220>
   <223> Synthetic Primer: nbla22438-1f(k)
<400> 1057
   agcaggcatg gcaattttag 20
<210> 1058
   <211> 21
   <212> DNA
   <213> Artificial Sequence
<220>
   <223> Synthetic Primer: nbla22438-1r
<400> 1058
   ccagaggtgc agagaagtgt g 21
<210> 1059
   <211> 21
   <212> DNA
   <213> Artificial Sequence
<220>
   <223> Synthetic Primer: nbla23111d-1f(k)
<400> 1059
   attcaccctc tttggagaac a 21
<210> 1060
   <211> 20
   <212> DNA
   <213> Artificial Sequence
<220>
   <223> Synthetic Primer: nbla23111d-1r
<400> 1060
   ctaaaaggcg acagcacaag 20
<210> 1061
   <211> 20
   <212> DNA
   <213> Artificial Sequence
<220>
   <223> Synthetic Primer: nbla23208-1f(k)
<400> 1061
   tggtctcctt cctgtgttcc 20
<210> 1062
   <211> 19
   <212> DNA
   <213> Artificial Sequence
<220>
   <223> Synthetic Primer: nbla23208-1r
<400> 1062
   gttgcctgca ttctccaca 19
<210> 1063
   <211> 20
   <212> DNA
   <213> Artificial Sequence
<220>
   <223> Synthetic Primer: nbla24118-1f(k)
<400> 1063
   acaagtccac accacagcac 20
<210> 1064
   <211> 20
   <212> DNA
   <213> Artificial Sequence
<220>
   <223> Synthetic Primer: nbla24118-1r
<400> 1064
   gagaaaccag aggccagaga 20
<210> 1065
   <211> 20
   <212> DNA
   <213> Artificial Sequence
<220>
   <223> Synthetic Primer: nbla24279-1f(k)
<400> 1065
   tggtcgggtc acaaatcttc 20
<210> 1066
   <211> 19
   <212> DNA
   <213> Artificial Sequence
<220>
   <223> Synthetic Primer: nbla24279-1r
<400> 1066
   aaccacactc ctgcctcca 19
<210> 1067
   <211> 23
   <212> DNA
   <213> Artificial Sequence
<220>
   <223> Synthetic Primer: nbla24771d-1f(k)
<400> 1067
   caagtttgcc tccttcatag aca 23
<210> 1068
   <211> 24
   <212> DNA
   <213> Artificial Sequence
<220>
   <223> Synthetic Primer: nbla24771d-1r
<400> 1068
   tgtacgctta ttgatctcat cctc 24
<210> 1069
   <211> 20
   <212> DNA
   <213> Artificial Sequence
<220>
   <223> Synthetic Primer: nbla24871-1f(k)
<400> 1069
   cagcagggaa caaaactcca 20
<210> 1070
   <211> 21
   <212> DNA
   <213> Artificial Sequence
<220>
   <223> Synthetic Primer: nbla24871-1r
<400> 1070
   tggctacatg aaacgcatac c 21
<210> 1071
   <211> 19
   <212> DNA
   <213> Artificial Sequence
<220>
   <223> Synthetic Primer: nbla24443r1-1f(k)
<400> 1071
   gctgccactg ctatgctct 19
<210> 1072
   <211> 19
   <212> DNA
   <213> Artificial Sequence
<220>
   <223> Synthetic Primer: nbla24443r1-1r
<400> 1072
   catgctgttc tgcttgtgg 19
<210> 1073
   <211> 22
   <212> DNA
   <213> Artificial Sequence
<220>
   <223> Synthetic Primer: nbla23300-1f
<400> 1073
   gagagcagcg attaaccaaa ag 22
<210> 1074
   <211> 20
   <212> DNA
   <213> Artificial Sequence
<220>
   <223> Synthetic Primer: nbla23300-1r
<400> 1074
   acatcaac ttccctccaa 20
<210> 1075
   <211> 21
   <212> DNA
   <213> Artificial Sequence
<220>
   <223> Synthetic Primer: nbla23664-1f
<400> 1075
   ctttcatttc tcctgctgtc c 20
<210> 1076
   <211> 22
   <212> DNA
   <213> Artificial Sequence
<220>
   <223> Synthetic Primer: nbla23664-1r
<400> 1076
   gggactcacc cattttctat tt 22
<210> 1077
   <211> 20
   <212> DNA
   <213> Artificial Sequence
<220>
   <223> Synthetic Primer: GAPD forward
<400> 1077
   acctgacctg ccgtctagaa 20
<210> 1078
   <211> 20
   <212> DNA
   <213> Artificial Sequence
<220>
   <223> synthetic Primer: GAPD reverse
<400> 1078
   tccaccaccc tgttgctgta 20
<210> 1079
   <211> 27
   <212> RNA
   <213> Artificial Sequence
<220>
   <223> Synthetic oligo-RNA
<400> 1079
   agcaucgagu cggccuuggc cuacugg 27
<210> 1080
   <211> 42
   <212> DNA
   <213> Artificial Sequence
<220>
   <223> Synthetic primer: oligo-dT adapter
<400> 1080
   gcggctgaag acggcctatg tggccttttt tttttttttt tt 42
<210> 1081
   <211> 21
   <212> DNA
   <213> Artificial Sequence
<220>
   <223> Synthetic Primer: forward
<400> 1081
   agcatcgagt cggccttgtt g 21
<210> 1082
   <211> 20
   <212> DNA
   <213> Artificial Sequence
<220>
   <223> Synthetic Primer: reverse
<400> 1082
   gcgctgaaga cggcctatgt 20